(19) Europäisches Patentamt
European Patent Office
Office européen des brevets

(11) EP 1 734 051 A2

(12) # EUROPEAN PATENT APPLICATION

(43) Date of publication:
**20.12.2006 Bulletin 2006/51**

(21) Application number: **06014175.1**

(22) Date of filing: **30.11.1999**

(51) Int Cl.:
*C07K 14/47* (2006.01) *G01N 33/574* (2006.01)
*C07K 16/18* (2006.01) *C12Q 1/68* (2006.01)

(84) Designated Contracting States:
**AT BE CH CY DE DK ES FI FR GB GR IE IT LI LU MC NL PT SE**

(30) Priority:
**01.12.1998 WOPCT/US98/25108**
**16.12.1998 US 112850 P**
**12.01.1999 US 115554 P**
**08.03.1999 WOPCT/US99/05028**
**12.03.1999 US 123957 P**
**28.04.1999 US 131445 P**
**14.05.1999 US 134287 P**
**02.06.1999 WOPCT/US99/12252**
**23.06.1999 US 141037 P**
**20.07.1999 US 144758 P**
**26.07.1999 US 145698 P**
**01.09.1999 WOPCT/US99/20111**
**08.09.1999 WOPCT/US99/20594**
**13.09.1999 WOPCT/US99/20944**
**15.09.1999 WOPCT/US99/21090**
**15.09.1999 WOPCT/US99/21547**
**05.10.1999 WOPCT/US99/23089**
**29.10.1999 US 162506 P**

(62) Document number(s) of the earlier application(s) in accordance with Art. 76 EPC:
**99960624.7 / 1 135 485**

(71) Applicant: **Genetech, Inc.**
**South San Francisco, CA 94080-4990 (US)**

(72) Inventors:
- **Ashkenazi, Avi J.**
  **San Mateo, CA 94402 (US)**
- **Baker, Kevin P.**
  **Darnestown, MD 20878 (US)**
- **Ferrara, Napoleone**
  **San Francisco, CA 94109 (US)**
- **Gerber, Hanspeter**
  **San Francisco, CA 94107 (US)**
- **Hillan, Kenneth J.**
  **San Francisco, CA 94114 (US)**
- **Goddard, Audrey**
  **San Francisco, CA 94131 (US)**
- **Godowski, Paul J.**
  **Burlingame, CA 94010 (US)**
- **Gurney, Austin L.**
  **San Francisco, CA 94114 (US)**
- **Klein, Robert D.**
  **Palo Alto, CA 94301 (US)**
- **Kuo, Sophia S.**
  **San Francisco, CA 94131 (US)**
- **Paoni, Nicholas F.**
  **Belmont, CA 94002 (US)**
- **Smith, Victoria**
  **Burlingame, CA 94010 (US)**
- **Watanabe, Colin K.**
  **Moraga, CA 94556 (US)**
- **Williams, P. Mickey**
  **Half Moon Bay, CA 94019 (US)**
- **Wood, William I.**
  **Hillsborough, CA 94010 (US)**

(74) Representative: **Denison, Christopher Marcus et al**
**Mewburn Ellis LLP**
**York House**
**23 Kingsway**
**London WC2B 6HP (GB)**

Remarks:
•This application was filed on 07 - 07 - 2006 as a divisional application to the application mentioned under INID code 62.
•The sequence listing, which is published as annex to the application documents, was filed after the date of filing. The applicant has declared that it does not include matter which goes beyond the content of the application as filed.

(54) **Composition and methods for the diagnosis of tumours**

(57) Compositions and methods are disclosed for stimulating or inhibiting angiogenesis and/or cardiovascularization in mammals, including humans. Pharmaceutical compositions are based on polypeptides or antagonists thereto that have been identified for one or more of these uses. Disorders that can be diagnosed, prevented, or treated by the compositions herein include trauma such as wounds, various cancers, and disorders of the vessels including atherosclerosis and cardiac hypertrophy. In addition, the present invention is directed to novel





(Cont. next page)

polypeptides and to nucleic acid molecules encoding those polypeptides. Also provided herein are vectors and host cells comprising those nucleic acid sequences, chimeric polypeptide molecules comprising the polypeptides of the present invention fused to heterologous polypeptide sequences, antibodies which bind to the polypeptides of the present invention and to methods for producing the polypeptides of the present invention.

Annex to the application documents - subsequently filed sequences listing

Sequence Listing

<110> GENENTECH, INC.

<120> PROMOTION OR INHIBITION OF ANGIOGENESIS AND CARDIOVASCULARIZATION

<130> CMD/FP6188379

<140> EP06014175.1
<141> 1999-11-30

<150> EP99960624.7
<151> 1999-11-30

<150> PCT/US99/28313
<151> 1999-11-30

<150> PCT/US98/25108
<151> 1998-12-01

<150> US 60/112,850
<151> 1998-12-16

<150> US 60/115,554
<151> 1999-01-12

<150> PCT/US99/05028
<151> 1999-03-08

<150> US 60/123,957
<151> 1999-03-12

<150> US 60/131,445
<151> 1999-04-28

<150> US 60/134,287
<151> 1999-05-14

<150> PCT/US99/12252
<151> 1999-06-02

<150> US 60/141,037
<151> 1999-06-23

<150> US 60/144,758
<151> 1999-07-20

<150> US 60/145,698
<151> 1999-07-26

<150> PCT/US99/20111
<151> 1999-09-01

<150> PCT/US99/20594
<151> 1999-09-08

<150> PCT/US99/20944
<151> 1999-09-13

<150> PCT/US99/21090
<151> 1999-09-15

<150> PCT/US99/21547
<151> 1999-09-15

<150> PCT/US99/23089
<151> 1999-10-05

<150> US 60/162,506
<151> 1999-10-29

<160> 260

<210> 1
<211> 43
<212> DNA
<213> Artificial Sequence

<220>
<223> Synthetic Oligonucleotide Probe

<400> 1
tgtaaaacga cggccagtta aatagacctg caattattaa tct 43

<210> 2
<211> 41
<212> DNA
<213> Artificial sequence

<220>
<223> Synthetic Oligonucleotide Probe

<400> 2
caggaaacag ctatgaccac ctgcacacct gcaaatccat t 41

<210> 3
<211> 2933
<212> DNA
<213> Homo Sapien

<400> 3
tggggggcccc ccaggctcgc gcgtggagcg aagcagcatg ggcagtcggt 50

gcgcgctggc cctggcggtg ctctcggcct tgctgtgtca ggtctggagc 100

tctggggtgt tcgaactgaa gctgcaggag ttcgtcaaca agaaggggct 150

gctggggaac cgcaattgct gccgcggggg cgcggggcca ccgccgtgcg 200

cctgccggac cttcttccgc gtgtgcctca agcactacca ggccagcgtg 250

tcccccgagc cgccctgcac ctacggcagc gccgtcaccc ccgtgctggg 300

cgtcgactcc ttcagtctgc ccgacggcgg gggcgccgac tccgcgttca 350

gcaaccccat ccgcttcccc ttcggcttca cctggccggg caccttctct 400
ctgattattg aagctctcca cacagattct cctgatgacc tcgcaacaga 450

aaacccagaa agactcatca gccgcctggc cacccagagg cacctgacgg 500

tgggcgagga gtggtcccag gacctgcaca gcagcggccg cacggacctc 550

aagtactcct accgcttcgt gtgtgacgaa cactactacg gagagggctg 600

ctccgttttc tgccgtcccc gggacgatgc cttcggccac ttcacctgtg 650
gggagcgtgg ggagaaagtg tgcaaccctg gctggaaagg gccctactgc 700
acagagccga tctgcctgcc tggatgtgat gagcagcatg gattttgtga 750
caaaccaggg gaatgcaagt gcagagtggg ctggcagggc cggtactgtg 800
acgagtgtat ccgctatcca ggctgtctcc atggcacctg ccagcagccc 850
tggcagtgca actgccagga aggctggggg ggccttttct gcaaccagga 900
cctgaactac tgcacacacc ataagccctg caagaatgga gccacctgca 950
ccaacacggg ccaggggagc tacacttgct cttgccggcc tgggtacaca 1000
ggtgccacct gcgagctggg gattgacgag tgtgacccca gcccttgtaa 1050
gaacggaggg agctgcacgg atctcgagaa cagctactcc tgtacctgcc 1100
cacccggctt ctacggcaaa atctgtgaat tgagtgccat gacctgtgcg 1150
gacggccctt gctttaacgg gggtcggtgc tcagacagcc ccgatggagg 1200
gtacagctgc cgctgccccg tgggctactc cggcttcaac tgtgagaaga 1250
aaattgacta ctgcagctct tcaccctgtt ctaatggtgc caagtgtgtg 1300
gacctcggtg atgcctacct gtgccgctgc caggccggct tctcggggag 1350
gcactgtgac gacaacgtgg acgactgcgc ctcctccccg tgcgccaacg 1400
gggcacctg ccgggatggc gtgaacgact tctcctgcac ctgcccgcct 1450
ggctacacgg gcaggaactg cagtgccccc gtcagcaggt gcgagcacgc 1500
accctgccac aatggggcca cctgccacga gaggggccac cgctatgtgt 1550
gcgagtgtgc ccgaggctac gggggtccca actgccagtt cctgctcccc 1600
gagctgcccc cgggcccagc ggtggtggac ctcactgaga agctagaggg 1650
ccagggcggg ccattcccct gggtggccgt gtgcgccggg gtcatccttg 1700
tcctcatgct gctgctgggc tgtgccgctg tggtggtctg cgtccggctg 1750
aggctgcaga agcaccggcc cccagccgac ccctgccggg gggagacgga 1800
gaccatgaac aacctggcca actgccagcg tgagaaggac atctcagtca 1850
gcatcatcgg ggccacgcag atcaagaaca ccaacaagaa ggcggacttc 1900
cacggggacc acagcgccga caagaatggc ttcaaggccc gctacccagc 1950
ggtggactat aacctcgtgc aggacctcaa gggtgacgac accgccgtca 2000
gggacgcgca cagcaagcgt gacaccaagt gccagcccca gggctcctca 2050
ggggaggaga aggggacccc gaccacactc aggggtggag aagcatctga 2100
aagaaaaagg ccggactcgg gctgttcaac ttcaaaagac accaagtacc 2150
agtcggtgta cgtcatatcc gaggagaagg atgagtgcgt catagcaact 2200

gaggtgtaaa atggaagtga gatggcaaga ctcccgtttc tcttaaaata 2250
agtaaaattc caaggatata tgccccaacg aatgctgctg aagaggaggg 2300
aggcctcgtg gactgctgct gagaaaccga gttcagaccg agcaggttct 2350
cctcctgagg tcctcgacgc ctgccgacag cctgtcgcgg cccggccgcc 2400
tgcggcactg ccttccgtga cgtcgccgtt gcactatgga cagttgctct 2450
taagagaata tatatttaaa tgggtgaact gaattacgca taagaagcat 2500
gcactgcctg agtgtatatt ttggattctt atgagccagt cttttcttga 2550
attagaaaca caaacactgc ctttattgtc ctttttgata cgaagatgtg 2600
ctttttctag atggaaaaga tgtgtgttat tttttggatt tgtaaaaata 2650
tttttcatga tatctgtaaa gcttgagtat tttgtgatgt tcgtttttta 2700
taatttaaat tttggtaaat atgtacaaag gcacttcggg tctatgtgac 2750
tatatttttt tgtatataaa tgtatttatg gaatattgtg caaatgttat 2800
ttgagttttt tactgttttg ttaatgaaga aattcctttt taaaatattt 2850
ttccaaaata aattttatga atgacaaaaa aaaaaaaaaa aaaaaaaaaa 2900
aaaaaaaaaa aaaaaaaaaa aaaaaaaaaa aaa 2933

<210> 4
<211> 723
<212> PRT
<213> Homo Sapien

<400> 4
Met Gly Ser Arg Cys Ala Leu Ala Leu Ala Val Leu Ser Ala Leu
1 5 10 15

Leu Cys Gln Val Trp Ser Ser Gly Val Phe Glu Leu Lys Leu Gln
20 25 30

Glu Phe Val Asn Lys Lys Gly Leu Leu Gly Asn Arg Asn Cys Cys
35 40 45

Arg Gly Gly Ala Gly Pro Pro Pro Cys Ala Cys Arg Thr Phe Phe
50 55 60

Arg Val Cys Leu Lys His Tyr Gln Ala Ser Val Ser Pro Glu Pro
65 70 75

Pro Cys Thr Tyr Gly Ser Ala Val Thr Pro Val Leu Gly Val Asp
80 85 90

Ser Phe Ser Leu Pro Asp Gly Gly Gly Ala Asp Ser Ala Phe Ser
95 100 105

Asn Pro Ile Arg Phe Pro Phe Gly Phe Thr Trp Pro Gly Thr Phe
110 115 120

Ser Leu Ile Ile Glu Ala Leu His Thr Asp Ser Pro Asp Asp Leu
125 130 135

```
Ala Thr Glu Asn Pro Glu Arg Leu Ile Ser Arg Leu Ala Thr Gln
                140                 145                 150

Arg His Leu Thr Val Gly Glu Glu Trp Ser Gln Asp Leu His Ser
                155                 160                 165

Ser Gly Arg Thr Asp Leu Lys Tyr Ser Tyr Arg Phe Val Cys Asp
                170                 175                 180

Glu His Tyr Tyr Gly Glu Gly Cys Ser Val Phe Cys Arg Pro Arg
                185                 190                 195

Asp Asp Ala Phe Gly His Phe Thr Cys Gly Glu Arg Gly Glu Lys
                200                 205                 210

Val Cys Asn Pro Gly Trp Lys Gly Pro Tyr Cys Thr Glu Pro Ile
                215                 220                 225

Cys Leu Pro Gly Cys Asp Glu Gln His Gly Phe Cys Asp Lys Pro
                230                 235                 240

Gly Glu Cys Lys Cys Arg Val Gly Trp Gln Gly Arg Tyr Cys Asp
                245                 250                 255

Glu Cys Ile Arg Tyr Pro Gly Cys Leu His Gly Thr Cys Gln Gln
                260                 265                 270

Pro Trp Gln Cys Asn Cys Gln Glu Gly Trp Gly Gly Leu Phe Cys
                275                 280                 285

Asn Gln Asp Leu Asn Tyr Cys Thr His His Lys Pro Cys Lys Asn
                290                 295                 300

Gly Ala Thr Cys Thr Asn Thr Gly Gln Gly Ser Tyr Thr Cys Ser
                305                 310                 315

Cys Arg Pro Gly Tyr Thr Gly Ala Thr Cys Glu Leu Gly Ile Asp
                320                 325                 330

Glu Cys Asp Pro Ser Pro Cys Lys Asn Gly Gly Ser Cys Thr Asp
                335                 340                 345

Leu Glu Asn Ser Tyr Ser Cys Thr Cys Pro Pro Gly Phe Tyr Gly
                350                 355                 360

Lys Ile Cys Glu Leu Ser Ala Met Thr Cys Ala Asp Gly Pro Cys
                365                 370                 375

Phe Asn Gly Gly Arg Cys Ser Asp Ser Pro Asp Gly Gly Tyr Ser
                380                 385                 390

Cys Arg Cys Pro Val Gly Tyr Ser Gly Phe Asn Cys Glu Lys Lys
                395                 400                 405

Ile Asp Tyr Cys Ser Ser Ser Pro Cys Ser Asn Gly Ala Lys Cys
                410                 415                 420

Val Asp Leu Gly Asp Ala Tyr Leu Cys Arg Cys Gln Ala Gly Phe
                425                 430                 435

Ser Gly Arg His Cys Asp Asp Asn Val Asp Asp Cys Ala Ser Ser
                440                 445                 450
```

```
Pro Cys Ala Asn Gly Gly Thr Cys Arg Asp Gly Val Asn Asp Phe
                455                 460                 465

Ser Cys Thr Cys Pro Pro Gly Tyr Thr Gly Arg Asn Cys Ser Ala
                470                 475                 480

Pro Val Ser Arg Cys Glu His Ala Pro Cys His Asn Gly Ala Thr
                485                 490                 495

Cys His Glu Arg Gly His Arg Tyr Val Cys Glu Cys Ala Arg Gly
                500                 505                 510

Tyr Gly Gly Pro Asn Cys Gln Phe Leu Leu Pro Glu Leu Pro Pro
                515                 520                 525

Gly Pro Ala Val Val Asp Leu Thr Glu Lys Leu Glu Gly Gln Gly
                530                 535                 540

Gly Pro Phe Pro Trp Val Ala Val Cys Ala Gly Val Ile Leu Val
                545                 550                 555

Leu Met Leu Leu Leu Gly Cys Ala Ala Val Val Val Cys Val Arg
                560                 565                 570

Leu Arg Leu Gln Lys His Arg Pro Pro Ala Asp Pro Cys Arg Gly
                575                 580                 585

Glu Thr Glu Thr Met Asn Asn Leu Ala Asn Cys Gln Arg Glu Lys
                590                 595                 600

Asp Ile Ser Val Ser Ile Ile Gly Ala Thr Gln Ile Lys Asn Thr
                605                 610                 615

Asn Lys Lys Ala Asp Phe His Gly Asp His Ser Ala Asp Lys Asn
                620                 625                 630

Gly Phe Lys Ala Arg Tyr Pro Ala Val Asp Tyr Asn Leu Val Gln
                635                 640                 645

Asp Leu Lys Gly Asp Asp Thr Ala Val Arg Asp Ala His Ser Lys
                650                 655                 660

Arg Asp Thr Lys Cys Gln Pro Gln Gly Ser Ser Gly Glu Glu Lys
                665                 670                 675

Gly Thr Pro Thr Thr Leu Arg Gly Gly Glu Ala Ser Glu Arg Lys
                680                 685                 690

Arg Pro Asp Ser Gly Cys Ser Thr Ser Lys Asp Thr Lys Tyr Gln
                695                 700                 705

Ser Val Tyr Val Ile Ser Glu Glu Lys Asp Glu Cys Val Ile Ala
                710                 715                 720

Thr Glu Val
        723
```

<210> 5
<211> 22
<212> DNA
<213> Artificial Sequence

<220>
<223> Synthetic Oligonucleotide Probe

<400> 5
ggatctcgag aacagctact cc 22

<210> 6
<211> 22
<212> DNA
<213> Artificial Sequence

<220>
<223> Synthetic Oligonucleotide Probe

<400> 6
tcgtccacgt tgtcgtcaca tg 22

<210> 7
<211> 48
<212> DNA
<213> Artificial Sequence

<220>
<223> Synthetic Oligonucleotide Probe

<400> 7
aaatctgtga attgagtgcc atggacctgt tgcggacggc ccttgctt 48

<210> 8
<211> 1780
<212> DNA
<213> Homo Sapien

<400> 8
ggctcagagg ccccactgga ccctcggctc ttccttggac ttcttgtgtg 50

ttctgtgagc ttcgctggat tcagggtctt gggcatcaga ggtgagaggg 100

tgggaaggtc cgccgcgatg gggaagccct ggctgcgtgc gctacagctg 150

ctgctcctgc tgggcgcgtc gtgggcgcgg gcgggcgccc cgcgctgcac 200

ctacaccttc gtgctgcccc cgcagaagtt cacgggcgct gtgtgctgga 250

gcggccccgc atccacgcgg gcgacgcccg aggccgccaa cgccagcgag 300

ctggcggcgc tgcgcatgcg cgtcggccgc cacgaggagc tgttacgcga 350

gctgcagagg ctggcggcgg ccgacggcgc cgtggccggc gaggtgcgcg 400

cgctgcgcaa ggagagccgc ggcctgagcg cgcgcctggg ccagttgcgc 450

gcgcagctgc agcacgaggc ggggcccggg gcgggcccgg gggcggatct 500

gggggcggag cctgccgcgg cgctggcgct gctcggggag cgcgtgctca 550
acgcgtccgc cgaggctcag cgcgcagccg cccggttcca ccagctggac 600

gtcaagttcc gcgagctggc gcagctcgtc acccagcaga gcagtctcat 650

cgcccgcctg gagcgcctgt gcccgggagg cgcgggcggg cagcagcagg 700

tcctgccgcc acccccactg gtgcctgtgg ttccggtccg tcttgtgggt 750

agcaccagtg acaccagtag gatgctggac ccagccccag agccccagag 800

agaccagacc cagagacagc aggagcccat ggcttctccc atgcctgcag 850

gtcaccctgc ggtccccacc aagcctgtgg gcccgtggca ggattgtgca 900

gaggcccgcc aggcaggcca tgaacagagt ggagtgtatg aactgcgagt 950

gggccgtcac gtagtgtcag tatggtgtga gcagcaactg gagggtggag 1000

gctggactgt gatccagcgg aggcaagatg gttcagtcaa cttcttcact 1050

acctggcagc actataaggc gggctttggg cggccagacg gagaatactg 1100

gctgggcctt gaacccgtgt atcagctgac cagccgtggg gaccatgagc 1150

tgctggttct cctggaggac tggggggccc gtggagcacg tgcccactat 1200

gatggcttct ccctggaacc cgagagcgac cactaccgcc tgcggcttgg 1250

ccagtaccat ggtgatgctg gagactctct ttcctggcac aatgacaagc 1300

ccttcagcac cgtggatagg gaccgagact cctattctgg taactgtgcc 1350

ctgtaccagc ggggaggctg gtggtaccat gcctgtgccc actccaacct 1400

caacggtgtg tggcaccacg gcggccacta ccgaagccgc taccaggatg 1450

gtgtctactg ggctgagttt cgtggtgggg catattctct caggaaggcc 1500

gccatgctca ttcggcccct gaagctgtga ctctgtgttc ctctgtcccc 1550

taggccctag aggacattgg tcagcaggag cccaagttgt tctggccaca 1600

ccttctttgt ggctcagtgc caatgtgtcc cacagaactt cccactgtgg 1650

atctgtgacc ctgggcgctg aaaatgggac ccaggaatcc cccccgtcaa 1700

tatcttggcc tcagatggct ccccaaggtc attcatatct cggtttgagc 1750

tcatatctta taataacaca aagtagccac 1780

<210> 9
<211> 470
<212> PRT
<213> Homo Sapien

<400> 9

Met Gly Lys Pro Trp Leu Arg Ala Leu Gln Leu Leu Leu Leu Leu
1 5 10 15

Gly Ala Ser Trp Ala Arg Ala Gly Ala Pro Arg Cys Thr Tyr Thr
20 25 30

Phe Val Leu Pro Pro Gln Lys Phe Thr Gly Ala Val Cys Trp Ser
35 40 45

Gly Pro Ala Ser Thr Arg Ala Thr Pro Glu Ala Ala Asn Ala Ser
50 55 60

Glu Leu Ala Ala Leu Arg Met Arg Val Gly Arg His Glu Glu Leu
65 70 75

Leu Arg Glu Leu Gln Arg Leu Ala Ala Ala Asp Gly Ala Val Ala
80 85 90

Gly Glu Val Arg Ala Leu Arg Lys Glu Ser Arg Gly Leu Ser Ala
95 100 105

Arg Leu Gly Gln Leu Arg Ala Gln Leu Gln His Glu Ala Gly Pro
110 115 120

Gly Ala Gly Pro Gly Ala Asp Leu Gly Ala Glu Pro Ala Ala Ala
125 130 135

Leu Ala Leu Leu Gly Glu Arg Val Leu Asn Ala Ser Ala Glu Ala
140 145 150

Gln Arg Ala Ala Ala Arg Phe His Gln Leu Asp Val Lys Phe Arg
155 160 165

Glu Leu Ala Gln Leu Val Thr Gln Gln Ser Ser Leu Ile Ala Arg
170 175 180

Leu Glu Arg Leu Cys Pro Gly Gly Ala Gly Gly Gln Gln Gln Val
185 190 195

Leu Pro Pro Pro Pro Leu Val Pro Val Val Pro Val Arg Leu Val
200 205 210

Gly Ser Thr Ser Asp Thr Ser Arg Met Leu Asp Pro Ala Pro Glu
215 220 225

Pro Gln Arg Asp Gln Thr Gln Arg Gln Gln Glu Pro Met Ala Ser
230 235 240

Pro Met Pro Ala Gly His Pro Ala Val Pro Thr Lys Pro Val Gly
245 250 255

Pro Trp Gln Asp Cys Ala Glu Ala Arg Gln Ala Gly His Glu Gln
260 265 270

Ser Gly Val Tyr Glu Leu Arg Val Gly Arg His Val Val Ser Val
275 280 285

Trp Cys Glu Gln Gln Leu Glu Gly Gly Gly Trp Thr Val Ile Gln
290 295 300

Arg Arg Gln Asp Gly Ser Val Asn Phe Phe Thr Thr Trp Gln His
305 310 315

Tyr Lys Ala Gly Phe Gly Arg Pro Asp Gly Glu Tyr Trp Leu Gly
320 325 330

Leu Glu Pro Val Tyr Gln Leu Thr Ser Arg Gly Asp His Glu Leu
335 340 345

Leu Val Leu Leu Glu Asp Trp Gly Gly Arg Gly Ala Arg Ala His
350 355 360

Tyr Asp Gly Phe Ser Leu Glu Pro Glu Ser Asp His Tyr Arg Leu
365 370 375

Arg Leu Gly Gln Tyr His Gly Asp Ala Gly Asp Ser Leu Ser Trp
380 385 390

```
His Asn Asp Lys Pro Phe Ser Thr Val Asp Arg Asp Arg Asp Ser
                395                 400                 405

Tyr Ser Gly Asn Cys Ala Leu Tyr Gln Arg Gly Gly Trp Trp Tyr
                410                 415                 420

His Ala Cys Ala His Ser Asn Leu Asn Gly Val Trp His His Gly
                425                 430                 435

Gly His Tyr Arg Ser Arg Tyr Gln Asp Gly Val Tyr Trp Ala Glu
                440                 445                 450

Phe Arg Gly Gly Ala Tyr Ser Leu Arg Lys Ala Ala Met Leu Ile
                455                 460                 465

Arg Pro Leu Lys Leu
                470
```

<210> 10
<211> 34
<212> DNA
<213> Artificial Sequence

<220>
<223> Synthetic Oligonucleotide Probe

<400> 10
acgtagttcc agtatggtgt gagcagcaac tgga 34

<210> 11
<211> 26
<212> DNA
<213> Artificial Sequence

<220>
<223> Synthetic Oligonucleotide Probe

<400> 11
agtccagcct ccaccctcca gttgct 26

<210> 12
<211> 25
<212> DNA
<213> Artificial Sequence

<220>
<223> Synthetic Oligonucleotide Probe

<400> 12
ccccagtcct ccaggagaac cagca 25

<210> 13
<211> 2042
<212> DNA
<213> Homo Sapien

<400> 13
gcggacgcgt gggtgaaatt gaaaatcaag ataaaaatgt tcacaattaa 50

gctccttctt tttattgttc ctctagttat ttcctccaga attgatcaag 100

acaattcatc atttgattct ctatctccag agccaaaatc aagatttgct 150

```
atgttagacg atgtaaaaat tttagccaat ggcctccttc agttgggaca 200
tggtcttaaa gactttgtcc ataagacgaa gggccaaatt aatgacatat 250
ttcaaaaact caacatattt gatcagtctt tttatgatct atcgctgcaa 300
accagtgaaa tcaaagaaga agaaaaggaa ctgagaagaa ctacatataa 350
actacaagtc aaaaatgaag aggtaaagaa tatgtcactt gaactcaact 400
caaaacttga aagcctccta gaagaaaaaa ttctacttca acaaaaagtg 450
aaatatttag aagagcaact aactaactta attcaaaatc aacctgaaac 500
tccagaacac ccagaagtaa cttcacttaa aacttttgta gaaaaacaag 550
ataatagcat caaagacctt ctccagaccg tggaagacca atataaacaa 600
ttaaaccaac agcatagtca aataaaagaa atagaaaatc agctcagaag 650
gactagtatt caagaaccca cagaaatttc tctatcttcc aagccaagag 700
caccaagaac tactcccttt cttcagttga atgaaataag aaatgtaaaa 750
catgatggca ttcctgctga atgtaccacc atttataaca gaggtgaaca 800
tacaagtggc atgtatgcca tcagacccag caactctcaa gtttttcatg 850
tctactgtga tgttatatca ggtagtccat ggacattaat tcaacatcga 900
atagatggat cacaaaactt caatgaaacg tgggagaact acaaatatgg 950
ttttgggagg cttgatggag aattttggtt gggcctagag aagatatact 1000
ccatagtgaa gcaatctaat tatgttttac gaattgagtt ggaagactgg 1050
aaagacaaca aacattatat tgaatattct tttacttgg gaaatcacga 1100
aaccaactat acgctacatc tagttgcgat tactggcaat gtccccaatg 1150
caatcccgga aaacaaagat ttggtgtttt ctacttggga tcacaaagca 1200
aaaggacact tcaactgtcc agagggttat tcaggaggct ggtggtggca 1250
tgatgagtgt ggagaaaaca acctaaatgg taaatataac aaaccaagag 1300
caaaatctaa gccagagagg agaagaggat tatcttggaa gtctcaaaat 1350
ggaaggttat actctataaa atcaaccaaa atgttgatcc atccaacaga 1400
ttcagaaagc tttgaatgaa ctgaggcaat ttaaaggcat atttaaccat 1450
taactcattc caagttaatg tggtctaata atctggtata aatccttaag 1500
agaaagcttg agaaatagat ttttttatc ttaaagtcac tgtctattta 1550
agattaaaca tacaatcaca taaccttaaa gaataccgtt tacatttctc 1600
aatcaaaatt cttataatac tatttgtttt aaattttgtg atgtgggaat 1650
caattttaga tggtcacaat ctagattata atcaataggt gaacttatta 1700
```

aataactttt ctaaataaaa aatttagaga cttttatttt aaaaggcatc 1750

atatgagcta atatcacaac tttcccagtt taaaaaacta gtactcttgt 1800

taaaactcta aacttgacta aatacagagg actggtaatt gtacagttct 1850

taaatgttgt agtattaatt tcaaaactaa aaatcgtcag cacagagtat 1900

gtgtaaaaat ctgtaataca aatttttaaa ctgatgcttc attttgctac 1950

aaaataattt ggagtaaatg tttgatatga tttatttatg aaacctaatg 2000

aagcagaatt aaatactgta ttaaaataag ttcgctgtct tt 2042

<210> 14
<211> 460
<212> PRT
<213> Homo Sapien

<400> 14
Met Phe Thr Ile Lys Leu Leu Leu Phe Ile Val Pro Leu Val Ile
1 5 10 15

Ser Ser Arg Ile Asp Gln Asp Asn Ser Ser Phe Asp Ser Leu Ser
20 25 30

Pro Glu Pro Lys Ser Arg Phe Ala Met Leu Asp Asp Val Lys Ile
35 40 45

Leu Ala Asn Gly Leu Leu Gln Leu Gly His Gly Leu Lys Asp Phe
50 55 60

Val His Lys Thr Lys Gly Gln Ile Asn Asp Ile Phe Gln Lys Leu
65 70 75

Asn Ile Phe Asp Gln Ser Phe Tyr Asp Leu Ser Leu Gln Thr Ser
80 85 90

Glu Ile Lys Glu Glu Glu Lys Glu Leu Arg Arg Thr Thr Tyr Lys
95 100 105

Leu Gln Val Lys Asn Glu Glu Val Lys Asn Met Ser Leu Glu Leu
110 115 120

Asn Ser Lys Leu Glu Ser Leu Leu Glu Glu Lys Ile Leu Leu Gln
125 130 135

Gln Lys Val Lys Tyr Leu Glu Glu Gln Leu Thr Asn Leu Ile Gln
140 145 150

Asn Gln Pro Glu Thr Pro Glu His Pro Glu Val Thr Ser Leu Lys
155 160 165

Thr Phe Val Glu Lys Gln Asp Asn Ser Ile Lys Asp Leu Leu Gln
170 175 180
Thr Val Glu Asp Gln Tyr Lys Gln Leu Asn Gln Gln His Ser Gln
185 190 195

Ile Lys Glu Ile Glu Asn Gln Leu Arg Arg Thr Ser Ile Gln Glu
200 205 210

Pro Thr Glu Ile Ser Leu Ser Ser Lys Pro Arg Ala Pro Arg Thr
215 220 225

```
Thr Pro Phe Leu Gln Leu Asn Glu Ile Arg Asn Val Lys His Asp
                230                 235                 240

Gly Ile Pro Ala Glu Cys Thr Thr Ile Tyr Asn Arg Gly Glu His
                245                 250                 255

Thr Ser Gly Met Tyr Ala Ile Arg Pro Ser Asn Ser Gln Val Phe
                260                 265                 270

His Val Tyr Cys Asp Val Ile Ser Gly Ser Pro Trp Thr Leu Ile
                275                 280                 285

Gln His Arg Ile Asp Gly Ser Gln Asn Phe Asn Glu Thr Trp Glu
                290                 295                 300

Asn Tyr Lys Tyr Gly Phe Gly Arg Leu Asp Gly Glu Phe Trp Leu
                305                 310                 315

Gly Leu Glu Lys Ile Tyr Ser Ile Val Lys Gln Ser Asn Tyr Val
                320                 325                 330

Leu Arg Ile Glu Leu Glu Asp Trp Lys Asp Asn Lys His Tyr Ile
                335                 340                 345

Glu Tyr Ser Phe Tyr Leu Gly Asn His Glu Thr Asn Tyr Thr Leu
                350                 355                 360

His Leu Val Ala Ile Thr Gly Asn Val Pro Asn Ala Ile Pro Glu
                365                 370                 375

Asn Lys Asp Leu Val Phe Ser Thr Trp Asp His Lys Ala Lys Gly
                380                 385                 390

His Phe Asn Cys Pro Glu Gly Tyr Ser Gly Gly Trp Trp Trp His
                395                 400                 405

Asp Glu Cys Gly Glu Asn Asn Leu Asn Gly Lys Tyr Asn Lys Pro
                410                 415                 420

Arg Ala Lys Ser Lys Pro Glu Arg Arg Arg Gly Leu Ser Trp Lys
                425                 430                 435

Ser Gln Asn Gly Arg Leu Tyr Ser Ile Lys Ser Thr Lys Met Leu
                440                 445                 450

Ile His Pro Thr Asp Ser Glu Ser Phe Glu
                455                 460
```

<210> 15
<211> 715
<212> DNA
<213> Homo Sapien

<400> 15
tatttaccat atcagattca cattcagtcc tcagcaaaat gaagggctcc 50

attttcactc tgtttttatt ctctgtccta tttgccatct cagaagtgcg 100

gagcaaggag tctgtgagac tctgtgggct agaatacata cggacagtca 150

tctatatctg tgctagctcc aggtggagaa ggcatctgga ggggatccct 200

caagctcagc aagctgagac aggaaactcc ttccagctcc cacataaacg 250

tgagttttct gaggaaaatc cagcgcaaaa ccttccgaag gtggatgcct 300

caggggaaga ccgtctttgg ggtggacaga tgcccactga agagctttgg 350

aagtcaaaga agcattcagt gatgtcaaga caagatttac aaactttgtg 400

ttgcactgat ggctgttcca tgactgattt gagtgctctt tgctaagaca 450

agagcaaata cccaatgggt ggcagagctt tatcacatgt ttaattacag 500

tgttttactg cctggtagaa cactaatatt gtgttattaa aatgatggct 550

tttgggtagg caaaacttct tttctaaaag gtatagctga gcggttgaaa 600

ccacagtgat ctctattttc tccctttgcc aaggttaatg aactgttctt 650

ttcaaattct actaatgctt tgaaatttca aatgctgcgc aaaattgcaa 700

taaaaatgct ataaa 715

<210> 16
<211> 135
<212> PRT
<213> Homo Sapien

<400> 16
Met Lys Gly Ser Ile Phe Thr Leu Phe Leu Phe Ser Val Leu Phe
1 5 10 15

Ala Ile Ser Glu Val Arg Ser Lys Glu Ser Val Arg Leu Cys Gly
20 25 30

Leu Glu Tyr Ile Arg Thr Val Ile Tyr Ile Cys Ala Ser Ser Arg
35 40 45

Trp Arg Arg His Leu Glu Gly Ile Pro Gln Ala Gln Gln Ala Glu
50 55 60

Thr Gly Asn Ser Phe Gln Leu Pro His Lys Arg Glu Phe Ser Glu
65 70 75

Glu Asn Pro Ala Gln Asn Leu Pro Lys Val Asp Ala Ser Gly Glu
80 85 90

Asp Arg Leu Trp Gly Gly Gln Met Pro Thr Glu Glu Leu Trp Lys
95 100 105

Ser Lys Lys His Ser Val Met Ser Arg Gln Asp Leu Gln Thr Leu
110 115 120

Cys Cys Thr Asp Gly Cys Ser Met Thr Asp Leu Ser Ala Leu Cys
125 130 135

<210> 17
<211> 25
<212> DNA
<213> Artificial Sequence

<220>
<223> Synthetic Oligonucleotide Probe

<400> 17
cacattcagt cctcagcaaa atgaa 25

<210> 18
<211> 40
<212> DNA
<213> Artificial Sequence

<220>
<223> Synthetic Oligonucleotide Probe

<400> 18
gagaataaaa acagagtgaa aatggagccc ttcattttgc 40

<210> 19
<211> 23
<212> DNA
<213> Artificial Sequence

<220>
<223> Synthetic Oligonucleotide Probe

<400> 19
ctcagcttgc tgagcttgag gga 23

<210> 20
<211> 1200
<212> DNA
<213> Homo Sapien

<400> 20
cccacgcgtc cgaacctctc cagcgatggg agccgcccgc ctgctgccca 50

acctcactct gtgcttacag ctgctgattc tctgctgtca aactcagtac 100

gtgagggacc agggcgccat gaccgaccag ctgagcaggc ggcagatccg 150

cgagtaccaa ctctacagca ggaccagtgg caagcacgtg caggtcaccg 200

ggcgtcgcat ctccgccacc gccgaggacg gcaacaagtt tgccaagctc 250

atagtggaga cggacacgtt tggcagccgg gttcgcatca aaggggctga 300

gagtgagaag tacatctgta tgaacaagag gggcaagctc atcgggaagc 350

ccagcgggaa gagcaaagac tgcgtgttca cggagatcgt gctggagaac 400

aactatacgg ccttccagaa cgcccggcac gagggctggt tcatggcctt 450

cacgcggcag gggcggcccc gccaggcttc ccgcagccgc cagaaccagc 500

gcgaggccca cttcatcaag cgcctctacc aaggccagct gcccttcccc 550

aaccacgccg agaagcagaa gcagttcgag tttgtgggct ccgcccccac 600

ccgccggacc aagcgcacac ggcggcccca gcccctcacg tagtctggga 650

ggcagggggc agcagcccct gggccgcctc cccacccctt tcccttctta 700

atccaaggac tgggctgggg tggcgggagg ggagccagat ccccgaggga 750

ggaccctgag ggccgcgaag catccgagcc cccagctggg aaggggcagg 800

ccggtgcccc aggggcggct ggcacagtgc cccttcccg gacgggtggc 850

aggccctgga gaggaactga gtgtcaccct gatctcaggc caccagcctc 900

tgccggcctc ccagccgggc tcctgaagcc cgctgaaagg tcagcgactg 950

aaggccttgc agacaaccgt ctggaggtgg ctgtcctcaa aatctgcttc 1000

tcggatctcc ctcagtctgc cccagcccc caaactcctc ctggctagac 1050

tgtaggaagg gacttttgtt tgtttgtttg tttcaggaaa aaagaaaggg 1100

agagagagga aaatagaggg ttgtccactc ctcacattcc acgacccagg 1150

cctgcacccc acccccaact cccagccccg gaataaaacc atttcctgc 1200

<210> 21
<211> 205
<212> PRT
<213> Homo Sapien

<400> 21
Met Gly Ala Ala Arg Leu Leu Pro Asn Leu Thr Leu Cys Leu Gln
1 5 10 15

Leu Leu Ile Leu Cys Cys Gln Thr Gln Tyr Val Arg Asp Gln Gly
20 25 30

Ala Met Thr Asp Gln Leu Ser Arg Arg Gln Ile Arg Glu Tyr Gln
35 40 45

Leu Tyr Ser Arg Thr Ser Gly Lys His Val Gln Val Thr Gly Arg
50 55 60

Arg Ile Ser Ala Thr Ala Glu Asp Gly Asn Lys Phe Ala Lys Leu
65 70 75

Ile Val Glu Thr Asp Thr Phe Gly Ser Arg Val Arg Ile Lys Gly
80 85 90

Ala Glu Ser Glu Lys Tyr Ile Cys Met Asn Lys Arg Gly Lys Leu
95 100 105

Ile Gly Lys Pro Ser Gly Lys Ser Lys Asp Cys Val Phe Thr Glu
110 115 120

Ile Val Leu Glu Asn Asn Tyr Thr Ala Phe Gln Asn Ala Arg His
125 130 135

Glu Gly Trp Phe Met Ala Phe Thr Arg Gln Gly Arg Pro Arg Gln
140 145 150

Ala Ser Arg Ser Arg Gln Asn Gln Arg Glu Ala His Phe Ile Lys
155 160 165

Arg Leu Tyr Gln Gly Gln Leu Pro Phe Pro Asn His Ala Glu Lys
170 175 180

Gln Lys Gln Phe Glu Phe Val Gly Ser Ala Pro Thr Arg Arg Thr
185 190 195

Lys Arg Thr Arg Arg Pro Gln Pro Leu Thr
200 205

<210> 22
<211> 28
<212> DNA
<213> Artificial Sequence

<220>
<223> Synthetic Oligonucleotide Probe

<400> 22
cagtacgtga gggaccaggg cgccatga 28

<210> 23
<211> 24
<212> DNA
<213> Artificial Sequence

<220>
<223> Synthetic Oligonucleotide Probe

<400> 23
ccggtgacct gcacgtgctt gcca 24

<210> 24
<211> 41
<212> DNA
<213> Artificial Sequence

<220>
<223> Synthetic Oligonucleotide Probe

<220>
<221> unsure
<222> 21
<223> unknown base

<400> 24
gcggatctgc cgcctgctca nctggtcggt catggcgccc t 41

<210> 25
<211> 3355
<212> DNA
<213> Homo Sapien

<400> 25
gcagctggtt actgcatttc tccatgtggc agacagagca aagccacaac 50

gctttctctg ctggattaaa gacggcccac agaccagaac ttccactata 100

ctacttaaaa ttacataggt ggcttgtcaa attcaattga ttagtattgt 150

aaaaggaaaa agaagttcct tcttacagct tggattcaac ggtccaaaac 200

aaaaatgcag ctgccattaa agtctcagat gaacaaactt ctacactgat 250

ttttaaaatc aagaataagg gcagcaagtt tctggattca ctgaatcaac 300
agacacaaaa agctggcaat atagcaacta tgaagagaaa agctactaat 350
aaaattaacc caacgcatag aagacttttt tttctcttct aaaaacaact 400
aagtaaagac ttaaatttaa acacatcatt ttacaacctc atttcaaaat 450
gaagactttt acctggaccc taggtgtgct attcttccta ctagtggaca 500
ctggacattg cagaggtgga caattcaaaa ttaaaaaaat aaaccagaga 550
agataccctc gtgccacaga tggtaaagag gaagcaaaga aatgtgcata 600
cacattcctg gtacctgaac aaagaataac agggccaatc tgtgtcaaca 650
ccaaggggca agatgcaagt accattaaag acatgatcac caggatggac 700
cttgaaaacc tgaaggatgt gctctccagg cagaagcggg agatagatgt 750
tctgcaactg gtggtggatg tagatggaaa cattgtgaat gaggtaaagc 800
tgctgagaaa ggaaagccgt aacatgaact ctcgtgttac tcaactctat 850
atgcaattat tacatgagat tatccgtaag agggataatt cacttgaact 900
ttcccaactg gaaaacaaaa tcctcaatgt caccacagaa atgttgaaga 950
tggcaacaag atacagggaa ctagaggtga aatacgcttc cttgactgat 1000
cttgtcaata accaatctgt gatgatcact ttgttggaag aacagtgctt 1050
gaggatattt tcccgacaag acacccatgt gtctccccca cttgtccagg 1100
tggtgccaca acatattcct aacagccaac agtatactcc tggtctgctg 1150
ggaggtaacg agattcagag ggatccaggt tatcccagag atttaatgcc 1200
accacctgat ctggcaactt ctcccaccaa aagccctttc aagataccac 1250
cggtaacttt catcaatgaa ggaccattca aagactgtca gcaagcaaaa 1300
gaagctgggc attcggtcag tgggatttat atgattaaac ctgaaaacag 1350
caatggacca atgcagttat ggtgtgaaaa cagtttggac cctgggggtt 1400
ggactgttat tcagaaaaga acagacggct ctgtcaactt cttcagaaat 1450
tgggaaaatt ataagaaagg gtttggaaac attgacggag aatactggct 1500
tggactggaa aatatctata tgcttagcaa tcaagataat tacaagttat 1550
tgattgaatt agaagactgg agtgataaaa aagtctatgc agaatacagc 1600
agctttcgtc tggaacctga aagtgaattc tatagactgc gcctgggaac 1650
ttaccaggga aatgcagggg attctatgat gtggcataat ggtaaacaat 1700
tcaccacact ggacagagat aaagatatgt atgcaggaaa ctgcgcccac 1750
tttcataaag gaggctggtg gtacaatgcc tgtgcacatt ctaacctaaa 1800
tggagtatgg tacagaggag gccattacag aagcaagcac caagatggaa 1850

ttttctgggc cgaatacaga ggcgggtcat actccttaag agcagttcag 1900

atgatgatca agcctattga ctgaagagag acactcgcca atttaaatga 1950

cacagaactt tgtacttttc agctcttaaa aatgtaaatg ttacatgtat 2000

attacttggc acaatttatt tctacacaga aagtttttaa aatgaatttt 2050

accgtaacta taaaagggaa cctataaatg tagtttcatc tgtcgtcaat 2100

tactgcagaa aattatgtgt atccacaacc tagttatttt aaaaattatg 2150

ttgactaaat acaaagtttg ttttctaaaa tgtaaatatt tgccacaatg 2200

taaagcaaat cttagctata ttttaaatca taaataacat gttcaagata 2250

cttaacaatt tatttaaaat ctaagattgc tctaacgtct agtgaaaaaa 2300

atatttttta aatttcagcc aaataatgca ttttatttta taaaaataca 2350

gacagaaaat tagggagaaa cttctagttt tgccaataga aaatgttctt 2400

ccattgaata aaagttattt caaattgaat ttgtgccttt cacacgtaat 2450

gattaaatct gaattcttaa taatatatcc tatgctgatt ttcccaaaac 2500

atgacccata gtattaaata catatcattt ttaaaaataa aaaaaaaccc 2550

aaaaataatg catgcataat ttaaatggtc aatttataaa gacaaatcta 2600

tgaatgaatt tttcagtgtt atcttcatat gatatgctga acaccaaaat 2650

ctccagaaat gcattttatg tagttctaaa atcagcaaaa tattggtatt 2700

acaaaaatgc agaatattta gtgtgctaca gatctgaatt atagttctaa 2750

tttattatta ctttttttct aatttactga tcttactact acaaagaaaa 2800

aaaaacccaa cccatctgca attcaaatca gaaagtttgg acagctttac 2850

aagtattagt gcatgctcag aacaggtggg actaaaacaa actcaaggaa 2900

ctgttggctg ttttcccgat actgagaatt caacagctcc agagcagaag 2950

ccacaggggc atagcttagt ccaaactgct aatttcattt tacagtgtat 3000

gtaacgctta gtctcacagt gtctttaact catctttgca atcaacaact 3050

ttactagtga ctttctggaa caatttcctt tcaggaatac atattcactg 3100

cttagaggtg accttgcctt aatatatttg tgaagttaaa attttaaaga 3150

tagctcatga aacttttgct taagcaaaaa gaaaacctcg aattgaaatg 3200

tgtgaggcaa actatgcatg ggaatagctt aatgtgaaga taatcatttg 3250

gacaactcaa atccatcaac atgaccaatg tttttcatct gccacatctc 3300

aaaataaaac ttctggtgaa acaaattaaa caaaatatcc aaacctcaaa 3350

aaaaa 3355

<210> 26

<211> 491
<212> PRT
<213> Homo Sapien

<400> 26
Met Lys Thr Phe Thr Trp Thr Leu Gly Val Leu Phe Phe Leu Leu
1 5 10 15

Val Asp Thr Gly His Cys Arg Gly Gly Gln Phe Lys Ile Lys Lys
20 25 30

Ile Asn Gln Arg Arg Tyr Pro Arg Ala Thr Asp Gly Lys Glu Glu
35 40 45

Ala Lys Lys Cys Ala Tyr Thr Phe Leu Val Pro Glu Gln Arg Ile
50 55 60

Thr Gly Pro Ile Cys Val Asn Thr Lys Gly Gln Asp Ala Ser Thr
65 70 75

Ile Lys Asp Met Ile Thr Arg Met Asp Leu Glu Asn Leu Lys Asp
80 85 90

Val Leu Ser Arg Gln Lys Arg Glu Ile Asp Val Leu Gln Leu Val
95 100 105

Val Asp Val Asp Gly Asn Ile Val Asn Glu Val Lys Leu Leu Arg
110 115 120

Lys Glu Ser Arg Asn Met Asn Ser Arg Val Thr Gln Leu Tyr Met
125 130 135

Gln Leu Leu His Glu Ile Ile Arg Lys Arg Asp Asn Ser Leu Glu
140 145 150

Leu Ser Gln Leu Glu Asn Lys Ile Leu Asn Val Thr Thr Glu Met
155 160 165

Leu Lys Met Ala Thr Arg Tyr Arg Glu Leu Glu Val Lys Tyr Ala
170 175 180

Ser Leu Thr Asp Leu Val Asn Asn Gln Ser Val Met Ile Thr Leu
185 190 195

Leu Glu Glu Gln Cys Leu Arg Ile Phe Ser Arg Gln Asp Thr His
200 205 210

Val Ser Pro Pro Leu Val Gln Val Val Pro Gln His Ile Pro Asn
215 220 225

Ser Gln Gln Tyr Thr Pro Gly Leu Leu Gly Gly Asn Glu Ile Gln
230 235 240

Arg Asp Pro Gly Tyr Pro Arg Asp Leu Met Pro Pro Pro Asp Leu
245 250 255

Ala Thr Ser Pro Thr Lys Ser Pro Phe Lys Ile Pro Pro Val Thr
260 265 270

Phe Ile Asn Glu Gly Pro Phe Lys Asp Cys Gln Gln Ala Lys Glu
275 280 285

```
Ala Gly His Ser Val Ser Gly Ile Tyr Met Ile Lys Pro Glu Asn
                290                 295                 300

Ser Asn Gly Pro Met Gln Leu Trp Cys Glu Asn Ser Leu Asp Pro
                305                 310                 315

Gly Gly Trp Thr Val Ile Gln Lys Arg Thr Asp Gly Ser Val Asn
                320                 325                 330

Phe Phe Arg Asn Trp Glu Asn Tyr Lys Lys Gly Phe Gly Asn Ile
                335                 340                 345

Asp Gly Glu Tyr Trp Leu Gly Leu Glu Asn Ile Tyr Met Leu Ser
                350                 355                 360

Asn Gln Asp Asn Tyr Lys Leu Leu Ile Glu Leu Glu Asp Trp Ser
                365                 370                 375

Asp Lys Lys Val Tyr Ala Glu Tyr Ser Ser Phe Arg Leu Glu Pro
                380                 385                 390

Glu Ser Glu Phe Tyr Arg Leu Arg Leu Gly Thr Tyr Gln Gly Asn
                395                 400                 405

Ala Gly Asp Ser Met Met Trp His Asn Gly Lys Gln Phe Thr Thr
                410                 415                 420

Leu Asp Arg Asp Lys Asp Met Tyr Ala Gly Asn Cys Ala His Phe
                425                 430                 435

His Lys Gly Gly Trp Trp Tyr Asn Ala Cys Ala His Ser Asn Leu
                440                 445                 450

Asn Gly Val Trp Tyr Arg Gly Gly His Tyr Arg Ser Lys His Gln
                455                 460                 465

Asp Gly Ile Phe Trp Ala Glu Tyr Arg Gly Gly Ser Tyr Ser Leu
                470                 475                 480

Arg Ala Val Gln Met Met Ile Lys Pro Ile Asp
                485                 490 491
```

<210> 27
<211> 29
<212> DNA
<213> Artificial Sequence

<220>
<223> Synthetic Oligonucleotide Probe

<400> 27
caggttatcc cagagattta atgccacca 29

<210> 28
<211> 34
<212> DNA
<213> Artificial Sequence

<220>
<223> Synthetic Oligonucleotide Probe

<400> 28
ttggtgggag aagttgccag atcaggtggt ggca 34

<210> 29
<211> 25
<212> DNA
<213> Artificial Sequence

<220>
<223> Synthetic Oligonucleotide Probe

<400> 29
ttcacaccat aactgcattg gtcca 25

<210> 30
<211> 1174
<212> DNA
<213> Homo Sapien

<400> 30
cggacgcgtg ggggaaaccc ttccgagaaa acagcaacaa gctgagctgc 50

tgtgacagag gggaacaaga tggcggcgcc gaaggggagc ctctgggtga 100

ggacccaact ggggctcccg ccgctgctgc tgctgaccat ggccttggcc 150

ggaggttcgg ggaccgcttc ggctgaagca tttgactcgg tcttgggtga 200

tacggcgtct tgccaccggg cctgtcagtt gacctacccc ttgcacacct 250

accctaagga agaggagttg tacgcatgtc agagaggttg caggctgttt 300

tcaatttgtc agtttgtgga tgatggaatt gacttaaatc gaactaaatt 350

ggaatgtgaa tctgcatgta cagaagcata ttcccaatct gatgagcaat 400

atgcttgcca tcttggttgc cagaatcagc tgccattcgc tgaactgaga 450

caagaacaac ttatgtccct gatgccaaaa atgcacctac tctttcctct 500

aactctggtg aggtcattct ggagtgacat gatggactcc gcacagagct 550

tcataacctc ttcatggact ttttatcttc aagccgatga cggaaaaata 600

gttatattcc agtctaagcc agaaatccag tacgcaccac atttggagca 650

ggagcctaca aatttgagag aatcatctct aagcaaaatg tcctatctgc 700

aaatgagaaa ttcacaagcg cacaggaatt ttcttgaaga tggagaaagt 750

gatggctttt taagatgcct ctctcttaac tctgggtgga ttttaactac 800

aactcttgtc ctctcggtga tggtattgct ttggatttgt tgtgcaactg 850

ttgctacagc tgtggagcag tatgttccct ctgagaagct gagtatctat 900

ggtgacttgg agtttatgaa tgaacaaaag ctaaacagat atccagcttc 950

ttctcttgtg gttgttagat ctaaaactga agatcatgaa gaagcagggc 1000

ctctacctac aaaagtgaat cttgctcatt ctgaaattta agcatttttc 1050

ttttaaaaga caagtgtaat agacatctaa aattccactc ctcatagagc 1100

ttttaaaatg gtttcattgg atataggcct taagaaatca ctataaaatg 1150

caaataaagt tactcaaatc tgtg 1174

<210> 31
<211> 323
<212> PRT
<213> Homo Sapien

<400> 31
Met Ala Ala Pro Lys Gly Ser Leu Trp Val Arg Thr Gln Leu Gly
1 5 10 15

Leu Pro Pro Leu Leu Leu Leu Thr Met Ala Leu Ala Gly Gly Ser
20 25 30

Gly Thr Ala Ser Ala Glu Ala Phe Asp Ser Val Leu Gly Asp Thr
35 40 45

Ala Ser Cys His Arg Ala Cys Gln Leu Thr Tyr Pro Leu His Thr
50 55 60

Tyr Pro Lys Glu Glu Glu Leu Tyr Ala Cys Gln Arg Gly Cys Arg
65 70 75

Leu Phe Ser Ile Cys Gln Phe Val Asp Asp Gly Ile Asp Leu Asn
80 85 90

Arg Thr Lys Leu Glu Cys Glu Ser Ala Cys Thr Glu Ala Tyr Ser
95 100 105

Gln Ser Asp Glu Gln Tyr Ala Cys His Leu Gly Cys Gln Asn Gln
110 115 120

Leu Pro Phe Ala Glu Leu Arg Gln Glu Gln Leu Met Ser Leu Met
125 130 135

Pro Lys Met His Leu Leu Phe Pro Leu Thr Leu Val Arg Ser Phe
140 145 150

Trp Ser Asp Met Met Asp Ser Ala Gln Ser Phe Ile Thr Ser Ser
155 160 165

Trp Thr Phe Tyr Leu Gln Ala Asp Asp Gly Lys Ile Val Ile Phe
170 175 180

Gln Ser Lys Pro Glu Ile Gln Tyr Ala Pro His Leu Glu Gln Glu
185 190 195

Pro Thr Asn Leu Arg Glu Ser Ser Leu Ser Lys Met Ser Tyr Leu
200 205 210

Gln Met Arg Asn Ser Gln Ala His Arg Asn Phe Leu Glu Asp Gly
215 220 225

Glu Ser Asp Gly Phe Leu Arg Cys Leu Ser Leu Asn Ser Gly Trp
230 235 240

Ile Leu Thr Thr Thr Leu Val Leu Ser Val Met Val Leu Leu Trp
245 250 255

Ile Cys Cys Ala Thr Val Ala Thr Ala Val Glu Gln Tyr Val Pro
260 265 270

Ser Glu Lys Leu Ser Ile Tyr Gly Asp Leu Glu Phe Met Asn Glu
275 280 285

Gln Lys Leu Asn Arg Tyr Pro Ala Ser Ser Leu Val Val Val Arg
290 295 300

Ser Lys Thr Glu Asp His Glu Glu Ala Gly Pro Leu Pro Thr Lys
305 310 315

Val Asn Leu Ala His Ser Glu Ile
320 323

<210> 32
<211> 22
<212> DNA
<213> Artificial Sequence

<220>
<223> Synthetic Oligonucleotide Probe

<400> 32
acaagctgag ctgctgtgac ag 22

<210> 33
<211> 22
<212> DNA
<213> Artificial Sequence

<220>
<223> Synthetic Oligonucleotide Probe

<400> 33
tgattctggc aaccaagatg gc 22

<210> 34
<211> 40
<212> DNA
<213> Artificial Sequence

<220>
<223> Synthetic Oligonucleotide Probe

<400> 34
atggccttgg ccggaggttc ggggaccgct tcggctgaag 40

<210> 35
<211> 1114
<212> DNA
<213> Homo Sapien

<400> 35
tccgcaggcg gaccgggggc aaaggaggtg gcatgtcggt caggcacagc 50

agggtcctgt gtccgcgctg agccgcgctc tccctgctcc agcaaggacc 100

atgagggcgc tggaggggcc aggcctgtcg ctgctgtgcc tggtgttggc 150

gctgcctgcc ctgctgccgg tgccggctgt acgcggagtg gcagaaacac 200

ccacctaccc ctggcgggac gcagagacag gggagcggct ggtgtgcgcc 250

cagtgccccc caggcacctt tgtgcagcgg ccgtgccgcc gagacagccc 300

cacgacgtgt ggcccgtgtc caccgcgcca ctacacgcag ttctggaact 350

acctggagcg ctgccgctac tgcaacgtcc tctgcgggga gcgtgaggag 400

gaggcacggg cttgccacgc cacccacaac cgtgcctgcc gctgccgcac 450

cggcttcttc gcgcacgctg gtttctgctt ggagcacgca tcgtgtccac 500

ctggtgccgg cgtgattgcc ccgggcaccc ccagccagaa cacgcagtgc 550

cagccgtgcc ccccaggcac cttctcagcc agcagctcca gctcagagca 600

gtgccagccc caccgcaact gcacggccct gggcctggcc ctcaatgtgc 650

caggctcttc ctcccatgac accctgtgca ccagctgcac tggcttcccc 700

ctcagcacca gggtaccagg agctgaggag tgtgagcgtg ccgtcatcga 750

ctttgtggct ttccaggaca tctccatcaa gaggctgcag cggctgctgc 800

aggccctcga ggccccggag ggctggggtc cgacaccaag ggcgggccgc 850

gcggccttgc agctgaagct gcgtcggcgg ctcacggagc tcctgggggc 900

gcaggacggg gcgctgctgg tgcggctgct gcaggcgctg cgcgtggcca 950

ggatgcccgg gctggagcgg agcgtccgtg agcgcttcct ccctgtgcac 1000

tgatcctggc cccctcttat ttattctaca tccttggcac cccacttgca 1050

ctgaaagagg cttttttta aatagaagaa atgaggtttc ttaaaaaaaa 1100

aaaaaaaaaa aaaa 1114

<210> 36
<211> 300
<212> PRT
<213> Homo Sapien

<400> 36
```
Met Arg Ala Leu Glu Gly Pro Gly Leu Ser Leu Leu Cys Leu Val
 1               5                  10                  15

Leu Ala Leu Pro Ala Leu Leu Pro Val Pro Ala Val Arg Gly Val
                20                  25                  30

Ala Glu Thr Pro Thr Tyr Pro Trp Arg Asp Ala Glu Thr Gly Glu
                35                  40                  45

Arg Leu Val Cys Ala Gln Cys Pro Pro Gly Thr Phe Val Gln Arg
                50                  55                  60

Pro Cys Arg Arg Asp Ser Pro Thr Thr Cys Gly Pro Cys Pro Pro
                65                  70                  75

Arg His Tyr Thr Gln Phe Trp Asn Tyr Leu Glu Arg Cys Arg Tyr
                80                  85                  90

Cys Asn Val Leu Cys Gly Glu Arg Glu Glu Glu Ala Arg Ala Cys
                95                 100                 105

His Ala Thr His Asn Arg Ala Cys Arg Cys Arg Thr Gly Phe Phe
               110                 115                 120
```

```
Ala His Ala Gly Phe Cys Leu Glu His Ala Ser Cys Pro Pro Gly
                125                 130                 135

Ala Gly Val Ile Ala Pro Gly Thr Pro Ser Gln Asn Thr Gln Cys
                140                 145                 150

Gln Pro Cys Pro Pro Gly Thr Phe Ser Ala Ser Ser Ser Ser Ser
                155                 160                 165

Glu Gln Cys Gln Pro His Arg Asn Cys Thr Ala Leu Gly Leu Ala
                170                 175                 180

Leu Asn Val Pro Gly Ser Ser Ser His Asp Thr Leu Cys Thr Ser
                185                 190                 195

Cys Thr Gly Phe Pro Leu Ser Thr Arg Val Pro Gly Ala Glu Glu
                200                 205                 210

Cys Glu Arg Ala Val Ile Asp Phe Val Ala Phe Gln Asp Ile Ser
                215                 220                 225

Ile Lys Arg Leu Gln Arg Leu Leu Gln Ala Leu Glu Ala Pro Glu
                230                 235                 240

Gly Trp Gly Pro Thr Pro Arg Ala Gly Arg Ala Ala Leu Gln Leu
                245                 250                 255

Lys Leu Arg Arg Arg Leu Thr Glu Leu Leu Gly Ala Gln Asp Gly
                260                 265                 270

Ala Leu Leu Val Arg Leu Leu Gln Ala Leu Arg Val Ala Arg Met
                275                 280                 285

Pro Gly Leu Glu Arg Ser Val Arg Glu Arg Phe Leu Pro Val His
                290                 295                 300
```

<210> 37
<211> 21
<212> DNA
<213> Artificial Sequence

<220>
<223> Synthetic Oligonucleotide Probe

<400> 37
cacgctggtt tctgcttgga g 21

<210> 38
<211> 22
<212> DNA
<213> Artificial Sequence

<220>
<223> Synthetic Oligonucleotide Probe

<400> 38
agctggtgca cagggtgtca tg 22

<210> 39
<211> 53
<212> DNA
<213> Artificial Sequence

<220>
<223> Synthetic Oligonucleotide Probe

<400> 39
cccaggcacc ttctcagcca gccagcagct ccagctcaga gcagtgccag 50

ccc 53

<210> 40
<211> 1838
<212> DNA
<213> Homo Sapien

<400> 40
cggacgcgtg ggcggacgcg tgggcggccc acggcgcccg cgggctgggg 50

cggtcgcttc ttccttctcc gtggcctacg agggtcccca gcctgggtaa 100

agatggcccc atggccccg aagggcctag tcccagctgt gctctggggc 150

ctcagcctct tcctcaacct cccaggacct atctggctcc agccctctcc 200

acctccccag tcttctcccc cgcctcagcc ccatccgtgt catacctgcc 250

ggggactggt tgacagcttt aacaagggcc tggagagaac catccgggac 300

aactttggag gtggaaacac tgcctgggag gaagagaatt tgtccaaata 350

caaagacagt gagacccgcc tggtagaggt gctggagggt gtgtgcagca 400

agtcagactt cgagtgccac cgcctgctgg agctgagtga ggagctggtg 450

gagagctggt ggtttcacaa gcagcaggag gccccggacc tcttccagtg 500

gctgtgctca gattccctga agctctgctg ccccgcaggc accttcgggc 550

cctcctgcct tccctgtcct gggggaacag agaggccctg cggtggctac 600

gggcagtgtg aaggagaagg gacacgaggg ggcagcgggc actgtgactg 650

ccaagccggc tacgggggtg aggcctgtgg ccagtgtggc cttggctact 700

ttgaggcaga acgcaacgcc agccatctgg tatgttcggc ttgttttggc 750

ccctgtgccc gatgctcagg acctgaggaa tcaaactgtt tgcaatgcaa 800

gaagggctgg gccctgcatc acctcaagtg tgtagacatt gatgagtgtg 850

gcacagaggg agccaactgt ggagctgacc aattctgcgt gaacactgag 900

ggctcctatg agtgccgaga ctgtgccaag gcctgcctag gctgcatggg 950

ggcagggcca ggtcgctgta agaagtgtag ccctggctat cagcaggtgg 1000

gctccaagtg tctcgatgtg gatgagtgtg agacagaggt gtgtccggga 1050

gagaacaagc agtgtgaaaa caccgagggc ggttatcgct gcatctgtgc 1100

cgagggctac aagcagatgg aaggcatctg tgtgaaggag cagatcccag 1150

agtcagcagg cttcttctca gagatgacag aagacgagtt ggtggtgctg 1200

cagcagatgt tctttggcat catcatctgt gcactggcca cgctggctgc 1250

taagggcgac ttggtgttca ccgccatctt cattggggct gtggcggcca 1300

tgactggcta ctggttgtca gagcgcagtg accgtgtgct ggagggcttc 1350

atcaagggca gataatcgcg gccaccacct gtaggacctc ctcccaccca 1400

cgctgccccc agagcttggg ctgccctcct gctggacact caggacagct 1450

tggtttattt ttgagagtgg ggtaagcacc cctacctgcc ttacagagca 1500

gcccaggtac ccaggcccgg gcagacaagg cccctggggt aaaaagtagc 1550

cctgaaggtg gataccatga gctcttcacc tggcggggac tggcaggctt 1600

cacaatgtgt gaatttcaaa agtttttcct taatggtggc tgctagagct 1650

ttggcccctg cttaggatta ggtggtcctc acaggggtgg ggccatcaca 1700

gctccctcct gccagctgca tgctgccagt tcctgttctg tgttcaccac 1750

atccccacac cccattgcca cttatttatt catctcagga aataaagaaa 1800

ggtcttggaa agttaaaaaa aaaaaaaaaa aaaaaaaa 1838

<210> 41
<211> 420
<212> PRT
<213> Homo Sapien

<400> 41

```
Met Ala Pro Trp Pro Pro Lys Gly Leu Val Pro Ala Val Leu Trp
 1               5                   10                  15

Gly Leu Ser Leu Phe Leu Asn Leu Pro Gly Pro Ile Trp Leu Gln
                20                  25                  30

Pro Ser Pro Pro Pro Gln Ser Ser Pro Pro Pro Gln Pro His Pro
                35                  40                  45

Cys His Thr Cys Arg Gly Leu Val Asp Ser Phe Asn Lys Gly Leu
                50                  55                  60

Glu Arg Thr Ile Arg Asp Asn Phe Gly Gly Gly Asn Thr Ala Trp
                65                  70                  75

Glu Glu Glu Asn Leu Ser Lys Tyr Lys Asp Ser Glu Thr Arg Leu
                80                  85                  90

Val Glu Val Leu Glu Gly Val Cys Ser Lys Ser Asp Phe Glu Cys
                95                  100                 105

His Arg Leu Leu Glu Leu Ser Glu Glu Leu Val Glu Ser Trp Trp
                110                 115                 120

Phe His Lys Gln Gln Glu Ala Pro Asp Leu Phe Gln Trp Leu Cys
                125                 130                 135

Ser Asp Ser Leu Lys Leu Cys Cys Pro Ala Gly Thr Phe Gly Pro
                140                 145                 150

Ser Cys Leu Pro Cys Pro Gly Gly Thr Glu Arg Pro Cys Gly Gly
                155                 160                 165
```

Tyr Gly Gln Cys Glu Gly Glu Gly Thr Arg Gly Gly Ser Gly His
170 175 180

Cys Asp Cys Gln Ala Gly Tyr Gly Gly Glu Ala Cys Gly Gln Cys
185 190 195

Gly Leu Gly Tyr Phe Glu Ala Glu Arg Asn Ala Ser His Leu Val
200 205 210

Cys Ser Ala Cys Phe Gly Pro Cys Ala Arg Cys Ser Gly Pro Glu
215 220 225

Glu Ser Asn Cys Leu Gln Cys Lys Lys Gly Trp Ala Leu His His
230 235 240

Leu Lys Cys Val Asp Ile Asp Glu Cys Gly Thr Glu Gly Ala Asn
245 250 255

Cys Gly Ala Asp Gln Phe Cys Val Asn Thr Glu Gly Ser Tyr Glu
260 265 270

Cys Arg Asp Cys Ala Lys Ala Cys Leu Gly Cys Met Gly Ala Gly
275 280 285

Pro Gly Arg Cys Lys Lys Cys Ser Pro Gly Tyr Gln Gln Val Gly
290 295 300

Ser Lys Cys Leu Asp Val Asp Glu Cys Glu Thr Glu Val Cys Pro
305 310 315

Gly Glu Asn Lys Gln Cys Glu Asn Thr Glu Gly Gly Tyr Arg Cys
320 325 330

Ile Cys Ala Glu Gly Tyr Lys Gln Met Glu Gly Ile Cys Val Lys
335 340 345

Glu Gln Ile Pro Glu Ser Ala Gly Phe Phe Ser Glu Met Thr Glu
350 355 360

Asp Glu Leu Val Val Leu Gln Gln Met Phe Phe Gly Ile Ile Ile
365 370 375

Cys Ala Leu Ala Thr Leu Ala Ala Lys Gly Asp Leu Val Phe Thr
380 385 390

Ala Ile Phe Ile Gly Ala Val Ala Ala Met Thr Gly Tyr Trp Leu
395 400 405

Ser Glu Arg Ser Asp Arg Val Leu Glu Gly Phe Ile Lys Gly Arg
410 415 420

<210> 42
<211> 22
<212> DNA
<213> Artificial Sequence

<220>
<223> Synthetic Oligonucleotide Probe

<400> 42
attctgcgtg aacactgagg gc 22

<210> 43
<211> 22
<212> DNA
<213> Artificial Sequence

<220>
<223> Synthetic Oligonucleotide Probe

<400> 43
atctgcttgt agccctcggc ac 22

<210> 44
<211> 50
<212> DNA
<213> Artificial Sequence

<220>
<223> Synthetic Oligonucleotide Probe

<400> 44
cctggctatc agcaggtggg ctccaagtgt ctcgatgtgg atgagtgtga 50

<210> 45
<211> 2033
<212> DNA
<213> Homo Sapien

<400> 45
ccaggccggg aggcgacgcg cccagccgtc taaacgggaa cagccctggc 50

tgagggagct gcagcgcagc agagtatctg acggcgccag gttgcgtagg 100

tgcggcacga ggagttttcc cggcagcgag gaggtcctga gcagcatggc 150

ccggaggagc gccttccctg ccgccgcgct ctggctctgg agcatcctcc 200

tgtgcctgct ggcactgcgg gcggaggccg ggccgccgca ggaggagagc 250

ctgtacctat ggatcgatgc tcaccaggca agagtactca taggatttga 300

agaagatatc ctgattgttt cagaggggaa aatggcacct tttacacatg 350

atttcagaaa agcgcaacag agaatgccag ctattcctgt caatatccat 400

tccatgaatt ttacctggca agctgcaggg caggcagaat acttctatga 450

attcctgtcc ttgcgctccc tggataaagg catcatggca gatccaaccg 500

tcaatgtccc tctgctggga acagtgcctc acaaggcatc agttgttcaa 550

gttggtttcc catgtcttgg aaaacaggat ggggtggcag catttgaagt 600

ggatgtgatt gttatgaatt ctgaaggcaa caccattctc caaacacctc 650

aaaatgctat cttctttaaa acatgtcaac aagctgagtg cccaggcggg 700

tgccgaaatg gaggcttttg taatgaaaga cgcatctgcg agtgtcctga 750

tgggttccac ggacctcact gtgagaaagc cctttgtacc ccacgatgta 800

tgaatggtgg actttgtgtg actcctggtt tctgcatctg cccacctgga 850

ttctatggag tgaactgtga caaagcaaac tgctcaacca cctgctttaa 900

tggagggacc tgtttctacc ctggaaaatg tatttgccct ccaggactag 950

agggagagca gtgtgaaatc agcaaatgcc cacaaccctg tcgaaatgga 1000

ggtaaatgca ttggtaaaag caaatgtaag tgttccaaag gttaccaggg 1050

agacctctgt tcaaagcctg tctgcgagcc tggctgtggt gcacatggaa 1100

cctgccatga acccaacaaa tgccaatgtc aagaaggttg gcatggaaga 1150

cactgcaata aaaggtacga agccagcctc atacatgccc tgaggccagc 1200

aggcgcccag ctcaggcagc acacgccttc acttaaaaag gccgaggagc 1250

ggcgggatcc acctgaatcc aattacatct ggtgaactcc gacatctgaa 1300

acgttttaag ttacaccaag ttcatagcct ttgttaacct ttcatgtgtt 1350

gaatgttcaa ataatgttca ttacacttaa gaatactggc ctgaatttta 1400

ttagcttcat tataaatcac tgagctgata tttactcttc cttttaagtt 1450

ttctaagtac gtctgtagca tgatggtata gattttcttg tttcagtgct 1500

ttgggacaga ttttatatta tgtcaattga tcaggttaaa attttcagtg 1550

tgtagttggc agatattttc aaaattacaa tgcatttatg gtgtctgggg 1600

gcaggggaac atcagaaagg ttaaattggg caaaaatgcg taagtcacaa 1650

gaatttggat ggtgcagtta atgttgaagt tacagcattt cagattttat 1700

tgtcagatat ttagatgttt gttacatttt taaaaattgc tcttaatttt 1750

taaactctca atacaatata ttttgacctt accattattc cagagattca 1800

gtattaaaaa aaaaaaaatt acactgtggt agtggcattt aaacaatata 1850

atatattcta aacacaatga aatagggaat ataatgtatg aactttttgc 1900

attggcttga agcaatataa tatattgtaa acaaaacaca gctcttacct 1950

aataaacatt ttatactgtt tgtatgtata aaataaaggt gctgctttag 2000

tttttggaa aaaaaaaaaa aaaaaaaaaa aaa 2033

<210> 46
<211> 379
<212> PRT
<213> Homo Sapien

<400> 46

```
Met Ala Arg Arg Ser Ala Phe Pro Ala Ala Ala Leu Trp Leu Trp
1               5                   10                  15

Ser Ile Leu Leu Cys Leu Leu Ala Leu Arg Ala Glu Ala Gly Pro
                20                  25                  30

Pro Gln Glu Glu Ser Leu Tyr Leu Trp Ile Asp Ala His Gln Ala
                35                  40                  45

Arg Val Leu Ile Gly Phe Glu Glu Asp Ile Leu Ile Val Ser Glu
                50                  55                  60
```

```
Gly Lys Met Ala Pro Phe Thr His Asp Phe Arg Lys Ala Gln Gln
                65                  70                  75

Arg Met Pro Ala Ile Pro Val Asn Ile His Ser Met Asn Phe Thr
                80                  85                  90

Trp Gln Ala Ala Gly Gln Ala Glu Tyr Phe Tyr Glu Phe Leu Ser
                95                  100                 105

Leu Arg Ser Leu Asp Lys Gly Ile Met Ala Asp Pro Thr Val Asn
                110                 115                 120

Val Pro Leu Leu Gly Thr Val Pro His Lys Ala Ser Val Val Gln
                125                 130                 135

Val Gly Phe Pro Cys Leu Gly Lys Gln Asp Gly Val Ala Ala Phe
                140                 145                 150

Glu Val Asp Val Ile Val Met Asn Ser Glu Gly Asn Thr Ile Leu
                155                 160                 165

Gln Thr Pro Gln Asn Ala Ile Phe Phe Lys Thr Cys Gln Gln Ala
                170                 175                 180

Glu Cys Pro Gly Gly Cys Arg Asn Gly Gly Phe Cys Asn Glu Arg
                185                 190                 195

Arg Ile Cys Glu Cys Pro Asp Gly Phe His Gly Pro His Cys Glu
                200                 205                 210

Lys Ala Leu Cys Thr Pro Arg Cys Met Asn Gly Gly Leu Cys Val
                215                 220                 225

Thr Pro Gly Phe Cys Ile Cys Pro Pro Gly Phe Tyr Gly Val Asn
                230                 235                 240

Cys Asp Lys Ala Asn Cys Ser Thr Thr Cys Phe Asn Gly Gly Thr
                245                 250                 255

Cys Phe Tyr Pro Gly Lys Cys Ile Cys Pro Pro Gly Leu Glu Gly
                260                 265                 270

Glu Gln Cys Glu Ile Ser Lys Cys Pro Gln Pro Cys Arg Asn Gly
                275                 280                 285

Gly Lys Cys Ile Gly Lys Ser Lys Cys Lys Cys Ser Lys Gly Tyr
                290                 295                 300

Gln Gly Asp Leu Cys Ser Lys Pro Val Cys Glu Pro Gly Cys Gly
                305                 310                 315

Ala His Gly Thr Cys His Glu Pro Asn Lys Cys Gln Cys Gln Glu
                320                 325                 330

Gly Trp His Gly Arg His Cys Asn Lys Arg Tyr Glu Ala Ser Leu
                335                 340                 345

Ile His Ala Leu Arg Pro Ala Gly Ala Gln Leu Arg Gln His Thr
                350                 355                 360

Pro Ser Leu Lys Lys Ala Glu Glu Arg Arg Asp Pro Pro Glu Ser
                365                 370                 375
```

Asn Tyr Ile Trp
379

<210> 47
<211> 22
<212> DNA
<213> Artificial Sequence

<220>
<223> Synthetic Oligonucleotide Probe

<400> 47
aaagacgcat ctgcgagtgt cc 22

<210> 48
<211> 23
<212> DNA
<213> Artificial Sequence

<220>
<223> Synthetic Oligonucleotide Probe

<400> 48
tgctgatttc acactgctct ccc 23

<210> 49
<211> 49
<212> DNA
<213> Artificial Sequence

<220>
<223> Synthetic Oligonucleotide Probe

<400> 49
cccacgatgt atgaatggtg gactttgtgt gactcctggt ttctgcatc 49

<210> 50
<211> 1210
<212> DNA
<213> Homo Sapien

<400> 50
cagcgcgtgg ccggcgccgc tgtggggaca gcatgagcgg cggttggatg 50

gcgcaggttg gagcgtggcg aacaggggct ctgggcctgg cgctgctgct 100

gctgctcggc ctcggactag gcctggaggc cgccgcgagc ccgctttcca 150

ccccgacctc tgcccaggcc gcaggcccca gctcaggctc gtgcccaccc 200

accaagttcc agtgccgcac cagtggctta tgcgtgcccc tcacctggcg 250

ctgcgacagg gacttggact gcagcgatgg cagcgatgag gaggagtgca 300

ggattgagcc atgtacccag aaagggcaat gcccaccgcc ccctggcctc 350

ccctgcccct gcaccggcgt cagtgactgc tctgggggaa ctgacaagaa 400

actgcgcaac tgcagccgcc tggcctgcct agcaggcgag ctccgttgca 450

cgctgagcga tgactgcatt ccactcacgt ggcgctgcga cggccaccca 500

gactgtcccg actccagcga cgagctcggc tgtggaacca atgagatcct 550

cccggaaggg gatgccacaa ccatggggcc ccctgtgacc ctggagagtg 600

tcacctctct caggaatgcc acaaccatgg ggccccctgt gaccctggag 650

agtgtcccct ctgtcgggaa tgccacatcc tcctctgccg gagaccagtc 700

tggaagccca actgcctatg gggttattgc agctgctgcg gtgctcagtg 750

caagcctggt caccgccacc ctcctccttt tgtcctggct ccgagcccag 800

gagcgcctcc gcccactggg gttactggtg gccatgaagg agtccctgct 850

gctgtcagaa cagaagacct cgctgccctg aggacaagca cttgccacca 900

ccgtcactca gccctgggcg tagccggaca ggaggagagc agtgatgcgg 950

atgggtaccc gggcacacca gccctcagag acctgagttc ttctggccac 1000

gtggaacctc gaacccgagc tcctgcagaa gtggccctgg agattgaggg 1050

tccctggaca ctccctatgg agatccgggg agctaggatg ggaacctgc 1100

cacagccaga actgaggggc tggccccagg cagctcccag gggtagaac 1150

ggccctgtgc ttaagacact ccctgctgcc ccgtctgagg gtggcgatta 1200

aagttgcttc 1210

<210> 51
<211> 282
<212> PRT
<213> Homo Sapien

<400> 51

Met Ser Gly Gly Trp Met Ala Gln Val Gly Ala Trp Arg Thr Gly
1 5 10 15

Ala Leu Gly Leu Ala Leu Leu Leu Leu Leu Gly Leu Gly Leu Gly
20 25 30

Leu Glu Ala Ala Ala Ser Pro Leu Ser Thr Pro Thr Ser Ala Gln
35 40 45

Ala Ala Gly Pro Ser Ser Gly Ser Cys Pro Pro Thr Lys Phe Gln
50 55 60

Cys Arg Thr Ser Gly Leu Cys Val Pro Leu Thr Trp Arg Cys Asp
65 70 75

Arg Asp Leu Asp Cys Ser Asp Gly Ser Asp Glu Glu Glu Cys Arg
80 85 90

Ile Glu Pro Cys Thr Gln Lys Gly Gln Cys Pro Pro Pro Pro Gly
95 100 105

Leu Pro Cys Pro Cys Thr Gly Val Ser Asp Cys Ser Gly Gly Thr
110 115 120

Asp Lys Lys Leu Arg Asn Cys Ser Arg Leu Ala Cys Leu Ala Gly
125 130 135

Glu Leu Arg Cys Thr Leu Ser Asp Asp Cys Ile Pro Leu Thr Trp
140 145 150

```
Arg Cys Asp Gly His Pro Asp Cys Pro Asp Ser Ser Asp Glu Leu
                155                 160                 165

Gly Cys Gly Thr Asn Glu Ile Leu Pro Glu Gly Asp Ala Thr Thr
                170                 175                 180

Met Gly Pro Pro Val Thr Leu Glu Ser Val Thr Ser Leu Arg Asn
                185                 190                 195

Ala Thr Thr Met Gly Pro Pro Val Thr Leu Glu Ser Val Pro Ser
                200                 205                 210

Val Gly Asn Ala Thr Ser Ser Ser Ala Gly Asp Gln Ser Gly Ser
                215                 220                 225

Pro Thr Ala Tyr Gly Val Ile Ala Ala Ala Ala Val Leu Ser Ala
                230                 235                 240

Ser Leu Val Thr Ala Thr Leu Leu Leu Leu Ser Trp Leu Arg Ala
                245                 250                 255

Gln Glu Arg Leu Arg Pro Leu Gly Leu Leu Val Ala Met Lys Glu
                260                 265                 270

Ser Leu Leu Leu Ser Glu Gln Lys Thr Ser Leu Pro
                275                 280     282
```

<210> 52
<211> 24
<212> DNA
<213> Artificial Sequence

<220>
<223> Synthetic Oligonucleotide Probe

<400> 52
aagttccagt gccgcaccag tggc 24

<210> 53
<211> 24
<212> DNA
<213> Artificial Sequence

<220>
<223> Synthetic Oligonucleotide Probe

<400> 53
ttggttccac agccgagctc gtcg 24

<210> 54
<211> 50
<212> DNA
<213> Artificial Sequence

<220>
<223> Synthetic Oligonucleotide Probe

<400> 54
gaggaggagt gcaggattga gccatgtacc cagaaagggc aatgcccacc 50

<210> 55
<211> 1514
<212> DNA

<213> Homo Sapien

<400> 55
gggtctccc tcagggccgg gaggcacagc ggtccctgct tgctgaaggg 50
ctggatgtac gcatccgcag gttcccgcgg acttgggggc gcccgctgag 100
ccccggcgcc cgcagaagac ttgtgtttgc ctcctgcagc ctcaacccgg 150
agggcagcga gggcctacca ccatgatcac tggtgtgttc agcatgcgct 200
tgtggacccc agtgggcgtc ctgacctcgc tggcgtactg cctgcaccag 250
cggcgggtgg ccctggccga gctgcaggag gccgatggcc agtgtccggt 300
cgaccgcagc ctgctgaagt tgaaaatggt gcaggtcgtg tttcgacacg 350
gggctcggag tcctctcaag ccgctcccgc tggaggagca ggtagagtgg 400
aaccccccagc tattagaggt cccaccccaa actcagtttg attacacagt 450
caccaatcta gctggtggtc cgaaaccata ttctccttac gactctcaat 500
accatgagac caccctgaag gggggcatgt ttgctgggca gctgaccaag 550
gtgggcatgc agcaaatgtt tgccttggga gagagactga ggaagaacta 600
tgtggaagac attccctttc tttcaccaac cttcaaccca caggaggtct 650
ttattcgttc cactaacatt tttcggaatc tggagtccac ccgttgtttg 700
ctggctgggc ttttccagtg tcagaaagaa ggacccatca tcatccacac 750
tgatgaagca gattcagaag tcttgtatcc caactaccaa agctgctgga 800
gcctgaggca gagaaccaga ggccggaggc agactgcctc tttacagcca 850
ggaatctcag aggatttgaa aaaggtgaag gacaggatgg gcattgacag 900
tagtgataaa gtggacttct tcatcctcct ggacaacgtg gctgccgagc 950
aggcacacaa cctcccaagc tgccccatgc tgaagagatt tgcacggatg 1000
atcgaacaga gagctgtgga cacatccttg tacatactgc ccaaggaaga 1050
cagggaaagt cttcagatgg cagtaggccc attcctccac atcctagaga 1100
gcaacctgct gaaagccatg gactctgcca ctgcccccga caagatcaga 1150
aagctgtatc tctatgcggc tcatgatgtg accttcatac cgctcttaat 1200
gaccctgggg atttttgacc acaaatggcc accgtttgct gttgacctga 1250
ccatggaact ttaccagcac ctggaatcta aggagtggtt tgtgcagctc 1300
tattaccacg ggaaggagca ggtgccgaga ggttgccctg atgggctctg 1350
cccgctggac atgttcttga atgccatgtc agtttatacc ttaagcccag 1400
aaaaatacca tgcactctgc tctcaaactc aggtgatgga agttggaaat 1450
gaagagtaac tgatttataa aagcaggatg tgttgatttt aaaataaagt 1500

gcctttatac aatg 1514

<210> 56
<211> 428
<212> PRT
<213> Homo Sapien

<400> 56
Met Ile Thr Gly Val Phe Ser Met Arg Leu Trp Thr Pro Val Gly
1               5                   10                  15

Val Leu Thr Ser Leu Ala Tyr Cys Leu His Gln Arg Arg Val Ala
                20                  25                  30

Leu Ala Glu Leu Gln Glu Ala Asp Gly Gln Cys Pro Val Asp Arg
                35                  40                  45

Ser Leu Leu Lys Leu Lys Met Val Gln Val Val Phe Arg His Gly
                50                  55                  60

Ala Arg Ser Pro Leu Lys Pro Leu Pro Leu Glu Glu Gln Val Glu
                65                  70                  75

Trp Asn Pro Gln Leu Leu Glu Val Pro Pro Gln Thr Gln Phe Asp
                80                  85                  90

Tyr Thr Val Thr Asn Leu Ala Gly Gly Pro Lys Pro Tyr Ser Pro
                95                  100                 105

Tyr Asp Ser Gln Tyr His Glu Thr Thr Leu Lys Gly Gly Met Phe
                110                 115                 120

Ala Gly Gln Leu Thr Lys Val Gly Met Gln Gln Met Phe Ala Leu
                125                 130                 135

Gly Glu Arg Leu Arg Lys Asn Tyr Val Glu Asp Ile Pro Phe Leu
                140                 145                 150

Ser Pro Thr Phe Asn Pro Gln Glu Val Phe Ile Arg Ser Thr Asn
                155                 160                 165

Ile Phe Arg Asn Leu Glu Ser Thr Arg Cys Leu Leu Ala Gly Leu
                170                 175                 180

Phe Gln Cys Gln Lys Glu Gly Pro Ile Ile Ile His Thr Asp Glu
                185                 190                 195

Ala Asp Ser Glu Val Leu Tyr Pro Asn Tyr Gln Ser Cys Trp Ser
                200                 205                 210

Leu Arg Gln Arg Thr Arg Gly Arg Arg Gln Thr Ala Ser Leu Gln
                215                 220                 225

Pro Gly Ile Ser Glu Asp Leu Lys Lys Val Lys Asp Arg Met Gly
                230                 235                 240

Ile Asp Ser Ser Asp Lys Val Asp Phe Phe Ile Leu Leu Asp Asn
                245                 250                 255

Val Ala Ala Glu Gln Ala His Asn Leu Pro Ser Cys Pro Met Leu
                260                 265                 270

```
Lys Arg Phe Ala Arg Met Ile Glu Gln Arg Ala Val Asp Thr Ser
                275                 280                 285

Leu Tyr Ile Leu Pro Lys Glu Asp Arg Glu Ser Leu Gln Met Ala
                290                 295                 300

Val Gly Pro Phe Leu His Ile Leu Glu Ser Asn Leu Leu Lys Ala
                305                 310                 315

Met Asp Ser Ala Thr Ala Pro Asp Lys Ile Arg Lys Leu Tyr Leu
                320                 325                 330

Tyr Ala Ala His Asp Val Thr Phe Ile Pro Leu Leu Met Thr Leu
                335                 340                 345

Gly Ile Phe Asp His Lys Trp Pro Pro Phe Ala Val Asp Leu Thr
                350                 355                 360

Met Glu Leu Tyr Gln His Leu Glu Ser Lys Glu Trp Phe Val Gln
                365                 370                 375

Leu Tyr Tyr His Gly Lys Glu Gln Val Pro Arg Gly Cys Pro Asp
                380                 385                 390

Gly Leu Cys Pro Leu Asp Met Phe Leu Asn Ala Met Ser Val Tyr
                395                 400                 405

Thr Leu Ser Pro Glu Lys Tyr His Ala Leu Cys Ser Gln Thr Gln
                410                 415                 420

Val Met Glu Val Gly Asn Glu Glu
                425         428
```

<210> 57
<211> 24
<212> DNA
<213> Artificial Sequence

<220>
<223> Synthetic Oligonucleotide Probe

<400> 57
ccaactacca aagctgctgg agcc 24

<210> 58
<211> 24
<212> DNA
<213> Artificial Sequence

<220>
<223> Synthetic Oligonucleotide Probe

<400> 58
gcagctctat taccacggga agga 24

<210> 59
<211> 24
<212> DNA
<213> Artificial Sequence

<220>
<223> Synthetic Oligonucleotide Probe

<400> 59
tccttcccgt ggtaatagag ctgc 24

<210> 60
<211> 45
<212> DNA
<213> Artificial Sequence

<220>
<223> Synthetic Oligonucleotide Probe

<400> 60
ggcagagaac cagaggccgg aggagactgc ctctttacag ccagg 45

<210> 61
<211> 2477
<212> DNA
<213> Homo Sapien

<400> 61
cgagggcttt tccggctccg gaatggcaca tgtgggaatc ccagtcttgt 50

tggctacaac atttttccct ttcctaacaa gttctaacag ctgttctaac 100

agctagtgat caggggttct tcttgctgga gaagaaaggg ctgagggcag 150

agcagggcac tctcactcag ggtgaccagc tccttgcctc tctgtggata 200

acagagcatg agaaagtgaa gagatgcagc ggagtgaggt gatggaagtc 250

taaaatagga aggaattttg tgtgcaatat cagactctgg gagcagttga 300

cctggagagc ctgggggagg gcctgcctaa caagctttca aaaaacagga 350

gcgacttcca ctgggctggg ataagacgtg ccggtaggat agggaagact 400

gggtttagtc ctaatatcaa attgactggc tgggtgaact tcaacagcct 450

tttaacctct ctgggagatg aaaacgatgg cttaaggggc cagaaataga 500

gatgctttgt aaaataaaat tttaaaaaaa gcaagtattt tatagcataa 550

aggctagaga ccaaaataga taacaggatt ccctgaacat tcctaagagg 600

gagaaagtat gttaaaaata gaaaaaccaa aatgcagaag gaggagactc 650

acagagctaa accaggatgg ggaccctggg tcaggccagc ctctttgctc 700

ctcccggaaa ttatttttgg tctgaccact ctgccttgtg ttttgcagaa 750

tcatgtgagg gccaaccggg gaaggtggag cagatgagca cacacaggag 800

ccgtctcctc accgccgccc ctctcagcat ggaacagagg cagccctggc 850

cccgggccct ggaggtggac agccgctctg tggtcctgct ctcagtggtc 900

tgggtgctgc tggccccccc agcagccggc atgcctcagt tcagcacctt 950

ccactctgag aatcgtgact ggaccttcaa ccacttgacc gtccaccaag 1000

ggacgggggc cgtctatgtg gggcccatca accgggtcta taagctgaca 1050

ggcaacctga ccatccaggt ggctcataag acagggccag aagaggacaa 1100

caagtctcgt tacccgcccc tcatcgtgca gccctgcagc gaagtgctca 1150
ccctcaccaa caatgtcaac aagctgctca tcattgacta ctctgagaac 1200
cgcctgctgg cctgtgggag cctctaccag gggtctgca agctgctgcg 1250
gctggatgac ctcttcatcc tggtggagcc atcccacaag aaggagcact 1300
acctgtccag tgtcaacaag acgggcacca tgtacggggt gattgtgcgc 1350
tctgagggtg aggatggcaa gctcttcatc ggcacggctg tggatgggaa 1400
gcaggattac ttcccgaccc tgtccagccg gaagctgccc cgagaccctg 1450
agtcctcagc catgctcgac tatgagctac acagcgattt tgtctcctct 1500
ctcatcaaga tcccttcaga caccctggcc ctggtctccc actttgacat 1550
cttctacatc tacggctttg ctagtggggg ctttgtctac tttctcactg 1600
tccagcccga gacccctgag ggtgtggcca tcaactccgc tggagacctc 1650
ttctacacct cacgcatcgt gcggctctgc aaggatgacc ccaagttcca 1700
ctcatacgtg tccctgccct tcggctgcac ccgggccggg gtggaatacc 1750
gcctcctgca ggctgcttac ctggccaagc ctggggactc actggcccag 1800
gccttcaata tcaccagcca ggacgatgta ctctttgcca tcttctccaa 1850
agggcagaag cagtatcacc acccgcccga tgactctgcc ctgtgtgcct 1900
tccctatccg ggccatcaac ttgcagatca aggagcgcct gcagtcctgc 1950
taccagggcg agggcaacct ggagctcaac tggctgctgg ggaaggacgt 2000
ccagtgcacg aaggcgcctg tccccatcga tgataacttc tgtggactgg 2050
acatcaacca gcccctggga ggctcaactc cagtggaggg cctgaccctg 2100
tacaccacca gcagggaccg catgacctct gtggcctcct acgtttacaa 2150
cggctacagc gtggtttttg tggggactaa gagtggcaag ctgaaaaagg 2200
taagagtcta tgagttcaga tgctccaatg ccattcacct cctcagcaaa 2250
gagtccctct tggaaggtag ctattggtgg agatttaact ataggcaact 2300
ttattttctt ggggaacaaa ggtgaaatgg ggaggtaaga aggggttaat 2350
tttgtgactt agcttctagc tacttcctcc agccatcagt cattgggtat 2400
gtaaggaatg caagcgtatt tcaatatttc ccaaacttta agaaaaaact 2450
ttaagaaggt acatctgcaa aagcaaa 2477

<210> 62
<211> 552
<212> PRT
<213> Homo Sapien

<400> 62

```
Met Gly Thr Leu Gly Gln Ala Ser Leu Phe Ala Pro Pro Gly Asn
1               5                   10                  15

Tyr Phe Trp Ser Asp His Ser Ala Leu Cys Phe Ala Glu Ser Cys
                20                  25                  30

Glu Gly Gln Pro Gly Lys Val Glu Gln Met Ser Thr His Arg Ser
                35                  40                  45

Arg Leu Leu Thr Ala Ala Pro Leu Ser Met Glu Gln Arg Gln Pro
                50                  55                  60

Trp Pro Arg Ala Leu Glu Val Asp Ser Arg Ser Val Val Leu Leu
                65                  70                  75

Ser Val Val Trp Val Leu Leu Ala Pro Pro Ala Ala Gly Met Pro
                80                  85                  90

Gln Phe Ser Thr Phe His Ser Glu Asn Arg Asp Trp Thr Phe Asn
                95                  100                 105

His Leu Thr Val His Gln Gly Thr Gly Ala Val Tyr Val Gly Ala
                110                 115                 120

Ile Asn Arg Val Tyr Lys Leu Thr Gly Asn Leu Thr Ile Gln Val
                125                 130                 135

Ala His Lys Thr Gly Pro Glu Glu Asp Asn Lys Ser Arg Tyr Pro
                140                 145                 150

Pro Leu Ile Val Gln Pro Cys Ser Glu Val Leu Thr Leu Thr Asn
                155                 160                 165

Asn Val Asn Lys Leu Leu Ile Ile Asp Tyr Ser Glu Asn Arg Leu
                170                 175                 180

Leu Ala Cys Gly Ser Leu Tyr Gln Gly Val Cys Lys Leu Leu Arg
                185                 190                 195

Leu Asp Asp Leu Phe Ile Leu Val Glu Pro Ser His Lys Lys Glu
                200                 205                 210

His Tyr Leu Ser Ser Val Asn Lys Thr Gly Thr Met Tyr Gly Val
                215                 220                 225

Ile Val Arg Ser Glu Gly Glu Asp Gly Lys Leu Phe Ile Gly Thr
                230                 235                 240

Ala Val Asp Gly Lys Gln Asp Tyr Phe Pro Thr Leu Ser Ser Arg
                245                 250                 255

Lys Leu Pro Arg Asp Pro Glu Ser Ser Ala Met Leu Asp Tyr Glu
                260                 265                 270

Leu His Ser Asp Phe Val Ser Ser Leu Ile Lys Ile Pro Ser Asp
                275                 280                 285

Thr Leu Ala Leu Val Ser His Phe Asp Ile Phe Tyr Ile Tyr Gly
                290                 295                 300

Phe Ala Ser Gly Gly Phe Val Tyr Phe Leu Thr Val Gln Pro Glu
                305                 310                 315
```

```
Thr Pro Glu Gly Val Ala Ile Asn Ser Ala Gly Asp Leu Phe Tyr
                320                 325                 330

Thr Ser Arg Ile Val Arg Leu Cys Lys Asp Asp Pro Lys Phe His
                335                 340                 345

Ser Tyr Val Ser Leu Pro Phe Gly Cys Thr Arg Ala Gly Val Glu
                350                 355                 360

Tyr Arg Leu Leu Gln Ala Ala Tyr Leu Ala Lys Pro Gly Asp Ser
                365                 370                 375

Leu Ala Gln Ala Phe Asn Ile Thr Ser Gln Asp Asp Val Leu Phe
                380                 385                 390

Ala Ile Phe Ser Lys Gly Gln Lys Gln Tyr His His Pro Pro Asp
                395                 400                 405

Asp Ser Ala Leu Cys Ala Phe Pro Ile Arg Ala Ile Asn Leu Gln
                410                 415                 420

Ile Lys Glu Arg Leu Gln Ser Cys Tyr Gln Gly Glu Gly Asn Leu
                425                 430                 435

Glu Leu Asn Trp Leu Leu Gly Lys Asp Val Gln Cys Thr Lys Ala
                440                 445                 450

Pro Val Pro Ile Asp Asp Asn Phe Cys Gly Leu Asp Ile Asn Gln
                455                 460                 465

Pro Leu Gly Gly Ser Thr Pro Val Glu Gly Leu Thr Leu Tyr Thr
                470                 475                 480

Thr Ser Arg Asp Arg Met Thr Ser Val Ala Ser Tyr Val Tyr Asn
                485                 490                 495

Gly Tyr Ser Val Val Phe Val Gly Thr Lys Ser Gly Lys Leu Lys
                500                 505                 510

Lys Val Arg Val Tyr Glu Phe Arg Cys Ser Asn Ala Ile His Leu
                515                 520                 525

Leu Ser Lys Glu Ser Leu Leu Glu Gly Ser Tyr Trp Trp Arg Phe
                530                 535                 540

Asn Tyr Arg Gln Leu Tyr Phe Leu Gly Glu Gln Arg
                545                 550     552
```

<210> 63
<211> 20
<212> DNA
<213> Artificial Sequence

<220>
<223> Synthetic Oligonucleotide Probe

<400> 63
tggaataccg cctcctgcag 20

<210> 64
<211> 24
<212> DNA
<213> Artificial Sequence

<220>
<223> Synthetic Oligonucleotide Probe

<400> 64
cttctgccct ttggagaaga tggc 24

<210> 65
<211> 43
<212> DNA
<213> Artificial Sequence

<220>
<223> Synthetic Oligonucleotide Probe

<400> 65
ggactcactg gcccaggcct tcaatatcac cagccaggac gat 43

<210> 66
<211> 1295
<212> DNA
<213> Homo Sapien

<400> 66
cccagaagtt caagggcccc cggcctcctg cgctcctgcc gccgggaccc 50

tcgacctcct cagagcagcc ggctgccgcc ccgggaagat ggcgaggagg 100

agccgccacc gcctcctcct gctgctgctg cgctacctgg tggtcgccct 150

gggctatcat aaggcctatg ggttttctgc cccaaaagac caacaagtag 200

tcacagcagt agagtaccaa gaggctattt tagcctgcaa aaccccaaag 250

aagactgttt cctccagatt agagtggaag aaactgggtc ggagtgtctc 300

ctttgtctac tatcaacaga ctcttcaagg tgattttaaa aatcgagctg 350

agatgataga tttcaatatc cggatcaaaa atgtgacaag aagtgatgcg 400

gggaaatatc gttgtgaagt tagtgcccca tctgagcaag gccaaaacct 450

ggaagaggat acagtcactc tggaagtatt agtggctcca gcagttccat 500

catgtgaagt accctcttct gctctgagtg gaactgtggt agagctacga 550

tgtcaagaca aagaagggaa tccagctcct gaatacacat ggtttaagga 600

tggcatccgt ttgctagaaa atcccagact tggctcccaa agcaccaaca 650

gctcatacac aatgaataca aaaactggaa ctctgcaatt taatactgtt 700

tccaaactgg acactggaga atattcctgt gaagcccgca attctgttgg 750

atatcgcagg tgtcctggga aacgaatgca agtagatgat ctcaacataa 800

gtggcatcat agcagccgta gtagttgtgg ccttagtgat ttccgtttgt 850

ggccttggtg tatgctatgc tcagaggaaa ggctactttt caaaagaaac 900

ctccttccag aagagtaatt cttcatctaa agccacgaca atgagtgaaa 950

atgtgcagtg gctcacgcct gtaatcccag cactttggaa ggccgcggcg 1000

ggcggatcac gaggtcagga gttctagacc agtctggcca atatggtgaa 1050

accccatctc tactaaaata caaaaattag ctgggcatgg tggcatgtgc 1100

ctgcagttcc agctgcttgg gagacaggag aatcacttga acccgggagg 1150

cggaggttgc agtgagctga gatcacgcca ctgcagtcca gcctgggtaa 1200

cagagcaaga ttccatctca aaaaataaaa taaataaata aataaatact 1250

ggtttttacc tgtagaattc ttacaataaa tatagcttga tattc 1295

<210> 67
<211> 312
<212> PRT
<213> Homo Sapien

<400> 67
Met Ala Arg Arg Ser Arg His Arg Leu Leu Leu Leu Leu Leu Arg
1 5 10 15

Tyr Leu Val Val Ala Leu Gly Tyr His Lys Ala Tyr Gly Phe Ser
20 25 30

Ala Pro Lys Asp Gln Gln Val Val Thr Ala Val Glu Tyr Gln Glu
35 40 45

Ala Ile Leu Ala Cys Lys Thr Pro Lys Lys Thr Val Ser Ser Arg
50 55 60

Leu Glu Trp Lys Lys Leu Gly Arg Ser Val Ser Phe Val Tyr Tyr
65 70 75

Gln Gln Thr Leu Gln Gly Asp Phe Lys Asn Arg Ala Glu Met Ile
80 85 90

Asp Phe Asn Ile Arg Ile Lys Asn Val Thr Arg Ser Asp Ala Gly
95 100 105

Lys Tyr Arg Cys Glu Val Ser Ala Pro Ser Glu Gln Gly Gln Asn
110 115 120

Leu Glu Glu Asp Thr Val Thr Leu Glu Val Leu Val Ala Pro Ala
125 130 135

Val Pro Ser Cys Glu Val Pro Ser Ser Ala Leu Ser Gly Thr Val
140 145 150

Val Glu Leu Arg Cys Gln Asp Lys Glu Gly Asn Pro Ala Pro Glu
155 160 165

Tyr Thr Trp Phe Lys Asp Gly Ile Arg Leu Leu Glu Asn Pro Arg
170 175 180

Leu Gly Ser Gln Ser Thr Asn Ser Ser Tyr Thr Met Asn Thr Lys
185 190 195

Thr Gly Thr Leu Gln Phe Asn Thr Val Ser Lys Leu Asp Thr Gly
200 205 210

Glu Tyr Ser Cys Glu Ala Arg Asn Ser Val Gly Tyr Arg Arg Cys
215 220 225

```
Pro Gly Lys Arg Met Gln Val Asp Asp Leu Asn Ile Ser Gly Ile
                230                 235                 240

Ile Ala Ala Val Val Val Val Ala Leu Val Ile Ser Val Cys Gly
                245                 250                 255

Leu Gly Val Cys Tyr Ala Gln Arg Lys Gly Tyr Phe Ser Lys Glu
                260                 265                 270

Thr Ser Phe Gln Lys Ser Asn Ser Ser Ser Lys Ala Thr Thr Met
                275                 280                 285

Ser Glu Asn Val Gln Trp Leu Thr Pro Val Ile Pro Ala Leu Trp
                290                 295                 300

Lys Ala Ala Ala Gly Gly Ser Arg Gly Gln Glu Phe
                305                 310     312
```

<210> 68
<211> 22
<212> DNA
<213> Artificial Sequence

<220>
<223> Synthetic Oligonucleotide Probe

<400> 68
atcgttgtga agttagtgcc cc 22

<210> 69
<211> 23
<212> DNA
<213> Artificial Sequence

<220>
<223> Synthetic Oligonucleotide Probe

<400> 69
acctgcgata tccaacagaa ttg 23

<210> 70
<211> 48
<212> DNA
<213> Artificial Sequence

<220>
<223> Synthetic Oligonucleotide Probe

<400> 70
ggaagaggat acagtcactc tggaagtatt agtggctcca gcagttcc 48

<210> 71
<211> 1266
<212> DNA
<213> Homo Sapien

<400> 71
gctggggaca tgagaggcac accgaagacc cacctcctgg ccttctccct 50

cctctgcctc ctctcaaagg tgcgtaccca gctgtgcccg acaccatgta 100

cctgcccctg gccacctccc cgatgcccgc tgggagtacc cctggtgctg 150

gatggctgtg gctgctgccg ggtatgtgca cggcggctgg gggagccctg 200

cgaccaactc cacgtctgcg acgccagcca gggcctggtc tgccagcccg 250

gggcaggacc cggtggccgg ggggccctgt gcctcttggc agaggacgac 300

agcagctgtg aggtgaacgg ccgcctgtat cgggaagggg agaccttcca 350

gccccactgc agcatccgct gccgctgcga ggacggcggc ttcacctgcg 400

tgccgctgtg cagcgaggat gtgcggctgc ccagctggga ctgcccccac 450

cccaggaggg tcgaggtcct gggcaagtgc tgccctgagt gggtgtgcgg 500

ccaaggaggg ggactgggga cccagcccct tccagcccaa ggaccccagt 550

tttctggcct tgtctcttcc ctgccccctg gtgtcccctg cccagaatgg 600

agcacggcct ggggaccctg ctcgaccacc tgtgggctgg gcatggccac 650

ccgggtgtcc aaccagaacc gcttctgccg actggagacc cagcgccgcc 700

tgtgcctgtc caggccctgc ccaccctcca ggggtcgcag tccacaaaac 750

agtgccttct agagccgggc tgggaatggg gacacggtgt ccaccatccc 800

cagctggtgg ccctgtgcct gggccctggg ctgatggaag atggtccgtg 850

cccaggccct tggctgcagg caacacttta gcttgggtcc accatgcaga 900

acaccaatat taacacgctg cctggtctgt ctggatcccg aggtatggca 950

gaggtgcaag acctagtccc ctttcctcta actcactgcc taggaggctg 1000

gccaaggtgt ccagggtcct ctagcccact ccctgcctac acacacagcc 1050

tatatcaaac atgcacacgg gcgagctttc tctccgactt cccctgggca 1100

agagatggga caagcagtcc cttaatattg aggctgcagc aggtgctggg 1150

ctggactggc catttttctg ggggtaggat gaagagaagg cacacagaga 1200

ttctggatct cctgctgcct tttctggagt ttgtaaaatt gttcctgaat 1250

acaagcctat gcgtga 1266

<210> 72
<211> 250
<212> PRT
<213> Homo Sapien

<400> 72
Met Arg Gly Thr Pro Lys Thr His Leu Leu Ala Phe Ser Leu Leu
1 5 10 15

Cys Leu Leu Ser Lys Val Arg Thr Gln Leu Cys Pro Thr Pro Cys
20 25 30

Thr Cys Pro Trp Pro Pro Pro Arg Cys Pro Leu Gly Val Pro Leu
35 40 45

Val Leu Asp Gly Cys Gly Cys Cys Arg Val Cys Ala Arg Arg Leu
50 55 60

Gly Glu Pro Cys Asp Gln Leu His Val Cys Asp Ala Ser Gln Gly
65 70 75

Leu Val Cys Gln Pro Gly Ala Gly Pro Gly Gly Arg Gly Ala Leu
80 85 90

Cys Leu Leu Ala Glu Asp Asp Ser Ser Cys Glu Val Asn Gly Arg
95 100 105

Leu Tyr Arg Glu Gly Glu Thr Phe Gln Pro His Cys Ser Ile Arg
110 115 120

Cys Arg Cys Glu Asp Gly Gly Phe Thr Cys Val Pro Leu Cys Ser
125 130 135

Glu Asp Val Arg Leu Pro Ser Trp Asp Cys Pro His Pro Arg Arg
140 145 150

Val Glu Val Leu Gly Lys Cys Cys Pro Glu Trp Val Cys Gly Gln
155 160 165

Gly Gly Gly Leu Gly Thr Gln Pro Leu Pro Ala Gln Gly Pro Gln
170 175 180

Phe Ser Gly Leu Val Ser Ser Leu Pro Pro Gly Val Pro Cys Pro
185 190 195

Glu Trp Ser Thr Ala Trp Gly Pro Cys Ser Thr Thr Cys Gly Leu
200 205 210

Gly Met Ala Thr Arg Val Ser Asn Gln Asn Arg Phe Cys Arg Leu
215 220 225

Glu Thr Gln Arg Arg Leu Cys Leu Ser Arg Pro Cys Pro Pro Ser
230 235 240

Arg Gly Arg Ser Pro Gln Asn Ser Ala Phe
245 250

<210> 73
<211> 23
<212> DNA
<213> Artificial Sequence

<220>
<223> Synthetic Oligonucleotide Probe

<400> 73
aaaggtgcgt acccagctgt gcc 23

<210> 74
<211> 24
<212> DNA
<213> Artificial Sequence

<220>
<223> Synthetic Oligonucleotide Probe

<400> 74
tccagtcggc agaagcggtt ctgg 24

<210> 75
<211> 51

<212> DNA
<213> Artificial Sequence

<220>
<223> Synthetic Oligonucleotide Probe

<400> 75
cctggtgctg gatggctgtg gctgctgccg ggtatgtgca cggcggctgg 50

g 51

<210> 76
<211> 2226
<212> DNA
<213> Homo Sapien

<400> 76
agtcgactgc gtcccctgta cccggcgcca gctgtgttcc tgaccccaga 50

ataactcagg gctgcaccgg gcctggcagc gctccgcaca catttcctgt 100

cgcggcctaa gggaaactgt tggccgctgg gcccgcgggg ggattcttgg 150

cagttggggg gtccgtcggg agcgagggcg gaggggaagg gagggggaac 200

cgggttgggg aagccagctg tagagggcgg tgaccgcgct ccagacacag 250

ctctgcgtcc tcgagcggga cagatccaag ttgggagcag ctctgcgtgc 300

ggggcctcag agaatgaggc cggcgttcgc cctgtgcctc ctctggcagg 350

cgctctggcc cgggccgggc ggcggcgaac accccactgc cgaccgtgct 400

ggctgctcgg cctcgggggc ctgctacagc ctgcaccacg ctaccatgaa 450

gcggcaggcg gccgaggagg cctgcatcct gcgaggtggg gcgctcagca 500

ccgtgcgtgc gggcgccgag ctgcgcgctg tgctcgcgct cctgcgggca 550

ggcccagggc ccggaggggg ctccaaagac ctgctgttct gggtcgcact 600

ggagcgcagg cgttcccact gcaccctgga gaacgagcct ttgcggggtt 650

tctcctggct gtcctccgac cccggcggtc tcgaaagcga cacgctgcag 700

tgggtggagg agccccaacg ctcctgcacc gcgcggagat gcgcggtact 750

ccaggccacc ggtggggtcg agcccgcagg ctggaaggag atgcgatgcc 800

acctgcgcgc caacggctac ctgtgcaagt accagtttga ggtcttgtgt 850

cctgcgccgc gccccggggc cgcctctaac ttgagctatc gcgcgccctt 900

ccagctgcac agcgccgctc tggacttcag tccacctggg accgaggtga 950

gtgcgctctg ccggggacag ctcccgatct cagttacttg catcgcggac 1000

gaaatcggcg ctcgctggga caaactctcg ggcgatgtgt tgtgtccctg 1050

ccccgggagg tacctccgtg ctggcaaatg cgcagagctc cctaactgcc 1100

tagacgactt gggaggcttt gcctgcgaat gtgctacggg cttcgagctg 1150

gggaaggacg gccgctcttg tgtgaccagt ggggaaggac agccgaccct 1200

tggggggacc ggggtgccca ccaggcgccc gccggccact gcaaccagcc 1250

ccgtgccgca gagaacatgg ccaatcaggg tcgacgagaa gctgggagag 1300

acaccacttg tccctgaaca agacaattca gtaacatcta ttcctgagat 1350

tcctcgatgg ggatcacaga gcacgatgtc tacccttcaa atgtcccttc 1400

aagccgagtc aaaggccact atcaccccat cagggagcgt gatttccaag 1450

tttaattcta cgacttcctc tgccactcct caggctttcg actcctcctc 1500

tgccgtggtc ttcatatttg tgagcacagc agtagtagtg ttggtgatct 1550

tgaccatgac agtactgggg cttgtcaagc tctgctttca cgaaagcccc 1600

tcttcccagc caaggaagga gtctatgggc ccgccgggcc tggagagtga 1650

tcctgagccc gctgctttgg gctccagttc tgcacattgc acaaacaatg 1700

gggtgaaagt cggggactgt gatctgcggg acagagcaga gggtgccttg 1750

ctggcggagt cccctcttgg ctctagtgat gcatagggaa acaggggaca 1800

tgggcactcc tgtgaacagt tttcactttt tgatgaaacg gggaaccaag 1850

aggaacttac ttgtgtaact gacaatttct gcagaaatcc cccttcctct 1900

aaattccctt tactccactg aggagctaaa tcagaactgc acactccttc 1950

cctgatgata gaggaagtgg aagtgccttt aggatggtga tactggggga 2000

ccgggtagtg ctggggagag atattttctt atgtttattc ggagaatttg 2050

gagaagtgat tgaacttttc aagacattgg aaacaaatag aacacaatat 2100

aatttacatt aaaaaataat ttctaccaaa atggaaagga aatgttctat 2150

gttgttcagg ctaggagtat attggttcga aatcccaggg aaaaaaataa 2200

aaataaaaaa ttaaaggatt gttgat 2226

<210> 77
<211> 490
<212> PRT
<213> Homo Sapien

<400> 77
Met Arg Pro Ala Phe Ala Leu Cys Leu Leu Trp Gln Ala Leu Trp
1 5 10 15

Pro Gly Pro Gly Gly Gly Glu His Pro Thr Ala Asp Arg Ala Gly
20 25 30

Cys Ser Ala Ser Gly Ala Cys Tyr Ser Leu His His Ala Thr Met
35 40 45

Lys Arg Gln Ala Ala Glu Glu Ala Cys Ile Leu Arg Gly Gly Ala
50 55 60

Leu Ser Thr Val Arg Ala Gly Ala Glu Leu Arg Ala Val Leu Ala
65 70 75

Leu Leu Arg Ala Gly Pro Gly Pro Gly Gly Gly Ser Lys Asp Leu
80 85 90

Leu Phe Trp Val Ala Leu Glu Arg Arg Arg Ser His Cys Thr Leu
95 100 105

Glu Asn Glu Pro Leu Arg Gly Phe Ser Trp Leu Ser Ser Asp Pro
110 115 120

Gly Gly Leu Glu Ser Asp Thr Leu Gln Trp Val Glu Glu Pro Gln
125 130 135

Arg Ser Cys Thr Ala Arg Arg Cys Ala Val Leu Gln Ala Thr Gly
140 145 150

Gly Val Glu Pro Ala Gly Trp Lys Glu Met Arg Cys His Leu Arg
155 160 165

Ala Asn Gly Tyr Leu Cys Lys Tyr Gln Phe Glu Val Leu Cys Pro
170 175 180

Ala Pro Arg Pro Gly Ala Ala Ser Asn Leu Ser Tyr Arg Ala Pro
185 190 195

Phe Gln Leu His Ser Ala Ala Leu Asp Phe Ser Pro Pro Gly Thr
200 205 210

Glu Val Ser Ala Leu Cys Arg Gly Gln Leu Pro Ile Ser Val Thr
215 220 225

Cys Ile Ala Asp Glu Ile Gly Ala Arg Trp Asp Lys Leu Ser Gly
230 235 240

Asp Val Leu Cys Pro Cys Pro Gly Arg Tyr Leu Arg Ala Gly Lys
245 250 255

Cys Ala Glu Leu Pro Asn Cys Leu Asp Asp Leu Gly Gly Phe Ala
260 265 270

Cys Glu Cys Ala Thr Gly Phe Glu Leu Gly Lys Asp Gly Arg Ser
275 280 285

Cys Val Thr Ser Gly Glu Gly Gln Pro Thr Leu Gly Gly Thr Gly
290 295 300

Val Pro Thr Arg Arg Pro Pro Ala Thr Ala Thr Ser Pro Val Pro
305 310 315

Gln Arg Thr Trp Pro Ile Arg Val Asp Glu Lys Leu Gly Glu Thr
320 325 330

Pro Leu Val Pro Glu Gln Asp Asn Ser Val Thr Ser Ile Pro Glu
335 340 345

Ile Pro Arg Trp Gly Ser Gln Ser Thr Met Ser Thr Leu Gln Met
350 355 360

Ser Leu Gln Ala Glu Ser Lys Ala Thr Ile Thr Pro Ser Gly Ser
365 370 375

```
Val Ile Ser Lys Phe Asn Ser Thr Thr Ser Ser Ala Thr Pro Gln
                380                 385                 390

Ala Phe Asp Ser Ser Ser Ala Val Val Phe Ile Phe Val Ser Thr
                395                 400                 405

Ala Val Val Val Leu Val Ile Leu Thr Met Thr Val Leu Gly Leu
                410                 415                 420

Val Lys Leu Cys Phe His Glu Ser Pro Ser Ser Gln Pro Arg Lys
                425                 430                 435

Glu Ser Met Gly Pro Pro Gly Leu Glu Ser Asp Pro Glu Pro Ala
                440                 445                 450

Ala Leu Gly Ser Ser Ser Ala His Cys Thr Asn Asn Gly Val Lys
                455                 460                 465

Val Gly Asp Cys Asp Leu Arg Asp Arg Ala Glu Gly Ala Leu Leu
                470                 475                 480

Ala Glu Ser Pro Leu Gly Ser Ser Asp Ala
                485                 490
```

<210> 78
<211> 24
<212> DNA
<213> Artificial Sequence

<220>
<223> Synthetic Oligonucleotide Probe

<400> 78
tggaaggaga tgcgatgcca cctg 24

<210> 79
<211> 20
<212> DNA
<213> Artificial Sequence

<220>
<223> Synthetic Oligonucleotide Probe

<400> 79
tgaccagtgg ggaaggacag 20

<210> 80
<211> 20
<212> DNA
<213> Artificial Sequence

<220>
<223> Synthetic Oligonucleotide Probe

<400> 80
acagagcaga gggtgccttg 20

<210> 81
<211> 24
<212> DNA
<213> Artificial Sequence

<220>

<223> Synthetic Oligonucleotide Probe

<400> 81
tcagggacaa gtggtgtctc tccc 24

<210> 82
<211> 24
<212> DNA
<213> Artificial Sequence

<220>
<223> Synthetic Oligonucleotide Probe

<400> 82
tcagggaagg agtgtgcagt tctg 24

<210> 83
<211> 50
<212> DNA
<213> Artificial Sequence

<220>
<223> Synthetic Oligonucleotide Probe

<400> 83
acagctcccg atctcagtta cttgcatcgc ggacgaaatc ggcgctcgct 50

<210> 84
<211> 2026
<212> DNA
<213> Homo Sapien

<400> 84
cggacgcgtg ggattcagca gtggcctgtg gctgccagag cagctcctca 50

ggggaaacta agcgtcgagt cagacggcac cataatcgcc tttaaaagtg 100

cctccgccct gccggccgcg tatcccccgg ctacctgggc cgccccgcgg 150

cggtgcgcgc gtgagaggga gcgcgcgggc agccgagcgc cggtgtgagc 200

cagcgctgct gccagtgtga gcggcggtgt gagcgcggtg ggtgcggagg 250

ggcgtgtgtg ccggcgcgcg cgccgtgggg tgcaaacccc gagcgtctac 300

gctgccatga ggggcgcgaa cgcctgggcg ccactctgcc tgctgctggc 350

tgccgccacc cagctctcgc ggcagcagtc cccagagaga cctgttttca 400

catgtggtgg cattcttact ggagagtctg gatttattgg cagtgaaggt 450

tttcctggag tgtaccctcc aaatagcaaa tgtacttgga aaatcacagt 500

tcccgaagga aaagtagtcg ttctcaattt ccgattcata gacctcgaga 550

gtgacaacct gtgccgctat gactttgtgg atgtgtacaa tggccatgcc 600

aatggccagc gcattggccg cttctgtggc actttccggc ctggagccct 650

tgtgtccagt ggcaacaaga tgatggtgca gatgatttct gatgccaaca 700

cagctggcaa tggcttcatg gccatgttct ccgctgctga accaaacgaa 750

agaggggatc agtattgtgg aggactcctt gacagacctt ccggctcttt 800

taaaaccccc aactggccag accgggatta ccctgcagga gtcacttgtg 850

tgtggcacat tgtagcccca aagaatcagc ttatagaatt aaagtttgag 900

aagtttgatg tggagcgaga taactactgc cgatatgatt atgtggctgt 950

gtttaatggc ggggaagtca acgatgctag aagaattgga aagtattgtg 1000

gtgatagtcc acctgcgcca attgtgtctg agagaaatga acttcttatt 1050

cagtttttat cagacttaag tttaactgca gatgggttta ttggtcacta 1100

catattcagg ccaaaaaaac tgcctacaac tacagaacag cctgtcacca 1150

ccacattccc tgtaaccacg ggtttaaaac ccaccgtggc cttgtgtcaa 1200

caaaagtgta gacggacggg gactctggag ggcaattatt gttcaagtga 1250

ctttgtatta gccggcactg ttatcacaac catcactcgc gatgggagtt 1300

tgcacgccac agtctcgatc atcaacatct acaaagaggg aaatttggcg 1350

attcagcagg cgggcaagaa catgagtgcc aggctgactg tcgtctgcaa 1400

gcagtgccct ctcctcagaa gaggtctaaa ttacattatt atgggccaag 1450

taggtgaaga tgggcgaggc aaaatcatgc caaacagctt tatcatgatg 1500

ttcaagacca agaatcagaa gctcctggat gccttaaaaa ataagcaatg 1550

ttaacagtga actgtgtcca tttaagctgt attctgccat tgcctttgaa 1600

agatctatgt tctctcagta gaaaaaaaaa tacttataaa attacatatt 1650

ctgaaagagg attccgaaag atgggactgg ttgactcttc acatgatgga 1700

ggtatgaggc ctccgagata gctgagggaa gttctttgcc tgctgtcaga 1750

ggagcagcta tctgattgga aacctgccga cttagtgcgg tgataggaag 1800

ctaaaagtgt caagcgttga cagcttggaa gcgtttattt atacatctct 1850

gtaaaaggat attttagaat tgagttgtgt gaagatgtca aaaaaagatt 1900

ttagaagtgc aatatttata gtgttatttg tttcaccttc aagcctttgc 1950

cctgaggtgt tacaatcttg tcttgcgttt tctaaatcaa tgcttaataa 2000

aatattttta aaggaaaaaa aaaaaa 2026

<210> 85
<211> 415
<212> PRT
<213> Homo Sapien

<400> 85
Met Arg Gly Ala Asn Ala Trp Ala Pro Leu Cys Leu Leu Leu Ala
1 5 10 15

Ala Ala Thr Gln Leu Ser Arg Gln Gln Ser Pro Glu Arg Pro Val
20 25 30

```
Phe Thr Cys Gly Gly Ile Leu Thr Gly Glu Ser Gly Phe Ile Gly
                35                  40                  45

Ser Glu Gly Phe Pro Gly Val Tyr Pro Pro Asn Ser Lys Cys Thr
                50                  55                  60

Trp Lys Ile Thr Val Pro Glu Gly Lys Val Val Val Leu Asn Phe
                65                  70                  75

Arg Phe Ile Asp Leu Glu Ser Asp Asn Leu Cys Arg Tyr Asp Phe
                80                  85                  90

Val Asp Val Tyr Asn Gly His Ala Asn Gly Gln Arg Ile Gly Arg
                95                  100                 105

Phe Cys Gly Thr Phe Arg Pro Gly Ala Leu Val Ser Ser Gly Asn
                110                 115                 120

Lys Met Met Val Gln Met Ile Ser Asp Ala Asn Thr Ala Gly Asn
                125                 130                 135

Gly Phe Met Ala Met Phe Ser Ala Ala Glu Pro Asn Glu Arg Gly
                140                 145                 150

Asp Gln Tyr Cys Gly Gly Leu Leu Asp Arg Pro Ser Gly Ser Phe
                155                 160                 165

Lys Thr Pro Asn Trp Pro Asp Arg Asp Tyr Pro Ala Gly Val Thr
                170                 175                 180

Cys Val Trp His Ile Val Ala Pro Lys Asn Gln Leu Ile Glu Leu
                185                 190                 195

Lys Phe Glu Lys Phe Asp Val Glu Arg Asp Asn Tyr Cys Arg Tyr
                200                 205                 210

Asp Tyr Val Ala Val Phe Asn Gly Gly Glu Val Asn Asp Ala Arg
                215                 220                 225

Arg Ile Gly Lys Tyr Cys Gly Asp Ser Pro Pro Ala Pro Ile Val
                230                 235                 240

Ser Glu Arg Asn Glu Leu Leu Ile Gln Phe Leu Ser Asp Leu Ser
                245                 250                 255

Leu Thr Ala Asp Gly Phe Ile Gly His Tyr Ile Phe Arg Pro Lys
                260                 265                 270

Lys Leu Pro Thr Thr Thr Glu Gln Pro Val Thr Thr Thr Phe Pro
                275                 280                 285

Val Thr Thr Gly Leu Lys Pro Thr Val Ala Leu Cys Gln Gln Lys
                290                 295                 300

Cys Arg Arg Thr Gly Thr Leu Glu Gly Asn Tyr Cys Ser Ser Asp
                305                 310                 315

Phe Val Leu Ala Gly Thr Val Ile Thr Thr Ile Thr Arg Asp Gly
                320                 325                 330

Ser Leu His Ala Thr Val Ser Ile Ile Asn Ile Tyr Lys Glu Gly
                335                 340                 345
```

Asn Leu Ala Ile Gln Gln Ala Gly Lys Asn Met Ser Ala Arg Leu
350 355 360

Thr Val Val Cys Lys Gln Cys Pro Leu Leu Arg Arg Gly Leu Asn
365 370 375

Tyr Ile Ile Met Gly Gln Val Gly Glu Asp Gly Arg Gly Lys Ile
380 385 390

Met Pro Asn Ser Phe Ile Met Met Phe Lys Thr Lys Asn Gln Lys
395 400 405

Leu Leu Asp Ala Leu Lys Asn Lys Gln Cys
410 415

<210> 86
<211> 22
<212> DNA
<213> Artificial Sequence

<220>
<223> Synthetic Oligonucleotide Probe

<400> 86
ccgattcata gacctcgaga gt 22

<210> 87
<211> 22
<212> DNA
<213> Artificial Sequence

<220>
<223> Synthetic Oligonucleotide Probe

<400> 87
gtcaaggagt cctccacaat ac 22

<210> 88
<211> 45
<212> DNA
<213> Artificial Sequence

<220>
<223> Synthetic Oligonucleotide Probe

<400> 88
gtgtacaatg gccatgccaa tggccagcgc attggccgct tctgt 45

<210> 89
<211> 1857
<212> DNA
<213> Homo Sapien

<400> 89
gtctgttccc aggagtcctt cggcggctgt tgtgtcagtg gcctgatcgc 50

gatggggaca aaggcgcaag tcgagaggaa actgttgtgc ctcttcatat 100

tggcgatcct gttgtgctcc ctggcattgg gcagtgttac agtgcactct 150

tctgaacctg aagtcagaat tcctgagaat aatcctgtga agttgtcctg 200

tgcctactcg ggcttttctt ctccccgtgt ggagtggaag tttgaccaag 250

gagacaccac cagactcgtt tgctataata acaagatcac agcttcctat 300
gaggaccggg tgaccttctt gccaactggt atcaccttca agtccgtgac 350
acgggaagac actgggacat acacttgtat ggtctctgag gaaggcggca 400
acagctatgg ggaggtcaag gtcaagctca tcgtgcttgt gcctccatcc 450
aagcctacag ttaacatccc ctcctctgcc accattggga accgggcagt 500
gctgacatgc tcagaacaag atggttcccc accttctgaa tacacctggt 550
tcaaagatgg gatagtgatg cctacgaatc ccaaaagcac ccgtgccttc 600
agcaactctt cctatgtcct gaatcccaca acaggagagc tggtctttga 650
tcccctgtca gcctctgata ctggagaata cagctgtgag gcacggaatg 700
ggtatgggac acccatgact tcaaatgctg tgcgcatgga agctgtggag 750
cggaatgtgg gggtcatcgt ggcagccgtc cttgtaaccc tgattctcct 800
gggaatcttg gtttttggca tctggtttgc ctatagccga ggccactttg 850
acagaacaaa gaaagggact tcgagtaaga aggtgattta cagccagcct 900
agtgcccgaa gtgaaggaga attcaaacag acctcgtcat tcctggtgtg 950
agcctggtcg gctcaccgcc tatcatctgc atttgcctta ctcaggtgct 1000
accggactct ggcccctgat gtctgtagtt tcacaggatg ccttatttgt 1050
cttctacacc ccacagggcc ccctacttct tcggatgtgt ttttaataat 1100
gtcagctatg tgccccatcc tccttcatgc cctccctccc tttcctacca 1150
ctgctgagtg gcctggaact tgtttaaagt gtttattccc catttctttg 1200
agggatcagg aaggaatcct gggtatgcca ttgacttccc ttctaagtag 1250
acagcaaaaa tggcgggggt cgcaggaatc tgcactcaac tgcccacctg 1300
gctggcaggg atctttgaat aggtatcttg agcttggttc tgggctcttt 1350
ccttgtgtac tgacgaccag ggccagctgt tctagagcgg gaattagagg 1400
ctagagcggc tgaaatggtt gtttggtgat gacactgggg tccttccatc 1450
tctggggccc actctcttct gtcttcccat gggaagtgcc actgggatcc 1500
ctctgccctg tcctcctgaa tacaagctga ctgacattga ctgtgtctgt 1550
ggaaaatggg agctcttgtt gtggagagca tagtaaattt tcagagaact 1600
tgaagccaaa aggatttaaa accgctgctc taaagaaaag aaaactggag 1650
gctgggcgca gtggctcacg cctgtaatcc cagaggctga ggcaggcgga 1700
tcacctgagg tcgggagttc gggatcagcc tgaccaacat ggagaaaccc 1750
tactggaaat acaaagttag ccaggcatgg tggtgcatgc ctgtagtccc 1800
agctgctcag gagcctggca acaagagcaa aactccagct caaaaaaaaa 1850

aaaaaaa 1857

<210> 90
<211> 299
<212> PRT
<213> Homo Sapien

<400> 90
Met Gly Thr Lys Ala Gln Val Glu Arg Lys Leu Leu Cys Leu Phe
1 5 10 15

Ile Leu Ala Ile Leu Leu Cys Ser Leu Ala Leu Gly Ser Val Thr
20 25 30

Val His Ser Ser Glu Pro Glu Val Arg Ile Pro Glu Asn Asn Pro
35 40 45

Val Lys Leu Ser Cys Ala Tyr Ser Gly Phe Ser Ser Pro Arg Val
50 55 60

Glu Trp Lys Phe Asp Gln Gly Asp Thr Thr Arg Leu Val Cys Tyr
65 70 75

Asn Asn Lys Ile Thr Ala Ser Tyr Glu Asp Arg Val Thr Phe Leu
80 85 90

Pro Thr Gly Ile Thr Phe Lys Ser Val Thr Arg Glu Asp Thr Gly
95 100 105

Thr Tyr Thr Cys Met Val Ser Glu Glu Gly Gly Asn Ser Tyr Gly
110 115 120

Glu Val Lys Val Lys Leu Ile Val Leu Val Pro Pro Ser Lys Pro
125 130 135

Thr Val Asn Ile Pro Ser Ser Ala Thr Ile Gly Asn Arg Ala Val
140 145 150

Leu Thr Cys Ser Glu Gln Asp Gly Ser Pro Pro Ser Glu Tyr Thr
155 160 165

Trp Phe Lys Asp Gly Ile Val Met Pro Thr Asn Pro Lys Ser Thr
170 175 180

Arg Ala Phe Ser Asn Ser Ser Tyr Val Leu Asn Pro Thr Thr Gly
185 190 195

Glu Leu Val Phe Asp Pro Leu Ser Ala Ser Asp Thr Gly Glu Tyr
200 205 210

Ser Cys Glu Ala Arg Asn Gly Tyr Gly Thr Pro Met Thr Ser Asn
215 220 225

Ala Val Arg Met Glu Ala Val Glu Arg Asn Val Gly Val Ile Val
230 235 240

Ala Ala Val Leu Val Thr Leu Ile Leu Leu Gly Ile Leu Val Phe
245 250 255

Gly Ile Trp Phe Ala Tyr Ser Arg Gly His Phe Asp Arg Thr Lys
260 265 270

Lys Gly Thr Ser Ser Lys Lys Val Ile Tyr Ser Gln Pro Ser Ala
275 280 285

Arg Ser Glu Gly Glu Phe Lys Gln Thr Ser Ser Phe Leu Val
290 295 299

<210> 91
<211> 24
<212> DNA
<213> Artificial Sequence

<220>
<223> Synthetic Oligonucleotide Probe

<400> 91
tcgcggagct gtgttctgtt tccc 24

<210> 92
<211> 20
<212> DNA
<213> Artificial Sequence

<220>
<223> Synthetic Oligonucleotide Probe

<400> 92
acacctggtt caaagatggg 20

<210> 93
<211> 20
<212> DNA
<213> Artificial Sequence

<220>
<223> Synthetic Oligonucleotide Probe

<400> 93
ttgccttact caggtgctac 20

<210> 94
<211> 24
<212> DNA
<213> Artificial Sequence

<220>
<223> Synthetic Oligonucleotide Probe

<400> 94
taggaagagt tgctgaaggc acgg 24

<210> 95
<211> 20
<212> DNA
<213> Artificial Sequence

<220>
<223> Synthetic Oligonucleotide Probe

<400> 95
actcagcagt ggtaggaaag 20

<210> 96
<211> 50

<212> DNA
<213> Artificial Sequence

<220>
<223> Synthetic Oligonucleotide Probe

<400> 96
tgatcgcgat ggggacaaag gcgcaagctc gagaggaaac tgttgtgcct 50

<210> 97
<211> 1520
<212> DNA
<213> Homo Sapien

<400> 97
gctgagtctg ctgctcctgc tgctgctgct ccagcctgta acctgtgcct 50

acaccacgcc aggccccccc agagccctca ccacgctggg cgcccccaga 100

gcccacacca tgccgggcac ctacgctccc tcgaccacac tcagtagtcc 150

cagcacccag ggcctgcaag agcaggcacg ggccctgatg cgggacttcc 200

cgctcgtgga cggccacaac gacctgcccc tggtcctaag gcaggtttac 250

cagaaagggc tacaggatgt taacctgcgc aatttcagct acggccagac 300

cagcctggac aggcttagag atggcctcgt gggcgcccag ttctggtcag 350

cctatgtgcc atgccagacc caggaccggg atgccctgcg cctcaccctg 400

gagcagattg acctcatacg ccgcatgtgt gcctcctatt ctgagctgga 450

gcttgtgacc tcggctaaag ctctgaacga cactcagaaa ttggcctgcc 500

tcatcggtgt agagggtggc cactcgctgg acaatagcct ctccatctta 550

cgtaccttct acatgctggg agtgcgctac ctgacgctca cccacacctg 600

caacacaccc tgggcagaga gctccgctaa gggcgtccac tccttctaca 650

acaacatcag cgggctgact gactttggtg agaaggtggt ggcagaaatg 700

aaccgcctgg gcatgatggt agacttatcc catgtctcag atgctgtggc 750

acggcgggcc ctggaagtgt cacaggcacc tgtgatcttc tcccactcgg 800

ctgcccgggg tgtgtgcaac agtgctcgga atgttcctga tgacatcctg 850

cagcttctga agaagaacgg tggcgtcgtg atggtgtctt tgtccatggg 900

agtaatacag tgcaacccat cagccaatgt gtccactgtg gcagatcact 950

tcgaccacat caaggctgtc attggatcca agttcatcgg gattggtgga 1000

gattatgatg ggccggcaa attccctcag ggctggaag acgtgtccac 1050

atacccggtc ctgatagagg agttgctgag tcgtggctgg agtgaggaag 1100

agcttcaggg tgtccttcgt ggaaacctgc tgcgggtctt cagacaagtg 1150

gaaaaggtac aggaagaaaa caaatggcaa agccccttgg aggacaagtt 1200

cccggatgag cagctgagca gttcctgcca ctccgacctc tcacgtctgc 1250

gtcagagaca gagtctgact tcaggccagg aactcactga gattcccata 1300

cactggacag ccaagttacc agccaagtgg tcagtctcag agtcctcccc 1350

ccacatggcc ccagtccttg cagttgtggc caccttccca gtccttattc 1400

tgtggctctg atgacccagt tagtcctgcc agatgtcact gtagcaagcc 1450

acagacaccc cacaaagttc ccctgttgtg caggcacaaa tatttcctga 1500

aataaatgtt ttggacatag 1520

<210> 98
<211> 433
<212> PRT
<213> Homo Sapien

<400> 98

```
Met Pro Gly Thr Tyr Ala Pro Ser Thr Thr Leu Ser Ser Pro Ser
 1               5                  10                  15

Thr Gln Gly Leu Gln Glu Gln Ala Arg Ala Leu Met Arg Asp Phe
                20                  25                  30

Pro Leu Val Asp Gly His Asn Asp Leu Pro Leu Val Leu Arg Gln
                35                  40                  45

Val Tyr Gln Lys Gly Leu Gln Asp Val Asn Leu Arg Asn Phe Ser
                50                  55                  60

Tyr Gly Gln Thr Ser Leu Asp Arg Leu Arg Asp Gly Leu Val Gly
                65                  70                  75

Ala Gln Phe Trp Ser Ala Tyr Val Pro Cys Gln Thr Gln Asp Arg
                80                  85                  90

Asp Ala Leu Arg Leu Thr Leu Glu Gln Ile Asp Leu Ile Arg Arg
                95                 100                 105

Met Cys Ala Ser Tyr Ser Glu Leu Glu Leu Val Thr Ser Ala Lys
               110                 115                 120

Ala Leu Asn Asp Thr Gln Lys Leu Ala Cys Leu Ile Gly Val Glu
               125                 130                 135

Gly Gly His Ser Leu Asp Asn Ser Leu Ser Ile Leu Arg Thr Phe
               140                 145                 150

Tyr Met Leu Gly Val Arg Tyr Leu Thr Leu Thr His Thr Cys Asn
               155                 160                 165

Thr Pro Trp Ala Glu Ser Ser Ala Lys Gly Val His Ser Phe Tyr
               170                 175                 180

Asn Asn Ile Ser Gly Leu Thr Asp Phe Gly Glu Lys Val Val Ala
               185                 190                 195

Glu Met Asn Arg Leu Gly Met Met Val Asp Leu Ser His Val Ser
               200                 205                 210
```

Asp Ala Val Ala Arg Arg Ala Leu Glu Val Ser Gln Ala Pro Val
215 220 225

Ile Phe Ser His Ser Ala Ala Arg Gly Val Cys Asn Ser Ala Arg
230 235 240

Asn Val Pro Asp Asp Ile Leu Gln Leu Leu Lys Lys Asn Gly Gly
245 250 255

Val Val Met Val Ser Leu Ser Met Gly Val Ile Gln Cys Asn Pro
260 265 270

Ser Ala Asn Val Ser Thr Val Ala Asp His Phe Asp His Ile Lys
275 280 285

Ala Val Ile Gly Ser Lys Phe Ile Gly Ile Gly Gly Asp Tyr Asp
290 295 300

Gly Ala Gly Lys Phe Pro Gln Gly Leu Glu Asp Val Ser Thr Tyr
305 310 315

Pro Val Leu Ile Glu Glu Leu Leu Ser Arg Gly Trp Ser Glu Glu
320 325 330

Glu Leu Gln Gly Val Leu Arg Gly Asn Leu Leu Arg Val Phe Arg
335 340 345

Gln Val Glu Lys Val Gln Glu Glu Asn Lys Trp Gln Ser Pro Leu
350 355 360

Glu Asp Lys Phe Pro Asp Glu Gln Leu Ser Ser Ser Cys His Ser
365 370 375

Asp Leu Ser Arg Leu Arg Gln Arg Gln Ser Leu Thr Ser Gly Gln
380 385 390

Glu Leu Thr Glu Ile Pro Ile His Trp Thr Ala Lys Leu Pro Ala
395 400 405

Lys Trp Ser Val Ser Glu Ser Ser Pro His Met Ala Pro Val Leu
410 415 420

Ala Val Val Ala Thr Phe Pro Val Leu Ile Leu Trp Leu
425 430 433

<210> 99
<211> 22
<212> DNA
<213> Artificial Sequence

<220>
<223> Synthetic Oligonucleotide Probe

<400> 99
agttctggtc agcctatgtg cc 22

<210> 100
<211> 24
<212> DNA
<213> Artificial Sequence

<220>
<223> Synthetic Oligonucleotide Probe

<400> 100
cgtgatggtg tctttgtcca tggg 24

<210> 101
<211> 24
<212> DNA
<213> Artificial Sequence

<220>
<223> Synthetic Oligonucleotide Probe

<400> 101
ctccaccaat cccgatgaac ttgg 24

<210> 102
<211> 50
<212> DNA
<213> Artificial Sequence

<220>
<223> Synthetic Oligonucleotide Probe

<400> 102
gagcagattg acctcatacg ccgcatgtgt gcctcctatt ctgagctgga 50

<210> 103
<211> 23
<212> DNA
<213> Artificial Sequence

<220>
<223> Synthetic Oligonucleotide Probe

<400> 103
tgctgctgct ccagcctgta acc 23

<210> 104
<211> 22
<212> DNA
<213> Artificial Sequence

<220>
<223> Synthetic Oligonucleotide probe

<400> 104
ctggccgtag ctgaaattgc gc 22

<210> 105
<211> 43
<212> DNA
<213> Artificial Sequence

<220>
<223> Synthetic Oligonucleotide Probe

<400> 105
actcagtagt cccagcaccc agggcctgca agagcaggca cgg 43

<210> 106
<211> 2906
<212> DNA
<213> Homo Sapien

<400> 106

```
ggggagagga attgaccatg taaaaggaga cttttttttt tggtggtggt 50
ggctgttggg tgccttgcaa aaatgaagga tgcaggacgc agctttctcc 100
tggaaccgaa cgcaatggat aaactgattg tgcaagagag aaggaagaac 150
gaagcttttt cttgtgagcc ctggatctta acacaaatgt gtatatgtgc 200
acacagggag cattcaagaa tgaaataaac cagagttaga cccgcggggg 250
ttggtgtgtt ctgacataaa taaataatct taaagcagct gttcccctcc 300
ccaccccaa aaaaaaggat gattggaaat gaagaaccga ggattcacaa 350
agaaaaaagt atgttcattt ttctctataa aggagaaagt gagccaagga 400
gatatttttg gaatgaaaag tttggggctt ttttagtaaa gtaaagaact 450
ggtgtggtgg tgttttcctt tctttttgaa tttcccacaa gaggagagga 500
aattaataat acatctgcaa agaaatttca gagaagaaaa gttgaccgcg 550
gcagattgag gcattgattg ggggagagaa accagcagag cacagttgga 600
tttgtgccta tgttgactaa aattgacgga taattgcagt tggatttttc 650
ttcatcaacc tccttttttt taaattttta ttccttttgg tatcaagatc 700
atgcgttttc tcttgttctt aaccacctgg atttccatct ggatgttgct 750
gtgatcagtc tgaaatacaa ctgtttgaat tccagaagga ccaacaccag 800
ataaattatg aatgttgaac aagatgacct tacatccaca gcagataatg 850
ataggtccta ggtttaacag ggccctattt gaccccctgc ttgtggtgct 900
gctggctctt caacttcttg tggtggctgg tctggtgcgg gctcagacct 950
gcccttctgt gtgctcctgc agcaaccagt tcagcaaggt gatttgtgtt 1000
cggaaaaacc tgcgtgaggt tccggatggc atctccacca acacacggct 1050
gctgaacctc catgagaacc aaatccagat catcaaagtg aacagcttca 1100
agcacttgag gcacttggaa atcctacagt tgagtaggaa ccatatcaga 1150
accattgaaa ttggggcttt caatggtctg gcgaacctca acactctgga 1200
actctttgac aatcgtctta ctaccatccc gaatggagct tttgtatact 1250
tgtctaaact gaaggagctc tggttgcgaa acaaccccat tgaaagcatc 1300
ccttcttatg cttttaacag aattccttct ttgcgccgac tagacttagg 1350
ggaattgaaa agactttcat acatctcaga aggtgccttt gaaggtctgt 1400
ccaacttgag gtatttgaac cttgccatgt gcaaccttcg ggaaatccct 1450
aacctcacac cgctcataaa actagatgag ctggatcttt ctgggaatca 1500
tttatctgcc atcaggcctg gctctttcca gggtttgatg caccttcaaa 1550
```

aactgtggat gatacagtcc cagattcaag tgattgaacg gaatgccttt 1600

gacaaccttc agtcactagt ggagatcaac ctggcacaca ataatctaac 1650

attactgcct catgacctct tcactccctt gcatcatcta gagcggatac 1700

atttacatca caacccttgg aactgtaact gtgacatact gtggctcagc 1750

tggtggataa aagacatggc cccctcgaac acagcttgtt gtgcccggtg 1800

taacactcct cccaatctaa aggggaggta cattggagag ctcgaccaga 1850

attacttcac atgctatgct ccggtgattg tggagccccc tgcagacctc 1900

aatgtcactg aaggcatggc agctgagctg aaatgtcggg cctccacatc 1950

cctgacatct gtatcttgga ttactccaaa tggaacagtc atgacacatg 2000

gggcgtacaa agtgcggata gctgtgctca gtgatggtac gttaaatttc 2050

acaaatgtaa ctgtgcaaga tacaggcatg tacacatgta tggtgagtaa 2100

ttccgttggg aatactactg cttcagccac cctgaatgtt actgcagcaa 2150

ccactactcc tttctcttac ttttcaaccg tcacagtaga gactatggaa 2200

ccgtctcagg atgaggcacg gaccacagat aacaatgtgg gtcccactcc 2250

agtggtcgac tgggagacca ccaatgtgac cacctctctc acaccacaga 2300

gcacaaggtc gacagagaaa accttcacca tcccagtgac tgatataaac 2350

agtgggatcc caggaattga tgaggtcatg aagactacca aaatcatcat 2400

tgggtgtttt gtggccatca cactcatggc tgcagtgatg ctggtcattt 2450

tctacaagat gaggaagcag caccatcggc aaaaccatca cgccccaaca 2500

aggactgttg aaattattaa tgtggatgat gagattacgg gagacacacc 2550

catggaaagc cacctgccca tgcctgctat cgagcatgag cacctaaatc 2600

actataactc atacaaatct cccttcaacc acacaacaac agttaacaca 2650

ataaattcaa tacacagttc agtgcatgaa ccgttattga tccgaatgaa 2700

ctctaaagac aatgtacaag agactcaaat ctaaaacatt tacagagtta 2750

caaaaaacaa acaatcaaaa aaaaagacag tttattaaaa atgacacaaa 2800

tgactgggct aaatctactg tttcaaaaaa gtgtctttac aaaaaaacaa 2850

aaaagaaaag aaatttattt attaaaaatt ctattgtgat ctaaagcaga 2900

caaaaa 2906

<210> 107
<211> 640
<212> PRT
<213> Homo Sapien

<400> 107

```
Met Leu Asn Lys Met Thr Leu His Pro Gln Gln Ile Met Ile Gly
1               5                   10                  15

Pro Arg Phe Asn Arg Ala Leu Phe Asp Pro Leu Leu Val Val Leu
                20                  25                  30

Leu Ala Leu Gln Leu Leu Val Val Ala Gly Leu Val Arg Ala Gln
                35                  40                  45

Thr Cys Pro Ser Val Cys Ser Cys Ser Asn Gln Phe Ser Lys Val
                50                  55                  60

Ile Cys Val Arg Lys Asn Leu Arg Glu Val Pro Asp Gly Ile Ser
                65                  70                  75

Thr Asn Thr Arg Leu Leu Asn Leu His Glu Asn Gln Ile Gln Ile
                80                  85                  90

Ile Lys Val Asn Ser Phe Lys His Leu Arg His Leu Glu Ile Leu
                95                  100                 105

Gln Leu Ser Arg Asn His Ile Arg Thr Ile Glu Ile Gly Ala Phe
                110                 115                 120

Asn Gly Leu Ala Asn Leu Asn Thr Leu Glu Leu Phe Asp Asn Arg
                125                 130                 135

Leu Thr Thr Ile Pro Asn Gly Ala Phe Val Tyr Leu Ser Lys Leu
                140                 145                 150

Lys Glu Leu Trp Leu Arg Asn Asn Pro Ile Glu Ser Ile Pro Ser
                155                 160                 165

Tyr Ala Phe Asn Arg Ile Pro Ser Leu Arg Arg Leu Asp Leu Gly
                170                 175                 180

Glu Leu Lys Arg Leu Ser Tyr Ile Ser Glu Gly Ala Phe Glu Gly
                185                 190                 195

Leu Ser Asn Leu Arg Tyr Leu Asn Leu Ala Met Cys Asn Leu Arg
                200                 205                 210

Glu Ile Pro Asn Leu Thr Pro Leu Ile Lys Leu Asp Glu Leu Asp
                215                 220                 225

Leu Ser Gly Asn His Leu Ser Ala Ile Arg Pro Gly Ser Phe Gln
                230                 235                 240

Gly Leu Met His Leu Gln Lys Leu Trp Met Ile Gln Ser Gln Ile
                245                 250                 255

Gln Val Ile Glu Arg Asn Ala Phe Asp Asn Leu Gln Ser Leu Val
                260                 265                 270

Glu Ile Asn Leu Ala His Asn Asn Leu Thr Leu Leu Pro His Asp
                275                 280                 285

Leu Phe Thr Pro Leu His His Leu Glu Arg Ile His Leu His His
                290                 295                 300

Asn Pro Trp Asn Cys Asn Cys Asp Ile Leu Trp Leu Ser Trp Trp
                305                 310                 315
```

```
Ile Lys Asp Met Ala Pro Ser Asn Thr Ala Cys Cys Ala Arg Cys
                320                 325                 330

Asn Thr Pro Pro Asn Leu Lys Gly Arg Tyr Ile Gly Glu Leu Asp
                335                 340                 345

Gln Asn Tyr Phe Thr Cys Tyr Ala Pro Val Ile Val Glu Pro Pro
                350                 355                 360

Ala Asp Leu Asn Val Thr Glu Gly Met Ala Ala Glu Leu Lys Cys
                365                 370                 375

Arg Ala Ser Thr Ser Leu Thr Ser Val Ser Trp Ile Thr Pro Asn
                380                 385                 390

Gly Thr Val Met Thr His Gly Ala Tyr Lys Val Arg Ile Ala Val
                395                 400                 405

Leu Ser Asp Gly Thr Leu Asn Phe Thr Asn Val Thr Val Gln Asp
                410                 415                 420

Thr Gly Met Tyr Thr Cys Met Val Ser Asn Ser Val Gly Asn Thr
                425                 430                 435

Thr Ala Ser Ala Thr Leu Asn Val Thr Ala Ala Thr Thr Thr Pro
                440                 445                 450

Phe Ser Tyr Phe Ser Thr Val Thr Val Glu Thr Met Glu Pro Ser
                455                 460                 465

Gln Asp Glu Ala Arg Thr Thr Asp Asn Asn Val Gly Pro Thr Pro
                470                 475                 480

Val Val Asp Trp Glu Thr Thr Asn Val Thr Thr Ser Leu Thr Pro
                485                 490                 495

Gln Ser Thr Arg Ser Thr Glu Lys Thr Phe Thr Ile Pro Val Thr
                500                 505                 510

Asp Ile Asn Ser Gly Ile Pro Gly Ile Asp Glu Val Met Lys Thr
                515                 520                 525

Thr Lys Ile Ile Ile Gly Cys Phe Val Ala Ile Thr Leu Met Ala
                530                 535                 540

Ala Val Met Leu Val Ile Phe Tyr Lys Met Arg Lys Gln His His
                545                 550                 555

Arg Gln Asn His His Ala Pro Thr Arg Thr Val Glu Ile Ile Asn
                560                 565                 570

Val Asp Asp Glu Ile Thr Gly Asp Thr Pro Met Glu Ser His Leu
                575                 580                 585

Pro Met Pro Ala Ile Glu His Glu His Leu Asn His Tyr Asn Ser
                590                 595                 600

Tyr Lys Ser Pro Phe Asn His Thr Thr Thr Val Asn Thr Ile Asn
                605                 610                 615

Ser Ile His Ser Ser Val His Glu Pro Leu Leu Ile Arg Met Asn
                620                 625                 630
```

Ser Lys Asp Asn Val Gln Glu Thr Gln Ile
635 640

<210> 108
<211> 28
<212> DNA
<213> Artificial Sequence

<220>
<223> Synthetic Oligonucleotide Probe

<400> 108
gcctttgaca accttcagtc actagtgg 28

<210> 109
<211> 24
<212> DNA
<213> Artificial Sequence

<220>
<223> Synthetic Oligonucleotide Probe

<400> 109
ccccatgtgt ccatgactgt tccc 24

<210> 110
<211> 45
<212> DNA
<213> Artificial Sequence

<220>
<223> Synthetic Oligonucleotide Probe

<400> 110
tactgcctca tgacctcttc actcccttgc atcatcttag agcgg 45

<210> 111
<211> 2212
<212> DNA
<213> Homo Sapien

<400> 111
gaaagctata ggctacccat tcagctcccc tgtcagagac tcaagctttg 50

agaaaggcta gcaaagagca aggaaagaga gaaaacaaca aagtggcgag 100

gccctcagag tgaaagcgta aggttcagtc agcctgctgc agctttgcag 150

acctcagctg ggcatctcca gactcccctg aaggaagagc cttcctcacc 200

caaacccaca aaagatgctg aaaaagcctc tctcagctgt gacctggctc 250

tgcattttca tcgtggcctt tgtcagccac ccagcgtggc tgcagaagct 300

ctctaagcac aagacaccag cacagccaca gctcaaagcg gccaactgct 350

gtgaggaggt gaaggagctc aaggcccaag ttgccaacct tagcagcctg 400

ctgagtgaac tgaacaagaa gcaggagagg gactgggtca gcgtggtcat 450

gcaggtgatg gagctggaga gcaacagcaa gcgcatggag tcgcggctca 500

cagatgctga gagcaagtac tccgagatga acaaccaaat tgacatcatg 550

cagctgcagg cagcacagac ggtcactcag acctccgcag atgccatcta 600
cgactgctct tccctctacc agaagaacta ccgcatctct ggagtgtata 650
agcttcctcc tgatgacttc ctgggcagcc ctgaactgga ggtgttctgt 700
gacatggaga cttcaggcgg aggctggacc atcatccaga gacgaaaaag 750
tggccttgtc tccttctacc gggactggaa gcagtacaag cagggctttg 800
gcagcatccg tggggacttc tggctgggga acgaacacat ccaccggctc 850
tccagacagc caacccggct gcgtgtagag atggaggact gggagggcaa 900
cctgcgctac gctgagtata gccactttgt tttgggcaat gaactcaaca 950
gctatcgcct cttcctgggg aactacactg gcaatgtggg gaacgacgcc 1000
ctccagtatc ataacaacac agccttcagc accaaggaca aggacaatga 1050
caactgcttg gacaagtgtg cacagctccg caaaggtggc tactggtaca 1100
actgctgcac agactccaac ctcaatggag tgtactaccg cctgggtgag 1150
cacaataagc acctggatgg catcacctgg tatggctggc atggatctac 1200
ctactccctc aaacgggtgg agatgaaaat ccgcccagaa gacttcaagc 1250
cttaaaagga ggctgccgtg gagcacggat acagaaactg agacacgtgg 1300
agactggatg agggcagatg aggacaggaa gagagtgtta gaaagggtag 1350
gactgagaaa cagcctataa tctccaaaga aagaataagt ctccaaggag 1400
cacaaaaaaa tcatatgtac caaggatgtt acagtaaaca ggatgaacta 1450
tttaaaccca ctgggtcctg ccacatcctt ctcaaggtgg tagactgagt 1500
ggggtctctc tgcccaagat ccctgacata gcagtagctt gtcttttcca 1550
catgatttgt ctgtgaaaga aaataatttt gagatcgttt tatctatttt 1600
ctctacggct taggctatgt gagggcaaaa cacaaatccc tttgctaaaa 1650
agaaccatat tattttgatt ctcaaaggat aggcctttga gtgttagaga 1700
aaggagtgaa ggaggcaggt gggaaatggt atttctattt ttaaatccag 1750
tgaaattatc ttgagtctac acattatttt taaaacacaa aaattgttcg 1800
gctggaactg acccaggctg gacttgcggg gaggaaactc cagggcactg 1850
catctggcga tcagactctg agcactgccc ctgctcgcct tggtcatgta 1900
cagcactgaa aggaatgaag caccagcagg aggtggacag agtctctcat 1950
ggatgccggc acaaaactgc cttaaaatat tcatagttaa tacaggtata 2000
tctattttta tttactttgt aagaaacaag ctcaaggagc ttccttttaa 2050
attttgtctg taggaaatgg ttgaaaactg aaggtagatg gtgttatagt 2100
taataataaa tgctgtaaat aagcatctca ctttgtaaaa ataaaatatt 2150

gtggttttgt tttaaacatt caacgtttct tttccttcta caataaacac 2200

tttcaaaatg tg 2212

<210> 112
<211> 346
<212> PRT
<213> Homo Sapien

<400> 112
Met Leu Lys Lys Pro Leu Ser Ala Val Thr Trp Leu Cys Ile Phe
1 5 10 15

Ile Val Ala Phe Val Ser His Pro Ala Trp Leu Gln Lys Leu Ser
20 25 30

Lys His Lys Thr Pro Ala Gln Pro Gln Leu Lys Ala Ala Asn Cys
35 40 45

Cys Glu Glu Val Lys Glu Leu Lys Ala Gln Val Ala Asn Leu Ser
50 55 60

Ser Leu Leu Ser Glu Leu Asn Lys Lys Gln Glu Arg Asp Trp Val
65 70 75

Ser Val Val Met Gln Val Met Glu Leu Glu Ser Asn Ser Lys Arg
80 85 90

Met Glu Ser Arg Leu Thr Asp Ala Glu Ser Lys Tyr Ser Glu Met
95 100 105

Asn Asn Gln Ile Asp Ile Met Gln Leu Gln Ala Ala Gln Thr Val
110 115 120

Thr Gln Thr Ser Ala Asp Ala Ile Tyr Asp Cys Ser Ser Leu Tyr
125 130 135

Gln Lys Asn Tyr Arg Ile Ser Gly Val Tyr Lys Leu Pro Pro Asp
140 145 150

Asp Phe Leu Gly Ser Pro Glu Leu Glu Val Phe Cys Asp Met Glu
155 160 165

Thr Ser Gly Gly Gly Trp Thr Ile Ile Gln Arg Arg Lys Ser Gly
170 175 180

Leu Val Ser Phe Tyr Arg Asp Trp Lys Gln Tyr Lys Gln Gly Phe
185 190 195

Gly Ser Ile Arg Gly Asp Phe Trp Leu Gly Asn Glu His Ile His
200 205 210

Arg Leu Ser Arg Gln Pro Thr Arg Leu Arg Val Glu Met Glu Asp
215 220 225

Trp Glu Gly Asn Leu Arg Tyr Ala Glu Tyr Ser His Phe Val Leu
230 235 240

Gly Asn Glu Leu Asn Ser Tyr Arg Leu Phe Leu Gly Asn Tyr Thr
245 250 255

Gly Asn Val Gly Asn Asp Ala Leu Gln Tyr His Asn Asn Thr Ala
260 265 270

Phe Ser Thr Lys Asp Lys Asp Asn Asp Asn Cys Leu Asp Lys Cys
275 280 285

Ala Gln Leu Arg Lys Gly Gly Tyr Trp Tyr Asn Cys Cys Thr Asp
290 295 300

Ser Asn Leu Asn Gly Val Tyr Tyr Arg Leu Gly Glu His Asn Lys
305 310 315

His Leu Asp Gly Ile Thr Trp Tyr Gly Trp His Gly Ser Thr Tyr
320 325 330

Ser Leu Lys Arg Val Glu Met Lys Ile Arg Pro Glu Asp Phe Lys
335 340 345

Pro
346

<210> 113
<211> 31
<212> DNA
<213> Artificial Sequence

<220>
<223> Synthetic Oligonucleotide Probe

<400> 113
ttcagcacca aggacaagga caatgacaac t 31

<210> 114
<211> 32
<212> DNA
<213> Artificial Sequence

<220>
<223> Synthetic Oligonucleotide Probe

<400> 114
tgtgcacact tgtccaagca gttgtcattg tc 32

<210> 115
<211> 23
<212> DNA
<213> Artificial Sequence

<220>
<223> Synthetic Oligonucleotide Probe

<400> 115
gtagtacact ccattgaggt tgg 23

<210> 116
<211> 1008
<212> DNA
<213> Homo Sapien

<400> 116
cacgcacttc acctgggtcg ggattctcag gtcatgaacg gtcccagcca 50

cctccgggca gggcgggtga ggacggggac ggggcgtgtc caactggctg 100

tgggctcttg aaacccgagc atggcacagc acggggcgat gggcgcgttt 150

cgggccctgt gcggcctggc gctgctgtgc gcgctcagcc tgggtcagcg 200
ccccaccggg ggtcccgggt gcggccctgg gcgcctcctg cttgggacgg 250
gaacggacgc gcgctgctgc cgggttcaca cgacgcgctg ctgccgcgat 300
tacccgggcg aggagtgctg ttccgagtgg gactgcatgt gtgtccagcc 350
tgaattccac tgcggagacc cttgctgcac gacctgccgg caccaccctt 400
gtcccccagg ccagggggta cagtcccagg ggaaattcag ttttggcttc 450
cagtgtatcg actgtgcctc ggggaccttc tccgggggcc acgaaggcca 500
ctgcaaacct tggacagact gcacccagtt cgggtttctc actgtgttcc 550
ctgggaacaa gacccacaac gctgtgtgcg tcccagggtc cccgccggca 600
gagccgcttg ggtggctgac cgtcgtcctc ctggccgtgg ccgcctgcgt 650
cctcctcctg acctcggccc agcttggact gcacatctgg cagctgagga 700
gtcagtgcat gtggccccga gagacccagc tgctgctgga ggtgccgccg 750
tcgaccgaag acgccagaag ctgccagttc cccgaggaag agcggggcga 800
gcgatcggca gaggagaagg ggcggctggg agacctgtgg gtgtgagcct 850
ggccgtcctc cggggccacc gaccgcagcc agcccctccc caggagctcc 900
ccaggccgca ggggctctgc gttctgctct gggccgggcc ctgctcccct 950
ggcagcagaa gtgggtgcag gaaggtggca gtgaccagcg ccctggacca 1000
tgcagttc 1008

<210> 117
<211> 241
<212> PRT
<213> Homo Sapien

<400> 117

```
Met Ala Gln His Gly Ala Met Gly Ala Phe Arg Ala Leu Cys Gly
 1               5                   10                  15

Leu Ala Leu Leu Cys Ala Leu Ser Leu Gly Gln Arg Pro Thr Gly
                20                  25                  30

Gly Pro Gly Cys Gly Pro Gly Arg Leu Leu Leu Gly Thr Gly Thr
                35                  40                  45

Asp Ala Arg Cys Cys Arg Val His Thr Thr Arg Cys Cys Arg Asp
                50                  55                  60

Tyr Pro Gly Glu Glu Cys Cys Ser Glu Trp Asp Cys Met Cys Val
                65                  70                  75

Gln Pro Glu Phe His Cys Gly Asp Pro Cys Cys Thr Thr Cys Arg
                80                  85                  90

His His Pro Cys Pro Pro Gly Gln Gly Val Gln Ser Gln Gly Lys
                95                  100                 105
```

Phe Ser Phe Gly Phe Gln Cys Ile Asp Cys Ala Ser Gly Thr Phe
110 115 120

Ser Gly Gly His Glu Gly His Cys Lys Pro Trp Thr Asp Cys Thr
125 130 135

Gln Phe Gly Phe Leu Thr Val Phe Pro Gly Asn Lys Thr His Asn
140 145 150

Ala Val Cys Val Pro Gly Ser Pro Pro Ala Glu Pro Leu Gly Trp
155 160 165

Leu Thr Val Val Leu Leu Ala Val Ala Ala Cys Val Leu Leu Leu
170 175 180

Thr Ser Ala Gln Leu Gly Leu His Ile Trp Gln Leu Arg Ser Gln
185 190 195

Cys Met Trp Pro Arg Glu Thr Gln Leu Leu Leu Glu Val Pro Pro
200 205 210

Ser Thr Glu Asp Ala Arg Ser Cys Gln Phe Pro Glu Glu Glu Arg
215 220 225

Gly Glu Arg Ser Ala Glu Glu Lys Gly Arg Leu Gly Asp Leu Trp
230 235 240

Val
241

<210> 118
<211> 19
<212> DNA
<213> Artificial Sequence

<220>
<223> Synthetic Oligonucleotide Probe

<400> 118
cacagcacgg ggcgatggg 19

<210> 119
<211> 18
<212> DNA
<213> Artificial Sequence

<220>
<223> Synthetic Oligonucleotide Probe

<400> 119
gctctgcgtt ctgctctg 18

<210> 120
<211> 28
<212> DNA
<213> Artificial Sequence

<220>
<223> Synthetic Oligonucleotide Probe

<400> 120
ggcacagcac ggggcgatgg gcgcgttt 28

<210> 121
<211> 24
<212> DNA
<213> Artificial Sequence

<220>
<223> Synthetic Oligonucleotide Probe

<400> 121
ctggtcactg ccaccttcct gcac 24

<210> 122
<211> 17
<212> DNA
<213> Artificial Sequence

<220>
<223> Synthetic Oligonucleotide Probe

<400> 122
cgctgaccca ggctgag 17

<210> 123
<211> 27
<212> DNA
<213> Artificial Sequence

<220>
<223> Synthetic Oligonucleotide Probe

<400> 123
gaaggtcccc gaggcacagt cgataca 27

<210> 124
<211> 40
<212> DNA
<213> Artificial Sequence

<220>
<223> Synthetic Oligonucleotide Probe

<400> 124
gaggagtgct gttccgagtg ggactgcatg tgtgtccagc 40

<210> 125
<211> 40
<212> DNA
<213> Artificial Sequence

<220>
<223> Synthetic Oligonucleotide Probe

<400> 125
agcctgggtc agcgccccac cgggggtccc gggtgcggcc 40

<210> 126
<211> 2236
<212> DNA
<213> Homo Sapien

<400> 126
ggcgccggtg caccgggcgg gctgagcgcc tcctgcggcc cggcctgcgc 50

gccccggccc gccgcgccgc ccacgcccca accccggccc gcgcccccta 100
gcccccgccc gggcccgcgc ccgcgcccgc gcccaggtga gcgctccgcc 150
cgccgcgagg ccccgccccg gcccgccccc gccccgcccc ggccggcggg 200
ggaaccgggc ggattcctcg cgcgtcaaac cacctgatcc cataaaacat 250
tcatcctccc ggcggcccgc gctgcgagcg ccccgccagt ccgcgccgcc 300
gccgccctcg ccctgtgcgc cctgcgcgcc ctgcgcaccc gcggcccgag 350
cccagccaga gccgggcgga gcggagcgcg ccgagcctcg tcccgcggcc 400
gggccggggc cgggccgtag cggcggcgcc tggatgcgga cccggccgcg 450
gggagacggg cgcccgcccc gaaacgactt tcagtccccg acgcgccccg 500
cccaacccct acgatgaaga gggcgtccgc tggagggagc cggctgctgg 550
catgggtgct gtggctgcag gcctggcagg tggcagcccc atgcccaggt 600
gcctgcgtat gctacaatga gcccaaggtg acgacaagct gcccccagca 650
gggcctgcag gctgtgcccg tgggcatccc tgctgccagc cagcgcatct 700
tcctgcacgg caaccgcatc tcgcatgtgc cagctgccag cttccgtgcc 750
tgccgcaacc tcaccatcct gtggctgcac tcgaatgtgc tggcccgaat 800
tgatgcggct gccttcactg gcctggccct cctggagcag ctggacctca 850
gcgataatgc acagctccgg tctgtggacc ctgccacatt ccacggcctg 900
ggccgcctac acacgctgca cctggaccgc tgcggcctgc aggagctggg 950
cccggggctg ttccgcggcc tggctgccct gcagtacctc tacctgcagg 1000
acaacgcgct gcaggcactg cctgatgaca ccttccgcga cctgggcaac 1050
ctcacacacc tcttcctgca cggcaaccgc atctccagcg tgcccgagcg 1100
cgccttccgt gggctgcaca gcctcgaccg tctcctactg caccagaacc 1150
gcgtggccca tgtgcacccg catgccttcc gtgaccttgg ccgcctcatg 1200
acactctatc tgtttgccaa caatctatca gcgctgccca ctgaggccct 1250
ggcccccctg cgtgccctgc agtacctgag gctcaacgac aacccctggg 1300
tgtgtgactg ccgggcacgc ccactctggg cctggctgca gaagttccgc 1350
ggctcctcct ccgaggtgcc ctgcagcctc ccgcaacgcc tggctggccg 1400
tgacctcaaa cgcctagctg ccaatgacct gcagggctgc gctgtggcca 1450
ccggccctta ccatcccatc tggaccggca gggccaccga tgaggagccg 1500
ctggggcttc ccaagtgctg ccagccagat gccgctgaca aggcctcagt 1550
actggagcct ggaagaccag cttcggcagg caatgcgctg aagggacgcg 1600
tgccgcccgg tgacagcccg ccgggcaacg gctctggccc acggcacatc 1650

aatgactcac cctttgggac tctgcctggc tctgctgagc ccccgctcac 1700

tgcagtgcgg cccgagggct ccgagccacc agggttcccc acctcgggcc 1750

ctcgccggag gccaggctgt tcacgcaaga accgcacccg cagccactgc 1800

cgtctgggcc aggcaggcag cgggggtggc gggactggtg actcagaagg 1850

ctcaggtgcc ctacccagcc tcacctgcag cctcaccccc ctgggcctgg 1900

cgctggtgct gtggacagtg cttgggccct gctgaccccc agcggacaca 1950

agagcgtgct cagcagccag gtgtgtgtac atacggggtc tctctccacg 2000

ccgccaagcc agccgggcgg ccgacccgtg gggcaggcca ggccaggtcc 2050

tccctgatgg acgcctgccg cccgccaccc ccatctccac cccatcatgt 2100

ttacagggtt cggcggcagc gtttgttcca gaacgccgcc tcccacccag 2150

atcgcggtat atagagatat gcattttatt ttacttgtgt aaaaatatcg 2200

gacgacgtgg aataaagagc tcttttctta aaaaaa 2236

<210> 127
<211> 473
<212> PRT
<213> Homo Sapien

<400> 127
Met Lys Arg Ala Ser Ala Gly Gly Ser Arg Leu Leu Ala Trp Val
1               5                   10                  15

Leu Trp Leu Gln Ala Trp Gln Val Ala Ala Pro Cys Pro Gly Ala
                20                  25                  30

Cys Val Cys Tyr Asn Glu Pro Lys Val Thr Thr Ser Cys Pro Gln
                35                  40                  45

Gln Gly Leu Gln Ala Val Pro Val Gly Ile Pro Ala Ala Ser Gln
                50                  55                  60

Arg Ile Phe Leu His Gly Asn Arg Ile Ser His Val Pro Ala Ala
                65                  70                  75

Ser Phe Arg Ala Cys Arg Asn Leu Thr Ile Leu Trp Leu His Ser
                80                  85                  90

Asn Val Leu Ala Arg Ile Asp Ala Ala Ala Phe Thr Gly Leu Ala
                95                  100                 105

Leu Leu Glu Gln Leu Asp Leu Ser Asp Asn Ala Gln Leu Arg Ser
                110                 115                 120

Val Asp Pro Ala Thr Phe His Gly Leu Gly Arg Leu His Thr Leu
                125                 130                 135

His Leu Asp Arg Cys Gly Leu Gln Glu Leu Gly Pro Gly Leu Phe
                140                 145                 150

Arg Gly Leu Ala Ala Leu Gln Tyr Leu Tyr Leu Gln Asp Asn Ala
                155                 160                 165

```
Leu Gln Ala Leu Pro Asp Asp Thr Phe Arg Asp Leu Gly Asn Leu
                170                 175                 180

Thr His Leu Phe Leu His Gly Asn Arg Ile Ser Ser Val Pro Glu
                185                 190                 195

Arg Ala Phe Arg Gly Leu His Ser Leu Asp Arg Leu Leu Leu His
                200                 205                 210

Gln Asn Arg Val Ala His Val His Pro His Ala Phe Arg Asp Leu
                215                 220                 225

Gly Arg Leu Met Thr Leu Tyr Leu Phe Ala Asn Asn Leu Ser Ala
                230                 235                 240

Leu Pro Thr Glu Ala Leu Ala Pro Leu Arg Ala Leu Gln Tyr Leu
                245                 250                 255

Arg Leu Asn Asp Asn Pro Trp Val Cys Asp Cys Arg Ala Arg Pro
                260                 265                 270

Leu Trp Ala Trp Leu Gln Lys Phe Arg Gly Ser Ser Ser Glu Val
                275                 280                 285

Pro Cys Ser Leu Pro Gln Arg Leu Ala Gly Arg Asp Leu Lys Arg
                290                 295                 300

Leu Ala Ala Asn Asp Leu Gln Gly Cys Ala Val Ala Thr Gly Pro
                305                 310                 315

Tyr His Pro Ile Trp Thr Gly Arg Ala Thr Asp Glu Glu Pro Leu
                320                 325                 330

Gly Leu Pro Lys Cys Cys Gln Pro Asp Ala Ala Asp Lys Ala Ser
                335                 340                 345

Val Leu Glu Pro Gly Arg Pro Ala Ser Ala Gly Asn Ala Leu Lys
                350                 355                 360

Gly Arg Val Pro Pro Gly Asp Ser Pro Pro Gly Asn Gly Ser Gly
                365                 370                 375

Pro Arg His Ile Asn Asp Ser Pro Phe Gly Thr Leu Pro Gly Ser
                380                 385                 390

Ala Glu Pro Pro Leu Thr Ala Val Arg Pro Glu Gly Ser Glu Pro
                395                 400                 405

Pro Gly Phe Pro Thr Ser Gly Pro Arg Arg Arg Pro Gly Cys Ser
                410                 415                 420

Arg Lys Asn Arg Thr Arg Ser His Cys Arg Leu Gly Gln Ala Gly
                425                 430                 435

Ser Gly Gly Gly Gly Thr Gly Asp Ser Glu Gly Ser Gly Ala Leu
                440                 445                 450

Pro Ser Leu Thr Cys Ser Leu Thr Pro Leu Gly Leu Ala Leu Val
                455                 460                 465

Leu Trp Thr Val Leu Gly Pro Cys
                470         473
```

<210> 128
<211> 24
<212> DNA
<213> Artificial Sequence

<220>
<223> Synthetic Oligonucleotide Probe

<400> 128
tggctgccct gcagtacctc tacc 24

<210> 129
<211> 24
<212> DNA
<213> Artificial Sequence

<220>
<223> Synthetic Oligonucleotide Probe

<400> 129
ccctgcaggt cattggcagc tagg 24

<210> 130
<211> 45
<212> DNA
<213> Artificial Sequence

<220>
<223> Synthetic Oligonucleotide Probe

<400> 130
aggcactgcc tgatgacacc ttccgcgacc tgggcaacct cacac 45

<210> 131
<211> 1829
<212> DNA
<213> Homo Sapien

<400> 131
gcgaggggag cgcggagccc ggcgcctaca gctcgccatg gtgcgccccc 50

tgaacccgcg accgctgccg cccgtagtcc tgatgttgct gctgctgctg 100

ccgccgtcgc cgctgcctct cgcagccgga gaccccttc ccacagaaag 150

ccgactcatg aacagctgtc tccaggccag gaggaagtgc caggctgatc 200

ccacctgcag tgctgcctac caccacctgg attcctgcac ctctagcata 250

agcaccccac tgccctcaga ggagccttcg gtccctgctg actgcctgga 300

ggcagcacag caactcagga acagctctct gataggctgc atgtgccacc 350

ggcgcatgaa gaaccaggtt gcctgcttgg acatctattg gaccgttcac 400

cgtgcccgca gccttggtaa ctatgagctg gatgtctccc cctatgaaga 450

cacagtgacc agcaaaccct ggaaaatgaa tctcagcaaa ctgaacatgc 500

tcaaaccaga ctcagacctc tgcctcaagt ttgccatgct gtgtactctc 550

aatgacaagt gtgaccggct gcgcaaggcc tacggggagg cgtgctccgg 600

gccccactgc cagcgccacg tctgcctcag gcagctgctc actttcttcg 650

agaaggccgc cgagccccac gcgcagggcc tgctactgtg cccatgtgcc 700

cccaacgacc ggggctgcgg ggagcgccgg cgcaacacca tcgcccccaa 750

ctgcgcgctg ccgcctgtgg cccccaactg cctggagctg cggcgcctct 800

gcttctccga cccgctttgc agatcacgcc tggtggattt ccagacccac 850

tgccatccca tggacatcct aggaacttgt gcaacagagc agtccagatg 900

tctacgagca tacctggggc tgattgggac tgccatgacc cccaactttg 950

tcagcaatgt caacaccagt gttgccttaa gctgcacctg ccgaggcagt 1000

ggcaacctgc aggaggagtg tgaaatgctg gaagggttct tctcccacaa 1050

cccctgcctc acggaggcca ttgcagctaa gatgcgtttt cacagccaac 1100

tcttctccca ggactggcca caccctacct ttgctgtgat ggcacaccag 1150

aatgaaaacc ctgctgtgag gccacagccc tgggtgccct ctcttttctc 1200

ctgcacgctt cccttgattc tgctcctgag cctatggtag ctggacttcc 1250

ccagggccct cttcccctcc accacaccca ggtggacttg cagcccacaa 1300

ggggtgagga aaggacagca gcaggaagga ggtgcagtgc gcagatgagg 1350

gcacaggaga agctaagggt tatgacctcc agatccttac tggtccagtc 1400

ctcattccct ccaccccatc tccacttctg attcatgctg cccctccttg 1450

gtggccacaa tttagccatg tcatctggtg gtgaccagct ccaccaagcc 1500

cctttctgag cccttcctct tgactaccag gatcaccaga atctaataag 1550

ttagcctttc tctattgcat tccagattag ggttagggta gggaggactg 1600

ggtgttctga ggcagcctag aaagtcattc tcctttgtga agaaggctcc 1650

tgccccctcg tctcctcctc tgagtggagg atggaaaact actgcctgca 1700

ctgccctgtc cccggatcct gccgaacatc tgggcatcag gagctggagc 1750

ctgtgggcct tgctttattc ctattattgt cctaaagtct ctctgggctc 1800

ttggatcatg attaaacctt tgacttaag 1829

<210> 132
<211> 400
<212> PRT
<213> Homo Sapien

<400> 132

```
Met Val Arg Pro Leu Asn Pro Arg Pro Leu Pro Pro Val Val Leu
  1               5                  10                  15

Met Leu Leu Leu Leu Leu Pro Pro Ser Pro Leu Pro Leu Ala Ala
                 20                  25                  30
```

Gly Asp Pro Leu Pro Thr Glu Ser Arg Leu Met Asn Ser Cys Leu
35 40 45

Gln Ala Arg Arg Lys Cys Gln Ala Asp Pro Thr Cys Ser Ala Ala
50 55 60

Tyr His His Leu Asp Ser Cys Thr Ser Ser Ile Ser Thr Pro Leu
65 70 75

Pro Ser Glu Glu Pro Ser Val Pro Ala Asp Cys Leu Glu Ala Ala
80 85 90

Gln Gln Leu Arg Asn Ser Ser Leu Ile Gly Cys Met Cys His Arg
95 100 105

Arg Met Lys Asn Gln Val Ala Cys Leu Asp Ile Tyr Trp Thr Val
110 115 120

His Arg Ala Arg Ser Leu Gly Asn Tyr Glu Leu Asp Val Ser Pro
125 130 135

Tyr Glu Asp Thr Val Thr Ser Lys Pro Trp Lys Met Asn Leu Ser
140 145 150

Lys Leu Asn Met Leu Lys Pro Asp Ser Asp Leu Cys Leu Lys Phe
155 160 165

Ala Met Leu Cys Thr Leu Asn Asp Lys Cys Asp Arg Leu Arg Lys
170 175 180

Ala Tyr Gly Glu Ala Cys Ser Gly Pro His Cys Gln Arg His Val
185 190 195

Cys Leu Arg Gln Leu Leu Thr Phe Phe Glu Lys Ala Ala Glu Pro
200 205 210

His Ala Gln Gly Leu Leu Leu Cys Pro Cys Ala Pro Asn Asp Arg
215 220 225

Gly Cys Gly Glu Arg Arg Arg Asn Thr Ile Ala Pro Asn Cys Ala
230 235 240

Leu Pro Pro Val Ala Pro Asn Cys Leu Glu Leu Arg Arg Leu Cys
245 250 255

Phe Ser Asp Pro Leu Cys Arg Ser Arg Leu Val Asp Phe Gln Thr
260 265 270

His Cys His Pro Met Asp Ile Leu Gly Thr Cys Ala Thr Glu Gln
275 280 285

Ser Arg Cys Leu Arg Ala Tyr Leu Gly Leu Ile Gly Thr Ala Met
290 295 300

Thr Pro Asn Phe Val Ser Asn Val Asn Thr Ser Val Ala Leu Ser
305 310 315

Cys Thr Cys Arg Gly Ser Gly Asn Leu Gln Glu Glu Cys Glu Met
320 325 330

Leu Glu Gly Phe Phe Ser His Asn Pro Cys Leu Thr Glu Ala Ile
335 340 345

Ala Ala Lys Met Arg Phe His Ser Gln Leu Phe Ser Gln Asp Trp
350 355 360

Pro His Pro Thr Phe Ala Val Met Ala His Gln Asn Glu Asn Pro
365 370 375

Ala Val Arg Pro Gln Pro Trp Val Pro Ser Leu Phe Ser Cys Thr
380 385 390

Leu Pro Leu Ile Leu Leu Leu Ser Leu Trp
395 400

<210> 133
<211> 20
<212> DNA
<213> Artificial Sequence

<220>
<223> Synthetic Oligonucleotide Probe

<400> 133
gcctctcgca gccggagacc 20

<210> 134
<211> 21
<212> DNA
<213> Artificial Sequence

<220>
<223> Synthetic Oligonucleotide Probe

<400> 134
caggtgggat cagcctggca c 21

<210> 135
<211> 41
<212> DNA
<213> Artificial Sequence

<220>
<223> Synthetic Oligonucleotide Probe

<400> 135
tctcgcagcc ggagaccccc ttcccacaga aagccgactc a 41

<210> 136
<211> 2849
<212> DNA
<213> Homo Sapien

<220>
<221> unsure
<222> 2715
<223> unknown base

<400> 136
cggacgcgtg ggcggacgcg tgggcggacg cgtgggcgga cgcgtgggct 50

ggttcaggtc caggttttgc tttgatcctt ttcaaaaact ggagacacag 100

aagagggctc taggaaaaag ttttggatgg gattatgtgg aaactaccct 150

gcgattctct gctgccagag caggctcggc gcttccaccc cagtgcagcc 200

ttcccctggc ggtggtgaaa gagactcggg agtcgctgct tccaaagtgc 250
ccgccgtgag tgagctctca ccccagtcag ccaaatgagc ctcttcgggc 300
ttctcctgct gacatctgcc ctggccggcc agagacaggg gactcaggcg 350
gaatccaacc tgagtagtaa attccagttt tccagcaaca aggaacagaa 400
cggagtacaa gatcctcagc atgagagaat tattactgtg tctactaatg 450
gaagtattca cagcccaagg tttcctcata cttatccaag aaatacggtc 500
ttggtatgga gattagtagc agtagaggaa aatgtatgga tacaacttac 550
gtttgatgaa agatttgggc ttgaagaccc agaagatgac atatgcaagt 600
atgattttgt agaagttgag gaacccagtg atggaactat attagggcgc 650
tggtgtggtt ctggtactgt accaggaaaa cagatttcta aaggaaatca 700
aattaggata agatttgtat ctgatgaata ttttccttct gaaccagggt 750
tctgcatcca ctacaacatt gtcatgccac aattcacaga agctgtgagt 800
ccttcagtgc taccccttc agctttgcca ctggacctgc ttaataatgc 850
tataactgcc tttagtacct tggaagacct tattcgatat cttgaaccag 900
agagatggca gttggactta gaagatctat ataggccaac ttggcaactt 950
cttggcaagg cttttgtttt tggaagaaaa tccagagtgg tggatctgaa 1000
ccttctaaca gaggaggtaa gattatacag ctgcacacct cgtaacttct 1050
cagtgtccat aagggaagaa ctaaagagaa ccgataccat tttctggcca 1100
ggttgtctcc tggttaaacg ctgtggtggg aactgtgcct gttgtctcca 1150
caattgcaat gaatgtcaat gtgtcccaag caaagttact aaaaaatacc 1200
acgaggtcct tcagttgaga ccaaagaccg gtgtcagggg attgcacaaa 1250
tcactcaccg acgtggccct ggagcaccat gaggagtgtg actgtgtgtg 1300
cagagggagc acaggaggat agccgcatca ccaccagcag ctcttgccca 1350
gagctgtgca gtgcagtggc tgattctatt agagaacgta tgcgttatct 1400
ccatccttaa tctcagttgt ttgcttcaag gacctttcat cttcaggatt 1450
tacagtgcat tctgaaagag gagacatcaa acagaattag gagttgtgca 1500
acagctcttt tgagaggagg cctaaaggac aggagaaaag gtcttcaatc 1550
gtggaaagaa aattaaatgt tgtattaaat agatcaccag ctagtttcag 1600
agttaccatg tacgtattcc actagctggg ttctgtattt cagttctttc 1650
gatacggctt agggtaatgt cagtacagga aaaaaactgt gcaagtgagc 1700
acctgattcc gttgccttgc ttaactctaa agctccatgt cctgggccta 1750
aaatcgtata aaatctggat tttttttttt ttttttgctc atattcacat 1800

atgtaaacca gaacattcta tgtactacaa acctggtttt taaaaaggaa 1850
ctatgttgct atgaattaaa cttgtgtcat gctgatagga cagactggat 1900
ttttcatatt tcttattaaa atttctgcca tttagaagaa gagaactaca 1950
ttcatggttt ggaagagata aacctgaaaa gaagagtggc cttatcttca 2000
ctttatcgat aagtcagttt atttgtttca ttgtgtacat ttttatattc 2050
tccttttgac attataactg ttggcttttc taatcttgtt aaatatatct 2100
atttttacca aaggtattta atattctttt ttatgacaac ttagatcaac 2150
tatttttagc ttggtaaatt tttctaaaca caattgttat agccagagga 2200
acaaagatga tataaaatat tgttgctctg acaaaaatac atgtatttca 2250
ttctcgtatg gtgctagagt tagattaatc tgcattttaa aaaactgaat 2300
tggaatagaa ttggtaagtt gcaaagactt tttgaaaata attaaattat 2350
catatcttcc attcctgtta ttggagatga aaataaaaag caacttatga 2400
aagtagacat tcagatccag ccattactaa cctattcctt ttttggggaa 2450
atctgagcct agctcagaaa aacataaagc accttgaaaa agacttggca 2500
gcttcctgat aaagcgtgct gtgctgtgca gtaggaacac atcctattta 2550
ttgtgatgtt gtggttttat tatcttaaac tctgttccat acacttgtat 2600
aaatacatgg atatttttat gtacagaagt atgtctctta accagttcac 2650
ttattgtact ctggcaattt aaaagaaaat cagtaaaata ttttgcttgt 2700
aaaatgctta atatngtgcc taggttatgt ggtgactatt tgaatcaaaa 2750
atgtattgaa tcatcaaata aaagaatgtg gctattttgg ggagaaaatt 2800
aaaaaaaaaa aaaaaaaaaa aggtttaggg ataacagggt aatgcggcc 2849

<210> 137
<211> 345
<212> PRT
<213> Homo Sapien

<400> 137
Met Ser Leu Phe Gly Leu Leu Leu Leu Thr Ser Ala Leu Ala Gly
1 5 10 15

Gln Arg Gln Gly Thr Gln Ala Glu Ser Asn Leu Ser Ser Lys Phe
20 25 30

Gln Phe Ser Ser Asn Lys Glu Gln Asn Gly Val Gln Asp Pro Gln
35 40 45

His Glu Arg Ile Ile Thr Val Ser Thr Asn Gly Ser Ile His Ser
50 55 60

Pro Arg Phe Pro His Thr Tyr Pro Arg Asn Thr Val Leu Val Trp
65 70 75

```
Arg Leu Val Ala Val Glu Glu Asn Val Trp Ile Gln Leu Thr Phe
                80                  85                  90

Asp Glu Arg Phe Gly Leu Glu Asp Pro Glu Asp Asp Ile Cys Lys
                95                  100                 105

Tyr Asp Phe Val Glu Val Glu Glu Pro Ser Asp Gly Thr Ile Leu
                110                 115                 120

Gly Arg Trp Cys Gly Ser Gly Thr Val Pro Gly Lys Gln Ile Ser
                125                 130                 135

Lys Gly Asn Gln Ile Arg Ile Arg Phe Val Ser Asp Glu Tyr Phe
                140                 145                 150

Pro Ser Glu Pro Gly Phe Cys Ile His Tyr Asn Ile Val Met Pro
                155                 160                 165

Gln Phe Thr Glu Ala Val Ser Pro Ser Val Leu Pro Pro Ser Ala
                170                 175                 180

Leu Pro Leu Asp Leu Leu Asn Asn Ala Ile Thr Ala Phe Ser Thr
                185                 190                 195

Leu Glu Asp Leu Ile Arg Tyr Leu Glu Pro Glu Arg Trp Gln Leu
                200                 205                 210

Asp Leu Glu Asp Leu Tyr Arg Pro Thr Trp Gln Leu Leu Gly Lys
                215                 220                 225

Ala Phe Val Phe Gly Arg Lys Ser Arg Val Val Asp Leu Asn Leu
                230                 235                 240

Leu Thr Glu Glu Val Arg Leu Tyr Ser Cys Thr Pro Arg Asn Phe
                245                 250                 255

Ser Val Ser Ile Arg Glu Glu Leu Lys Arg Thr Asp Thr Ile Phe
                260                 265                 270

Trp Pro Gly Cys Leu Leu Val Lys Arg Cys Gly Gly Asn Cys Ala
                275                 280                 285

Cys Cys Leu His Asn Cys Asn Glu Cys Gln Cys Val Pro Ser Lys
                290                 295                 300

Val Thr Lys Lys Tyr His Glu Val Leu Gln Leu Arg Pro Lys Thr
                305                 310                 315

Gly Val Arg Gly Leu His Lys Ser Leu Thr Asp Val Ala Leu Glu
                320                 325                 330

His His Glu Glu Cys Asp Cys Val Cys Arg Gly Ser Thr Gly Gly
                335                 340                 345
```

<210> 138
<211> 21
<212> DNA
<213> Artificial Sequence

<220>
<223> Synthetic Oligonucleotide Probe

<400> 138

acttctcagt gtccataagg g 21

<210> 139
<211> 40
<212> DNA
<213> Artificial Sequence

<220>
<223> Synthetic Oligonucleotide Probe

<400> 139
gaactaaaga gaaccgatac cattttctgg ccaggttgtc 40

<210> 140
<211> 20
<212> DNA
<213> Artificial Sequence

<220>
<223> Synthetic Oligonucleotide Probe

<400> 140
caccacagcg tttaaccagg 20

<210> 141
<211> 20
<212> DNA
<213> Artificial Sequence

<220>
<223> Synthetic Oligonucleotide Probe

<400> 141
acaacaggca cagttcccac 20

<210> 142
<211> 1510
<212> DNA
<213> Homo Sapien

<400> 142
ggacgagggc agatctcgtt ctggggcaag ccgttgacac tcgctccctg 50

ccaccgcccg ggctccgtgc cgccaagttt tcattttcca ccttctctgc 100

ctccagtccc ccagcccctg gccgagagaa gggtcttacc ggccgggatt 150

gctggaaaca ccaagaggtg gtttttgttt tttaaaactt ctgtttcttg 200

ggagggggtg tggcggggca ggatgagcaa ctccgttcct ctgctctgtt 250

tctggagcct ctgctattgc tttgctgcgg ggagccccgt accttttggt 300

ccagagggac ggctggaaga taagctccac aaacccaaag ctacacagac 350

tgaggtcaaa ccatctgtga ggtttaacct ccgcacctcc aaggacccag 400

agcatgaagg atgctacctc tccgtcggcc acagccagcc cttagaagac 450

tgcagtttca acatgacagc taaaaccttt ttcatcattc acggatggac 500

gatgagcggt atctttgaaa actggctgca caaactcgtg tcagccctgc 550

acacaagaga gaaagacgcc aatgtagttg tggttgactg gctccccctg 600

gcccaccagc tttacacgga tgcggtcaat aataccaggg tggtgggaca 650

cagcattgcc aggatgctcg actggctgca ggagaaggac gatttttctc 700

tcgggaatgt ccacttgatc ggctacagcc tcggagcgca cgtggccggg 750

tatgcaggca acttcgtgaa aggaacggtg ggccgaatca caggtttgga 800

tcctgccggg cccatgtttg aaggggccga catccacaag aggctctctc 850

cggacgatgc agattttgtg gatgtcctcc acacctacac gcgttccttc 900

ggcttgagca ttggtattca gatgcctgtg ggccacattg acatctaccc 950

caatgggggt gacttccagc caggctgtgg actcaacgat gtcttgggat 1000

caattgcata tggaacaatc acagaggtgg taaaatgtga gcatgagcga 1050

gccgtccacc tctttgttga ctctctggtg aatcaggaca agccgagttt 1100

tgccttccag tgcactgact ccaatcgctt caaaaagggg atctgtctga 1150

gctgccgcaa gaaccgttgt aatagcattg gctacaatgc caagaaaatg 1200

aggaacaaga ggaacagcaa aatgtaccta aaaacccggg caggcatgcc 1250

tttcagaggt aaccttcagt ccctggagtg tccctgagga aggcccttaa 1300

tacctccttc ttaataccat gctgcagagc agggcacatc ctagcccagg 1350

agaagtggcc agcacaatcc aatcaaatcg ttgcaaatca gattacactg 1400

tgcatgtcct aggaaaggga atctttacaa aataaacagt gtggacccct 1450

aataaaaaaa aaaaaaaaaa aaaaaaaaaa aaaaaaaaaa aaaaaaaaaa 1500

aaaaaaaaaa 1510

<210> 143
<211> 354
<212> PRT
<213> Homo Sapien

<400> 143

```
Met Ser Asn Ser Val Pro Leu Leu Cys Phe Trp Ser Leu Cys Tyr
  1               5                  10                  15

Cys Phe Ala Ala Gly Ser Pro Val Pro Phe Gly Pro Glu Gly Arg
                 20                  25                  30

Leu Glu Asp Lys Leu His Lys Pro Lys Ala Thr Gln Thr Glu Val
                 35                  40                  45

Lys Pro Ser Val Arg Phe Asn Leu Arg Thr Ser Lys Asp Pro Glu
                 50                  55                  60

His Glu Gly Cys Tyr Leu Ser Val Gly His Ser Gln Pro Leu Glu
                 65                  70                  75

Asp Cys Ser Phe Asn Met Thr Ala Lys Thr Phe Phe Ile Ile His
                 80                  85                  90
```

```
Gly Trp Thr Met Ser Gly Ile Phe Glu Asn Trp Leu His Lys Leu
                 95                 100                 105

Val Ser Ala Leu His Thr Arg Glu Lys Asp Ala Asn Val Val Val
                110                 115                 120

Val Asp Trp Leu Pro Leu Ala His Gln Leu Tyr Thr Asp Ala Val
                125                 130                 135

Asn Asn Thr Arg Val Val Gly His Ser Ile Ala Arg Met Leu Asp
                140                 145                 150

Trp Leu Gln Glu Lys Asp Asp Phe Ser Leu Gly Asn Val His Leu
                155                 160                 165

Ile Gly Tyr Ser Leu Gly Ala His Val Ala Gly Tyr Ala Gly Asn
                170                 175                 180

Phe Val Lys Gly Thr Val Gly Arg Ile Thr Gly Leu Asp Pro Ala
                185                 190                 195

Gly Pro Met Phe Glu Gly Ala Asp Ile His Lys Arg Leu Ser Pro
                200                 205                 210

Asp Asp Ala Asp Phe Val Asp Val Leu His Thr Tyr Thr Arg Ser
                215                 220                 225

Phe Gly Leu Ser Ile Gly Ile Gln Met Pro Val Gly His Ile Asp
                230                 235                 240

Ile Tyr Pro Asn Gly Gly Asp Phe Gln Pro Gly Cys Gly Leu Asn
                245                 250                 255

Asp Val Leu Gly Ser Ile Ala Tyr Gly Thr Ile Thr Glu Val Val
                260                 265                 270

Lys Cys Glu His Glu Arg Ala Val His Leu Phe Val Asp Ser Leu
                275                 280                 285

Val Asn Gln Asp Lys Pro Ser Phe Ala Phe Gln Cys Thr Asp Ser
                290                 295                 300

Asn Arg Phe Lys Lys Gly Ile Cys Leu Ser Cys Arg Lys Asn Arg
                305                 310                 315

Cys Asn Ser Ile Gly Tyr Asn Ala Lys Lys Met Arg Asn Lys Arg
                320                 325                 330

Asn Ser Lys Met Tyr Leu Lys Thr Arg Ala Gly Met Pro Phe Arg
                335                 340                 345

Gly Asn Leu Gln Ser Leu Glu Cys Pro
                350             354
```

<210> 144
<211> 23
<212> DNA
<213> Artificial Sequence

<220>
<223> Synthetic Oligonucleotide Probe

<400> 144

gtgagcatga gcgagccgtc cac 23

<210> 145
<211> 26
<212> DNA
<213> Artificial Sequence

<220>
<223> Synthetic Oligonucleotide Probe

<400> 145
gctattacaa cggttcttgc ggcagc 26

<210> 146
<211> 44
<212> DNA
<213> Artificial Sequence

<220>
<223> Synthetic Oligonucleotide Probe

<400> 146
ttgactctct ggtgaatcag gacaagccga gttttgcctt ccag 44

<210> 147
<211> 1964
<212> DNA
<213> Homo Sapien

<400> 147
cgcgccgggc gcagggagct gagtggacgg ctcgagacgg cggcgcgtgc 50

agcagctcca gaaagcagcg agttggcaga gcagggctgc atttccagca 100

ggagctgcga gcacagtgct ggctcacaac aagatgctca aggtgtcagc 150

cgtactgtgt gtgtgtgcag ccgcttggtg cagtcagtct ctcgcagctg 200

ccgcggcggt ggctgcagcc ggggggcggt cggacggcgg taattttctg 250

gatgataaac aatggctcac cacaatctct cagtatgaca aggaagtcgg 300

acagtggaac aaattccgag acgaagtaga ggatgattat ttccgcactt 350

ggagtccagg aaaacccttc gatcaggctt tagatccagc taaggatcca 400

tgcttaaaga tgaaatgtag tcgccataaa gtatgcattg ctcaagattc 450

tcagactgca gtctgcatta gtcaccggag gcttacacac aggatgaaag 500

aagcaggagt agaccatagg cagtggaggg gtcccatatt atccacctgc 550

aagcagtgcc cagtggtcta tcccagccct gtttgtggtt cagatggtca 600

tacctactct tttcagtgca aactagaata tcaggcatgt gtcttaggaa 650

aacagatctc agtcaaatgt gaaggacatt gcccatgtcc ttcagataag 700

cccaccagta caagcagaaa tgttaagaga gcatgcagtg acctggagtt 750

cagggaagtg gcaaacagat tgcgggactg gttcaaggcc cttcatgaaa 800

gtggaagtca aaacaagaag acaaaaacat tgctgaggcc tgagagaagc 850

agattcgata ccagcatctt gccaatttgc aaggactcac ttggctggat 900

gtttaacaga cttgatacaa actatgacct gctattggac cagtcagagc 950

tcagaagcat ttaccttgat aagaatgaac agtgtaccaa ggcattcttc 1000

aattcttgtg acacatacaa ggacagttta atatctaata atgagtggtg 1050

ctactgcttc cagagacagc aagacccacc ttgccagact gagctcagca 1100

atattcagaa gcggcaaggg gtaaagaagc tcctaggaca gtatatcccc 1150

ctgtgtgatg aagatggtta ctacaagcca acacaatgtc atggcagtgt 1200

tggacagtgc tggtgtgttg acagatatgg aaatgaagtc atgggatcca 1250

gaataaatgg tgttgcagat tgtgctatag attttgagat ctccggagat 1300

tttgctagtg gcgattttca tgaatggact gatgatgagg atgatgaaga 1350

cgatattatg aatgatgaag atgaaattga agatgatgat gaagatgaag 1400

gggatgatga tgatggtggt gatgaccatg atgtatacat ttgattgatg 1450

acagttgaaa tcaataaatt ctacatttct aatatttaca aaaatgatag 1500

cctatttaaa attatcttct tccccaataa caaaatgatt ctaaacctca 1550

catatatttt gtataattat ttgaaaaatt gcagctaaag ttatagaact 1600

ttatgtttaa ataagaatca tttgctttga gtttttatat tccttacaca 1650

aaaagaaaat acatatgcag tctagtcaga caaaataaag ttttgaagtg 1700

ctactataat aaatttttca cgagaacaaa ctttgtaaat cttccataag 1750

caaaatgaca gctagtgctt gggatcgtac atgttaattt tttgaaagat 1800

aattctaagt gaaatttaaa ataaataaat ttttaatgac ctgggtctta 1850

aggatttagg aaaaatatgc atgctttaat tgcatttcca aagtagcatc 1900

ttgctagacc tagatgagtc aggataacag agagatacca catgactcca 1950

aaaaaaaaaa aaaa 1964

<210> 148
<211> 436
<212> PRT
<213> Homo Sapien

<400> 148
Met Leu Lys Val Ser Ala Val Leu Cys Val Cys Ala Ala Ala Trp
1               5                   10                  15

Cys Ser Gln Ser Leu Ala Ala Ala Ala Ala Val Ala Ala Ala Gly
                20                  25                  30

Gly Arg Ser Asp Gly Gly Asn Phe Leu Asp Asp Lys Gln Trp Leu
                35                  40                  45

Thr Thr Ile Ser Gln Tyr Asp Lys Glu Val Gly Gln Trp Asn Lys
                50                  55                  60

```
Phe Arg Asp Glu Val Glu Asp Asp Tyr Phe Arg Thr Trp Ser Pro
                65                  70                  75

Gly Lys Pro Phe Asp Gln Ala Leu Asp Pro Ala Lys Asp Pro Cys
                80                  85                  90

Leu Lys Met Lys Cys Ser Arg His Lys Val Cys Ile Ala Gln Asp
                95                  100                 105

Ser Gln Thr Ala Val Cys Ile Ser His Arg Arg Leu Thr His Arg
                110                 115                 120

Met Lys Glu Ala Gly Val Asp His Arg Gln Trp Arg Gly Pro Ile
                125                 130                 135

Leu Ser Thr Cys Lys Gln Cys Pro Val Val Tyr Pro Ser Pro Val
                140                 145                 150

Cys Gly Ser Asp Gly His Thr Tyr Ser Phe Gln Cys Lys Leu Glu
                155                 160                 165

Tyr Gln Ala Cys Val Leu Gly Lys Gln Ile Ser Val Lys Cys Glu
                170                 175                 180

Gly His Cys Pro Cys Pro Ser Asp Lys Pro Thr Ser Thr Ser Arg
                185                 190                 195

Asn Val Lys Arg Ala Cys Ser Asp Leu Glu Phe Arg Glu Val Ala
                200                 205                 210

Asn Arg Leu Arg Asp Trp Phe Lys Ala Leu His Glu Ser Gly Ser
                215                 220                 225

Gln Asn Lys Lys Thr Lys Thr Leu Leu Arg Pro Glu Arg Ser Arg
                230                 235                 240

Phe Asp Thr Ser Ile Leu Pro Ile Cys Lys Asp Ser Leu Gly Trp
                245                 250                 255

Met Phe Asn Arg Leu Asp Thr Asn Tyr Asp Leu Leu Leu Asp Gln
                260                 265                 270

Ser Glu Leu Arg Ser Ile Tyr Leu Asp Lys Asn Glu Gln Cys Thr
                275                 280                 285

Lys Ala Phe Phe Asn Ser Cys Asp Thr Tyr Lys Asp Ser Leu Ile
                290                 295                 300

Ser Asn Asn Glu Trp Cys Tyr Cys Phe Gln Arg Gln Gln Asp Pro
                305                 310                 315

Pro Cys Gln Thr Glu Leu Ser Asn Ile Gln Lys Arg Gln Gly Val
                320                 325                 330

Lys Lys Leu Leu Gly Gln Tyr Ile Pro Leu Cys Asp Glu Asp Gly
                335                 340                 345

Tyr Tyr Lys Pro Thr Gln Cys His Gly Ser Val Gly Gln Cys Trp
                350                 355                 360

Cys Val Asp Arg Tyr Gly Asn Glu Val Met Gly Ser Arg Ile Asn
                365                 370                 375
```

Gly Val Ala Asp Cys Ala Ile Asp Phe Glu Ile Ser Gly Asp Phe
380 385 390

Ala Ser Gly Asp Phe His Glu Trp Thr Asp Asp Glu Asp Asp Glu
395 400 405

Asp Asp Ile Met Asn Asp Glu Asp Glu Ile Glu Asp Asp Asp Glu
410 415 420

Asp Glu Gly Asp Asp Asp Asp Gly Gly Asp Asp His Asp Val Tyr
425 430 435

Ile
436

<210> 149
<211> 25
<212> DNA
<213> Artificial Sequence

<220>
<223> Synthetic Oligonucleotide Probe

<400> 149
cagcaatatt cagaagcggc aaggg 25

<210> 150
<211> 28
<212> DNA
<213> Artificial Sequence

<220>
<223> Synthetic Oligonucleotide Probe

<400> 150
catcatggtc atcaccacca tcatcatc 28

<210> 151
<211> 48
<212> DNA
<213> Artificial Sequence

<220>
<223> Synthethic Oligonucleotide Probe

<400> 151
ggttactaca agccaacaca atgtcatggc agtgttggac agtgctgg 48

<210> 152
<211> 550
<212> DNA
<213> Homo Sapien

<400> 152
ccagtctgtc gccacctcac ttggtgtctg ctgtccccgc caggcaagcc 50

tggggtgaga gcacagagga gtgggccggg accatgcggg ggacgcggct 100

ggcgctcctg gcgctggtgc tggctgcctg cggagagctg gcgccggccc 150

tgcgctgcta cgtctgtccg gagcccacag gagtgtcgga ctgtgtcacc 200

atcgccacct gcaccaccaa cgaaaccatg tgcaagacca cactctactc 250

ccgggagata gtgtacccct tccaggggga ctccacggtg accaagtcct 300

gtgccagcaa gtgtaagccc tcggatgtgg atggcatcgg ccagaccctg 350

cccgtgtcct gctgcaatac tgagctgtgc aatgtagacg gggcgcccgc 400

tctgaacagc ctccactgcg gggccctcac gctcctccca ctcttgagcc 450

tccgactgta gagtccccgc ccaccccccat ggccctatgc ggcccagccc 500

cgaatgcctt gaagaagtgc cccctgcacc aggaaaaaaa aaaaaaaaaa 550

<210> 153
<211> 125
<212> PRT
<213> Homo Sapien

<400> 153
Met Arg Gly Thr Arg Leu Ala Leu Leu Ala Leu Val Leu Ala Ala
1               5                   10                  15

Cys Gly Glu Leu Ala Pro Ala Leu Arg Cys Tyr Val Cys Pro Glu
                20                  25                  30

Pro Thr Gly Val Ser Asp Cys Val Thr Ile Ala Thr Cys Thr Thr
                35                  40                  45

Asn Glu Thr Met Cys Lys Thr Thr Leu Tyr Ser Arg Glu Ile Val
                50                  55                  60

Tyr Pro Phe Gln Gly Asp Ser Thr Val Thr Lys Ser Cys Ala Ser
                65                  70                  75

Lys Cys Lys Pro Ser Asp Val Asp Gly Ile Gly Gln Thr Leu Pro
                80                  85                  90

Val Ser Cys Cys Asn Thr Glu Leu Cys Asn Val Asp Gly Ala Pro
                95                  100                 105

Ala Leu Asn Ser Leu His Cys Gly Ala Leu Thr Leu Leu Pro Leu
                110                 115                 120

Leu Ser Leu Arg Leu
                125

<210> 154
<211> 1355
<212> DNA
<213> Homo Sapien

<400> 154
cggacgcgtg ggcggacgcg tgggcggacg cgtgggcgga cgcgtgggct 50

gggtgcctgc atcgccatgg acaccaccag gtacagcaag tggggcggca 100

gctccgagga ggtccccgga gggccctggg gacgctgggt gcactggagc 150

aggagacccc tcttcttggc cctggctgtc ctggtcacca cagtcctttg 200

ggctgtgatt ctgagtatcc tattgtccaa ggcctccacg gagcgcgcgg 250

cgctgcttga cggccacgac ctgctgagga caaacgcctc gaagcagacg 300

gcggcgctgg gtgccctgaa ggaggaggtc ggagactgcc acagctgctg 350
ctcggggacg caggcgcagc tgcagaccac gcgcgcggag cttggggagg 400
cgcaggcgaa gctgatggag caggagagcg ccctgcggga actgcgtgag 450
cgcgtgaccc agggcttggc tgaagccggc aggggccgtg aggacgtccg 500
cactgagctg ttccgggcgc tggaggccgt gaggctccag aacaactcct 550
gcgagccgtg ccccacgtcg tggctgtcct tcgagggctc ctgctacttt 600
ttctctgtgc caaagacgac gtgggcggcg gcgcaggatc actgcgcaga 650
tgccagcgcg cacctggtga tcgttggggg cctggatgag cagggcttcc 700
tcactcggaa cacgcgtggc cgtggttact ggctgggcct gagggctgtg 750
cgccatctgg gcaaggttca gggctaccag tgggtggacg gagtctctct 800
cagcttcagc cactggaacc agggagagcc caatgacgct tgggggcgcg 850
agaactgtgt catgatgctg cacacggggc tgtggaacga cgcaccgtgt 900
gacagcgaga aggacggctg gatctgtgag aaaaggcaca actgctgacc 950
ccgcccagtg ccctggagcc gcgcccattg cagcatgtcg tatcctgggg 1000
gctgctcacc tccctggctc ctggagctga ttgccaaaga gttttttct 1050
tcctcatcca ccgctgctga gtctcagaaa cacttggccc aacatagccc 1100
tgtccagccc agtgcctggg ctctgggacc tccatgccga cctcatccta 1150
actccactca cgcagaccca acctaacctc cactagctcc aaaatccctg 1200
ctcctgcgtc cccgtgatat gcctccactt ctctccctaa ccaaggttag 1250
gtgactgagg actggagctg tttggttttc tcgcattttc caccaaactg 1300
gaagctgttt ttgcagcctg aggaagcatc aataaatatt tgagaaatga 1350
aaaaa 1355

<210> 155
<211> 293
<212> PRT
<213> Homo Sapien

<400> 155
Met Asp Thr Thr Arg Tyr Ser Lys Trp Gly Gly Ser Ser Glu Glu
1 5 10 15

Val Pro Gly Gly Pro Trp Gly Arg Trp Val His Trp Ser Arg Arg
20 25 30

Pro Leu Phe Leu Ala Leu Ala Val Leu Val Thr Thr Val Leu Trp
35 40 45

Ala Val Ile Leu Ser Ile Leu Leu Ser Lys Ala Ser Thr Glu Arg
50 55 60

```
Ala Ala Leu Leu Asp Gly His Asp Leu Leu Arg Thr Asn Ala Ser
                65                  70                  75

Lys Gln Thr Ala Ala Leu Gly Ala Leu Lys Glu Glu Val Gly Asp
                80                  85                  90

Cys His Ser Cys Cys Ser Gly Thr Gln Ala Gln Leu Gln Thr Thr
                95                  100                 105

Arg Ala Glu Leu Gly Glu Ala Gln Ala Lys Leu Met Glu Gln Glu
                110                 115                 120

Ser Ala Leu Arg Glu Leu Arg Glu Arg Val Thr Gln Gly Leu Ala
                125                 130                 135

Glu Ala Gly Arg Gly Arg Glu Asp Val Arg Thr Glu Leu Phe Arg
                140                 145                 150

Ala Leu Glu Ala Val Arg Leu Gln Asn Asn Ser Cys Glu Pro Cys
                155                 160                 165

Pro Thr Ser Trp Leu Ser Phe Glu Gly Ser Cys Tyr Phe Phe Ser
                170                 175                 180

Val Pro Lys Thr Thr Trp Ala Ala Ala Gln Asp His Cys Ala Asp
                185                 190                 195

Ala Ser Ala His Leu Val Ile Val Gly Gly Leu Asp Glu Gln Gly
                200                 205                 210

Phe Leu Thr Arg Asn Thr Arg Gly Arg Gly Tyr Trp Leu Gly Leu
                215                 220                 225

Arg Ala Val Arg His Leu Gly Lys Val Gln Gly Tyr Gln Trp Val
                230                 235                 240

Asp Gly Val Ser Leu Ser Phe Ser His Trp Asn Gln Gly Glu Pro
                245                 250                 255

Asn Asp Ala Trp Gly Arg Glu Asn Cys Val Met Met Leu His Thr
                260                 265                 270

Gly Leu Trp Asn Asp Ala Pro Cys Asp Ser Glu Lys Asp Gly Trp
                275                 280                 285

Ile Cys Glu Lys Arg His Asn Cys
                290         293
```

<210> 156
<211> 24
<212> DNA
<213> Artificial Sequence

<220>
<223> Synthetic Oligonucleotide Probe

<400> 156
gcgagaactg tgtcatgatg ctgc 24

<210> 157
<211> 24
<212> DNA
<213> Artificial Sequence

<220>
<223> Synthetic Oligonucleotide Probe

<400> 157
gtttctgaga ctcagcagcg gtgg 24

<210> 158
<211> 50
<212> DNA
<213> Artificial Sequence

<220>
<223> Synthetic Oligonucleotide Probe

<400> 158
caccgtgtga cagcgagaag gacggctgga tctgtgagaa aaggcacaac 50

<210> 159
<211> 434
<212> DNA
<213> Homo Sapien

<400> 159
gtcgaatcca aatcactcat tgtgaaagct gagctcacag ccgaataagc 50

caccatgagg ctgtcagtgt gtctcctgat ggtctcgctg gccctttgct 100

gctaccaggc ccatgctctt gtctgcccag ctgttgcttc tgagatcaca 150

gtcttcttat tcttaagtga cgctgcggta aacctccaag ttgccaaact 200

taatccacct ccagaagctc ttgcagccaa gttggaagtg aagcactgca 250

ccgatcagat atcttttaag aaacgactct cattgaaaaa gtcctggtgg 300

aaatagtgaa aaaatgtggt gtgtgacatg taaaaatgct caacctggtt 350

tccaaagtct ttcaacgaca ccctgatctt cactaaaaat tgtaaaggtt 400

tcaacacgtt gctttaataa atcacttgcc ctgc 434

<210> 160
<211> 83
<212> PRT
<213> Homo Sapien

<400> 160
Met Arg Leu Ser Val Cys Leu Leu Met Val Ser Leu Ala Leu Cys
1 5 10 15

Cys Tyr Gln Ala His Ala Leu Val Cys Pro Ala Val Ala Ser Glu
20 25 30

Ile Thr Val Phe Leu Phe Leu Ser Asp Ala Ala Val Asn Leu Gln
35 40 45

Val Ala Lys Leu Asn Pro Pro Pro Glu Ala Leu Ala Ala Lys Leu
50 55 60

Glu Val Lys His Cys Thr Asp Gln Ile Ser Phe Lys Lys Arg Leu
65 70 75

```
Ser Leu Lys Lys Ser Trp Trp Lys
                 80          83

<210> 161
<211> 1885
<212> DNA
<213> Homo Sapien

<400> 161
gcgaggtggc gatcgctgag aggcaggagg gccgaggcgg gcctgggagg 50
cggcccggag gtggggcgcc gctggggccg gcccgcacgg gcttcatctg 100
agggcgcacg gcccgcgacc gagcgtgcgg actggcctcc caagcgtggg 150
gcgacaagct gccggagctg caatgggccg cggctgggga ttcttgtttg 200
gcctcctggg cgccgtgtgg ctgctcagct cgggccacgg agaggagcag 250
cccccggaga cagcggcaca gaggtgcttc tgccaggtta gtggttactt 300
ggatgattgt acctgtgatg ttgaaaccat tgatagattt aataactaca 350
ggcttttccc aagactacaa aaacttcttg aaagtgacta ctttaggtat 400
tacaaggtaa acctgaagag gccgtgtcct ttctggaatg acatcagcca 450
gtgtggaaga agggactgtg ctgtcaaacc atgtcaatct gatgaagttc 500
ctgatggaat taaatctgcg agctacaagt attctgaaga agccaataat 550
ctcattgaag aatgtgaaca agctgaacga cttggagcag tggatgaatc 600
tctgagtgag gaaacacaga aggctgttct tcagtggacc aagcatgatg 650
attcttcaga taacttctgt gaagctgatg acattcagtc ccctgaagct 700
gaatatgtag atttgcttct taatcctgag cgctacactg gttacaaggg 750
accagatgct tggaaaatat ggaatgtcat ctacgaagaa aactgtttta 800
agccacagac aattaaaaga cctttaaatc ctttggcttc tggtcaaggg 850
acaagtgaag agaacacttt ttacagttgg ctagaaggtc tctgtgtaga 900
aaaaagagca ttctacagac ttatatctgg cctacatgca agcattaatg 950
tgcatttgag tgcaagatat cttttacaag agacctggtt agaaaagaaa 1000
tggggacaca acattacaga atttcaacag cgatttgatg gaattttgac 1050
tgaaggagaa ggtccaagaa ggcttaagaa cttgtatttt ctctacttaa 1100
tagaactaag ggctttatcc aaagtgttac cattcttcga gcgcccagat 1150
tttcaactct ttactggaaa taaaattcag gatgaggaaa acaaaatgtt 1200
acttctggaa atacttcatg aaatcaagtc atttcctttg cattttgatg 1250
agaattcatt ttttgctggg gataaaaaag aagcacacaa actaaaggag 1300
gactttcgac tgcattttag aaatatttca agaattatgg attgtgttgg 1350
```

ttgttttaaa tgtcgtctgt ggggaaagct tcagactcag ggtttgggca 1400

ctgctctgaa gatcttattt tctgagaaat tgatagcaaa tatgccagaa 1450

agtggaccta gttatgaatt ccatctaacc agacaagaaa tagtatcatt 1500

attcaacgca tttggaagaa tttctacaag tgtgaaagaa ttagaaaact 1550

tcaggaactt gttacagaat attcattaaa gaaaacaagc tgatatgtgc 1600

ctgtttctgg acaatggagg cgaaagagtg gaatttcatt caaaggcata 1650

atagcaatga cagtcttaag ccaaacattt tatataaagt tgcttttgta 1700

aaggagaatt atattgtttt aagtaaacac atttttaaaa attgtgttaa 1750

gtctatgtat aatactactg tgagtaaaag taatacttta ataatgtggt 1800

acaaatttta aagtttaata ttgaataaaa ggaggattat caaattaaaa 1850

aaaaaaaaaa aaaaaaaaaa aaaaaaaaaa aaaaa 1885

<210> 162
<211> 468
<212> PRT
<213> Homo Sapien

<400> 162
Met Gly Arg Gly Trp Gly Phe Leu Phe Gly Leu Leu Gly Ala Val
1 5 10 15

Trp Leu Leu Ser Ser Gly His Gly Glu Glu Gln Pro Pro Glu Thr
20 25 30

Ala Ala Gln Arg Cys Phe Cys Gln Val Ser Gly Tyr Leu Asp Asp
35 40 45

Cys Thr Cys Asp Val Glu Thr Ile Asp Arg Phe Asn Asn Tyr Arg
50 55 60

Leu Phe Pro Arg Leu Gln Lys Leu Leu Glu Ser Asp Tyr Phe Arg
65 70 75

Tyr Tyr Lys Val Asn Leu Lys Arg Pro Cys Pro Phe Trp Asn Asp
80 85 90

Ile Ser Gln Cys Gly Arg Arg Asp Cys Ala Val Lys Pro Cys Gln
95 100 105

Ser Asp Glu Val Pro Asp Gly Ile Lys Ser Ala Ser Tyr Lys Tyr
110 115 120

Ser Glu Glu Ala Asn Asn Leu Ile Glu Glu Cys Glu Gln Ala Glu
125 130 135

Arg Leu Gly Ala Val Asp Glu Ser Leu Ser Glu Glu Thr Gln Lys
140 145 150

Ala Val Leu Gln Trp Thr Lys His Asp Asp Ser Ser Asp Asn Phe
155 160 165

Cys Glu Ala Asp Asp Ile Gln Ser Pro Glu Ala Glu Tyr Val Asp
170 175 180

```
Leu Leu Leu Asn Pro Glu Arg Tyr Thr Gly Tyr Lys Gly Pro Asp
                185                 190                 195

Ala Trp Lys Ile Trp Asn Val Ile Tyr Glu Glu Asn Cys Phe Lys
                200                 205                 210

Pro Gln Thr Ile Lys Arg Pro Leu Asn Pro Leu Ala Ser Gly Gln
                215                 220                 225

Gly Thr Ser Glu Glu Asn Thr Phe Tyr Ser Trp Leu Glu Gly Leu
                230                 235                 240

Cys Val Glu Lys Arg Ala Phe Tyr Arg Leu Ile Ser Gly Leu His
                245                 250                 255

Ala Ser Ile Asn Val His Leu Ser Ala Arg Tyr Leu Leu Gln Glu
                260                 265                 270

Thr Trp Leu Glu Lys Lys Trp Gly His Asn Ile Thr Glu Phe Gln
                275                 280                 285

Gln Arg Phe Asp Gly Ile Leu Thr Glu Gly Glu Gly Pro Arg Arg
                290                 295                 300

Leu Lys Asn Leu Tyr Phe Leu Tyr Leu Ile Glu Leu Arg Ala Leu
                305                 310                 315

Ser Lys Val Leu Pro Phe Phe Glu Arg Pro Asp Phe Gln Leu Phe
                320                 325                 330

Thr Gly Asn Lys Ile Gln Asp Glu Glu Asn Lys Met Leu Leu Leu
                335                 340                 345

Glu Ile Leu His Glu Ile Lys Ser Phe Pro Leu His Phe Asp Glu
                350                 355                 360

Asn Ser Phe Phe Ala Gly Asp Lys Lys Glu Ala His Lys Leu Lys
                365                 370                 375

Glu Asp Phe Arg Leu His Phe Arg Asn Ile Ser Arg Ile Met Asp
                380                 385                 390

Cys Val Gly Cys Phe Lys Cys Arg Leu Trp Gly Lys Leu Gln Thr
                395                 400                 405

Gln Gly Leu Gly Thr Ala Leu Lys Ile Leu Phe Ser Glu Lys Leu
                410                 415                 420

Ile Ala Asn Met Pro Glu Ser Gly Pro Ser Tyr Glu Phe His Leu
                425                 430                 435

Thr Arg Gln Glu Ile Val Ser Leu Phe Asn Ala Phe Gly Arg Ile
                440                 445                 450

Ser Thr Ser Val Lys Glu Leu Glu Asn Phe Arg Asn Leu Leu Gln
                455                 460                 465

Asn Ile His
        468
```

<210> 163
<211> 20
<212> DNA

<213> Artificial Sequence

<220>
<223> Synthetic Oligonucleotide Probe

<400> 163
aagctgccgg agctgcaatg 20

<210> 164
<211> 21
<212> DNA
<213> Artificial Sequence

<220>
<223> Synthetic Oligonucleotide Probe

<400> 164
ttgcttctta atcctgagcg c 21

<210> 165
<211> 20
<212> DNA
<213> Artificial Sequence

<220>
<223> Synthetic Oligonucleotide Probe

<400> 165
aaaggaggac tttcgactgc 20

<210> 166
<211> 26
<212> DNA
<213> Artificial Sequence

<220>
<223> Synthetic Oligonucleotide Probe

<400> 166
agagattcat ccactgctcc aagtcg 26

<210> 167
<211> 25
<212> DNA
<213> Artificial Sequence

<220>
<223> Synthetic Oligonucleotide Probe

<400> 167
tgtccagaaa caggcacata tcagc 25

<210> 168
<211> 50
<212> DNA
<213> Artificial Sequence

<220>
<223> Synthetic Oligonucleotide Probe

<400> 168
agacagcggc acagaggtgc ttctgccagg ttagtggtta cttggatgat 50

<210> 169
<211> 1523
<212> DNA
<213> Homo Sapien

<400> 169
gagaggacga ggtgccgctg cctggagaat cctccgctgc cgtcggctcc 50

cggagcccag ccctttccta acccaaccca acctagccca gtcccagccg 100

ccagcgcctg tccctgtcac ggaccccagc gttaccatgc atcctgccgt 150

cttcctatcc ttacccgacc tcagatgctc ccttctgctc ctggtaactt 200

gggtttttac tcctgtaaca actgaaataa caagtcttgc tacagagaat 250

atagatgaaa ttttaaacaa tgctgatgtt gctttagtaa atttttatgc 300

tgactggtgt cgtttcagtc agatgttgca tccaattttt gaggaagctt 350

ccgatgtcat taaggaagaa tttccaaatg aaaatcaagt agtgtttgcc 400

agagttgatt gtgatcagca ctctgacata gcccagagat acaggataag 450

caaataccca accctcaaat tgtttcgtaa tgggatgatg atgaagagag 500

aatacagggg tcagcgatca gtgaaagcat tggcagatta catcaggcaa 550

caaaaaagtg accccattca agaaattcgg gacttagcag aaatcaccac 600

tcttgatcgc agcaaaagaa atatcattgg atattttgag caaaaggact 650

cggacaacta tagagttttt gaacgagtag cgaatatttt gcatgatgac 700

tgtgcctttc tttctgcatt tggggatgtt tcaaaaccgg aaagatatag 750

tggcgacaac ataatctaca aaccaccagg gcattctgct ccggatatgg 800

tgtacttggg agctatgaca aattttgatg tgacttacaa ttggattcaa 850

gataaatgtg ttcctcttgt ccgagaaata acatttgaaa atggagagga 900

attgacagaa gaaggactgc cttttctcat actctttcac atgaaagaag 950

atacagaaag tttagaaata ttccagaatg aagtagctcg gcaattaata 1000

agtgaaaaag gtacaataaa ctttttacat gccgattgtg acaaatttag 1050

acatcctctt ctgcacatac agaaaactcc agcagattgt cctgtaatcg 1100

ctattgacag ctttaggcat atgtatgtgt ttggagactt caaagatgta 1150

ttaattcctg gaaaactcaa gcaattcgta tttgacttac attctggaaa 1200

actgcacaga gaattccatc atggacctga cccaactgat acagccccag 1250

gagagcaagc ccaagatgta gcaagcagtc cacctgagag ctccttccag 1300

aaactagcac ccagtgaata taggtatact ctattgaggg atcgagatga 1350

gctttaaaaa cttgaaaaac agtttgtaag cctttcaaca gcagcatcaa 1400

cctacgtggt ggaaatagta aacctatatt ttcataattc tatgtgtatt 1450

tttattttga ataaacagaa agaaatttaa aaaaaaaaaa aaaaaaaaaa 1500

aaaaaaaaaa aaaaaaaaaa aaa 1523

<210> 170
<211> 406
<212> PRT
<213> Homo Sapien

<400> 170

```
Met His Pro Ala Val Phe Leu Ser Leu Pro Asp Leu Arg Cys Ser
  1               5                  10                  15

Leu Leu Leu Leu Val Thr Trp Val Phe Thr Pro Val Thr Thr Glu
                 20                  25                  30

Ile Thr Ser Leu Ala Thr Glu Asn Ile Asp Glu Ile Leu Asn Asn
                 35                  40                  45

Ala Asp Val Ala Leu Val Asn Phe Tyr Ala Asp Trp Cys Arg Phe
                 50                  55                  60

Ser Gln Met Leu His Pro Ile Phe Glu Glu Ala Ser Asp Val Ile
                 65                  70                  75

Lys Glu Glu Phe Pro Asn Glu Asn Gln Val Val Phe Ala Arg Val
                 80                  85                  90

Asp Cys Asp Gln His Ser Asp Ile Ala Gln Arg Tyr Arg Ile Ser
                 95                 100                 105

Lys Tyr Pro Thr Leu Lys Leu Phe Arg Asn Gly Met Met Met Lys
                110                 115                 120

Arg Glu Tyr Arg Gly Gln Arg Ser Val Lys Ala Leu Ala Asp Tyr
                125                 130                 135

Ile Arg Gln Gln Lys Ser Asp Pro Ile Gln Glu Ile Arg Asp Leu
                140                 145                 150

Ala Glu Ile Thr Thr Leu Asp Arg Ser Lys Arg Asn Ile Ile Gly
                155                 160                 165

Tyr Phe Glu Gln Lys Asp Ser Asp Asn Tyr Arg Val Phe Glu Arg
                170                 175                 180

Val Ala Asn Ile Leu His Asp Asp Cys Ala Phe Leu Ser Ala Phe
                185                 190                 195

Gly Asp Val Ser Lys Pro Glu Arg Tyr Ser Gly Asp Asn Ile Ile
                200                 205                 210

Tyr Lys Pro Pro Gly His Ser Ala Pro Asp Met Val Tyr Leu Gly
                215                 220                 225

Ala Met Thr Asn Phe Asp Val Thr Tyr Asn Trp Ile Gln Asp Lys
                230                 235                 240

Cys Val Pro Leu Val Arg Glu Ile Thr Phe Glu Asn Gly Glu Glu
                245                 250                 255

Leu Thr Glu Glu Gly Leu Pro Phe Leu Ile Leu Phe His Met Lys
                260                 265                 270
```

Glu Asp Thr Glu Ser Leu Glu Ile Phe Gln Asn Glu Val Ala Arg
275 280 285

Gln Leu Ile Ser Glu Lys Gly Thr Ile Asn Phe Leu His Ala Asp
290 295 300

Cys Asp Lys Phe Arg His Pro Leu Leu His Ile Gln Lys Thr Pro
305 310 315

Ala Asp Cys Pro Val Ile Ala Ile Asp Ser Phe Arg His Met Tyr
320 325 330

Val Phe Gly Asp Phe Lys Asp Val Leu Ile Pro Gly Lys Leu Lys
335 340 345

Gln Phe Val Phe Asp Leu His Ser Gly Lys Leu His Arg Glu Phe
350 355 360

His His Gly Pro Asp Pro Thr Asp Thr Ala Pro Gly Glu Gln Ala
365 370 375

Gln Asp Val Ala Ser Ser Pro Pro Glu Ser Ser Phe Gln Lys Leu
380 385 390

Ala Pro Ser Glu Tyr Arg Tyr Thr Leu Leu Arg Asp Arg Asp Glu
395 400 405

Leu
406

<210> 171
<211> 22
<212> DNA
<213> Artificial Sequence

<220>
<223> Synthetic Oligonucleotide Probe

<400> 171
tgagaggcct ctctggaagt tg 22

<210> 172
<211> 19
<212> DNA
<213> Artificial Sequence

<220>
<223> Synthetic Oligonucleotide Probe

<400> 172
gtcagcgatc agtgaaagc 19

<210> 173
<211> 20
<212> DNA
<213> Artificial Sequence

<220>
<223> Synthetic Oligonucleotide Probe

<400> 173
ccagaatgaa gtagctcggc 20

<210> 174
<211> 20
<212> DNA
<213> Artificial Sequence

<220>
<223> Synthetic Oligonucleotide Probe

<400> 174
ccgactcaaa atgcattgtc 20

<210> 175
<211> 19
<212> DNA
<213> Artificial Sequence

<220>
<223> Synthetic Oligonucleotide Probe

<400> 175
catttggcag gaattgtcc 19

<210> 176
<211> 18
<212> DNA
<213> Artificial Sequence

<220>
<223> Synthetic Oligonucleotide Probe

<400> 176
ggtgctatag gccaaggg 18

<210> 177
<211> 24
<212> DNA
<213> Artificial Sequence

<220>
<223> Synthetic Oligonucleotide Probe

<400> 177
ctgtatctct gggctatgtc agag 24

<210> 178
<211> 25
<212> DNA
<213> Artificial Sequence

<220>
<223> Synthetic Oligonucleotide Probe

<400> 178
ctacatataa tggcacatgt cagcc 25

<210> 179
<211> 46
<212> DNA
<213> Artificial Sequence

<220>
<223> Synthetic Oligonucleotide Probe

<400> 179
cgtcttccta tccttacccg acctcagatg ctcccttctg ctcctg 46

<210> 180
<211> 2475
<212> DNA
<213> Homo Sapien

<400> 180
gaggatttgc cacagcagcg gatagagcag gagagcacca ccggagccct 50

tgagacatcc ttgagaagag ccacagcata agagactgcc ctgcttggtg 100

ttttgcagga tgatggtggc ccttcgagga gcttctgcat tgctggttct 150

gttccttgca gcttttctgc ccccgccgca gtgtacccag gacccagcca 200

tggtgcatta catctaccag cgctttcgag tcttggagca agggctggaa 250

aaatgtaccc aagcaacgag ggcatacatt caagaattcc aagagttctc 300

aaaaaatata tctgtcatgc tgggaagatg tcagacctac acaagtgagt 350

acaagagtgc agtgggtaac ttggcactga gagttgaacg tgcccaacgg 400

gagattgact acatacaata ccttcgagag gctgacgagt gcatcgtatc 450

agaggacaag acactggcag aaatgttgct ccaagaagct gaagaagaga 500

aaaagatccg gactctgctg aatgcaagct gtgacaacat gctgatgggc 550

ataaagtctt tgaaaatagt gaagaagatg atggacacac atggctcttg 600

gatgaaagat gctgtctata actctccaaa ggtgtactta ttaattggat 650

ccagaaacaa cactgtttgg gaatttgcaa acatacgggc attcatggag 700

gataacacca agccagctcc ccggaagcaa atcctaacac tttcctggca 750

gggaacaggc caagtgatct acaaaggttt tctatttttt cataaccaag 800

caacttctaa tgagataatc aaatataacc tgcagaagag gactgtggaa 850

gatcgaatgc tgctcccagg aggggtaggc cgagcattgg tttaccagca 900

ctccccctca acttacattg acctggctgt ggatgagcat gggctctggg 950

ccatccactc tgggccaggc acccatagcc atttggttct cacaaagatt 1000

gagccgggca cactgggagt ggagcattca tgggataccc catgcagaag 1050

ccaggatgct gaagcctcat tcctcttgtg tggggttctc tatgtggtct 1100

acagtactgg gggccagggc cctcatcgca tcacctgcat ctatgatcca 1150

ctgggcacta tcagtgagga ggacttgccc aacttgttct tccccaagag 1200

accaagaagt cactccatga tccattacaa ccccagagat aagcagctct 1250

atgcctggaa tgaaggaaac cagatcattt acaaactcca gacaaagaga 1300

aagctgcctc tgaagtaatg cattacagct gtgagaaaga gcactgtggc 1350

tttggcagct gttctacagg acagtgaggc tatagcccct tcacaatata 1400

gtatccctct aatcacacac aggaagagtg tgtagaagtg gaaatacgta 1450

tgcctccttt cccaaatgtc actgccttag gtatcttcca agagcttaga 1500

tgagagcata tcatcaggaa agtttcaaca atgtccatta ctcccccaaa 1550

cctcctggct ctcaaggatg accacattct gatacagcct acttcaagcc 1600

ttttgtttta ctgctcccca gcatttactg taactctgcc atcttccctc 1650

ccacaattag agttgtatgc cagcccctaa tattcaccac tggcttttct 1700

ctcccctggc ctttgctgaa gctcttccct ctttttcaaa tgtctattga 1750

tattctccca ttttcactgc ccaactaaaa tactattaat atttctttct 1800

tttcttttct tttttttgag acaaggtctc actatgttgc ccaggctggt 1850

ctcaaactcc agagctcaag agatcctcct gcctcagcct cctaagtacc 1900

tgggattaca ggcatgtgcc accacacctg gcttaaaata ctatttctta 1950

ttgaggttta acctctattt cccctagccc tgtccttcca ctaagcttgg 2000

tagatgtaat aataaagtga aaatattaac atttgaatat cgctttccag 2050

gtgtggagtg tttgcacatc attgaattct cgtttcacct ttgtgaaaca 2100

tgcacaagtc tttacagctg tcattctaga gtttaggtga gtaacacaat 2150

tacaaagtga aagatacagc tagaaaatac tacaaatccc atagtttttc 2200

cattgcccaa ggaagcatca aatacgtatg tttgttcacc tactcttata 2250

gtcaatgcgt tcatcgtttc agcctaaaaa taatagtctg tccctttagc 2300

cagttttcat gtctgcacaa gacctttcaa taggcctttc aaatgataat 2350

tcctccagaa aaccagtcta agggtgagga ccccaactct agcctcctct 2400

tgtcttgctg tcctctgttt ctctctttct gctttaaatt caataaaagt 2450

gacactgagc aaaaaaaaaa aaaaa 2475

<210> 181
<211> 402
<212> PRT
<213> Homo Sapien

<400> 181
Met Met Val Ala Leu Arg Gly Ala Ser Ala Leu Leu Val Leu Phe
1 5 10 15

Leu Ala Ala Phe Leu Pro Pro Pro Gln Cys Thr Gln Asp Pro Ala
20 25 30

Met Val His Tyr Ile Tyr Gln Arg Phe Arg Val Leu Glu Gln Gly
35 40 45

Leu Glu Lys Cys Thr Gln Ala Thr Arg Ala Tyr Ile Gln Glu Phe
50 55 60

Gln Glu Phe Ser Lys Asn Ile Ser Val Met Leu Gly Arg Cys Gln
65 70 75

Thr Tyr Thr Ser Glu Tyr Lys Ser Ala Val Gly Asn Leu Ala Leu
80 85 90

Arg Val Glu Arg Ala Gln Arg Glu Ile Asp Tyr Ile Gln Tyr Leu
95 100 105

Arg Glu Ala Asp Glu Cys Ile Val Ser Glu Asp Lys Thr Leu Ala
110 115 120

Glu Met Leu Leu Gln Glu Ala Glu Glu Glu Lys Lys Ile Arg Thr
125 130 135

Leu Leu Asn Ala Ser Cys Asp Asn Met Leu Met Gly Ile Lys Ser
140 145 150

Leu Lys Ile Val Lys Lys Met Met Asp Thr His Gly Ser Trp Met
155 160 165

Lys Asp Ala Val Tyr Asn Ser Pro Lys Val Tyr Leu Leu Ile Gly
170 175 180

Ser Arg Asn Asn Thr Val Trp Glu Phe Ala Asn Ile Arg Ala Phe
185 190 195

Met Glu Asp Asn Thr Lys Pro Ala Pro Arg Lys Gln Ile Leu Thr
200 205 210

Leu Ser Trp Gln Gly Thr Gly Gln Val Ile Tyr Lys Gly Phe Leu
215 220 225

Phe Phe His Asn Gln Ala Thr Ser Asn Glu Ile Ile Lys Tyr Asn
230 235 240

Leu Gln Lys Arg Thr Val Glu Asp Arg Met Leu Leu Pro Gly Gly
245 250 255

Val Gly Arg Ala Leu Val Tyr Gln His Ser Pro Ser Thr Tyr Ile
260 265 270

Asp Leu Ala Val Asp Glu His Gly Leu Trp Ala Ile His Ser Gly
275 280 285

Pro Gly Thr His Ser His Leu Val Leu Thr Lys Ile Glu Pro Gly
290 295 300

Thr Leu Gly Val Glu His Ser Trp Asp Thr Pro Cys Arg Ser Gln
305 310 315

Asp Ala Glu Ala Ser Phe Leu Leu Cys Gly Val Leu Tyr Val Val
320 325 330

Tyr Ser Thr Gly Gly Gln Gly Pro His Arg Ile Thr Cys Ile Tyr
335 340 345

Asp Pro Leu Gly Thr Ile Ser Glu Glu Asp Leu Pro Asn Leu Phe
350 355 360

Phe Pro Lys Arg Pro Arg Ser His Ser Met Ile His Tyr Asn Pro
365 370 375

Arg Asp Lys Gln Leu Tyr Ala Trp Asn Glu Gly Asn Gln Ile Ile
380 385 390

Tyr Lys Leu Gln Thr Lys Arg Lys Leu Pro Leu Lys
395 400 402

<210> 182
<211> 1337
<212> DNA
<213> Homo Sapien

<400> 182
gttgatggca aacttcctca aaggaggggc agagcctgcg cagggcagga 50

gcagctggcc cactggcggc ccgcaacact ccgtctcacc ctctgggccc 100

actgcatcta gaggagggcc gtctgtgagg ccactacccc tccagcaact 150

gggaggtggg actgtcagaa gctggcccag ggtggtggtc agctgggtca 200

gggacctacg gcacctgctg gaccacctcg ccttctccat cgaagcaggg 250

aagtgggagc ctcgagccct cgggtggaag ctgaccccaa gccacccttc 300

acctggacag gatgagagtg tcaggtgtgc ttcgcctcct ggccctcatc 350

tttgccatag tcacgacatg gatgtttatt cgaagctaca tgagcttcag 400

catgaaaacc atccgtctgc cacgctggct ggcagcctcg cccaccaagg 450

agatccaggt taaaaagtac aagtgtggcc tcatcaagcc ctgcccagcc 500

aactactttg cgtttaaaat ctgcagtggg gccgccaacg tcgtgggccc 550

tactatgtgc tttgaagacc gcatgatcat gagtcctgtg aaaaacaatg 600

tgggcagagg cctaaacatc gccctggtga atggaaccac gggagctgtg 650

ctgggacaga aggcatttga catgtactct ggagatgtta tgcacctagt 700

gaaattcctt aaagaaattc cggggggtgc actggtgctg gtggcctcct 750

acgacgatcc agggaccaaa atgaacgatg aaagcaggaa actcttctct 800

gacttgggga gttcctacgc aaaacaactg ggcttccggg acagctgggt 850

cttcatagga gccaaagacc tcaggggtaa aagccccttt gagcagttct 900

taaagaacag cccagacaca aacaaatacg agggatggcc agagctgctg 950

gagatggagg gctgcatgcc cccgaagcca ttttagggtg gctgtggctc 1000

ttcctcagcc aggggcctga agaagctcct gcctgactta ggagtcagag 1050

cccggcaggg gctgaggagg aggagcaggg ggtgctgcgt ggaaggtgct 1100

gcaggtcctt gcacgctgtg tcgcgcctct cctcctcgga aacagaaccc 1150

tcccacagca catcctaccc ggaagaccag cctcagaggg tccttctgga 1200

accagctgtc tgtggagaga atggggtgct ttcgtcaggg actgctgacg 1250

gctggtcctg aggaaggaca aactgcccag acttgagccc aattaaattt 1300

tatttttgct ggttttgaaa aaaaaaaaaa aaaaaaa 1337

<210> 183
<211> 224
<212> PRT
<213> Homo Sapien

<400> 183
Met Arg Val Ser Gly Val Leu Arg Leu Leu Ala Leu Ile Phe Ala
1               5                   10                  15

Ile Val Thr Thr Trp Met Phe Ile Arg Ser Tyr Met Ser Phe Ser
                20                  25                  30

Met Lys Thr Ile Arg Leu Pro Arg Trp Leu Ala Ala Ser Pro Thr
                35                  40                  45

Lys Glu Ile Gln Val Lys Lys Tyr Lys Cys Gly Leu Ile Lys Pro
                50                  55                  60

Cys Pro Ala Asn Tyr Phe Ala Phe Lys Ile Cys Ser Gly Ala Ala
                65                  70                  75

Asn Val Val Gly Pro Thr Met Cys Phe Glu Asp Arg Met Ile Met
                80                  85                  90

Ser Pro Val Lys Asn Asn Val Gly Arg Gly Leu Asn Ile Ala Leu
                95                  100                 105

Val Asn Gly Thr Thr Gly Ala Val Leu Gly Gln Lys Ala Phe Asp
                110                 115                 120

Met Tyr Ser Gly Asp Val Met His Leu Val Lys Phe Leu Lys Glu
                125                 130                 135

Ile Pro Gly Gly Ala Leu Val Leu Val Ala Ser Tyr Asp Asp Pro
                140                 145                 150

Gly Thr Lys Met Asn Asp Glu Ser Arg Lys Leu Phe Ser Asp Leu
                155                 160                 165

Gly Ser Ser Tyr Ala Lys Gln Leu Gly Phe Arg Asp Ser Trp Val
                170                 175                 180

Phe Ile Gly Ala Lys Asp Leu Arg Gly Lys Ser Pro Phe Glu Gln
                185                 190                 195

Phe Leu Lys Asn Ser Pro Asp Thr Asn Lys Tyr Glu Gly Trp Pro
                200                 205                 210

Glu Leu Leu Glu Met Glu Gly Cys Met Pro Pro Lys Pro Phe
                215                 220             224

<210> 184
<211> 21
<212> DNA
<213> Artificial Sequence

<220>
<223> Synthetic Oligonucleotide Probe

<400> 184
gccatagtca cgacatggat g 21

<210> 185
<211> 18
<212> DNA
<213> Artificial Sequence

<220>
<223> Synthetic Oligonucleotide Probe

<400> 185
ggatggccag agctgctg 18

<210> 186
<211> 26
<212> DNA
<213> Artificial Sequence

<220>
<223> Synthetic Oligonucleotide Probe

<400> 186
aaagtacaag tgtggcctca tcaagc 26

<210> 187
<211> 24
<212> DNA
<213> Artificial Sequence

<220>
<223> Synthetic Oligonucleotide Probe

<400> 187
tctgactcct aagtcaggca ggag 24

<210> 188
<211> 24
<212> DNA
<213> Artificial Sequence

<220>
<223> Synthetic Oligonucleotide Probe

<400> 188
attctctcca cagacagctg gttc 24

<210> 189
<211> 46
<212> DNA
<213> Artificial Sequence

<220>
<223> Synthetic Oligonucleotide Probe

<400> 189
gtacaagtgt ggcctcatca agccctgccc agccaactac tttgcg 46

<210> 190
<211> 3225
<212> DNA
<213> Homo Sapien

<400> 190
gggggagcta ggccggcggc agtggtggtg gcggcggcgc aagggtgagg 50

gcggccccag aaccccaggt aggtagagca agaagatggt gtttctgccc 100
ctcaaatggt cccttgcaac catgtcattt ctactttcct cactgttggc 150
tctcttaact gtgtccactc cttcatggtg tcagagcact gaagcatctc 200
caaaacgtag tgatgggaca ccatttcctt ggaataaaat acgacttcct 250
gagtacgtca tcccagttca ttatgatctc ttgatccatg caaaccttac 300
cacgctgacc ttctggggaa ccacgaaagt agaaatcaca gccagtcagc 350
ccaccagcac catcatcctg catagtcacc acctgcagat atctagggcc 400
accctcagga agggagctgg agagaggcta tcggaagaac ccctgcaggt 450
cctggaacac ccccctcagg agcaaattgc actgctggct cccgagcccc 500
tccttgtcgg gctcccgtac acagttgtca ttcactatgc tggcaatctt 550
tcggagactt tccacggatt ttacaaaagc acctacagaa ccaaggaagg 600
ggaactgagg atactagcat caacacaatt tgaacccact gcagctagaa 650
tggcctttcc ctgctttgat gaacctgcct tcaaagcaag tttctcaatc 700
aaaattagaa gagagccaag gcacctagcc atctccaata tgccattggt 750
gaaatctgtg actgttgctg aaggactcat agaagaccat tttgatgtca 800
ctgtgaagat gagcacctat ctggtggcct tcatcatttc agattttgag 850
tctgtcagca agataaccaa gagtggagtc aaggtttctg tttatgctgt 900
gccagacaag ataaatcaag cagattatgc actggatgct gcggtgactc 950
ttctagaatt ttatgaggat tatttcagca taccgtatcc cctacccaaa 1000
caagatcttg ctgctattcc cgactttcag tctggtgcta tggaaaactg 1050
gggactgaca acatatagag aatctgctct gttgtttgat gcagaaaagt 1100
cttctgcatc aagtaagctt ggcatcacag tgactgtggc ccatgaactg 1150
gcccaccagt ggtttgggaa cctggtcact atggaatggt ggaatgatct 1200
ttggctaaat gaaggatttg ccaaatttat ggagtttgtg tctgtcagtg 1250
tgacccatcc tgaactgaaa gttggagatt atttctttgg caaatgtttt 1300
gacgcaatgg aggtagatgc tttaaattcc tcacaccctg tgtctacacc 1350
tgtggaaaat cctgctcaga tccgggagat gtttgatgat gtttcttatg 1400
ataagggagc ttgtattctg aatatgctaa gggagtatct tagcgctgac 1450
gcatttaaaa gtggtattgt acagtatctc cagaagcata gctataaaaa 1500
tacaaaaaac gaggacctgt gggatagtat ggcaagtatt tgccctacag 1550
atggtgtaaa agggatggat ggcttttgct ctagaagtca acattcatct 1600
tcatcctcac attggcatca ggaaggggtg gatgtgaaaa ccatgatgaa 1650

cacttggaca ctgcagaggg gttttcccct aataaccatc acagtgaggg 1700
ggaggaatgt acacatgaag caagagcact acatgaaggg ctctgacggc 1750
gccccggaca ctgggtacct gtggcatgtt ccattgacat tcatcaccag 1800
caaatccaac atggtccatc gatttttgct aaaaacaaaa acagatgtgc 1850
tcatcctccc agaagaggtg gaatggatca aatttaatgt gggcatgaat 1900
ggctattaca ttgtgcatta cgaggatgat ggatgggact ctttgactgg 1950
ccttttaaaa ggaacacaca cagcagtcag cagtaatgat cgggcaagtc 2000
tcattaacaa tgcatttcag ctcgtcagca ttgggaagct gtccattgaa 2050
aaggccttgg atttatccct gtacttgaaa catgaaactg aaattatgcc 2100
cgtgtttcaa ggtttgaatg agctgattcc tatgtataag ttaatggaga 2150
aaagagatat gaatgaagtg gaaactcaat tcaaggcctt cctcatcagg 2200
ctgctaaggg acctcattga taagcagaca tggacagacg agggctcagt 2250
ctcagagcaa atgctgcgga gtgaactact actcctcgcc tgtgtgcaca 2300
actatcagcc gtgcgtacag agggcagaag gctatttcag aaagtggaag 2350
gaatccaatg gaaacttgag cctgcctgtc gacgtgacct tggcagtgtt 2400
tgctgtgggg gcccagagca cagaaggctg ggattttctt tatagtaaat 2450
atcagttttc tttgtccagt actgagaaaa gccaaattga atttgccctc 2500
tgcagaaccc aaaataagga aaagcttcaa tggctactag atgaaagctt 2550
taagggagat aaaataaaaa ctcaggagtt tccacaaatt cttacactca 2600
ttggcaggaa cccagtagga tacccactgg cctggcaatt tctgaggaaa 2650
aactggaaca aacttgtaca aaagtttgaa cttggctcat cttccatagc 2700
ccacatggta atgggtacaa caaatcaatt ctccacaaga acacggcttg 2750
aagaggtaaa aggattcttc agctctttga aagaaaatgg ttctcagctc 2800
cgttgtgtcc aacagacaat tgaaaccatt gaagaaaaca tcggttggat 2850
ggataagaat tttgataaaa tcagagtgtg gctgcaaagt gaaaagcttg 2900
aacgtatgta aaaattcctc ccttgcccgg ttcctgttat ctctaatcac 2950
caacattttg ttgagtgtat tttcaaacta gagatggctg ttttggctcc 3000
aactggagat actttttttcc cttcaactca ttttttgact atccctgtga 3050
aaagaatagc tgttagtttt tcatgaatgg gctttttcat gaatgggcta 3100
tcgctaccat gtgttttgtt catcacaggt gttgccctgc aacgtaaacc 3150
caagtgttgg gttccctgcc acagaagaat aaagtacctt attcttctca 3200
aaaaaaaaaa aaaaaaaaaa aaaaa 3225

<210> 191
<211> 941
<212> PRT
<213> Homo Sapien

<400> 191
```
Met Val Phe Leu Pro Leu Lys Trp Ser Leu Ala Thr Met Ser Phe
  1               5                  10                  15

Leu Leu Ser Ser Leu Leu Ala Leu Leu Thr Val Ser Thr Pro Ser
                 20                  25                  30

Trp Cys Gln Ser Thr Glu Ala Ser Pro Lys Arg Ser Asp Gly Thr
                 35                  40                  45

Pro Phe Pro Trp Asn Lys Ile Arg Leu Pro Glu Tyr Val Ile Pro
                 50                  55                  60

Val His Tyr Asp Leu Leu Ile His Ala Asn Leu Thr Thr Leu Thr
                 65                  70                  75

Phe Trp Gly Thr Thr Lys Val Glu Ile Thr Ala Ser Gln Pro Thr
                 80                  85                  90

Ser Thr Ile Ile Leu His Ser His His Leu Gln Ile Ser Arg Ala
                 95                 100                 105

Thr Leu Arg Lys Gly Ala Gly Glu Arg Leu Ser Glu Glu Pro Leu
                110                 115                 120

Gln Val Leu Glu His Pro Pro Gln Glu Gln Ile Ala Leu Leu Ala
                125                 130                 135

Pro Glu Pro Leu Leu Val Gly Leu Pro Tyr Thr Val Val Ile His
                140                 145                 150

Tyr Ala Gly Asn Leu Ser Glu Thr Phe His Gly Phe Tyr Lys Ser
                155                 160                 165

Thr Tyr Arg Thr Lys Glu Gly Glu Leu Arg Ile Leu Ala Ser Thr
                170                 175                 180

Gln Phe Glu Pro Thr Ala Ala Arg Met Ala Phe Pro Cys Phe Asp
                185                 190                 195

Glu Pro Ala Phe Lys Ala Ser Phe Ser Ile Lys Ile Arg Arg Glu
                200                 205                 210

Pro Arg His Leu Ala Ile Ser Asn Met Pro Leu Val Lys Ser Val
                215                 220                 225

Thr Val Ala Glu Gly Leu Ile Glu Asp His Phe Asp Val Thr Val
                230                 235                 240

Lys Met Ser Thr Tyr Leu Val Ala Phe Ile Ile Ser Asp Phe Glu
                245                 250                 255

Ser Val Ser Lys Ile Thr Lys Ser Gly Val Lys Val Ser Val Tyr
                260                 265                 270

Ala Val Pro Asp Lys Ile Asn Gln Ala Asp Tyr Ala Leu Asp Ala
                275                 280                 285
```

```
Ala Val Thr Leu Leu Glu Phe Tyr Glu Asp Tyr Phe Ser Ile Pro
                290                 295                 300

Tyr Pro Leu Pro Lys Gln Asp Leu Ala Ala Ile Pro Asp Phe Gln
                305                 310                 315

Ser Gly Ala Met Glu Asn Trp Gly Leu Thr Thr Tyr Arg Glu Ser
                320                 325                 330

Ala Leu Leu Phe Asp Ala Glu Lys Ser Ser Ala Ser Ser Lys Leu
                335                 340                 345

Gly Ile Thr Val Thr Val Ala His Glu Leu Ala His Gln Trp Phe
                350                 355                 360

Gly Asn Leu Val Thr Met Glu Trp Trp Asn Asp Leu Trp Leu Asn
                365                 370                 375

Glu Gly Phe Ala Lys Phe Met Glu Phe Val Ser Val Ser Val Thr
                380                 385                 390

His Pro Glu Leu Lys Val Gly Asp Tyr Phe Phe Gly Lys Cys Phe
                395                 400                 405

Asp Ala Met Glu Val Asp Ala Leu Asn Ser Ser His Pro Val Ser
                410                 415                 420

Thr Pro Val Glu Asn Pro Ala Gln Ile Arg Glu Met Phe Asp Asp
                425                 430                 435

Val Ser Tyr Asp Lys Gly Ala Cys Ile Leu Asn Met Leu Arg Glu
                440                 445                 450

Tyr Leu Ser Ala Asp Ala Phe Lys Ser Gly Ile Val Gln Tyr Leu
                455                 460                 465

Gln Lys His Ser Tyr Lys Asn Thr Lys Asn Glu Asp Leu Trp Asp
                470                 475                 480

Ser Met Ala Ser Ile Cys Pro Thr Asp Gly Val Lys Gly Met Asp
                485                 490                 495

Gly Phe Cys Ser Arg Ser Gln His Ser Ser Ser Ser Ser His Trp
                500                 505                 510

His Gln Glu Gly Val Asp Val Lys Thr Met Met Asn Thr Trp Thr
                515                 520                 525

Leu Gln Arg Gly Phe Pro Leu Ile Thr Ile Thr Val Arg Gly Arg
                530                 535                 540

Asn Val His Met Lys Gln Glu His Tyr Met Lys Gly Ser Asp Gly
                545                 550                 555

Ala Pro Asp Thr Gly Tyr Leu Trp His Val Pro Leu Thr Phe Ile
                560                 565                 570

Thr Ser Lys Ser Asn Met Val His Arg Phe Leu Leu Lys Thr Lys
                575                 580                 585

Thr Asp Val Leu Ile Leu Pro Glu Glu Val Glu Trp Ile Lys Phe
                590                 595                 600
```

```
Asn Val Gly Met Asn Gly Tyr Tyr Ile Val His Tyr Glu Asp Asp
                605                 610                 615

Gly Trp Asp Ser Leu Thr Gly Leu Leu Lys Gly Thr His Thr Ala
                620                 625                 630

Val Ser Ser Asn Asp Arg Ala Ser Leu Ile Asn Asn Ala Phe Gln
                635                 640                 645

Leu Val Ser Ile Gly Lys Leu Ser Ile Glu Lys Ala Leu Asp Leu
                650                 655                 660

Ser Leu Tyr Leu Lys His Glu Thr Glu Ile Met Pro Val Phe Gln
                665                 670                 675

Gly Leu Asn Glu Leu Ile Pro Met Tyr Lys Leu Met Glu Lys Arg
                680                 685                 690

Asp Met Asn Glu Val Glu Thr Gln Phe Lys Ala Phe Leu Ile Arg
                695                 700                 705

Leu Leu Arg Asp Leu Ile Asp Lys Gln Thr Trp Thr Asp Glu Gly
                710                 715                 720

Ser Val Ser Glu Gln Met Leu Arg Ser Glu Leu Leu Leu Leu Ala
                725                 730                 735

Cys Val His Asn Tyr Gln Pro Cys Val Gln Arg Ala Glu Gly Tyr
                740                 745                 750

Phe Arg Lys Trp Lys Glu Ser Asn Gly Asn Leu Ser Leu Pro Val
                755                 760                 765

Asp Val Thr Leu Ala Val Phe Ala Val Gly Ala Gln Ser Thr Glu
                770                 775                 780

Gly Trp Asp Phe Leu Tyr Ser Lys Tyr Gln Phe Ser Leu Ser Ser
                785                 790                 795

Thr Glu Lys Ser Gln Ile Glu Phe Ala Leu Cys Arg Thr Gln Asn
                800                 805                 810

Lys Glu Lys Leu Gln Trp Leu Leu Asp Glu Ser Phe Lys Gly Asp
                815                 820                 825

Lys Ile Lys Thr Gln Glu Phe Pro Gln Ile Leu Thr Leu Ile Gly
                830                 835                 840

Arg Asn Pro Val Gly Tyr Pro Leu Ala Trp Gln Phe Leu Arg Lys
                845                 850                 855

Asn Trp Asn Lys Leu Val Gln Lys Phe Glu Leu Gly Ser Ser Ser
                860                 865                 870

Ile Ala His Met Val Met Gly Thr Thr Asn Gln Phe Ser Thr Arg
                875                 880                 885

Thr Arg Leu Glu Glu Val Lys Gly Phe Phe Ser Ser Leu Lys Glu
                890                 895                 900

Asn Gly Ser Gln Leu Arg Cys Val Gln Gln Thr Ile Glu Thr Ile
                905                 910                 915
```

Glu Glu Asn Ile Gly Trp Met Asp Lys Asn Phe Asp Lys Ile Arg
920 925 930

Val Trp Leu Gln Ser Glu Lys Leu Glu Arg Met
935 940 941

<210> 192
<211> 2213
<212> DNA
<213> Homo Sapien

<400> 192
gagcgaacat ggcagcgcgt tggcggtttt ggtgtgtctc tgtgaccatg 50

gtggtggcgc tgctcatcgt ttgcgacgtt ccctcagcct ctgcccaaag 100

aaagaaggag atggtgttat ctgaaaaggt tagtcagctg atggaatgga 150

ctaacaaaag acctgtaata agaatgaatg gagacaagtt ccgtcgcctt 200

gtgaaagccc caccgagaaa ttactccgtt atcgtcatgt tcactgctct 250

ccaactgcat agacagtgtg tcgtttgcaa gcaagctgat gaagaattcc 300

agatcctggc aaactcctgg cgatactcca gtgcattcac caacaggata 350

ttttttgcca tggtggattt tgatgaaggc tctgatgtat ttcagatgct 400

aaacatgaat tcagctccaa ctttcatcaa ctttcctgca aaagggaaac 450

ccaaacgggg tgatacatat gagttacagg tgcggggttt ttcagctgag 500

cagattgccc ggtggatcgc cgacagaact gatgtcaata ttagagtgat 550

tagaccccca aattatgctg gtcccttat gttgggattg cttttggctg 600

ttattggtgg acttgtgtat cttcgaagaa gtaatatgga atttctcttt 650

aataaaactg gatgggcttt tgcagctttg tgttttgtgc ttgctatgac 700

atctggtcaa atgtggaacc atataagagg accaccatat gcccataaga 750

atccccacac gggacatgtg aattatatcc atggaagcag tcaagcccag 800

tttgtagctg aaacacacat tgttcttctg tttaatggtg gagttacctt 850

aggaatggtg cttttatgtg aagctgctac ctctgacatg gatattggaa 900

agcgaaagat aatgtgtgtg gctggtattg gacttgttgt attattcttc 950

agttggatgc tctctatttt tagatctaaa tatcatggct acccatacag 1000

ctttctgatg agttaaaaag gtcccagaga tatatagaca ctggagtact 1050

ggaaattgaa aaacgaaaat cgtgtgtgtt tgaaaagaag aatgcaactt 1100

gtatattttg tattacctct ttttttcaag tgatttaaat agttaatcat 1150

ttaaccaaag aagatgtgta gtgccttaac aagcaatcct ctgtcaaaat 1200

ctgaggtatt tgaaaataat tatcctctta accttctctt cccagtgaac 1250

tttatggaac atttaattta gtacaattaa gtatattata aaaattgtaa 1300

aactactact ttgttttagt tagaacaaag ctcaaaacta ctttagttaa 1350

cttggtcatc tgattttata ttgccttatc caaagatggg gaaagtaagt 1400

cctgaccagg tgttcccaca tatgcctgtt acagataact acattaggaa 1450

ttcattctta gcttcttcat ctttgtgtgg atgtgtatac tttacgcatc 1500

tttccttttg agtagagaaa ttatgtgtgt catgtggtct tctgaaaatg 1550

gaacaccatt cttcagagca cacgtctagc cctcagcaag acagttgttt 1600

ctcctcctcc ttgcatattt cctactgcgc tccagcctga gtgatagagt 1650

gagactctgt ctcaaaaaaa agtatctcta aatacaggat tataatttct 1700

gcttgagtat ggtgttaact accttgtatt tagaaagatt tcagattcat 1750

tccatctcct tagttttctt ttaaggtgac ccatctgtga taaaaatata 1800

gcttagtgct aaaatcagtg taacttatac atggcctaaa atgtttctac 1850

aaattagagt ttgtcactta ttccatttgt acctaagaga aaaataggct 1900

cagttagaaa aggactccct ggccaggcgc agtgacttac gcctgtaatc 1950

tcagcacttt gggaggccaa ggcaggcaga tcacgaggtc aggagttcga 2000

gaccatcctg gccaacatgg tgaaaccccg tctctactaa aaatataaaa 2050

attagctggg tgtggtggca ggagcctgta atcccagcta cacaggaggc 2100

tgaggcacga gaatcacttg aactcaggag atggaggttt cagtgagccg 2150

agatcacgcc actgcactcc agcctggcaa cagagcgaga ctccatctca 2200

aaaaaaaaaa aaa 2213

<210> 193
<211> 335
<212> PRT
<213> Homo Sapien

<400> 193
Met Ala Ala Arg Trp Arg Phe Trp Cys Val Ser Val Thr Met Val
1 5 10 15

Val Ala Leu Leu Ile Val Cys Asp Val Pro Ser Ala Ser Ala Gln
20 25 30

Arg Lys Lys Glu Met Val Leu Ser Glu Lys Val Ser Gln Leu Met
35 40 45

Glu Trp Thr Asn Lys Arg Pro Val Ile Arg Met Asn Gly Asp Lys
50 55 60

Phe Arg Arg Leu Val Lys Ala Pro Pro Arg Asn Tyr Ser Val Ile
65 70 75

Val Met Phe Thr Ala Leu Gln Leu His Arg Gln Cys Val Val Cys
80 85 90

```
Lys Gln Ala Asp Glu Glu Phe Gln Ile Leu Ala Asn Ser Trp Arg
                95                  100                 105

Tyr Ser Ser Ala Phe Thr Asn Arg Ile Phe Phe Ala Met Val Asp
                110                 115                 120

Phe Asp Glu Gly Ser Asp Val Phe Gln Met Leu Asn Met Asn Ser
                125                 130                 135

Ala Pro Thr Phe Ile Asn Phe Pro Ala Lys Gly Lys Pro Lys Arg
                140                 145                 150

Gly Asp Thr Tyr Glu Leu Gln Val Arg Gly Phe Ser Ala Glu Gln
                155                 160                 165

Ile Ala Arg Trp Ile Ala Asp Arg Thr Asp Val Asn Ile Arg Val
                170                 175                 180

Ile Arg Pro Pro Asn Tyr Ala Gly Pro Leu Met Leu Gly Leu Leu
                185                 190                 195

Leu Ala Val Ile Gly Gly Leu Val Tyr Leu Arg Arg Ser Asn Met
                200                 205                 210

Glu Phe Leu Phe Asn Lys Thr Gly Trp Ala Phe Ala Ala Leu Cys
                215                 220                 225

Phe Val Leu Ala Met Thr Ser Gly Gln Met Trp Asn His Ile Arg
                230                 235                 240

Gly Pro Pro Tyr Ala His Lys Asn Pro His Thr Gly His Val Asn
                245                 250                 255

Tyr Ile His Gly Ser Ser Gln Ala Gln Phe Val Ala Glu Thr His
                260                 265                 270

Ile Val Leu Leu Phe Asn Gly Gly Val Thr Leu Gly Met Val Leu
                275                 280                 285

Leu Cys Glu Ala Ala Thr Ser Asp Met Asp Ile Gly Lys Arg Lys
                290                 295                 300

Ile Met Cys Val Ala Gly Ile Gly Leu Val Val Leu Phe Phe Ser
                305                 310                 315

Trp Met Leu Ser Ile Phe Arg Ser Lys Tyr His Gly Tyr Pro Tyr
                320                 325                 330

Ser Phe Leu Met Ser
                335
```

<210> 194
<211> 2476
<212> DNA
<213> Homo Sapien

<400> 194

```
aagcaaccaa actgcaagct ttgggagttg ttcgctgtcc ctgccctgct 50

ctgctaggga gagaacgcca gagggaggcg gctggcccgg cggcaggctc 100

tcagaaccgc taccggcgat gctactgctg tgggtgtcgg tggtcgcagc 150
```

cttggcgctg gcggtactgg cccccggagc aggggagcag aggcggagag 200
cagccaaagc gcccaatgtg gtgctggtcg tgagcgactc cttcgatgga 250
aggttaacat ttcatccagg aagtcaggta gtgaaacttc cttttatcaa 300
ctttatgaag acacgtggga cttcctttct gaatgcctac acaaactctc 350
caatttgttg cccatcacgc gcagcaatgt ggagtggcct cttcactcac 400
ttaacagaat cttggaataa ttttaagggt ctagatccaa attatacaac 450
atggatggat gtcatggaga ggcatggcta ccgaacacag aaatttggga 500
aactggacta tacttcagga catcactcca ttagtaatcg tgtggaagcg 550
tggacaagag atgttgcttt cttactcaga caagaaggca ggcccatggt 600
taatcttatc cgtaacagga ctaaagtcag agtgatggaa agggattggc 650
agaatacaga caaagcagta aactggttaa gaaaggaagc aattaattac 700
actgaaccat ttgttattta cttgggatta aatttaccac acccttaccc 750
ttcaccatct tctggagaaa attttggatc ttcaacattt cacacatctc 800
tttattggct tgaaaaagtg tctcatgatg ccatcaaaat cccaaagtgg 850
tcacctttgt cagaaatgca ccctgtagat tattactctt cttatacaaa 900
aaactgcact ggaagattta caaaaaaaga aattaagaat attagagcat 950
tttattatgc tatgtgtgct gagacagatg ccatgcttgg tgaaattatt 1000
ttggcccttc atcaattaga tcttcttcag aaaactattg tcatatactc 1050
ctcagaccat ggagagctgg ccatggaaca tcgacagttt tataaaatga 1100
gcatgtacga ggctagtgca catgttccgc ttttgatgat gggaccagga 1150
attaaagccg gcctacaagt atcaaatgtg gtttctcttg tggatattta 1200
ccctaccatg cttgatattg ctggaattcc tctgcctcag aacctgagtg 1250
gatactcttt gttgccgtta tcatcagaaa catttaagaa tgaacataaa 1300
gtcaaaaacc tgcatccacc ctggattctg agtgaattcc atggatgtaa 1350
tgtgaatgcc tccacctaca tgcttcgaac taaccactgg aaatatatag 1400
cctattcgga tggtgcatca atattgcctc aactctttga tctttcctcg 1450
gatccagatg aattaacaaa tgttgctgta aaatttccag aaattactta 1500
ttctttggat cagaagcttc attccattat aaactaccct aaagtttctg 1550
cttctgtcca ccagtataat aaagagcagt ttatcaagtg gaaacaaagt 1600
ataggacaga attattcaaa cgttatagca aatcttaggt ggcaccaaga 1650
ctggcagaag gaaccaagga agtatgaaaa tgcaattgat cagtggctta 1700
aaacccatat gaatccaaga gcagtttgaa caaaaagttt aaaaatagtg 1750

ttctagagat acatataaat atattacaag atcataatta tgtattttaa 1800

atgaaacagt tttaataatt accaagtttt ggccgggcac agtggctcac 1850

acctgtaatc ccaggacttt gggaggctga ggaaagcaga tcacaaggtc 1900

aagagattga gaccatcctg gccaacatgg tgaaaccctg tctctactaa 1950

aaatacaaaa attagctggg cgcggtggtg cacacctata gtctcagcta 2000

ctcagaggct gaggcaggag gatcgcttga acccgggagg cagcagttgc 2050

agtgagctga gattgcgcca ctgtactcca gcctggcaac agagtgagac 2100

tgtgtcgcaa aaaaataaaa ataaaataat aataattacc aatttttcat 2150

tattttgtaa gaatgtagtg tattttaaga taaaatgcca atgattataa 2200

aatcacatat tttcaaaaat ggttattatt taggcctttg tacaatttct 2250

aacaatttag tggaagtatc aaaaggattg aagcaaatac tgtaacagtt 2300

atgttccttt aaataataga gaatataaaa tattgtaata atatgtatca 2350

taaaatagtt gtatgtgagc atttgatggt gaaaaaaaaa aaaaaaaaaa 2400

aaaaaaaaaa aaaaaaaaaa aaaaaaaaaa aaaaaaaaaa aaaaaaaaaa 2450

aaaaaaaaaa aaaaaaaaaa aaaaaa 2476

<210> 195
<211> 536
<212> PRT
<213> Homo Sapien

<400> 195
Met Leu Leu Leu Trp Val Ser Val Val Ala Ala Leu Ala Leu Ala
1               5                   10                  15

Val Leu Ala Pro Gly Ala Gly Glu Gln Arg Arg Arg Ala Ala Lys
                20                  25                  30

Ala Pro Asn Val Val Leu Val Val Ser Asp Ser Phe Asp Gly Arg
                35                  40                  45

Leu Thr Phe His Pro Gly Ser Gln Val Val Lys Leu Pro Phe Ile
                50                  55                  60

Asn Phe Met Lys Thr Arg Gly Thr Ser Phe Leu Asn Ala Tyr Thr
                65                  70                  75

Asn Ser Pro Ile Cys Cys Pro Ser Arg Ala Ala Met Trp Ser Gly
                80                  85                  90

Leu Phe Thr His Leu Thr Glu Ser Trp Asn Asn Phe Lys Gly Leu
                95                  100                 105

Asp Pro Asn Tyr Thr Thr Trp Met Asp Val Met Glu Arg His Gly
                110                 115                 120

Tyr Arg Thr Gln Lys Phe Gly Lys Leu Asp Tyr Thr Ser Gly His
                125                 130                 135

His Ser Ile Ser Asn Arg Val Glu Ala Trp Thr Arg Asp Val Ala
140 145 150

Phe Leu Leu Arg Gln Glu Gly Arg Pro Met Val Asn Leu Ile Arg
155 160 165

Asn Arg Thr Lys Val Arg Val Met Glu Arg Asp Trp Gln Asn Thr
170 175 180

Asp Lys Ala Val Asn Trp Leu Arg Lys Glu Ala Ile Asn Tyr Thr
185 190 195

Glu Pro Phe Val Ile Tyr Leu Gly Leu Asn Leu Pro His Pro Tyr
200 205 210

Pro Ser Pro Ser Ser Gly Glu Asn Phe Gly Ser Ser Thr Phe His
215 220 225

Thr Ser Leu Tyr Trp Leu Glu Lys Val Ser His Asp Ala Ile Lys
230 235 240

Ile Pro Lys Trp Ser Pro Leu Ser Glu Met His Pro Val Asp Tyr
245 250 255

Tyr Ser Ser Tyr Thr Lys Asn Cys Thr Gly Arg Phe Thr Lys Lys
260 265 270

Glu Ile Lys Asn Ile Arg Ala Phe Tyr Tyr Ala Met Cys Ala Glu
275 280 285

Thr Asp Ala Met Leu Gly Glu Ile Ile Leu Ala Leu His Gln Leu
290 295 300

Asp Leu Leu Gln Lys Thr Ile Val Ile Tyr Ser Ser Asp His Gly
305 310 315

Glu Leu Ala Met Glu His Arg Gln Phe Tyr Lys Met Ser Met Tyr
320 325 330

Glu Ala Ser Ala His Val Pro Leu Leu Met Met Gly Pro Gly Ile
335 340 345

Lys Ala Gly Leu Gln Val Ser Asn Val Val Ser Leu Val Asp Ile
350 355 360

Tyr Pro Thr Met Leu Asp Ile Ala Gly Ile Pro Leu Pro Gln Asn
365 370 375

Leu Ser Gly Tyr Ser Leu Leu Pro Leu Ser Ser Glu Thr Phe Lys
380 385 390

Asn Glu His Lys Val Lys Asn Leu His Pro Pro Trp Ile Leu Ser
395 400 405

Glu Phe His Gly Cys Asn Val Asn Ala Ser Thr Tyr Met Leu Arg
410 415 420

Thr Asn His Trp Lys Tyr Ile Ala Tyr Ser Asp Gly Ala Ser Ile
425 430 435

Leu Pro Gln Leu Phe Asp Leu Ser Ser Asp Pro Asp Glu Leu Thr
440 445 450

Asn Val Ala Val Lys Phe Pro Glu Ile Thr Tyr Ser Leu Asp Gln
455 460 465

Lys Leu His Ser Ile Ile Asn Tyr Pro Lys Val Ser Ala Ser Val
470 475 480

His Gln Tyr Asn Lys Glu Gln Phe Ile Lys Trp Lys Gln Ser Ile
485 490 495

Gly Gln Asn Tyr Ser Asn Val Ile Ala Asn Leu Arg Trp His Gln
500 505 510

Asp Trp Gln Lys Glu Pro Arg Lys Tyr Glu Asn Ala Ile Asp Gln
515 520 525

Trp Leu Lys Thr His Met Asn Pro Arg Ala Val
530 535 536

<210> 196
<211> 771
<212> DNA
<213> Homo Sapien

<400> 196
ctccactgca accacccaga gccatggctc cccgaggctg catcgtagct 50

gtctttgcca ttttctgcat ctccaggctc ctctgctcac acggagcccc 100

agtggccccc atgactcctt acctgatgct gtgccagcca cacaagagat 150

gtggggacaa gttctacgac cccctgcagc actgttgcta tgatgatgcc 200

gtcgtgccct tggccaggac ccagacgtgt ggaaactgca ccttcagagt 250

ctgctttgag cagtgctgcc cctggacctt catggtgaag ctgataaacc 300

agaactgcga ctcagcccgg acctcggatg acaggctttg tcgcagtgtc 350

agctaatgga acatcagggg aacgatgact cctggattct ccttcctggg 400

tgggcctgga gaaagaggct ggtgttacct gagatctggg atgctgagtg 450

gctgtttggg ggccagagaa acacacactc aactgcccac ttcattctgt 500

gacctgtctg aggcccaccc tgcagctgcc ctgaggaggc ccacaggtcc 550

ccttctagaa ttctggacag catgagatgc gtgtgctgat gggggcccag 600

ggactctgaa ccctcctgat gacccctatg gccaacatca acccggcacc 650

accccaaggc tggctgggga acccttcacc cttctgtgag attttccatc 700

atctcaagtt ctcttctatc caggagcaaa gcacaggatc ataataaatt 750

tatgtacttt ataaatgaaa a 771

<210> 197
<211> 110
<212> PRT
<213> Homo Sapien

<400> 197

```
Met Ala Pro Arg Gly Cys Ile Val Ala Val Phe Ala Ile Phe Cys
  1               5                  10                  15

Ile Ser Arg Leu Leu Cys Ser His Gly Ala Pro Val Ala Pro Met
                 20                  25                  30

Thr Pro Tyr Leu Met Leu Cys Gln Pro His Lys Arg Cys Gly Asp
                 35                  40                  45

Lys Phe Tyr Asp Pro Leu Gln His Cys Cys Tyr Asp Asp Ala Val
                 50                  55                  60

Val Pro Leu Ala Arg Thr Gln Thr Cys Gly Asn Cys Thr Phe Arg
                 65                  70                  75

Val Cys Phe Glu Gln Cys Cys Pro Trp Thr Phe Met Val Lys Leu
                 80                  85                  90

Ile Asn Gln Asn Cys Asp Ser Ala Arg Thr Ser Asp Asp Arg Leu
                 95                 100                 105

Cys Arg Ser Val Ser
                110
```

<210> 198
<211> 693
<212> DNA
<213> Homo Sapien

<400> 198

```
ctagcctgcg ccaaggggta gtgagaccgc gcggcaacag cttgcggctg 50

cggggagctc ccgtgggcgc tccgctggct gtgcaggcgg ccatggattc 100

cttgcggaaa atgctgatct cagtcgcaat gctgggcgca ggggctggcg 150

tgggctacgc gctcctcgtt atcgtgaccc cgggagagcg gcggaagcag 200

gaaatgctaa aggagatgcc actgcaggac ccaaggagca gggaggaggc 250

ggccaggacc cagcagctat tgctggccac tctgcaggag gcagcgacca 300

cgcaggagaa cgtggcctgg aggaagaact ggatggttgg cggcgaaggc 350

ggcgccagcg ggaggtcacc gtgagaccgg acttgcctcc gtgggcgccg 400

gaccttggct tgggcgcagg aatccgaggc agcctttctc cttcgtgggc 450

ccagcggaga gtccggaccg agataccatg ccaggactct ccggggtcct 500

gtgagctgcc gtcgggtgag cacgtttccc ccaaaccctg gactgactgc 550

tttaaggtcc gcaaggcggg ccagggccga gacgcgagtc ggatgtggtg 600

aactgaaaga accaataaaa tcatgttcct ccaaaaaaaa aaaaaaaaaa 650

aaaaaaaaaa aaaaaaaaaa aaaaaaaaaa aaaaaaaaaa aaa 693
```

<210> 199
<211> 93
<212> PRT
<213> Homo Sapien

<400> 199

Met Asp Ser Leu Arg Lys Met Leu Ile Ser Val Ala Met Leu Gly
1               5                   10                  15

Ala Gly Ala Gly Val Gly Tyr Ala Leu Leu Val Ile Val Thr Pro
                20                  25                  30

Gly Glu Arg Arg Lys Gln Glu Met Leu Lys Glu Met Pro Leu Gln
                35                  40                  45

Asp Pro Arg Ser Arg Glu Glu Ala Ala Arg Thr Gln Gln Leu Leu
                50                  55                  60

Leu Ala Thr Leu Gln Glu Ala Ala Thr Thr Gln Glu Asn Val Ala
                65                  70                  75

Trp Arg Lys Asn Trp Met Val Gly Gly Glu Gly Gly Ala Ser Gly
                80                  85                  90

Arg Ser Pro
        93

<210> 200
<211> 1883
<212> DNA
<213> Homo Sapien

<400> 200

caggagagaa ggcaccgccc ccaccccgcc tccaaagcta accctcgggc 50

ttgaggggaa gaggctgact gtacgttcct tctactctgg caccactctc 100

caggctgcca tggggcccag cacccctctc ctcatcttgt tccttttgtc 150

atggtcggga cccctccaag gacagcagca ccaccttgtg gagtacatgg 200

aacgccgact agctgcttta gaggaacggc tggcccagtg ccaggaccag 250

agtagtcggc atgctgctga gctgcgggac ttcaagaaca agatgctgcc 300

actgctggag gtggcagaga aggagcggga ggcactcaga actgaggccg 350

acaccatctc cgggagagtg gatcgtctgg agcgggaggt agactatctg 400

gagacccaga acccagctct gccctgtgta gagtttgatg agaaggtgac 450

tggaggccct gggaccaaag gcaagggaag aaggaatgag aagtacgata 500

tggtgacaga ctgtggctac acaatctctc aagtgagatc aatgaagatt 550

ctgaagcgat ttggtggccc agctggtcta tggaccaagg atccactggg 600

gcaaacagag aagatctacg tgttagatgg gacacagaat gacacagcct 650

ttgtcttccc aaggctgcgt gacttcaccc ttgccatggc tgcccggaaa 700

gcttcccgag tccgggtgcc cttcccctgg gtaggcacag ggcagctggt 750

atatggtggc tttctttatt ttgctcggag gcctcctgga agacctggtg 800

gaggtggtga gatggagaac actttgcagc taatcaaatt ccacctggca 850

aaccgaacag tggtggacag ctcagtattc ccagcagagg ggctgatccc 900

cccctacggc ttgacagcag acacctacat cgacctggta gctgatgagg 950

aaggtctttg ggctgtctat gccacccggg aggatgacag gcacttgtgt 1000

ctggccaagt tagatccaca gacactggac acagagcagc agtgggacac 1050

accatgtccc agagagaatg ctgaggctgc ctttgtcatc tgtgggaccc 1100

tctatgtcgt ctataacacc cgtcctgcca gtcgggcccg catccagtgc 1150

tcctttgatg ccagcggcac cctgacccct gaacgggcag cactccctta 1200

ttttccccgc agatatggtg cccatgccag cctccgctat aacccccgag 1250

aacgccagct ctatgcctgg gatgatggct accagattgt ctataagctg 1300

gagatgagga agaaagagga ggaggtttga ggagctagcc ttgtttttg 1350

catctttctc actcccatac atttatatta tatccccact aaatttcttg 1400

ttcctcattc ttcaaatgtg ggccagttgt ggctcaaatc ctctatattt 1450

ttagccaatg gcaatcaaat tctttcagct cctttgtttc atacggaact 1500

ccagatcctg agtaatcctt ttagagcccg aagagtcaaa accctcaatg 1550

ttccctcctg ctctcctgcc ccatgtcaac aaatttcagg ctaaggatgc 1600

cccagaccca gggctctaac cttgtatgcg ggcaggccca gggagcaggc 1650

agcagtgttc ttcccctcag agtgacttgg ggagggagaa ataggaggag 1700

acgtccagct ctgtcctctc ttcctcactc ctcccttcag tgtcctgagg 1750

aacaggactt tctccacatt gttttgtatt gcaacatttt gcattaaaag 1800

gaaaatccac aaaaaaaaaa aaaaaaaaaa aaaaaaaaaa aaaaaaaaaa 1850

aaaaaaaaaa aaaaaaaaaa aaaaaaaaaa aaa 1883

<210> 201
<211> 406
<212> PRT
<213> Homo Sapien

<400> 201
Met Gly Pro Ser Thr Pro Leu Leu Ile Leu Phe Leu Leu Ser Trp
1 5 10 15

Ser Gly Pro Leu Gln Gly Gln Gln His His Leu Val Glu Tyr Met
20 25 30

Glu Arg Arg Leu Ala Ala Leu Glu Glu Arg Leu Ala Gln Cys Gln
35 40 45

Asp Gln Ser Ser Arg His Ala Ala Glu Leu Arg Asp Phe Lys Asn
50 55 60

Lys Met Leu Pro Leu Leu Glu Val Ala Glu Lys Glu Arg Glu Ala
65 70 75

Leu Arg Thr Glu Ala Asp Thr Ile Ser Gly Arg Val Asp Arg Leu
80 85 90

```
Glu Arg Glu Val Asp Tyr Leu Glu Thr Gln Asn Pro Ala Leu Pro
                95                  100                 105

Cys Val Glu Phe Asp Glu Lys Val Thr Gly Gly Pro Gly Thr Lys
                110                 115                 120

Gly Lys Gly Arg Arg Asn Glu Lys Tyr Asp Met Val Thr Asp Cys
                125                 130                 135

Gly Tyr Thr Ile Ser Gln Val Arg Ser Met Lys Ile Leu Lys Arg
                140                 145                 150

Phe Gly Gly Pro Ala Gly Leu Trp Thr Lys Asp Pro Leu Gly Gln
                155                 160                 165

Thr Glu Lys Ile Tyr Val Leu Asp Gly Thr Gln Asn Asp Thr Ala
                170                 175                 180

Phe Val Phe Pro Arg Leu Arg Asp Phe Thr Leu Ala Met Ala Ala
                185                 190                 195

Arg Lys Ala Ser Arg Val Arg Val Pro Phe Pro Trp Val Gly Thr
                200                 205                 210

Gly Gln Leu Val Tyr Gly Gly Phe Leu Tyr Phe Ala Arg Arg Pro
                215                 220                 225

Pro Gly Arg Pro Gly Gly Gly Gly Glu Met Glu Asn Thr Leu Gln
                230                 235                 240

Leu Ile Lys Phe His Leu Ala Asn Arg Thr Val Val Asp Ser Ser
                245                 250                 255

Val Phe Pro Ala Glu Gly Leu Ile Pro Pro Tyr Gly Leu Thr Ala
                260                 265                 270

Asp Thr Tyr Ile Asp Leu Val Ala Asp Glu Glu Gly Leu Trp Ala
                275                 280                 285

Val Tyr Ala Thr Arg Glu Asp Asp Arg His Leu Cys Leu Ala Lys
                290                 295                 300

Leu Asp Pro Gln Thr Leu Asp Thr Glu Gln Gln Trp Asp Thr Pro
                305                 310                 315

Cys Pro Arg Glu Asn Ala Glu Ala Ala Phe Val Ile Cys Gly Thr
                320                 325                 330

Leu Tyr Val Val Tyr Asn Thr Arg Pro Ala Ser Arg Ala Arg Ile
                335                 340                 345

Gln Cys Ser Phe Asp Ala Ser Gly Thr Leu Thr Pro Glu Arg Ala
                350                 355                 360

Ala Leu Pro Tyr Phe Pro Arg Arg Tyr Gly Ala His Ala Ser Leu
                365                 370                 375

Arg Tyr Asn Pro Arg Glu Arg Gln Leu Tyr Ala Trp Asp Asp Gly
                380                 385                 390
```

Tyr Gln Ile Val Tyr Lys Leu Glu Met Arg Lys Lys Glu Glu Glu
395 400 405

Val
406

<210> 202
<211> 1091
<212> DNA
<213> Homo Sapien

<400> 202
caagcaggtc atccccttgg tgaccttcaa agagaagcag agagggcaga 50

ggtgggggc acagggaaag ggtgacctct gagattcccc ttttccccca 100

gactttggaa gtgacccacc atggggctca gcatcttttt gctcctgtgt 150

gttcttgggc tcagccaggc agccacaccg aagattttca atggcactga 200

gtgtgggcgt aactcacagc cgtggcaggt ggggctgttt gagggcacca 250

gcctgcgctg cgggggtgtc cttattgacc acaggtgggt cctcacagcg 300

gctcactgca gcggcagcag gtactgggtg cgcctggggg aacacagcct 350

cagccagctc gactggaccg agcagatccg gcacagcggc ttctctgtga 400

cccatcccgg ctacctggga gcctcgacga gccacgagca cgacctccgg 450

ctgctgcggc tgcgcctgcc cgtccgcgta accagcagcg ttcaacccct 500

gcccctgccc aatgactgtg caaccgctgg caccgagtgc cacgtctcag 550

gctggggcat caccaaccac ccacggaacc cattcccgga tctgctccag 600

tgcctcaacc tctccatcgt ctcccatgcc acctgccatg gtgtgtatcc 650

cgggagaatc acgagcaaca tggtgtgtgc aggcggcgtc ccggggcagg 700

atgcctgcca gggtgattct gggggccccc tggtgtgtgg gggagtcctt 750

caaggtctgg tgtcctgggg gtctgtgggg ccctgtggac aagatggcat 800

ccctggagtc tacacctata tttgcaagta tgtggactgg atccggatga 850

tcatgaggaa caactgacct gtttcctcca cctccacccc cacccettaa 900

cttgggtacc cctctggccc tcagagcacc aatatctcct ccatcacttc 950

ccctagctcc actcttgttg gcctgggaac ttcttggaac tttaactcct 1000

gccagccctt ctaagaccca cgagcggggt gagagaagtg tgcaatagtc 1050

tggaataaat ataaatgaag gaggggcaaa aaaaaaaaaa a 1091

<210> 203
<211> 248
<212> PRT
<213> Homo Sapien

<400> 203
Met Gly Leu Ser Ile Phe Leu Leu Leu Cys Val Leu Gly Leu Ser
1 5 10 15

Gln Ala Ala Thr Pro Lys Ile Phe Asn Gly Thr Glu Cys Gly Arg
20 25 30

Asn Ser Gln Pro Trp Gln Val Gly Leu Phe Glu Gly Thr Ser Leu
35 40 45

Arg Cys Gly Gly Val Leu Ile Asp His Arg Trp Val Leu Thr Ala
50 55 60

Ala His Cys Ser Gly Ser Arg Tyr Trp Val Arg Leu Gly Glu His
65 70 75

Ser Leu Ser Gln Leu Asp Trp Thr Glu Gln Ile Arg His Ser Gly
80 85 90

Phe Ser Val Thr His Pro Gly Tyr Leu Gly Ala Ser Thr Ser His
95 100 105

Glu His Asp Leu Arg Leu Leu Arg Leu Arg Leu Pro Val Arg Val
110 115 120

Thr Ser Ser Val Gln Pro Leu Pro Leu Pro Asn Asp Cys Ala Thr
125 130 135

Ala Gly Thr Glu Cys His Val Ser Gly Trp Gly Ile Thr Asn His
140 145 150

Pro Arg Asn Pro Phe Pro Asp Leu Leu Gln Cys Leu Asn Leu Ser
155 160 165

Ile Val Ser His Ala Thr Cys His Gly Val Tyr Pro Gly Arg Ile
170 175 180

Thr Ser Asn Met Val Cys Ala Gly Gly Val Pro Gly Gln Asp Ala
185 190 195

Cys Gln Gly Asp Ser Gly Gly Pro Leu Val Cys Gly Gly Val Leu
200 205 210

Gln Gly Leu Val Ser Trp Gly Ser Val Gly Pro Cys Gly Gln Asp
215 220 225

Gly Ile Pro Gly Val Tyr Thr Tyr Ile Cys Lys Tyr Val Asp Trp
230 235 240

Ile Arg Met Ile Met Arg Asn Asn
245 248

<210> 204
<211> 907
<212> DNA
<213> Homo Sapien

<400> 204
gagcagtgtt ctgctggagc cgatgccaaa aaccatgcat ttcttattca 50

gattcattgt tttcttttat ctgtggggcc tttttactgc tcagagacaa 100

aagaaagagg agagcaccga agaagtgaaa atagaagttt tgcatcgtcc 150

agaaaactgc tctaagacaa gcaagaaggg agacctacta aatgcccatt 200

atgacggcta cctggctaaa gacggctcga aattctactg cagccggaca 250

caaaatgaag gccaccccaa atggtttgtt cttggtgttg ggcaagtcat 300

aaaaggccta gacattgcta tgacagatat gtgccctgga gaaaagcgaa 350

aagtagttat acccccttca tttgcatacg gaaaggaagg ctatgcagaa 400

ggcaagattc caccggatgc tacattgatt tttgagattg aactttatgc 450

tgtgaccaaa ggaccacgga gcattgagac atttaaacaa atagacatgg 500

acaatgacag gcagctctct aaagccgaga taaacctcta cttgcaaagg 550

gaatttgaaa aagatgagaa gccacgtgac aagtcatatc aggatgcagt 600

tttagaagat atttttaaga agaatgacca tgatggtgat ggcttcattt 650

ctcccaagga atacaatgta taccaacacg atgaactata gcatatttgt 700

atttctactt ttttttttta gctatttact gtactttatg tataaaacaa 750

agtcactttt ctccaagttg tatttgctat ttttccccta tgagaagata 800

ttttgatctc cccaatacat tgattttggt ataataaatg tgaggctgtt 850

ttgcaaactt aaaaaaaaaa aaaaaaaaaa aaaaaaaaaa aaaaaaaaaa 900

aaaaaaa 907

<210> 205
<211> 222
<212> PRT
<213> Homo Sapien

<400> 205

Met Pro Lys Thr Met His Phe Leu Phe Arg Phe Ile Val Phe Phe
1 5 10 15

Tyr Leu Trp Gly Leu Phe Thr Ala Gln Arg Gln Lys Lys Glu Glu
20 25 30

Ser Thr Glu Glu Val Lys Ile Glu Val Leu His Arg Pro Glu Asn
35 40 45

Cys Ser Lys Thr Ser Lys Lys Gly Asp Leu Leu Asn Ala His Tyr
50 55 60

Asp Gly Tyr Leu Ala Lys Asp Gly Ser Lys Phe Tyr Cys Ser Arg
65 70 75

Thr Gln Asn Glu Gly His Pro Lys Trp Phe Val Leu Gly Val Gly
80 85 90

Gln Val Ile Lys Gly Leu Asp Ile Ala Met Thr Asp Met Cys Pro
95 100 105

Gly Glu Lys Arg Lys Val Val Ile Pro Pro Ser Phe Ala Tyr Gly
110 115 120

Lys Glu Gly Tyr Ala Glu Gly Lys Ile Pro Pro Asp Ala Thr Leu
125 130 135

Ile Phe Glu Ile Glu Leu Tyr Ala Val Thr Lys Gly Pro Arg Ser
140 145 150

Ile Glu Thr Phe Lys Gln Ile Asp Met Asp Asn Asp Arg Gln Leu
155 160 165

Ser Lys Ala Glu Ile Asn Leu Tyr Leu Gln Arg Glu Phe Glu Lys
170 175 180

Asp Glu Lys Pro Arg Asp Lys Ser Tyr Gln Asp Ala Val Leu Glu
185 190 195

Asp Ile Phe Lys Lys Asn Asp His Asp Gly Asp Gly Phe Ile Ser
200 205 210

Pro Lys Glu Tyr Asn Val Tyr Gln His Asp Glu Leu
215 220 222

<210> 206
<211> 22
<212> DNA
<213> Artificial Sequence

<220>
<223> Synthetic Oligonucleotide Probe

<400> 206
gtgttctgct ggagccgatg cc 22

<210> 207
<211> 18
<212> DNA
<213> Artificial Sequence

<220>
<223> Synthetic Oligonucleotide Probe

<400> 207
gacatggaca atgacagg 18

<210> 208
<211> 18
<212> DNA
<213> Artificial Sequence

<220>
<223> Synthetic Oligonucleotide Probe

<400> 208
cctttcagga tgtaggag 18

<210> 209
<211> 18
<212> DNA
<213> Artificial Sequence

<220>
<223> Synthetic Oligonucleotide Probe

<400> 209

gatgtctgcc accccaag 18

<210> 210
<211> 27
<212> DNA
<213> Artificial Sequence

<220>
<223> Synthetic Oligonucleotide Probe

<400> 210
gcatcctgat atgacttgtc acgtggc 27

<210> 211
<211> 24
<212> DNA
<213> Artificial Sequence

<220>
<223> Synthetic Oligonucleotide Probe

<400> 211
tacaagaggg aagaggagtt gcac 24

<210> 212
<211> 52
<212> DNA
<213> Artificial Sequence

<220>
<223> Synthetic Oligonucleotide Probe

<400> 212
gcccattatg acggctacct ggctaaagac ggctcgaaat tctactgcag 50

cc 52

<210> 213
<211> 1346
<212> DNA
<213> Homo Sapien

<400> 213
gaaagaatgt tgtggctgct ctttttctg gtgactgcca ttcatgctga 50

actctgtcaa ccaggtgcag aaaatgcttt taaagtgaga cttagtatca 100

gaacagctct gggagataaa gcatatgcct gggataccaa tgaagaatac 150

ctcttcaaag cgatggtagc tttctccatg agaaaagttc ccaacagaga 200

agcaacagaa atttcccatg tcctactttg caatgtaacc cagagggtat 250

cattctggtt tgtggttaca gacccttcaa aaaatcacac ccttcctgct 300

gttgaggtgc aatcagccat aagaatgaac aagaaccgga tcaacaatgc 350

cttctttcta aatgaccaaa ctctggaatt tttaaaaatc ccttccacac 400

ttgcaccacc catggaccca tctgtgccca tctggattat tatatttggt 450

gtgatatttt gcatcatcat agttgcaatt gcactactga ttttatcagg 500

gatctggcaa cgtagaagaa agaacaaaga accatctgaa gtggatgacg 550

ctgaagataa gtgtgaaaac atgatcacaa ttgaaaatgg catcccctct 600

gatcccctgg acatgaaggg gggcatatta atgatgcctt catgacagag 650

gatgagaggc tcacccctct ctgaagggct gttgttctgc ttcctcaaga 700

aattaaacat ttgtttctgt gtgactgctg agcatcctga aataccaaga 750

gcagatcata tattttgttt caccattctt cttttgtaat aaattttgaa 800

tgtgcttgaa agtgaaaagc aatcaattat acccaccaac accactgaaa 850

tcataagcta ttcacgactc aaaatattct aaaatatttt tctgacagta 900

tagtgtataa atgtggtcat gtggtatttg tagttattga tttaagcatt 950

tttagaaata agatcaggca tatgtatata ttttcacact tcaaagacct 1000

aaggaaaaat aaattttcca gtggagaata catataatat ggtgtagaaa 1050

tcattgaaaa tggatccttt ttgacgatca cttatatcac tctgtatatg 1100

actaagtaaa caaaagtgag aagtaattat tgtaaatgga tggataaaaa 1150

tggaattact catatacagg gtggaatttt atcctgttat cacaccaaca 1200

gttgattata tattttctga atatcagccc ctaataggac aattctattt 1250

gttgaccatt tctacaattt gtaaaagtcc aatctgtgct aacttaataa 1300

agtaataatc atctcttttt aaaaaaaaaa aaaaaaaaaa aaaaaa 1346

<210> 214
<211> 212
<212> PRT
<213> Homo Sapien

<400> 214
```
Met Leu Trp Leu Leu Phe Phe Leu Val Thr Ala Ile His Ala Glu
 1               5                  10                  15

Leu Cys Gln Pro Gly Ala Glu Asn Ala Phe Lys Val Arg Leu Ser
                20                  25                  30

Ile Arg Thr Ala Leu Gly Asp Lys Ala Tyr Ala Trp Asp Thr Asn
                35                  40                  45

Glu Glu Tyr Leu Phe Lys Ala Met Val Ala Phe Ser Met Arg Lys
                50                  55                  60

Val Pro Asn Arg Glu Ala Thr Glu Ile Ser His Val Leu Leu Cys
                65                  70                  75

Asn Val Thr Gln Arg Val Ser Phe Trp Phe Val Val Thr Asp Pro
                80                  85                  90

Ser Lys Asn His Thr Leu Pro Ala Val Glu Val Gln Ser Ala Ile
                95                 100                 105

Arg Met Asn Lys Asn Arg Ile Asn Asn Ala Phe Phe Leu Asn Asp
               110                 115                 120
```

Gln Thr Leu Glu Phe Leu Lys Ile Pro Ser Thr Leu Ala Pro Pro
125 130 135

Met Asp Pro Ser Val Pro Ile Trp Ile Ile Ile Phe Gly Val Ile
140 145 150

Phe Cys Ile Ile Ile Val Ala Ile Ala Leu Leu Ile Leu Ser Gly
155 160 165

Ile Trp Gln Arg Arg Arg Lys Asn Lys Glu Pro Ser Glu Val Asp
170 175 180

Asp Ala Glu Asp Lys Cys Glu Asn Met Ile Thr Ile Glu Asn Gly
185 190 195

Ile Pro Ser Asp Pro Leu Asp Met Lys Gly Gly Ile Leu Met Met
200 205 210

Pro Ser
212

<210> 215
<211> 1181
<212> DNA
<213> Homo Sapien

<400> 215
aagaccctct ctttcgctgt ttgagagtct ctcggctcaa ggaccgggag 50

gtaagaggtt tgggactgcc ccggcaactc cagggtgtct ggtccacgac 100

ctatcctagg cgccatgggt gtgataggta tacagctggt tgttaccatg 150

gtgatggcca gtgtcatgca gaagattata cctcactatt ctcttgctcg 200

atggctactc tgtaatggca gtttgaggtg gtatcaacat cctacagaag 250

aagaattaag aattcttgca gggaaacaac aaaaagggaa aaccaaaaaa 300

gataggaaat ataatggtca cattgaaagt aagccattaa ccattccaaa 350

ggatattgac cttcatctag aaacaaagtc agttacagaa gtggatactt 400

tagcattgca ttactttcca gaataccagt ggctggtgga tttcacagtg 450

gctgctacag ttgtgtatct agtaactgaa gtctactaca attttatgaa 500

gcctacacag gaaatgaata tcagcttagt ctggtgccta cttgttttgt 550

cttttgcaat caaagttcta ttttcattaa ctacacacta ttttaaagta 600

gaagatggtg gtgaaagatc tgtttgtgtc acctttggat ttttttttctt 650

tgtcaaagca atggcagtgt tgattgtaac agaaaattat ctggaatttg 700

gacttgaaac agggtttaca aatttttcag acagtgcgat gcagtttctt 750

gaaaagcaag gtttagaatc tcagagtcct gtttcaaaac ttactttcaa 800

atttttcctg gctattttct gttcattcat tggggctttt ttgacatttc 850

ctggattacg actggctcaa atgcatctgg atgccctgaa tttggcaaca 900

gaaaaaatta cacaaacttt acttcatatc aacttcttgg cacctttatt 950

tatggttttg ctctgggtaa aaccaatcac caaagactac attatgaacc 1000

caccactggg caaagaaatt tccccatctg gaagatgaag ataatagtat 1050

ctaactcaca aggttatcat tggaataaat gaaagaacac atgtaatgca 1100

accagctgga attaagtgct taataaatgt tcttttcact gctttgcctc 1150

atcagaatta aaatagaaat acttgactag t 1181

<210> 216
<211> 307
<212> PRT
<213> Homo Sapien

<400> 216

Met Gly Val Ile Gly Ile Gln Leu Val Val Thr Met Val Met Ala
1 5 10 15

Ser Val Met Gln Lys Ile Ile Pro His Tyr Ser Leu Ala Arg Trp
20 25 30

Leu Leu Cys Asn Gly Ser Leu Arg Trp Tyr Gln His Pro Thr Glu
35 40 45

Glu Glu Leu Arg Ile Leu Ala Gly Lys Gln Gln Lys Gly Lys Thr
50 55 60

Lys Lys Asp Arg Lys Tyr Asn Gly His Ile Glu Ser Lys Pro Leu
65 70 75

Thr Ile Pro Lys Asp Ile Asp Leu His Leu Glu Thr Lys Ser Val
80 85 90

Thr Glu Val Asp Thr Leu Ala Leu His Tyr Phe Pro Glu Tyr Gln
95 100 105

Trp Leu Val Asp Phe Thr Val Ala Ala Thr Val Val Tyr Leu Val
110 115 120

Thr Glu Val Tyr Tyr Asn Phe Met Lys Pro Thr Gln Glu Met Asn
125 130 135

Ile Ser Leu Val Trp Cys Leu Leu Val Leu Ser Phe Ala Ile Lys
140 145 150

Val Leu Phe Ser Leu Thr Thr His Tyr Phe Lys Val Glu Asp Gly
155 160 165

Gly Glu Arg Ser Val Cys Val Thr Phe Gly Phe Phe Phe Phe Val
170 175 180

Lys Ala Met Ala Val Leu Ile Val Thr Glu Asn Tyr Leu Glu Phe
185 190 195

Gly Leu Glu Thr Gly Phe Thr Asn Phe Ser Asp Ser Ala Met Gln
200 205 210

Phe Leu Glu Lys Gln Gly Leu Glu Ser Gln Ser Pro Val Ser Lys
215 220 225

Leu Thr Phe Lys Phe Phe Leu Ala Ile Phe Cys Ser Phe Ile Gly
230 235 240

Ala Phe Leu Thr Phe Pro Gly Leu Arg Leu Ala Gln Met His Leu
245 250 255

Asp Ala Leu Asn Leu Ala Thr Glu Lys Ile Thr Gln Thr Leu Leu
260 265 270

His Ile Asn Phe Leu Ala Pro Leu Phe Met Val Leu Leu Trp Val
275 280 285

Lys Pro Ile Thr Lys Asp Tyr Ile Met Asn Pro Pro Leu Gly Lys
290 295 300

Glu Ile Ser Pro Ser Gly Arg
305 307

<210> 217
<211> 904
<212> DNA
<213> Homo Sapien

<400> 217
gaagtagagg tgttgtgctg agcggcgctc ggcgaactgt gtggaccgtc 50

tgctgggact ccggccctgc gtccgctcag ccccgtggcc ccgcgcacct 100

actgccatgg agacgcggcc tcgtctcggg gccacctgtt tgctgggctt 150

cagtttcctg ctcctcgtca tctcttctga tggacataat gggcttggaa 200

agggttttgg agatcatatt cattggagga cactggaaga tgggaagaaa 250

gaagcagctg ccagtggact gcccctgatg gtgattattc ataaatcctg 300

gtgtggagct tgcaaagctc taaagcccaa atttgcagaa tctacggaaa 350

tttcagaact ctcccataat tttgttatgg taaatcttga ggatgaagag 400

gaacccaaag atgaagattt cagccctgac gggggttata ttccacgaat 450

cctttttctg gatcccagtg gcaaggtgca tcctgaaatc atcaatgaga 500

atggaaaccc cagctacaag tatttttatg tcagtgccga gcaagttgtt 550

caggggatga aggaagctca ggaaaggctg acgggtgatg ccttcagaaa 600

gaaacatctt gaagatgaat tgtaacatga atgtgcccct tctttcatca 650

gagttagtgt tctggaagga aagcagcagg gaagggaata ttgaggaatc 700

atctagaaca attaagccga ccaggaaacc tcattcctac ctacactgga 750

aggagcgctc tcactgtgga agagttctgc taacagaagc tggtctgcat 800

gtttgtggat ccagcggaga gtggcagact ttcttctcct tttccctctc 850

acctaaatgt caacttgtca ttgaatgtaa agaatgaaac cttctgacac 900

aaaa 904

<210> 218

<211> 172
<212> PRT
<213> Homo Sapien

<400> 218

```
Met Glu Thr Arg Pro Arg Leu Gly Ala Thr Cys Leu Leu Gly Phe
1               5                   10                  15

Ser Phe Leu Leu Leu Val Ile Ser Ser Asp Gly His Asn Gly Leu
                20                  25                  30

Gly Lys Gly Phe Gly Asp His Ile His Trp Arg Thr Leu Glu Asp
                35                  40                  45

Gly Lys Lys Glu Ala Ala Ala Ser Gly Leu Pro Leu Met Val Ile
                50                  55                  60

Ile His Lys Ser Trp Cys Gly Ala Cys Lys Ala Leu Lys Pro Lys
                65                  70                  75

Phe Ala Glu Ser Thr Glu Ile Ser Glu Leu Ser His Asn Phe Val
                80                  85                  90

Met Val Asn Leu Glu Asp Glu Glu Glu Pro Lys Asp Glu Asp Phe
                95                  100                 105

Ser Pro Asp Gly Gly Tyr Ile Pro Arg Ile Leu Phe Leu Asp Pro
                110                 115                 120

Ser Gly Lys Val His Pro Glu Ile Ile Asn Glu Asn Gly Asn Pro
                125                 130                 135

Ser Tyr Lys Tyr Phe Tyr Val Ser Ala Glu Gln Val Val Gln Gly
                140                 145                 150

Met Lys Glu Ala Gln Glu Arg Leu Thr Gly Asp Ala Phe Arg Lys
                155                 160                 165

Lys His Leu Glu Asp Glu Leu
                170         172
```

<210> 219
<211> 1630
<212> DNA
<213> Homo Sapien

<400> 219

```
cggctcgagt gcagctgtgg ggagatttca gtgcattgcc tcccctgggt 50

gctcttcatc ttggatttga aagttgagag cagcatgttt tgcccactga 100

aactcatcct gctgccagtg ttactggatt attccttggg cctgaatgac 150

ttgaatgttt ccccgcctga gctaacagtc catgtgggtg attcagctct 200

gatgggatgt gttttccaga gcacagaaga caaatgtata ttcaagatag 250

actggactct gtcaccagga gagcacgcca aggacgaata tgtgctatac 300

tattactcca atctcagtgt gcctattggg cgcttccaga accgcgtaca 350

cttgatgggg gacatcttat gcaatgatgg ctctctcctg ctccaagatg 400
```

tgcaagaggc tgaccaggga acctatatct gtgaaatccg cctcaaaggg 450

gagagccagg tgttcaagaa ggcggtggta ctgcatgtgc ttccagagga 500

gcccaaagag ctcatggtcc atgtgggtgg attgattcag atgggatgtg 550

ttttccagag cacagaagtg aaacacgtga ccaaggtaga atggatattt 600

tcaggacggc gcgcaaagga ggagattgta tttcgttact accacaaact 650

caggatgtct gtggagtact cccagagctg gggccacttc cagaatcgtg 700

tgaacctggt gggggacatt ttccgcaatg acggttccat catgcttcaa 750

ggagtgaggg agtcagatgg aggaaactac acctgcagta tccacctagg 800

gaacctggtg ttcaagaaaa ccattgtgct gcatgtcagc ccggaagagc 850

ctcgaacact ggtgaccccg gcagccctga ggcctctggt cttgggtggt 900

aatcagttgg tgatcattgt gggaattgtc tgtgccacaa tcctgctgct 950

ccctgttctg atattgatcg tgaagaagac ctgtggaaat aagagttcag 1000

tgaattctac agtcttggtg aagaacacga agaagactaa tccagagata 1050

aaagaaaaac cctgccattt tgaaagatgt gaaggggaga aacacattta 1100

ctccccaata attgtacggg aggtgatcga ggaagaagaa ccaagtgaaa 1150

aatcagaggc cacctacatg accatgcacc cagtttggcc ttctctgagg 1200

tcagatcgga acaactcact tgaaaaaaag tcaggtgggg gaatgccaaa 1250

aacacagcaa gccttttgag aagaatggag agtcccttca tctcagcagc 1300

ggtggagact ctctcctgtg tgtgtcctgg gccactctac cagtgatttc 1350

agactcccgc tctcccagct gtcctcctgt ctcattgttt ggtcaataca 1400

ctgaagatgg agaatttgga gcctggcaga gagactggac agctctggag 1450

gaacaggcct gctgagggga ggggagcatg gacttggcct ctggagtggg 1500

acactggccc tgggaaccag gctgagctga gtggcctcaa accccccgtt 1550

ggatcagacc ctcctgtggg cagggttctt agtggatgag ttactgggaa 1600

gaatcagaga taaaaaccaa cccaaatcaa 1630

<210> 220
<211> 394
<212> PRT
<213> Homo Sapien

<400> 220
Met Phe Cys Pro Leu Lys Leu Ile Leu Leu Pro Val Leu Leu Asp
1 5 10 15

Tyr Ser Leu Gly Leu Asn Asp Leu Asn Val Ser Pro Pro Glu Leu
20 25 30

```
Thr Val His Val Gly Asp Ser Ala Leu Met Gly Cys Val Phe Gln
                35                  40                  45

Ser Thr Glu Asp Lys Cys Ile Phe Lys Ile Asp Trp Thr Leu Ser
                50                  55                  60

Pro Gly Glu His Ala Lys Asp Glu Tyr Val Leu Tyr Tyr Tyr Ser
                65                  70                  75

Asn Leu Ser Val Pro Ile Gly Arg Phe Gln Asn Arg Val His Leu
                80                  85                  90

Met Gly Asp Ile Leu Cys Asn Asp Gly Ser Leu Leu Leu Gln Asp
                95                  100                 105

Val Gln Glu Ala Asp Gln Gly Thr Tyr Ile Cys Glu Ile Arg Leu
                110                 115                 120

Lys Gly Glu Ser Gln Val Phe Lys Lys Ala Val Val Leu His Val
                125                 130                 135

Leu Pro Glu Glu Pro Lys Glu Leu Met Val His Val Gly Gly Leu
                140                 145                 150

Ile Gln Met Gly Cys Val Phe Gln Ser Thr Glu Val Lys His Val
                155                 160                 165

Thr Lys Val Glu Trp Ile Phe Ser Gly Arg Arg Ala Lys Glu Glu
                170                 175                 180

Ile Val Phe Arg Tyr Tyr His Lys Leu Arg Met Ser Val Glu Tyr
                185                 190                 195

Ser Gln Ser Trp Gly His Phe Gln Asn Arg Val Asn Leu Val Gly
                200                 205                 210

Asp Ile Phe Arg Asn Asp Gly Ser Ile Met Leu Gln Gly Val Arg
                215                 220                 225

Glu Ser Asp Gly Gly Asn Tyr Thr Cys Ser Ile His Leu Gly Asn
                230                 235                 240

Leu Val Phe Lys Lys Thr Ile Val Leu His Val Ser Pro Glu Glu
                245                 250                 255

Pro Arg Thr Leu Val Thr Pro Ala Ala Leu Arg Pro Leu Val Leu
                260                 265                 270

Gly Gly Asn Gln Leu Val Ile Ile Val Gly Ile Val Cys Ala Thr
                275                 280                 285

Ile Leu Leu Leu Pro Val Leu Ile Leu Ile Val Lys Lys Thr Cys
                290                 295                 300

Gly Asn Lys Ser Ser Val Asn Ser Thr Val Leu Val Lys Asn Thr
                305                 310                 315

Lys Lys Thr Asn Pro Glu Ile Lys Glu Lys Pro Cys His Phe Glu
                320                 325                 330

Arg Cys Glu Gly Glu Lys His Ile Tyr Ser Pro Ile Ile Val Arg
                335                 340                 345
```

Glu Val Ile Glu Glu Glu Glu Pro Ser Glu Lys Ser Glu Ala Thr
350 355 360

Tyr Met Thr Met His Pro Val Trp Pro Ser Leu Arg Ser Asp Arg
365 370 375

Asn Asn Ser Leu Glu Lys Lys Ser Gly Gly Gly Met Pro Lys Thr
380 385 390

Gln Gln Ala Phe
394

<210> 221
<211> 496
<212> DNA
<213> Homo Sapien

<220>
<221> unsure
<222> 396
<223> unknown base

<400> 221
tctgcctcca ctgctctgtg ctgggatcat ggaacttgca ctgctgtgtg 50

ggctggtggt gatggctggt gtgattccaa tccagggcgg gatcctgaac 100

ctgaacaaga tggtcaagca agtgactggg aaaatgccca tcctctccta 150

ctggccctac ggctgtcact gcggactagg tggcagaggc caacccaaag 200

atgccacgga ctggtgctgc cagacccatg actgctgcta tgaccacctg 250

aagacccagg ggtgcggcat ctacaaggac aacaacaaaa gcagcataca 300

ttgtatggat ttatctcaac gctattgttt aatggctgtg tttaatgtga 350

tctatctgga aaatgaggac tccgaataaa aagctattac tawttnaaaa 400

aaaaaaaaaa aaaaaaaaaa aaaaaaaaaa aaaaaaaaaa aaaaaaaaaa 450

aaaaaaaaaa aaaaaaaaaa aaaaaaaaaa aaaaaaaaaa aaaaaa 496

<210> 222
<211> 116
<212> PRT
<213> Homo Sapien

<400> 222
Met Glu Leu Ala Leu Leu Cys Gly Leu Val Val Met Ala Gly Val
1 5 10 15

Ile Pro Ile Gln Gly Gly Ile Leu Asn Leu Asn Lys Met Val Lys
20 25 30

Gln Val Thr Gly Lys Met Pro Ile Leu Ser Tyr Trp Pro Tyr Gly
35 40 45

Cys His Cys Gly Leu Gly Gly Arg Gly Gln Pro Lys Asp Ala Thr
50 55 60

Asp Trp Cys Cys Gln Thr His Asp Cys Cys Tyr Asp His Leu Lys
65 70 75

```
Thr Gln Gly Cys Gly Ile Tyr Lys Asp Asn Asn Lys Ser Ser Ile
                80                  85                  90

His Cys Met Asp Leu Ser Gln Arg Tyr Cys Leu Met Ala Val Phe
                95                  100                 105

Asn Val Ile Tyr Leu Glu Asn Glu Asp Ser Glu
                110                 115 116
```

<210> 223
<211> 24
<212> DNA
<213> Artificial Sequence

<220>
<223> Synthetic Oligonucleotide Probe

<400> 223
ctgcctccac tgctctgtgc tggg 24

<210> 224
<211> 24
<212> DNA
<213> Artificial Sequence

<220>
<223> Synthetic Oligonucleotide Probe

<400> 224
cagagcagtg gatgttcccc tggg 24

<210> 225
<211> 45
<212> DNA
<213> Artificial Sequence

<220>
<223> Synthetic Oligonucleotide probe

<400> 225
ctgaacaaga tggtcaagca agtgactggg aaaatgccca tcctc 45

<210> 226
<211> 1835
<212> DNA
<213> Homo Sapien

<400> 226
cacaagcatc ttaatttgaa tccacaaagt ttcatgtaat gaaaagaaat 50

acataatttt aattcaaccc gagtgttttc caagaagatt gtatttgctt 100

aaattgctac agtaattcaa gagacagccc tgtctggaca cagagttact 150

gtggattttt aagagactca gttaaagaat ttaggaattt ctgattcatt 200

taaaggattt acaaattcat caacccctga aaactaaagc aaattgaaca 250

ggaaaaaaaa aaagaagatg ggttttttaa gtccaatata tgttattttc 300

ttcttttttg gagtcaaagt acattgccaa tatgaaactt atcagtggga 350

tgaagactat gaccaagagc cagatgatga ttaccaaaca ggattcccat 400

ttcgtcaaaa tgtagactac ggagttcctt ttcatcagta tactttaggc 450
tgtgtcagtg aatgcttctg tccaactaac tttccatcat caatgtactg 500
tgataatcgc aaactcaaga ctatcccaaa tattccgatg cacattcagc 550
aactctacct tcagttcaat gaaattgagg ctgtgactgc aaattcattc 600
atcaatgcaa ctcatcttaa agaaattaac ctcagccaca acaaaattaa 650
atctcaaaag attgattatg gtgtgtttgc taagcttcca aatctactac 700
aacttcatct agagcataat aatttagaag aatttccatt tcctcttcct 750
aaatctctgg aaagactcct tcttggttac aatgaaatct ccaaactgca 800
gacaaatgct atggatgggc tagtaaactt gaccatgctt gatctctgtt 850
ataattatct tcatgattct ctgctaaaag acaaaatctt tgccaaaatg 900
gaaaaactaa tgcagctcaa cctctgcagt aacagattag aatcaatgcc 950
tcctggtttg ccttcttcac ttatgtatct gtctttagaa aataattcaa 1000
tttcttctat acccgaaaaa tacttcgaca aacttccaaa acttcatact 1050
ctaagaatgt cacacaacaa actacaagac atcccatata atatttttaa 1100
tcttcccaac attgtagaac tcagtgttgg acacaacaaa ttgaagcaag 1150
cattctatat tccaagaaat ttggaacacc tatacctaca aaataatgaa 1200
atagaaaaga tgaatcttac agtgatgtgt ccttctattg acccactaca 1250
ttaccaccat ttaacataca ttcgtgtgga ccaaaataaa ctaaaagaac 1300
caataagctc atacatcttc ttctgcttcc ctcatataca cactatttat 1350
tatggtgaac aacgaagcac taatggtcaa acaatacaac taaagacaca 1400
agttttcagg agatttccag atgatgatga tgaaagtgaa gatcacgatg 1450
atcctgacaa tgctcatgag agcccagaac aagaaggagc agaagggcac 1500
tttgaccttc attattatga aaatcaagaa tagcaagaaa ctatataggt 1550
atacacttac gacttcacaa aacctatact taatatagta aatctaagta 1600
aacatgtatt actcaaagta atatatttag aattatgtat tagtataaga 1650
tcagaattga atttaagttg ttggtgacat ctgcatcatt tcataggatt 1700
agaacttact caaaataatg taaatcttta aaaatataaa ttagaatgac 1750
aagtgggaat cataaattaa acgttaatgg tttcttatgc tctttttaaa 1800
tatagaaata tcatgttaaa gaaaaaaaaa aaaaa 1835

<210> 227
<211> 421
<212> PRT
<213> Homo Sapien

<400> 227

```
Met Gly Phe Leu Ser Pro Ile Tyr Val Ile Phe Phe Phe Phe Gly
 1               5                  10                  15

Val Lys Val His Cys Gln Tyr Glu Thr Tyr Gln Trp Asp Glu Asp
                20                  25                  30

Tyr Asp Gln Glu Pro Asp Asp Asp Tyr Gln Thr Gly Phe Pro Phe
                35                  40                  45

Arg Gln Asn Val Asp Tyr Gly Val Pro Phe His Gln Tyr Thr Leu
                50                  55                  60

Gly Cys Val Ser Glu Cys Phe Cys Pro Thr Asn Phe Pro Ser Ser
                65                  70                  75

Met Tyr Cys Asp Asn Arg Lys Leu Lys Thr Ile Pro Asn Ile Pro
                80                  85                  90

Met His Ile Gln Gln Leu Tyr Leu Gln Phe Asn Glu Ile Glu Ala
                95                 100                 105

Val Thr Ala Asn Ser Phe Ile Asn Ala Thr His Leu Lys Glu Ile
               110                 115                 120

Asn Leu Ser His Asn Lys Ile Lys Ser Gln Lys Ile Asp Tyr Gly
               125                 130                 135

Val Phe Ala Lys Leu Pro Asn Leu Leu Gln Leu His Leu Glu His
               140                 145                 150

Asn Asn Leu Glu Glu Phe Pro Phe Pro Leu Pro Lys Ser Leu Glu
               155                 160                 165

Arg Leu Leu Leu Gly Tyr Asn Glu Ile Ser Lys Leu Gln Thr Asn
               170                 175                 180

Ala Met Asp Gly Leu Val Asn Leu Thr Met Leu Asp Leu Cys Tyr
               185                 190                 195

Asn Tyr Leu His Asp Ser Leu Leu Lys Asp Lys Ile Phe Ala Lys
               200                 205                 210

Met Glu Lys Leu Met Gln Leu Asn Leu Cys Ser Asn Arg Leu Glu
               215                 220                 225

Ser Met Pro Pro Gly Leu Pro Ser Ser Leu Met Tyr Leu Ser Leu
               230                 235                 240

Glu Asn Asn Ser Ile Ser Ser Ile Pro Glu Lys Tyr Phe Asp Lys
               245                 250                 255

Leu Pro Lys Leu His Thr Leu Arg Met Ser His Asn Lys Leu Gln
               260                 265                 270

Asp Ile Pro Tyr Asn Ile Phe Asn Leu Pro Asn Ile Val Glu Leu
               275                 280                 285

Ser Val Gly His Asn Lys Leu Lys Gln Ala Phe Tyr Ile Pro Arg
               290                 295                 300

Asn Leu Glu His Leu Tyr Leu Gln Asn Asn Glu Ile Glu Lys Met
               305                 310                 315
```

```
Asn Leu Thr Val Met Cys Pro Ser Ile Asp Pro Leu His Tyr His
                320                 325                 330

His Leu Thr Tyr Ile Arg Val Asp Gln Asn Lys Leu Lys Glu Pro
                335                 340                 345

Ile Ser Ser Tyr Ile Phe Phe Cys Phe Pro His Ile His Thr Ile
                350                 355                 360

Tyr Tyr Gly Glu Gln Arg Ser Thr Asn Gly Gln Thr Ile Gln Leu
                365                 370                 375

Lys Thr Gln Val Phe Arg Arg Phe Pro Asp Asp Asp Asp Glu Ser
                380                 385                 390

Glu Asp His Asp Asp Pro Asp Asn Ala His Glu Ser Pro Glu Gln
                395                 400                 405

Glu Gly Ala Glu Gly His Phe Asp Leu His Tyr Tyr Glu Asn Gln
                410                 415                 420

Glu
421
```

<210> 228
<211> 21
<212> DNA
<213> Artificial Sequence

<220>
<223> Synthetic Oligonucleotide Probe

<400> 228
tcacgatgat cctgacaatg c 21

<210> 229
<211> 24
<212> DNA
<213> Artificial Sequence

<220>
<223> Synthetic Oligonucleotide Probe

<400> 229
aataatgaag gtcaaagtgc cctt 24

<210> 230
<211> 27
<212> DNA
<213> Artificial Sequence

<220>
<223> Synthetic Oligonucleotide probe

<400> 230
tgctccttct tgttctgggc tctcatg 27

<210> 231
<211> 48
<212> DNA
<213> Artificial Sequence

<220>

<223> Synthetic Oligonucleotide Probe

<400> 231
ggattctaat acgactcact atagggccac gggcgctgtg tgctggag 48

<210> 232
<211> 48
<212> DNA
<213> Artificial Sequence

<220>
<223> Synthetic Oligonucleotide Probe

<400> 232
ctatgaaatt aaccctcact aaagggatgg tggggaccgc agggtgac 48

<210> 233
<211> 48
<212> DNA
<213> Artificial Sequence

<220>
<223> Synthetic Oligonucleotide Probe

<400> 233
ggattctaat acgactcact atagggcccg ccacgaggag ctgttacg 48

<210> 234
<211> 48
<212> DNA
<213> Artificial Sequence

<220>
<223> Synthetic Oligonucleotide Probe

<400> 234
ctatgaaatt aaccctcact aaagggatga cctgcaggca tgggagaa 48

<210> 235
<211> 48
<212> DNA
<213> Artificial Sequence

<220>
<223> Synthetic Oligonucleotide Probe

<400> 235
ggattctaat acgactcact atagggcggc cgccacgagg agctgtta 48

<210> 236
<211> 46
<212> DNA
<213> Artificial Sequence

<220>
<223> Synthetic Oligonucleotide Probe

<400> 236
ctatgaaatt aaccctcact aaagggaggg gctctggggc tgggtc 46

<210> 237
<211> 47
<212> DNA

<213> Artificial Sequence

<220>
<223> Synthetic Oligonucleotide Probe

<400> 237
ggattctaat acgactcact atagggccaa caccaagggg caagatg 47

<210> 238
<211> 48
<212> DNA
<213> Artificial Sequence

<220>
<223> Synthetic Oligonucleotide Probe

<400> 238
ctatgaaatt aaccctcact aaagggaggg cttttggtgg gagaagtt 48

<210> 239
<211> 48
<212> DNA
<213> Artificial Sequence

<220>
<223> Synthetic Oligonucleotide Probe

<400> 239
ggattctaat acgactcact atagggctcg ctgctgtgcc tggtgttg 48

<210> 240
<211> 48
<212> DNA
<213> Artificial Sequence

<220>
<223> Synthetic Oligonucleotide Probe

<400> 240
ctatgaaatt aaccctcact aaagggaccg ctgcagcctc ttgatgga 48

<210> 241
<211> 47
<212> DNA
<213> Artificial Sequence

<220>
<223> Synthetic Oligonucleotide Probe

<400> 241
ggattctaat acgactcact atagggcccc tcctgccttc cctgtcc 47

<210> 242
<211> 48
<212> DNA
<213> Artificial Sequence

<220>
<223> Synthetic Oligonucleotide Probe

<400> 242
ctatgaaatt aaccctcact aaagggagtg gtggccgcga ttatctgc 48

<210> 243
<211> 48
<212> DNA
<213> Artificial Sequence

<220>
<223> Synthetic Oligonucleotide Probe

<400> 243
ggattctaat acgactcact atagggctca gaaaagcgca acagagaa 48

<210> 244
<211> 47
<212> DNA
<213> Artificial Sequence

<220>
<223> Synthetic Oligonucleotide Probe

<400> 244
ctatgaaatt aaccctcact aaagggatgt cttccatgcc aaccttc 47

<210> 245
<211> 48
<212> DNA
<213> Artificial Sequence

<220>
<223> Synthetic Oligonucleotide Probe

<400> 245
ggattctaat acgactcact atagggcgca gcgatggcag cgatgagg 48

<210> 246
<211> 47
<212> DNA
<213> Artificial Sequence

<220>
<223> Synthetic Oligonucleotide Probe

<400> 246
ctagaaatta accctcacta aagggacaga cggggcagca gggagtg 47

<210> 247
<211> 47
<212> DNA
<213> Artificial Sequence

<220>
<223> Synthetic Oligonucleotide Probe

<400> 247
ggattctaat acgactcact atagggcggg aagatggcga ggaggag 47

<210> 248
<211> 48
<212> DNA
<213> Artificial Sequence

<220>
<223> Synthetic Oligonucleotide Probe

<400> 248
ctatgaaatt aaccctcact aaagggacca aggccacaaa cggaaatc 48

<210> 249
<211> 45
<212> DNA
<213> Artificial Sequence

<220>
<223> Synthetic Oligonucleotide Probe

<400> 249
ggattctaat acgactcact atagggcgcg aggacggcgg cttca 45

<210> 250
<211> 48
<212> DNA
<213> Artificial Sequence

<220>
<223> Synthetic Oligonucleotide Probe

<400> 250
ctatgaaatt aaccctcact aaagggaaga gtcgcggccg cccttttt 48

<210> 251
<211> 46
<212> DNA
<213> Artificial Sequence

<220>
<223> Synthetic Oligonucleotide Probe

<400> 251
ggattctaat acgactcact atagggcgag tccttcggcg gctgtt 46

<210> 252
<211> 48
<212> DNA
<213> Artificial Sequence

<220>
<223> Synthetic Oligonucleotide Probe

<400> 252
ctatgaaatt aaccctcact aaagggacgg gtgcttttgg gattcgta 48

<210> 253
<211> 48
<212> DNA
<213> Artificial Sequence

<220>
<223> Synthetic Oligonucleotide Probe

<400> 253
ggattctaat acgactcact atagggcaac ccgagcatgg cacagcac 48

<210> 254
<211> 47
<212> DNA
<213> Artificial Sequence

<220>
<223> Synthetic Oligonucleotide Probe

<400> 254
ctatgaaatt aaccctcact aaagggatct cccagccgcc ccttctc 47

<210> 255
<211> 48
<212> DNA
<213> Artificial Sequence

<220>
<223> Synthetic Oligonucleotide Probe

<400> 255
ggattctaat acgactcact atagggcggc ggaatccaac ctgagtag 48

<210> 256
<211> 47
<212> DNA
<213> Artificial Sequence

<220>
<223> Synthetic Oligonucleotide Probe

<400> 256
ctatgaaatt aaccctcact aaagggagcg gctatcctcc tgtgctc 47

<210> 257
<211> 45
<212> DNA
<213> Artificial Sequence

<220>
<223> Synthetic Oligonucleotide Probe

<400> 257
ggattctaat acgactcact atagggcccc gagtgttttc caaga 45

<210> 258
<211> 48
<212> DNA
<213> Artificial Sequence

<220>
<223> Synthetic Oligonucleotide Probe

<400> 258
ctatgaaatt aaccctcact aaagggacaa gtttactagc ccatccat 48

<210> 259
<211> 48
<212> DNA
<213> Artificial Sequence

<220>
<223> Synthetic Oligonucleotide Probe

<400> 259
ggattctaat acgactcact atagggctgg atgggctagt aaacttga 48

<210> 260
<211> 47

<212> DNA
<213> Artificial Sequence

<220>
<223> Synthetic Oligonucleotide Probe

<400> 260
ctatgaaatt aaccctcact aaagggaccc ttctgctcct tcttgtt 47

## Description

Background of the Invention

Field of the Invention

The present invention relates to compositions and methods useful for promoting or inhibiting angiogenesis and/or cardiovascularization in mammals in need of such biological effect. This includes the diagnosis and treatment of cardiovascular disorders as well as oncological disorders.

Description of Background

A. Cardiac Disorders and Factors

Heart failure affects approximately five million Americans, and new cases of heart failure number about 400,000 each year. It is the single most frequent cause of hospitalization for people age 65 and older in the United States. Recent advances in the management of acute cardiac diseases, including acute myocardial infarction, are resulting in an expanding patient population that will eventually develop chronic heart failure. From 1979 to 1995, hospitalizations for congestive heart failure (CHF) rose from 377,000 to 872,000 (a 130 percent increase) and CHF deaths increased 116 percent.

CHF is a syndrome characterized by left ventricular dysfunction, reduced exercise tolerance, impaired quality of life, and markedly shortened life expectancy. The sine qua non of heart failure is an inability of the heart to pump blood at a rate sufficient to meet the metabolic needs of the body's tissues (in other words, there is insufficient cardiac output).

At least four major compensatory mechanisms are activated in the setting of heart failure to boost cardiac output, including peripheral vasoconstriction, increased heart rate, increased cardiac contractility, and increased plasma volume. These effects are mediated primarily by the sympathetic nervous system and the renin-angiotensin system. *See.* Eichhorn, American Journal of Medicine, 104: 163-169 (1998). Increased output from the sympathetic nervous system increases vascular tone, heart rate, and contractility. Angiotensin 11 elevates blood pressure by 1) directly stimulating vascular smooth muscle contraction, 2) promoting plasma volume expansion by stimulating aldosterone and antidiuretic hormone secretion, 3) stimulating sympathetic-mediated vascular tone, and 4) catalyzing the degradation of bradykinin, which has vasodilatory and natriuretic activity. *See,* review by Brown and Vaughan, Circulation. 97: 1411-1420(1998). As noted below, angiotensin 11 may also have directly deleterious effects on the heart by promoting myocyte necrosis (impairing systolic function) and intracardiac fibrosis (impairing diastolic and in some cases systolic function). *See,* Weber, Circulation, 96: 4065-4082 (1998).

A consistent feature of congestive heart failure (CHF) is cardiac hypertrophy, an enlargement of the heart that is activated by both mechanical and hormonal stimuli and enables the heart to adapt to demands for increased cardiac output. Morgan and Baker. Circulation, 83: 13-25 (1991). This hypertrophic response is frequently associated with a variety of distinct pathological conditions such as hypertension, aortic stenosis, myocardial infarction, cardiomyopathy, valvular regurgitation, and intracardiac shunt, all of which result in chronic hemodynamic overload.

Hypertrophy is generally defined as an increase in size of an organ or structure independent of natural growth that does not involve tumor formation. Hypertrophy of the heart is due either to an increase in the mass of the individual cells (myocytes), or to an increase in the number ofcells making up the tissue (hyperplasia), or both. While the enlargement of an embryonic heart is largely dependent on an increase in myocyte number (which continues until shortly after birth), post-natal cardiac myocytes lose their proliferative capacity. Further growth occurs through hypertrophy of the individual cells.

Adult myocyte hypertrophy is initially beneficial as a short term response to impaired cardiac function by permitting a decrease in the load on individual muscle fibers. With severe, long-standing overload, however, the hypertrophied cells begin to deteriorate and die. Katz, "Heart Failure", in: Katz A.M. ed., Physiology of the Heart (New York: Raven Press, 1992) pp. 638-668. Cardiac hypertrophy is a significant risk factor for both mortality and morbidity in the clinical course of heart failure. Katz. Trends Cardiovasc. Med., 5: 37-44 (1995). For further details of the causes and pathology of cardiac hypertrophy *see, e.g.,* Heart Disease. A Textbook of Cardiovascular Medicine, Braunwald, E. ed. (W.B. Saunders Co., 1988), Chapter 14, "Pathophysiology of Heart Failure."

On a cellular level, the heart is composed of myocytes and surrounding support cells, generically called non-myocytes. While non-myocytes are primarily fibroblast/mesenchymal cells, they also include endothelial and smooth muscle cells. Indeed, although myocytes make up most of the adult myocardial mass, they represent only about 30% of the total cell numbers present in heart. In response to hormonal, physiological, hemodynamic, and pathological stimuli, adult ventricular muscle cells can adapt to increased workloads through the activation of a hypertrophic process. This response is characterized by an increase in myocyte cell size and contractile protein content of individual cardiac muscle cells, without concomitant cell division and activation of embryonic genes, including the gene for atrial natriuretic peptide (ANP). Chien er al., FASEB J., 5: 3037-3046 (1991); Chien et al., Annu. Rev. Physiol., 55: 77-95 (1993). An increment in myocardial mass as a result of an increase in myocyte size that is associated with an accumulation of interstitial collagen within the extracellular matrix and around intramyocardial coronary arteries has been described in left ventricular hypertrophy secondary to pressure overload in humans. Caspari et al., Cardiovase. Res., II : 554-558 (1977): Schwarz et al., Am. J. Cardiol., 42: 895-903 (1978); Hess et al., Circulation, 63: 360-371 (1981); Pearlman et al., Lab. Invest., 46: 158-164 (1982).

It has also been suggested that paracrine factors produced by non-myocyte supporting cells may additionally be involved in the development of cardiac hypertrophy, and various non-myocyte derived hypertrophic factors, such as, leukocyte inhibitory factor (LIF) and endothelin, have been identified. Metcalf, Growth Factors, 7: 169-173 (1992); Kurzrock et al., Endocrine Reviews, 12: 208-217 (1991): Inoue et al., Proc. Natl. Acad. Sci. USA, 86: 2863-2867 (1989) ; Yanagisawa and Masaki, Trends Pharm. Sci., 10: 374-378 (1989); U.S. Patent No. 5,573,762 (issued November 12, 1996). Further exemplary factors that have been identified as potential mediators of cardiac hypertrophy include cardiotrophin-1 (CT-1) (Pennica et al., Proc. Nat. Acad. Sci. USA, 92: 1142-1146 (1995)), catecholamines, adrenocorticosteroids, angiotensin, and prostaglandins.

At present, the treatment of cardiac hypertrophy varies depending on the underlying cardiac disease. Catecholamines, adrenocorticosteroids, angiotensin, prostaglandins, LIF, endothelin (including endothelin-1, -2, and -3 and big endothelin), and CT- 1 are among the factors identified as potential mediators of hypertrophy. For example, beta-adrenergic receptor blocking drugs (beta-blockers, *e.g.,* propranolol, timolol, tertalolol, carteolol, nadolol, betaxolol, penbutolol, acetobutolol, atenolol, metoprolol, carvedilol, etc.) and verapamil have been used extensively in the treatment of hypertrophic cardiomyopathy. The beneficial effects of beta-blockers on symptoms (*e.g.*, chest pain) and exercise tolerance are largely due to a decrease in the heart rate with a consequent prolongation of diastole and increased passive ventricular filling. Thompson et al., Br. Heart J., 44: 488-98 (1980); Harrison et al., Circulation, 29: 84-98 (1964). Verapamil has been described to improve ventricular filling and probably reducing myocardial ischemia. Bonow et al., Circulation, 72: 853-64 (1985).

Nifedipine and diltiazem have also been used occasionally in the treatment of hypertrophic cardiomyopathy. Lorell et al., Circulation, 65: 499-507 (1982); Betocchi et al., Am. J. Cardiol., 78: 451-457 (1996). However, because of its potent vasodilating properties, nifedipine may be harmful, especially in patients with outflow obstruction. Disopyramide has been used to relieve symptoms by virtue of its negative inotropic properties. Pollick, N. Engl. J. Med., 307: 997-999 (1982). In many patients, however, the initial benefits decrease with time. Wigle et al., Circulation, 92: 1680-1692 (1995). Antihypertensive drug therapy has been reported to have beneficial effects on cardiac hypertrophy associated with elevated blood pressure. Examples of drugs used in antihypertensive therapy, alone or in combination, are calcium antagonists, *e.g.,* nitrendipine; adrenergicreceptor blocking agents, *e.g.,* those listed above; angiotensin converting enzyme (ACE) inhibitors such as quinapril, captopril, enalapril, ramipril, benazepril, fosinopril, and lisinopril; diuretics, *e.g.,* chlorothiazide, hydrochlorothiazide,hydroflumethazide, methylchlothiazide, benzthiazide, dichlorphenamide, acetazolamide,and indapamide; and calcium channel blockers, *e.g.,* diltiazem, nifedipine, verapamil, and nicardipine.

For example, treatment of hypertension with diltiazem and captopril showed a decrease in left ventricular muscle mass, but the Doppler indices of diastolic function did not normalize. Szlachcic et al., Am. J. Cardiol. 63: 198-201 (1989) ; Shahi et al., Lancet, 336: 458-461 (1990). These findings were interpreted to indicate that excessive amounts of interstitial collagen may remain after regression of left ventricular hypertrophy. Rossi et al., Am. Heart J., 124: 700-709 (1992). Rossi *et al., supra.* investigated the effect of captopril on the prevention and regression of myocardial cell hypertrophy and interstitial fibrosis in pressure overload cardiac hypertrophy, in experimental rats.

Agents that increase cardiac contractility directly (iontropic agents) were initially thought to benefit patients with heart failure because they improved cardiac output in the short term. However, all positive inotropic agents except digoxigenin have been found to result in increased long-term mortality, in spite of short-term improvements in cardiac performance. Massie, Curr. Op. in Cardiology, 12: 209-217 (1997); Reddy et al., Curr. Opin. Cardiol., 12: 233-241 (1997). Beta-adrenergic receptor blockers have recently been advocated for use in heart failure. Evidence from clinical trials suggests that improvements in cardiac function can be achieved without increased mortality, though documented improvements patient survival have not yet been demonstrated. *See* also, U.S. Pat. Nos. 5,935,924, 5,624,806; 5,661,122; and 5,610,134 and WO 95/28173 regarding the use of cardiotropin-1 or antagonists thereof, or growth hormone and/or insulin-like growth factor-I in the treatment of CHF. Another treatment modality is heart transplantation, but this is limited by the availability of donor hearts.

Endothelin is a vasoconstricting peptide comprising 21 amino acids, isolated from swine arterial endothelial culture supernatant and structurally determined. Yanagisawa et al., Nature, 332: 411-415 (1988). Endothelin was later found to exhibit various actions, and endothelin antibodies as endothelin antagonists have proven effective in the treatment of myocardial infarction, renal failure, and other diseases. Since endothelin is present in live bodies and exhibits vasoconstricting action, it is expected to be an endogenous factor involved in the regulation of the circulatory system, and may be associated with hypertension, cardiovascular diseases such as myocardial infarction, and renal diseases such as acute renal failure. Endothelin antagonists are described, for example, in U.S. Pat. No. 5,773,414; JP Pat. Publ. 3130299/1991, EP 457,195; EP 460,679; and EP 552,489. A new endothelin B receptor for identifying endothelin receptor antagonists is described in U.S. Pat. No. 5,773,223.

Current therapy for heart failure is primarily directed to using angiotensin-converting enzyme (ACE) inhibitors, such as captopril, and diuretics. These drugs improve hemodynamic profile and exercise tolerance and reduce the incidence of morbidity and mortality in patients with CHF. Kramer et al., Circulation, 67(4): 807-816 (1983); Captopril Multicenter Research Group, J.A.C.C., 2(4): 755-763 (1983): The CONSENSUS Trial Study Group, N. Engl. J. Med., 316(23): 1429-1435 (1987); The SOLVD Investigators, N. Engl. J. Med., 325(5):293-302 (1991). Further, they are useful in treating hypertension, left ventricular dysfunction, atherosclerotic vascular disease, and diabetic nephropathy. Brown and Vaughan, *supra.* However, despite proven efficacy, response to ACE inhibitors has been limited. For example, while prolonging survival in the setting of heart failure, ACE inhibitors appear to slow the progression towards end-stage heart failure, and substantial numbers of patients on ACE inhibitors have functional class III heart failure.

Moreover, improvement of functional capacity and exercise time is only small and mortality, although reduced, continues to be high. The CONSENSUS Trial Study Group, N. Engl. J. Med., 316(23): 1429-1453(1987); The SOLVD Investigators. N. Engl. J. Med. 325(5): 293-302 (1991); Cohn et al., N. Engl. J. Med., 325(5): 303-310 (1991): The Captopril-Digoxin Multicenter Research Group, JAMA. 259(4): 539-544 (1988). Hence, ACE inhibitors consistently appear unable to relieve symptoms in more than 60% of heart failure patients and reduce mortality of heart failure only by approximately 15-20%. For further adverse effects, *see* Brown and Vaughan, *supra.*

An alternative to ACE inhibitors is represented by specifie ATI receptor antagonists. Clinical studies are planned to compare the efficacy of these two modalities in the treatment of cardiovascular and renal discase. However, animal model data suggests that the ACE/Ang II pathway, while clearly involved in cardiac hypertrophy, is not the only, or even the primary pathway active in this role. Mouse genetic "knockout" models have been made to test individual components of the pathway. In one such model, the primary cardiac receptor for Ang II, AT sub 1A, has been genetically deleted: these mice do not develop hypertrophy when Ang II is given experimentally (confirming the basic success of the model in eliminating hypertrophy secondary to Ang II). However, when the aorta is constricted in these animals (a model of hypertensive cardiac stress), the hearts still become hypertrophic. This suggests that alternative signaling pathways, not depending on this receptor (AT sub 1A), are activated in hypertension. ACE inhibitors would presumably not be able to inhibit these pathways. *See,* Harada et al., Circulation, 97: 1952-1959 (1998). *See* also, Homcy, Circulation, 97: 1890-1892 (1998) regarding the enigma associated with the process and mechanism of cardiac hypertrophy.

About 750,000 patients suffer from acute myocardial infarction (AMI) annually, and approximately one-fourth of all deaths in the United States are due to AMI. In recent years, thrombolytic agents, *e.g.,* streptokinase, urokinase, and in particular tissue plasminogen activator (t-PA) have significantly increased the survival of patients who suffered myocardial infarction. When administered as a continuous intravenous infusion over 1.5 to 4 hours, t-PA produces coronary patency at 90 minutes in 69% to 90% of the treated patients. Topol el al., Am. J. Cardiol., 61, 723-728 (1988); Neuhaus et al., J. Am. Coll. Cardiol., 12: 581-587 (1988); Neuhaus et al., J. Am. Coll. Cardiol., 14: 1566-1569 (1989). The highest patency rates have been reported with high dose or accelerated dosing regimens. Topol, J. Am. Coll. Cardiol., 15: 922-924 (1990). t-PA may also be administered as a single bolus, although due to its relatively short half-life, it is better suited for infusion therapy. Tebbe et al., Am. J. Cardiol., 64: 448-453 (1989). A t-PA variant, specifically designed to have longer half-life and very high fibrin specificity, TNK t-PA (a T103N, N 117Q, KHRR(296-299)AAAA t-PA variant, Keyt et al., Proc. Natl. Acad. Sci. USA, 91: 3670-3674 (1994)) is particularly suitable for bolus administration. However, despite all these advances, the long-term prognosis of patient survival depends greatly on the post-infarction monitoring and treatment of the patients, which should include monitoring and treatment of cardiac hypertrophy.

B. Growth Factors

Various naturally occurring polypeptides reportedly induce the proliferation of endothelial cells. Among those polypeptides are the basic and acidic fibroblast growth factors (FGF) (Burgess and Maciag, Annual Rev. Biochem., 58: 575 (1989)), platelet-derived endothelial cell growth factor (PD-ECGF) (Ishikawa et al., Nature, 338: 557 (1989)), and vascular endothelial growth factor (VEGF). Leung et al., Science, 246: 1306 (1989); Ferrara and Henzel, Biochem. Biophys. Res. Commun. 161: 851 (1989); Tischer et al., Biochem. Biophys. Res. Commun., 165: 1198 (1989); EP 471,754B granted July 31, 1996.

Media conditioned by cells transfected with the human VEGF (hVEGF) cDNA promoted the proliferation of capillary endothelial cells, whereas control cells did not. Leung et al., Science. 246: 1306 (1989). Several additional cDNAs were identified in human cDNA libraries that encode 121-, 189-. and 206-amino acid isoforms of hVEGF (also collectively referred to as hVEGF-related proteins). The 121-amino acid protein differs from hVEGF by virtue of the deletion of the 44 amino acids between residues 116 and 159 in hVEGF. The 189-amino acid protein differs from hVEGF by virtue of the insertion of 24 amino acids at residue 116 in hVEGF, and apparently is identical to human vascular permeability factor (hVPF). The 206-amino acid protein differs from hVEGF by virtue of an insertion of 41 amino acids at residue 116 in hVEGF. Houck et al., Mol. Endocrin.. 5: 1806 (1991); Ferrara et al., J. Cell. Biochem., 47: 211 (1991); Ferrara et al., Endocrine Reviews, 13: 18 (1992): Keck et al., Science, 246: 1309 (1989); Connolly et al., J. Biol. Chem., 264: 20017 (1989); EP 370,989 published May 30, 1990.

It is now well established that angiogenesis, which involves the formation of new blood vessels from preexisting endothelium, is implicated in the pathogenesis of a variety of disorders. These include solid tumors and metastasis, atherosclerosis, retrolental fibroplasia, hemangiomas, chronic inflammation, intraocular neovascular syndromessuch as proliferative retinopathies, *e.g.,* diabetic retinopathy, age-related macular degeneration (AMD), neovascular glaucoma, immune rejection of transplanted corneal tissue and other tissues, rheumatoid arthritis, and psoriasis. Folkman et al., J. Biol. Chem., 267: 10931-10934 (1992); Klagsbrun et al., Annu. Rev. Physiol., 53: 217-239 (1991); and Garner A., "Vascular diseases", In: Pathobiology of Ocular Disease. A Dynamic Approach, Gamer A., Klintworth GK, eds., 2nd Edition (Marcel Dekker, NY, 1994), pp 1625-1710.

In the case of tumor growth, angiogenesis appears to be crucial for the transition from hyperplasia to neoplasia, and for providing nourishment to the growing solid tumor. Folkman er al., Nature, 339: 58 (1989). The neovascularization allows the tumor cells to acquire a growth advantage and proliferative autonomy compared to the normal cells. Accordingly, a correlation has been observed between density of microvessels in tumor sections and patient survival in breast cancer as well as in several other tumors. Weidner et al., N. Engl. J. Med, 324: 1-6 (1991); Horak el al., Lancet, 340: 1120-1124 (1992); Macchiarini et al., Lancet. 340: 145-146 (1992).

The search for positive regulators ofangiogenesis has yielded many candidates, including aFGF, bFGF, TGF-α, TGF-β, HGF, TNF-α, angiogenin. IL-8, etc. Folkman *et al.,* J.B.C., *supra,* and Klagsbrun *et al., supra.* The negative regulators so far identified include thrombospondin (Good et al., Proc. Natl. Acad. Sci. USA., 87: 6624-6628 (1990)), the 16-kilodalion N-terminal fragment of prolactin (Clapp et al., Endocrinology, 133: 1292-1299 (1993)), angiostatin (O'Reilly et al., Cell, 79: 315-328 (1994)), and endostatin. O'Reilly et al., Cell, 88: 277-285 (1996).

Work done over the last several years has established the key role of VEGF. not only in stimulating vascular endothelial cell proliferation, hut also in inducing vascular permeability and angiogenesis. Ferrara et al., Endocr. Rev., 18: 4-25 (1997). The finding that the loss of even a single VEGF allele results in embryonic lethality points to an irreplaceable role played by this factor in the development and differentiation of the vascular system. Furthermore, VEGF has been shown to be a key mediator of neovascularization associated with tumors and intraocular disorders. Ferrara *et al.,* Endocr. Rev., *supra.* The VEGF mRNA is overexpressed by the majority of human tumors examined. Berkman et al., J. Clin. Invest., 91: 153-159 (1993); Brown et al., Human Pathol. 26: 86-91 (1995): Brown et al., Cancer Res., 53: 4727-4735 (1993); Mattern et al., Brit. J. Cancer. 73: 931-934 (1996): Dvorak et al., Am. J. Pathol., 146: 1029-1039 (1995).

Also, the concentration levels of VEGF in eye fluids are highly correlated to the presence of active proliferation of blood vessels in patients with diabetic and other ischemia-related retinopathies. Aiello et al., N. Engl. J. Med., 331: 1480-1487 (1994). Furthermore, recent studies have demonstrated the localization of VEGF in choroidal neovascular membranes in patients affected by AMD. Lopez et al., Invest. Ophthalmol. Vis. Sci., 37: 855-868 (1996).

Anti-VEGF neutralizing antibodies suppress the growth of a variety of human tumor cell lines in nude mice (Kim et al., Nature, 362: 841-844 (1993): Warren et al., J. Clin. Invest., 95: 1789-1797 (1995); Borgström et al., Cancer Res., 56: 4032-4039 (1996); Melnyk et al., Cancer Res., 56: 921-924 (1996)) and also inhibit intraocular angiogenesis in models of ischemic retinal disorders. Adamis et al., Arch. Ophthalmol., 114: 66-71 (1996). Therefore, anti-VEGF monoclonal antibodies or other inhibitors of VEGF action are promising candidates for the treatment of solid tumors and various intraocular neovascular disorders. Such antibodies are described, for example, in EP 817,648 published January 14, 1998 and in PCT/US 98/06724 filed April 3, 1998.

There exist several other growth factors and mitogens, including transforming oncogenes, that are capable of rapidly inducing a complex set of genes to be expressed by certain cells. Lau and Nathans, Molecular Aspects of Cellular Regulation, 6: 165-202 (1991). These genes, which have been named immediate-early- or early-response genes, are transcriptionally activated within minutes after contact with a growth factor or mitogen, independent of *de novo* protein synthesis. A group of these intermediate-early genes encodes secreted, extracellular proteins that are needed for coordination of complex biological processes such as differentiation and proliferation, regeneration, and wound healing. Ryseck et al., Cell Growth Differ., 2: 235-233 (1991).

Highly-related proteins that belong to this group include *cef 10* (Simmons et al., Proc. Natl. Acad. Sci. USA, 86: 1178-1182 (1989)), *cyr 61,* which is rapidly activated by serum- or platelet-derived growth factor (PDGF) (O'Brien et al., Mol. Cell Biol., 10: 3569-3577 (1990), human connective tissue growth factor (CTGF) (Bradham et al., J. Cell. Biol., 114: 1285-1294 (1991)), which is secreted by human vascular endothelial cells in high levels after activation with transforming growth factor beta (TGF-β), exhibits PDGF-like biological and immunological activities, and competes with PDGF for a particular cell surface receptor, *fisp-12 (*Ryseck et al., Cell Growth Differ., 2: 235-233 (1991)). human vascular IBP-like growth factor (VIGF) (WO 96/17931), and nov, normally arrested in adult kidney cells, which was found to be overexpressed in myeloblastosis-associated-virus-type-1-induced nephroblastomas. Joloit et al., Mol. Cell. Biol., 12: 10-21 (1992).

The expression of these immediate-early genes acts as "third messengers" in the cascade of events triggered by growth factors. It is also thought that they are needed to integrate and coordinate complex biological processes, such as differentiation and wound healing in which cell proliferation is a common event.
As additional mitogens, insulin-like growth factor binding proteins (IGFBPs) have been shown, in complex with insulin-like growth factor (IGF), to stimulate increased binding of IGF to fibroblast and smooth muscle cell surface receptors. Clemmons et al., J. Clin. Invest., 77: 1548 (1986). Inhibitory effects of IGFBP on various IGF actions *in vitro* include stimulation of glucose transport by adipocytes, sulfate incorporation by chondrocytes, and thymidine incorporation in fibroblast. Zapf et al., J. Clin. Invest., 63: 1077(1979). In addition, inhibitory effects of IGFBPs on growth factor-mediated mitogen activity in normal cells have been shown.

C. Need for Further Treatments

In view of the role of vascular endothelial cell growth and angiogenesis in many diseases and disorders, it is desirable to have a means of reducing or inhibiting one or more of the biological effects causing these processes. It is also desirable to have a means of assaying for the presence of pathogenic polypeptides in normal and diseased conditions, and especially cancer. Further, in a specific aspect, as there is no generally applicable therapy for the treatment of cardiac hypertrophy, the identification of factors that can prevent or reduce cardiac myocyte hypertrophy is of primary importance in.the development of new therapeutic strategies to inhibit pathophysiological cardiac growth. While there are several treatment modalities for various cardiovascular and oncologic disorders, there is still a need for additional therapeutic approaches.

Summary of the Invention

A. Embodiments

Accordingly, the present invention concerns compositions and methods for promoting or inhibiting angiogenesis and/or cardiovascularization in mammals. The present invention is based on the identification of proteins that test positive in various cardiovascular assays that test promotion or inhibition of certain biological activities. Accordingly, the proteins are believed to be useful drugs for the diagnosis and/or treatment (including prevention) of disorders where such effects are desired, such as the promotion or inhibition of angiogenesis, inhibition or stimulation of vascular endothelial cell growth, stimulation of growth or proliferation of vascular endothelial cells, inhibition of tumor growth, inhibition of angiogenesis-dependent tissue growth, stimulation of angiogenesis-dependent tissue growth, inhibition of cardiac hypertrophy and stimulation of cardiac hypertrophy, *e.g.*, for the treatment of congestive heart failure.
In one embodiment, the present invention provides a compositioncomprising a PRO polypeptide in admixture with a pharmaceutically acceptable carrier. In one aspect, the composition comprises a therapeutically effective amount of the polypeptide. In another aspect, the composition comprises a further active ingredient, namely, a cardiovascular, endothelial or angiogenic agent or an angiostatic agent, preferably an angiogenic or angiostatic agent. Preferably, the composition is sterile. The PRO polypeptide may be administered in the form of a liquid pharmaceutical formulation, which may be preserved to achieve extended storage stability. Preserved liquid pharmaceutical formulations might contain multiple doses of PRO polypeptide, and might, therefore, be suitable for repeated use.
In a further embodiment, the present invention provides a method for preparing such a composition useful for the treatment of a cardiovascular, endothelial or angiogenic disorder comprising admixing a therapeutically effective amount of a PRO polypeptide with a pharmaceutically acceptable carrier.
In another embodiment, the present invention provides a composition comprising an agonist or antagonist of a PRO polypeptide in admixture with a pharmaceutically acceptable carrier. In one aspect, the composition comprises a therapeutically effective amount of the agonist or antagonist. In another aspect, the composition comprises a further active ingredient, namely, a cardiovascular, endothelial or angiogenic agent or an angiostatic agent, preferably an angiogenic or angiostatic agent. Preferably, the composition is sterile. The PRO polypeptide agonist or antagonist may be administered in the form of a liquid pharmaceutical formulation, which may be preserved to achieve extended storage stability Preserved liquid pharmaceutical formulations might contain multiple doses of a PRO polypeptide agonist or antagonist, and might, therefore be suitable for repeated use.
In a further embodiment, the present invention provides a method for preparing such a composition useful for the treatment of a cardiovascular endothelial or angiogenic disorder comprising admixing a therapeutically effective amount of a PRO polypeptide agonist or antagonist with a pharmaceutically acceptable carrier.
In yet another embodiment, the present invention concerns a composition comprising an anti-PRO antibody in admixture with a pharmaceutically acceptable carrier. In one aspect, the composition comprises a therapeutically effective amount of the antibody. In another aspect, the composition comprises a further active ingredient, namely, a cardiovascular, endothelial or angiogenic agent or an angiostatic agent, preferably an angiogenic or angiostatic agent.

Preferably, the composition is sterile. The composition may be administered in the form of a liquid pharmaceutical formulation, which may be preserved to achieve extended storage stability. Preserved liquid pharmaceutical formulations might contain multiple doses of the anti-PRO antibody, and might, therefore, be suitable for repeated use. In preferred embodiments, the antibody is a monoclonal antibody, an antibody fragment, a humanized antibody, or a single-chain antibody.

In a further embodiment, the present invention provides a method for preparing such a composition useful for the treatment of a cardiovascular, endothelial or angiogenic disorder comprising admixing a therapeutically effective amount of an anti-PRO antibody with a pharmaceutically acceptable carrier.

In a still further aspect, the present invention provides an article of manufacture comprising:

(a) a composition of matter comprising a PRO polypeptide or agonist or antagonist thereof;
(b) a container containing said composition; and
(c) a label affixed to said container, or a package insert included in said container referring to the use of said PRO polypeptide or agonist or antagonist thereof in the treatment of a cardiovascular, endothelial or angiogenic disorder, wherein the agonist or antagonist may be an antibody which binds to the PRO polypeptide. The composition may comprise a therapeutically effective amount of the PRO polypeptide or the agonist or antagonist thereof.

In another embodiment, the present invention provides a method for identifying an agonist of a PRO polypeptide comprising:

(a) contacting cells and a test compound to be screened under conditions suitable for the induction of a cellular response normally induced by a PRO polypeptide; and
(b) determining the induction of said cellular response to determine if the test compound is an effective agonist, wherein the induction of said cellular response is indicative of said test compound being an effective agonist.

In another embodiment, the present invention provides a method for identifying an aeonist of a PRO polypeptide comprising:

(a) contacting cells and a test compound to be screened under conditions suitable for the stimulation of cell proliferation by a PRO polypeptide: and
(b) measuring the proliferation of said cells to determine if the test compound is an effective agonist, wherein the stimulation of cell proliferation is indicative of said test compound being an effective agonist.

In another embodiment, the invention provides a method for identifying a compound that inhibits the activity of a PRO polypeptide comprising contacting a test compound with a PRO polypeptide under conditions and for a time sufficient to allow the test compound and polypeptide to interact and determining whether the activity of the PRO polypeptide is inhibited. In a specific preferred aspect, either the test compound or the PRO polypeptide is immobilized on a solid support. In another preferred aspect, the non-immobilized component carries a detectable label. In a preferred aspect, this method comprises the steps of:

(a) contacting cells and a test compound to be screened in the presence of a PRO polypeptide under conditions suitable for the induction of a cellular response normally induced by a PRO polypeptide; and
(b) determining the induction of said cellular response to determine if the test compound is an effective antagonist.

In another preferred aspect, this process comprises the steps of:

(a) contacting cells and a test compound to be screened in the presence of a PRO polypeptide under conditions suitable for the stimulation of cell proliferation by a PRO polypeptide; and
(b) measuring the proliferation of the cells to determine if the test compound is an effective antagonist.

In another embodiment, the invention provides a method for identifying a compound that inhibits the expression of a PRO polypeptide in cells that normally expresses the polypeptide, wherein the method comprises contacting the cells with a test compound and determining whether the expression of the PRO polypeptide is inhibited. In a preferred aspect, this method comprises the steps of:

(a) contacting cells and a test compound to be screened under conditions suitable for allowing expression of the PRO polypeptide; and
(b) determining the inhibition of expression of said polypeptide.

In a still further embodiment, the invention provides a compound that inhibits the expression of a PRO polypeptide, such as a compound that is identified by the methods set forth above.

Another aspect of the present invention is directed to an agonist or an antagonist of a PRO polypeptide which may optionally be identified by the methods described above.

One type of antagonist of a PRO polypeptide that inhibits one or more of the functions or activities of the PRO polypeptide is an antibody. Hence, in another aspect, the invention provides an isolated antibody that binds a PRO polypeptide. In a preferred aspect, the antibody is a monoclonal antibody, which preferably has non-human complementarity-determining-region (CDR) residues and human framework-region (FR) residues. The antibody may be labeled and may be immobilized on a solid support. In a further aspect, the antibody is an antibody fragment, a single-chain antibody or a humanized antibody. Preferably, the antibody specifically binds to the polypeptide.

In a still further aspect, the present invention provides a method for diagnosing a disease or susceptibility to a disease which is related to a mutation in a PRO polypeptide-encoding nucleic acid sequence comprising determining the presence or absence of said mutation in the PRO polypeptide nucleic acid sequence, wherein the presence or absence of said mutation is indicative of the presence of said disease or susceptibility to said disease.

In a still further aspect, the invention provides a method of diagnosing a cardiovascular, endothelial or angiogenic disorder in a mammal which comprises analyzing the level of expression of a gene encoding a PRO polypeptide (a) in a test sample of tissue cells obtained from said mammal, and (b) in a control sample of known normal tissue cells of the same cell type, wherein a higher or lower expression level in the test sample as compared to the control sample is indicative of the presence of a cardiovascular, endothelial or angiogenic disorder in said mammal. The expression of a gene encoding a PRO polypeptide may optionally be accomplished by measuring the level of mRNA or the polypeptide in the test sample as compared to the control sample.

In a still further aspect, the present invention provides a method of diagnosing a cardiovascular, endothelial or angiogenic disorder in a mammal which comprises detecting the presence or absence of a PRO polypeptide in a test sample of tissue cells obtained from said mammal, wherein the presence or absence of said PRO polypeptide in said test sample is indicative of the presence of a cardiovascular, endothelial or angiogenic disorder in said mammal.

In a still further embodiment, the invention provides a method of diagnosing a cardiovascular, endothelial or angiogenic disorder in a mammal comprising (a) contacting an anti-PRO antibody with a test sample of tissue cells obtained from the mammal, and (b) detecting the formation of a complex between the antibody and the PRO polypeptide in the test sample, wherein the formation of said complex is indicative of the presence of a cardiovascular, endothelial or angiogenic disorder in the mammal. The detection may be qualitative or quantitative, and may be performed in comparison with monitoring the complex formation in a control sample of known normal tissue cells of the same cell type. A larger or smaller quantity of complexes formed in the test sample indicates the presence of a cardiovascular, endothelial or angiogenic dysfunction in the mammal from which the test tissue cells were obtained. The antibody preferably carries a detectable label. Complex formation can be monitored, for example, by light microscopy, flow cytometry, fluorimetry, or other techniques known in the art. The test sample is usually obtained from an individual suspected to have a cardiovascular, endothelial or angiogenic disorder.

In another embodiment, the invention provides a method for determining the presence of a PRO polypeptide in a sample comprising exposing a sample suspected of containing the PRO polypeptide to an anti-PRO antibody and determining binding of said antibody to a component of said sample. In a specific aspect, the sample comprises a cell suspected of containing the PRO polypeptide and the antibody binds to the cell. The antibody is preferably detectably labeled and/or bound to a solid support.

In further aspects, the invention provides a cardiovascular, endothelial or angiogenic disorder diagnostic kit comprising an anti-PRO antibody and a carrier in suitable packaging. Preferably, such kit further comprises instructions for using said antibody to detect the presence of the PRO polypeptide. Preferably, the carrier is a buffer, for example. Preferably, the cardiovascular, endothelial or angiogenic disorder is cancer.

In yet another embodiment, the present invention provides a method for treating a cardiovascular, endothelial or angiogenic disorder in a mammal comprising administering to the mammal an effective amount of a PRO polypeptide. Preferably, the disorder is cardiac hypertrophy, trauma such as wounds or burns, or a type of cancer. In a further aspect, the mammal is further exposed to angioplasty or a drug that treats cardiovascular, endothelial or angiogenic disorders such as ACE inhibitors or chemotherapeutic agents if the cardiovascular, endothelial or angiogenic disorder is a type of cancer. Preferably, the mammal is human, preferably one who is at risk of developing cardiac hypertrophy and more preferably has suffered myocardial infarction.

In another preferred aspect, the cardiac hypertrophy is characterized by the presence of an elevated level of $PGF_{2\alpha}$. Alternatively, the cardiac hypertrophy may be induced by myocardial infarction, wherein preferably the administration of the PRO polypeptide is initiated within 48 hours, more preferably within 24 hours, following myocardial infarction.

In anotherpreferredembodiment,the cardiovascular, endothelial or angiogenic disorder is cardiac hypertrophy and said PRO polypeptide is administered together with a cardiovascular, endothelial or angiogenic agent. The preferred cardiovascular endothelial or angiogenic agent for this purpose is selected from the group consisting of an antihypertensive drug, an ACE inhibitor, an endothelin receptor antagonist and a thrombolytic agent. If a thrombolytic agent is administered, preferably the PRO polypeptide is administered following administration of such agent. More preferably, the thrombolytic agent is recombinant human tissue plasminogen activator.

In another preferred aspect, the cardiovascular, endothelial or angiogenic disorder is cardiac hypertrophy and the PRO polypeptide is administered following primary angioplasty for the treatment of acute myocardial infarction, preferably wherein the mammal is further exposed to angioplasty or a cardiovascular, endothelial, or angiogenic agent.

In another preferred embodiment, the cardiovascular, endothelial or angiogenic disorder is a cancer and the PRO polypeptide is administered in combination with a chemotherapeutic agent, a growth inhibitory agent or a cytotoxic agent.

In a further embodiment, the invention concerns a method for treating a cardiovascular, endothelial or angiogenic disorder in a mammal comprising administering to the mammal an effective amount of an agonist of a PRO polypeptide. Preferably, the cardiovascular, endothelial or angiogenic disorder is cardiac hypertrophy, trauma, a cancer, or age-related macular degeneration. Also preferred is where the mammal is human, and where an effective amount of an angiogenic or angiostatic agent is administered in conjunction with the agonist.

In a further embodiment, the invention concerns a method for treating a cardiovascular, endothelial or angiogenic disorder in a mammal comprising administering to the mammal an effective amount of an antagonist of a PRO polypeptide. Preferably, the cardiovascular, endothelial or angiogenic disorder is cardiac hypertrophy, trauma, a cancer, or age-related macular degeneration. Also preferred is where the mammal is human, and where an effective amount of an angiogenic or angiostatic agent is administered in conjunction with the antagonist.

In a further embodiment, the invention concerns a method for treating a cardiovascular, endothelial or angiogenic disorder in a mammal comprising administering to the mammal an effective amount of an anti-PRO antibody. Preferably, the cardiovascular, endothelial or angiogenic disorder is cardiac hypertrophy, trauma, a cancer, or age-related macular degeneration. Also preferred is where the mammal is human, and where an effective amount of an angiogenic or angiostatic agent is administered in conjunction with the antibody.

In still further embodiments, the invention provicies a method for treating a cardiovascular, endothelial or angiogenic disorder in a mammal that suffers therefrom comprising administering to the mammal a nucleic acid molecule that codes for either (a) a PRO polypeptide, (b) an agonist of a PRO polypeptide or (c) an antagonist of a PRO polypeptide, wherein said agonist or antagonist may be an anti-PRO antibody. In a preferred embodiment, the mammal is human. In another preferred embodiment, the gene is administered via *ex vivo* gene therapy. In a further preferred embodiment, the gene is comprised within a vector, more preferably an adenoviral, adeno-associated viral, lentiviral, or retroviral vector.

In yet another aspect, the invention provides a recombinant retroviral particle comprising a retroviral vector consisting essentially of a promoter, nucleic acid encoding (a) a PRO polypeptide, (b) an agonist polypeptide of a PRO polypeptide, or (c) an antagonist polypeptide of a PRO polypeptide, and a signal sequence for cellular secretion of the polypeptide, wherein the retroviral vector is in association with retroviral structural proteins. Preferably, the signal sequence is from a mammal, such as from a native PRO polypeptide.

In a still further embodiment, the invention supplies an *ex vivo* producer cell comprising a nucleic acid construct that expresses retroviral structural proteins and also comprises a retroviral vector consisting essentially of a promoter, nucleic acid encoding (a) a PRO polypeptide, (b) an agonist polypeptide of a PRO polypeptide or (c) an antagonist polypeptide of a PRO polypeptide, and a signal sequence for cellular secretion of the polypeptide, wherein said producer cell packages the retroviral vector in association with the structural proteins to produce recombinant retroviral particles.

In yet another embodiment, the invention provides a method for inhibiting endothelial cell growth in a mammal comprising administering to the mammal (a) a PRO polypeptide, (b) an agonist of a PRO polypeptide, or (c) an antagonist of a PRO polypeptide, wherein endothelial cell growth in said mammal is inhibited, and wherein said agonist or antagonist may be an anti-PRO antibody. Preferably, the mammal is human and the endothelial cell growth is associated with a tumor or a retinal disorder.

In yet another embodiment, the invention provides a method for stimulating endothelial cell growth in a mammal comprising administering to the mammal (a) a PRO polypeptide, (b) an agonist of a PRO polypeptide, or (c) an antagonist of a PRO polypeptide, wherein endothelial cell growth in said mammal is stimulated, and wherein said agonist or antagonist may be an anti-PRO antibody. Preferably, the mammal is human.

In yet another embodiment, the invention provides a method for inhibiting cardiac hypertrophy in a mammal comprising administering to the mammal (a) a PRO polypeptide, (b) an agonist of a PRO polypeptide, or (c) an antagonist of a PRO polypeptide, wherein cardiac hypertrophy in said mammal is inhibited, and wherein said agonist or antagonist may be an anti-PRO antibody . Preferably, the mammal is human and the cardiac hypertrophy has been induced by myocardial infarction.

In yet another embodiment, the invention provides a method for stimulating cardiac hypertrophy in a mammal comprising administering to the mammal (a) a PRO polypeptide, (b) an agonist of a PRO polypeptide, or (c) an antagonist of a PRO polypeptide, wherein cardiac hypertrophy in said mammal is stimulated, and wherein said agonist or antagonist may be an anti-PRO antibody. Preferably, the mammal is human who suffers from congestive heart failure.

In yet another embodiment, the invention provides a method for inhibiting angiogenesis induced by a PRO polypeptide in a mammal comprising administering a therapeutically effective amount ofan anti-PRO antibody to the mammal. Preferably, the mammal is a human, and more preferably the mammal has a tumor or a retinal disorder.

In yet another embodiment, the invention provides a method for stimulating angiogenesis induced by a PRO polypeptide in a mammal comprising administering a therapeutically effective amount of a PRO polypeptide to the mammal. Preferably, the mammal is a human, and more preferably angiogeneisis would promote tissue regeneration or wound healing.

B. Additional Embodiments

In other embodiments of the present invention, the invention provides an isolated nucleic acid molecule comprising a nucleotide sequence that encodes a PRO polypeptide.

In one aspect, the isolated nucleic acid molecule comprises a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule comprising the coding sequence of a full-length PRO polypeptide cDNA as disclosed herein, the coding sequence of a PRO polypeptide lacking the signal peptide as disclosed herein the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect the invention concerns an isolated nucleic acid molecule comprising a nucleotide sequence having at least about 80% sequence identity, preferably at least about 81% sequence identity, more preferably at least about 82% sequence identity, yet more preferahiy at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule that encodes the same mature polypeptide encoded by any of the human protein cDNAs deposited with the ATCC as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, preferably at least about 30 nucleotides in length, more preferably at least about 40 nucleotides in length, yet more preferably at least about 50 nucleotides in length, yet more preferably at least about 60 nucleotides in length, yet more preferably at least about 70 nucleotides in length, yet more preferably at least about 80 nucleotides in length, yet more preferably at least about 90 nucleotides in length, yet more preferably at least about 100 nucleotides in length, yet more preferably at least about 110 nucleotides in length, yet more preferably at least about 120 nucleotides in length, yet more preferably at least about 130 nucleotides in length, yet more preferably at least about 140 nucleotides in length, yet more preferably at least about 150 nucleotides in length, yet more preferably at least about 160 nucleotides in length, yet more preferably at least about 170 nucleotides in length, yet more preferably at least about 180 nucleotides in length, yet more preferably at least about 190 nucleotides in length, yet more preferably at least about 200 nucleotides in length, yet more preferably at least about 250 nucleotides in length, yet more preferably at least about 300 nucleotides in length, yet more preferably at least about 350 nucleotides in length, vet more preferably at least about 400 nucleotides in length, yet more preferably at least about 450 nucleotides in length, yet more preferably at least about 500 nucleotides in length, yet more preferably at least about 600 nucleotides in length, yet more preferably at least about 700 nucleotides in length, vet more preferably at least about 800 nucleotides in length, yet more preferably at least about 900 nucleotides in length and yet more preferably at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotidemolecule fragments, preferably those PRO polypeptide fragments that comprise a binding site for an anti-PRO antibody.

In another embodiment, the invention provides isolated PRO polypeptide encoded by any of the isolated nucleic acid sequences hereinabove identified.

In a certain aspect, the invention concerns an isolated PRO polypeptide, comprising an amino acid sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the fun-length amino acid sequence as disclosed herein.

In a further aspect, the invention concerns an isolated PRO polypeptide comprising an amino acid sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least ahout 99% sequence identity to an amino acid sequence encoded by any of the human protein cDNAs deposited with the ATCC as disclosed herein.

In a further aspect the invention concerns an isolated PRO polypeptide comprising an amino acid sequence scoring at least about 80% positives, preferably at least about 81% positives, more preferably at least about 82% positives, yet more preferably at least about 83% positives, yet more preferably at least about 84% positives, yet more preferably at least about 85% positives, yet more preferably at least about 86% positives, yet more preferably at least about 87% positives, yet more preferably at least about 88% positives, yet more preferably at least about 89% positives, yet more preferably at least about 90% positives, yet more preferably at least about 91% positives, yet more preferably at least about 92% positives, yet more preferably at least about 93% positives, yet more preferably at least about 94% positives, yet more preferably at least about 95% positives, yet more preferably at least about 96% positives, yet more preferably at least about 97% positives, yet more preferably at least about 98% positives and yet more preferably at least about 99% positives when compared with the amino acid sequence of a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a specific aspect, the invention provides an isolated PRO polypeptide without the N-terminal signal sequence and/orthe initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect of the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist or antagonist thereof as here in before described, or an anti-PRO antibody, for the preparation of a medicament useful in the treatment of a condition which is responsive to the PRO polypeptide, an agonist or antagonist thereof or an anti-PRO antibody.

In additional embodiments of the present invention, the invention provides vectors comprising DNA encoding any of the herein described polypeptides. Host cell comprising any such vector are also provided. By way of example, the host cells may be CHO cells. *E. coli,* yeast, or Baculovirus-infected insect cells. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In yet another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

## Brief Description of the Drawings

Figure 1 shows a nucleotide sequence (SEQ ID NO:3) of a native sequence PRO172 cDNA, wherein SEQ ID NO: 3 is a clone designated herein as "DNA35916-1161".

Figure 2 shows the amino acid sequence (SEQ ID NO:4) derived from the coding sequence of SEQ ID NO:3 shown in Figure I.

Figure 3 shows a nucleotide sequence (SEQ ID NO:8) of a native sequence PRO178 cDNA, wherein SEQ ID NO:

8 is a clone designated herein as "DNA23339-1130".

Figure 4 shows the amino acid sequence (SEQ ID NO:9) derived from the coding sequence of SEQ ID NO:8 shown in Figure 3.

Figure 5 shows a nucleotide sequence (SEQ ID NO: 13) of a native sequence PRO 179 cDNA, wherein SEQ ID NO:13 is a clone designated herein as "DNA 16451-1388".

Figure 6 shows the amino acid sequence (SEQ ID NO:14) derived from the coding sequence of SEQ ID NO:13 shown in Figure 5.

Figure 7 shows a nucleotide sequence (SEQ ID NO:15) of a native sequence PRO 182 cDNA. wherein SEQ ID NO: 15 is a clone designated herein as "DNA27865-1091".

Figure 8 shows the amino acid sequence (SEQ ID NO:16) derived from the coding sequence of SEQ ID NO:15 shown in Figure 7.

Figure 9 shows a nucleotide sequence (SEQ ID NO:20) of a native sequence PRO187 cDNA, wherein SEQ ID NO: 20 is a clone designated herein as "DNA27864-1155".

Figure 10 shows the amino acid sequence (SEQ ID NO:21) derived from the coding sequence of SEQ ID NO:20 shown in Figure 9.

Figure 11 shows a nucleotide sequence (SEQ ID NO:25) of a native sequence PRO188 cDNA, wherein SEQ ID NO:25 is a clone designated herein as "DNA28497-1130".

Figure 12 shows the amino acid sequence (SEQ ID NO:26) derived from the coding sequence of SEQ ID NO:25 shown in Figure 11.

Figure 13 shows a nucleotide sequence (SEQ ID NO:30) of a native sequence PRO195 cDNA, wherein SEQ ID NO:30 is a clone designated herein as "DNA26847-1395".

Figure 14 shows the amino acid sequence (SEQ ID NO:31) derived from the coding sequence of SEQ ID NO:30 shown in Figure 13.

Figure 15 shows a nucleotide sequence (SEQ ID NO:35) of a native sequence PRO212 cDNA, wherein SEQ ID NO:35 is a clone designated herein as "DNA30942-1134".

Figure 16 shows the amino acid sequence (SEQ ID NO:36) derived from the coding sequence of SEQ ID NO:35 shown in Figure 15.

Figure 17 shows a nucleotide sequence (SEQ ID NO:40) of a native sequence PRO214 cDNA, wherein SEQ ID NO:40 is a clone designated herein as "DNA32286-1191".

Figure 18 shows the amino acid sequence (SEQ ID NO:41) derived from the coding sequence of SEQ ID NO:40 shown in Figure 17.

Figure 19 shows a nucleotide sequence (SEQ ID NO:45) of a native sequence PRO217 cDNA, wherein SEQ ID NO:45 is a clone designated herein as "DNA33094-1131".

Figure 20 shows the amino acid sequence (SEQ ID NO:46) derived from the coding sequence of SEQ ID NO:45 shown in Figure 19.

Figure 21 shows a nucleotide sequence (SEQ ID NO:50) of a native sequence PRO224 cDNA, wherein SEQ ID NO:50 is a clone designated herein as "DNA33221-1133".

Figure 22 shows the amino acid sequence (SEQ ID NO:51) derived from the coding sequence of SEQ ID NO:50 shown in Figure 21.

Figure 23 shows a nucleotide sequence (SEQ ID NO:55) of a native sequence PRO231 cDNA, wherein SEQ ID NO:55 is a clone designated herein as "DNA34434-1139".

Figure 24 shows the amino acid sequence (SEQ ID NO:56) derived from the coding sequence of SEQ ID NO:55 shown in Figure 23.

Figure 25 shows a nucleotide sequence (SEQ ID NO:(61) of a native sequence PRO235 cDNA, wherein SEQ ID NO:61 is a clone designated herein as "DNA35558-1167".

Figure 26 shows the amino acid sequence (SEQ ID NO:62) derived from the coding sequence of SEQ ID NO:61 shown in Figure 25.

Figure 27 shows a nucleotide sequence (SEQ ID NO:66) of a native sequence PRO245 cDNA, wherein SEQ ID NO:66 is a clone designated herem as "DNA35638-1141".

Figure 28 shows the amino acid sequence (SEQ ID NO:67) derived from the coding sequence of SEQ ID NO:66 shown in Figure 27.

Figure 29 shows a nucleotide sequence (SEQ ID NO:71) of a native sequence PRO261 cDNA, wherein SEQ ID NO:71 is a clone designated herein as "DNA33473-1176".

Figure 30 shows the amino acid sequence (SEQ ID NO:72) derived from the coding sequence of SEQ ID NO:71 shown in Figure 29.

Figure 31 shows a nucleotide sequence (SEQ ID NO:76) of a native sequence PRO269 cDNA, wherein SEQ ID NO:76 is a clone designated herein as "DNA38260-1180".

Figure 32 shows the amino acid sequence (SEQ ID NO:77) derived from the coding sequence of SEQ ID NO:76 shown in Figure 31.

Figure 33 shows a nucleotide sequence (SEQ ID NO:84) of a native sequence PRO287 cDNA, wherein SEQ ID NO:84 is a clone designated herein as "DNA39969-1185".

Figure 34 shows the amino acid sequence (SEQ ID NO:85) derived from the coding sequence of SEQ ID NO:84 shown in Figure 33.

Figure 35 shows a nucleotide sequence (SEQ ID NO:89) of a native sequence PRO301 cDNA, wherein SEQ ID NO:89 is a clone designated herein as "DNA40628-1216".

Figure 36 shows the amino acid sequence (SEQ ID NO:90) derived from the coding sequence of SEQ ID NO:89 shown in Figure 35.

Figure 37 shows a nucleotide sequence (SEQ ID NO:97) of a native sequence PRO323 cDNA, wherein SEQ ID NO:97 is a clone designated herein as "DNA35595-1228".

Figure 38 shows the amino acid sequence (SEQ ID NO:98) derived from the coding sequence of SEQ ID NO:97 shown in Figure 37.

Figure 39 shows a nucleotide sequence (SEQ ID NO:106) of a native sequence PRO331 cDNA, wherein SEQ ID NO:106 is a clone designated herein as "DNA40981-1234".

Figure 40 shows the amino acid sequence (SEQ ID NO:107) derived from the coding sequence of SEQ ID NO:106 shown in Figure 39.

Figure 41 shows a nucleotide sequence (SEQ ID NO:111) of a native sequence PRO356 cDNA, wherein SEQ ID NO:111 is a clone designated herein as "DNA47470-1130-P1".

Figure 42 shows the amino acid sequence (SEQ ID NO:112) derived from the coding sequence of SEQ ID NO:111 shown in Figure 41.

Figure 43 shows a nucleotide sequence (SEQ ID NO:116) of a native sequence PRO364 cDNA. wherein SEQ ID NO:116 is a clone designated herein as "DNA47365-1206".

Figure 44 shows the amino acid sequence (SEQ ID NO:117) derived from the coding sequence of SEQ ID NO:116 shown in Figure 43.

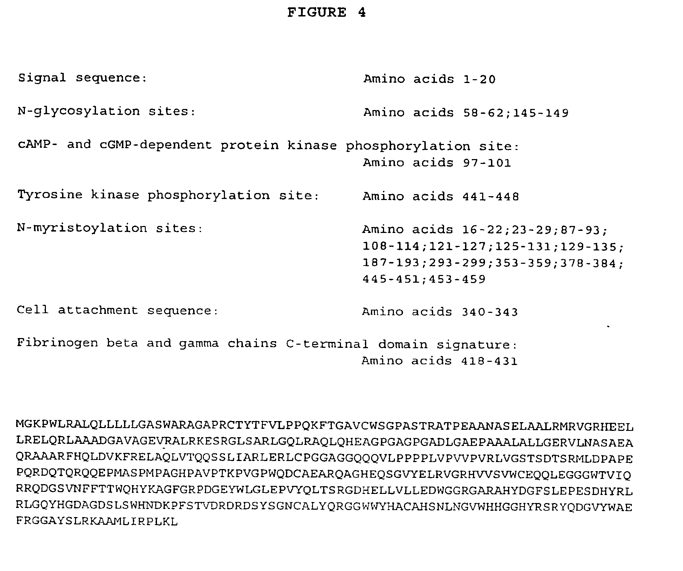

Figure 4 5 shows a nucleotide sequence (SEQ ID NO:126) of a native sequence PRO526 cDNA, wherein SEQ ID NO:126 is a clone designated herein as "DNA44184-1319".

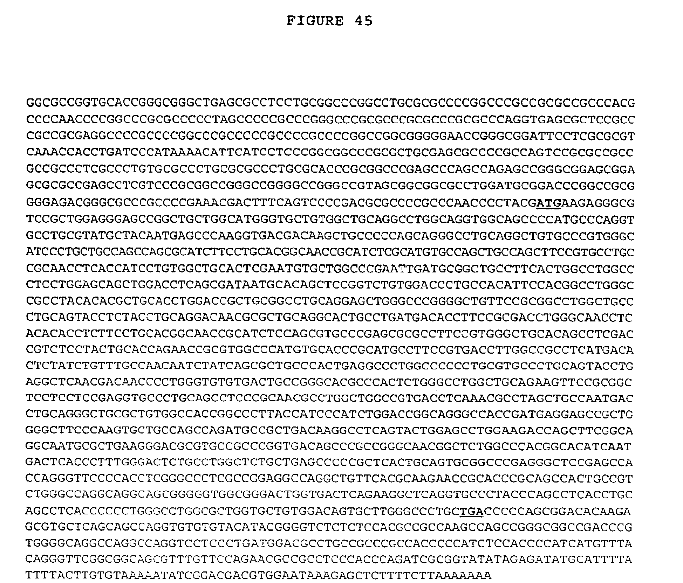

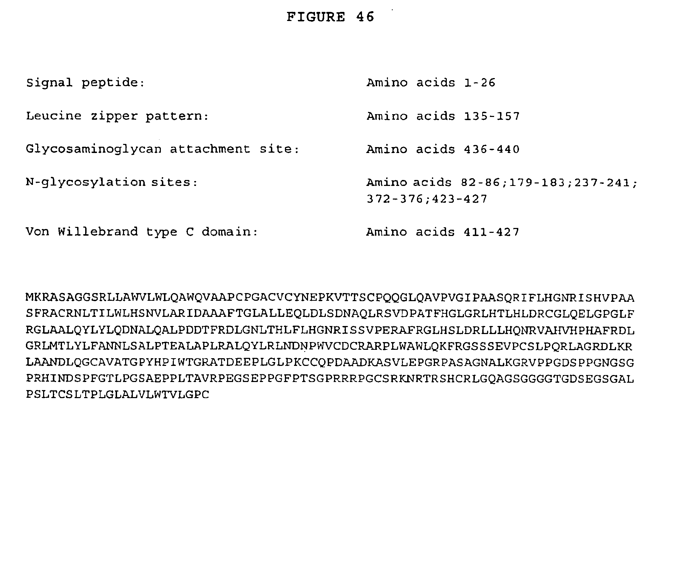

Figure 46 shows the amino acid sequence (SEQ ID NO:127) derived from the coding sequence of SEQ ID NO:126 shown in Figure 45.

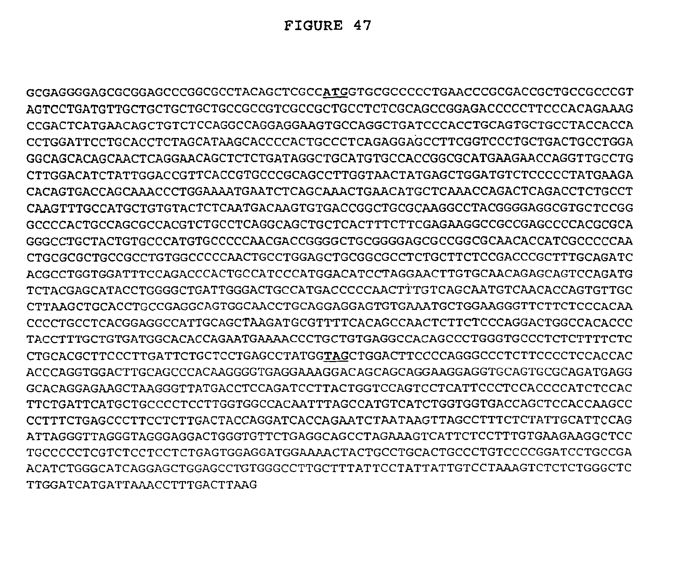

Figure 47 shows a nucleotide sequence (SEQ ID NO:131) of a native sequence PRO538 cDNA, wherein SEQ ID NO:131 is a clone designated herein as "DNA48613-1268".

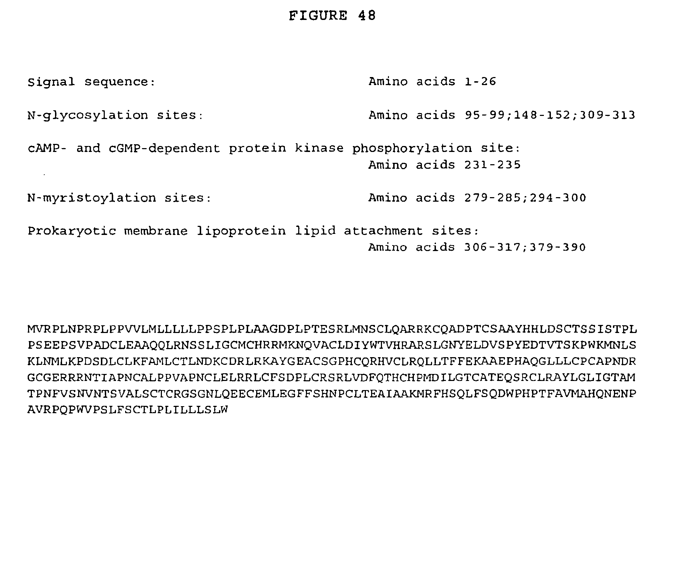

Figure 48 shows the amino acid sequence (SEQ ID NO:132) derived from the coding sequence of SEQ ID NO:131 shown in Figure 47.

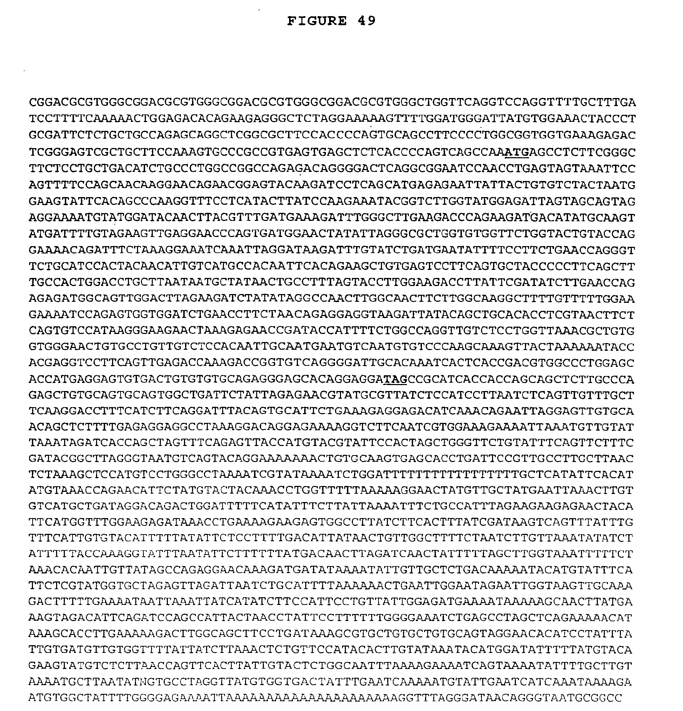

Figure 49 shows a nucleotide sequence (SEQ ID NO:136) of a native sequence PRO713 cDNA, wherein SEQ ID NO:136 is a clone designated herein as "DNA29101-1122".

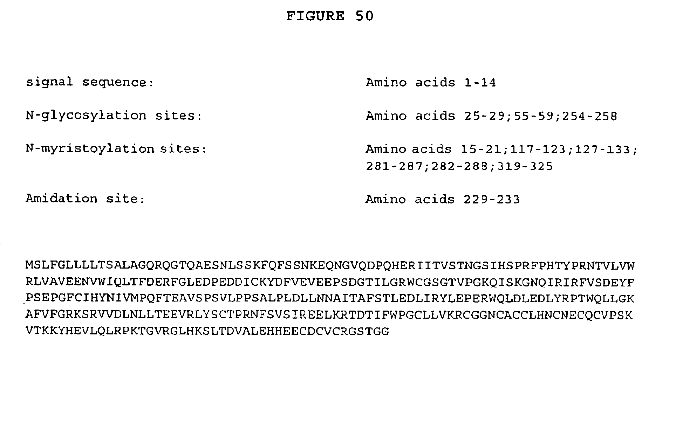

Figure 50 shows the amino acid sequence (SEQ ID NO:137) derived from the coding sequence of SEQ ID NO:136 shown in Figure 49.

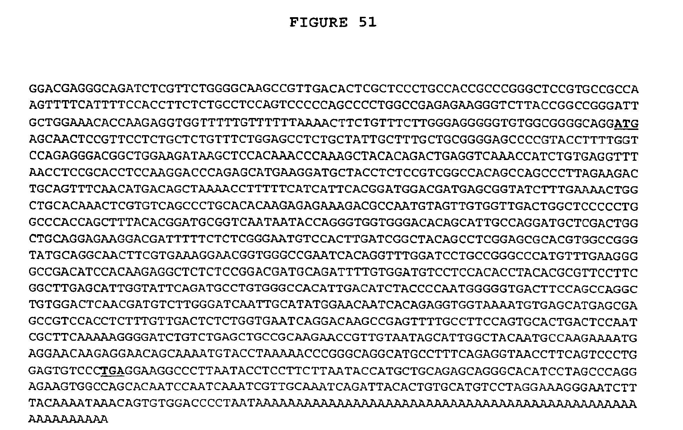

Figure 51 shows a nucleotide sequence (SEQ ID NO:142) of a native sequence PRO719 cDNA, wherein SEQ ID NO:142 is a clone designated herein as "DNA49646-1327".

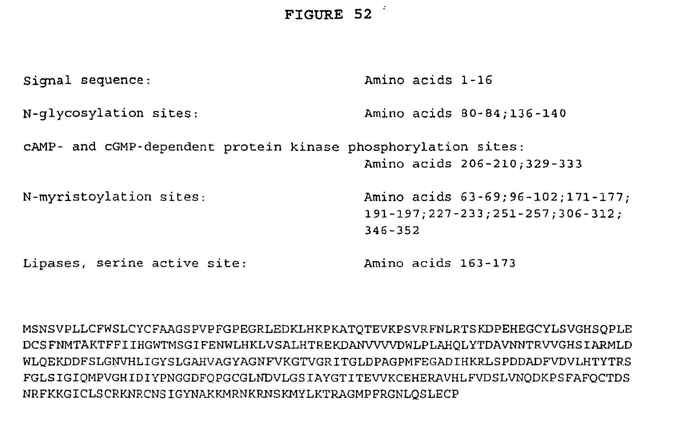

Figure 52 shows the amino acid sequence (SEQ ID NO:143) derived from the coding sequence of SEQ ID NO:142 shown in Figure 51.

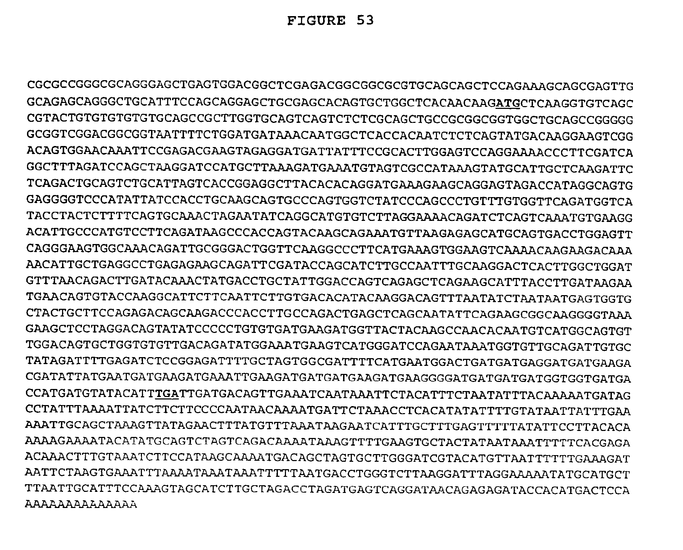

Figure 53 shows a nucleotide sequence (SEQ ID NO:147) of a native sequence PRO771 cDNA, wherein SEQ ID NO:147 is a clone designated herein as "DNA49829-1346".

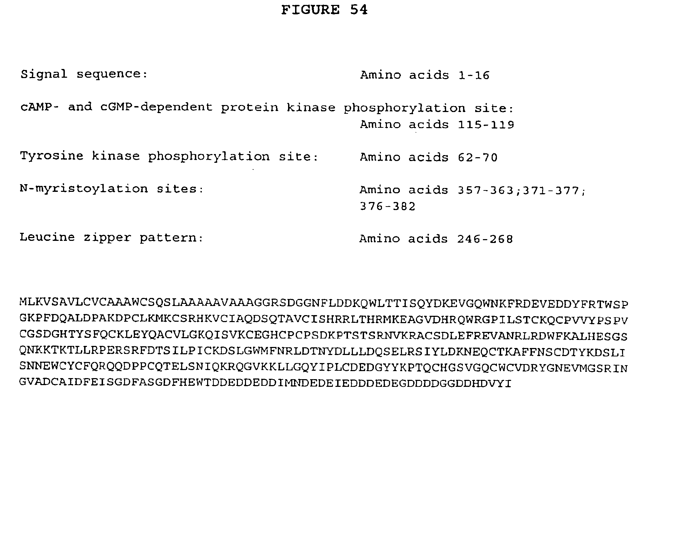

Figure 54 shows the amino acid sequence (SEQ ID NO:148) derived from the coding sequence of SEQ ID NO:147 shown in Figure 53.

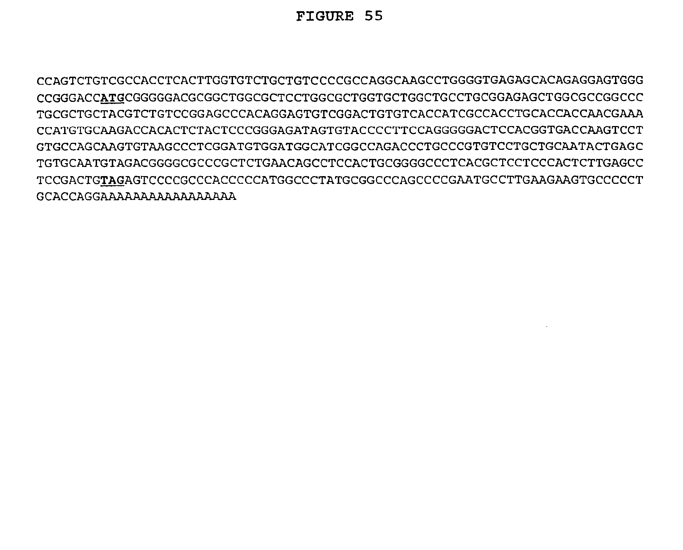

Figure 55 shows a nucleotide sequence (SEQ ID NO:152) of a native sequence PRO788 cDNA, wherein SEQ ID NO:152 is a clone designated herein as "DNA56405-1357".

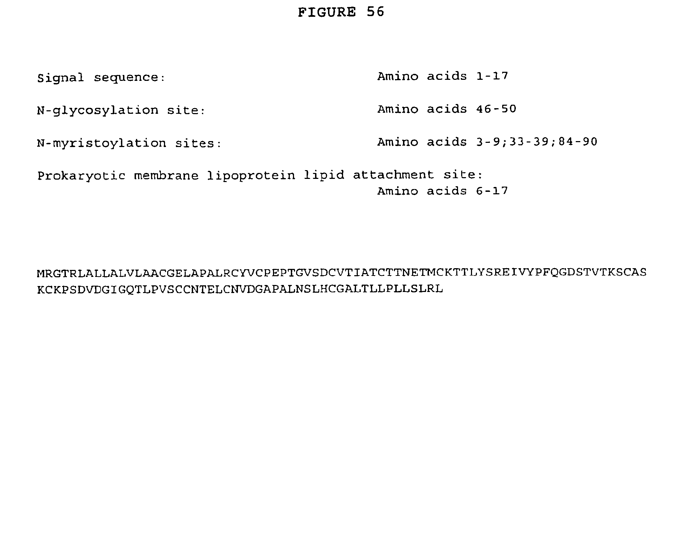

Figure 56 shows the amino acid sequence (SEQ ID NO:153) derived from the coding sequence of SEQ ID NO:152 shown in Figure 55.

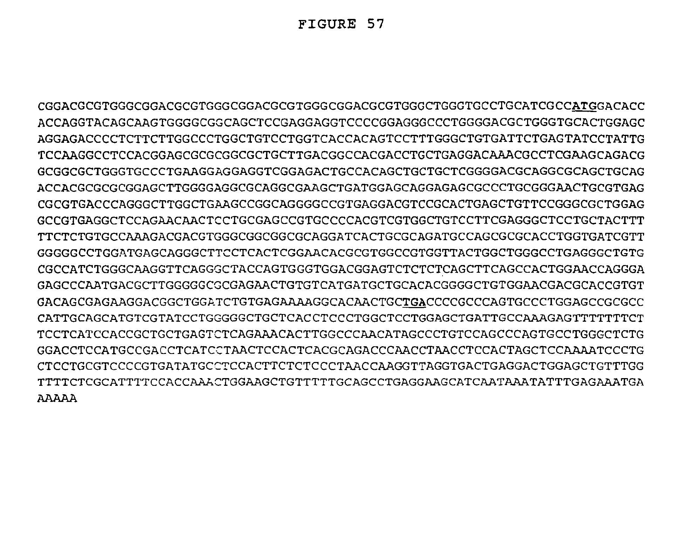

Figure 57 shows a nucleotide sequence (SEQ ID NO:154) of a native sequence PRO792 cDNA, wherein SEQ ID NO:154 is a clone designated herein as "DNA56352-1358".

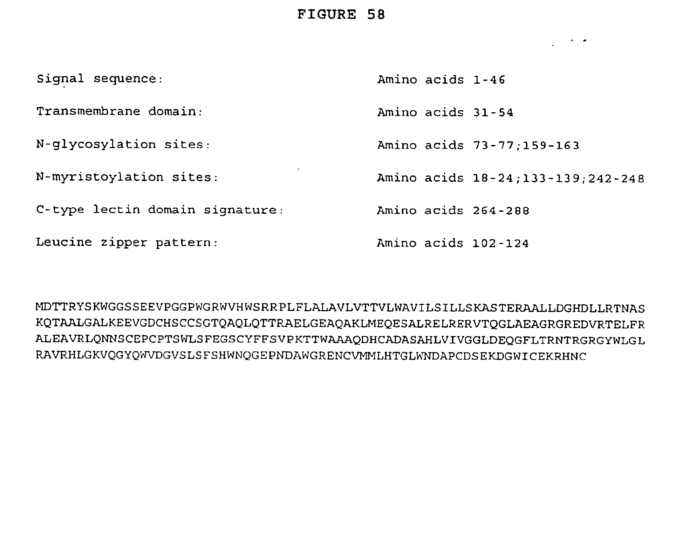

Figure 58 shows the amino acid sequence (SEQ ID NO:155) derived from the coding sequence of SEQ ID NO:154 shown in Figure 57.

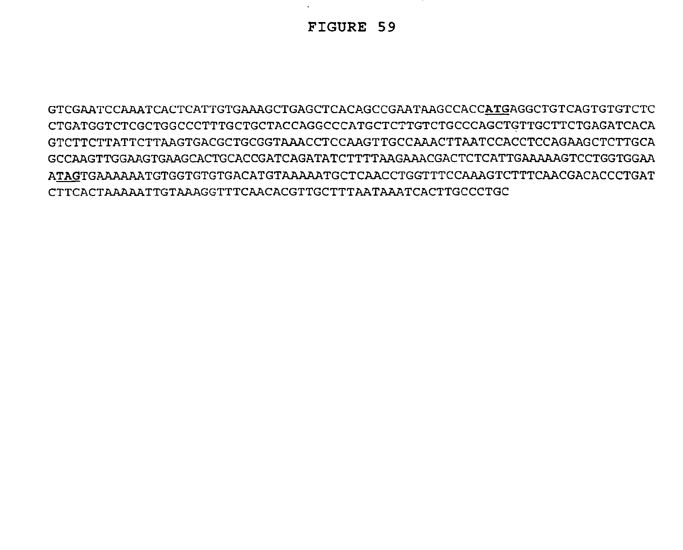

Figure 59 shows a nucleotide sequence (SEQ ID NO: 159) of a native sequence PRO812 cDNA, wherein SEQ ID NO:159 is a clone designated herein as "DNA59205-1421".

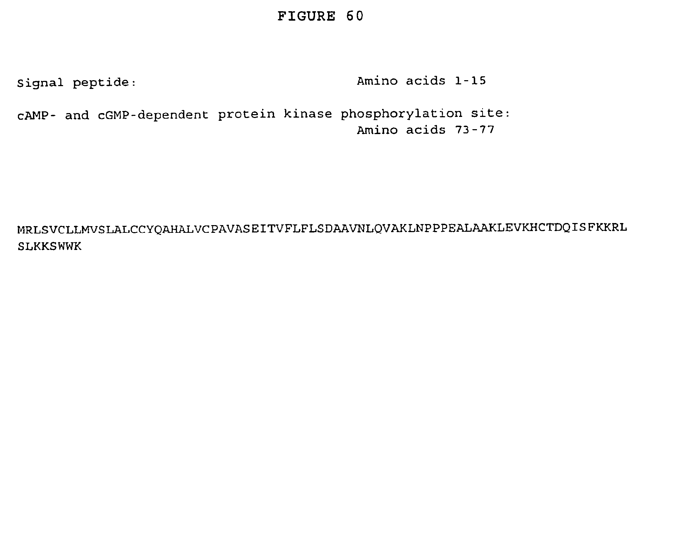

Figure 60 shows the amino acid sequence (SEQ ID NO:160) derived from the coding sequence of SEQ ID NO: 159 shown in Figure 59.

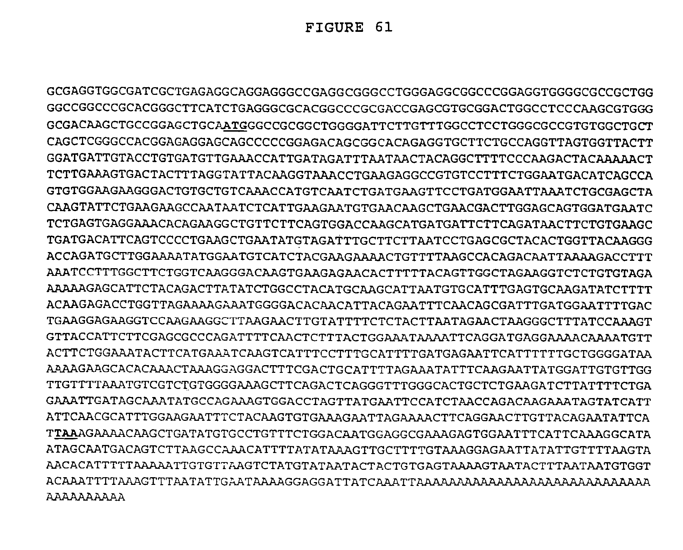

Figure 61 shows a nucleotide sequence (SEQ ID NO:161) of a native sequence PRO865 cDNA, wherein SEQ ID NO:161 is a clone designated herein as "DNA53974-1401".

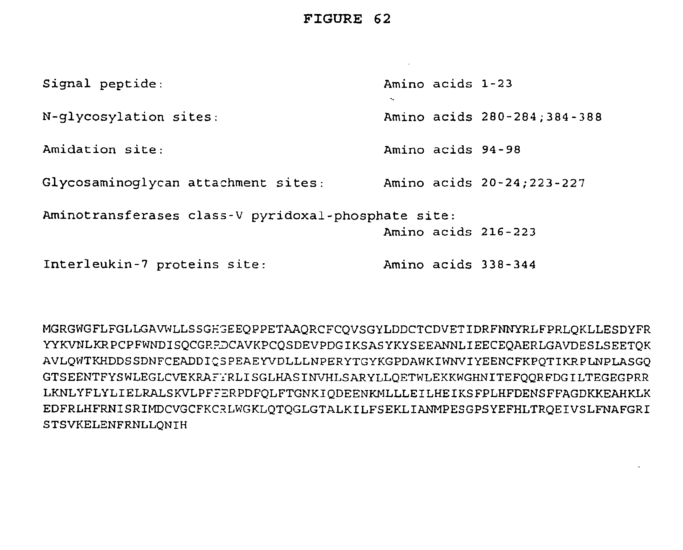

Figure 62 shows the amino acid sequence (SEQ ID NO:162) derived from the coding sequence of SEQ ID NO: 161 shown in Figure 61.

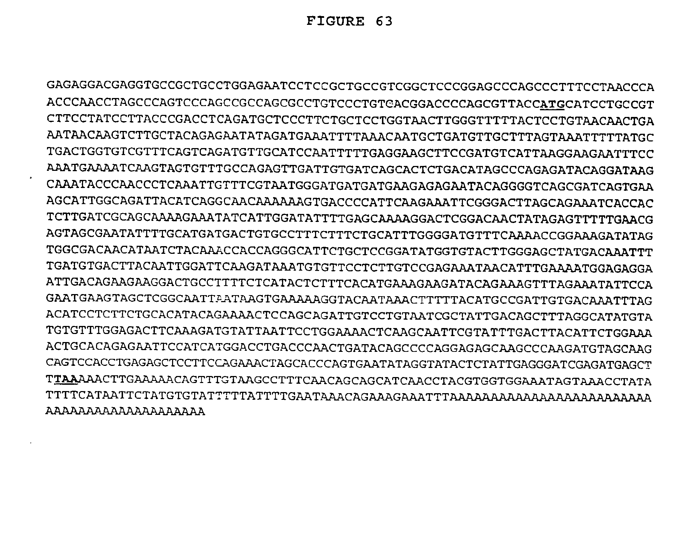

Figure 63 shows a nucleotide sequence (SEQ ID NO:169) of a native sequence PRO1075 cDNA, wherein SEQ ID NO: 169 is a clone designated herein as "DNA57689-1385".

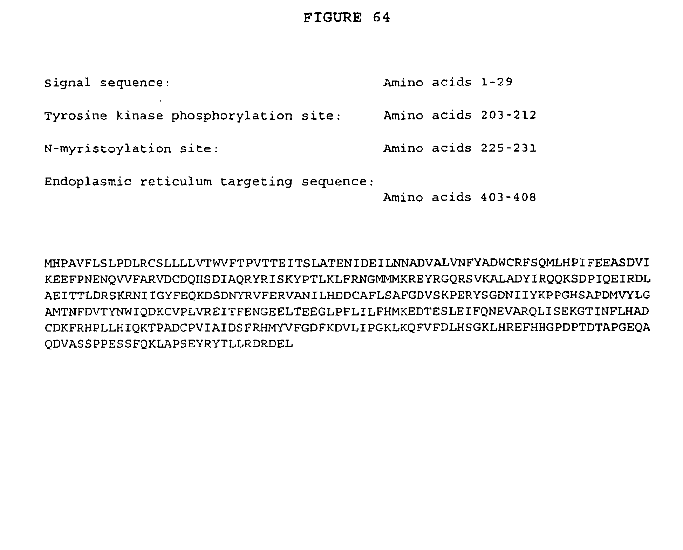

Figure 64 shows the amino acid sequence (SEQ ID NO:170) derived from the coding sequence of SEQ ID NO:169 shown in Figure 63.

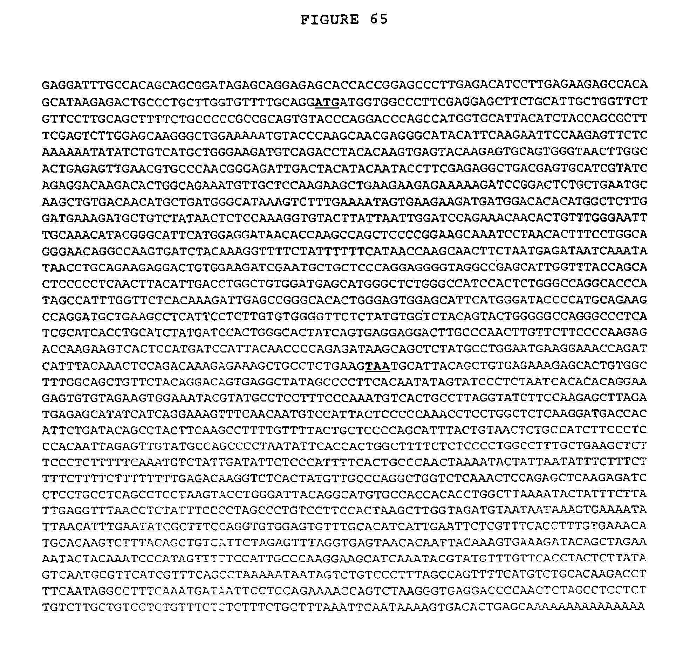

Figure 65 shows a nucleotide sequence (SEQ ID NO:180) of a native sequence PRO1126 cDNA, wherein SEQ ID NO:180 is a clone designated herein as "DNA60615-1483".

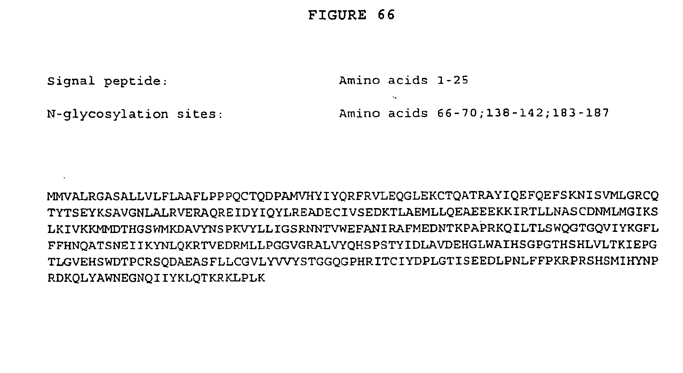

Figure 66 shows the amino acid sequence (SEQ ID NO:181) derived from the coding sequence of SEQ ID NO:180 shown in Figure 65.

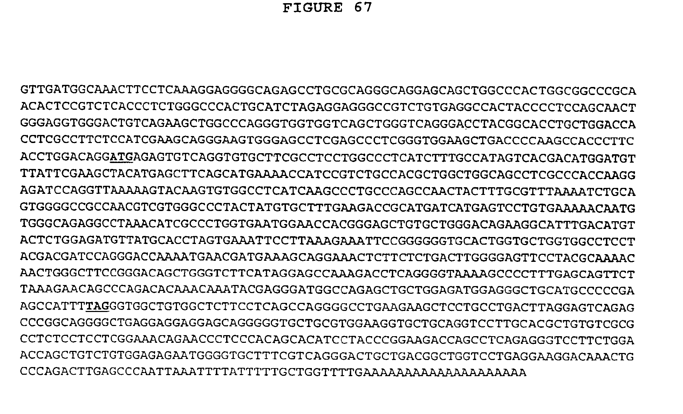

Figure 67 shows a nucleotide sequence (SFQ ID NO:182) of a native sequence PRO1130 cDNA, wherein SEQ ID NO:182 is a clone designated herein as "DNA59814-1486".

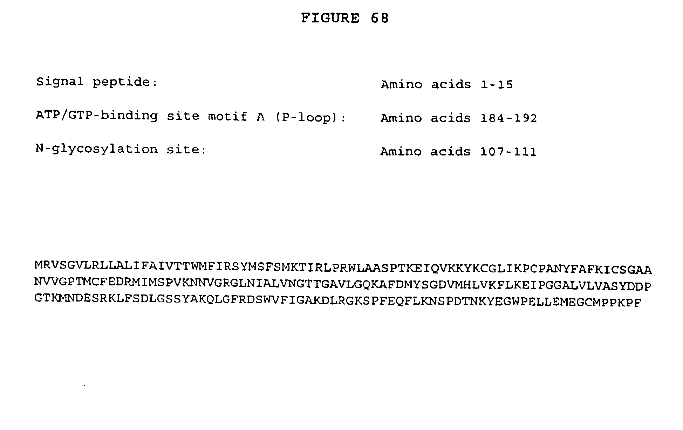

Figure 68 shows the amino acid sequence (SEQ ID NO:183) derived from the coding sequence of SEQ ID NO:182 shown in Figure 67.

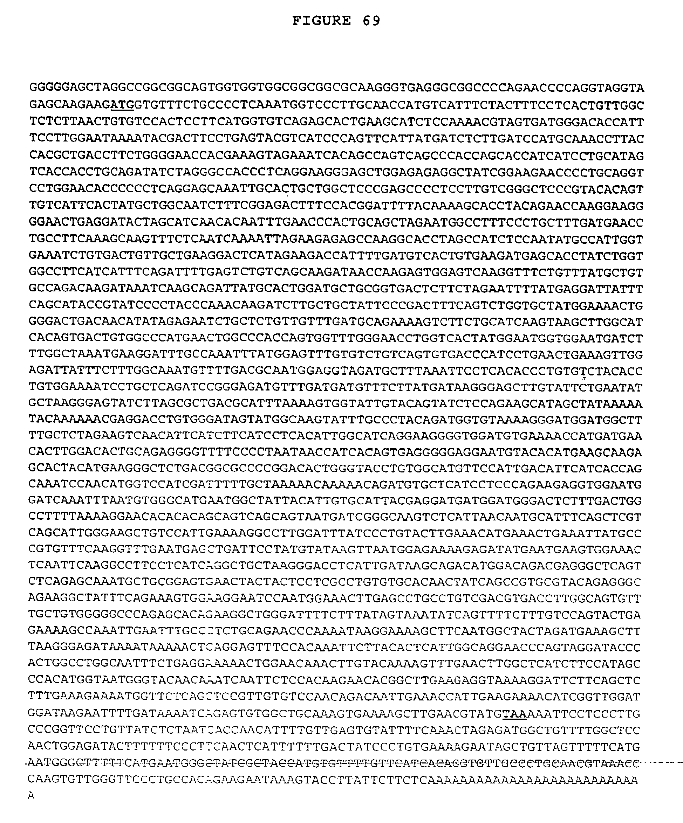

Figure 69 shows a nucleotide sequence (SEQ ID NO:190) of a native sequence PRO1154 cDNA, wherein SEQ ID NO:190 is a clone designated herein as "DNA59846-1503".

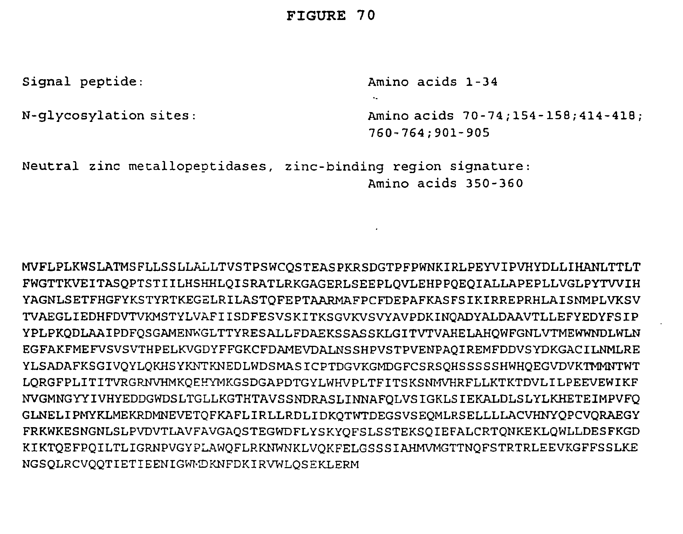

Figure 70 shows the amino acid sequence (SEQ ID NO:191) derived from the coding sequence of SEQ ID NO:190 shown in Figure 69.

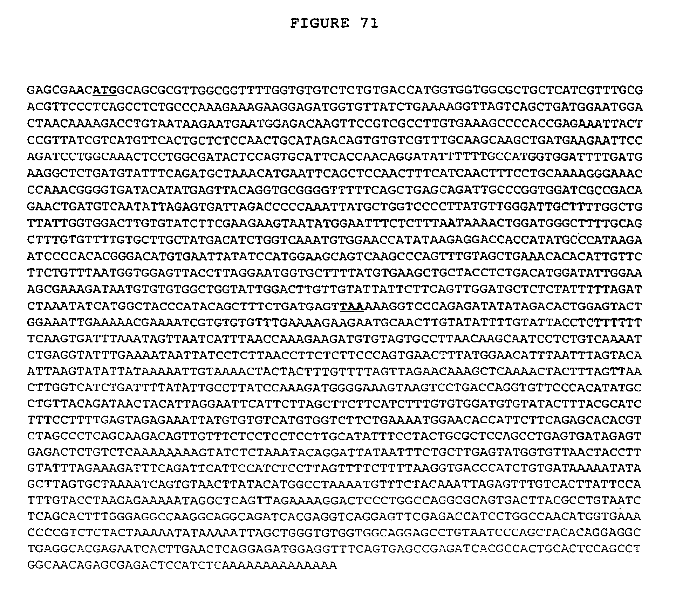

Figure 71 shows a nucleotide sequence (SEQ ID NO:192) of a native sequence PRO1244 cDNA, wherein SEQ ID NO:192 is a clone designated herein as "DNA64883-1526".

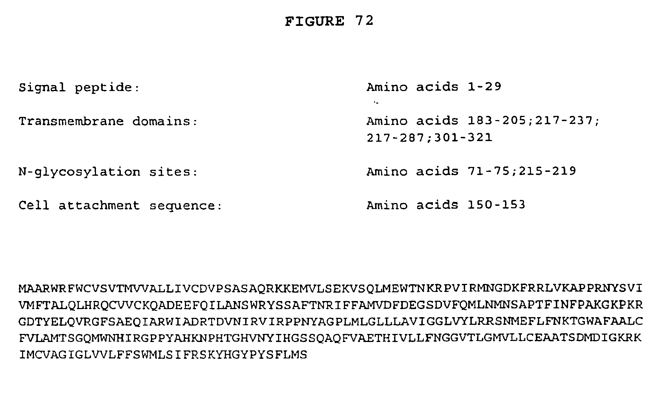

Figure 72 shows the amino acid sequence (SEQ ID NO:193) derived from the coding sequence of SEQ ID NO:192 shown in Figure 71.

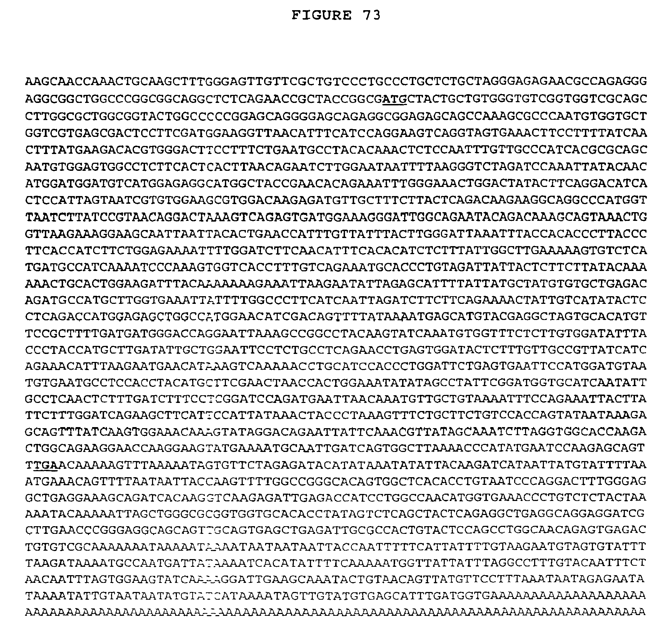

Figure 73 shows a nucleotide sequence (SEQ ID NO:194) of a native sequence PRO1246 cDNA, wherein SEQ ID NO:194 is a clone designated herein as "DNA64885-1529".

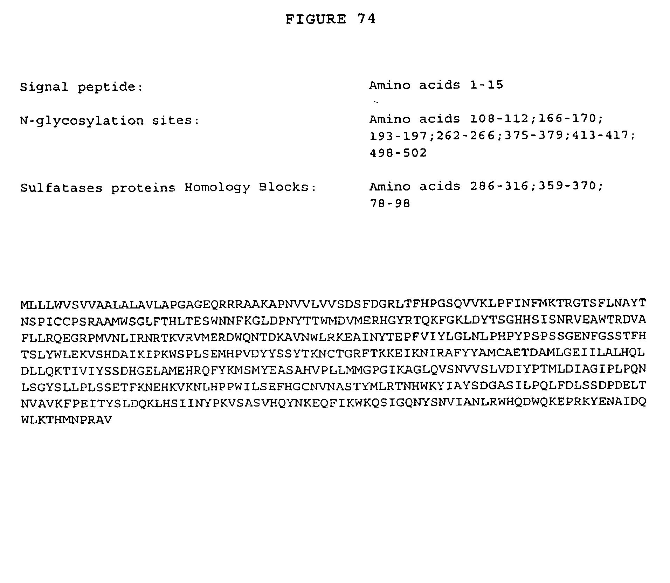

Figure 74 shows the amino acid sequence (SEQ ID NO:195) derived from the coding sequence of SEQ ID NO:194 shown in Figure 73.

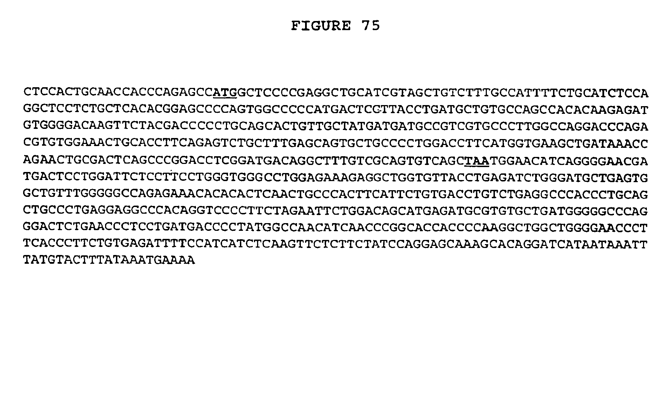

Figure 75 shows a nucleotide sequence (SEQ ID NO:196) of a native sequence PRO1274 cDNA, wherein SEQ ID NO:196 is a clone designated herein as "DNA64889-1541".

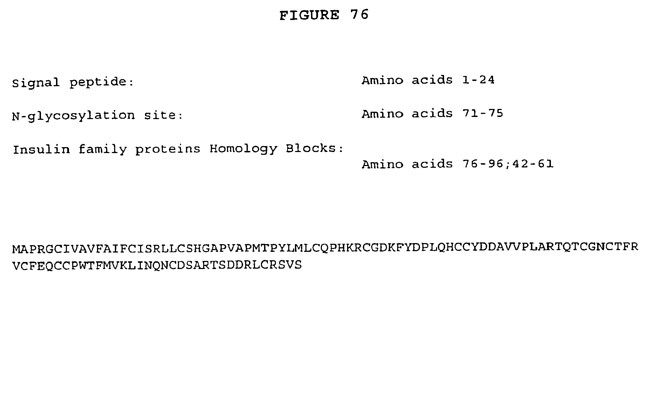

Figure 76 shows the amino acid sequence (SEQ ID NO:197) derived from the coding sequence of SEQ ID NO:196 shown in Figure 75.

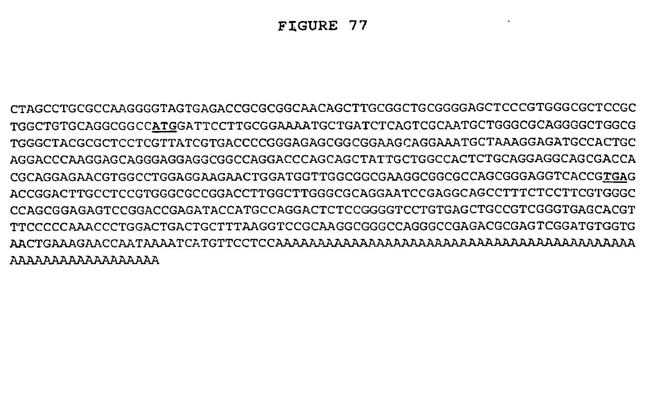

Figure 77 shows a nucleotide sequence (SEQ ID NO:198) of a native sequence PRO 1286 cDNA, wherein SEQ ID NO:198 is a clone designated herein as "DNA64903-1553".

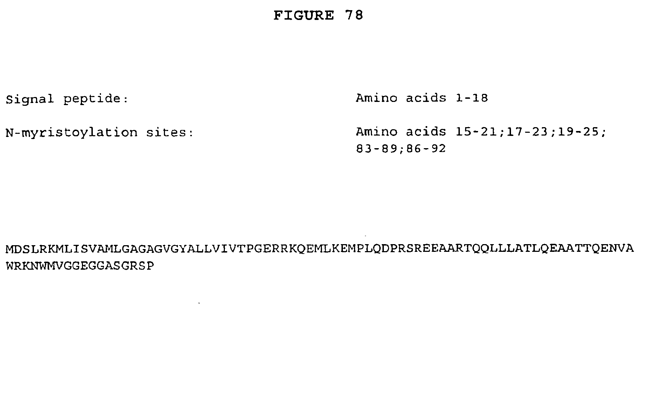

Figure 78 shows the amino acid sequence (SEQ ID NO:199) derived from the coding sequence of SEQ ID NO:198 shown in Figure 77.

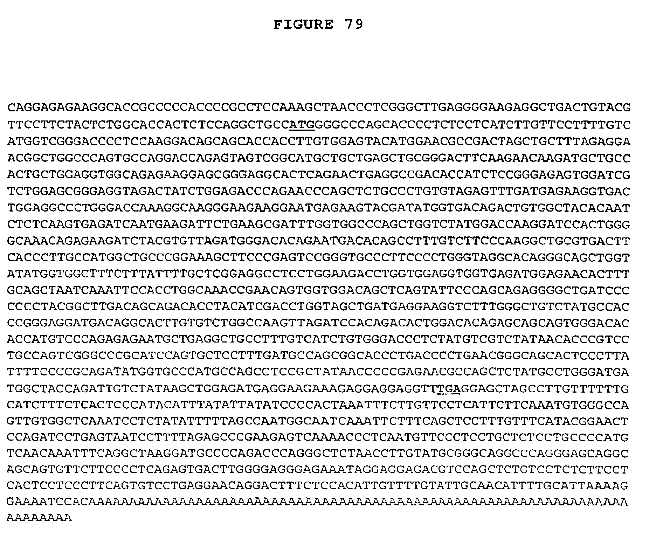

Figure 79 shows a nucleotide sequence (SEQ ID NO:200) of a native sequence PRO1294 cDNA, wherein SEQ ID NO:200 is a clone designated herein as "DNA64905-1558".

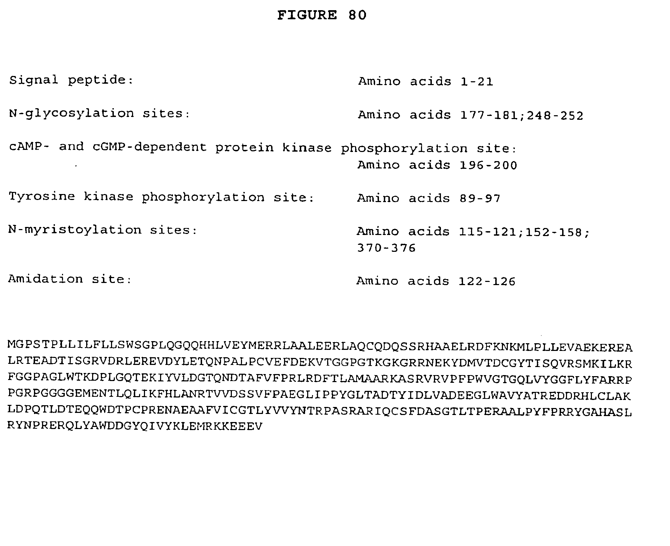

Figure 80 shows the amino acid sequence (SEQ ID NO:201) derived from the coding sequence of SEQ ID NO:200 shown in Figure 79.

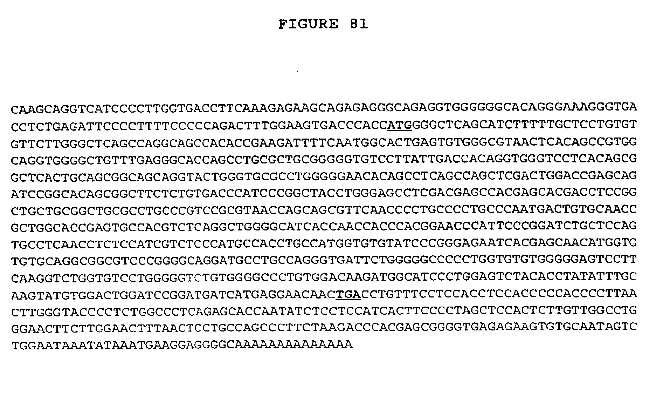

Figure 81 shows a nucleotide sequence (SEQ ID NO:202) of a native sequence PRO1303 cDNA, wherein SEQ ID NO:202 is a clone designated herein as "DNA65409-1566".

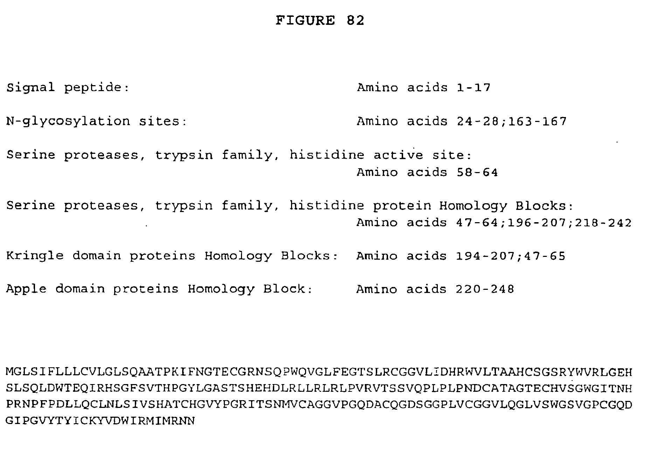

Figure 82 shows the amino acid sequence (SEQ ID NO:203) derived from the coding sequence of SEQ ID NO:202 shown in Figure 81.

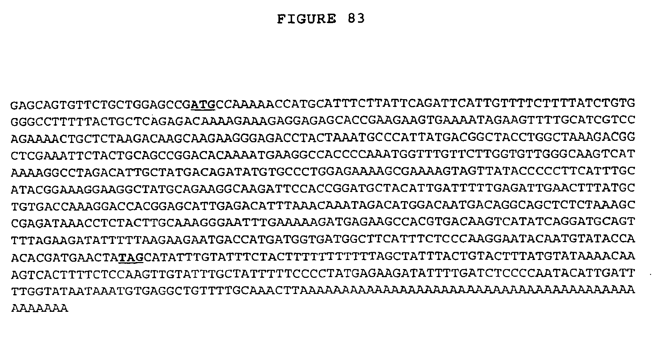

Figure 83 shows a nucleotide sequence (SEQ ID NO:204) of a native sequence PRO1304 cDNA, wherein SEQ ID NO:204 is a clone designated herein as "DNA65406-1567".

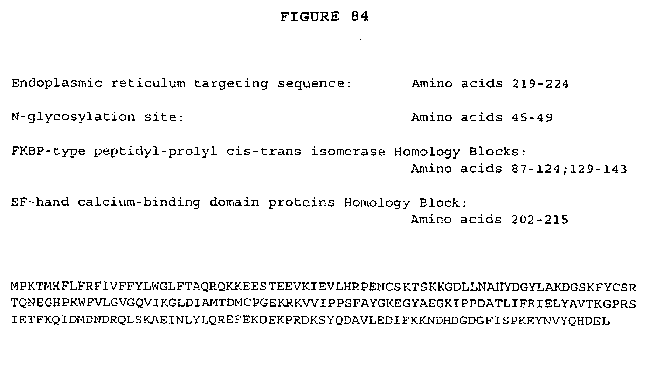

Figure 84 shows the amino acid sequence (SEQ ID NO:205) derived from the coding sequence of SEQ ID NO:204 shown in Figure 83.

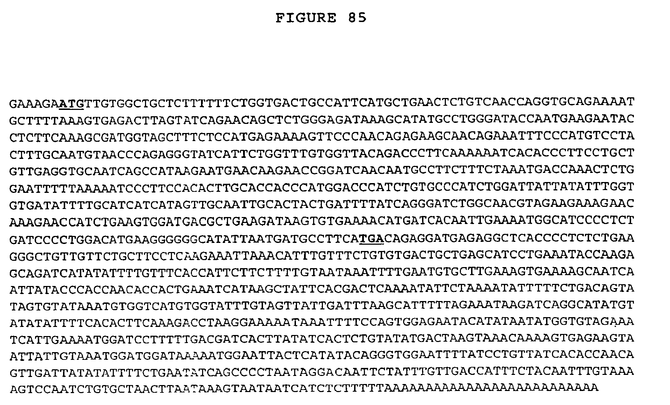

Figure 85 shows a nucleotide sequence (SEQ ID NO:213) of a native sequence PRO1312 cDNA, wherein SEQ ID NO:213 is a clone designated herein as "DNA61873-1574".

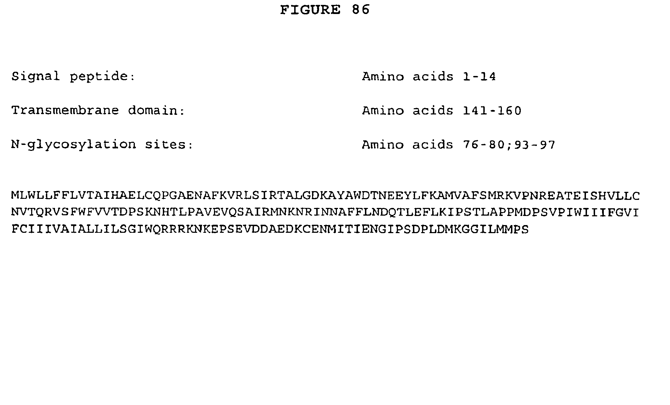

Figure 86 shows the amino acid sequence (SEQ ID NO:214) derived from the coding sequence of SEQ ID NO:213 shown in Figure 85.

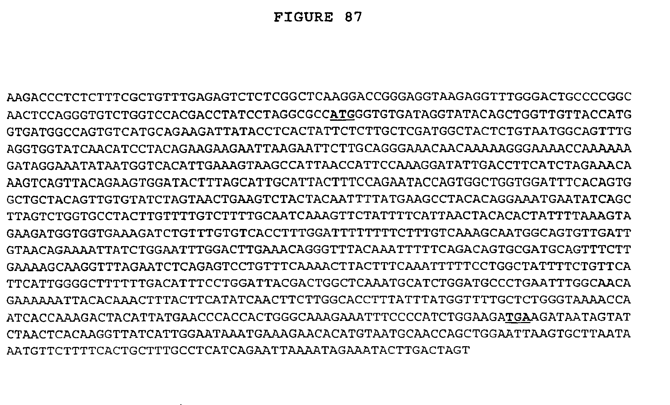

Figure 87 shows a nucleotide sequence (SEQ ID NO:215) of a native sequence PRO1313 cDNA, wherein SEQ ID NO:215 is a clone designated herein as "DNA64966-1575".

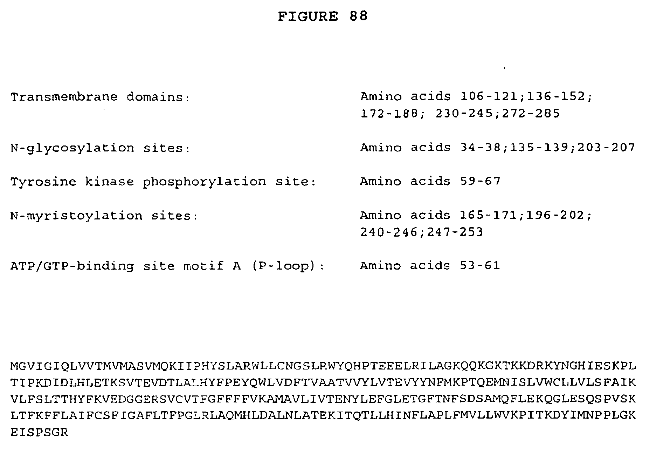

Figure 88 shows the amino acid sequence (SEQ ID NO:216) derived from the coding sequence of SEQ ID NO:215 shown in Figure 87.

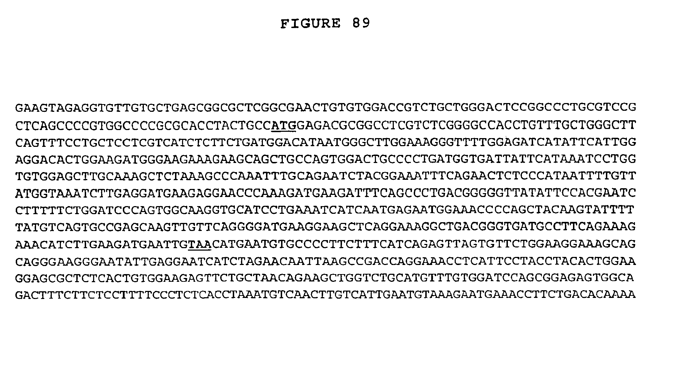

Figure 89 shows a nucleotide sequence (SEQ ID NO:217) of a native sequence PRO1376 cDNA, wherein SEQ ID NO:217 is a clone designated herein as "DNA67300-1605".

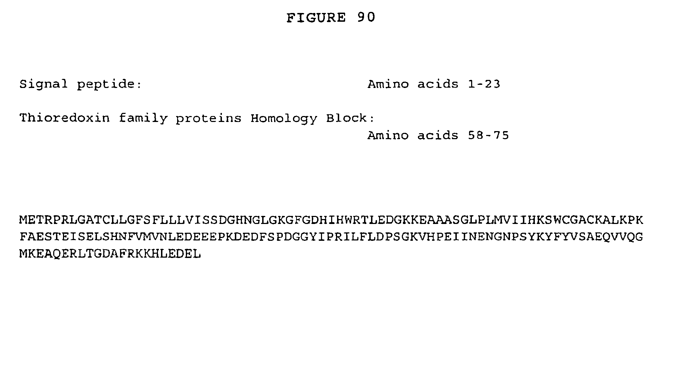

Figure 90 shows the amino acid sequence (SEQ ID NO:218) derived from the coding sequence of SEQ ID NO:217 shown in Figure 89.

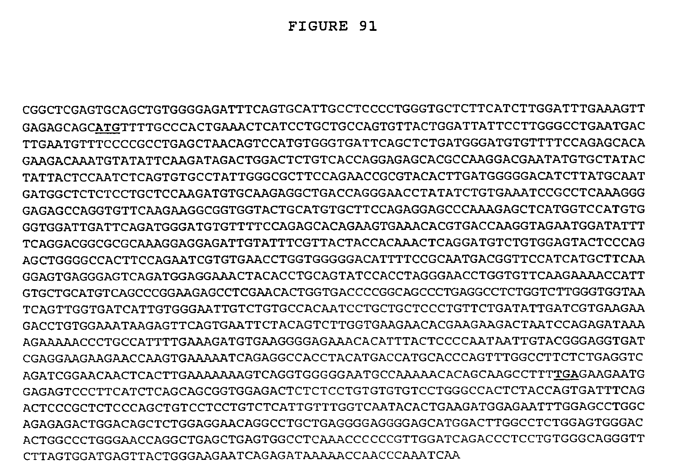

Figure 91 shows a nucleotide sequence (SEQ ID NO:219) of a native sequence PRO1387 cDNA, wherein SEQ ID NO:219 is a clone designated herein as "DNA68872-1620".

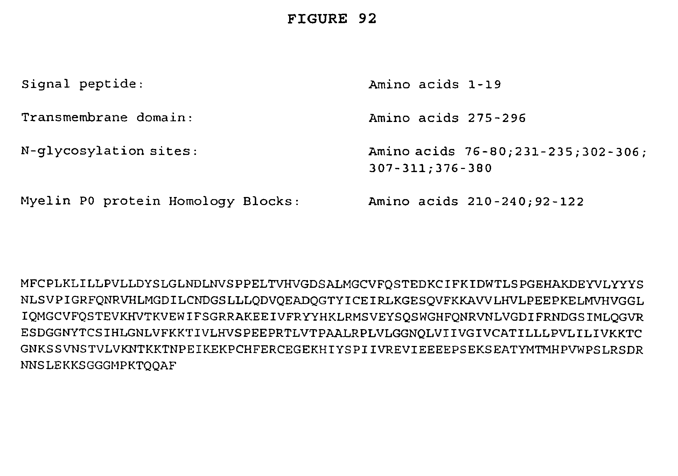

Figure 92 shows the amino acid sequence (SEQ ID NO:220) derived from the coding sequence of SEQ ID NO:219 shown in Figure 91.

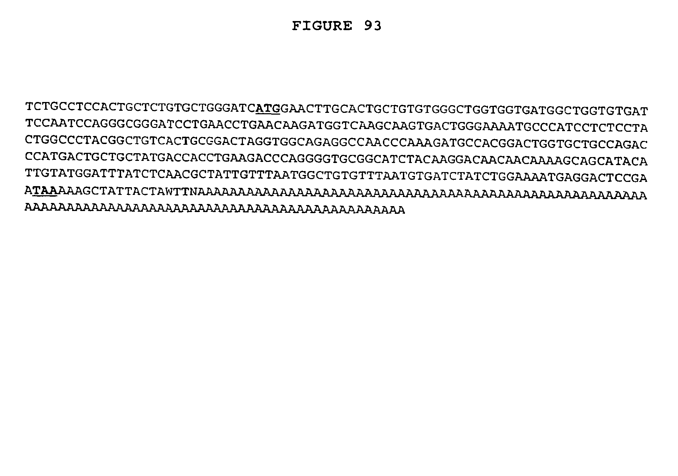

Figure 93 shows a nucleotide sequence (SEQ ID NO:221) of a native sequence PRO1561 cDNA, wherein SEQ ID NO:221 is a clone designated herein as "DNA76538-1670".

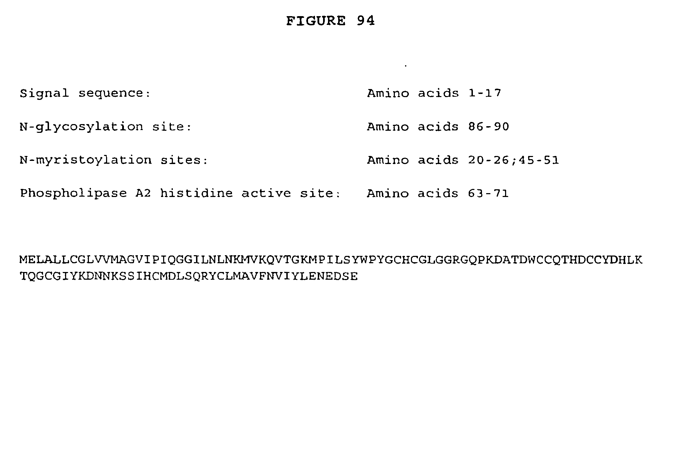

Figure 94 shows the amino acid sequence (SEQ ID NO:222) derived from the coding sequence of SEQ ID NO:221 shown in Figure 93.

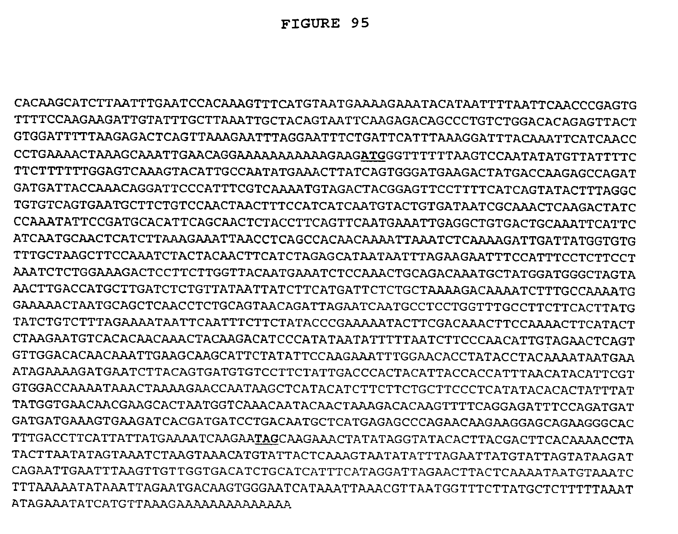

Figure 95 shows a nucleotide sequence (SEQ ID NO:226) ofa native sequence PRO216 cDNA, wherein SEQ ID NO:226 is a clone designated herein as "DNA33087".

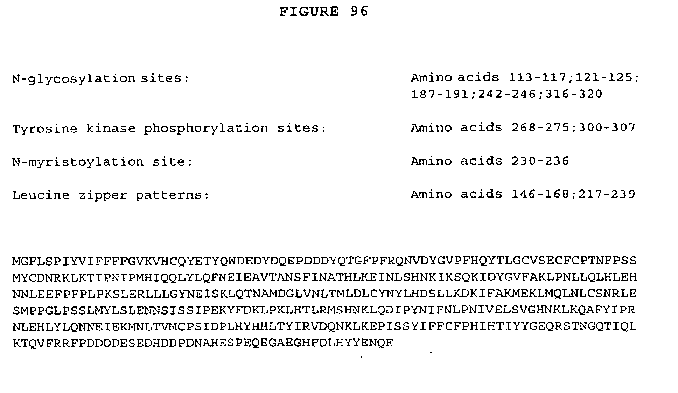

Figure 96 shows the amino acid sequence (SEQ ID NO:227) derived from the coding sequence of SEQ ID NO:226 shown in Figure 95.

## Detailed Description of the Invention

### I. Definitions

The phrases "cardiovascular, endothelial and angiogenic disorder", "cardiovascular, endothelial and angiogenic dysfunction", "cardiovascular, endothelial or angiogenic disorder'' and "cardiovascular, endothelial or angiogenic disfunction" are used interchangeably and refer in part to systemic disorders that affect vessels, such as diabetes mellitus, as well as diseases of the vessels themselves, such as of the arteries, capillaries, veins, and/or lymphatics. This would include indications that stimulate angiogenesis and/or cardiovascularization, and those thai inhibit angiogenesis and/or cardiovascularization. Such disorders include, for example, arterial disease, such as atherosclerosis, hypertension, inflammatory vasculitides, Reynaud's disease and Reynaud's phenomenon, aneurysms, and arterial restenosis: venous and lymphatic disorders such as thrombophlebitis, lymphangitis, and lymphedema: and other vascular disorders such as peripheral vascular disease, cancer such as vascular tumors, *e.g.,* hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma, tumor angiogenesis, trauma such as wounds, burns, and other injured tissue, implant fixation, scarring, ischemia reperfusion injury, rheumatoid arthritis, cerebrovascular disease, renal diseases such as acute renal failure, and osteoporosis. This would also include angina, myocardial infarctions such as acute myocardial infarctions, cardiac hypertrophy, and heart failure such as CHF.

"Hypertrophy'', as used herein, is defined as an increase in mass of an organ or structure independent of natural growth that does not involve tumor formation. Hypertrophy of an organ or tissue is due either to an increase in the mass of the individual cells (true hypertrophy), or to an increase in the number of cells making up the tissue (hyperplasia), or both. Certain organs, such as the heart, lose the ability to divide shortly after birth. Accordingly, "cardiac hypertrophy" is defined as an increase in mass of the heart, which, in adults, is characterized by an increase in myocyte cell size and contractile protein content without concomitant cell division. The character of the stress responsible for inciting the hypertrophy, (e.g., increased preload, increased afterload, loss of myocytes, as in myocardial infarction, or primary depression of contractility), appears to play a critical role in determining the nature of the response. The early stage of cardiac hypertrophy is usually characterized morphologically by increases in the size of myofibrils and mitochondria, as well as by enlargement of mitochondria and nuclei. At this stage, while muscle cells are larger than normal, cellular organization is largely preserved. At a more advanced stage of cardiac hypertrophy, there are preferential increases in the size or number of specific organelles, such as mitochondria, and new contractile elements are added in localized areas of the cells, in an irregular manner. Cells subjected to long-standing hypertrophy show more obvious disruptions in cellular organization, including markedly enlarged nuclei with highly lobulated membranes, which displace adjacent myofibrils and cause breakdown of normal Z-band registration. The phrase "cardiac hypertrophy" is used to include all stages of the progression of this condition, characterized by various degrees of structural damage of the heart muscle, regardless of the underlying cardiac disorder. Hence, the term also includes physiological conditions instrumental in the development of cardiac hypertrophy, such as elevated blood pressure, aortic stenosis, or myocardial infarction.

"Heart failure" refers to an abnormality of cardiac function where the heart does not pump blood at the rate needed for the requirements of metabolizing tissues. The heart failure can be caused by a number of factors, including ischemic, congenital, rheumatic, or idiopathic forms.

"Congestive heart failure" (CHF) is a progressive pathologic state where the heart is increasingly unable to supply adequate cardiac output (the volume of blood pumped by the heart over time) to deliver the oxygenated blood to peripheral tissues. As CHF progresses, structural and hemodynamic damages occur. While these damages have a variety of manifestations, one characteristic symptom is ventricular hypertrophy. CHF is a common end result of a number of various cardiac disorders.

"Myocardial infarction" generally results from atherosclerosis of the coronary arteries, often with superimposed coronary thrombosis. It may be divided into two major types: transmural infarcts, in which myocardial necrosis involves the full thickness of the ventricular wall, and subendocardia (nontransmural) infarcts, in which the necrosis involves the subendocardium, the intramural myocardium, or both, without extending all the way through the ventricular wall to the epicardium. Myocardial infarction is known to cause both a change in hemodynamic effects and an alteration in structure in the damaged and healthy zones of the heart. Thus, for example, myocardial infarction reduces the maximum cardiac output and the stroke volume of the heart. Also associated with myocardial infarction is a stimulation of the DNA synthesis occurring in the interstice as well as an increase in the formation of collagen in the areas of the heart not affected.

As a result of the increased stress or strain placed on the heart in prolonged hypertension due, for example, to the increased total peripheral resistance, cardiac hypertrophy has long been associated with "hypertension". A characteristic of the ventricle that becomes hypertrophic as a result of chronic pressure overload is an impaired diastolic performance. Fouad et al., J. Am. Coll. Cardiol., 4: 1500-1506 (1984); Smith et al., J. Am. Coll. Cardiol., 5: 869-874 (1985). A prolonged left ventricular relaxation has been detected in early essential hypertension, in spite of normal or supranonnal systolic function. Hartford et al., Hypertension, 6: 329-338 (1984). However, there is no close parallelism between blood pressure levels and cardiac hypertrophy. Although improvement in left ventricular function in response to antihypertensive therapy has been reported in humans, patients variously treated with a diuretic (hydrochlorothiazide), a β-blocker (propranolol), or a calcium channel blocker (diltiazem), have shown reversal of left ventricular hypertrophy, without improvement in diastolic function. Inouye et al., Am. J. Cardiol., 53: 1583-7 (1984).

Another complex cardiac disease associated with cardiac hypertrophy is "hypertrophic cardiomyopathy". This condition is characterized by a great diversity of morphologic, functional, and clinical features (Maron et al., N. Engl. J. Med., 316: 780-789 (1987); Spirito et al., N. Engl. J. Med., 320: 749-755 (1989); Louie and Edwards, Prog. Cardiovasc. Dis., 36: 275-308 (1994); Wigle et al., Circulation, 92: 1680-1692 (1995)), the heterogeneity of which is accentuated by the fact that it afflicts patients of all ages. Spirito et al., N. Engl. J. Med., 336: 775-785 (1997). The causative factors of hypertrophic cardiomyopathy are also diverse and little understood. In general, mutations in genes encoding sarcomeric proteins are associated with hypertrophic cardiomyopathy. Recent data suggest that β-myosin heavy chain mutations may account for approximately 30 to 40 percent of cases of familial hypertrophic cardiomyopathy. Watkins et al., N. Engl.J. Med., 326: 1108-1114 (1992); Schwartz et al, Circulation, 91: 532-540 (1995); Marian and Roberts, Circulation, 92: 1336-1347 (1995); Thierfclder et al., Cell, 77: 701-712 (1994); Watkins et al., Nat. Gen., 11: 434-437 (1995). Besides β-myosin heavy chain, other locations ofgenetic mutations include cardiac troponin T. alpha topomyosin, cardiac myosin binding protein C. essential myosin light chain, and regulatory myosin light chain. *See,* Malik and Watkins, Curr. Opin. Cardiol., 12: 295-302 (1997).

Supravalvular "aortic stenosis" is an inherited vascular disorder characterized by narrowing of the ascending aorta, but other arteries, including the pulmonary arteries, may also be affected. Untreated aortic stenosis may lead to increased intracardiac pressure resulting in myocardial hypertrophy and eventually heart failure and death. The pathogenesis of this disorder is not fully understood, but hypertrophy and possibly hyperplasia of medial smooth muscle are prominent features of this disorder. It has been reported that molecular variants of the elastin gene are involved in the development and pathogenesis of aortic stenosis. U.S. Patent No. 5,650,282 issued July 22, 1997.

"Valvular regurgitation" occurs as a result of heart diseases resulting in disorders of the cardiac valves. Various diseases, like rheumatic fever, can cause the shrinking or pulling apart of the valve orifice, while other diseases may result in endocarditis, an inflammation of the endocardium or lining membrane of the atrioventricular orifices and operation of the heart. Defects such as the narrowing of the valve stenosis or the defective closing of the valve result in an accumulation of blood in the heart cavity or regurgitation of blood past the valve. If uncorrected, prolonged valvular stenosis or insufficiency may result in cardiac hypertrophy and associated damage to the heart muscle, which may eventually necessitate valve replacement.

The treatment of all these, and other cardiovascular, endothelial and angiogenic disorders, which may or may not be accompanied by cardiac hypertrophy, is encompassed by the present invention.

The terms "cancer", "cancerous", and "malignant" refer to or describe the physiological condition in mammals that is typically characterized by unregulated cell growth. Examples of cancer include but are not limited to, carcinoma including adenocarcinoma, lymphoma, blastoma, melanoma, sarcoma, and leukemia. More particular examples of such cancers include squamous cell cancer, small-cell lung cancer, non-small cell lung cancer, gastrointestinal cancer, Hodgkin's and non-Hodgkin's lymphoma, pancreatic cancer, glioblastoma, cervical cancer, ovarian cancer, liver cancer such as hepatic carcinoma and hepatoma, bladder cancer, breast cancer, colon cancer, colorectal cancer, endometrial carcinoma, salivary gland carcinoma, kidney cancer such as renal cell carcinoma and Wilms' tumors, basal cell carcinoma, melanoma, prostate cancer, vulval cancer, thyroid cancer, testicular cancer, esophageal cancer, and various types of head and neck cancer. The preferred cancers for treatment herein are breast, colon, lung, melanoma, ovarian, and others involving vascular tumors as noted above.

The term "cytotoxic agent" as used herein refers to a substance that inhibits or prevents the function of cells and/or causes destruction of cells. The term is intended to include radioactive isotopes (*e.g.*, $^{131}I$, $^{125}I$, $^{90}Y$, and $^{186}Re$),

chemotherapeutic agents, and toxins such as enzymatically active toxins of bacterial, fungal, plant, oranimal origin, or fragments thereof.

A "chemotherapeutic agent" is a chemical compound useful in the treatment of cancer. Examples of chemotherapeutic agents include alkylating agents, folic acid antagonists, anti-metabolites of nucleic acid metabolism, antibiotics, pyrimidine analogs, 5-fluorouracil, cisplatin, purine nucleosides, amines, amino acids, triazol nucleosides, or corticosteroids. Specific examples include Adriamycin, Doxorubicin, 5-Fluorouracil, Cytosine arabinoside ("Ara-C"), Cyclophosphamide, Thiotepa, Busulfan, Cytoxin, Taxol, Toxotere, Methotrexate, Cisplatin, Melphalan, Vinblastine, Bleomycin, Etoposide, Ifosfamide, Mitomycin C, Mitoxantrone, Vincreistine, Vinorelbine, Carboplatin. Teniposide, Daunomycin, Carminomycin, Aminopterin, Dactinomycin, Mitomycins, Esperamicins (sce U.S. Pat. No. 4.675,187), Melphalan, and other related nitrogen mustards. Also included in this definition are hormonal agents that act to regulate or inhibit hormone action on tumors, such as tamoxifen and onapristone.

A "growth-inhibitory agent" when used herein refers to a compound or composition that inhibits growth of a cell, such as an Wnt-overexpressing cancer cell, either *in vitro* or *in vivo.* Thus, the growth-inhibitory agent is one which significantly reduces the percentage of malignant cells in S phase. Examples of growth-inhibitory agents include agents that block cell cycle progression (at a place other than S phase), such as agents that induce G 1 arrest and M-phase arrest. Classical M-phase blockers include the vincas (vincristine and vinblastine), taxol, and topo II inhibitors such as doxorubicin, daunorubicin, etoposide, and bleomycin. Those agents that arrest G 1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer. Mendelsohn and Israel, eds., Chapter I, entitled "Cell cycle regulation, oncogenes, and antineoplasticdrugs" by Murakami et al. (WB Saunders: Philadelphia. 1995), especially p. 13. Additional examples include tumor necrosis factor (TNF), an antibody capable of inhibiting or neutralizing the angiogenic activity of acidic or basic FGF or hepatocyte growth factor (HGF), an antibody capable of inhibiting or neutralizing the coagulant activities of tissue factor, protein C, or protein S (*see,* WO 91/01753. published 21 February 1991), or an antibody capable of binding to HER2 receptor (WO 89/06692), such as the 4D5 antibody (and functional equivalents thereof) *(e.g.,* WO 92/22653).

"Treatment" is an intervention performed with the intention of preventing the development or altering the pathology of a cardiovascular, endothelial, and angiogenic disorder. The concept of treatment is used in the broadest sense, and specifically includes the prevention (prophylaxis), moderation, reduction, and curing of cardiovascular, endothelial, and angiogenic disorders of any stage. Accordingly, "treatment" refers to both therapeutic treatment and prophylactic or preventative measures, wherein the object is to prevent or slow down (lessen) a cardiovascular, endothclial, and angiogenic disorder such as hypertrophy. Those in need of treatment include those already with the disorder as well as those prone to have the disorder or those in whom the disorder is to be prevented. The disorder may result from any cause, including idiopathic, cardiotrophic, or myotrophic causes, or ischemia or ischemic insults, such as myocardial infarction.

"Chronic" administration refers to administration of the agent(s) in a continuous mode as opposed to an acute mode, so as to maintain the initial effect, such as an anti-hypertrophic effect, for an extended period of time.

"Mammal" for purposes of treatment refers to any animal classified as a mammal, including humans, domestic and farm animals, and zoo, sports, or pet animals, such as dogs, horses, cats, cows, sheep, pigs, etc. Preferably, the mammal is human.

Administration "in combination with" one or more further therapeutic agents includes simultaneous (concurrent) and consecutive administration in any order.

The phrase "cardiovascular, endothelial or angiogenic agents" refers generically to any drug that acts in treating cardiovascular, endothelial, and angiogenic disorders. Examples of cardiovascular agents are those that promote vascular homeostasis by modulating blood pressure, heart rate, heart contractility, and endothelial and smooth muscle biology, all of which factors have a role in cardiovascular disease. Specific examples of these include angiotensin-II receptor antagonists; endothelin receptor antagonists such as, for example, BOSENTAN™ and MOXONODIN™; interferon-gamma (IFN-γ); des-aspartate-angiotensin I; thrombolytic agents. *e.g.*, streptokinase, urokinase, t-PA, and a t-PA variant specifically designed to have longer half-life and very high fibrin specificity, TNK t-PA (a T103N, N 117Q, KHRR (296-299)AAAA t-PA variant, Keyt et al., Proc. Natl. Acad. Sci. USA 91, 3670-3674 (1994)); inotropic or hypertensive agents such as digoxigenin and β-adrenergic receptor blocking agents, *e.g.*, propranolol, timolol, tertalolol, carteolol, nadolol, betaxolol, penbutolol, acetobutolol, atenolol, metoprolol, and carvedilol; angiotensin converting enzyme (ACE) inhibitors, *e.g.*, quinapril, captopril, enalapril, ramipril, benazepril, fosinopril, and lisinopril; diuretics, *e.g.,* chlorothiazide, hydrochlorothiazide, hydroflumethazide, methylchlothiazide, benzthiazide, dichlorphenamide, acetazolamide, and indapamide; and calcium channel blockers, *e g.,* diltiazem, nitedipine, verapamil, nicardipine. One preferred category of this type is a therapeutic agent used for the treatment of cardiac hypertrophy or of a physiological condition instrumental in the development of cardiac hypertrophy, such as elevated blood pressure, aortic stenosis, or myocardial infarction.

"Angiogenic agents" and "endothelial agents" are active agents that promote angiogenesis and/or endothelial cell growth, or, if applicable, vasculogenesis. This would include factors that accelerate wound healing, such as growth hormone, insulin-like growth factor-I(IGF-1), VEGF, VIGF, PDGF, epidermal growth factor (EGF), CTGF and members of its family. FGF, and TGF-$\alpha$ and TGF-$\beta$.

"Angiostatic agents" are active agents that inhibit angiogenesis or vasculogenesis or otherwise inhibit or prevent growth of cancer cells. Examples include antibodies or other antagonists to angiogenic agents as defined above, such as antibodies to VEGF. They additionally include cytotherapeutic agents such as cytotoxic agents, chemotherapeutic agents, growth-inhibitory agents, apoptotic agents, and other agents to treat cancer, such as anti-HER-2, anti-CD20, and other bioactive and organic chemical agents.

In a pharmacological sense, in the context of the present invention, a "therapeutically effective amount" of an active agent such as a PRO polypeptide or agonist or antagonist thereto or an anti-PRO antibody, refers to an amount effective in the treatment of a cardiovascular, endothelial or angiogenic disorder in a mammal and can be determined empirically.

As used herein, an "effective amount" of an active agent such as a PRO polypeptide or agonist or antagonist thereto or an anti-PRO antibody, refers to an amount effective for carrying out a stated purpose, wherein such amounts may be determined empirically for the desired effect.

The terms "PRO polypeptide" and "PRO" as used herein and when immediately followed by a numerical designation refer to various polypeptides, wherein the complete designation (*i.e.,* PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (*e.g.,* an extracellular domain sequence), naturally-occurring variant forms (*e.g.*, alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are comtemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (*e.g.*, Nielsen et al., Prot. Eng., 10:1-6 (1997) and von Heinje et al., Nucl. Acids Res., 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and most preferably at least about 99% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, more often at least about 150 amino acids in length, more often at least about 200 amino acids in length, more often at least about 300 amino acids in length, or more.

As shown below, Table I provides the complete source code for the ALIGN-2 sequence comparison computer program. This source code may be routinely compiled for use on a UNIX operating system to provide the ALIGN-2 sequence comparison computer program.

In addition, Tables 2A-2D show hypothetical exemplifications for using the below described method to determine % amino acid sequence identity (Tables 2A-2B) and % nucleic acid sequence identity (Tables 2C-2D) using the ALIGN-2 sequence comparison computer program, wherein "PRO" represents the amino acid sequence of a hypothetical PRO polypeptide of interest, "Comparison Protein" represents the amino acid sequence of a polypeptide against which the "PRO" polypeptide of interest is being compared, "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, "X", "Y", and "Z" each represent different hypothetical amino acid residues and "N", "L" and "V" each represent different hypothetical nucleotides.

## Table 1

```
/*
 *
 * C-C increased from 12 to 15
 * Z is average of EQ
 * B is average of ND
 * match with stop is _M; stop-stop = 0; J (joker) match = 0
 */
#define   _M      -8        /* value of a match with a stop */

int   _day[26][26] = {
/*      A  B  C  D  E  F  G  H  I  J  K  L  M  N  O  P  Q  R  S  T  U  V  W  X  Y  Z */
/* A */    { 2, 0,-2, 0, 0,-4, 1,-1,-1, 0,-1,-2,-1, 0,_M, 1, 0,-2, 1, 1, 0, 0,-6, 0,-3, 0},
/* B */    { 0, 3,-4, 3, 2,-5, 0, 1,-2, 0, 0,-3,-2, 2,_M,-1, 1, 0, 0, 0, 0,-2,-5, 0,-3, 1},
/* C */    {-2,-4,15,-5,-5,-4,-3,-3,-2, 0,-5,-6,-5,-4,_M,-3,-5,-4, 0,-2, 0,-2,-8, 0, 0,-5},
/* D */    { 0, 3,-5, 4, 3,-6, 1, 1,-2, 0, 0,-4,-3, 2,_M,-1, 2,-1, 0, 0, 0,-2,-7, 0,-4, 2},
/* E */    { 0, 2,-5, 3, 4,-5, 0, 1,-2, 0, 0,-3,-2, 1,_M,-1, 2,-1, 0, 0, 0,-2,-7, 0,-4, 3},
/* F */    {-4,-5,-4,-6,-5, 9,-5,-2, 1, 0,-5, 2, 0,-4,_M,-5,-5,-4,-3,-3, 0,-1, 0, 0, 7,-5},
/* G */    { 1, 0,-3, 1, 0,-5, 5,-2,-3, 0,-2,-4,-3, 0,_M,-1,-1,-3, 1, 0, 0,-1,-7, 0,-5, 0},
/* H */    {-1, 1,-3, 1, 1,-2,-2, 6,-2, 0, 0,-2,-2, 2,_M, 0, 3, 2,-1,-1, 0,-2,-3, 0, 0, 2},
/* I */    {-1,-2,-2,-2,-2, 1,-3,-2, 5, 0,-2, 2, 2,-2,_M,-2,-2,-2,-1, 0, 0, 4,-5, 0,-1,-2},
/* J */    { 0, 0, 0, 0, 0, 0, 0, 0, 0, 0, 0, 0, 0, 0,_M, 0, 0, 0, 0, 0, 0, 0, 0, 0, 0, 0},
/* K */    {-1, 0,-5, 0, 0,-5,-2, 0,-2, 0, 5,-3, 0, 1,_M,-1, 1, 3, 0, 0, 0,-2,-3, 0,-4, 0},
/* L */    {-2,-3,-6,-4,-3, 2,-4,-2, 2, 0,-3, 6, 4,-3,_M,-3,-2,-3,-3,-1, 0, 2,-2, 0,-1,-2},
/* M */    {-1,-2,-5,-3,-2, 0,-3,-2, 2, 0, 0, 4, 6,-2,_M,-2,-1, 0,-2,-1, 0, 2,-4, 0,-2,-1},
/* N */    { 0, 2,-4, 2, 1,-4, 0, 2,-2, 0, 1,-3,-2, 2,_M,-1, 1, 0, 1, 0, 0,-2,-4, 0,-2, 1},
/* O */    {_M,_M,_M,_M,_M,_M,_M,_M,_M,_M,_M,_M,_M,_M, 0,_M,_M,_M,_M,_M,_M,_M,_M,_M,_M,_M},
/* P */    { 1,-1,-3,-1,-1,-5,-1, 0,-2, 0,-1,-3,-2,-1,_M, 6, 0, 0, 1, 0, 0,-1,-6, 0,-5, 0},
/* Q */    { 0, 1,-5, 2, 2,-5,-1, 3,-2, 0, 1,-2,-1, 1,_M, 0, 4, 1,-1,-1, 0,-2,-5, 0,-4, 3},
/* R */    {-2, 0,-4,-1,-1,-4,-3, 2,-2, 0, 3,-3, 0, 0,_M, 0, 1, 6, 0,-1, 0,-2, 2, 0,-4, 0},
/* S */    { 1, 0, 0, 0, 0,-3, 1,-1,-1, 0, 0,-3,-2, 1,_M, 1,-1, 0, 2, 1, 0,-1,-2, 0,-3, 0},
/* T */    { 1, 0,-2, 0, 0,-3, 0,-1, 0, 0, 0,-1,-1, 0,_M, 0,-1,-1, 1, 3, 0, 0,-5, 0,-3, 0},
/* U */    { 0, 0, 0, 0, 0, 0, 0, 0, 0, 0, 0, 0, 0, 0,_M, 0, 0, 0, 0, 0, 0, 0, 0, 0, 0, 0},
/* V */    { 0,-2,-2,-2,-2,-1,-1,-2, 4, 0,-2, 2, 2,-2,_M,-1,-2,-2,-1, 0, 0, 4,-6, 0,-2,-2},
/* W */    {-6,-5,-8,-7,-7, 0,-7,-3,-5, 0,-3,-2,-4,-4,_M,-6,-5, 2,-2,-5, 0,-6,17, 0, 0,-6},
/* X */    { 0, 0, 0, 0, 0, 0, 0, 0, 0, 0, 0, 0, 0, 0,_M, 0, 0, 0, 0, 0, 0, 0, 0, 0, 0, 0},
/* Y */    {-3,-3, 0,-4,-4, 7,-5, 0,-1, 0,-4,-1,-2,-2,_M,-5,-4,-4,-3,-3, 0,-2, 0, 0,10,-4},
/* Z */    { 0, 1,-5, 2, 3,-5, 0, 2,-2, 0, 0,-2,-1, 1,_M, 0, 3, 0, 0, 0, 0,-2,-6, 0,-4, 4}
};
```

```
/*
 */
#include <stdio.h>
#include <ctype.h>

#define  MAXJMP     16      /* max jumps in a diag */
#define  MAXGAP     24      /* don't continue to penalize gaps larger than this */
#define  JMPS       1024    /* max jmps in an path */
#define  MX         4       /* save if there's at least MX-1 bases since last jmp */

#define  DMAT       3       /* value of matching bases */
#define  DMIS       0       /* penalty for mismatched bases */
#define  DINS0      8       /* penalty for a gap */
#define  DINS1      1       /* penalty per base */
#define  PINS0      8       /* penalty for a gap */
#define  PINS1      4       /* penalty per residue */

struct jmp {
        short               n[MAXJMP];      /* size of jmp (neg for dely) */
        unsigned short      x[MAXJMP];      /* base no. of jmp in seq x */
};                                          /* limits seq to 2^16 -1 */

struct diag {
        int         score;          /* score at last jmp */
        long                offset;         /* offset of prev block */
        short               ijmp;           /* current jmp index */
        struct jmp  jp;             /* list of jmps */
};

struct path {
        int spc;                    /* number of leading spaces */
        short       n[JMPS];        /* size of jmp (gap) */
        int         x[JMPS];        /* loc of jmp (last elem before gap) */
};

char    *ofile;                     /* output file name */
char    *namex[2];                  /* seq names: getseqs() */
char    *prog;                      /* prog name for err msgs */
char    *seqx[2];                   /* seqs: getseqs() */
int     dmax;                       /* best diag: nw() */
int     dmax0;                      /* final diag */
int     dna;                        /* set if dna: main() */
int     endgaps;                    /* set if penalizing end gaps */
int     gapx, gapy;                 /* total gaps in seqs */
int     len0, len1;                 /* seq lens */
int     ngapx, ngapy;               /* total size of gaps */
int     smax;                       /* max score: nw() */
int     *xbm;                       /* bitmap for matching */
long    offset;                     /* current offset in jmp file */
struct  diag    *dx;                /* holds diagonals */
struct  path    pp[2];              /* holds path for seqs */

char    *calloc(), *malloc(), *index(), *strcpy();
char    *getseq(), *g_calloc();
```

```
/* Needleman-Wunsch alignment program
 *
 * usage: progs file1 file2
 *   where file1 and file2 are two dna or two protein sequences.
 *   The sequences can be in upper- or lower-case an may contain ambiguity
 *   Any lines beginning with ';', '>' or '<' are ignored
 *   Max file length is 65535 (limited by unsigned short x in the jmp struct)
 *   A sequence with 1/3 or more of its elements ACGTU is assumed to be DNA
 *   Output is in the file "align.out"
 *
 * The program may create a tmp file in /tmp to hold info about traceback.
 * Original version developed under BSD 4.3 on a vax 8650
 */
#include "nw.h"
#include "day.h"

static    _dbval[26] = {
     1,14,2,13,0,0,4,11,0,0,12,0,3,15,0,0,0,5,6,8,8,7,9,0,10,0
};

static    _pbval[26] = {
     1, 2|(1<<('D'-'A'))|(1<<('N'-'A')), 4, 8, 16, 32, 64,
     128, 256, 0xFFFFFFF, 1<<10, 1<<11, 1<<12, 1<<13, 1<<14,
     1<<15, 1<<16, 1<<17, 1<<18, 1<<19, 1<<20, 1<<21, 1<<22,
     1<<23, 1<<24, 1<<25|(1<<('E'-'A'))|(1<<('Q'-'A'))
};

main(ac, av)                                                              main
     int ac;
     char       *av[];
{
     prog = av[0];
     if (ac != 3) {
          fprintf(stderr,"usage: %s file1 file2\n", prog);
          fprintf(stderr,"where file1 and file2 are two dna or two protein sequences.\n");
          fprintf(stderr,"The sequences can be in upper- or lower-case\n");
          fprintf(stderr,"Any lines beginning with ';' or '<' are ignored\n");
          fprintf(stderr,"Output is in the file \"align.out\"\n");
          exit(1);
     }
     namex[0] = av[1];
     namex[1] = av[2];
     seqx[0] = getseq(namex[0], &len0);
     seqx[1] = getseq(namex[1], &len1);
     xbm = (dna)? _dbval : _pbval;

     endgaps = 0;                    /* 1 to penalize endgaps */
     ofile = "align.out";            /* output file */

     nw();          /* fill in the matrix, get the possible jmps */
     readjmps();    /* get the actual jmps */
     print();       /* print stats, alignment */

     cleanup(0);    /* unlink any tmp files */
}
```

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()                                                                          nw
{
      char             *px, *py;            /* seqs and ptrs */
      int         *ndely, *dely;      /* keep track of dely */
      int         ndelx, delx;        /* keep track of delx */
      int         *tmp;               /* for swapping row0, row1 */
      int         mis;                /* score for each type */
      int         ins0, ins1;         /* insertion penalties */
      register         id;                  /* diagonal index */
      register         ij;                  /* jmp index */
      register         *col0, *col1;        /* score for curr, last row */
      register         xx, yy;              /* index into seqs */

      dx = (struct diag *)g_calloc("to get diags", len0+len1+1, sizeof(struct diag));

      ndely = (int *)g_calloc("to get ndely", len1+1, sizeof(int));
      dely = (int *)g_calloc("to get dely", len1+1, sizeof(int));
      col0 = (int *)g_calloc("to get col0", len1+1, sizeof(int));
      col1 = (int *)g_calloc("to get col1", len1+1, sizeof(int));
      ins0 = (dna)? DINS0 : PINS0;
      ins1 = (dna)? DINS1 : PINS1;

      smax = -10000;
      if (endgaps) {
          for (col0[0] = dely[0] = -ins0, yy = 1; yy <= len1; yy++) {
                    col0[yy] = dely[yy] = col0[yy-1] - ins1;
                    ndely[yy] = yy;
          }
          col0[0] = 0;        /* Waterman Bull Math Biol 84 */
      }
      else
          for (yy = 1; yy <= len1; yy++)
                    dely[yy] = -ins0;

      /* fill in match matrix
       */
      for (px = seqx[0], xx = 1; xx <= len0; px++, xx++) {
          /* initialize first entry in col
           */
          if (endgaps) {
                    if (xx == 1)
                              col1[0] = delx = -(ins0+ins1);
                    else
                              col1[0] = delx = col0[0] - ins1;
                    ndelx = xx;
          }
          else {
                    col1[0] = 0;
                    delx = -ins0;
                    ndelx = 0;
          }
```

...nw

```
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];

        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
                } else
                        ndely[yy]++;
        }

        /* update penalty for del in y seq;
         * favor new del over ongong del
         */
        if (endgaps || ndelx < MAXGAP) {
                if (col1[yy-1] - ins0 >= delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) >= delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

...nw

```
                    id = xx - yy + len1 - 1;
                    if (mis >= delx && mis >= dely[yy])
                            col1[yy] = mis;
                    else if (delx >= dely[yy]) {
                            col1[yy] = delx;
                            ij = dx[id].ijmp;
                            if (dx[id].jp.n[0] && (!dna || (ndelx >= MAXJMP
                            && xx > dx[id].jp.x[ij]+MX) || mis > dx[id].score+DINS0)) {
                                    dx[id].ijmp++;
                                    if (++ij >= MAXJMP) {
                                            writejmps(id);
                                            ij = dx[id].ijmp = 0;
                                            dx[id].offset = offset;
                                            offset += sizeof(struct jmp) + sizeof(offset);
                                    }
                            }
                            dx[id].jp.n[ij] = ndelx;
                            dx[id].jp.x[ij] = xx;
                            dx[id].score = delx;
                    }
                    else {
                            col1[yy] = dely[yy];
                            ij = dx[id].ijmp;

            if (dx[id].jp.n[0] && (!dna || (ndely[yy] >= MAXJMP
                            && xx > dx[id].jp.x[ij]+MX) || mis > dx[id].score+DINS0)) {
                                    dx[id].ijmp++;
                                    if (++ij >= MAXJMP) {
                                            writejmps(id);
                                            ij = dx[id].ijmp = 0;
                                            dx[id].offset = offset;
                                            offset += sizeof(struct jmp) + sizeof(offset);
                                    }
                            }
                            dx[id].jp.n[ij] = -ndely[yy];
                            dx[id].jp.x[ij] = xx;
                            dx[id].score = dely[yy];
                    }
                    if (xx == len0 && yy < len1) {
                            /* last col
                             */
                            if (endgaps)
                                    col1[yy] -= ins0+ins1*(len1-yy);
                            if (col1[yy] > smax) {
                                    smax = col1[yy];
                                    dmax = id;
                            }
                    }
            }
            if (endgaps && xx < len0)
                    col1[yy-1] -= ins0+ins1*(len0-xx);
            if (col1[yy-1] > smax) {
                    smax = col1[yy-1];
                    dmax = id;
            }
            tmp = col0; col0 = col1; col1 = tmp;
    }
    (void) free((char *)ndely);
    (void) free((char *)dely);
    (void) free((char *)col0);
    (void) free((char *)col1);
}
```

```
/*
 *
 * print() -- only routine visible outside this module
 *
 * static:
 * getmat() -- trace back best path, count matches: print()
 * pr_align() -- print alignment of described in array p[]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */

#include "nw.h"

#define SPC       3
#define P_LINE    256        /* maximum output line */
#define P_SPC     3          /* space between name or num and seq */

extern    _day[26][26];
int   olen;                  /* set output line length */
FILE      *fx;               /* output file */

print()
{
     int lx, ly, firstgap, lastgap;        /* overlap */

     if ((fx = fopen(ofile, "w")) == 0) {
         fprintf(stderr,"%s: can't write %s\n", prog, ofile);
         cleanup(1);
     }
     fprintf(fx, "<first sequence: %s (length = %d)\n", namex[0], len0);
     fprintf(fx, "<second sequence: %s (length = %d)\n", namex[1], len1);
     olen = 60;
     lx = len0;
     ly = len1;
     firstgap = lastgap = 0;
     if (dmax < len1 - 1) {              /* leading gap in x */
         pp[0].spc = firstgap = len1 - dmax - 1;
         ly -= pp[0].spc;
     }
     else if (dmax > len1 - 1) {         /* leading gap in y */
         pp[1].spc = firstgap = dmax - (len1 - 1);
         lx -= pp[1].spc;
     }
     if (dmax0 < len0 - 1) {             /* trailing gap in x */
         lastgap = len0 - dmax0 -1;
         lx -= lastgap;
     }
     else if (dmax0 > len0 - 1) {        /* trailing gap in y */
         lastgap = dmax0 - (len0 - 1);
         ly -= lastgap;
     }
     getmat(lx, ly, firstgap, lastgap);
     pr_align();
}
```

print

getmat

```
/*
 * trace back the best path, count matches
 */
static
getmat(lx, ly, firstgap, lastgap)
	int lx, ly;			/* "core" (minus endgaps) */
	int firstgap, lastgap;		/* leading trailing overlap */
{
	int		nm, i0, i1, siz0, siz1;
	char		outx[32];
	double		pct;
	register	n0, n1;
	register char	*p0, *p1;

	/* get total matches, score
	 */
	i0 = i1 = siz0 = siz1 = 0;
	p0 = seqx[0] + pp[1].spc;
	p1 = seqx[1] + pp[0].spc;
	n0 = pp[1].spc + 1;
	n1 = pp[0].spc + 1;

	nm = 0;
	while ( *p0 && *p1 ) {
		if (siz0) {
			p1++;
			n1++;
			siz0--;
		}
		else if (siz1) {
			p0++;
			n0++;
			siz1--;
		}
		else {
			if (xbm[*p0-'A']&xbm[*p1-'A'])
				nm++;
			if (n0++ == pp[0].x[i0])
				siz0 = pp[0].n[i0++];
			if (n1++ == pp[1].x[i1])
				siz1 = pp[1].n[i1++];
			p0++;
			p1++;
		}
	}

	/* pct homology:
	 * if penalizing endgaps, base is the shorter seq
	 * else, knock off overhangs and take shorter core
	 */
	if (endgaps)
		lx = (len0 < len1)? len0 : len1;
	else
		lx = (lx < ly)? lx : ly;
	pct = 100.*(double)nm/(double)lx;
	fprintf(fx, "\n");
	fprintf(fx, "<%d match%s in an overlap of %d: %.2f percent similarity\n",
		nm, (nm == 1)? "" : "es", lx, pct);
```

...getmat

```
	fprintf(fx, "<gaps in first sequence: %d", gapx);
	if (gapx) {
		(void) sprintf(outx, " (%d %s%s)",
			ngapx, (dna)? "base":"residue", (ngapx == 1)? "":"s");
		fprintf(fx,"%s", outx);

	fprintf(fx, ", gaps in second sequence: %d", gapy);
	if (gapy) {
		(void) sprintf(outx, " (%d %s%s)",
			ngapy, (dna)? "base":"residue", (ngapy == 1)? "":"s");
		fprintf(fx,"%s", outx);
	}
	if (dna)
		fprintf(fx,
		"\n<score: %d (match = %d, mismatch = %d, gap penalty = %d + %d per base)\n",
		smax, DMAT, DMIS, DINS0, DINS1);
	else
		fprintf(fx,
		"\n<score: %d (Dayhoff PAM 250 matrix, gap penalty = %d + %d per residue)\n",
		smax, PINS0, PINS1);
	if (endgaps)
		fprintf(fx,
		"<endgaps penalized. left endgap: %d %s%s, right endgap: %d %s%s\n",
		firstgap, (dna)? "base" : "residue", (firstgap == 1)? "" : "s",
		lastgap, (dna)? "base" : "residue", (lastgap == 1)? "" : "s");
	else
		fprintf(fx, "<endgaps not penalized\n");
}

static		nm;		/* matches in core -- for checking */
static		lmax;		/* lengths of stripped file names */
static		ij[2];		/* jmp index for a path */
static		nc[2];		/* number at start of current line */
static		ni[2];		/* current elem number -- for gapping */
static		siz[2];
static char	*ps[2];		/* ptr to current element */
static char	*po[2];		/* ptr to next output char slot */
static char	out[2][P_LINE];	/* output line */
static char	star[P_LINE];	/* set by stars() */

/*
 * print alignment of described in struct path pp[]
 */
static
pr_align()
{
	int		nn;	/* char count */
	int		more;
	register		i;

	for (i = 0, lmax = 0; i < 2; i++) {
		nn = stripname(namex[i]);
		if (nn > lmax)
			lmax = nn;

		nc[i] = 1;
		ni[i] = 1;
		siz[i] = ij[i] = 0;
		ps[i] = seqx[i];
		po[i] = out[i];
	}
```

pr_align

...pr_align

```
    for (nn = nm = 0, more = 1; more; ) {
        for (i = more = 0; i < 2; i++) {
                /*
                 * do we have more of this sequence?
                 */
                if (!*ps[i])
                        continue;

                more++;

                if (pp[i].spc) {        /* leading space */
                        *po[i]++ = ' ';
                        pp[i].spc--;
                }
                else if (siz[i]) {      /* in a gap */
                        *po[i]++ = '-';
                        siz[i]--;
                }
                else {                  /* we're putting a seq element
                                         */
                        *po[i] = *ps[i];
                        if (islower(*ps[i]))
                                *ps[i] = toupper(*ps[i]);
                        po[i]++;
                        ps[i]++;

                        /*
                         * are we at next gap for this seq?
                         */
                        if (ni[i] == pp[i].x[ij[i]]) {
                                /*
                                 * we need to merge all gaps
                                 * at this location
                                 */
                                siz[i] = pp[i].n[ij[i]++];
                                while (ni[i] == pp[i].x[ij[i]])
                                        siz[i] += pp[i].n[ij[i]++];
                        }
                        ni[i]++;
                }
        }
        if (++nn == olen || !more && nn) {
                dumpblock();
                for (i = 0; i < 2; i++)
                        po[i] = out[i];
                nn = 0;
        }
    }
}

/*
 * dump a block of lines, including numbers, stars: pr_align()
 */
static
dumpblock()
{
    register    i;

    for (i = 0; i < 2; i++)
        *po[i]-- = '\0';
```

dumpblock

...dumpblock

```
        (void) putc('\n', fx);
        for (i = 0; i < 2; i++) {
            if (*out[i] && (*out[i] != ' ' || *(po[i]) != ' ')) {
                    if (i == 0)
                            nums(i);
                    if (i == 0 && *out[1])
                            stars();
                    putline(i);
                    if (i == 0 && *out[1])
                            fprintf(fx, star);
                    if (i == 1)
                            nums(i);
            }
        }
}

/*
 * put out a number line: dumpblock()
 */
static
nums(ix)
        int ix;         /* index in out[] holding seq line */
{
        char                    nline[P_LINE];
        register                i, j;
        register char           *pn, *px, *py;

        for (pn = nline, i = 0; i < lmax+P_SPC; i++, pn++)
            *pn = ' ';
        for (i = nc[ix], py = out[ix]; *py; py++, pn++) {
            if (*py == ' ' || *py == '-')
                    *pn = ' ';
            else {
                    if (i%10 == 0 || (i == 1 && nc[ix] != 1)) {
                            j = (i < 0)? -i : i;
                            for (px = pn; j; j /= 10, px--)
                                    *px = j%10 + '0';
                            if (i < 0)
                                    *px = '-';
                    }
                    else
                            *pn = ' ';
                    i++;
            }
        }
        *pn = '\0';
        nc[ix] = i;
        for (pn = nline; *pn; pn++)
            (void) putc(*pn, fx);
        (void) putc('\n', fx);
}

/*
 * put out a line (name, [num], seq, [num]): dumpblock()
 */
static
putline(ix)
        int ix;
{
```

putline

nums

...putline

```
	int		i;
	register char		*px;

	for (px = namex[ix], i = 0; *px && *px != ':'; px++, i++)
		(void) putc(*px, fx);
	for (; i < lmax+P_SPC; i++)
		(void) putc(' ', fx);

	/* these count from 1:
	 * ni[] is current element (from 1)
	 * nc[] is number at start of current line
	 */
	for (px = out[ix]; *px; px++)
		(void) putc(*px&0x7F, fx);
	(void) putc('\n', fx);
}


/*
 * put a line of stars (seqs always in out[0], out[1]): dumpblock()
 */
static
stars()
{
	int		i;
	register char		*p0, *p1, cx, *px;

	if (!*out[0] || (*out[0] == ' ' && *(po[0]) == ' ') ||
	   !*out[1] || (*out[1] == ' ' && *(po[1]) == ' '))
		return;
	px = star;
	for (i = lmax+P_SPC; i; i--)
		*px++ = ' ';

	for (p0 = out[0], p1 = out[1]; *p0 && *p1; p0++, p1++) {
		if (isalpha(*p0) && isalpha(*p1)) {

			if (xbm[*p0-'A']&xbm[*p1-'A']) {
				cx = '*';
				nm++;
			}
			else if (!dna && _day[*p0-'A'][*p1-'A'] > 0)
				cx = '.';
			else
				cx = ' ';
		}
		else
			cx = ' ';
		*px++ = cx;
	}
	*px++ = '\n';
	*px = '\0';
}
```

stars

```
/*
 * strip path or prefix from pn, return len: pr_align()
 */
static
stripname(pn)
      char         *pn;     /* file name (may be path) */
{
      register char        *px, *py;

      py = 0;
      for (px = pn; *px; px++)
          if (*px == '/')
                  py = px + 1;
      if (py)
          (void) strcpy(pn, py);
      return(strlen(pn));

}
```

stripname

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>

char *jname = "/tmp/homgXXXXXX";                /* tmp file for jmps */
FILE    *fj;

int     cleanup();                      /* cleanup tmp file */
long    lseek();

/*
 * remove any tmp file if we blow
 */
cleanup(i)                                                              cleanup
        int i;
{
        if (fj)
            (void) unlink(jname);
        exit(i);
}

/*
 * read, return ptr to seq, set dna, len, maxlen
 * skip lines starting with ';', '<', or '>'
 * seq in upper or lower case
 */
char *
getseq(file, len)                                                       getseq
        char            *file;      /* file name */
        int *len;       /* seq len */
{
        char                    line[1024], *pseq;
        register char           *px, *py;
        int             natgc, tlen;
        FILE                    *fp;

        if ((fp = fopen(file,"r")) == 0) {
            fprintf(stderr,"%s: can't read %s\n", prog, file);
            exit(1);
        }
        tlen = natgc = 0;
        while (fgets(line, 1024, fp)) {
            if (*line == ';' || *line == '<' || *line == '>')
                        continue;
            for (px = line; *px != '\n'; px++)
                        if (isupper(*px) || islower(*px))
                                    tlen++;
        }
        if ((pseq = malloc((unsigned)(tlen+6))) == 0) {
            fprintf(stderr,"%s: malloc() failed to get %d bytes for %s\n", prog, tlen+6, file);
            exit(1);
        }
        pseq[0] = pseq[1] = pseq[2] = pseq[3] = '\0';
```

...getseq

```
        py = pseq + 4;
        *len = tlen;
        rewind(fp);

        while (fgets(line, 1024, fp)) {
            if (*line == ';' || *line == '<' || *line == '>')
                    continue;
            for (px = line; *px != '\n'; px++) {
                    if (isupper(*px))
                            *py++ = *px;
                    else if (islower(*px))
                            *py++ = toupper(*px);
                    if (index("ATGCU",*(py-1)))
                            natgc++;
            }
        }
        *py++ = '\0';
        *py = '\0';
        (void) fclose(fp);
        dna = natgc > (tlen/3);
        return(pseq+4);
}
```

g_calloc

```
char *
g_calloc(msg, nx, sz)
        char        *msg;           /* program, calling routine */
        int nx, sz;                 /* number and size of elements */
{
        char                *px, *calloc();

        if ((px = calloc((unsigned)nx, (unsigned)sz)) == 0) {
            if (*msg) {
                    fprintf(stderr, "%s: g_calloc() failed %s (n=%d, sz=%d)\n", prog, msg, nx, sz);
                    exit(1);
            }
        }
        return(px);
}
```

readjmps

```
/*
 * get final jmps from dx[] or tmp file, set pp[], reset dmax: main()
 */
readjmps()
{
        int         fd = -1;
        int         siz, i0, i1;
        register    i, j, xx;

        if (fj) {
            (void) fclose(fj);
            if ((fd = open(jname, O_RDONLY, 0)) < 0) {
                    fprintf(stderr, "%s: can't open() %s\n", prog, jname);
                    cleanup(1);
            }
        }
        for (i = i0 = i1 = 0, dmax0 = dmax, xx = len0; ; i++) {
            while (1) {
                    for (j = dx[dmax].ijmp; j >= 0 && dx[dmax].jp.x[j] >= xx; j--)
                            ;
```

...readjmps

```
			if (j < 0 && dx[dmax].offset && fj) {
				(void) lseek(fd, dx[dmax].offset, 0);
				(void) read(fd, (char *)&dx[dmax].jp, sizeof(struct jmp));
				(void) read(fd, (char *)&dx[dmax].offset, sizeof(dx[dmax].offset));
				dx[dmax].ijmp = MAXJMP-1;
			}
			else
				break;
		}
		if (i > = JMPS) {
			fprintf(stderr, "%s: too many gaps in alignment\n", prog);
			cleanup(1);
		}
		if (j > = 0) {
			siz = dx[dmax].jp.n[j];
			xx = dx[dmax].jp.x[j];
			dmax + = siz;
			if (siz < 0) {			/* gap in second seq */
				pp[1].n[i1] = -siz;
				xx + = siz;

				/* id = xx - yy + len1 - 1
				 */
				pp[1].x[i1] = xx - dmax + len1 - 1;
				gapy + +;
				ngapy - = siz;
/* ignore MAXGAP when doing endgaps */
				siz = (-siz < MAXGAP || endgaps)? -siz : MAXGAP;
				i1 + +;
			}
			else if (siz > 0) {  /* gap in first seq */
				pp[0].n[i0] = siz;
				pp[0].x[i0] = xx;
				gapx + +;
				ngapx + = siz;
/* ignore MAXGAP when doing endgaps */
				siz = (siz < MAXGAP || endgaps)? siz : MAXGAP;
				i0 + +;
			}
		}
		else
			break;
	}

	/* reverse the order of jmps
	 */
	for (j = 0, i0--; j < i0; j + +, i0--) {
		i = pp[0].n[j]; pp[0].n[j] = pp[0].n[i0]; pp[0].n[i0] = i;
		i = pp[0].x[j]; pp[0].x[j] = pp[0].x[i0]; pp[0].x[i0] = i;
	}
	for (j = 0, i1--; j < i1; j + +, i1--) {
		i = pp[1].n[j]; pp[1].n[j] = pp[1].n[i1]; pp[1].n[i1] = i;
		i = pp[1].x[j]; pp[1].x[j] = pp[1].x[i1]; pp[1].x[i1] = i;
	}
	if (fd > = 0)
		(void) close(fd);
	if (fj) {
		(void) unlink(jname);
		fj = 0; offset = 0;}}
```

```
/*
 * write a filled jmp struct offset of the prev one (if any): nw()
 */
writejmps(ix)                                                    writejmps
    int ix;
{
    char        *mktemp();

    if (!fj) {
        if (mktemp(jname) < 0) {
                fprintf(stderr, "%s: can't mktemp() %s\n", prog, jname);
                cleanup(1);
        }
        if ((fj = fopen(jname, "w")) == 0) {
                fprintf(stderr, "%s: can't write %s\n", prog, jname);
                exit(1);
        }
    }
    (void) fwrite((char *)&dx[ix].jp, sizeof(struct jmp), 1, fj);
    (void) fwrite((char *)&dx[ix].offset, sizeof(dx[ix].offset), 1, fj);
}
```

Table 2A

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity = (the number of identically matching amino acid residues between the two polypeptide sequences as determined by ALIGN-2) divided by (the total number of amino acid residues of the PRO polypeptide) = 5 divided by 15 = 33.3%

Table 2B

| | | |
|---|---|---|
| PRO | XXXXXXXXXX | (Length = 10 amino acids) |
| Comparison Protein | XXXXX*YYYYYYZZYZ* | (Length = 15 amino acids) |

% amino acid sequence identity = (the number of identically matching amino acid residues between the two polypeptide sequences as determined by ALIGN-2) divided by (the total number of amino acid residues of the PRO polypeptide) = 5 divided by 10 = 50%

Table 2C

| | | |
|---|---|---|
| PRO-DNA | NNNNNNNNNNNNNN | (Length = 14 nucleotides) |

(continued)

| | | |
|---|---|---|
| Comparison DNA | NNNNNNLLLLLLLLLL | (Length = 16 nucleotides) |

% nucleic acid sequence identity =
(the number of identically matching nucleotides between the two nucleic acid sequences as determined by ALIGN-2) divided by (the total number of nucleotides of the PRO-DNA nucleic acid sequence) =
6 divided by 14 = 42.9 %

Table 2D

| | | |
|---|---|---|
| PRO-DNA | NNNNNNNNNNNN | (Length = 12 nucleotides) |
| Comparison DNA | NNNNLLLVV | (Length = 9 nucleotides) |

% nucleic acid sequence identity =
(the number of identically matching nucleotides between the two nucleic acid sequences as determined by ALIGN-2) divided by (the total number of nucleotides of the PRO-DNA nucleic acid sequence) =
4 divided by 12 = 33.3%

"Percent (%) amino acid sequence identity" with respect to the PRO polypeptide sequences identified herein is defined as the percentage of amino acid residues in a candidate sequence that are identical with the amino acid residues in a PRO sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are obtained as described below by using the sequence comparison computer program ALIGN-2, wherein the complete source code for the ALIGN-2 program is provided in Table 1. The ALIGN-2 sequence comparison computer program was authored by Genentech, Inc., and the source code shown in Table I has been filed with user documentation in the U.S. Copyright Office. Washington D.C., 20559, where it is registered under U.S. Copyright Registration No. TXU510087. The ALIGN-2 program is publicly available through Genentech, Inc., South San Francisco, California or may be compiled from the source code provided in Table 1. The ALIGN-2 program should be compiled for use on a UNIX operating system, preferably digital UNIX V4.0D. All sequence comparison parameters are set by the ALIGN-2 program and do not vary.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given am ino acid sequence B) is calculated as follows:

$$100 \text{ times the fraction } X/Y$$

where X is the number of amino acid residues scored as identical matches by the sequence alignment program ALIGN-2 in that program s alignment of A and B, and where Y is the total number of amino acid residues in B. It will be appreciated that where the length of amino acid sequence A is not equal to the length of amino acid sequence B. the % amino acid sequence identity of A to B will not equal the % amino acid sequence identity of B to A. As examples of % amino acid sequence identity calculations, Tables 2A-2B demonstrate how to calculate the % amino acid sequence identity of the amino acid sequence designated "Comparison Protein" to the amino acid sequence designated "PRO".

Unless specifically stated otherwise, all % amino acid sequence identity values used herein are obtained as described above using the ALIGN-2 sequence comparison computer program. However. % amino acid sequence identity may also be determined using the sequence comparison program NCB1-BLAST2 (Altschul et al., Nucleic Acids Res., 25:3389-3402 (1997)). The NCBI-BLAST2 sequence comparison program may be downloaded from http://www.ncbi.nlm.nih.gov. NCB1-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program NCBI-BLAST2 in that program's alignment of A and B, and where Y is the total number of amino acid residues in B. It will be appreciated that where the length ofamino acid sequence A is not equal to the length of amino acid sequence B, the % amino acid sequence identity of A to B will not equal the % amino acid sequence identity of B to A.

In addition, % amino acid sequence identity may also be determined using the WU-BLAST-2 computer program (Altschul et al., Methods in Enzymology, 266:460-480 (1996)). Most of the WU-BLAST-2 search parameters are set to the default values. Those not set to default values, *i.e.,* the adjustable parameters, are set with the following values: overlap span = 1, overlap fraction = 0.125, word threshold (T) = 11, and scoring matrix = BLOSUM62. For purposes herein, a % amino acid sequence identity value is determined by dividing (a) the number of matching identical amino acids residues between the amino acid sequence of the PRO polypeptide of interest having a sequence derived from the native PRO polypeptide and the comparison amino acid sequence of interest (*i.e.*, the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

"PRO variant polynucleotide" or "PRO variant nucleic acid sequence" means a nucleic acid molecule which encodes an active PRO polypeptide as defined below and which has at least about 80% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, often at least about 60 nucleotides in length, more often at least about 90 nucleotides in length, more often at least about 120 nucleotides in length, more often at least about 150 nucleotides in length, more often at least about 180 nucleotides in length, more often at least about 210 nucleotides in length, more often at least about 240 nucleotides in length, more often at least about 270 nucleotides in length, more often at least about 300 nucleotides in length, more often at least about 450 nucleotides in length, more often at least about 600 nucleotides in length, more often at least about 900 nucleotides in length, or more.

"Percent (%) nucleic acid sequence identity" with respect to the PRO polypeptide-encoding nucleic acid sequences identified herein is defined as the percentage of nucleotides in a candidate sequence that are identical with the nucleotides in a PRO polypeptide-encoding nucleic acid sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % nucleic acid sequence identity values are obtained as described below by using the sequence comparison computer program ALIGN-2, wherein the complete source code for the ALIGN-2 program is provided in Table 1. The ALIGN-2 sequence comparison computer program was authored by Genentech. Inc., and the source code shown in Table I has been filed with user documentation in the U.S. Copyright Office, Washington D.C., 20559. where it is registered under U.S. Copyright Registration No. TXU510087. The ALIGN-2 program is publicly available through Genentech, Inc., South San Francisco, California or may be compiled from the source code provided in Table 1. The ALIGN-2 program should be compiled for use on a UNIX operating system, preferably digital UNIX V4.0D. All sequence comparison parameters are set by the ALIGN-2 program and do not vary.

For purposes herein, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

100 times the fraction W/Z

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of% nucleic acid sequence identity calculations, Tables 2C-2D demonstrate how to calculate the % nucleicacid sequence identity of the nucleic acid sequence designated "Comparison DNA" to the nucleic acid sequence designated "PRO-DNA".

Unless specifically stated otherwise, all % nucleic acid sequence identity values used herein are obtained as described above using the ALIGN-2 sequence comparison computer program. However, %nucleic acid sequence identity may also be determined using the sequence comparison program NCBI-BLAST2 (Altschul et al., Nucleic Acids Res., 25:3389-3402 (1997)). The NCBI-BLAST2 sequence comparison program may be downloaded from http://www.ncbi.nlm.nih.gov. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix - BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

100 times the fraction W/Z

where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In addition, % nucleic acid sequence identity values may also be generated using the WU-BLAST-2 computer program (Altschul et al., Methods in Enzymology, 266:460-480 (1996)). Most of the WU-BLAST-2 search parameters are set to the default values. Those not set to default values, *i.e.,* the adjustable parameters, are set with the following values: overlap span = 1, overlap fraction = 0.125, word threshold (T) = 11, and scoring matrix = BLOSUM62. For purposes herein, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (*i.e.,* the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

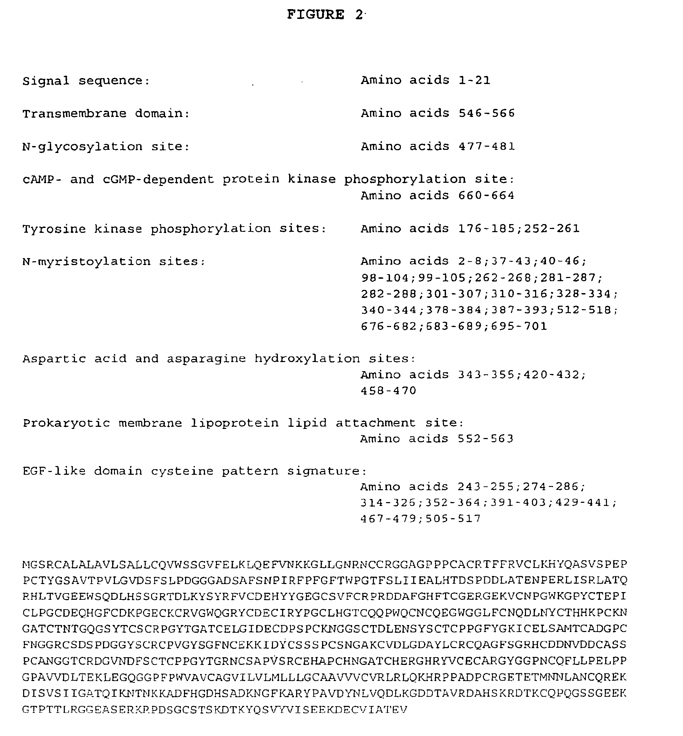

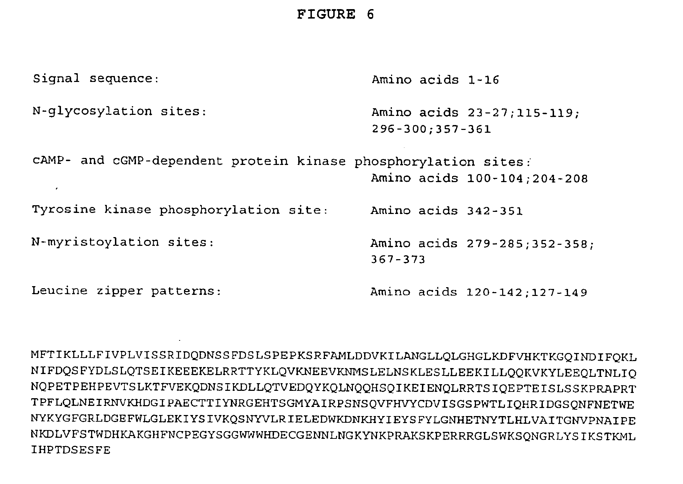

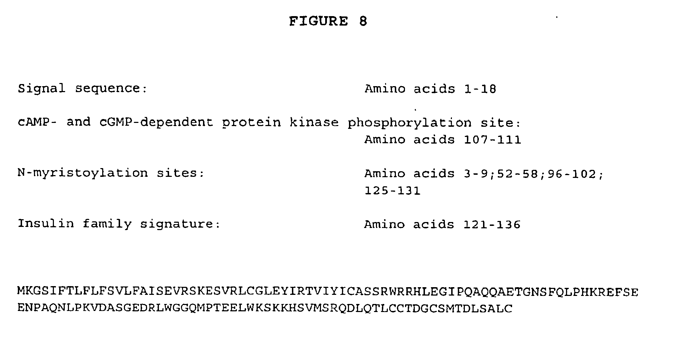

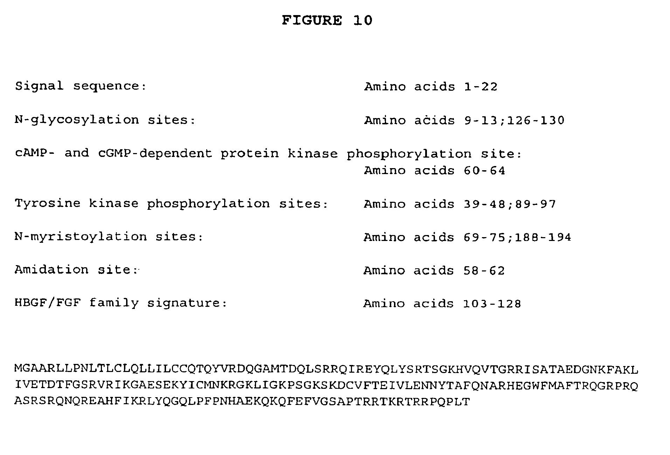

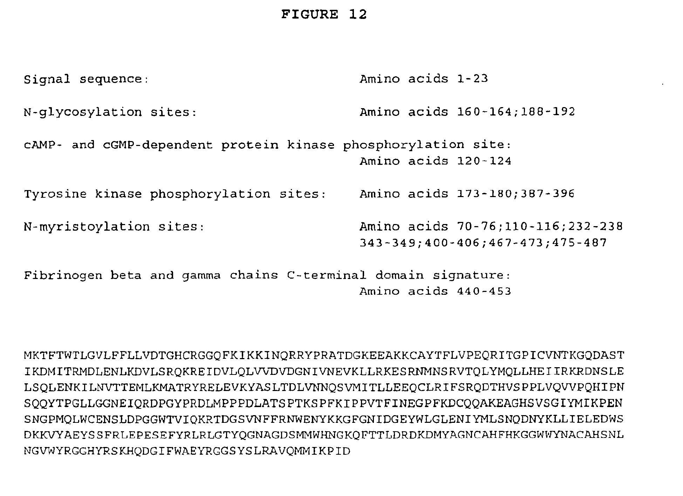

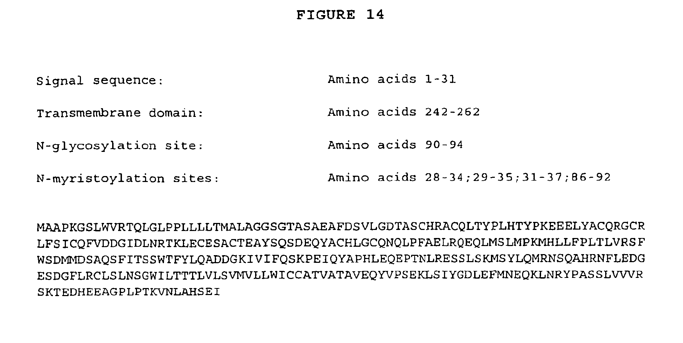

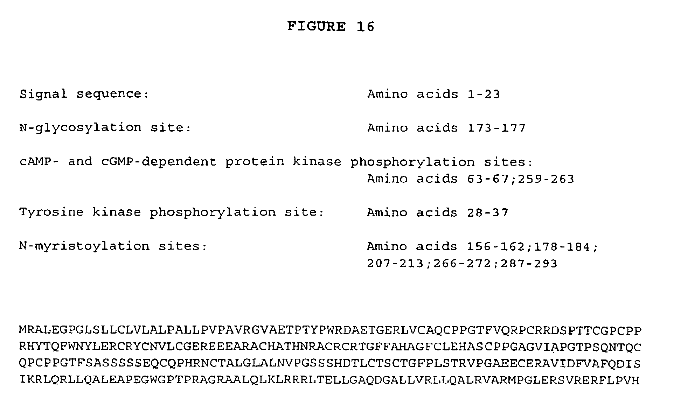

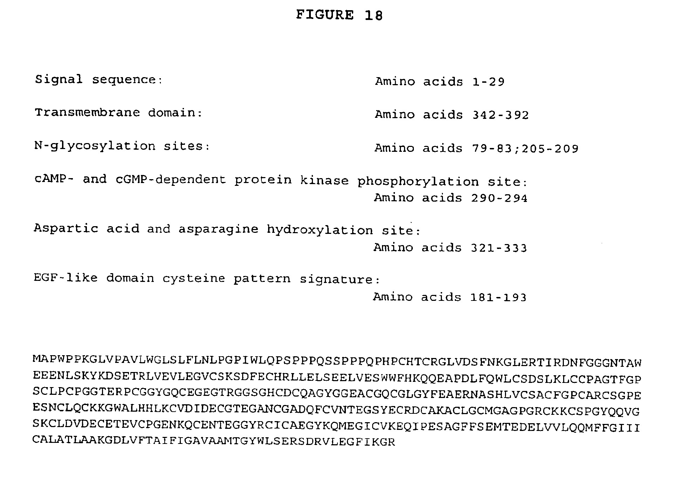

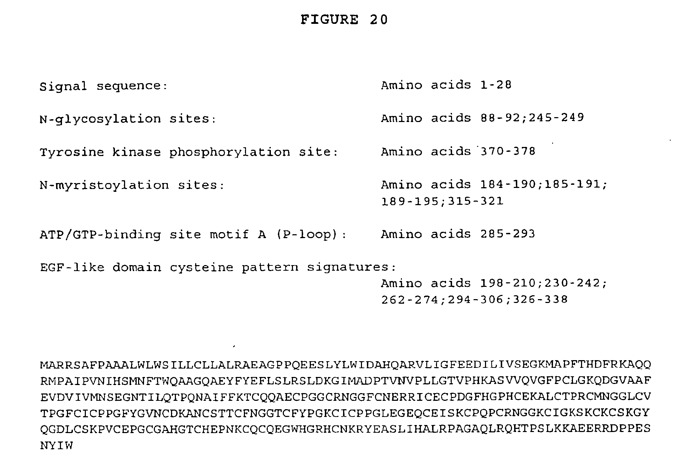

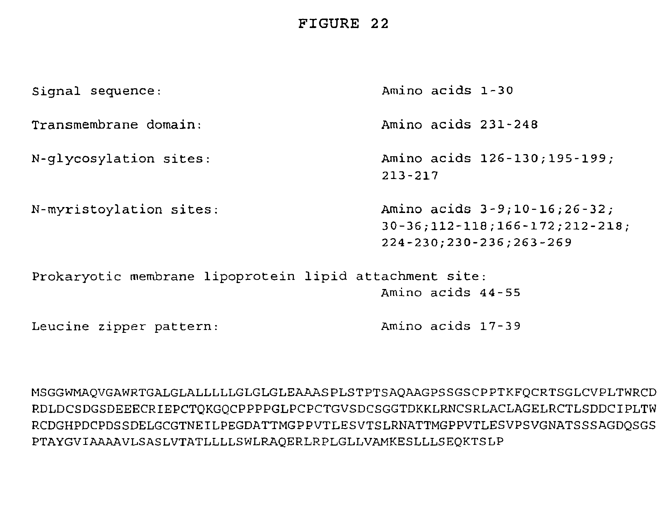

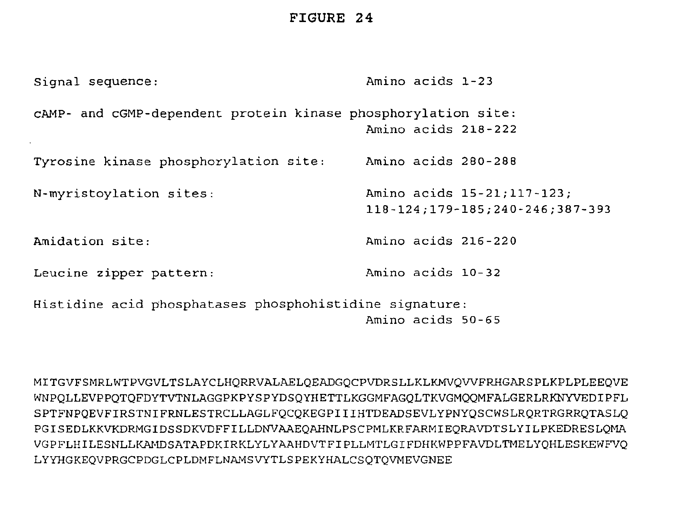

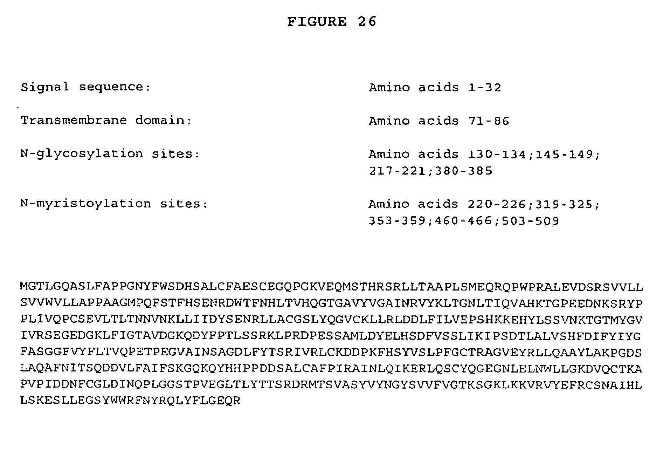

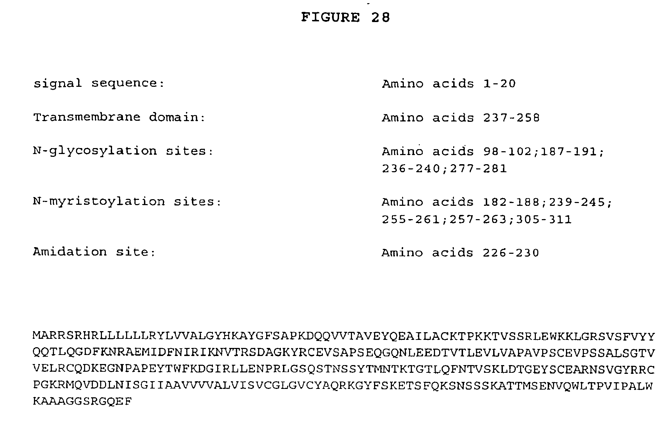

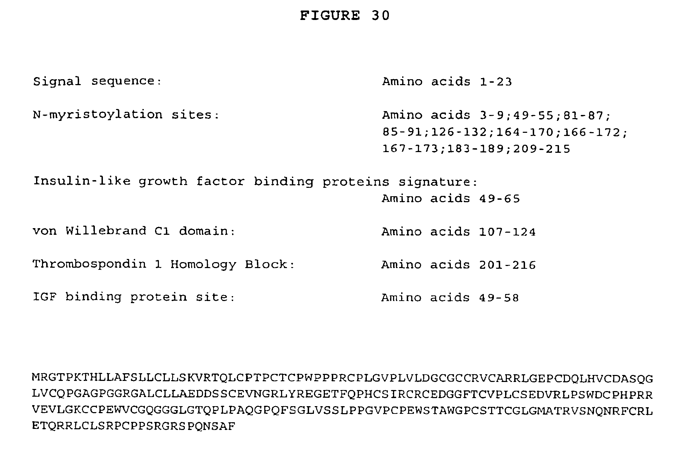

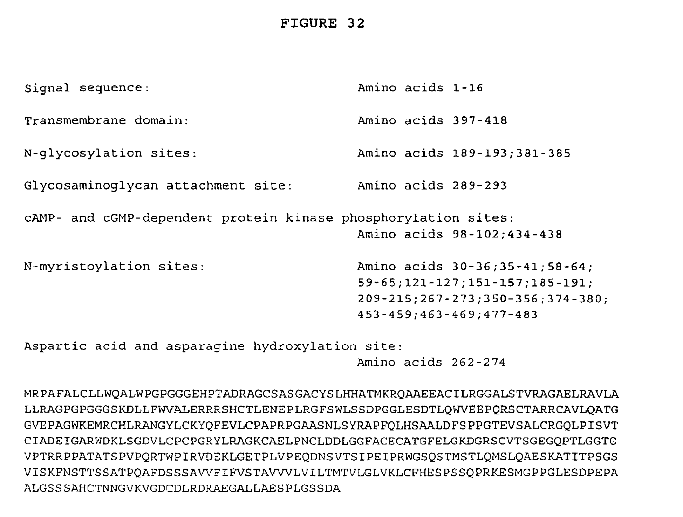

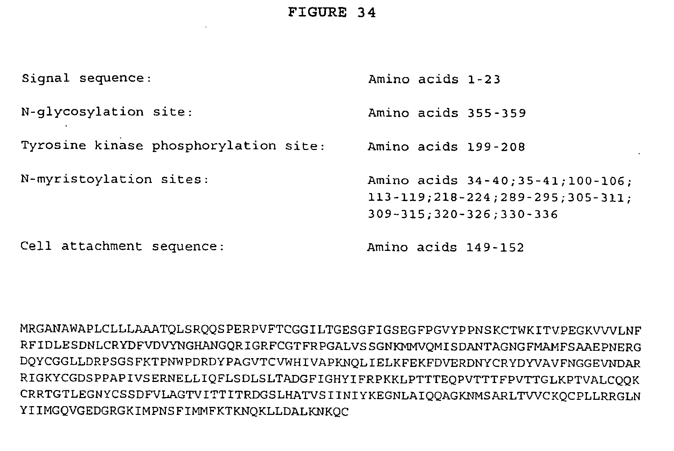

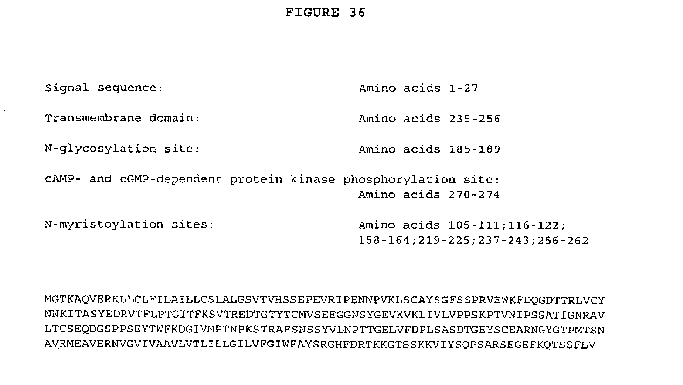

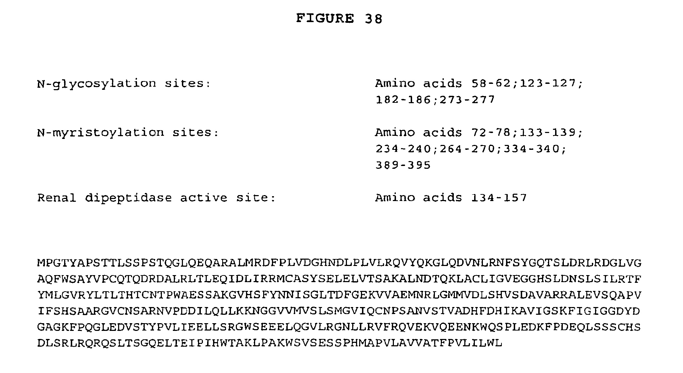

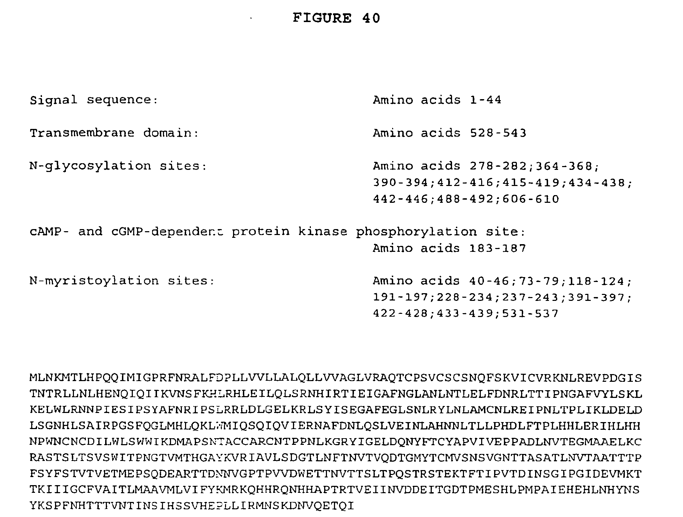

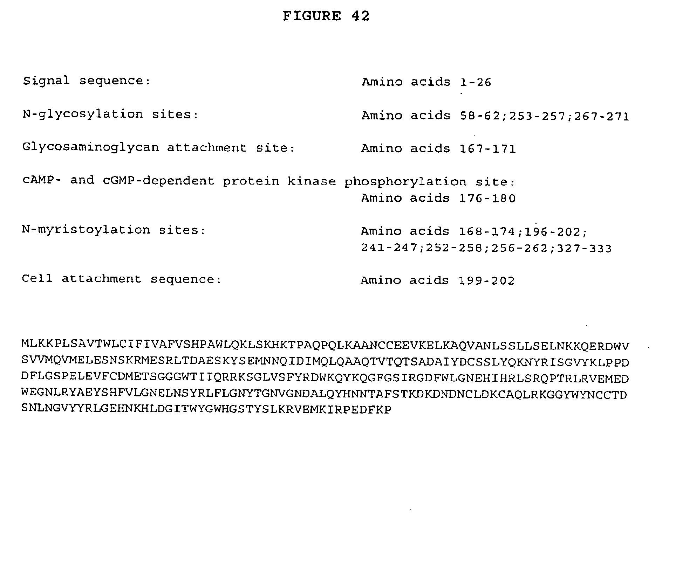

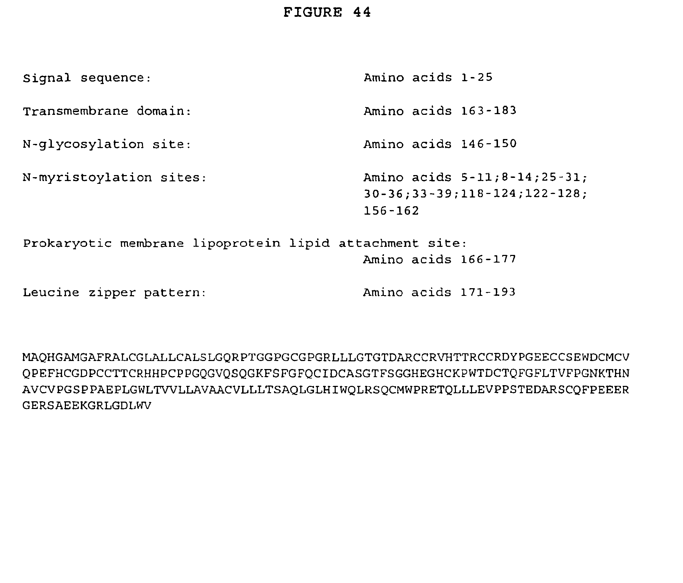

In other embodiments, PRO variant polynucleotides are nucleic acid molecules that encode an active PRO polypeptide and which are capable of hybridizing, preferably under stringent hybridization and wash conditions, to nucleotide sequences encoding the full-length PRO polypeptide shown in Figure 2 (SEQ ID NO:4), Figure 4 (SEQ ID NO:9), Figure 6 (SEQ ID NO:14), Figure 8 (SEQ ID NO:16), Figure 10 (SEQ ID NO:21), Figure 12 (SEQ ID NO:26), Figure 14 (SEQ ID NO:31), Figure 16 (SEQ ID NO:36), Figure 18 (SEQ ID NO:41), Figure 20 (SEQ ID NO:46), Figure 22 (SEQ ID NO:51), Figure 24 (SEQ ID NO:56), Figure 26 (SEQ ID NO:62), Figure 28 (SEQ ID NO:67), Figure 30 (SEQ ID NO:72), Figure 32 (SEQ ID NO:77), Figure 34 (SEQ ID NO:85), Figure 36 (SEQ ID NO:90), Figure 38 (SEQ ID NO: 98), Figure 40 (SEQ ID NO:107), Figure 42 (SEQ ID NO:112), Figure 44 (SEQ ID NO:117), Figure 46 (SEQ ID NO:127), Figure 48 (SEQ ID NO:132), Figure 50 (SEQ ID NO:137), Figure 52 (SEQ ID NO:143), Figure 54 (SEQ ID NO:148), Figure 56 (SEQ ID NO:153), Figure 58 (SEQ ID NO:155), Figure 60 (SEQ ID NO:160), Figure 62 (SEQ ID NO:162), Figure 64 (SEQ ID NO:170), Figure 66 (SEQ ID NO:181), Figure 68 (SEQ ID NO:183), Figure 70 (SEQ ID NO:191), Figure 72 (SEQ ID NO:193), Figure 74 (SEQ ID NO: 195), Figure 76 (SEQ ID NO:197), Figure 78 (SEQ ID NO:199). Figure 80 (SEQ ID NO:201), Figure 82 (SEQ ID NO:203), Figure 84 (SEQ ID NO:205), Figure 86 (SEQ ID NO:214), Figure 88 (SEQ ID NO:216), Figure 90 (SEQ ID NO:218), Figure 92 (SEQ ID NO:220), Figure 94 (SEQ ID NO:222),and Figure 96 (SEQ ID NO:227), respectively. PRO variant polypeptides may be those that are encoded by a PRO variant polynucleotide.

The term "positives", in the context of the am ino acid sequence identity comparisons performed as described above, includes amino acid residues in the sequences compared that are not only identical, but also those that have similar properties. Amino acid residues that score a positive value to an amino acid residue of interest are those that are either identical to the amino acid residue of interest or are a preferred substitution (as defined in Table 3 below) of the amino acid residue of interest.

Fur purposes herein, the % value of positives of a given amino acid sequence A to, with, or against a given amino acid sequence B (which can alternatively be phrased as a given amino acid sequence A that has or comprises a certain % positives to, with, or against a given amino acid sequence B) is calculated as follows:

$$100 \text{ times the fraction } X/Y$$

where X is the number of amino acid residues scoring a positive value by the sequence alignment program ALIGN-2 in that program's alignment of A and B, and where Y is the total number of amino acid residues in B. It will be appreciated that where the length of amino acid sequence A is not equal to the length of amino acid sequence B, the % positives of A to B will not equal the % positives of B to A.

"Isolated'', when used to describe the various polypeptides disclosed herein, means a polypeptide that has been identified and separated and/or recovered from a component of its natural environment. Preferably, the isolated polypeptide is free of association with all components with which it is naturally associated. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO natural environment will not be present. Ordinarily, however, isolated polypeptide will be prepared by at least one purification step.

An "isolated" nucleic acid molecule encoding a PRO polypeptide or an "isolated" nucleic acid molecule encoding an anti-PRO antibody is a nucleic acid molecule that is identified and separated from at least one contaminant nucleic acid molecule with which it is ordinarily associated in the natural source ofthe PRO-encoding nucleic acid or the natural source of the anti-PRO-encoding nucleic acid. Preferably, the isolated nucleic acid is free of association with all components with which it is naturally associated. An isolated PRO-encoding nucleic acid molecule or an isolated anti-PRO-encoding nucleic acid molecule is other than in the form or setting in which it is found in nature. Isolated nucleic acid molecules therefore are distinguished from the PRO-encoding nucleic acid molecule or from the anti-PRO-encoding nucleic acid molecule as it exists in natural cells. However, an isolated nucleic acid molecule encoding a PRO polypeptide or an isolated nucleic acid molecule encoding an anti-PRO antibody includes PRO-nucleic acid molecules or anti-PRO-nucleic acid molecules contained in cells that ordinarily express PRO polypeptides or anti-PRO antibodies where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example. DNA for a presequence or secretory leader is operably linked to DNA for a PRO polypeptide if it is expressed as a prcprotein that participates in the secretion of the polypeptide; a promoter or enhancer is operably linked to a coding sequence if it effects the transcription of the sequence: or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Stringency" ofhybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, while lower temperatures less so. For additional details and explanation of stringency of hybridization reactions, *see*, Ausubel et al., Current Protocols in Molecular Biology (Wiley Interscience Publishers, 1995).

"Stringent conditions" or "high-stringency conditions", as defined herein, may be identified by those that: (1) employ low ionic strength and high temperature for washing, for example, 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately-stringent conditions" may be identified as described by Sambrook et al., Molecular Cloning: A Laboratory Manual (New York: Cold Spring Harbor Press, 1989), and include the use of washing solution and hybridization conditions (*e.g.,* temperature, ionic strength, and % SDS) less stringent than those described above. An example of moderately stringent conditions is overnight incubation at 37°C in a solution comprising: 20% formamide. 5 x SSC (150 mM NaCl. 15 mM trisodium citrate), 50 mM sodium phosphate (pH 7.6), 5 x Denhardt's solution, 10%dextran sulfate, and 20 mg/ml denatured sheared salmon sperm DNA, followed by washing the filters in I x SSC at about 37-50°C. The skilled artisan will recognize how to adjust the temperature, ionic strength, etc. as necessary to accommodate factors such as probe length and the like.

The modifier "epitope-tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

"Active" or "activity" in the context of PRO variants refers to form(s) of PRO proteins that retain the biologic and/or immunologic activities of a native or naturally-occurring PRO polypeptide.

"Biological activity" in the context of a molecule that antagonizes a PRO polypeptide that can be identified by the screening assays disclosed herein (*e.g.*, an organic or inorganic small molecule, peptide, etc.) is used to refer to the ability of such molecules to bind or complex with the PRO polypeptide identified herein, or otherwise interfere with the interaction of the PRO polypeptides with other cellular proteins or otherwise inhibits the transcription or translation of the PRO polypeptide. Particularly preferred biological activity includes cardiac hypertrophy, activity that acts on systemic disorders that affect vessels, such as diabetes mellitus, as well as diseases of the arteries, capillaries, veins, and/or lymphatics, and cancer.

The term "antagonist" is used in the broadest sense, and includes any molecule that partially or fully blocks, inhibits, or neutralizes one or more of the biological activities of a native PRO polypeptide disclosed herein, for example, if applicable, its mitogenic or angiogenic activity. Antagonists of a PRO polypeptide may act by interfering with the binding of a PRO polypeptide to a cellular receptor, by incapacitating or killing cells that have been activated by a PRO polypeptide, or by interfering with vascular endothelial cell activation after binding of a PRO polypeptide to a cellular receptor. All such points of intervention by a PRO polypeptide antagonist shall be considered equivalent for purposes of this invention. The antagonists inhibit the mitogenic, angiogenic, or other biological activity of PRO polypeptides, and thus are useful for the treatment of diseases or disorders characterized by undesirable excessive neovascularization, including by way of example tumors, and especially solid malignant tumors, rheumatoid arthritis, psoriasis, atherosclerosis, diabetic and other retinopathies, retrolental fibroplasia, age-related macular degeneration, neovascular glaucoma, hemangiomas, thyroid hyperplasias (including Grave's disease), corneal and other tissue transplantation, and chronic inflammation. The antagonists also are useful for the treatment of diseases or disorders characterized by undesirable excessive vascular permeability, such as edema associated with brain tumors, ascites associated with malignancies, Meigs'syndrome, lung inflammation, nephrotic syndrome, pericardial effusion (such as that associated with pericarditis), and pleural effusion. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments, or amino acid sequence variants of native PRO polypeptides, peptides, small organic molecules, etc.

A "small molecule" is defined herein to have a molecular weight below about 500 daltons.

The term "PRO polypeptide receptor" as used herein refers to a cellular receptor for a PRO polypeptide, ordinarily a cell-surface receptor found on vascular endothelial cells, as well as variants there of that retain the ability to bind a PRO polypeptide.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules that lack antigen specificity. Polypeptides of the latter kind are, for example, produced at low levels by the lymph system and at increased levels by myelomas. The term "antibody" is used in the broadest sense and specifically covers, without limitation, intact monoclonal antibodies, polyclonal antibodies, multispecific antibodies (*e.g.,* bispecific antibodies) formed from at least two intact antibodies, and antibody fragments, so long as they exhibit the desired biological activity.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody to and for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework regions (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies. *See*, Kabat et al., NIH Publ. No.91-3242. Vol. I, pages 647-669 (1991). The constant domains are not involved directly in binding an antibody to an antigen, but exhibit various effector functions, such as participation of the antibody in antibody-dependent cellular toxicity.

"Antibody fragments" comprise a portion of an intact antibody, preferably the antigen-binding or variable region of the intact antibody. Examples of antibody fragments include Fab. Fab', $F(ab')_2$, and Fv fragments; diabodies;linearantibodies(Zapata et al., Protein Eng., 8(10): 1057-1062 (1995)): single-chain antibody molecules; and multispecific antibodies formed from antibody fragments.

Papain digestion of antibodies produces two identical antigen-binding fragments, called "Fab" fragments,each with a single antigen-binding site, and a residual "Fc" fragment, whose name reflects its ability to crystallize readily. Pepsin treatment yields an $F(ab')_2$ fragment that has two antigen-combining sites and is still capable of cross-linking antigen.

"Fv" is the minimum antibody fragment that contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the $V_H$-$V_l$ dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH I domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM; and several of these may be further divided into subclasses (isotypes), *e.g.*, IgG1, IgG2, IgG3, IgG4, IgA, and IgA2. The heavy-chain constant domains that correspond to the different classes of immunoglobulins are called α, δ, ε, γ, and μ, respectively. The subunit structures and three-dimensional configurations of different classes of immunoglobulins are well known.

The term "monoclonal antibody" as used herein refers to an antibody obtained from a population of substantially homogeneous antibodies, *i.e.,* the individual antibodies comprising the population are identical except for possible naturally-occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256: 495 (1975), or may be made by recombinant DNA methods (*see, e.g.,* U.S. Patent No. 4,816,567). The "monoclonal antibodies" may also be isolated from phage antibody libraries using the techniques described in Clackson et al., Nature, 352: 624-628 (1991) and Marks et al., J. Mol. Biol., 222: 581-597 (1991), for example.

The monoclonal antibodies herein specifically include "chimeric" antibodies (immunoglobulins) in which a portion of the heavy and'or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity. U.S. Patent No. 4,816,567; Morrison et al., Proc. Natl. Acad. Sci. USA, 81:6851-6855 (1984).

"Humanized" forms of non-human (*e.g.,* murine) antibodies are chimeric immunoglobulins, immunoglobulin chains, or fragments thereof (such as Fv, Fab, Fab', $F(ab')_2$ or other antigen-binding subsequences of antibodies) that contain minimal sequence derived from non-human immunoglobulin. For the most part, humanized antibodies are human immunoglobulins (recipient antibody) in which residues from a CDR of the recipient are replaced by residues from a CDR of a non-human species (donor antibody) such as mouse, rat or rabbit having the desired specificity, affinity, and capacity. In some instances, Fv FR residues of the human immunoglobulin are replaced by corresponding non-human residues. Furthermore, humanized antibodies may comprise residues that are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all ofthe FR regions are those of a human immunoglobulin sequence. The humanized antibody preferably also will comprise at least a portion of an immunoglobulin constant region (Fc), typically that of a human immunoglobulin. For further details, *see* Jones el al., Nature, 321: 522-525 (1986); Reichmann el al., Nature, 332: 323-329 (1988); and Presta, Curr. Op. Struct. Biol., 2: 593-596 (1992). The humanized antibody includes a PRIMATIZED™ antibody wherein the antigen-binding region of the antibody is derived from an antibody produced by immunizing macaque monkeys with the antigen of interest.

"Single-chain Fv" or "sFv" antibody fragments comprise the $V_H$ and $V_L$ domains of an antibody, wherein these domains are present in a single polypeptide chain. Preferably, the Fv polypeptide further comprises a polypeptide linker between the $V_H$ and $V_L$ domains that enables the sFv to form the desired structure for antigen binding. For a review of sFv *see,* Pluckthun in The Pharmacology of Monoclonal Antibodies, Vol. 113, Rosenburg and Moore, eds. (Springer-Verlag: New York, 1994), pp. 269-315.

The term "diabodies" refers to small antibody fragments with two antigen-binding sites, which fragments comprise a heavy-chain variable domain ($V_H$) connected to a light-chain variable domain ($V_L$) in the same polypeptide chain

($V_H$ - $V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90: 6444-6448 (1993).

An "isolated" antibody is one that has been identified and separated and/or recovered from a component of its natural environment. Contaminant components of its natural environment are materials that would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells, since at least one component of the antibody's natural environment will not be present. Ordinarily, however, isolated antibody will be prepared by at least one purification step.

The word "label" when used herein refers to a detectable compound or other composition that is conjugated directly or indirectly to the antibody so as to generate a "labeled" antibody. The label may be detectable by itself (*e.g.*, radioisotope labels or fluorescent labels) or, in the case of an enzymatic label, may catalyze chemical alteration of a substrate compound or composition that is detectable. Radionuclides that can serve as detectable labels include, for example, I-131, I-123, I-125, Y-90, Re-188. At-211, Cu-67, Bi-212, and Pd-109. The label may also be a non-detectable entity such as a toxin.

By "solid phase" is meant a non-aqueous matrix to which an antibody of the present invention can adhere. Examples of solid phases encompassed herein include those formed partially or entirely of glass (*e.g.,* controlled pore glass), polysaccharides (*e.g.,* agarose), polyacrylamides, polystyrene, polyvinyl alcohol and silicones. In certain embodiments, depending on the context, the solid phase can comprise the well of an assay plate; in others it is a purification column (*e.g.*, an affinity chromatography column). This term also includes a discontinuous solid phase of discrete particles, such as those described in U.S. Patent No. 4.275,149.

A "liposome" is a small vesicle composed of various types of lipids, phospholipids and/or surfactant that is useful for delivery of a drug (such as the PRO polypeptide or antibodies thereto disclosed herein) to a mammal. The components of the liposome are commonly arranged in a bilayer formation, similar to the lipid arrangement of biological membranes.

As used herein, the term "immunoadhesin" designates antibody-like molecules that combine the binding specificity of a heterologous protein (an "adhesin") with the effector functions of immunoglobulin constant domains. Structurally, the immunoadhesins comprise a fusion ofan amino acid sequence with the desired binding specificity that is other than the antigen recognition and binding site of an antibody *(i.e.,* is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes. IgA (including IgA-1 and IgA-2), IgE, IgD, or IgM.

11. <u>Compositions and Methods of the Invention</u>

A. <u>PRO Variants</u>

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO polypeptide, such as changing the number or position of glycosylation sites or altering the membrane anchoring characteristics.

Variations in the native full-length sequence PRO polypeptide or in various domains of the PRO polypeptide described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO polypeptide that results in a change in the amino acid sequence of the PRO polypeptide as compared with the native sequence PRO polypeptide. Optionally the variation is by substitution of at least one amino acid with any other amino acid in one or more of the domains of the PRO polypeptide. Guidance in determining which amino acid residue may be inserted, substituted or deleted without adversely affecting the desired activity may be found by comparing the sequence of the PRO polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, *i.e.*, conservative amino acid replacements.

Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

In particular embodiments, conservative substitutions of interest are shown in Table 3 under the heading of preferred substitutions. If such substitutions result in a change in biological activity, then more substantial changes, denominated exemplary substitutions in Table 3, or as further described below in reference to amino acid classes, are introduced and the products screened.

Table 3

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu: val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res.. 13: 4331 (1986); Zoller et al., Nucl. Acids Res., 10:6487 (1987)], cassette mutagenesis [ Wells et al., Gene, 34:315 (1985)], restriction selection mutagenesis [Wells et a/., Philos. Trans. R. Soc. London SerA. 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

B. Modifications of PRO Polypeptides

Covalent modifications of PRO polypeptides are included within the scope of this invention. One type of covalent modification includes reacting targeted amino acid residues of a PRO polypeptide with an organic derivatizing agent that is capable of reacting with selected side chains or the N- or C- terminal residues of the PRO polypeptide. Derivatization with bifunctional agents is useful, for instance, for crosslinking a PRO polypeptide to a water-insoluble support matrix or surface for use in the method for purifying anti-PRO antibodies, and vice-versa. Commonly used cross linking agents include, *e.g.,* 1,1-bis(diazoacetyl)-2-phenylethane, glutaraldchyde, N-hydroxy, succinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1.8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\alpha$-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO polypeptides (either by removing the underlying glycosylation site or by deleting the glycosylation by chemical and/or enzymatic means), and/or adding one or more glycosylation sites that are not present in the native sequence PRO polypeptide. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO polypeptide (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number ofcarbohydrate moieties on the PRO polypeptide is by chemical or enzymatic coupling of glycosides to the polypeptide. Such methods are described in the art, *e.g.*, in WO 87/05330 published I 1 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO polypeptides comprises linking the PRO polypeptide to one of a variety of nonproteinaceous polymers, *e.g.,* polyethylene glycol (PEG), polypropylene glycol, or polyoxyalkylenes, in the manner set forth in U.S. Patent Nos. 4,640,835; 4.496,689; 4,301,144; 4,670,417; 4,791,192 or 4,179.337.

The PRO poiypeptidcs of the present invention may also be modified in a way to form a chimeric molecule comprising the PRO polypeptide fused to another, heterologous polypeptide or amino acid sequence.

In one embodiment, such a chimeric molecule comprises a fusion of the PRO polypeptide with a tag polypeptide which provides an epitope to which an anti-tag antibody can selectively bind. The epitope tag is generally placed at the amino- or carboxyl- terminus of the PRO polypeptide. The presence of such epitope-tagged forms of the PRO polypeptide can be detected using an antibody against the tag polypeptide. Also, provision of the epitopetag enables the PRO polypeptide to be readily purified by affinity purification using an anti-tag antibody or another type of affinity matrix that binds to the epitope tag. Various tag polypeptides and their respective antibodies are well known in the art. Examples include poly-histidine (poly-His) or poly-histidine-olycine (poly-His-gly) tags; the flu HA tag polypeptide and its antibody 12CA5 [Field el al., Mol. Cell. Biol., 8:2159-2165 (1988)]; the c-myc tag and the 8F9, 3C7, 6E10, G4, B7 and 9E10 antibodies thereto [Evan el al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypept ides include the Flag-peptide[Hopp et al., BioTechnology, 6:1204-1210(1988)]; the KT3 epitope peptide [Martin et al., Science, 255:192-194 (1992)]; an $\alpha$-tubulin epitope peptide [Skinner et al., J. Biol. Chem., 266:15163-15166 (1991)]; and the T7 gene 10 protein peptide tag [Lutz-Freyetmuth et al., Proc. Natl. Acad. Sci. USA, 87:6393-6397 (1990)].

In an alternative embodiment, the chimeric molecule may comprise a fusion of the PRO polypeptide with an immunoglobulin or a particular region of an immunoglobulin. For a bivalent form of the chimeric molecule (also referred to as an "immunoadhesin"), such a fusion could be to the Fc region of an IgG molecule. The fusions preferably include the substitution of a soluble (transmembrane domain deleted or inactivated) form of a PRO polypeptide in place ofat least one variable region within an Ig molecule. In a particularly preferred embodiment, the immunoglobulin fusion includes the hinge, CH2 and CH3, or the hinge, CH1, CH2 and CH3 regions of an IgG I molecule. For the production of immunoglobulin fusions *see* also, US Patent No. 5,428,130 issued June 27, 1995.

C. <u>Preparation of the PRO polypeptides</u>

The present invention provides newly identified and isolated nucleotide sequences encoding polypeptides referred to in the present application as PRO. In particular, cDNAs encoding PRO polypeptides have been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by DNA35916-1161, DNA23339-1130, DNA16451-1388, DNA27865-1091, DNA27864-1155, DNA28497-1130. DNA26847-1395, DNA30942-1134, DNA32286-1191, DNA33094-1131, DNA33221-1133. DNA34434-1139. DNA35558-1167, DNA35638-1141, DNA33473-1176. DNA38260-1180, DNA39969-1185,DNA40628-1216, DNA35595-1228,DNA40981-1234,DNA47470-1130-PLDNA47365-1206, DNA44184-1319, DNA48613-1268, DNA29101-1122, DNA49646-1327, DNA49829-1346, DNA56405-1357, DNA56352-1358, DNA59205-1421, DNA53974-1401, DNA57689-1385, DNA60615-1483, DNA59814-1486, DNA59846-1503, DNA64883-1526. DNA64885-1529, DNA64889-1541, DNA64903-1553, DNA64905-1558, DNA65409-1566, DNA65406-1567. DNA61873-1574, DNA64966-1575, DNA67300-1605, DNA68872-1620, DNA76538-1670, or DNA33087 as well as all further native homologues and variants included in the foregoing definition of PRO, will be referred to as "PRO", respectively, regardless of their origin or mode of preparation.

The description below relates primarily to production of PRO polypeptides by culturing cells transformed or transfected with a vector containing nucleic acid encoding PRO polypeptides. It is, of course, contemplated that alternative methods that are well known in the art may be employed to prepare PRO polypeptides. For instance, the PRO polypeptide sequence, or portions thereof, may be produced by direct peptide synthesis using solid-phase techniques. *See, e.g.,* Stewart et al., Solid-Phase Peptide Synthesis (W.H. Freeman Co.: San Francisco, CA, 1969); Merrifield. J. Am. Chem. Soc., 85: 2149-2154 (1963). *In vitro* protein synthesis may be performed using manual techniques or by automation. Automated synthesis may be accomplished, for instance, with an Applied Biosystems Peptide Synthesizer (Foster City, CA) using manufacturer's instructions. Various portions of a PRO polypeptide may be chemically synthesized separately and combined using chemical or enzymatic methods to produce the full-length PRO polypeptide.

i. <u>Isolation of DNA Encoding PRO Polypeptides</u>

DNA encoding a PRO polypeptide may be obtained from a cDNA library prepared from tissue believed to possess the mRNA encoding the PRO polypeptide and to express it at a detectable level. Accordingly, DNAs encoding human PRO polypeptides can be conveniently obtained from cDNA libraries prepared from human tissues, such as described in the Examples. The gene encoding a PRO polypeptide may also be obtained from a genomic library or by oligonucleotide synthesis.

Libraries can be screened with probes (such as antibodies to the PRO polypeptide or oligonucleotides of at least about 20-80 bases) designed to identify the gene of interest or the protein encoded by it. Screening the cDNA or genomic library with the selected probe may be conducted using standard procedures, such as described in Sambrook *et al., supra.* An alternative means to isolate the gene encoding a PRO polypeptide is to use PCR methodology. Sambrook *et al., supra;* Dieffenbach et al. PCR Primer: A Laboratory Manual (New York: Cold Spring Harbor Laboratory Press, 1995).

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like $^{32}P$-labeled ATP, biotinylation, or enzyme labeling. Hybridization conditions, including moderate stringency and high stringency, are provided in Sambrook *et al., supra.*

Sequences identified in such library screening methods can be compared and aligned to other known sequences deposited and available in public databases such as GenBank or other private sequence databases. Sequence identity(at either the amino acid or nucleotide level) within defined regions of the molecule or across the full-length sequence can be determined through sequence alignment using computer software programs such as ALIGN, DNAstar, and INHERIT. which employ various algorithms to measure homology.

Nucleic acid having protein coding sequence may be obtained by screening selected cDNA or genomic libraries using the deduced amino acid sequence disclosed herein for the first time, and, if necessary, using conventional primer extension procedures as described in Sambrook *et al., supra,* to detect precursors and processing intermediates of mRNA that may not have been reverse-transcribed into cDNA.

ii. Selection and Transformation of Host Cells

Host cells are transfected or transformed with expression or cloning vectors described herein for PRO polypeptide production and cultured in conventional nutrient media modified as appropriate for inducing promoters. selecting transformants, or amplifying the genes encoding the desired sequences. The culture conditions, such as media, temperature, pH, and the like, can be selected by the skilled artisan without undue experimentation. In general, principles, protocols, and practical techniques for maximizing the productivity of cell cultures can be found in Mammalian Cell Biotechnology: A Practical Approach, M. Butler, ed. (IRL Press, 1991) and Sambrook *et al., supra.*

Methods of transfection are known to the ordinarily skilled artisan, for example, $CaPO_4$ treatment and electroporation. Depending on the host cell used, transformation is performed using standard techniques appropriate to such cells. The calcium treatment employing calcium chloride, as described in Sambrook *et al., supra,* or electroporation is generally used for prokaryotes or other cells that contain substantial cell-wall barriers. Infection with *Agrobacterium tumefaciens* is used for transformation of certain plant cells, as described by Shaw et al., Gene, 23: 315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transformations have been described in U.S. Patent No. 4,399,216. Transformations into yeast are typically carried out according to the method of Van Solingen et al., J. Bact., 130: 946 (1977) and Hsiao et al., Proc. Natl. Acad. Sci. (USA), 76: 3829 (1979). However, other methods for introducing DNA into cells, such as by nuclear microinjection, electroporation, bacterial protoplast fusion with intact cells, or polycations, *e.g.,* polybrene or polyornithine, may also be used. For various techniques for transforming mammalian cells. *see,* Keown et al., Methods in Enzymology, 185: 527-537 (1990) and Mansour et al., Nature, 336: 348-352 (1988).

Suitable host cells for cloning or expressing the DNA in the vectors herein include prokaryote, yeast, or higher eukaryote cells. Suitable prokaryotes include, but are not limited to, eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X 1776 (ATCC 31.537); *E. coli* strain W3110 (ATCC 27,325); and K5 772 (ATCC 53,635). Other suitable prokaryotic host cells include Enterobacteriaceae such as *Escherichia, e.g., E. coli. Enterobacter, Erwinia, Klebsiella, Proteus, Salmonella, e.g., Salmonella typhimurium, Serratia, e.g., Serratia marcescans,* and *Shigella,* as well as *Bacilli* such as *B. subtilis* and *B. licheniformis* (*e.g., B. licheniformis* 41 P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of pruteolytic enzymes. Fur example, strain W3110 may be modified to effect a genetic mutation in the genes encoding proteins endogenous to the host, with examples of such hosts including *E coli* W3110 strain 1A2, which has the complete genotype *tonA*; *E. coli* W3110 strain 9E4, which has the complete genotype *tonA ptr3*: *E. coli* W3110 strain 27C7(ATCC 55,244), which has the complete genotype *tonA ptr3 phoA E15 (argF-lac)169 degP ompT kan'*; *E. coli* W3110 strain 37D6, which has the complete genotype *tonA ptr3 phoA E15 (argF-lac)169 degP ompT rbs7 ilvG kan$^{t}$*; *E coli* W3110 strain 40B4, which is strain 37D6 with a non-kanamycin resistant *degP* deletion mutation: and an *E. coli* strain having mutant periplasmic protease disclosed in U.S. Patent No. 4,946.783 issued 7 August 1990. Alternatively, *in vitro* methods of cloning, *e.g.,* PCR or other nucleic acid polymerase reactions, are suitable.

In addition to prokaryotes, eukaryotic microbes such as filamentous fungi or yeast are suitable cloning or expression hosts for vectors encoding PRO polypeptides. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9: 968-975 (1991)) such as, *e.g., K. lactis* (MW98-8C, CBS683, CBS4574; Louvencourt et al., J. Bacteriol., 737 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8: 135 (1990)), *K. thermotolerans,* and *K. marxianus; yarrowia* (EP 402,226); *Pichia pastoris* (EP 183,070; Sreekrishna et al., J. Basic Microbiol., 28: 265-278 [1988]); *Candida; Trichodermu reesia* (EP 244,234); *Neurospora crassa* (Case et al., Proc. Natl. Acad. Sci. USA, 76: 5259-5263 [1979] ); *Schwanniomyces* such as *Schwanniomyces occidentalis* (EP 394,538 published 31 October 1990); and filamentousfungi such as, *e.g., Neurospora, Penicillium, Tolypocladium* (WO 91/00357 published 10 January 1991 ), and *Aspergillus* hosts such as *A. nidulans* (Ballance et al., Biochem. Biophys. Res. Commun., 112: 284-289 [1983]; Tilbum et al., Gene, 26: 205-221 [1983]; Yelton et al., Proc. Natl. Acad. Sci. USA, 81: 1470-1474 [1984]) and *A. niger* (Kelly and Hynes, EMBO J., 4: 475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of nucleic acid encoding a glycosylated PRO polypeptide are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV 1 line transformed by SV40 (COS-7, ATCC CRL 1651); human embryonic kidney line (293 or 293 cells subcloned for growth in suspension culture, Graham et al.. J. Gen Virol., 36: 59 (1977)); Chinese hamster ovary cells/-DHFR (CHO, Urlaub and Chasin, Proc. Natl. Acad. Sci. USA. 77:4216 (1980)); mouse sertoli cells (TM4, Mather, Biol, Reprod., 23:243-251 (1980)); human lung cells (W 138, ATCC CCL 75); human liver cells (Hep G2, HB 8065); and mouse mammary tumor (MMT 060562, ATCC CCL51 The selection of the appropriate host cell is deemed to he within the skill in the art.

iii. Selection and Use of a Replicable Vector

The nucleic acid (*e.g.,* cDNA or genomic DNA) encoding a PRO polypeptide may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence if the sequence is to be secreted, an origin of replication, one or more marker genes, an enhancer element, a promoter, and a transcription termination sequence. Construction of suitable vectors containing one or more ofthese components employs standard ligation techniques that are known to the skilled artisan.

The PRO polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the DNA encoding the PRO polypeptide that is inserted into the vector. The signal sequence may be a prokaryotic signal sequence selected, for example, from the group of the alkaline phosphatase, penicillinase, Ipp, or heat-stable enterotoxin II leaders. For yeast secretion the signal sequence may be, *e.g.,* the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV, or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.,* ampicillin, neomycin, methotrexate, or tetracycline, (b) complement auxotrophic deficiencies, or (c) supply critical nutrients not available from complex media, *e.g.,* the gene encoding D-alanine racemase for *Bacilli.*

An example of suitable selectable markers for mammalian cells are those that enable the identification of cells competent to take up the nucleic acid encoding a PRO polypeptide, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77: 4216 (1980). A suitable selection gene for use in yeast is the *trp*l gene present in the yeast plasmid YRp7. Stinchcomb et al., Nature, 282: 39(1979); Kingsman et al., Gene, 7: 141 (1979): Tschemper et al., Gene, 10: 157 (1980). The *trp* I gene provides a selection marker for a mutant strain of yeast lacking the ability to grow in tryptophan, for example, ATCC No. 44076 or PEP4-1. Jones, Genetics. 85: 12 (1977).

Expression and cloning vectors usually contain a promoter operably linked to the nucleic acid sequence encoding a PRO polypeptide to direct mRNA synthesis. Promoters recognized by a variety of potential host cells are well known. Promoters suitable for use with prokaryotic hosts include the β-lactamase and lactose promoter systems (Chang et al., Nature. 275: 615 (1978). Goeddel et al., Nature, 281: 544 (1979)), alkaline phosphatase, a tryptophan (trp) promoter system (Goeddel, Nucleic Acids Res.. 8: 4057 (1980): EP 36,776), and hybrid promoters such as the tac promoter, deBoer et al., Proc. Natl. Acad. Sci. USA, 80: 21-25 (1983). Promoters for use in bacterial systems also will contain a Shine-Dalgarno (S.D.) sequence operably linked to the UNA encoding the PRO polypeptide.

Examples of suitable promoting sequences for use with yeast hosts include the promoters for 3-phosphoglycerate kinase (Hitzeman et al., J. Biol. Chem., 255: 2073 (1980)) or other glycolytic enzymes (Hess et al., J. Adv. Enzyme Reg., 7: 149 (1968): Holland, Biochemistry, 17: 4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters that are inducible promoters having the additional advantage of transcription controlled by growth conditions are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO nucleic acid transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus, and Simian Virus 40 (SV40); by heterologous mammalian promoters, *e.g.,* the actin promoter or an immunoglobulin promoter; and by heat-shock promoters, provided such promoters are compatible with the host cell systems.

Transcription of a DNA encoding the PRO polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, $\alpha$-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the sequence coding for a PRO polypeptide, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragmems in the untranslated portion of the mRNA encoding the PRO polypeptide.

Still other methods, vectors, and host cells suitable for adaptatiun to the synthesis of a PRO polypeptide in recombinant vertebrate cell culture are described in Gething et al., Nature. 293: 620-625 (1981): Mantei et al., Nature, 281: 40-46 (1979): EP 117.060; and EP 117.058.

iv. <u>Detecting Gene Amplification/Expression</u>

Gene amplification and/or expression may be measured in a sample directly, for example, by conventional Southern blotting. Northern blotting to quantitate the transcription ofmRNA (Thomas, Proc. Natl. Acad. Sci. USA. 77: 5201-5205 (1980)), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native-sequence PRO polypeptide or against a synthetic peptide based on the DNA sequences provided herein or against exogenous sequence fused to DNA encoding the PRO polypeptide and encoding a specific antibody epitope.

v. <u>Purification of Polypeptide</u>

Forms of PRO polypeptides may be recovered from culture medium or from host cell lysates. If membrane-bound, it can be released from the membrane using a suitable detergent solution (*e.g.,* TRITON-X™ 100) or by enzymatic cleavage. Cells employed in expression of nucleic acid encoding the PRO polypeptide can be disrupted by various physical or chemical means, such as freeze-thaw cycling, sonication, mechanical disruption, or cell-lysing agents.

It may be desired to purify the PRO polypeptide from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE: chromatofocusing; SDS-PAGE; ammonium sulfate precipitation; gel filtration using, for example, Sephadex G-75: protein A Sepharose columns to remove contaminants such as IgG; and metal chelating columns to bind epitope-tagged forms of the PRO polypeptide. Various methods of protein purification may be employed and such methods arc known in the art and described, for example, in Deutscher, Methods in Enzymology. 182 (1990); Scopes. Protein Purification: Principles and Practice (Springer-Verlag: New York, 1982). The purification step(s) selected will depend, for example, on the nature of the production process used and the particular PRO polypeptide produced.

D Uses of the PRO polypeptides

i. Assays for Cardiovascular, Endothelial, and Angiogenic Activity

Various assays can be used to test the polypeptide herein for cardiovascular, endothelial, and angiogenic activity. Such assays include those provided in the Examples below.

Assays for testing for endothelin antagonist activity, as disclosed in U.S. Pat. No. 5,773,414. include a rat heart ventricle binding assay where the polypeptide is tested for its ability to inhibit iodinized endothelin-1 binding in a receptor assay, an endothelin receptor binding assay testing for intact cell binding of radiolabeled endothelin-I using rabbit renal artery vascular smooth muscle cells, an inositol phosphate accumulation assay where functional activity is determined in Rat-1 cells by measuring intra-cellular levels of second messengers, an arachidonic acid releaseassay that measures the ability of added compounds to reduce endothelin-stimulated arachidonic acid release in cultured vascular smooth muscles, *in vitro* (isolated vessel) studies using endothelium from male New Zealand rabbits, and *in vivo* studies using male Sprague-Dawley rats.

Assays for tissue generation activity include, without limitation, those described in WO 95/16035 (bone, cartilage, tendon); WO 95/05846 (nerve, neuronal), and WO 91/07491 (skin, endothelium).

Assays for wound-healing activity include, for example, those described in Winter, Epidermal Wound Healing, Maibach, HI and Rovee, DT, eds. (Year Book Medical Publishers, Inc., Chicago), pp. 71-112, as modified by the article of Eaglstein and Mertz, J. Invest. Dermatol., 71: 382-384 (1978).

An assay to screen for a test molecule relating to a PRO polypeptide that binds an endothelin $B_1$ ($ETB_1$) receptor polypeptide and modulates signal transduction activity involves providing a host cell transformed with a DNA encoding endothelin $B_1$ receptor polypeptide, exposing the cells to the test candidate, and measuring endothelin B, receptor signal transduction activity, as described, *e.g.*, in U.S. Pat. No. 5,773,223.

There are several cardiac hypertrophy assays. *In vitro* assays include induction of spreading of adult rat cardiac myocytes. In this assay, ventricular myocytes are isolated from a single (male Sprague-Dawley) rat, essentially following a modification of the procedure described in detail by Piper et al., "Adult ventricular rat heart muscle cells" in Cell Culture Techniques in Heart and Vessel Research, H.M. Piper, ed. (Berlin: Springer-Verlag, 1990), pp. 36-60. This procedure permits the isolation of adult ventricular myocytes and the long-term culture of these cells in the rod-shaped phenotype. Phenylephrine and Prostaglandin $F_{2\alpha}$ ($PGF_{2\alpha}$) have been shown to induce a spreading response in these adult cells. The inhibition of myocyte spreading induced by $PGF_{2\alpha}$ or $PGF_{2\alpha}$ analogs (*e.g.,* fluprostenol) and phenylephrine by various potential inhibitors of cardiac hypertrophy is then tested.

One example of an *in vivo* assay is a test for inhibiting cardiac hypertrophy induced by fluprostenol *in vivo.* This pharmacological model tests the ability of the PRO polypeptide to inhibit cardiac hypertrophy induced in rats (*e.g.,* male Wistar or Sprague-Dawley) by subcutaneous injection of fluprostenol (an agonist analog of $PGF_{2\alpha}$). It is known that rats with pathologic cardiac hypertrophy induced by myocardial infarction have chronically elevated levels of extractable $PGF_{2\alpha}$ in their myocardium. Lai et al., Am. J. Physiol. (Heart Circ. Physiol.), 271: H2197-H2208 (1996), Accordingly, factors that can inhibit the effects of fluprostenol on myocardial growth *in vivo* are potentially useful for treating cardiac hypertrophy. The effects of the PRO polypeptide on cardiac hypertrophy are determined by measuring the weight of heart, ventricles, and left ventricle (normalized by body weight) relative to fluprostenol-treated rats not receiving the PRO polypeptide.

Another example of an *in vivo* assay is the pressure-overload cardiac hypertrophy assay. For *in vivo* testing it is common to induce pressure-overload cardiac hypertrophy by constriction of the abdominal aorta of test animals. In a typical protocol, rats (*e.g.,* male Wistar or Sprague-Dawley) are treated under anesthesia, and the abdominal aorta of each rat is narrowed down just below the diaphragm. Beznak M., Can. J. Biochem. Physiol., 33: 985-94 (1955). The aorta is exposed through a surgical incision, and a blunted needle is placed next to the vessel. The aorta is constricted with a ligature of silk thread around the needle, which is immediately removed and which reduces the lumen of the aorta to the diameter of the needle. This approach is described, for example, in Rossi et al., Am. Heart J., 124: 700-709 (1992) and O'Rourke and Reibel, P.S.E.M.B., 200: 95-100 (1992).

In yet another *in vivo* assay, the effect on cardiac hypertrophy following experimentally induced myocardial infarction (MI) is measured. Acute MI is induced in rats by left coronary artery ligation and confirmed by electrocardiographic examination. A sham-operated group of animals is also prepared as control animals. Earlier data have shown that cardiac hypertrophy is present in the group of animals with MI, as evidenced by an 18% increase in heart weight-to-body weight ratio. Lai *el al., supra.* Treatment of these animals with candidate blockers of cardiac hypertrophy, *e.g..* a PRO polypeptide, provides valuable information about the therapeutic potential of the candidates tested. One further such assay test for induction of cardiac hypertrophy is disclosed in U.S. Pat. No. 5,773,415, using Sprague-Dawley rats.

For cancer, a variety of well-known animal models can be used to further understand the role of the genes identified herein in the development and pathogenesis of tumors, and to test the efficacy of candidate therapeutic agents, including antibodies and other antagonists of the native PRO polypeptides, such as small-molecule antagonists. The *in vivo* nature of such models makes them particularly predictive of responses in human patients. Animal models of tumors and cancers (*e.g.,* breast cancer, colon cancer, prostate cancer, lung cancer, etc.) include both non-recombinant and recombinant (transgenic) animals. Non-recombinant animal models include, for example, rodent, *e.g.,* murine models. Such models can be generated by introducing tumor cells into syngeneic mice using standard techniques, *e.g..* subcutaneous injection, tail vein injection, spleen implantation, intraperitoneal implantation, implantation under the renal capsule, or orthopin implantation, *e.g.,* colon cancer cells implanted in colonic tissue. *See, e.g.,* PCT publication No. WO 97/33551, published September 18, 1997. Probably the most often used animal species in oncological studies are immunodeficient mice and, in particular, nude mice. The observation that the nude mouse with thymic hypo/aplasia could successfully act as a host for human tumor xenografts has lead to its widespread use for this purpose. The autosomal recessive *nu* gene has been introduced into a very large number of distinct congenic strains of nude mouse, including, for example, ASW, A/He, AKR, BALB/c, B10.LP, C17, C3H, C57BL, C57, CBA, DBA, DDD, I/st, NC, NFR. NFS, NFS/N, NZB, NZC, NZW, P, RIII, and SJL. In addition, a wide variety of other animals with inherited immunological defects other than the nude mouse have been bred and used as recipients of tumor xenografts. For further details *see, e.g.,* The Nude Mouse in Oncology Research, E. Boven and R. Winograd, eds. (CRC Press, Inc., 1991).

The cells introduced into such animals can be derived from known tumor/cancer cell lines, such as any of the above-listed tumor cell lines, and, for example, the B104-1-1 cell line (stable NIH-3T3 cell line transfected with the *neu* protooncogene): *ras*-transfected NIH-3T3 cells: Caco-2 (ATCC HTB-37); or a moderately well-differentiated grade 11 human colon adenocarcinoma cell line, HT-29 (ATCC HTB-38); or from tumors and cancers. Samples of tumor or cancer cells can be obtained from patients undergoing surgery, using standard conditions involving freezing and storing in liquid nitrogen. Karmali et al., Br. J. Cancer. 48: 689-696 (1983).

Tumor cells can be introduced into animals such as nude mice by a variety of procedures. The subcutaneous (s.c.) space in mice is very suitable for tumor implantation. Tumors can he transplanted s.c. as solid blocks, as needle biopsies by use of a trochar, or as cell suspensions. For solid-block or trochar implantation, tumor tissue fragments of suitable size are introduced into the s.c. space. Cell suspensions are freshly prepared from primary tumors or stable tumor cell lines, and injected subcutaneously. Tumor cells can also be injected as subdermal implants. In this location, the inoculum is deposited between the lower part of the dermal connective tissue and the s.c. tissue.

Animal models of breast cancer can be generated, for example, by implanting rat neuroblastoma cells (from which the *neu* oncogene was initially isolated), or *neu*-transformed NIH-3T3 cells into nude mice, essentially as described by Drebin et al. Proc. Nat. Acad. Sci. USA, 83: 9129-9133 (1986).

Similarly, animal models of colon cancer can be generated by passaging colon cancer cells in animals, *e.g.*, nude mice, leading to the appearance of tumors in these animals. An orthotopic transplant model of human colon cancer in nude mice has been described, for example, by Wang et al., Cancer Research, 54: 4726-4728 (1994) and Too et al., Cancer Research, 55: 681-684 (1995). This model is based on the so-called "METAMOUSE"™ sold by AntiCancer, Inc., (San Diego, California).

Tumors that arise in animals can be removed and cultured *in vitro.* Cells from the *in vitro* cultures can then be passaged to animals. Such tumors can serve as targets for further testing or drug screening. Alternatively, the tumors resulting from the passage can be isolated and RNA from pre-passage cells and cells isolated after one or more rounds of passage analyzed for differential expression of genes of interest. Such passaging techniques can be performed with any known tumor or cancer cell lines.

For example, Meth A. CMS4. CMS5, CMS21, and WEHI-164 are chemically induced fibrosarcomas of BALB/c female mice (DeLeo et al., J. Exp. Med., 146: 720 (1977)), which provide a highly controllable model system for studying the anti-turnor activities of various agents. Palladino et al., J. Immunol., 138: 4023-4032 (1987). Briefly, tumor cells are propagated *in vitro* in cell culture. Prior to injection into the animals, the cell lines are washed and suspended in buffer, at a cell density of about 10x $10^6$ to 10x $10^7$ cells/ml. The animals are then infected subcutaneously with 10 to 100 μl of the cell suspension, allowing one to three weeks for a tumor to appear.

In addition, the Lewis lung (3LL) carcinoma of mice, which is one of the most thoroughly studied experimental tumors, can be used as an investigational tumor model. Efficacy in this tumor model has been correlated with beneficial effects in the treatment of human patients diagnosed with small-cell carcinoma of the long (SCCL). This tumor can be introduced in normal mice upon injection of tumor fragments from an affected mouse or of cells maintained in culture. Zupi et al., Br. J. Cancer. 41:suppl. 4, 30 (1980). Evidence indicates that tumors can be started from injection of even a single cell and that a very high proportion of infected tumor cells survive. For further information about this tumor model *see.* Zacharski, Haemostasis, 16: 300-320 (1986).

One way of evaluating the efficacy of a test compound in an animal model with an implanted tumor is to measure the size of the tumor before and after treatment. Traditionally, the size of implanted tumors has been measured with a slide caliper in two or three dimensions. The measure limited to two dimensions does not accurately reflect the size of the tumor: therefore, it is usually converted into the corresponding volume by using a mathematical formula. However, the measurement of tumor size is very inaccurate. The therapeutic effects of a drug candidate can be better described as treatment-induced growth delay and specific growth delay. Another important variable in the description of tumor growth is the tumor volume doubling time. Computer programs for the calculation and description of tumor growth are also available, such as the program reported by Rygaard and Spang-Thomsen, Proc. 6th Int. Workshop on Immune-Deficient Animals, Wu and Sheng eds. (Basel, 1989), p. 301. It is noted, however, that necrosis and inflammatory responses following treatment may actually result in an increase in tumor size, at least initially. Therefore, these changes need to be carefully monitored, by a combination of a morphometric method and flow cytometric analysis.

Further, recombinant (transgenic) animal models can be engineered by introducing the coding portion of the PRO genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g.*, baboons, chimpanzees and monkeys. Techniques known in the art to introduce a transgene into such animals include pronucleic microinjection (U.S. Patent No. 4,873,191); retrovirus-mediated gene transfer into germ lines (*e.g.*, Van der Putten et al., Proc. Natl. Acad. Sci. USA, 82: 6148-615 (1985)); gene targeting in embryonic stem cells (Thompson et al., Cell, 56: 313-321 (1989)); electroporation of embryos (Lo, Mol. Cell. Biol., 3: 1803-1814 (1983)); and sperm-mediated gene transfer. Lavitrano et al., Cell, 57: 717-73 (1989). For a review, *see* for example, U.S. Patent No. 4,736,866.

For the purpose of the present invention, transgenic animals include those that carry the transgene only in part of their cells ("mosaic animals"). The transgene can be integrated either as a single transgene, or in concatamers, *e.g.*, head-to-head or head-to-tail tandems. Selective introduction of a transgene into a particular cell type is also possible by following, for example, the technique of Lasko et al., Proc. Natl. Acad. Sci. USA, 89: 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis. PCR, or immunocytochemistry. The animals are further examined for signs of tumor or cancer development.

Alternatively, "knock-out" animals can be constructed that have a defective or altered gene encoding a PRO polypeptide identified herein, as a result of homologous recombination between the endogenous gene encoding the PRO polypeptide and altered genomic DNA encoding the same polypeptide introduced into an embryonic cell of the animal. For example, cDNA encoding a particular PRO polypeptide can be used to clone genomic DNA encoding that polypeptide in accordance with established techniques. A portion of the genomic DNA encoding a particular PRO polypeptide can be deleted or replaced with another gene, such as a gene encoding a selectable marker that can be used to monitor integration. Typically, several kilobases of unaltered flanking DNA (both at the 5' and 3' ends) are included in the vector. *See. e.g.,* Thomas and Capecchi, Cell, 51: 503 (1987) for.a description of homologous recombination vectors. The vector is introduced into an embryonic stem cell line (*e.g.,* by electroporation and cells in which the introduced DNA has homologously recombined with the endogenous DNA are selected. *See. e.g.,* Li et al., Cell. 69: 915 (1992). The selected cells are then injected into a blastocyst of an animal (*e.g.,* a mouse or rat) to form aggregation chimeras. *See*, *e.g.,* Bradley, in Teratocarcinomas and Embryonic Stem Cells: A Practical Approach, E. J. Robertson, ed. (IRL: Oxford, 1987), pp. 113-152. A chimeric embryo can then be implanted into a suitable pseudopregnant female foster animal and the embryo brought to term to create a "knock-out" animal. Progeny harboring the homologously recombined DNA in their germ cells can be identified by standard techniques and used to breed animals in which all cells of the animal contain the homologously recombined DNA. Knockout animals can be characterized, for instance, by their ability to defend against certain pathological conditions and by their development of pathological conditions due to absence of the PRO polypeptide.

The efficacy of antibodies specifically binding the PRO polypeptides identified herein, and other drug candidates, can be tested also in the treatment of spontaneous animal tumors. A suitable target for such studies is the feline oral squamous cell carcinoma (SCC). Feline oral SCC is a highly invasive, malignant tumor that is the most common oral malignancy of cats, accounting for over 60% of the oral tumors reported in this species. It rarely metastasizes to distant sites, although this low incidence of metastasis may merely be a reflection of the short survival times for cats with this tumor. These tumors are usually not amenable to surgery, primarily because of the anatomy of the feline oral cavity. At present, there is no effective treatment for this tumor. Prior to entry into the study, each cat undergoes complete clinical examination and biopsy, and is scanned by computed tomography (CT). Cats diagnosed with sublingual oral squamous cell tumors are excluded from the study. The tongue can become paralyzed as a result of such tumor, and even if the treatment kills the tumor, the animals may not be able to feed themselves. Each cat is treated repeatedly, over a longer period of time. Photographs of the tumors will be taken daily during the treatment period, and at each subsequent recheck. After treatment, each cat undergoes another CT scan. CT scans and thoracic radiograms are evaluated every 8 weeks thereafter. The data are evaluated for differences in survival, response, and toxicity as compared to control groups. Positive response may require evidence of tumor regression, preferably with improvement of quality of life and/or increased life span.

In addition.other spontaneous animal tumors, such as fibrosarcoma, adenocarcinoma, lymphoma, chondroma, or leiomyosarcoma of dogs, cats, and baboons can also be tested. Of these, mammary adenocarcinoma in dogs and cats is a preferred model as its appearance and behavior are very similar to those in humans. However, the use of this model is limited by the rare occurrence of this type of tumor in animals.

Other *in vitro* and *in vivo* cardiovascular, endothelial, and angiogenic tests known in the art are also suitable herein.

ii. Tissue Distribution

The results of the cardiovascular, endothelial, and angiogenic assays herein can be verified by further studies, such as by determining mRNA expression in various human tissues.

As noted before, gene amplification and/or gene expression in various tissues may be measured by conventional Southern blotting, Northern blotting to quantitate the transcription of mRNA (Thomas. Proc. Natl. Acad. Sci. USA, 77: 5201-5205 (1980)), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native-sequence PRO polypeptide or against a synthetic peptide based on the DNA sequences provided herein or against exogenous sequence fused to PRO DNA and encoding a specific antibody epitope. General techniques for generating antibodies, and special protocols for *in situ* hybridization are provided hereinbelow.

iii. Antibody Binding Studies

The results of the cardiovascular,endothelial, and angiogenic study can be further verified by antibody binding studies, in which the ability of anti-PRO antibodies to inhibit the effect ofthe PRO polypeptides on endothelial cells or other cells used in the cardiovascular, endothelial, and angiogenic assays is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques (CRC Press, Inc., 1987). pp.147-158.

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte that remain unbound.

Sandwich assays involve the use of two antibodies, each capable of binding to a different immunogenic portion, or epitope, of the protein to he detected. In a sandwich assay, the test sample analyte is bound by a first antibody that is immobilized on a solid support, and thereafter a second antibody binds to the analyte, thus forming an insoluble three-part complex. *See. e.g.,* US Pat No. 4.376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tissue sample may be fresh or frozen or may be embedded in paraffin and fixed with a preservative such as formalin, for example

iv. Cell-Based Tumor Assays

Cell-based assays and animal models for cardiovascular, endothelial, and angiogenic disorders, such as tumors, can be used to verity the findings of a cardiovascular, endothelial, and angiogenic assay herein, and further to understand the relationship between the genes identified herein and the development and pathogenesis of undesirable cardiovascular, endothelial, and angiogenic cell growth. The role of gene products identified herein in the development and pathology of undesirable cardiovascular, endothelial, and angiogenic cell growth, *e.g.,* tumor cells, can be tested by using cells or cells lines that have been identified as being stimulated or inhibited by the PRO polypeptide herein. Such cells include, for example, those set forth in the Examples below.

In a different approach, cells of a cell type known to be involved in a particular cardiovascular, endothelial, and angiogenic disorder are transfected with the cDNAs herein, and the ability of these cDNAs to induce excessive growth or inhibit growth is analyzed. If the cardiovascular, endothelial, and angiogenic disorder is cancer, suitable tumor cells include, for example, stable tumor cells lines such as the B104-1-1 cell line (stable NIH-3T3 cell line transfected with the *neu* protooncogene) and *ras*-transfected NIH-3T3 cells, which can be transfected with the desired gene and monitored for tumorigenic growth. Such transfected cell lines can then be used to test the ability ofpoly- or monoclonal antibodies or antibody compositions to inhibit tumorigenic cell growth by exerting cytostatic or cytotoxic activity on the growth of the transformed cells, or by mediating antibody-dependent cellular cytotoxicity (ADCC). Cells transfected with the coding sequences ofthe genes identified herein can further be used to identify drug candidates for the treatment of cardiovascular, endothelial, and angiogenic disorders such as cancer.

In addition, primary cultures derived from tumors in transgenic animals (as described above) can be used in the cell-based assays herein, although stable cell lines are preferred. Techniques to derive continuous cell lines from transgenic animals are well known in the art. *See, e.g.,* Small et al., Mol. Cell. Biol., 5: 642-648 (1985).

v. Gene Therapy

The PRO polypeptide herein and polypeptidyl agonists and antagonists may be employed in accordance with the present invention by expression of such polypeptides *in vivo*, which is often referred to as gene therapy.

There are two major approaches to getting the nucleic acid (optionally contained in a vector) into the patient's cells: *in vivo* and *ex vivo.* For *in vivo* delivery the nucleic acid is injected directly into the patient, usually at the sites where the PRO polypeptide is required, *i.e.,* the site of synthesis of the PRO polypeptide, if known, and the site *(e.g.,* wound) where biological activity of PRO polypeptide is needed. For *ex vivo* treatment, the patient's cells are removed, the nucleic acid is introduced into these isolated cells, and the modilied cells are administered to the patient either directly or, for example, encapsulated within porous membranes that are implanted into the patient *(see, e.g.,* U.S. Pat. Nos. 4.892,538 and 5,283,187). There are a variety of techniques available for introducing nucleic acids into viable cells. The techniques vary depending upon whether the nucleic acid is transferred into cultured cells *in vitro,* or transferred *in vivo* in the cells of the intended host. Techniques suitable for the transfer of nucleic acid into mammalian cells *in vitro* include the use of liposomes, electroporation, microinjection, transduction, cell fusion. DEAE-dextran, the calcium phosphate precipitation method, etc. Transduction involves the association of a replication-defective, recombinant viral (preferably retroviral) particle with a cellular receptor, followed by introduction of the nucleic acids contained by the particle into the cell. A commonly used vector for *ex vivo* delivery of the gene is a retrovirus.

The currently preferred *in vivo* nucleic acid transfer techniques include transfection with viral or non-viral vectors (such as adenovirus. lentivirus. Herpes simplex 1 virus, or adeno-associated virus (AAV)) and lipid-based systems (useful lipids for lipid-mediated transfer of the gene are, for example, DOTMA. DOPE, and DC-Chol; *see. e.g.,* Tonkinson et al., Cancer Investigation, 14(1): 54-65 (1996)). The most preferred vectors for use in gene therapy are viruses, most preferably adenoviruses, AAV, lentiviruses, or retroviruses. A viral vector such as a retroviral vector includes at least one transcriptional promoter/enhancer or locus-defining element(s), or other elements that control gene expression by other means such as alternate splicing, nuclear RNA export, or post-translational modification of messenger. In addition, a viral vector such as a retroviral vector includes a nucleic acid molecule that, when transcribed in the presence of a gene encoding PRO polypeptide, is operably linked thereto and acts as a translation initiation sequence. Such vector constructs also include a packaging signal, long terminal repeats (LTRs) or portions thereof, and positive and negative strand primer binding sites appropriate to the virus used (if these are not already present in the viral vector). In addition, such vector typically includes a signal sequence for secretion of the PRO polypeptide from a host cell in which it is placed. Preferably the signal sequence for this purpose is a mammalian signal sequence, most preferably the native signal sequence for the PRO polypeptide. Optionally, the vector construct may also include a signal that directs polyadenylation, as well as one or more restriction sites and a translation termination sequence. By way of example, such vectors will typically include a 5' LTR, a tRNA binding site, a packaging signal, an origin ofsecond-strand DNA synthesis, and a 3' LTR or a portion thereof. Other vectors can be used that are non-viral, such as cationic lipids, polylysine, and dendrimers.

In some situations, it is desirable to provide the nucleic acid source with an agent that targets the target cells, such as an antibody specific for a cell-surface membrane protein or the target cell, a ligand for a receptor on the target cell, etc. Where liposoines are employed, proteins that bind to a cell-surface membrane protein associated with endocytosis may be used for targeting and/or to facilitate uptake, *e.g.,* capsid proteins or fragments thereof tropic for a particular cell type, antibodies for proteins that undergo internalization in cycling, and proteins that target intracellular localization and enhance intracellular half-life. The technique of rcceptor-mediated endocytosis is described, for example, by Wu et al., J. Biol. Chem., 262: 4429-4432 (1987); and Wagner et al., Proc. Natl. Acad. Sci. USA, 87: 3410-3414 (1990). For a review of the currently known gene marking and gene therapy protocols, see, Anderson et al., Science. 256: 808-813 (1992). *See* also WO 93/25673 and the references cited therein.

Suitable gene therapy and methods for making retroviral particles and structural proteins can be found in, *e.g.,* U.S. Pat. No. 5,681,746.

vi. Use of Gene as Diagnostic

This invention is also related to the use of the gene encoding the PRO polypeptide as a diagnostic. Detection of a mutated form of the PKO polypeptide will allow a diagnosis of a cardiovascular, endothelial, and angiogenic disease or susceptibility to a cardiovascular, endothelial, and angiogenic disease, such as a tumor, since mutations in the PRO polypeptide may cause tumors.

Individuals carrying mutations in the genes encoding a human PRO polypeptide may be detected at the DNA level by a variety or techniques. Nucleic acids for diagnosis may be obtained from a patient's cells, such as from blood, urine, saliva, tissue biopsy, and autopsy material. The genomic DNA may be used directly for detection or may be amplified enzymatically by using PCR (Saiki et al., Nature, 324: 163-166 (1986)) prior to analysis. RNA or cDNA may also be used for the same purpose. As an example, PCR primers complementary to the nucleic acid encoding the PRO polypeptide can be used to identify and analyze PRO polypeptide mutations. For example, deletions and insertions can be detected by a change in size of the amplified product in comparison to the normal genotype. Point mutations can be identified by hybridizing amplified DNA to radiolabeled RNA encoding the PRO polypeptide, or alternatively, radiolabeled antisense DNA sequences encoding the PRO polypeptide. Perfectly matched sequences can be distinguished from mismatched duplexes by RNase A digestion or by differences in melting temperatures.

Genetic testing based on DNA sequence differences may be achieved by detection of alteration in electrophoretic mobility of DNA fragments in gels with or without denaturing agents. Small sequence deletions and insertions can be visualized by high resolution gel electrophoresis. DNA fragments of different sequences may be distinguished on denaturing formamidine gradient gels in which the mobilities of different DNA fragments are retarded in the gel at different positions according to their specific melting or partial melting temperatures. *See, e.g.,* Myers et al., Science, 230: 1242 (1985).

Sequence changes at specific locations may also be revealed by nuclease protection assays, such as RNase and S l protection or the chemical cleavage method, for example, Cotton et al., Proc. Natl. Acad. Sci. USA, 85: 4397-4401 (1985).

Thus, the detection of a specific DNA sequence may be achieved by methods such as hybridization, RNase protection, chemical cleavage, direct DNA sequencing, or the use of restriction enzymes, *e.g.,* restriction fragment length polymorphisms (RFLP), and Southern blotting of genomic DNA.

vii. Use to Detect PRO Polypeptide Levels

In addition to more conventional gel-electrophoresis and DNA sequencing, mutations can also be detected by *in situ* analysis.

Expression of nucleic acid encoding the PRO polypeptide may be linked to vascular disease or neovascularization associated with tumor formation. If the PRO polypeptide has a signal sequence and the mRNA is highly expressed in endothelial cells and to a lesser extent in smooth muscle cells, this indicates that the PRO polypeptide is present in serum. Accordingly, an anti-PRO polypeptide antibody could be used to diagnose vascular disease or neovascularization associated with tumor formation, since an altered level of this PRO polypeptide may be indicative of such disorders.

A competition assay may be employed wherein antibodies specific to the PRO polypeptide are attached to a solid support and the labeled PRO polypeptide and a sample derived from the host are passed over the solid support and the amount of label detected attached to the solid support can be correlated to a quantity of PRO polypeptide in the sample.

viii. Chromosome Mapping

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis for the 3'- untranslated region is used to rapidly select primers that do not span more than one exon in the genomic DNA. thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include *in situ* hybridization, prescreening with labeled flow-sorted chromosomes, and preselection by hybridization to construct chromosome-specific cDNA libraries.

Fluorescence *in situ* hybridization (FISH) of a cDNA clone to a metaphase chromosomal spread can be used to provide a precise chromosomal location in one step. This technique can be used with cDNA as short as 500 or 600 bases; however, clones larger than 2,000 bp have a higher likelihood of binding to a unique chromosomal location with sufficient signal intensity for simple detection. FISH requires use of the clones from which the gene encoding the PRO polypeptide was derived, and the longer the better. For example, 2,000 bp is good. 4,000 bp is better, and more than 4,000 is probably not necessary to get good results a reasonable percentage of the time. For a review of this technique, *see.* Verma et al., Human Chromosomes: a Manual of Basic Techniques (Pergamon Press, New York, 1988).

Once a sequence has been mapped to a precise chromosomal location, the physical position of the sequence on the chromosome can be correlated with genetic map data. Such data are found, for example, in V McKusick, Mendelian Inheritance in Man (available online through Johns Hopkins University Welch Medical Library). The relationship between genes and diseases that have been mapped to the same chromosomal region is then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes I megabase mapping resolution and one gene per 20 kb).

ix. Screening Assays for Drug Candidates

This invention encompasses methods of screening compounds to identify those that mimic the PRO polypeptide (agonists) or prevent the effect of the PRO polypeptide (antagonists). Screening assays for antagonist drug candidates are designed to identify compounds that bind or complex with the PRO polypeptide encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a PRO polypeptide encoded by a nucleic acid identified herein under conditions and for a time sufficient to allow these two components to interact.

In binding assays, the interaction is binding and the complex formed can be isolated or detected in the reaction mixture. In a particular embodiment, the PRO polypeptide encoded by the gene identified herein or the drug candidate is immobilized on a solid phase, *e.g.,* on a microtiter plate, by covalent or non-covalent attachments. Non-covalent attachment generally is accomplished by coating the solid surface with a solution of the PRO polypeptide and drying. Alternatively, an immobilized antibody, *e.g.*, a monoclonal antibody, specific for the PRO polypeptide to be immobilized can be used to anchor it to a solid surface. The assay is performed by adding the non-immobilized component, which may be labeled by a detectable label, to the immobilized component, *e.g.*, the coated surface containing the anchored component. When the reaction is complete, the non-reacted components are removed, *e.g.*, by washing, and complexes anchored on the solid surface are detected. When the originally non-immobilized component carries a detectable label, the detection of label immobilized on the surface indicates that complexing occurred. Where the originally non-immobilized component does not carry a label, cornplexing can be detected, for example, by using a labeled antibody specifically binding the immobilized complex.

If the candidate compound interacts with but does not bind to a particular PRO polypeptide encoded by a gene identified herein, its interaction with that polypeptide can be assayed by methods well known for detecting protein-protein interactions. Such assays include traditional approaches, such as, *e.g.,* cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340: 245-246 (1989); Chien et al., Proc. Natl. Acad. Sci. USA, 88: 9578-9582 (1991)) as disclosed by Chevray and Nathans, Proc. Natl. Acad. Sci. USA. 89: 5789-5793 (1991). Many transcriptiortal activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lacZ* reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a gene encoding a PRO polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

If the PRO polypeptide has the ability to stimulate the proliferation of endothelial cells in the presence of the co-mitogen ConA, then one example of a screening method takes advantage of this ability. Specifically, in the proliferation assay, human umbilical vein endothelial cells are obtained and cultured in 96-well flat-bottomed culture plates (Costar, Cambridge, MA) and supplemented with a reaction mixture appropriate for facilitating proliferation of the cells, the mixture containing Con-A (Calbiochem, La Jolla, CA). Con-A and the compound to be screened are added and after incubation at 37 °C, cultures are pulsed with $^{3-}$H-thymidine and harvested onto glass fiber filters(phD; Cambridge Technology, Watertown, MA). Mean $^{3-}$H-thymidine incorporation(cpm) of triplicate cultures is determined using a liquid scintillation counter (Beckman Instruments, Irvine, CA). Significant $^{3-}$(H) thymidine incorporation indicates stimulation of endothelial cell proliferation.

To assay for antagonists, the assay described above is performed: however, in this assay the PRO polypeptide is added along with the compound to be screened and the ability of the compound to inhibit $^{3-}$(H)-thymidine incorporation in the presence of the PRO polypeptide indicates that the compound is an antagonist to the PRO polypeptide. Alternatively, antagonists may be detected by combining the PRO polypeptide and a potential antagonist with membrane-bound PRO polypeptide receptors or recombinant receptors under appropriate conditions for a competitive inhibition assay. The PRO polypeptide can be labeled, such as by radioactivity, such that the number of PRO polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the PRO polypeptide and a cDNA library created from this RNA is divided into pools and used to transfect COS cells or other cells that are not responsive to the PRO polypeptide. Transfected cells that are grown on glass slides are exposed to the labeled PRO polypeptide. The PRO polypeptide can be labeled by a variety of means including iodination or inclusion of a recognition site for a site-specific protein kinase. Following fixation and incubation, the slides are subjected to autoradiographic analysis. Positive pools arc identified and subpools are prepared and re-transfected using an interactive sub-pooling and re-screening process, eventually yielding a single clone that encodes the putative receptor.

As an alternative approach for receptor identification, labeled PRO polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro-sequencing would be used to design a set of degenerate oligonucleotide probes to screen a cDNA library to identify the gene encoding the putative receptor.

In another assay for antagonists, mammalian cells or a membrane preparation expressing the receptor would be incubated with labeled PRO polypeptide in the presence of the candidate compound. The ability of the compound to enhance or block this interaction could then be measured.

The compositions useful in the treatment of cardiovascular, endothelial, and angiogenic disorders include, without limitation, antibodies, small organic and inorganic molecules, peptides, phosphopeptides, antisense and ribozyme molecules, triple-helix molecules, etc., that inhibit the expression and/or activity of the target gene product.

More specific examples of potential antagonists include an oligonucleotide that binds to the fusions of immunoglobulin with a PRO polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the PRO polypeptide that recognizes the receptor but imparts no effect, thereby competitively inhibiting the action of the PRO polypeptide.

Another potential PRO polypeptide antagonist or agonist is an antisense RNA or DNA construct prepared using antisense technology, where, *e.g.,* an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature PRO polypeptides herein, is used to design an antisense RNA oligonucleotide of from about 10 to 40 base pairs in length. A DNA oligonucleotide is designed to be complementary to a region of the gene involved in transcription (triple helix - *see,* Lee et al., Nucl. Acids Res., 6:3073 (1979); Cooney et al., Science, 241: 456 (1988); Dervan et al., Science, 251:1360 (1991)), thereby preventing transcription and the production of the PRO polypeptide. The antisense RNA oligonucleotide hybridizes to the mRNA *in vivo* and blocks translation of the mRNA molecule into the PRO polypeptide (antisense - Okano. Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the PRO polypeptide. When antisense DNA is used, oligodeoxyribonucleotides derived from the translation-initiation site, *e.g.,* between about -10 and +10 positions of the target gene nucleotide sequence, are preferred.

Antisense RNA or DNA molecules are generally at least about 5 bases in length, about 10 bases in length, about 15 bases in length, about 20 bases in length, about 25 bases in length, about 30 bases in length, about 35 bases in length, about 40 bases in length, about 45 bases in length, about 50 bases in length, about 55 bases in length, about 60 bases in length, about 65 bases in length, about 70 bases in length, about 75 bases in length, about 80 bases in length, about 85 bases in length, about 90 bases in length, about 95 bases in length, about 100 bases in length, or more.

Potential antagonists include small molecules that bind to the active site, the receptor binding site, or growth factor or other relevant binding site of the PRO polypeptide, thereby blocking the normal biological activity of the PRO polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details *see, e.g.,* Rossi, Current Biology, 4: 469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details *see, e.g.,* PCT publication No. WO 97/33551, *supra.*

These small molecules can be identified by any one or more of the screening assays discussed hereinabove and/or by any other screening techniques well known for those skilled in the art.

x. <u>Types of Cardiovascular. Endothelial, and Angiogenic Disorders to be Treated</u>

The PRO polypeptides, or agonists or antagonists thereto, that have activity in the cardiovascular, angiogenic, and endothelial assays described herein, and/or whose gene product has been found to be localized to the cardiovascular system, are likely to have therapeutic uses in a variety of cardiovascular, endothelial, and angiogenic disorders, including systemic disorders that affect vessels, such as diabetes mellitus. Their therapeutic utility could include diseases of the arteries, capillaries, veins, and/or lymphatics. Examples of treatments hereunder include treating muscle wasting disease, treating osteoporosis, aiding in implant fixation to stimulate the growth of cells around the implant and therefore facilitate its attachment to its intended site, increasing IGF stability in tissues or in serum, if applicable, and increasing binding to the IGF receptor (since IGF has been shown *in vitro* to enhance human marrow erythroid and granulocytic progenitor cell growth).

The PRO polypeptides or agonists or antagonists thereto may also be employed to stimulate erythropoiesis or granulopoiesis, to stimulate wound healing or tissue regeneration and associated therapies concerned with regrowth of tissue, such as connective tissue, skin, bone, cartilage, muscle, lung, or kidney, to promote angiogenesis, to stimulate or inhibit migration of endothelial cells, and to proliferate the growth of vascular smooth muscle and endothelial cell production. The increase in angiogenesis mediated by the PRO polypeptide or antagonist would be beneficial to ischemic tissues and to collateral coronary development in the heart subsequent to coronary stenosis. Antagonists are used to inhibit the action of such polypeptides, for example, to limit the production of excess connective tissue during wound healing or pulmonary fibrosis if the PRO polypeptide promotes such production. This would include treatment of acute myocardial infarction and heart failure.

Moreover, the present invention concerns the treatment of cardiac hypertrophy, regardless of the underlying cause, by administering a therapeutically effective dose of the PRO polypeptide, or agonist or antagonist thereto. If the objective is the treatment of human patients, the PRO polypeptide preferably is recombinant human PRO polypeptide (rhPRO polypeptide). The treatment for cardiac hypertrophy can be performed at any of its various stages, which may result from a variety of diverse pathologic conditions, including myocardial infarction, hypertension, hypertrophic cardiomyopathy, and valvular regurgitation. The treatment extends to all stages of the progression of cardiac hypertrophy, with or without structural damage of the heart muscle, regardless of the underlying cardiac disorder.

The decision of whether to use the molecule itself or an agonist thereof for any particular indication, as opposed to an antagonist to the molecule, would depend mainly on whether the molecule herein promotes cardiovascularization, genesis of endothelial cells, orangiogenesis or inhibits these conditions. For example, if the molecule promotes angiogenesis, an antagonist thereof would be useful for treatment of disorders where it is desired to limit or prevent angiogenesis. Examples of such disorders include vascular tumors such as haemangioma, tumor angiogenesis, neovascularization in the retina, choroid, or comea, associated with diabetic retinopathy or premature infant retinopathy or macular degeneration and proliferative vitreoretinopathy, rheumatoid arthritis, Crohn's disease, atherosclerosis, ovarian hyperstimulation, psoriasis, endometriosis associated with neovascularization, restenosis subsequent to balloon angioplasty, scar tissue overproduction, for example, that seen in a keloid that forms after surgery, fibrosis after myocardial infarction, or fibrotic lesions associated with pulmonary fibrosis.

If, however, the molecule inhibits angiogenesis, it would be expected to be used directly for treatment of the above conditions.

On the other hand, if the molecule stimulates angiogenesis it would be used itself (or an agonist thereof) for indications where angiogenesis is desired such as peripheral vascular disease, hypertension, inflammatory vasculitides, Reynaud's disease and Reynaud's phenomenon, aneurysms, arterial restenosis, thrombophlebitis, lymphangitis, lymphedema, wound healing and tissue repair, ischemia reperfusion injury, angina, myocardial infarctions such as acute myocardial infarctions, chronic heart conditions, heart failure such as congestive heart failure, and osteoporosis.

If, however, the molecule inhibits angiogenesis, an antagonist thereof would be used for treatment of those conditions where angiogenesis is desired.

Specific types of diseases are described below, where the PRO polypeptide herein or antagonists thereof may serve as useful for vascular-related drug targeting or as therapeutic targets for the treatment or prevention of the disorders. Atherosclerosis is a disease characterized by accumulation of plaques of intimal thickening in arteries, due to accumulation of lipids, proliferation of smooth muscle cells, and formation of fibrous tissue within the arterial wall. The disease can affect large, medium, and small arteries in any organ. Changes in endothelial and vascular smooth muscle cell function are known to play an important role in modulating the accumulation and regression of these plaques.

Hypertension is characterized by raised vascular pressure in the systemic arterial, pulmonary arterial, or portal venous systems. Elevated pressure may result from or result in impaired endothelial function and/or vascular disease.

Inflammatory vasculitides include giant cell arteritis, Takayasu's arteritis, polyarteritis nodosa (including the microangiopathic form), Kawasaki's disease, microscopic polyangiitis, Wegener's granulomatosis, and a variety of infectious-related vascular disorders (including Henoch-Schonlein prupura). Altered endothelial cell function has been shown to be important in these diseases.

Reynaud's disease and Reynaud's phenomenon are characterized by intermittent abnormal impairment of the circulation through the extremities on exposure to cold. Altered endothelial cell function has been shown to be important in this disease.

Aneurysms arc saccular or fusiform dilatations of the arterial or venous tree that are associated with altered endothelial cell and/or vascular smooth muscle cells.

Arterial restenosis (restenosis of the arterial wall) may occur following angioplasty as a result of alteration in the function and proliferation of endothelial and vascular smooth muscle cells.

Thrombophlebitis and lymphangitis are inflammatory disorders of veins and lymphatics, respectively, that may result from, and/or in, altered endothelial cell function. Similarly, lymphedema is a condition involving impaired lymphatic vessels resulting from endothelial cell function.

The family of benign and malignant vascular tumors are characterized by abnormal proliferation and growth of cellular elements of the vascular system. For example, lymphangiomas are benign tumors of the lymphatic system that are congenital, often cystic, malformations of the lymphatics that usually occur in newborns. Cystic tumors tend to grow into the adjacent tissue. Cystic tumors usually occur in the cervical and axillary region. They can also occur in the soft tissue ofthe extremities. The main symptoms are dilated, sometimes reticular, structured lymphatics and lymphocysts surrounded by connective tissue. Lymphangiomas are assumed to be caused by improperly connected embryonic lymphatics or their deficiency. The result is impaired local lymph drainage. Griener et al., Lymphology, 4: 140-144 (1971).

Another use for the PRO polypeptides herein or antagonists thereto is in the prevention of tumor angiogenesis, which involves vascularization of a tumor to enable it to growth and/or metastasize. This process is dependent on the growth of new blood vessels. Examples of neoplasms and related conditions that involve tumor angiogenesis include breast carcinomas, lung carcinomas, gastric carcinomas, esophageal carcinomas, colorectal carcinomas, liver carcinomas, ovarian carcinomas, thecomas, arrhenoblastomas, cervical carcinomas, endometrial carcinoma, endometrial hyperplasia, endometriosis, fibrosarcomas, choriocarcinoma, head and neck cancer, nasopharyngeal carcinoma, laryngeal carcinomas, hepatoblastoma, Kaposi's sarcoma, melanoma, skin carcinomas, hemangioma, cavernous hemangioma, hemangioblastoma, pancreas carcinomas, retinoblastoma, astrocytoma, glioblastoma, Schwannoma, oligodendroglioma, medulloblastoma, neuroblastomas, rhabdomyosarcoma, osteogenic sarcoma, leiomyosarcomas, urinary tract carcinomas, thyroid carcinomas, Wilm's tumor, renal cell carcinoma, prostate carcinoma, abnormal vascular proliferation associated with phakomatoses, edema (such as that associated with brain tumors), and Meigs' syndrome.

Age-related macular degeneration (AMD) is a leading cause of severe visual loss in the elderly population. The exudative form of AMD is characterized by choroidal neovascularization and retinal pigment epithelial cell detachment. Because choroidal neovascularization is associated with a dramatic worsening in prognosis, the PRO polypeptides or antagonist thereto is expected to be useful in reducing the severity of AMD.

Healing of trauma such as wound healing and tissue repair is also a targeted use for the PRO polypeptides herein or their antagonists. Formation and regression of new blood vessels is essential for tissue healing and repair. This category includes bone, cartilage, tendon, ligament, and/or nerve tissue growth or regeneration, as well as wound healing and tissue repair and replacement, and in the treatment of burns, incisions, and ulcers. A PRO polypeptide or antagonist thereof that induces cartilage and/or bone growth in circumstances where bone is not normally formed has application in the healing of bone fractures and cartilage damage or defects in humans and other animals. Such a preparation employing a PRO polypeptide or antagonist thereof may have prophylactic use in closed as well as open fracture reduction and also in the improved fixation of artificial joints. *De novo* bone formation induced by an osteogenic agent contributes to the repair of congenital, trauma-induced, or oncologic, resection-induced craniofacial defects, and also is useful in cosmetic plastic surgery.

PRO polypeptides or antagonists thereto may also be useful to promote better or faster closure of non-healing wounds, including without limitation pressure ulcers, ulcers associated with vascular insufficiency, surgical and traumatic wounds, and the like.

It is expected that a PRO polypeptide or antagonist thereto may also exhibit activity for generation or regeneration of other tissues, such as organs (including, for example, pancreas, liver, intestine, kidney, skin, or endothelium), muscle (smooth, skeletal, or cardiac), and vascular (including vascular endothelium) tissue, or for promoting the growth of cells comprising such tissues. Part of the desired effects may be by inhibition or modulation of fibrotic scarring to allow normal tissue to regenerate.

A PRO polypeptide herein or antagonist thereto may also be useful for gut protection or regeneration and treatment of lung or liver fibrosis, reperfusion injury in various tissues, and conditions resulting from systemic cytokine damage. Also, the PRO polypeptide or antagonist thereto may be useful for promoting or inhibiting differentiation of tissues described above from precursor tissues or cells, or for inhibiting the growth of tissues described above.

A PRO polypeptide or antagonist thereto may also be used in the treatment of periodontal diseases and in other tooth-repair processes. Such agents may provide an environment to attract bone forming cells stimulate growth of bone-forming cells, or induce differentiation of progenitors of bone-forming cells. A PRO polypeptide herein or an antagonist thereto may also be useful in the treatment of osteoporosis or osteoarthritis, such as through stimulation of hone and or cartilage repair or by blocking inflammation or processes of tissue destruction (collagenase activity, osteoclast activity, etc.) mediated by inflammatory processes, since blood vessels play an important role in the regulation of bone turnover and growth.

Another category of tissue regeneration activity that may be attributable to the PRO polypeptide herein or antagonist thereto is tendon/ligament formation. A protein that induces tendon/ligament-like tissue or other tissue formation in circumstances where such tissue is not normally formed has application in the healing of tendon or ligament tears, deformities, and other tendon or ligament defects in humans and other animals. Such a preparation may have prophylactic use in preventing damage to tendon or ligament tissue, as well as use in the improved fixation of tendon or ligament to bone or other tissues, and in repairing defects to tendon or ligament tissue. De *novo* tendon/ligament-like tissue formation induced by a composition of the PRO polypeptide herein or antagonist thereto contributes to the repair of congenital, trauma-induced, or other tendon or ligament defects of other origin, and is also useful in cosmetic plastic surgery for attachment or repair of tendons or ligaments. The compositions herein may provide an environment to attract tendon- or ligament-forming cells, stimulate growth of tendon- or ligament-forming cells, induce differentiation of progenitors oftendon- or ligament-forming cells, or induce growth of tendon/ligament cells or progenitors *ex vivo* for return *in vivo* to effect tissue repair. The compositions herein may also be useful in the treatment of tendinitis, carpal tunnel syndrome, and other tendon or ligament defects. The compositions may also include an appropriate matrix and/or sequestering agent as a carrier as is well known in the art.

The PRO polypeptide or its antagonist may also be useful for proliferation of neural cells and for regeneration of nerve and brain tissue, *i.e.,* for the treatment of central and peripheral nervous system disease and neuropathies, as well as mechanical and traumatic disorders, that involve degeneration, death, or trauma to neural cells or nerve tissue. More specifically, a PRO polypeptide or its antagonist may be used in the treatment of diseases of the peripheral nervous system, such as peripheral nerve injuries, peripheral neuropathy and localized neuropathies, and central nervous system diseases, such as Alzheimer's, Parkinson's disease, Huntington's disease, amyotroph iclateral sclerosis, and Shy-Drager syndrome. Further conditions that may be treated in accordance with the present invention include mechanical and traumatic disorders, such as spinal cord disorders, head trauma, and cerebrovascular diseases such as stroke. Peripheral neuropathies resulting from chemotherapy or other medical therapies may also be treatable using a PRO polypeptide herein or antagonist thereto.

Ischemia-reperfusion injury is another indication. Endothelial cell dysfunction may be important in both the initiation of, and in regulation of the sequelae of events that occur following ischemia-reperfusion injury.

Rheumatoid arthritis is a further indication. Blood vessel growth and targeting of inflammatory cells through the vasculature is an important component in the pathogenesis of rheumatoid and sero-negative forms of arthritis.

PRO polypeptide or its antagonist may also he administered prophylactically to patients with cardiac hypertrophy, to prevent the progression of the condition, and avoid sudden death, including death of asymptomatic patients. Such preventative therapy is particularly warranted in the case of patients diagnosed with massive left ventricular cardiac hypertrophy (a maximal wall thickness of 35 mm or more in adults, or a comparable value in children), or in instances when the hemodynamic burden on the heart is particularly strong.

PRO polypeptide or its antagonist may also he useful in the management of atrial fibrillation, which develops in a substantial portion of patients diagnosed with hypertrophic cardiomyopathy.

Further indications include angina, myocardial infarctions such as acute myocardial infarctions, and heart failure such as congestive heart failure. Additional non-neoplastic conditions include psoriasis, diabetic and other proliferative retinopathies including retinopathy of prematurity, retrolental fibroplasia, neovascular glaucoma, thyroid hyperplasias (including Grave's disease), corneal and other tissue transplantation, chronic inflammation, lung inflammation, nephrotic syndrome, preeclampsia, ascites, pericardial effusion (such as that associated with pericarditis), and pleural effusion.

In view of the above, the PRO polypeptides or agonists or antagonists thereof described herein, which are shown to alter or impact endothelial cell function, proliferation, and/or form, are likely to play an important role in the etiology and pathogenesis of many or all of the disorders noted above, and as such can serve as therapeutic targets to augment or inhibit these processes or for vascular-related drug targeting in these disorders.

xi. <u>Administration Protocols, Schedules, Doses, and Formulations</u>

The molecules herein and agonists and antagonists thereto are pharmaceutically useful as a prophylactic and therapeutic agent for various disorders and diseases as set forth above.

Therapeutic compositions of the PRO polypeptides or agonists or antagonists are prepared for storage by mixing the desired molecule having the appropriate degree of purity with optional pharmaceutically acceptable carriers, excipients, or stabilizers (Remington's Pharmaceutical Sciences, 16th edition, Osol, A. ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such asoctadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides: proteins, such as serum albumin, gelatin, or immunoglobulins; hydrophilic polymers such as polyvinylpyrrolidone; amino acids such as glycine, glutamine, asparagine, histidine, arginine, or lysine: monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.,* Zn-protein complexes): and/or non-ionic surfactants such as TWEEN™, PLURONICS™ or polyethylene glycol (PEG).

Additional examples of such carriers include ion exchangers, alurnina, aluminum stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protaminc sulfate, disodium hydrogen phosphate, potassium hydrogen phosphate, sodium chloride, zine salts, colloidal silica, magnesium trisilicate, polyvinyl pyrrolidone, cellulose-based substances, and polyethylene glycol Carriers for topical or gel-based forms of antagonist include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. For all administrations, conventional depot forms are suitably used. Such forms include, for example, microcapsules, nano-capsules, liposomes, plasters, inhalation forms, nose sprays, sublingual tablets, and sustained-release preparations. The PRO polypeptides or agonists or antagonists will typically be formulated in such vehicles at a concentration of about 0 1 mg/ml to 100 mg/ml.

Another formulation comprises incorporating a PRO polypeptide or antagonist thereof into formed articles. Such articles can be used in modulating endothelial cell growth and angiogenesis. In addition, tumor invasion and metastasis may be modulated with these articles.

The PRO polypeptide or antagonist to be used for *in vivo* administration must be sterile. This is readily accomplished by filtration through sterile filtration membranes, prior to or following lyophilization and reconstitution. The PRO polypeptide ordinarily will be stored in lyophilized form or in solution if administered systemically. If in lyophilized form, the PRO polypeptide or antagonist thereto is typically formulated in combination with other ingredients for reconstitution with an appropriate diluent at the time for use. An example of a liquid formulation of the PRO polypeptide or antagonist is a sterile, clear, colorless unprescrved solution filled in a single-dose vial for subcutaneous injection. Preserved pharmaceutical compositions suitable for repeated use may contain, for example, depending mainly on the indication and type of polypeptide:

a) a PRO polypeptide or agonist or antagonist thereto;
b) a buffer capable of maintaining the pH in a range of maximum stability of the polypeptide or other molecule in solution, preferably about 4-8;
c) a detergent/surfactant primarily to stabilize the polypeptide or molecule against agitation-induced aggregation;
d) an isotonifier;
e) a preservative selected from the group of phenol, benzyl alcohol and a benzethonium halide, *e.g.*, chloride; and
f) water,

If the detergent employed is non-ionic, it may, for example, be polysorbates (*e.g.*, POLYSORBATE™ (TWEEN™) 20, 80, etc.) or poloxamers (*e.g.*, POLOXAMER™ 188). The use of non-ionic surfactants Permits the formulation to be exposed to shear surface stresses without causing denaturation of the polypeptide. Further, such surfactant-containing formulations may be employed in aerosol devices such as those used in a pulmonary dosing, and needleless jet injector guns (*see, e.g.,* EP 257,956).

An isotonifier may be present to ensure isotonicity of a liquid composition of the PRO polypeptide or antagonist thereto, and includes polyhydric sugar alcohols, preferably trihydric or higher sugar alcohols, such as glycerin, erythritol, arabitol, xylitol, sorbitol, and mannitol. These sugar alcohols can be used alone or in combination. Alternatively, sodium chloride or other appropriate inorganic salts may be used to render the solutions isotonic.

The buffer may, for example, be an acetate, citrate, succinate, or phosphate buffer depending on the pH desired. The pH of one type of liquid formulation of this invention is buffered in the range of about 4 to 8, preferably about physinlugical pH.

The preservatives phenol, benzyl alcohol and benzethonium halides, *e.g.,* chloride, are known antirnicrobial agents that may be employed.

Therapeutic PRO polypeptide compositions generally arc placed into a container having a sterile access port, for example, an intravenous solution bag or vial having a stopper pierceable by a hypodermic injection needle. The formulations are preferably administered as repeated intravenous (i.v.), subcutaneous (s.c.), or intramuscular(i.m.) injections, oras aerosol formulations suitable for intranasal or intrapulmonary delivery (for intrapulmonary delivery *see, e.g.,* EP 257,956).

PRO polypeptides can also be administered in the form of sustained-released preparations. Suitable examples of sustained-release preparations include semipermeable matrices of solid hydrophobic polymers containing the protein, which matrices are in the form of shaped articles, *e.g.*, films, or microcapsules. Examples of sustained-release matrices include polyesters, hydrogels (*e.g.,* poly(2-hydroxyethyl-methacrylate) as described by Langer et al., J. Biomed. Mater. Res., 15: 167-277 (1981) and Langer, Chem. Tech., 12:98-105(1982) or poly(vinylalcohol)), polylactides (U.S. Patent No. 3,773,919, EP 58,481), copolymers of L-glutamic acid and gamma ethyl-L-glutamate (Sidman el al., Biopolymers, 22: 547-556 (1983)), non-degradable ethylene-vinyl acetate (Langer *et al., supra*), degradable lactic acid-glycolic acid copolymers such as the Lupron Depot™ (injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, certain hydrogels release proteins for shorter time periods. When encapsulated proteins remain in the body for a long time, they may denature or aggregate as a result of exposure to moisture at 37°C, resulting in a loss of biological activity and possible changes in immunogenicity. Rational strategies can be devised for protein stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Sustained-release PRO polypeptide compositions also include liposomally entrapped PRO polypeptides. Liposomes containing the PRO polypeptide are prepared by methods known *per se:* DE 3,218.121; Epstein et al., Proc. Natl. Acad. Sci. USA. 82: 3688-3692 (1985): Hwang et al., Proc. Natl. Acad. Sci. USA, 77: 4030-4034 (1980); EP 52,322; EP 36.676: EP 88,046; EP 143,949; EP 142,641; Japanese patent application 83-118008; U.S. Patent Nos. 4,485.045 and 4,544.545; and EP 102,324. Ordinarily the liposomes are of the small (about 200-800 angstroms) unilamellar type in which the lipid content is greater than about 30 mol. % cholesterol, the selected proportion being adjusted for the optimal therapy.

The therapeutically effective dose of a PRO polypeptide or antagonist thereto will, of course, vary depending on such factors as the pathological condition to be treated (including prevention), the method of administration, the type of compound being used for treatment, any co-therapy involved, the patient's age, weight, general medical condition, medical history, etc., and its determination is well within the skill of a practicing physician. Accordingly, it will be necessary for the therapist to titer the dosage and modify the route of administration as required to obtain the maximal therapeutic effect. If the PRO polypeptide has a narrow host range, for the treatment of human patients formulations comprising the human PRO polypeptide, more preferably the native-sequence human PRO polypeptide, are preferred. The clinician will administer the PRO polypeptide until a dosage is reached that achieves the desired effect for treatment of the condition in question. For example, if the objective is the treatment of CHF, the amount would be one that inhibits the progressive cardiac hypertrophy associated with this condition. The progress of this therapy is easily monitored by echo cardiography. Similarly, in patients with hypertrophic cardiomyopathy, the PRO polypeptide can be administered on an empirical basis.

With the above guidelines, the effective dose generally is within the range of from about 0.001 to about 1.0 mg/kg, more preferably about 0.01-1.0 mg/kg, most preferably about 0.01-0.1 mg/kg.

For non-oral use in treating human adult hypertension, it is advantageous to administer the PRO polypeptide in the form of an injection at about 0.01 to 50 mg, preferably about 0.05 to 20 mg, most preferably 1 to 20 mg, per kg body weight, 1 to 3 times daily by intravenous injection. For oral administration, a molecule based on the PRO polypeptide is preferably administered at about 5 mg to 1 g, preferably about 10 to 100 mg, per kg body weight, 1 to 3 times daily. It should be appreciated that endotoxin contamination should be kept minimally at a safe level, for example, less than 0.5 ng/mg protein. Moreover, for human administration, the formulations preferably meet sterility, pyrogenicity, general safety, and purity as required by FDA Office and Biologics standards.

The dosage regimen of a pharmaceutical composition containing a PRO polypeptide to be used in tissue regeneration will be determined by the attending physician considering various factors that modify the action of the polypeptides, *e.g.,* amount of tissue weight desired to be formed, the site of damage, the condition of the damaged tissue, the size of a wound, type of damaged tissue (*e.g.,* bone), the patient's age, sex, and diet, the severity of any infection, time of administration, and other clinical factors. The dosage may vary with the type of matrix used in the reconstitution and with inclusion of other proteins in the pharmaceutical composition. For example, the addition of other known growth factors, such as IGF-I, to the final composition may also affect the dosage. Progress can be monitored by periodic assessment of tissue/bone growth and/or repair, for example, X-rays, histomorphometric determinations, and tetracycline labeling.

The route of PRO polypeptide or antagonist or agonist administration is in accord with known methods, *e.g.,* by injection or infusion by intravenous, intramuscular, intracerebral, intraperitoneal, intracerobrospinal, subcutaneous, intraocular, intraanicular, intrasynovial, intrathecal, oral, topical, or inhalation routes, or by sustained-release systems as noted below. The PRO polypeptide or antagonists thereof also are suitably administered by intratumoral, peritumoral, intralesional, or perilesional routes, to exert local as well as systemic therapeutic effects. The intraperitoneal route is expected to be particularly useful, for example, in the treatment of ovarian tumors.

If a peptide or small molecule is employed as an antagonist or agonist, it is preferably administered orally or non-orally in the form of a liquid or solid to mammals.

Examples of pharmacologically acceptable salts of molecules that form salts and are useful hereunder include alkali metal salts (*e.g.,* sodium salt, potassium salt), alkaline earth metal salts (*e.g.,* calcium salt, magnesium salt), ammonium salts, organic base salts (*e.g.,* pyridine salt, triethylamine salt), inorganic acid salts (*e.g.,* hydrochloride, sulfate, nitrate), and salts of organic acid (*e.g.,* acetate, oxalate, p-toluenesulfonate).

For compositions herein that are useful for bone, cartilage, tendon, or ligament regeneration, the therapeutic method includes administering the composition topically, systemically, or locally as an implant or device. When administered, the therapeutic composition for use is in a pyrogen-free, physiologically acceptable form. Further, the composition may desirably be encapsulated or injected in a viscous form for delivery to the site of bone, cartilage, or tissue damage. Topical administration may be suitable for wound healing and tissue repair. Preferably, for bone and/or cartilage formation, the composition would include a matrix capable of delivering the protein-containing composition to the site of bone and/or cartilage damage, providing a structure for the developing bone and cartilage and preferably capable of being resorbed into the body. Such matrices may be formed of materials presently in use for other implanted medical applications.

The choice ofmatrix material is based on biocompatibility, biodegradability, mechanical properties, cosmetic appearance, and interface properties. The particular application of the compositions will define the appropriate formulation. Potential matrices for the compositions may be biodegradable and chemically defined calcium sulfate, tricalcium phosphate, hydroxyapatite, polylactic acid, polyglycolic acid, and polyanhydrides. Other potential materials are biodegradable and biologically well-defined, such as bone or dermal collagen. Further matrices are comprised of pure proteins or extracellular matrix components. Other potential matrices are nonbiodegradable and chemically defined, such as sintered hydroxyapatite, bioglass, aluminates, or other ceramics. Matrices may be comprised of combinations of any of the above-mentioned types of material, such as polylactic acid and hydroxyapatite or collagen and tricalcium phosphate. The bioceramics may be altered in composition, such as in calcium-aluminate-phosphate and processing to alter pore size, particle size, particle shape, and biodegradability.

One specific embodiment is a 50:50 (mole weight) copolymer of lactic acid and glycolic acid in the form of porous particles having diameters ranging from 150 to 800 microns. In some applications, it will be useful to utilize a sequestering agent, such as carboxymethyl cellulose or autologous blood clot, to prevent the polypeptide compositions from disassociating from the matrix.

One suitable family of sequestering agents is cellulosic materials such as alkylcelluloses (including hydroxyalkylcelluloses).includingmethylcellulose,ethylcellulose, hydoxyethylcellulose, hydroxypropylcellulose, hydroxypropylmethylcellulose, and carboxymethylcellulose, one preferred being cationic salts of carboxymethylcellulose (CMC). Other preferred sequestering agents include hyaluronic acid, sodium alginate, poly(ethylene glycol), polyoxyethylene oxide, carboxyvinyl polymer, and poly(vinyl alcohol). The amount of sequestering agent useful herein is 0.5-20 wt%, preferably 1-10 wt%, based on total formulation weight, which represents the amount necessary to prevent desorption of the polypeptide (or its antagonist) from the polymer matrix and to provide appropriate handling of the composition, yet not so much that the progenitor cells are prevented from infiltrating the matrix, thereby providing the polypeptide (or its antagonist) the opportunity to assist the osteogenic activity of the progenitor cells.

xii. Combination Therapies

The effectiveness of the PRO polypeptide or an agonist or antagonist thereof in preventing or treating the disorder in question mav be improved by administering the active agent serially or in combination with another agent that is effective for those purposes, either in the same composition or as separate compositions.

For example, for treatment of cardiac hypertrophy, PRO polypeptide therapy can be combined with the administration of inhibitors of known cardiac myocyte hypertrophy factors. *e.g.,* inhibitors of α-adrenergic agonists such as phenylephrine, endothelin-1 inhibitors such as BOSENTAN™ and MOXONODIN™; inhibitors to CT-1 (US Pat. No. 5,679,545); inhibitors to LIF; ACE inhibitors; des-aspartate-angiotensin I inhibitors (U.S. Pat. No. 5,773,415), and angiotensin II inhibitors.

For treatment of cardiac hypertrophy associated with hypertension, a PRO polypeptide can be administered in combination with β-adrenergic receptor blocking agents, *e.g.*, propranolol, timolol, tertalolol, carteolol, nadolol, betaxolol, penbutolol, acetobutolol, atenolol, metoprolol, or carvedilol; ACE inhibitors, *e.g.*, quinapril, captopril, enalapril, ramipril, benazepril, fosinopril, or lisinopril; diuretics, *e.g.*, chlorothiazide, hydrochlorothiazide, hydroflumethazide, methylchlothiazide, benzthiazide, dichlorphenamide, acetazolamide, or indapamide; and/or calcium channel blockers, *e.g.*, diltiazem, nifedipine, verapamil, or nicardipine. Pharmaceutical compositions comprising the therapeutic agents identified herein by their generic names are commercially available, and are to be administered following the manufacturers' instructions for dosage, administration, adverse effects, contraindications, etc. *See, e.g.,* Physicians' Desk Reference (Medical Economics Data Production Co.: Montvale, N.J., 1997), 51th Edition.

Preferred candidates for combination therapy in the treatment of hypertrophic cardiomyopathy are β-adrenergic-blocking drugs (*e.g.*. propranolol, timolol, tertalolol, carteolol, nadolol, betaxolol, penbutolol, acetobutolol, atenolol, metoprolol, or carvedilol), verapamil, difedipine, or diltiazem. Treatment of hypertrophy associated with high blood pressure may require the use of antihypertensive drug therapy, using calcium channel blockers, *e.g.,* diltiazem, nifedipine, verapamil, or nicardipine; β-adrenergic blocking agents; diuretics, *e.g.*, chlorothiazide, hydrochlorothiazide, hydroflumethazide, methylchlothiazide, benzthiazide, dichlorphenamide, acetazolamide, or indapamide; and/or ACE-inhibitors, *e.g.*, quinapril, captopril, enalapril, ramipril, benazepril, fosinopril, or lisinopril.

For other indications, PRO polypeptides or their antagonists may be combined with other agents beneficial to the treatment of the bone and/or cartilage defect, wound, or tissue in question. These agents include various growth factors such as EGF, PDGF, TGF-α or TGF-β, IGF, FGF, and CTGF.

In addition, PRO polypeptides or their antagonists used to treat cancer may be combined with cytotoxic, chemotherapeutic, or growth-inhibitory agents as identified above. Also, for cancer treatment, the PRO polypeptide or antagonist thereof is suitably administered serially or in combination with radiological treatments, whether involving irradiation or administration of radioactive substances.

The effective amounts of the therapeutic agents administered in combination with the PRO polypeptide or antagonist thereof will be at the physician's or veterinarian's discretion. Dosage administration and adjustment is done to achieve maximal management of the conditions to be treated For example, for treating hypertension, these amounts ideally take into account use of diuretics or digitalis, and conditions such as hyper- or hypotension, renal impairment, etc. The dose will additionally depend on such factors as the type of the therapeutic agent to be used and the specific patient being treated. Typically, the amount employed will be the same dose as that used, if the given therapeutic agent is administered without the PRO polypeptide.

xiii. Articles of Manufacture

An article of manufacture such as a kit containing a PRO polypeptide or antagonists thereof useful for the diagnosis or treatment of the disorders described above comprises at least a container and a label. Suitable containers include, for example, bottles, vials, syringes, and test tubes. The containers may be formed from a variety of materials such as glass or plastic. The container holds a composition that is effective for diagnosing or treating the condition and may have a sterile access port (for example, the container may be an intravenous solution bag or a vial having a stopper pierceable by a hypodermic injection needle). The active agent in the composition is the PRO polypeptide or an agonist or antagonist thereto. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use. The article of manufacture may also comprise a second or third container with another active agent as described above.

E. Antibodies

Some of the most promising drug candidates according to the present invention are antibodies and antibody fragments that may inhibit the production or the gene product of the genes identified herein and/or reduce the activity of the gene products.

i. Polyclonal Antibodies

Methods of preparing polyclonal antibodies arc known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may he useful to conjugate the immunizing agent to a protein known to be immunogenic in the mammal being immunized. Examples of such immunogenic proteins include, but are not limited to, keyhole limpet hemocyanin, serum albumin, bovine thyroglobulin, and soybean trypsin inhibitor. Examples of adjuvants that may be employed include Freund's complete adjuvant and MPL-TDM adjuvant (monophosphoryl Lipid A or synthetic trehalose dicorynomycolate). The immunization protocol may be selected by one skilled in the art without undue experimentation.

ii. Monoclonal Antibodies

The anti-PRO antibodies may, alternatively, be monoclonal antibodies. Monoclonal antibodies may be prepared using hybridoma methods, such as those described by Kohler and Milstein. Nature, 256:495 (1975). In a hybridoma method, a mouse, hamster, or other appropriate host animal is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Goding, Monoclonal Antibodies: Principles and Practice (New York: Academic Press, 1986), pp. 59-103. Immortalized cell lines are usually transformed mammalian cells, particularly myeloma cells ofrodent, bovine, and human origin. Usually, rat or mouse myeloma cell lines are employed. The hybridoma cells may be cultured in a suitable culture medium that preferably contains one or more substances that inhibit the growth or survival of the unfused, immortalized cells. For example. if the parental cells lack the enzyme hypoxanthine guanine phosphoribosyl transferase (HGPRT or HPRT). the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high-level expression of antibody by the selected antibody-producing cells, and are sensitive to a medium such as HAT medium. More preferred immortalized cell lines are murine myeloma lines, which can be obtained, for instance, from the Salk Institute Cell Distribution Center. San Diego, California and the American Type Culture Collection, Manassas, Virginia. Human myelomaand mouse-human heteromyeloma cell lines also have been described for the production of human monoclonal antibodies. Kozbor, J. Immunol., 133:3001 (1984); Brodeur et al., Monoclonal Antibody Production Techniques and Applications (Marcel Dekker, Inc.: New York, 1987) pp. 51-63.

The culture medium in which the hybridoma cells are cultured can then be assayed for the presence of monoclonal antibodies directed against the PRO polypeptide. Preferably, the binding specificity of monoclonal antibodies produced by the hybridoma cells is determined by immunoprecipitation or by an *in vitro* binding assay, such as radioimmunoassay(RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods. Coding, *supra.* Suitable culture media for this purpose include, for example, Dulbecco's Moditied Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purificationprocedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4.816,567. DNA encoding the monoclonal antibodies of the invention can be readily isolated and sequenced using conventional procedures (*e.g,* by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells. Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy- and light-chain constant domains in place of the homologous murine sequences (U.S. Patent No. 4,816,567; Morrison *et al., supra*) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are wel known in the art. For example, one method involves recombinant expression of immunoglobulin light chain and modified heavy chain. The heavy chain is truncated generally at any point in the Fc region so as to prevent heavy-chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly Fab fragments, can be accomplished using routine techniques known in the art.

iii. <u>Human and Humanized Antibodies</u>

The anti-PRO antibodies may further comprise humanized antibodies or human antibodies. Humanized forms of non-human (*e.g.,* murine) antibodies are chimeric immunoglobulins, immunoglobulin chains, or fragments thereof (such as Fv, Fab. Fab', $F(ab')_2$ or other antigen-binding subsequences of antibodies) that contain minimal sequence derived from non-human immunoglobulin. Humanized antibodies include human immunoglobulins (recipient antibody) in which residues from a CDR of the recipient are replaced by residues from a CDR of a non-human species (donor antibody) such as mouse, rat, or rabbit having the desired specificity, affinity, and capacity. In some instances, Fv framework residues of the human immunoglobulin are replaced by corresponding non-human residues. Humanized antibodies may also comprise residues that are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin, and all or substantially all of the FR regions are those of a human immunoglobulin consensus sequence. The humanized antibody preferably also will comprise at least a portion of an immunoglobulin constant region (Fc), typically that of a human immunoglobulin. Jones et al., Nature, 321: 522-525 (1986): Riechmann et al., Nature, 332:

323-329 (1988); Presta, Curr. Op Struct. Biol., 2:593-596 (1992).

Methods for humanizing non-human antibodies arc well known in the art. Generally, a humanized antibody has one or more amino acid residues introduced into it from a source that is non-human. These non-human amino acid residues are often referred to as "import" residues, which are typically taken from an "import" variable domain. Humanization can be essentially performed following the method of Winter and co-workers (Jones et al., Nature. 321: 522-525 (1986): Riechmann et al., Nature, 332: 323-327 (1988); Verhoeyen et al., Science, 239: 1534-1536 (1988)), by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries. Hoogenboom and Winter, J. Mol. Biol., 227: 381 (1991); Marks et al., J. Mol. Biol., 222: 581 (1991). The techniques of Cole *el al.* and Boemer *et al.* are also available for the preparation of human monoclonal antibodies. Cole el al., Monoclonal Antibodies and Cancer Therapy, Alan R. Liss, p. 77 (1985) and Boemer et al., J. Immunol., 147(1): 86-95 (1991). Similarly, human antibodies can be made by introducing human immunoglobulin loci intotransgenic animals, *e.g.*, mice in which the endogenous immunoglobulin genes have been partially or completely inactivated. Upon challenge, human antibody production is observed that closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; and 5,661,016, and in the followingscientific publications: Marks et al., Bio/Technology, 10: 779-783 (1992); Lonberg et al., Nature. 368:856-859 (1994): Morrison, Nature, 368: 812-813 (1994); Fishwild et al., Nature Biotechnology, 14: 845-851 (1996); Neuberger, Nature Biotechnology, 14: 826 (1996); Lonberg and Huszar, Intern. Rev. Immunol., 13: 65-93 (1995).

iv. Bispecific Antibodies

Bispecific antibodies are monoclonal, preferably human or humanized, antibodies that have binding specificities for at least two different antigens. In the present case, one of the binding specificities is for the PRO polypeptide, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities. Milstein and Cuello, Nature, 305: 537-539 (1983). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10: 3655-3659 (1991).

Antibody variable domains with the desired binding specificities (antibody-antigen combining sites) can be fused to immunoglobulin constant-domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecilic antibodies, *see,* for example, Suresh et al., Methods in Enzymology, 121 210 (1986).

v. Heteroconiugate Antibodies

Heteroconjugate antibodies are composed of two covalently joined antibodies. Such antibodies have, for example, been proposed to target immune-system cells to unwanted cells (U.S. Patent No. 4.676,980), and for treatment of HIV infection. WO 91/00360; WO 92/200373; EP 03089. It is contemplated that the antibodies may be prepared *in vitro* using known methods in synthetic protein chemistry, including those involving crosslinking agents. For example, immunotoxins may be constructed using a disulfide-exchange reaction or by forming a thioether bond. Examples of suitable reagents for this purpose include iminothiolate and methyl-4-mercaptobutyrimidate and those disclosed, for example, in U.S. Patent No. 4,676,980.

vi. Effector Function Engineering

It may be desirable to modify the antibody of the invention with respect to effector function, so as to enhance, *e.g.*, the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediatcd cell killing and antibody-dependent cellular cytotoxicity (ADCC). *See,* Caron et al., J. Exp. Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al., Cancer Research, 53: 2560-2565 (1993). Alternatively, an antibody can be engineered that has dual Fc regions and may thereby have enhanced complement lysis and ADCC capabilities. *See,* Stevenson et al., Anti-Cancer Drug Design, 3: 219-230 (1989).

vii. Immunoconjugates

The invention also pertains to immunoconjugates comprising an antibody conjugated to a cytotoxic agent such as a chemotherapeutic agent, toxin (*e.g.,* an enzymatically active toxin of bacterial, fungal, plant, or animal origin, or fragments thereof), or a radioactive isotope (*i.e.,* a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), moinordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}$Bi, $^{131}$I, $^{131}$In, $^{90}$Y, and $^{186}$Re.

Conjugates of the antibody and cytotoxic agent are made using a variety of bifunctional protein-coupling agents such as N-succinimidyl-3-(2-pyridyldithiol)propoionate (SPDP), iminothiolane (IT), bifunctional derivatives of imidoesters (such as dimethyl adipimidate HCl), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta er al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. *See.* WO94/11026.

In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (*e.g.,* avidin) that is conjugated to a cytotoxic agent (*e.g.,* a radionucleotide).

viii. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl. Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin ct al., J. Biol. Chem., 257: 286-288 (1982) via a disulfide-interchange reaction. A chemotherapeutic agent (such as Doxorubicin) is optionally contained within the liposome. *See,* Gabizon et al., J. National Cancer Inst., 81(19): 1484 (1989).

ix. Pharmaceutical Compositions of Antibodies

Antibodies specifically binding a PRO polypeptide identified herein, as well as other molecules identified by the screening assays disclosed hereinbefore, can be administered for the treatment of various disorders as noted above and below in the form of pharmaceutical compositions.

If the PRO polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. *See, e.g.,* Marasco et al., Proc. Nail. Acad. Sci. USA. 90: 7889-7893 (1993).

The formulation herein may also contain more than one active compound as necessary for the Particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, *supra.*

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g.*, films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and $\gamma$ ethyl-L-alutamate, non-degradable ethylene-vinyl acetate, degradable lactic acid-glycolic acid copolymers such as the LUPRON DEPOT ™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

x. Methods of Treatment using the Antibody

It is contemplated that the antibodies to a PRO polypeptide may be used to treat various cardiovascular, endothelial, and angiogenic conditions as noted above.

The antibodies are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration of the antibody is preferred.

Other therapeutic regimens may be combined with the adm inistration of the antibodies of the instant invention as noted above. For example, if the antibodies are to treat cancer, the patient to be treated with such antibodies may also receive radiation therapy Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service. Ed., M.C. Perry (Willianns & Wilkins: Baltimore. MD. 1992). The chemotherapeutic agent may precede, or follow administration of the antibody, or may be given simultaneously therewith. The antibody may be combined with an anti-estrogen compound such as tamoxifen or EVISTA™ or an anti-progesterone such as onapristone (see, EP 616812) in dosages known for such molecules.

If the antibodies are used for treating cancer, it may be desirable also to administer antibodies against other tumor-associated antigens, such as antibodies that bind to one or more of the ErbB2. EGFR, ErbB3, ErbB4, or VEGF receptor(s). These also include the agents set forth above. Also, the antibody is suitably administered serially or in combination with radiological treatments, whether involving irradiation or administration of radioactive substances. Alternatively, or in addition, two or more antibodies binding the same or two or more different antigens disclosed herein may be co-administered to the patient. Sometimes, it may be beneficial also to administer one or more cytokines to the patient. In a preferred embodiment, the antibodies herein are co-administered with a growth-inhibitory agent. For example, the growth-inhibitory agent may be administered first, followed by an antibody of the present invention. However, simultaneous administration or administration of the antibody of the present invention first is also contemplated. Suitable dosages for the growth-inhibitory agent are those presently used and may be lowered due to the combined action (synergy) of the growth-inhibitory agent and the antibody herein.

In one embodiment, vascularization of tumors is attacked in combination therapy. The anti-PRO polypeptide and anotherantibody (*e.g.*, anti-VEGF) are administered to tumor-bearing patients at therapeutically effective doses as determined, for example, by observing necrosis of the tumor or its metastatic foci, if any. This therapy is continued until such time as no further beneficial effect is observed or clinical examination shows no trace of the tumor or any metastatic foci. Then TNF is administered, alone or in combination with an auxiliary agent such as alpha-, beta-, orgamma-interferon, anti-HER2 antibody,heregulin, anti-heregulin antibody, D-factor, interleukin-I (IL-1), interleukin-2 (IL-2), granulocyte-macrophage colony stimulating factor (GM-CSF), or agents that promote microvascular coagulation in tumors, such as anti-protein C antibody, anti-protein S antibody, or C4b binding protein (*see.* WO 91/01753, published 21 February 1991), or heat or radiation.

Since the auxiliary agents will vary in their effectiveness, it is desirable to compare their impact on the tumor by matrix screening in conventional fashion. The administration of anti-PRO polypeptide antibody and TNF is repeated until the desired clinical effect is achieved. Alternatively, the anti-PRO polypeptide antibody is administered together with TNF and, optionally, auxiliary agent(s). In instances where solid tumors are found in the limbs or in other locations susceptible to isolation from the general circulation, the therapeutic agents described herein are administered to the isolated tumor or organ. In other embodiments, a FGF or PDGF antagonist, such as an anti-FGF or an anti-PDGF neutralizing antibody, is administered to the patient in conjunction with the anti-PRO polypeptide antibody. Treatment with anti-PRO polypeptide antibodies preferably may be suspended during periods of wound healing or desirable neovascularization.

For the prevention or treatment of cardiovascular, endothelial, and angiogenic disorder, the appropriate dosage of an antibody herein will depend on the type of disorder to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

For example, depending on the type and severity of the disorder, about 1 μg/kg to 50 mg/kg (*e.g.*, 0.1-20 mg/kg) of antibody is an initial candidate dosage for administration to the patient, whether, for example, by one or more separate administrations, or by continuous infusion. A typical daily or weekly dosage might range from about I μg/kg to 100 mg/kg or more, depending on the factors mentioned above. For repeated administrations over several days or longer, depending on the condition, the treatment is repeated or sustained until a desired suppression of disorder symptoms occurs. However, other dosage regimens may be useful. The progress of this therapy is easily monitored by conventional techniques and assays, including, for example, radiographic tumor imaging.

xi. Articles of Manufacture with Antibodies

An article of manufacture containing a container with the antibody and a label is also provided. Such articles are described above, wherein the active agent is an anti-PRO antibody.

xii. Diagnosis and Prognosis of Tumors using Antibodies

If the indication for which the antibodies are used is cancer, while cell-surface proteins, such as growth receptors over expressed in certain tumors, are excellent targets for drug candidates or tumor (*e.g.*, cancer) treatment, the same proteins along with PRO polypeptides find additional use in the diagnosis and prognosis of tumors. For example, antibodies directed against the PRO polypeptides may be used as tumor diagnostics or prognostics.

For example, antibodies, including antibody fragments, can be used qualitatively or quantitatively to detect the expression of genes including the gene encoding the PRO polypeptide. The antibody preferably is equipped with a detectable, *e.g.,* fluorescent label, and binding can be monitored by light microscopy, flow cytometry, fluorimetry, or other techniques known in the art Such binding assays are performed essentially as described above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent to those skilled in the art that a wide variety of histological methods are readily available for *in situ* detection.

The following Examples are offercd for illustrative purposes only, and are not intended to limit the scope of the present invention in any way.

The disclosures of all patent and literature references cited in the present specification are hereby incorporated by reference in their entirety.

## EXAMPLES

Commercially available reagents referred to in the Examples were used according to manufacturer's instructions unless otherwise indicated. The source of those cells identified in the following Examples, and throughout the specification, by ATCC accession numbers is the American Type Culture Collection, Manassas, VA. Unless otherwise noted, the present invention uses standard procedures of recombinant DNA technology, such as those described hereinabove and in the following textbooks: Sambrook *et al., supra;* Ausubel et al., Current Protocols in Molecular Biology (Green Publishing Associates and Wiley Interscience, N.Y., 1989); Innis et al., PCR Protocols: A Guide to Methods and Applications (Academic Press, Inc.: N.Y., 1990); Harlow et al., Antibodies: A Laboratory Manual (Cold Spring Harbor Press: Cold Spring Harbor, 1988); Gait, Oligonucleotide Synthesis (IRL Press: Oxford, 1984); Freshney, Animal Cell Culture, 1987; Coligan et al., Current Protocols in Immunology, 1991.

EXAMPLE 1

Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (*e.g.,* Dayhoff, GenBank), and proprietary databases (*e.g.* LIFESEQ®, Incyte Pharmaceuticals, Palo Alto, CA). The search was performed using the computer program BLAST or BLAST-2 (Altschul et al., Methods in Enzymology, 266:460-480 (1996)) as a comparison of the ECD protein sequences to a 6 frame translation of the EST sequences. Those comparisons with a BLAST score of 70 (or in some cases 90) or greater that did not encode known proteins were clustered and assembled into consensus DNA sequences with the program "phrap" (Phil Green. University of Washington, Seattle, WA).

Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100- 1000 bp in length. The probe sequences are typically 40-55 bp in length. In some cases, additional oligonucleotides are synthesized when the consensus sequence is greater than about 1-1.5 kbp. In order to screen several libraries for a full-length clone. DNA from the libraries was screened by PCR amplification, as per Ausubel *et al.,* Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI sites.

EXAMPLE 2

Isolation of cDNA clones by Amylase Screening

I. Preparation of oligo dT primed cDNA library

mRNA was isolated from a human tissue of interest using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

2. Preparation of random primed cDNA library

A secondary cDNA library was generated in order to preferentially represent the 5' ends of the primary cDNA clones. Sp6 RNA was generated from the primary library (described above), and this RNA was used to generate a random primed cDNA library in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, *e.g.,* CsCl-gradient. The purified DNA was then carried on to the yeast protocols below.

The yeast methods were divided into three categories (1) Transformation of yeast with the plasmid/cDNA combined vector: (2) Detection and isolation of yeast clones secreting amylase: and (3) PCR amplification of the insert directly from the yeast colony and purification of the DNA for sequencing and further analysis.

The yeast strain used was HD56-5A (ATCC-90785). This strain has the following genotype: MAT alpha, ura3-52, leu2-3, leu2-112, his3-11, his3-15. $MAL^-$, $SUC^+$, $GAL^-$. Preferably, yeast mutants can be employed that have deficient post-translational pathways. Such mutants may have translocation deficient alleles in *sec*71, *sec*72, *sec*62, with truncated *sec*71 being most preferred. Alternatively, antagonists (including antisense nucleotides and/or ligands) which interfere with the normal operation of these genes, other proteins implicated in this post translation pathway (*e.g.,* SEC61p, SEC72p, SEC62p, SEC63p, TDJ1p or SSA 1p-4p) or the complex formation of these proteins may also be preferably employed in combination with the amylase-expressing yeast.

Transformation was performed based on the protocol outlined by Gietz et al., Nucl. Acid. Res., 20:1425 (1992). Transformed cells were then inoculated from agar into YEPD complex media broth (100 ml) and grown overnight at 30°C. The YEPD broth was prepared as described in Kaiser er al., Methods in Yeast Genetics Cold Spring Harbor Press, Cold Spring Harbor, NY, p. 207 (1994). The overnight culture was then diluted to about 2 x $10^6$ cells/ml (approx. $OD_{600}$=0.1) into fresh YEPD broth (500 ml) and regrown to I x $10^7$ cells/ml (approx. $OD_{600}$=0.4-0.5).

The cells were then harvested and prepared for transformation by transfer into GS3 rotor bottles in a Sorval GS3 rotor at 5.000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCl, 1 mM EDTA pH 7.5, 100 mM $Li_2OOCCH_3$), and resuspended into LiAc/TE (2.5 ml).

Transformation took place by mixing the prepared cells (100 μl) with freshly denaturedsingle stranded salmon testes DNA (Lofstrand Labs, Gaithersburg, MD) and transforming DNA (1 μg. vol. < 10 μl) in microfuge tubes. The mixture was mixed briefly by vortexing, then 40% PEG/TE (600 μl, 40% polyethylene glycol-4000, 10 mM Tris-HCl. I mM EDTA, 100 mM $Li_2OOCCH_3$, pH 7.5) was added. This mixture was gently mixed and incubated at 30°C while agitating for 30 minutes. The cells were then heat shocked at 42°C for 15 minutes, and the reaction vessel centrifuged in a microfuge at 12,000 rpm for 5-10 seconds, decanted and resuspended into TE (500 μl, 10 mM Tris-HCl. I mM EDTA pH 7.5) followed by recentrifugation. The cells were then diluted into TE (1 ml) and aliquots (200 μl) were spread onto the selective media previously prepared in 150 mm growth plates (VWR).

Alternatively, instead of multiple small reactions, the transfonnation was performed using a single, large scale reaction, wherein reagent amounts were scaled up accordingly.

The selective media used was a synthetic complete dextrose agar lacking uracil (SCD-Ura) prepared as described in Kaiser et al., Methods in Yeast Genetics, Cold Spring Harbor Press. Cold Spring Harbor, NY. p. 208-210 (1994). Transformants were grown at 30°C for 2-3 days.

The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15% (w·v), and was buffered with potassium phosphate to a p11 of 7.0 (50-100 mM final concentration).

The positive colonies were picked and streaked across fresh selective media (onto 150 mm plates) in order to obtain well isolated and identifiable single colonies. Well isolated single colonies positive for amylase secretion were detected by direct incorporation of red starch into buffered SCD-Ura agar. Positive colonies were determined by their ability to break down starch resulting in a clear halo around the positive colony visualized directly.

4. Isolation of DNA by PCR Amplification

When a positive colony was isolated, a portion of it was picked by a toothpick and diluted into sterile water (30

μl) in a 96 well plate. At this time, the positive colonies were either frozen and stored for subsequent analysis or immediately amplified. An aliquot of cells (5 μl) was used as a template for the PCR reaction in a 25 μl volume containing: 0.5 μl Klentaq (Clontech. Palo Alto, CA); 4.0 μl 10 mM dNTP's (Perkin Elmer-Cetus); 2.5 μl Kentaq buffer (Clontech); 0.25 μl forward oligo 1; 0.25 μl reverse oligo 2; 12.5 μl distilled water. The sequence of the forward oligonucleotide I was:

5'-TGTAAAACGACGGCCAGTTAAATAGACCTGCAATTATTAATCT-3' (SEQ ID NO:1)

The sequence of reverse oligonucleotide 2 was:

5'-CAGGAAACAGCTATGACCACCTGCACACCTGCAAATCCATT-3' (SEQ ID NO:2)

PCR was then performed as follows:

| | | | | |
|---|---|---|---|---|
| a. | | Denature | 92°C, | 5 minutes |
| b. | 3 cycles of: | Denature | 92°C, | 30 seconds |
| | | Anneal | 59°C, | 30 seconds |
| | | Extend | 72°C, | 60 seconds |
| c. | 3 cycles of: | Denature | 92°C, | 30 seconds |
| | | Anneal | 57°C, | 30 seconds |
| | | Extend | 72°C, | 60 seconds |
| d. | 25 cycles of: | Denature | 92°C, | 30 seconds |
| | | Anneal | 55°C, | 30 seconds |
| | | Extend | 72°C, | 60 seconds |
| e. | | Hold | 4°C | |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.

Following the PCR. an aliquot of the reaction (5 μl) was examined by agarose gel electrophoresis in a 1% agarose gel using a Tris-Borate-EDTA (TBE) buffering system as described by Sambrook *et al., supra.* Clones resulting in a single strong PCR product larger than 400 bp were further analyzed by DNA sequencing after purification with a 96 Qiaquick PCR clean-up column (Qiagen Inc., Chatsworth, CA).

EXAMPLE 3

Isolation of cDNA Clones Using Signal Algorithm Analysis

Various polypeptide-encoding nucleic acid sequences were identified by applying a proprietary signal sequence finding algorithm developed by Genentech, Inc., (South San Francisco, CA) upon ESTs as well as clustered and assembled EST fragments from public (*e.g.*, GenBank) and/or private (LIFESEQ®, Incytc Pharmaceuticals, Inc., Palo Alto. CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In orderto determ ine whetherthe EST sequence contains an authentic signal sequence, the DNA and corresponding amino acid sequences surrounding the ATG codon are scored using a set of seven sensors (evaluation parameters) known to be associated with secretion signals. Use of this algorithm resulted in the identification of numerous polypeptide-encoding nucleic acid sequences.

EXAMPLE 4

Isolation of cDNA Clones Encoding Human PRO172

A consensus DNA sequence encoding a delta-like homologue was assembled relative to other ESTsequences using phrap as described in Example 1 above. This consensus sequence is designated herein as DNA28765. Based on the DNA28765 consensus sequence, oligonucleotides were synthesized: 1) to identify by PCR a cDNA library that contained the sequence of interest, and 2) for use as probes to isolate a clone of the full-length coding sequence for PRO172.

A pair of PCR primers (forward and reverse) were synthesized:

28765.f (OL1644):

5'-GGATCTCGAGAACAGCTACTCC-3' (SEQ ID NO:5)

28765.r (OLI645):

5'-TCGTCCACGTTGTCGTCACATG-3' (SEQ ID NO:6)

Additionally, a synthetic oligonucleotide hybridization probe was constructed from the consensus DNA28765 sequence which had the following nucleotide sequence:

28765.p (OLI643) hybridization probe:

5'-AAATCTGTGAATTGAGTGCCATGGACCTGTTGCGGACGGCCCTTGCTT-3' (SEQ ID NO:7)

In order to screen several libraries for a source of a full-length clone, DNA from the libraries was screened by PCR amplification with the PCR primer pair identified above. A positive library was then used to isolate clones encoding the PRO 172 gene using the probe oligonucleotide and one of the PCR primers. RNA for construction of the cDNA libraries was isolated from human fetal kidney tissue.

DNA sequencing of the isolated clones isolated as described above gave the full-leneth DNA sequence for DNA35916-1161 [Figure I, SEQ ID NO:3]; and the derived protein sequence for PRO172.

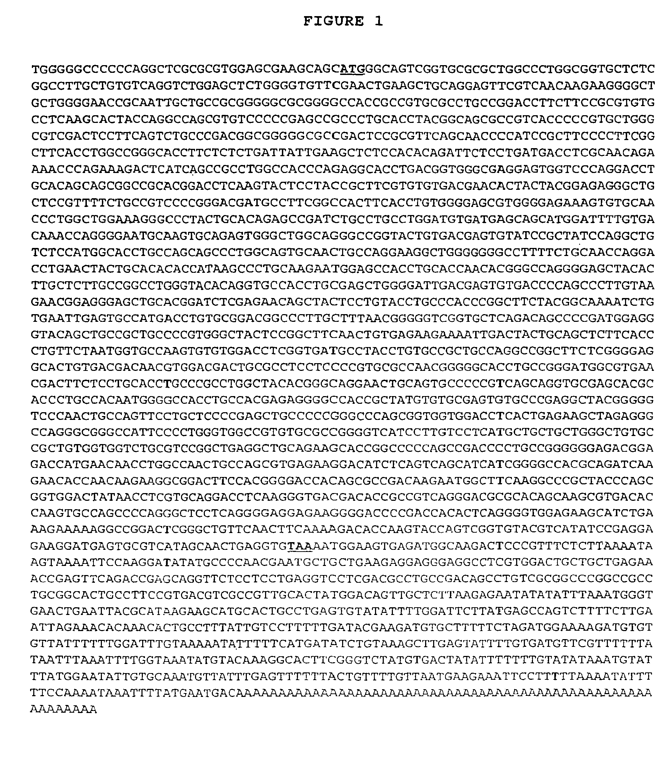

The entire coding sequence of DNA35916-1161 is included in Figure 1 (SEQ ID NO:3). Clone DNA35916-116 I contains a single open reading frame with an apparent translational initiation site at nucleotide positions 38-40, and an apparent stop codon at nucleotide positions 2207-2209. The predicted polypeptide precursor is 723 amino acids long. Analysis of the full-length PRO172 sequence shown in Figure 2 (SEQ ID NO:4) evidences the presence of a variety of important polypeptide domains, wherein the locations given for those important polypeptide domains are approximate as described above. Analysis of the full-length PRO172 polypeptide shown in Figure 2 evidences the presence of the following: a signal peptide from about amino acid 1 to about amino acid 21; a transmembrane domain from about amino acid 546 to about amino acid 566; an N-glycosylation site from about amino acid 477 to about amino acid 481; a cAMP- and cGMP-dependent protein kinase phosphorylation site from about amino acid 660 to about amino acid 664; tyrosine kinase phosphorylation sites from about amino acid 176 to about amino acid 185, and from about amino acid 252 to about amino acid 261; N-myristoylation sites from about amino acid 2 to about amino acid 8, from about amino acid 37 to about amino acid 43, from about amino acid 40 to about amino acid 46, from about amino acid 98 to about amino acid 104, from about amino acid 99 to about amino acid 105, from about amino acid 262 to about amino acid 268, from about amino acid 281 to about amino acid 287, from about amino acid 282 to about amino acid 288, from about amino acid 301 to about amino acid 307, from about amino acid 310 to about amino acid 316, from about amino acid 328 to about amino acid 334, from about amino acid 340 to about amino acid 344, from about amino acid 378 to about amino acid 384, from about amino acid 387 to about amino acid 393. from about amino acid 512 to about amino acid 518. from about amino acid 676 to about amino acid 682, from about amino acid 683 to about amino acid 689, and from about amino acid 695 to about amino acid 701: aspartic acid and asparagine hydroxylation sites from about amino acids 343 tu about amino acid 355, from about amino acid 420 to about amino acid 432. and from about amino acid 458 to about amino acid 470; a prokaryotic membrane lipoprotein lipid attachment site from about amino acid 552 to about amino acid 563; and EGF-like domain cysteine pattern signatures from about amino acid 243 to about amnino acid 255, from about amino acid 274 to about amino acid 286, from about amino acid 314 to about amino acid 326, from about amino acid 352 to about amino acid 364, from about amino acid 391 to about amino acid 403, from about amino acid 429 to about amino acid 441. from about amino acid 467 to about amino acid 479. and from about amino acid 505 to about amino acid 517. Clone DNA35916-1161 has been deposited with the ATCC on October 28, 1997 and is assigned ATCC deposit no. 209419.

Based on a BLAST and FastA sequence alignment analysis of the full-length sequence shown in Figure 2 (SEQ ID NO:4), PRO172 shows 89% amino acid sequence identity to delta-1 mouse protein.

EXAMPLE 5

Isolation of cDNA Clones Encoding Human PRO178

An expressed sequence tag (EST) DNA database ( LIFESEQ®. Incyte Pharmacenticals, Palo Alto, CA) was searched and an EST was identified which showed homology to the human TIE ligand family.

RNA for construction of cDNA libraries was then isolated from human fetal lung tissue. The cDNA libraries used to isolate the cDNA clones encoding human PRO178 were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI.

Oligonucleotides probes based upon the above described EST sequence were then synthesized: 1) to identify by PCR a cDNA library that contained the sequence of interest, and 2) for use as probes to isolate a clone of the full-length coding sequence for PRO178. Forward and reverse PCR primers generally range from 20 to 30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubel *et al.,* Current Protocols in Molecular Biology, *supra,* with the PCR primer pair. A positive library was then used to isolate clones encoding the gene of interest using the probe oligonucleotide and one of the primer pairs.

The oligonucleotide probes employed were as follows:

NL8.5-1:

5'-ACGTAGTTCCAGTATGGTGTGAGCAGCAACTGGA-3' (SEQ ID NO:10)

NL8.3-1:

5'-AGTCCAGCCTCCACCCTCCAGTTGCT-3' (SEQ ID NO:11)

NL8.3-2:

5'-CCCCAGTCCTCCAGGAGAACCAGCA-3' (SEQ ID NO:12)

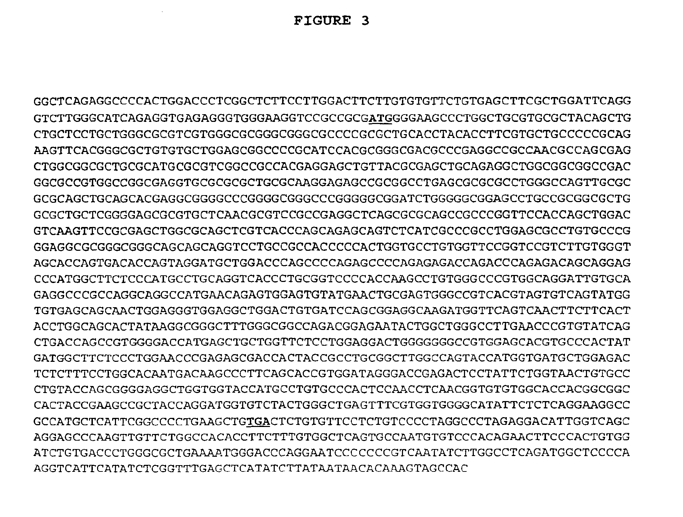

A full length clone [DNA23339-1130]was identified that contained a single open reading frame with an apparent translational initiation site at nucleotide positions 118-120 and a stop signal at nucleotide positions 1528-1530 (Figure 3, SEQ ID NO:8). The predicted polypeptide precursor is 470 amino acids long, has a calculated molecular weight of approximately 51,694 daltons and an estimated pI of approximately 8.86. Analysis of the full-length PRO178 sequence shown in Figure 4 (SEQ ID NO:9) evidences the presence of a variety of important polypeptide domains as shown in Figure 4, wherein the locations given for those important polypeptide domains are approximate as described above. Analysis of the full-length PRO 178 polypeptide shown in Figure 4 evidences the presence of the following a signal peptide from about amino acid I to about amino acid 20; N-glycosylation sites from about amino acid 58 to about amino acid 62, and from about amino acid 145 to about amino acid 149; a cAMP-and cGMP-dependent protein kinase phosphorylation site from about amino acid 97 to about amino acid 101 : a tyrosine kinase phosphorylation site from about amino acid 441 to about amino acid 448; N-myristoylation sites from about amino acid 16 to about amino acid 22; from about amino acid 23 to about amino acid 29, from about amino acid 87 to ahout amino acid 93, from about amino acid 108 to about amino acid 114, from about amino acid 121 to about amino acid 127. from about amino acid 125 to about amino acid 131, from about amino acid 129 to about amino acid 135. from about amino acid 187 to about amino acid 193. from about amino acid 293 to about amino acid 299, from about amino acid.353 to about amino acid 359. from about amino acid 378 to about amino acid 384, from about amino acid 445 to about amino acid 451, and from about amino acid 453 to about amino acid 459; a cell attachment site from about amino acid 340 to about amino acid 343; and a fibrinogen beta and gamma chains C-terminal domain signature from about amino acid 4 18 to about amino acid 431. Clone DNA23339-1130 has been deposited with ATCC on September 18, 1997 and is assigned ATCC deposit no. 209282.

Based on a BLAST and FastA sequence alignment analysis of the full-length sequence shown in Figure 4 (SEQ ID NO:9), PRO178 (herein designated NL8) shows a 23% amino acid sequence identity to both ligand I and ligand 2 of the TIE2 receptor. Ligand I and ligand 2 of the TIE-2 receptor are 64% identical and 40-43% identical, respectively, to PRO178. The abbreviation "TIE" is an acronym which stands for "tyrosine kinase containing Ig and EGF homology domains" and was coined to designate a new family of receptor tyrosine kinases.

EXAMPLE 6

Isolation of cDNA Clones Encoding Human PRO179

A cDNA sequence isolated in the amylase screen described in Example 2 above was found, by BLAST and FastA sequence alignment, to have sequence homology to a nucleotide sequence encoding various angiopoietin proteins. This cDNA sequence is herein designated DNA10028 and/or DNA25250. Based on the sequence homology, probes were generated from the sequence of the DNA 10028 molecule and used to screen a human fetal liver library (LIB6) prepared as described in paragraph 1 of Example 2 above. The cloning vector was pRK5B (pRK5B is a precursor of pRK5D that does not contain the SfiI site; *see*, Holmes et al., Science, 253:1278-1280 (1991)), and the cDNA size cut was less than 2800 bp.

In order to screen several libraries for a source of a full-length clone, DNA from the libraries was screened by PCR amplification with the PCR primer pair identified above. A positive library was then used to isolate clones encoding the PRO179 gene using a probe oligonucleotide and one of the PCR primers.

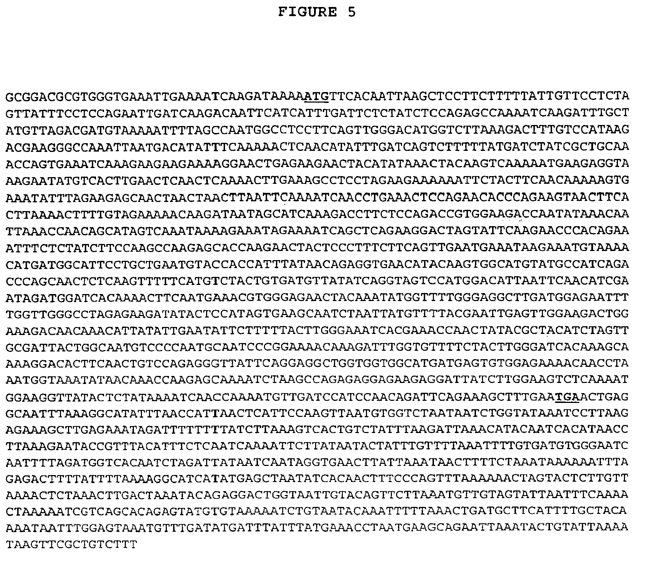

A full length clone [DNA16451-1388] was identified that contained a single open reading frame with an apparent translational initiation site at nucleotide positions 37-39, and a stop signal at nucleotide positions 14 17-1419 (Figure 5; SEQ ID NO:13). The predicted polypeptide precursor is 460 amino acids long, and has a calculated molecular weight of approximately 53,637 daltons and an estimated pI of approximately 6.61. Analysis of the full-length PRO179 sequence shown in Figure 6 (SEQ ID NO:14) evidences the presence of a variety of important polypeptide domains as shown in Figure 6, wherein the locations given for those important polypeptide domains are approximate as described above. Analysis of the full-length PRO 179 polypeptide shown in Figure 6 evidences the presence of the following: a signal peptide from about amino acid 1 to about amino acid 16, N-glycosylation sites from about amino acid 23 to about amino acid 27, from about amino acid 115 to about amino acid 119, from about amino acid 296 to about amino acid 300, and from about amino acid 357 to about amino acid 361; cAMP-and cGMP-dependent protein kinase phosphorylation sites from about amino acid 100 to about amino acid 104, and from about amino acid 204 to about amino acid 208; a tyrosine kinase phosphorylation site from about amino acid 342 to about amino acid 351: N-myristoylation sites from about amino acid 279 to about amino acid 285, from about amino acid 352 to about amino acid 358, and from about amino acid 367 to about amino acid 373; and leucine zipper patterns from about amino acid 120 to about amino acid 142, and from about amino acid 127 to about amino acid 149. Clone DNA 16451-1388 was deposited with the ATCC on April 14, 1998, and is assigned ATCC deposit no. 209776.

Analysis of the amino acid sequence of the full-length PRO179 polypeptide shown in Figure 6 (SEQ ID NO: 14) suggests that it possesses significant similarity to the angiopoietin family of proteins, thereby indicating that PRO179 may be a novel angiopoietin family member. More specifically, an analysis ofthe Dayhoffdatabase (version 35.45 Swiss-Prot 35) evidenced significant homology between the PRO179 amino acid sequence and the following Dayhoff sequences: AF004326_1, P_R94605, HSU83508_1, P_R94603, P_R94317, AF025818_1, HSY16132_1, P_R65760, 137391 and HUMRSC192_1.

EXAMPLE 7

isolation of cDNA Clones Encoding Human PRO182

The nucleic acid sequence of the relaxin molecule, a member of the insulin family of proteins, was used to search for homologous sequences in a human colon cDNA library of expressed sequence tags (ESTs) from Incyte, Inc. (LIFESEQ®. Incyte Pharmaceuticals, Palo Alto, CA). Two ESTs were obtained, Incyte EST nos. INC2328985 and INC778319, each having approximately 40% homology to the region ofthe relaxin nucleic acid sequence, and represent sequences within a gene of an insulin-like polypeptide (ILP).

A cDNA library was constructed from human uterus mRNA obtained from Clontech Laboratories, Inc. Palo Alto, CA, catalog no. 6537-1. The full-length nucleic acid sequence of PRO182 was obtained by screening a plasmid cDNA library described above, by colony hybridization using oligonucleotides designed based on the EST sequences from Incyte. Inc. (Incyte EST INC2328985 and Incyte EST INC778319). The cDNA libraries used to isolate the cDNA clones encoding human PRO178 were constructed by standard methods using commercially available reagents such as those from Invitrogen, San Diego. CA. The cDNA was primed with oligo dT containing a NotI site, linked with blunt to Sall hemikinased adaptors, cleaved with Notl, sized appropriately by gel electrophoresis, and cloned in a defined orientation into a suitable cloning vector (such aspRKB or pRKD; pRK5B is a precursor of pRK5D that does not contain the Sfil site; *see,* Holmes et al., Science, 253:1278-1280 (1991)) in the unique Xhol and Notl.

Oligonucleotides probes based upon the above described EST sequences were then synthesized: 1) to identify by PCR a cDNA library that contained the sequence of interest, and 2) for use as probes to isolate a clone of the full-length coding sequence for PRO178. Forward and reverse PCR primers generally range from 20 to 30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubel *et al.,* Current Protocols in Molecular Biology, *supra,* with the PCR primer pair. A positive library was then used to isolate clones encoding the gene of interest using the probe oligonucleotide and one of the primer pairs.

The primer oligonucleotides sequences used are as follows:

5'-CACATTCAGTCCTCAGCAAAATGAA-3' (SEQ ID NO:17)
5'-GAGAATAAAAACAGAGTGAAAATGGAGCCCTTCATTTTGC-3' (SEQ ID NO: 18)
5'-CTCAGCTTGCTGAGCTTGAGGGA-3' (SEQ ID NO:19)

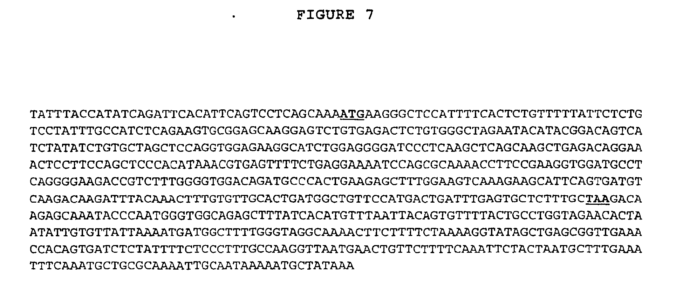

A full length clone [DNA27865-1091]was identified that contained a single open reading frame with an apparent translational initiation site at nucleotide positions 39-41 and a stop signal at nucleotide positions 444-446 (Figure 7, SEQ ID NO:15). The predicted polypeptide precursor is 135 amino acids long, and has a calculated molecular weight of approximately 15,319 daltons and an estimated pl of approximately 7.39. Analysis of the full-length PRO 182 sequence shown in Figure 8 (SEQ ID NO: 16) evidences the presence of a variety of important polypeptide domains as shown in Figure 8, wherein the locations given for those important polypeptide domains are approximate as described above. Analysis ofthe full-length PRO182 polypeptide shown in Figure 8 evidences the presence of the following: a signal peptide from about amino acid 1 to about amino acid 18; a cAMP- and cGMP-dependent protein kinase phosphorylation site from about amino acid 107 to about amino acid 111; N-myristoylation sites from about amino acid 3 to about amino acid 9, from about amino acid 52 to about amino acid 58, from about amino acid 96 to about amino acid 102, and from about amino acid 125 to about amino acid 131; and an insulin family signature from about amino acid 121 to about amino acid 136. Clone DNA27865-1091 has been deposited with ATCC on September 23, 1997 and is assigned ATCC deposit no. 209296.

Based on a BLAST and FastA sequence alignment analysis (using the ALIGN computer program) of the full-length sequence shown in Figure 8 (SEQ ID NO:16), the PRO182 polypeptide sequence was homologous to but clearly different from any known polypeptide molecule, and therefore the PRO 182 polypeptide constitutes a novel member of the insulin family of proteins.

EXAMPLE 8

Isolation of cDNA Clones Encoding Human PRO187

An expressed sequence tag (EST) DNA database ( LIFESEQ®, Incyte Pharmaceuticals, Palo Alto, CA) was searched and an EST (no. IN843193)was identified which showed homology to fibroblast growth factor(FGF-8) also known as androgen-induced growth factor.

RNA for construction ofcDNA libraries was then isolated from human fetal lung tissue. The cDNA libraries used to isolate the cDNA clones encoding human PRO187 were constructed by standard methods using commercially available reagents such as those from Invitrogen, San Diego, CA. The cDNA was primed with oligo dT containing a Notl site. linked with blunt to Sall hemikinased adaptors, cleaved with Notl, sized appropriately by gel electrophoresis, and cloned in a defined orientation into a suitable cloning vector (such as pRKB of pRKD; pRK5B is a precursor of pRK5D that does not contain the Sfil site: *sec,* Holmes et al., Science, 253: 1278-1280 (1991)) in the unique Xhol and Notl.

Oligonucleotides probes based upon the above described EST sequence were then synthesized: l) to identify by PCR a cDNA library that contained the sequence of interest, and 2) for use as probes to isolate a clone of the full-length coding sequence for PRO187. Forward and reverse PCR primers generally range from 20 to 30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubel *et al.,* Current Protocols in Molecular Biology, *supra,* with the PCR primer pair. A positive library was then used to isolate clones encoding the gene of interest using the probe oligonucleotide and one of the primer pairs.

Several libraries from various tissue sources were screened by PCR amplification with the following oligonucleotide probes:

IN843193.f (OLI315):

5'-CAGTACGTGAGGGACCAGGGCGCCATGA-3' (SEQ ID NO:22)

IN843193.r (OLI317):

5'-CCGGTGACCTGCACGTGCTTGCCA-3' (SEQ ID NO:23)

A positive library was then used to isolate clones encoding the FGF-8 homologue gene using one ofthe above oligonucleotides and the following oligonucleotide probe:

IN843193.p (OL1316):

5'-GCGGATCTGCCGCCTGCTCANCTGGTCGGTCATGGCGCCCT-3' (SEQ ID NO:24)

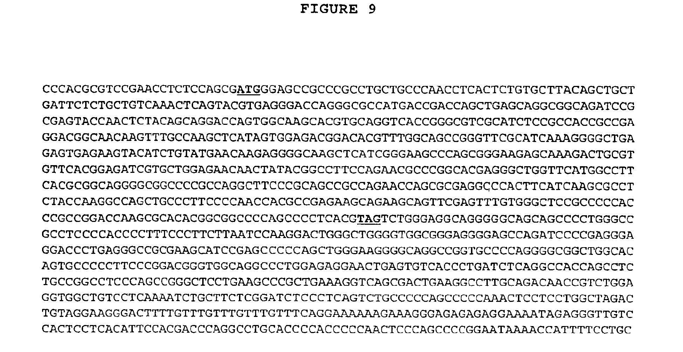

A full length clone [DNA27864-1155] was identified that contained a single open reading frame with an apparent translational initiation site at nucleotide positions 26-28 and a stop signal at nucleotide positions 641-643 (Figure 9, SEQ ID NO:20). The predicted polypeptide precursor is 205 amino acids long, has a calculated molecular weight of approximately 23,669 daltons and an estimated pl of approximately 10.75. Analysis of the full-length PRO187 sequence shown in Figure 10 (SEQ ID NO:21) evidences the presence of a variety of important polypeptide domains as shown in Figure 10, wherein the locations given for those important polypeptide domains are approximate as described above. Analysis of the full-length PRO187 polypeptide shown in Figure 10 evidences the presence of the following: a signal peptide from about amino acid 1 to about amino acid 22; N-glycosylation sites from about amino acid 9 to about amino acid 13, and from about amino acid 126 to about amino acid 130: a cAMP- and cGMP-dependent protein kinase phosphorylation site from about amino acid 60 to about amino acid 64; tyrosine kinase phosphorylation sites from about amino acid 39 to about amino acid 48, and from about amino acid 89 to about amino acid 97; N-myristoylation sites from about amino acid 69 to about amino acid 75, and from about amino acid 188 to about amino acid 194; an amidation site from about amino acid 58 to about amino acid 62; and a HBGF/FGF family signature from about amino acid 103 to about amino acid 128. Clone DNA27864-1155 has been deposited with ATCC on October 16, 1997 and is assigned ATCC deposit no. 209375.

Based on a BLAST and FastA sequence alignment analysis (using the ALIGN computer program) of the full-length sequence shown in Figure 10 (SEQ ID NO:21). PRO 187 shows 74% amino acid sequence identity to human fibroblast growth factor-8 (androgen-induced growth factor).

EXAMPLE 9

Isolation of cDNAClones Encoding Human PRO188

An expressed sequence tag (EST) DNA database ( LIFESEQ®, Incyte Pharmaceuticals, Palo Alto, CA) was searched and an EST was identified which showed homology to the human TIE ligand family.

RNA for construction of cDNA libraries was then isolated from human fetal lung tissue. The cDNA libraries used to isolate the cDNA clones encoding human PRO188 were constructed by standard methods using commercially available reagents such as those from Invitrogen, San Diego, CA. The cDNA was primed with oligo dT containing a Notl site, linked with blunt to Sall hemikinased adaptors, cleaved with Notl, sized appropriately by gel electrophoresis, and cloned in a defined orientation into a suitable cloning vector (such as pRKB or pRKD; pRK5B is a precursor of pRK5D that does not contain the Sfil site; *see,* Holmes et al., Science, 253:1278-1280 (1991)) in the unique Xhol and Notl.

Oligonucleotides probes based upon the above described EST sequence were then synthesized: 1) to identify by PCR a cDNA library that contained the sequence of interest, and 2) for use as probes to isolate a clone of the full-length coding sequence for PRO188. Forward and reverse PCR primers generally range from 20 to 30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubel *et al.,* Current Protocols in Molecular Biology, *supra,* with the PCR primer pair. A positive library was then used to isolate clones encoding the gene of interest using the probe oligonucleotide and one of the primer pairs.

The oligonucleotide probes employed were as follows:

NL5.5-1:

5'-CAGGTTATCCCAGAGATTTAATGCCACCA-3' (SEQ ID NO:27)

NL5.3-1:

5'-TTGGTGGGAGAAGTTGCCAGATCAGGTGGTGGCA-3' (SEQ ID NO:28)

NL5.3-2:

5'-TTCACACCATAACTGCATTGGTCCA-3' (SEQ ID NO:29)

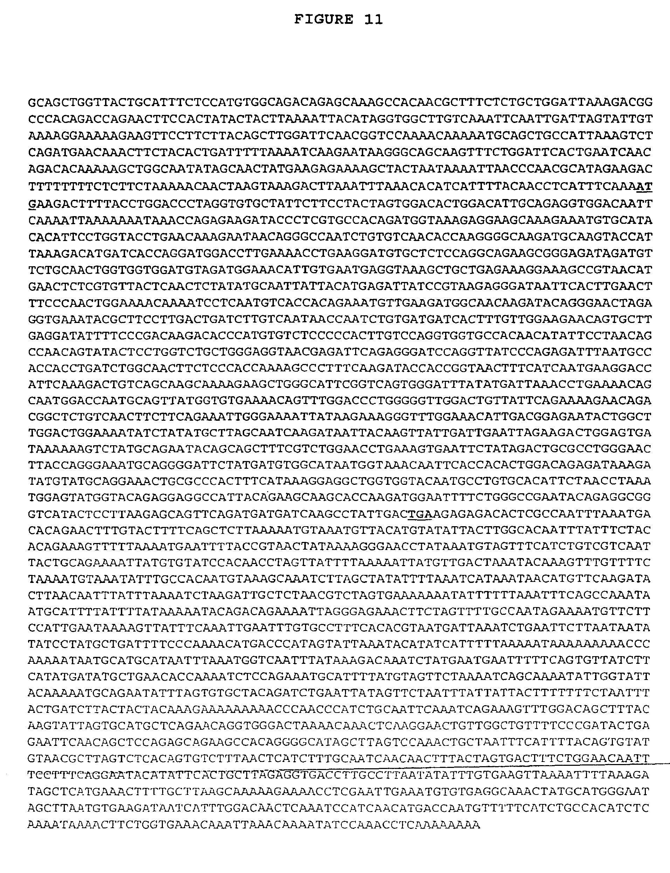

A full length clone [DNA28497-1130] was identified that contained a single open reading frame with an apparent translational initiation site at nucleotide positions 449-451 and a stop signal at nucleotide positions 1922-1924 (Figure 11, SEQ ID NO:25). The predicted polypeptide precursor is 491 amino acids long, and has a calculated molecular weight of approximately 56,720 daltons and an estimated pl of approximately 8.56. Analysis of the full-length PRO188 sequence shown in Figure 12 (SEQ ID NO:26) evidences the presence of a variety of important pulypeptide domains as shown in Figure 12, wherein the locations given for those important polypeptide domains are approximate as described above. Analysis of the full-length PRO188 polypeptide shown in Figure 12 evidences the presence of the following: a signal peptide from about amino acid 1 to about amino acid 23; N-glycosylation sites from about amino acid 160 to about amino acid 164, and from about amino acid 188 to about amino acid 192: a cAMP- and cGMP-dependent protein kinase phosphorylation site from about amino acid 120 to about amino acid 124: tyrosine kinase phosphorylation sites from about amino acid 173 to about amino acid 180, and from about amino acid 387 to about amino acid 396: N-myristoylation sites from about amino acid 70 to about amino acid 76. from about amino acid 110 to about amino acid 116, from about amino acid 232 to about amino acid 238, from about amino acid 343 to about amino acid 349, from about amino acid 400 to about amino acid 406, from about amino acid 467 to about amino acid 473, and from about amino acid 475 to about amino acid 487; and a fibrinogen beta and gamma chains C-terminal domain signature from about amino acid 440 to about amino acid 453. Clone DNA28497-1130 has been deposited with ATCC on September 18,1997 and is assigned ATCC deposit no. 209279.

Based on a BLAST and FastA sequence alignment analysis of the full-length sequence shown in Figure 12 (SEQ ID NO:26), PRO 188 (herein designated NL5) shows 24% amino acid sequence identity to both ligand 1 and ligand 2 of the TIE2 receptor. Ligand 1 and ligand 2 of the TIE-2 receptor are 64% identical and 40-43% identical, respectively, to PRO188. The abbreviation "TIE" is an acronym which stands for "tyrosine kinase containing Ig and EGF homology domains" and was coined to designate a new family of receptor tyrosine kinases.

EXAMPLE 10

Isolation of cDNA Clones Encoding Human PRO195

A cDNA sequence isolated in the amylase screen described in Example 2 above is herein designated DNA 13199_ABI2. The DNA13199_ABI2 sequence was then compared to a variety of expressed sequence tag (EST) databases which included public EST databases (*e.g.*, GenBank) to identify existing homologies. The homology search was performed using the computer program BLAST or BLAST2 (Altshul et al., Methods in Enzymology, 266:460-480 (1996)). Those comparisons resulting in a BLAST score of 70 (or in some cases 90) or greater that did not encode known proteins were clustered and assembled into consensus DNA sequences with the program "phrap" (Phil Green. University of Washington, Seattle, Washington). The consensus sequence identified is herein designated DNA22778.

Based on the DNA 13199_AB12 sequence and DNA22778 sequences, oligonucleotide probes were generated and used to screen a human placenta library (LIB89) prepared as described in paragraph I of Example 2 above. The cloning vector was pRK5B (pRK5B is a precursor of pRK5D that does not contain the Sfil site: *see,* Holmes et al., Science, 253:1278-1280 (1991)), and the cDNA size cut was less than 2800 bp.

PCR primers (forward and reverse) were synthesized:

forward PCR primer (22778.f):

5'-ACAAGCTGAGCTGCTGTGACAG-3 (SEQ ID NO:32)

reverse PCR primer (22778.r):

5'-TGATTCTGGCAACCAAGATGGC-3' (SEQ ID NO:33)

Additionally, a synthetic oligonucleotide hybridization probe was constructed from the DNA22778 consensus sequence which had the following nucleotide sequence:

hybridization probe (22778.p):

5'-ATGGCCTTGGCCGGAGGTTCGGGGACCGCTTCGGCTGAAG-3' (SEQ ID NO:34)

In order to screen several libraries for a source of a full-length clone, DNA from the libraries was screened by PCR amplification with the PCR primer pair identified above. A positive library was then used to isolate clones encoding the PRO195 gene using a probe oligonucleotide and one of the PCR primers.

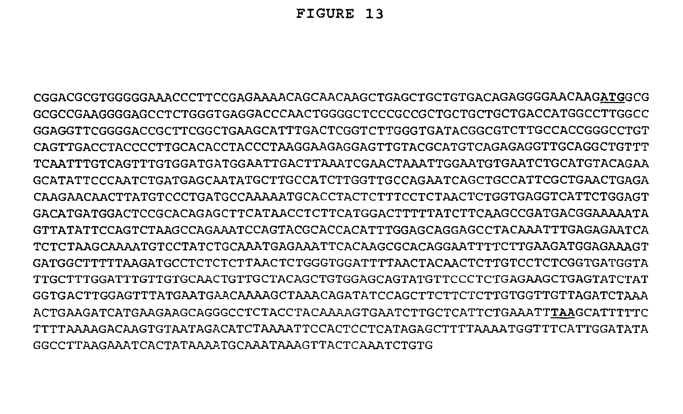

A full length clone [DNA26847-1395] was identified that contained a single open reading frame with an apparent translational initiation site at nucleotide positions 70-72, and a stop signal at nucleotide positions 1039-1041 (Figure 13; SEQ ID NO:30). The predicted polypeptide precursor is 323 amino acids long, and has a calculated molecular weight of approximately 36,223 daltons and an estimated pl of approximately 5.06. Analysis of the full-length PRO195 sequence shown in Figure 14 (SEQ ID NO:31) evidences the presence of a variety of important polypeptide domains as shown in Figure 14, wherein the locations given for those important polypeptide domains are approximate as described above. Analysis of the full-length PRO195 polypeptide shown in Figure 14 evidences the presence of the following: a signal peptide from about amino acid 1 to about amino acid 31, a transmembrane domain from about amino acid 242 to about amino acid 262; an N-glycosylation site from about amino acid 90 to about amino acid 94; and N-myristoylation sites from about amino acid 28 to about amino acid 34, from about amino acid 29 to about amino acid 35, from about amino acid 31 to about amino acid 37, and from about amino acid 86 to about amino acid 92. Clone DNA26847-1395 was deposited with the ATCC on April 14, 1998 , and is assigned ATCC deposit no. 209772.

Analysis of the amino acid sequence of the full-length PRO195 polypeptide (Figure 14; SEQ ID NO:31) suggests that it possesses no significant similarity to any known protein. However, an analysis of the Dayhoff database (version 35.45 SwissProt 35) evidenced some degree of homology between the PRO195 amino acid sequence and the following Dayhoff sequences: P_P91380, AF035118_1, HUMTROPCS_ 1, NUOD_SALTY and E70002.

EXAMPLE 11

Isolation of cDNA Clones Encoding Human PRO212

A consensus DNA sequence was assembled relative to other EST sequences (including an EST proprietary to Genentech) using phrap as described in Example I above. Based on the assembled consensus sequence, oligonucleotides were synthesized: 1) to identify by PCR a cDNA library that contained the sequence of interest, and 2) for use as probes to isolate a clone of the full-length coding sequence for PRO212.

A pair of PCR primers (forward and reverse) were synthesized:

forward PCR primer:

5'-CACGCTGGTTTCTGCTTGGAG-3' (SEQ ID NO:37)

reverse PCR primer

5'-AGCTGGTGCACAGGGTGTCATG-3' (SEQ ID NO:38)

Additionally, a synthetic oligonucleotide hybridization probe was constructed from the consensus sequence which had the following nucleotide sequence:

hybridization probe:

5'-CCCAGGCACCTTCTCAGCCAGCCAGCAGCTCCAGCTCAGAGCAGTGCCAGCCC-3(SEQ ID NO:39)

In order to screen several libraries for a source of a full-length clone, DNA from the libraries was screened by PCR amplification with the PCR primer pair identified above. A positive library was then used to isolate clones encoding the PRO212 gene using the probe oligonucleotide and one of the PCR primers. RNA for construction of the cDNA libraries was isolated from human fetal lung tissue.

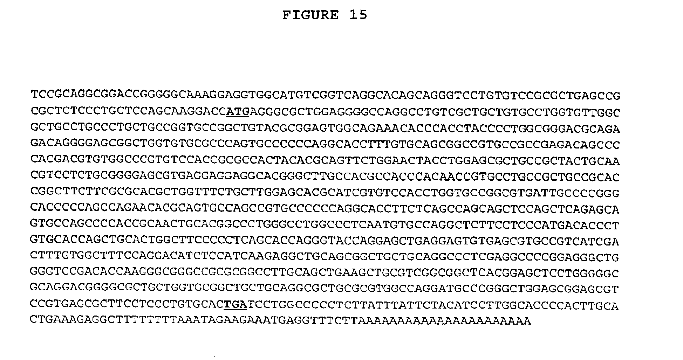

DNA sequencing of the isolated clones isolated as described above gave the full-length DNA sequence for DNA30942-1134 [Figure 15, SEQ ID NO:35]; and the derived protein sequence for PRO212.

The entire coding sequence of DNA30942-1134 is included in Figure 15 (SEQ ID NO:35). Clone DNA30942-1134 contains a single open reading frame with an apparent translational initiation site at nucleotide positions 101-103, and an apparent stop codon at nucleotide positions 1001-1003. The predicted polypeptide precursor is 300 amino acids long. Analysis of the full-length PRO212 sequence shown in Figure 16 (SEQ ID NO:36)evidences the presence of variety of important polypeptide domains, wherein the locations given for those important polypeptide domains are approximate as described above. Analysis of the full-length PRO212 polypeptide shown in Figure 16 evidences the presence of the following: a signal peptide from about amino acid 1 to about amino acid 23: an N-glycosylation site from about amino acid 173 to about amino acid 177; cAMP- and cGMP-dependent protein kinase phosphorylation sites from about amino acid 63 to about amino acid 67, and from about amino acid 259 to about amino acid 263; a tyrosine kinase phosphorylation site from about amino acid 28 to about amino acid 37; N-myristoylation sites from about amino acid 156 to about amino acid 162, from about amino acid 178 to about amino acid 184, from about amino acid 207 to about amino acid 213, from about amino acid 266 to about amino acid 272, and from about amino acid 287 to about amino acid 293. Clone DNA30942-1134 has been deposited with the ATCC on September 16, 1997 and is assigned ATCC deposit no. 209254.

Based on a BLAST and FastA sequence alignment analysis of the full-length sequence shown in Figure 16 (SEQ ID NO:36), PRO212 shows some amino acid sequence identity to TNFR2 (28.7%).

EXAMPLE 12

Isolation of cDNA Clones Encoding Human PRO214

A consensus DNA sequence encoding an EGF-like homologue was assembled relative to other EST sequences using phrap as described in Example 1 above. This consensus sequence is designated herein as DNA28744. Based on the assembled DNA28744 consensus sequence, oligonucleotides were synthesized: 1) to identify by PCR a cDNA library that contained the sequence of interest, and 2) for use as probes to isolate a clone of the full-length coding sequence for PRO214.

A pair of PCR primers (forward and reverse) were synthesized:

forward PCR primer (OL1556):

5'-ATTCTGCGTGAACACTGAGGGC-3' (SEQ ID NO:42)

reverse PCR primer (OLI557):

5'-ATCTGCTTGTAGCCCTCGGCAC-3' (SEQ ID NO:43)

Additionally, a synthetic oligonucleotide hybridization probe was constructed from the DNA28744 consensus sequence which had the following nucleotide sequence:

hybridization probe (OLI555):

5'-CCTGGCTATCAGCAGGTGGGCTCCAAGTGTCTCGATGTGGATGAGTGTGA-3' (SEQ ID NO:44)

In order to screen several libraries for a source of a full-length clone, DNA from the libraries was screened by PCR amplification with the PCR primer pair identified above. A positive library was then used to isolate clones encoding the PRO214 gene using the probe oligonucleotide and one of the PCR primers. RNA for construction of the cDNA libraries was isolated from human fetal lung tissue.

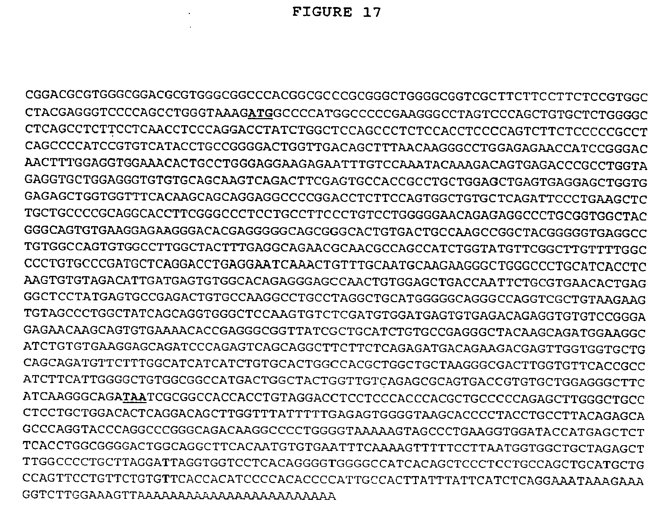

DNA sequencing of the isolated clones isolated as described above gave the full-length DNA sequence for DNA32286-1191 [Figure 17, SEQ ID NO:40]; and the derived protein sequence for PRO214.

The entire coding sequence of DNA32286-1191 is included in Figure 17 (SEQ ID NO:40). Clone DNA32286-1191 contains a single open reading frame with an apparent translational initiation site at nucleotide positions 103-105, and an apparent stop codon at nucleotide positions 1363-1365. The predicted polypeptide precursor is 420 amino acids long. Analysis of the full-length PRO214 sequence shown in Figure 18 (SEQ ID NO:41) evidences the presence of a variety of important polypeptide domains, wherein the locations given forthose important polypeptide domains are approximate as described above. Analysis of the full-length PRO214 polypeptide shown in Figure 18 evidences the presence of the following: a signal peptide from about amino acid I to about amino acid 29; a transmembrane domain from about amino acid 342 to about amino acid 392: N-glycosylation sites from about amino acid 79 to about amino acid 83. and from about amino acid 205 to about amino acid 209; a cAMP- and cGMP-dependent protein kinase phosphorylation site from about amino acid 290 to about amino acid 294; an aspartic acid and asparagine hydroxylation site from about amino acid 321 to about amino acid 333; and an EGF-like domain cysteine pattern signature from about amino acid 181 to about amino acid 193. Clone DNA32286-1191 has been deposited with the ATCC on October 16, 1997 and is assigned ATCC deposit no. 209385.

Based on a BLAST and FastA sequence alignment analysis of the full-length sequence shown in Figure 18 (SEQ ID NO:41), PRO214 shows amino acid sequence identity to HT protein and/or Fibulin (49% and 38%, respectively).

EXAMPLE 13

Isolation of cDNA Clones Encoding Human PRO217

A consensus UNA sequence was assembled relative to other EST sequences using phrap as described in Example I above. This consensus sequence is designated herein as DNA28760. Based on the assembled DNA28760 consensus sequence, oligonucleotides were synthesized: 1) to identify by PCR a cDNA library thar contained the sequence of interest, and 2) for use as probes to isolate a clone ofthe full-length coding sequence for PRO217.

A pair of PCR primers (forward and reverse) were synthesized:

forward PCR primer:

5'-AAAGACGCATCTGCGAGTGTCC-3' (SEQ ID NO:47)

reverse PCR primer:

5'-TGCTGATTTCACACTGCTCTCCC-3' (SEQ ID NO:48)

Additionally, a synthetic oligonucleotide hybridization probe was constructed from the DNA28760 consensus sequence which had the following nucleotide sequence:

hybridization probe:

5'-CCCACGATGTATGAATGGTGGACTTTGTGTGACTCCTGGTTTCTGCATC-3' (SEQ ID NO:49)

In order to screen several libraries for a source of a full-length clone, DNA from the libraries was screened by PCR amplification with the PCR primer pair identified above. A positive library was then used to isolate clones encoding the PRO217 gene using the probe oligonucleotide and one of the PCR primers. RNA for construction of the cDNA libraries was isolated from human fetal lung tissue.

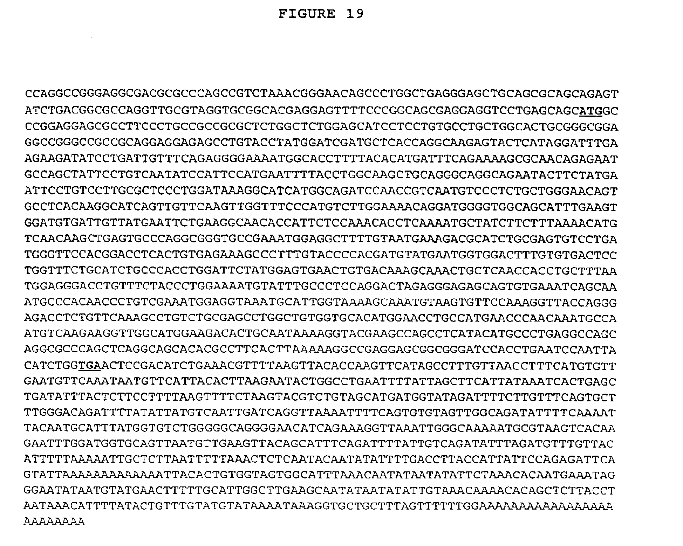

DNA sequencing of the isolated clones isolated as described above gave the full-length DNA sequence for DNA33094-1131 [Figure 19, SEQ ID NO:45]; and the derived protein sequence for PRO217.

The entire coding sequence of DNA33094-1131 is included in Figure 19 (SEQ ID NO:45). Clone DNA33094-1131 contains a single open reading frame with an apparent translational initiation site at nucleotide positions 146-148, and an apparent stop codon at nucleotide positions 1283-1285. The predicted polypeptide precursor is 379 amino acids long with a molecular weight of approximately 41.528 daltons and an estimated pl of about 7.97. Analysis of the full-length PRO217 sequence shown in Figure 20 (SEQ ID NO:46) evidences the presence of a variety of important polypeptide domains, wherein the locations given for those important polypeptide domains are approximate as described above. Analysis of the full-length PRO217 polypeptide shown in Figure 20 evidences the presence of the following: a signal peptide from about amino acid I to about amino acid 28; N-glycosylation sites from about amino acid 88 to about amino acid 92, and from about amino acid 245 to about amino acid 249; a tyrosine kinase phosphorylation site from about amino acid 370 to about amino acid 378; N-myristoylation sites from about amino acid 184 to about amino acid 190, from about amino acid 185 to about amino acid 191, from about amino acid 189 to about amino acid 195, and from about amino acid 315 to about amino acid 321; an ATP/GTP-binding site motif A (P-loop) from about amino acid 285 to about amino acid 293; and EGF-like domain cysteine pattern signatures from about amino acid 198 to about amino acid 210, from about amino acid 230 to about amino acid 242, from about amino acid 262 to about amino acid 274. from about amino acid 294 to about amino acid 306, and from about ammo acid 326 to about amino acid 338. Clone DNA33094-1131 has been deposited with the ATCC on September 16, 1997 and is assigned ATCC deposit no. 209256.

Based on a BLAST and FastA sequence alignment analysis of the full-length sequence shown in Figure 20 (SEQ ID NO:46), PRO217 appears to be a novel EGF-like homologue.

EXAMPLE 14

Isolation of cDNA Clones Encoding Human PRO224

A consensus DNA sequence was assembled relative to other EST sequences using phrap as described in Example 1 above. This consensus sequence is designated herein as DNA30845. Based on the assembled DNA30845 consensus sequence, oligonucleotides were synthesized: 1) to identify by PCR a cDNA library that contained the sequence of interest, and 2) for use as probes to isolate a clone of the full-length coding sequence for PR0224.

A pair of PCR primers (forward and reverse) were synthesized:

forward PCR primer:

5'-AAGTTCCAGTGCCGCACCAGTGGC-3' (SEQ ID NO:52)

reverse PCR primer:

5'-TTGGTTCCACAGCCGAGCTCGTCG-3' (SEQ ID NO:53)

Additionally, a synthetic oligonucleotide hybridization probe was constructed from the DNA30845 consensus sequence which had the following nucleotide sequence:

hybridization probe:

5'-GAGGAGGAGTGCAGGATTGAGCCATGTACCCAGAAAGGGCAATGCCCACC-3' (SEQ ID NO:54)

In order to screen several libraries for a source of a full-length clone, DNA from the libraries was screened by PCR amplification with the PCR primer pair identified above. A positive library was then used to isolate clones encoding the PRO224 gene using the probe oligonucleotide and one of the PCR primers. RNA for construction of the cDNA libraries was isolated from human fetal liver tissue.

DNA sequencing of the isolated clones isolated as described above gave the full-length DNA sequence for DNA33221-1133 [Figure 2 1, SEQ ID NO:50]; and the derived protein sequence for PRO224.

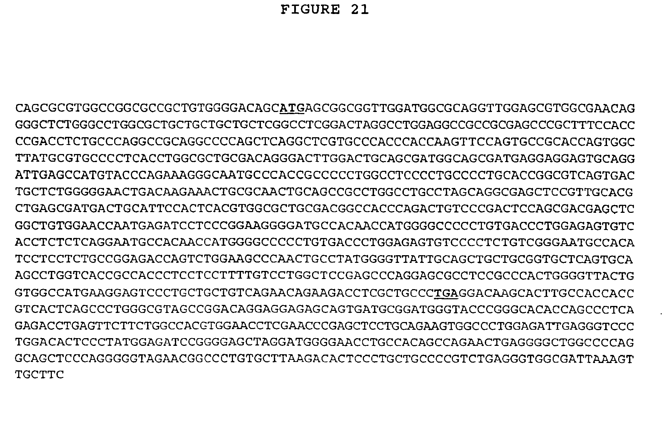

The entire coding sequence of DNA33221-1133 is included in Figure 21 (SEQ ID NO:50). Clone DNA33221-1133 contains a single open reading frame with an apparent translational initiation site at nucleotide positions 33-35. and an apparent stop codon at nucleotide positions 879-881. The predicted polypeptide precursor is 282 amino acids long with a molecular weight of approximately 28,991 daltons and an estimated pI of about 4.62. Analysis of the full-length PRO224 sequence shown in Figure 22 (SEQ ID NO:51) evidences the presence of a variety of important polypeptide domains, wherein the locations given for those important polypeptide domains are approximate as described above. Analysis of the full-length PRO224 polypeptide shown in Figure 22 evidences the presence of the following: a signal peptide from about amino acid 1 to about amino acid 30: a transmembrane domain from about amino acid 231 to about amino acid 248; N-glycosylation sites from about amino acid 126 to about amino acid 130, from about amino acid 195 to about amino acid 199, and from about amino acid 213 to about amino acid 17: N-myristoylation sites from about amino acid 3 to about amino acid 9. from about amino acid I 0 to about amino acid 16, from about amino acid 26 to about amino acid 32, from about amino acid 30 to about amino acid 36, from about amino acid 112 to about amino acid 118, from about amino acid 166 to about amino acid 172, from about amino acid 212 to about amino acid 218, from about amino acid 224 to about amino acid 230, from about amino acid 230 to about amino acid 236, and from about amino acid 263 to about amino acid 269; a prokaryotic membrane lipoprotein lipid attachment site from about amino acid 44 to about amino acid 55; and a leucine zipper pattern from about amino acid 17 to about amino acid 39. Clone DNA33221-1133 has been deposited with the ATCC on September 16, 1997 and is assigned ATCC deposit no. 209263.

Analysis of the amino acid sequence of the full-length PRO224 sequence suggests that it has homology to very low-density lipoprotein receptors, apolipoprotein E receptor and chicken oocyte receptor P95. Based on a BLAST and FastA sequence alignment analysis of the full-length sequence shown in Figure 22 (SEQ ID NO:51), PRO224 has amino acid identity to portions of these proteins in the range from 28% to 45%, and overall identity with these proteins of about 33%.

EXAMPLE 15

Isolation of cDNA Clones Encoding Human PRO231

A consensus DNA sequence was assembled relative to other EST sequences using phrap as described in Example 1 above. This consensus sequence is designated herein as DNA30933, wherein the encoded polypeptide showed some similarity to a putative acid phosphatase protein. Based on the assembled DNA30933 consensus sequence, oligonucleotides were synthesized: 1) to identify by PCR a cDNA library that contained the sequence of interest, and 2) for use as probes to isolate a clone of the full-length coding sequence for PRO231.

A pair of PCR primers (forward and reverse) were synthesized:

forward PCR primer I:

5'-CCAACTACCAAAGCTGCTGGAGCC-3' (SEQ ID NO:57)

forward PCR primer 2:

5'-GCAGCTCTATTACCACGGGAAGGA-3' (SEQ ID NO:58)

reverse PCR primer:

5'-TCCTTCCCGTGGTAATAGAGCTGC-3' (SEQ ID NO:59)

Additionally, a synthetic oligonucleotide hybridization probe was constructed from the DNA30933 consensus sequence which had the following nucleotide sequence:

hybridization probe:

5'-GGCAGAGAACCAGAGGCCGGAGGAGACTGCCTCTTTACAGCCAGG-3' (SEQ ID NO:60)

In order to screen several libraries for a source of a full-length clone. DNA from the libraries was screened by PCR amplification with the PCR primer pairs identified above. A positive library was then used to isolate clones encoding the PRO231 gene using the probe oligonucleotide and one of the PCR primers. RNA for construction of the cDNA libraries was isolated from human fetal liver tissue.

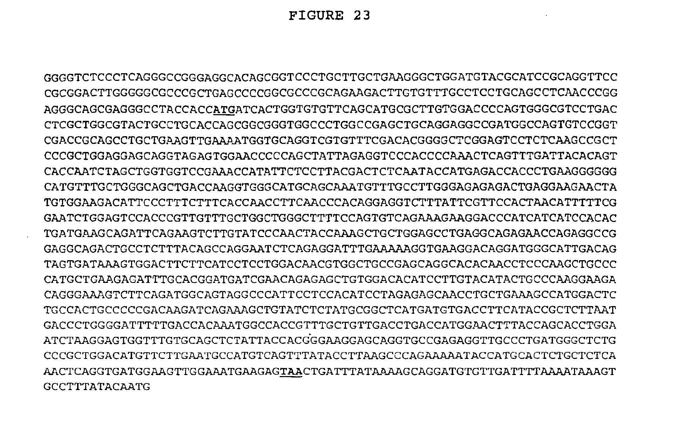

DNA sequencing of the isolated clones isolated as described above gave the full-length DNA sequence for DNA34434-1139 [Figure 23, SEQ ID NO:55]; and the derived protein sequence for PRO231.

The entire coding sequence of DNA34434-1139 is included in Figure 23 (SEQ ID NO:55). Clone DNA34434-1139 contains a single open reading frame with an apparent translational initiation site at nucleotide positions 173-175, and an apparent stop codon at nucleotide positions 1457-1459. The predicted polypeptide precursor is 428 amino acids long with a molecular weight of approximately 48.886 daltons and an estimated pl of about 6.39. Analysis of the full-length PRO231 sequence shown in Figure 24 (SEQ ID NO:56) evidences the presence of a variety of important polypeptide domains, wherein the locations given for those important polypeptide domains are approximate as described above. Analysis of the full-length PRO231 polypeptide shown in Figure 24 evidences the presence of the following: a signal peptide from about amino acid 1 to about amino acid 23; a cAMP-and cGMP-dependent protein kinase phosphorylation site from about amino acid 218 to about amino acid 222; a tyrosine kinase phosphorylation site from about amino acid 280 to about amino acid 288; N-myristoylation sites from about amino acid 15 to about amino acid 21, from about amino acid 117 to about amino acid 123, from about amino acid 118 to about amino acid 124, from about amino acid 179 to about amino acid 185, from about amino acid 240 to about amino acid 246. and from about amino acid 387 to about amino acid 393; an amidation site from about amino acid 216 to about amino acid 220; a leucine zipper pattern from about amino acid 10 to about amino acid 32; and a histidine acid phosphatases phosphohistidine signature from about amino acid 50 to about amino acid 65. Clone DNA34434-1139 has been deposited with the ATCC on September 16, 1997 and is assigned ATCC deposit no. 209252.

Analysis of the amino acid sequence of the full-length PRO23 1 sequence suggests that it possesses 30% and 31% amino acid identity with the human and rat prostatic acid phosphatase precursor proteins, respectively.

EXAMPLE 16

Isolation of cDNA Clones Encoding Human PRO235

A consensus DNA sequence was assembled relative to other EST sequences using phrap as described in Example I above. This consensus sequence is designated herein as DNA30927. Based on the assembled DNA30927 consensus sequence, oligonucleotides were synthesized: 1 ) to identify by PCR a cDNA library that contained the sequence of interest, and 2) for use as probes to isolate a clone of the full-length coding sequence for PRO235.

A pair of PCR primers (forward and reverse) were synthesized:

forward PCR primer:

5'-TGGAATACCGCCTCCTGCAG-3' (SEQ ID NO:63)

reverse PCR primer:

5'-CTTCTGCCCTTTGGAGAAGATGGC-3' (SEQ ID NO:64)

Additionally, a synthetic oligonucleotide hybridization probe was constructed from the DNA30927 consensus sequence which had the following nucleotide sequence:

hybridization probe:

5'-GGACTCACTGGCCCAGGCCTTCAATATCACCAGCCAGGACGAT-3' (SEQ ID NO:65)

In order to screen several libraries for a source of a full-length clone, DNA from the libraries was screened by PCR amplification with the PCR primer pair identified above. A positive library was then used to isolate clones encoding the PRO235 gene using the probe oligonucleotide and one of the PCR primers. RNA for construction of the cDNA libraries was isolated from human fetal liver tissue.

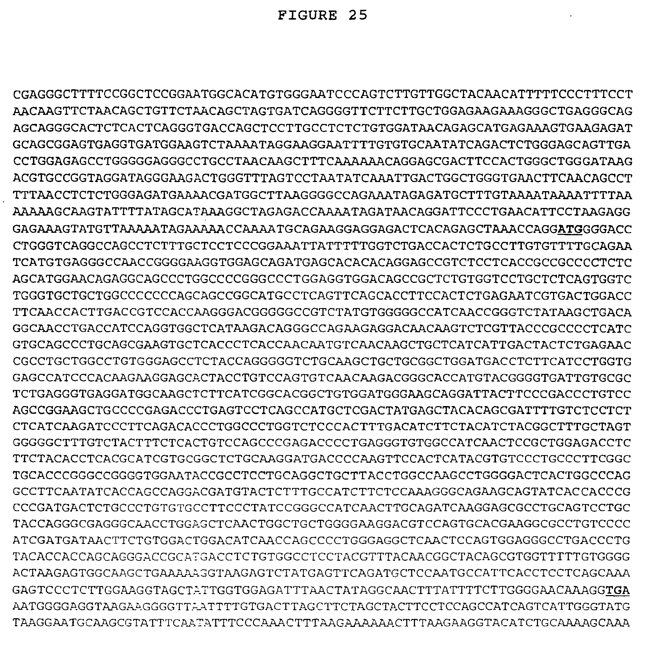

DNA sequencing of the isolated clones isolated as described above gave the full-length DNA sequence for DNA35558-1167 [Figure 25, SEQ ID NO:61]; and the derived protein sequence for PRO235.

The entire coding sequence of DNA35558-1167 is included in Figure 25 (SEQ ID NO:61). Clone DNA35558-1167 contains a single open reading frame with an apparent translational initiation site at nucleotide positions 667-669, and an apparent stop codon at nucleotide positions 2323-2325. The predicted polypeptide precursor is 552 amino acids long with a molecular weight of approximately 61,674 daltons and an estimated pl of about 6.95. Analysis of the full-length PRO235 sequence shown in Figure 26 (SEQ ID NO:62) evidences the presence of a variety of important polypeptide domains, wherein the locations given forthose important polypeptide domains are approximate as described above. Analysis of the full-length PRO235 polypeptide shown in Figure 26 evidences the presence of the following: a signal peptide from about amino acid 1 to about amino acid 32; a transmembrane domain from about amino acid 71 to about amino acid 86; N-glycosylation sites from about amino acid 130 to about amino acid 134, from about amino acid 145 to about amino acid 149, from about amino acid 217 to about amino acid 22 1, and from about amino acid 380 to about amino acid 385; N-myristoylation sites from about amino acid 220 to about amino acid 226, from about amino acid 319 to about amino acid 325, from about amino acid 353 to about amino acid 359, from about amino acid 460 to about amino acid 466, and from about amino acid 503 to about amino acid 509. Clone DNA35558-1 167 has been deposited with the ATCC on October 16, 1997 and is assigned ATCC deposit no. 209374.

Analysis of the amino acid sequence of the full-length PRO235 sequence shown in Figure 26 (SEQ ID NO: 62), suggests that portions of it possess significant homology to the human, mouse and *Xenopus* plexin protein, thereby indicating that PR0235 may be a novel plexin protein.

EXAMPLE 17

Isolation of cDNA Clones Encoding Human PRO245

A consensus DNA sequence was assembled relative to other EST sequences using phrap as described in Example 1 above. This consensus sequence is designated herein as DNA30954. wherein the polypeptide showed some structural homology to transmembrane protein receptor tyrosine kinase proteins. Based on the assembled DNA30954 consensus sequence, oligonucleotides were synthesized: 1) to identify by PCR a cDNA library that contained the sequence of interest, and 2) for use as probes to isolate a clone of the full-length coding sequence for PRO245.

A pair of PCR primers (forward and reverse) were synthesized:

forward PCR primer:

5'-ATCGTTGTGAAGTTAGTGCCCC-3' (SEQ ID NO:68)

reverse PCR primer:

5'-ACCTGCGATATCCAACAGAATTG-3' (SEQ ID NO:69)

Additionally, a synthetic oligonucleotide hybridization probe was constructed from the DNA30954 consensus sequence which had the following nucleotide sequence:

hybridization probe:

5'-GGAAGAGGATACAGTCACTCTGGAAGTATTAGTGGCTCCAGCAGTTCC-3' (SEQ ID NO:70)

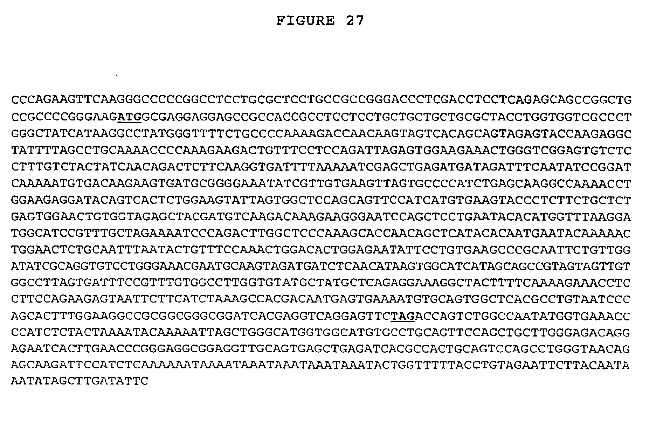

In order to screen several libraries for a source of a full-length clone, DNA from the libraries was screened by PCR amplification with the PCR primer pair identified above. A positive library was then used to isolate clones encoding the PRO245 gene using the probe oligonucleotide and one of the PCR primers. RNA for construction of the cDNA libraries was isolated from human fetal liver tissue.
DNA sequencing of the isolated clones isolated as described above gave the full-length DNA sequence for DNA35638-1141 [Figure 27, SEQ ID NO:66]; and the derived protein sequence for PRO245.
The entire coding sequence of DNA35638-1141 is included in Figure 27 (SEQ ID NO:66). Clone DNA35638-1141 contains a single open reading frame with an apparent translational initiation site at nucleotide positions 89-91, and an apparent stop codon at nucleotide positions 1025-1027. The predicted polypeptide precursor is 312 amino acids long with a molecular weight of approximately 34.554 daltons and an estimated pl of about 9.39. Analysis of the full-length PRO245 sequence shown in Figure 28 (SEQ ID NO:67) evidences the presence of a variety of important polypeptide domains, wherein the locations given for those important polypeptide domains are approximate as described above. Analysis of the full-length PRO245 polypeptide shown in Figure 28 evidences the presence of the following: a signal peptide from about amino acid 1 to about amino acid 20; a transmembrane domain from about amino acid 237 to about amino acid 258: N-glycosylation sites from about amino acid 98 to about amino acid 102, from about amino acid 187 to about amino acid 191, from about amino acid 236 to about amino acid 240, and from about amino acid 277 to about amino acid 281 ; N-myristoylation sites from about amino acid 182 to about amino acid 188, from about amino acid 239 to about amino acid 245, from about amino acid 255 to about amino acid 26 1, from about amino acid 257 to about amino acid 263, and from about amino acid 305 to about amino acid 311; and an amidation site from about amino acid 226 to about amino acid 230. Clone UNA35638-1141 has been deposited with the ATCC on September 16, 1997 and is assigned ATCC deposit no. 209265.
Analysis of the amino acid sequence of the full-length PRO245 sequence shown in Figure 28 (SEQ ID NO: 67), suggests that a portion of it possesses 60% amino acid identity with the human c-myb protein and, therefore, may be a new member of the transmembrane protein receptor tyrosine kinase family.

EXAMPLE 18

Isolation of cDNA Clones Encoding Human PRO261

A consensus DNA sequence was assembled relative to other EST sequences using phrap as described in Example 1 above. This consensus sequence is designated herein as DNA30843. Based on the assembled DNA30843 consensus sequence, oligonucleotides were synthesized: 1) to identify by PCR a cDNA library that contained the sequence of interest, and 2) for use as probes to isolate a clone of the full-length coding sequence for PRO261.
A pair of PCR primers (forward and reverse) were synthesized:

forward PCR primer:

5'-AAAGGTGCGTACCCAGCTGTGCC-3' (SEQ ID NO:73)

reverse PCR primer:

5'-TCCAGTCGGCAGAAGCGGTTCTGG-3' (SEQ ID NO:74)

Additionally, a synthetic oligonucleotide hybridization probe was constructed from the DNA30843 consensus sequence which had the following nucleotide sequence:

hybridization probe:

5'-CCTGGTGCTGGATGGCTGTGGCTGCTGCCGGGTATGTGCACGGCGGCTGGG-3' (SEQ ID NO:75)

In order to screen several libraries for a source of a full-length clone, DNA from the libraries was screened by PCR amplification with the PCR primer pair identified above. A positive library was then used to isolate clones encoding the PRO261 gene using the probe oligonucleotide and one of the PCR primers. RNA for construction of the cDNA libraries was isolated from human fetal lung tissue.

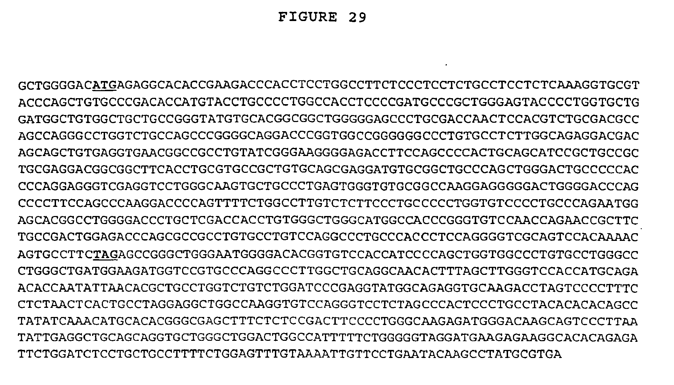

DNA sequencing of the isolated clones isolated as described above gave the full-length DNA sequence for DNA33473-1176 [Figure 29, SEQ ID NO:71]; and the derived protein sequence for PRO261.

The entire coding sequence of DNA33473-1176 is included in Figure 29 (SEQ ID NO:71). Clone DNA33473-1176 contains a single open reading frame with an apparent translational initiation site at nucleotide positions 10-12, and an apparent stop codon at nucleotide positions 760-762. The predicted polypeptide precursor is 250 amino acids long with a molecular weight of approximately 26.825 daltons and an estimated pI of about 8.36. Analysis of the full-length PRO261 sequence shown in Figure 30 (SEQ ID NO:72) evidences the presence of a variety of important polypeptide domains, wherein the locations given for those important polypeptide domains are approximate as described above Analysis of the full-length PRO261 polypeptide shown in Figure 30 evidences the presence of the following: a signal peptide from about amino acid 1 to about amino acid 23; N-myristoylation sites from about amino acid 3 to about amino acid 9, from about amino acid 49 to about amino acid 55, from about amino acid 81 to about amino acid 87, from about amino acid 85 to about amino acid 91, from about amino acid 126 to about amino acid 132, from about amino acid 164 to about amino acid 170, from about amino acid 166 to about amino acid 172, from about amino acid 167 to about amino acid 173, from about amino acid 183 to about amino acid 189, and from about amino acid 209 to about amino acid 215; an insulin-like growth factor binding protein signature from about amino acid 49 to about amino acid 65: a von Willebrand C1 domain from about amino acid 107 to about amino acid 124: a thrombospondin 1 homology block from about amino acid 201 to about amino acid 216; and an IGF binding protein site from about amino acid 49 to about amino acid 58. Clone DNA33473-1176 has been deposited with the ATCC on October 17, 1997 and is assigned ATCC deposit no. 209391.

Analysis of the amino acid sequence of the full-length PRO261 sequence shown in Figure 30 (SEQ 1D NO: 72), suggests that portions of it possess significant homology to CTGF, thereby indicating that PRO261 is a novel growth factor.

EXAMPLE 19

Isolation of cDNA Clones Encoding Human PRO269

A consensus DNA sequence was assembled relative to other EST sequences using phrap as described in Example 1 above. This consensus sequence is designated herein as DNA35705. Based on the assembled DNA35705 consensus sequence, oligonucleotides were synthesized: 1) to identify by PCR a cDNA library that contained the sequence of interest, and 2) for use as probes to isolate a clone of the full-length coding sequence for PR0269.

PCR primers (three forward and two reverse) were synthesized:

forward PCR primer 1:

5'-TGGAAGGAGATGCGATGCCACCTG-3' (SEQ ID NO:78)

forward PCR primer 2:

5'-TGACCAGTGGGGAAGGACAG-3' (SEQ ID NO:79)

forward PCR primer 3:

5'-ACAGAGCAGAGGGTGCCTTG-3' (SEQ ID NO:80)

reverse PCR primer 1

5'-TCAGGGACAAGTGGTGTCTCTCCC-3' (SEQ ID NO:81)

reverse PCR primer 2:

5'-TCAGGGAAGGAGTGTGCAGTTCTG-3' (SEQ ID NO:82)

Additionally, a synthetic oligonucleotide hybridization probe was constructed from the DNA35705 consensus sequence which had the following nucleotide sequence:

hybridization probe:

5'-ACAGCTCCCGATCTCAGTTACTTGCATCGCGGACGAAATCGGCGCTCGCT-3' (SEQ ID NO:83)

In order to screen several libraries for a source of a full-length clone. DNA from the libraries was screened by PCR amplification with the PCR primers identified above. A positive library was then used to isolate clones encoding the PRO269 gene using the probe oligonucleotide and one of the PCR primers. RNA for construction of the cDNA libraries was isolated from human fetal kidney tissue.

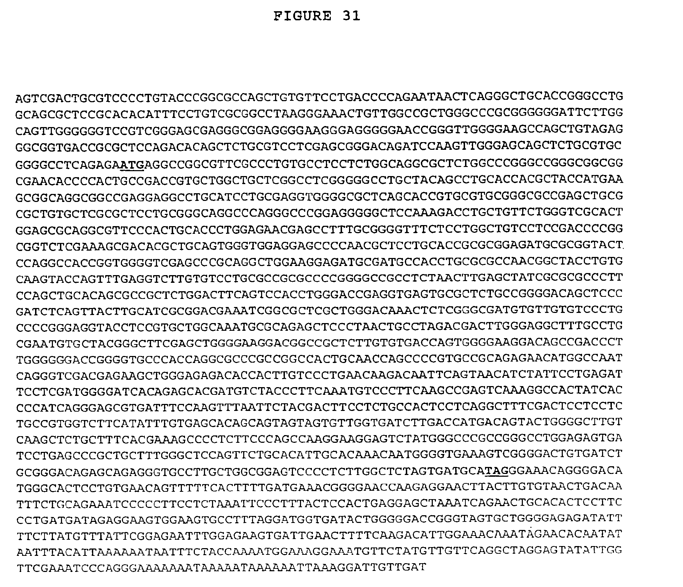

DNA sequencing of the isolated clones isolated as described above gave the full-length DNA sequence fur DNA38260-1180 [Figure 31. SEQ ID NO:76]; and the derived protein sequence for PRO269.

The entire coding sequence of DNA38260-1180 is included in Figure 31 (SEQ ID NO:76). Clone DNA38260-1180 contains a single open reading frame with an apparent translational initiation site at nucleotide positions 314-316, and an apparent stop codon at nucleotide positions 1784-1786. The predicted polypeptide precursor is 490 amino acids long with a molecular weight of approximately 51.636 daltons and an estimated p1 of about 6.29. Analysis of the full-length PRO269 sequence shown in Figure 32 (SEQ ID NO:77) evidences the presence of a variety of important polypeptide domains, wherein the locations given for those important polypeptide domains are approximate as described above. Analysis of the full-length PRO269 polypeptide shown in Figure 32 evidences the presence of the following: a signal peptide from about amino acid 1 to about amino acid 16; a transmembrane domain from about amino acid 397 to about amino acid 418; N-glycosylation sites from about amino acid 189 to about amino acid 193, and from about amino acid 381 to about amino acid 385; a glycosaminoglycan attachment site from about amino acid 289 to about amino acid 293; cAMP- and cGMP-dependent protein kinase phosphorylation sites from about amino acid 98 to about amino acid 102, and from about amino acid 434 to about amino acid 438; N-myristoylation sites from about amino acid 30 to about amino acid 36, from about amino acid 35 to about am ino acid 41, from about amino acid 58 to about am ino acid 64, from about amino acid 59 to about amino acid 65, from about amino acid 121 to about amino acid 127, from about amino acid 151 to about amino acid 157, from about amino acid 185 to about amino acid 191, from about amino acid 209 to about amino acid 215, from about amino acid 267 to about amino acid 273, from about amino acid 350 to about amino acid 356, from about amino acid 374 to about amino acid 380, from about amino acid 453 to about amino acid 459, from about amino acid 463 to about amino acid 469, and from about amino acid 477 to about amino acid 483; and an aspartic acid and asparagine hydroxylation site from about amino acid 262 to about amino acid 274. Clone DNA38260-1180 has been deposited with the ATCC on October 17, 1997 and is assigned ATCC deposit no. 209397.

Analysis of the amino acid sequence of the full-length PRO269 sequence shown in Figure 32 (SEQ ID NO: 77), suggests that portions of it possess significant homology to the human thrombomodulin proteins, thereby indicating that PRO269 may possess one or more thrombomodulin-like domains.

EXAMPLE 20

Isolation of cDNA Clones Encoding Human PRO287

A consensus DNA sequence was assembled relative to other EST sequences using phrap as described in Example 1 above. This consensus sequence is designated herein as DNA28728 or an extended consensus sequence encoding PRO287 was assembled and which showed similarity to type I procollagen C-proteinase enhancer protein and type I procollagen C-proteinase enhancer protein precursor. Based on the assembled DNA28729 consensus sequence, oligonucleotides were synthesized: 1) to identify by PCR a cDNA library that contained the sequence of interest, and 2) for use as probes to isolate a clone of the full-length coding sequence for PRO287.

A pair of PCR primers (forward and reverse) were synthesized:

forward PCR primer:

5'-CCGATTCATAGACCTCGAGAGT-3' (SEQ ID NO:86)

reverse PCR primer:

5'-GTCAAGGAGTCCTCCACAATAC-3' (SEQ ID NO:87)

Additionally, a synthetic oligonucleotide hybridization probe was constructed from the DNA28728 consensus sequence which had the following nucleotide sequence:

hybridization probe:

5'-GTGTACAATGGCCATGCCAATGGCCAGCGCATTGGCCGCTTCTGT-3' (SEQ ID NO:88)

In order to screen several libraries for a source of a full-length clone, DNA from the libraries was screened by PCR amplification with the PCR primer pair identified above. A positive library was then used to isolate clones encoding the PRO287 gene using the probe oligonucleotide and one of the PCR primers. RNA for construction of the cDNA libraries was isolated from human fetal kidney tissue.

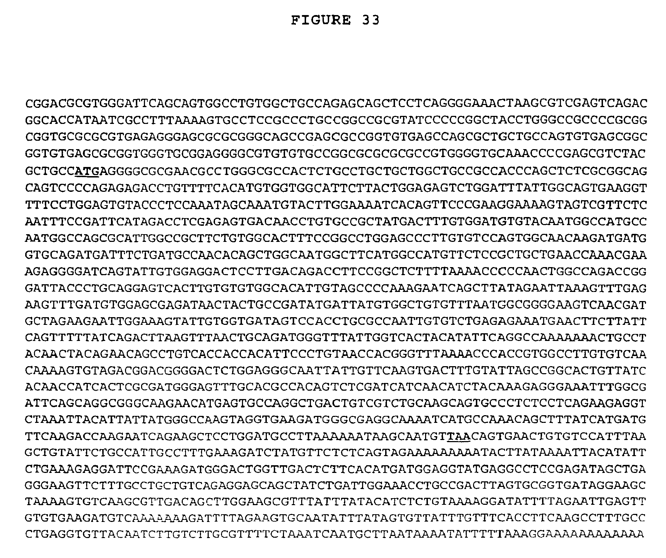

DNA sequencing of the isolated clones isolated as described above gave the full-length DNA sequence for DNA39969-1185 [Figure 33, SEQ ID NO:84]; and the derived protein sequence for PRO287.

The entire coding sequence of DNA39969-1185 is included in Figure 33 (SEQ ID NO:84). Clone DNA39969-1185 contains a single open reading frame with an apparent translational initiation site at nucleotide positions 307-309, and an apparent stop codon at nucleotide positions 1552-1554. The predicted polypeptide precursor is 415 amino acids long with a molecular weight of approximately 45,716 daltons and an estimated pI of about 8.89. Analysis of the full-length PRO287 sequence shown in Figure 34 (SEQ ID NO:85) evidences the presence of a variety of important polypeptide domains, wherein the locations given forthose important polypeptide domains are approximate as described above. Analysis of the full-length PRO287 polypeptide shown in Figure 34 evidences the presence of the following: a signal peptide from about amino acid 1 to about amino acid 23; an N-glycosylation site from about amino acid 355 to about amino acid 359; a tyrosine kinase phosphorylation site from about amino acid 199 to about amino acid 208; N-myristoylation sites from about amino acid 34 to about amino acid 40, from about amino acid 35 to about amino acid 41, from about amino acid 100 to about amino acid 106, from about amino acid 113 to about amino acid 119, from about amino acid 218 to about amino acid 224, from about amino acid 289 to about amino acid 295, from about amino acid 305 to about amino acid 311, from about amino acid 309 to about amino acid 315, from about amino acid 320 to about amino acid 326, and from about amino acid 330 to about amino acid 336: and a cell attachment sequence from about amino acid 149 to about amino acid 152. Clone DNA39969-1185 has been deposited with the ATCC on October 17, 1997 and is assigned ATCC deposit no. 209400.

Analysis of the amino acid sequence of the full-length PRO287 sequence shown in Figure 34 (SEQ ID NO: 85), suggests that it may possess one or more procollagen C-proteinase enhancer protein precursor or procollagen C-proteinase enhancer protein-like domains. Based on a BLAST and FastA sequence alignment analysis of the full-length sequence, PRO287 shows amino acid sequence identity to procollagen G-proteinase enhancer protein precursor and procollagen C-proteinase enhancer protein (47% and 54%, respectively).

EXAMPLE 21

Isolation of cDNA Clones Encoding Human PR0301

A consensus DNA sequence was assembled relative to other EST sequences using phrap as described in Example I above. This consensus sequence is designated herein as DNA35936. Based on the assembled DNA35936 consensus sequence, oligonucleotides were synthesized: 1) to identify by PCR a cDNA library that contained the sequence of interest, and 2) for use as probes to isolate a clone of the full-length coding sequence for PRO301.

PCR primers (three forward and two reverse) were synthesized:

forward PCR primer 1:

5'-TCGCGGAGCTGTGTTCTGTTTCCC-3' (SEQ ID NO:91)

forward PCR primer 2:

5'-ACACCTGGTTCAAAGATGGG-3' (SEQ ID NO:92)

forward PCR primer 3:

5'-TTGCCTTACTCAGGTGCTAC-3' (SEQ ID NO:93)

reverse PCR primer 1:

5'-TAGGAAGAGTTGCTGAAGGCACGG-3' (SEQ ID NO:94)

reverse PCR primer 2:

5'-ACTCAGCAGTGGTAGGAAAG-3' (SEQ ID NO:95)

Additionally, a synthetic oligonucleotide hybridization probe was constructed from the DNA35936 consensus sequence which had the following nucleotide sequence:

hybridization probe:

5'-TGATCGCGATGGGGACAAAGGCGCAAGCTCGAGAGGAAACTGTTGTGCCT-3'(SEQ ID NO:96)

In order to screen several libraries for a source of a full-length clone, DNA from the libraries was screened by PCR amplification with the PCR primers identified above. A positive library was then used to isolate clones encoding the PRO301 gene using the probe oligonucleotide and one of the PCR primers. RNA for construction of the cDNA libraries was isolated from human fetal kidney tissue.

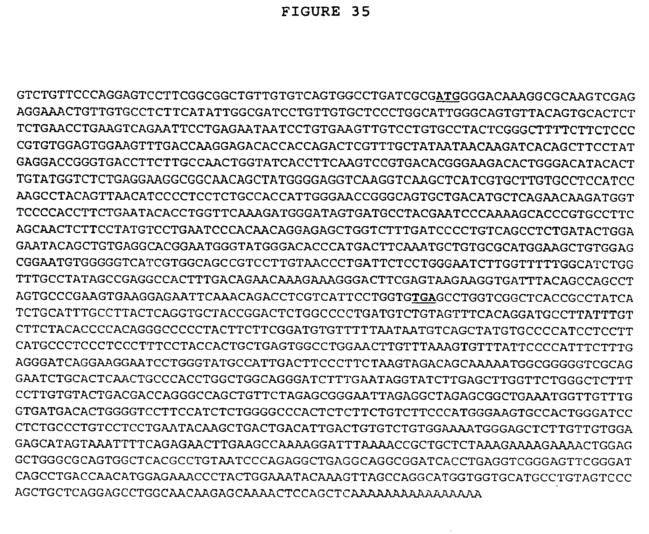

DNA sequencing of the isolated clones isolated as described above gave the full-length DNA sequence for DNA40628-1216 [Figure 35. SEQ ID NO:89]; and the derived protein sequence for PRO301.

The entire coding sequence of DNA40628-1216 is included in Figure 35 (SEQ ID NO:89). Clone DNA40628-1216 contains a single open reading frame with an apparent translational initiation site at nucleotide positions 52-54, and an apparent stop codon at nucleotide positions 949-95 1. The predicted polypeptide precursor is 299 amino acids long with a molecular weight of approximately 32,583 daltons and an estimated pI of about 8.29. Analysis of the full-length PRO301 sequence shown in Figure 36 (SEQ ID NO:90) evidences the presence of a variety of important polypeptide domains, wherein the locations given for those important polypeptide domains are approximate as described above. Analysis of the full-length PRO301 polypeptide shown in Figure 36 evidences the presence of the following: a signal peptide from about amino acid 1 to about amino acid 27; a transmembrane domain from about amino acid 235 to about amino acid 256; an N-glycosylation site from about amino acid 185 to about amino acid 189; a cAMP-and cGMP-dependent protein kinase phosphorylation site from about amino acid 270 to about amino acid 274; N-myristoylation sites from about amino acid 105 to about amino acid 111, from about amino acid 116 to about amino acid 122, from about amino acid 158 to about amino acid 164, from about amino acid 219 to about amino acid 225, from about amino acid 237 to about amino acid 243, and from about amino acid 256 to about amino acid 262. Clone DNA40628-1216 has been deposited with the ATCC on November 7, 1997 and is assigned ATCC deposit no. 209432.

Based on a BLAST and FastA sequence alignment analysis of the full-length PRO301sequence shown in Figure 36 (SEQ ID NO:90), PRO301 shows amino acid sequence identity to A33 antigen precursor (30%) and coxsackie and adenovirus receptor protein (29%).

EXAMPLE 22

Isolation of cDNA Clones Encoding Human PRO323

A consensus DNA sequence was assembled relative to other EST sequences using phrap as described in Example 1 above. This consensus sequence is designated herein as DNA30875. Based on the assembled DNA30875 consensus sequence, oligonucleotides were synthesized: 1) to identify by PCR a cDNA library that contained the sequence of interest, and 2) for use as probes to isolate a clone of the full-length coding sequence for PRO235.

PCR primers (two forward and one reverse) were synthesized:

forward PCR primer 1:

5'-AGTTCTGGTCAGCCTATGTGCC-3' (SEQ ID NO:99)

forward PCR primer 2:

5'-CGTGATGGTGTCTTTGTCCATGGG-3' (SEQ ID NO:100)

reverse PCR primer I :

5'-CTCCACCAATCCCGATGAACTTGG-3' (SEQ ID NO:101)

Additionally, a synthetic oligonucleotide hybridization probe was constructed from the DNA30875 consenSus sequence which had the following nucleotide sequence:

hybridization probe:

3'-GAGCAGATTGACCTCATACGCCGCATGTGTGCCTCCTATTCTGAGCTGGA-3' (SEQ ID NO:102)

In order to screen several libraries for a source of a full-length clone, DNA from the libraries was screened by PCR amplification with the PCR primer pairs identified above. A positive library was then used to isolate clones encoding the PRO323 gene using the probe oligonucleotide and one of the PCR primers. RNA for construction of the cDNA libraries was isolated from human fetal liver tissue (LIB6).

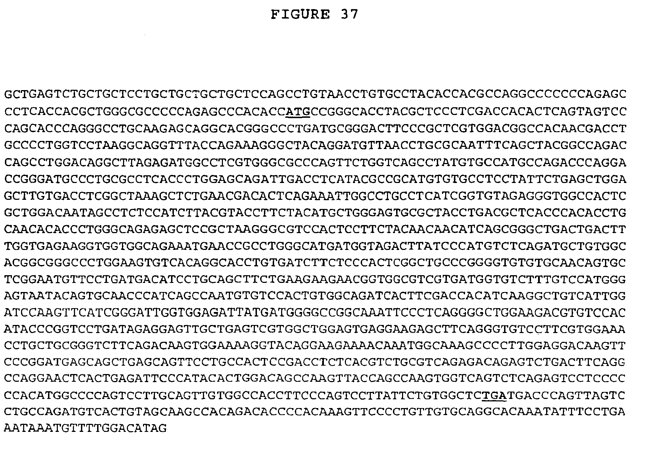

DNA sequencing of the isolated clones isolated as described above gave the full-length DNA sequence for DNA35595-1228 [Figure 37, SEQ ID NO:97]; and the derived protein sequence for PRO323. Additional probes were made from the DNA35595 sequence as follows for use in characterization of the DNA35595 sequence and for screening other cDNA libraries:

forward PCR primer:

5'-TGCTGCTGCTCCAGCCTGTAACC-3' (SEQ ID NO:103)

reverse PCR primer:

5'-CTGGCCGTAGCTGAAATTGCGC-3' (SEQ ID NO:104)

hYbridization probe:

5'-ACTCAGTAGTCCCAGCACCCAGGGCCTGCAAGAGCAGGCACGG-3' (SEQ ID NO:105)

The entire coding sequence of DNA35595-1228 is included in Figure 37 (SEQ ID NO:97). Clone DNA35595-1228 contains a single open reading frame with an apparent translational initiation site at nucleotide positions 110-112, and an apparent stop codon at nucleotide positions 1409-1411. The predicted polypeptide precursor is 433 amino acids long with a molecular weight of approximately 47,787 daltons and an estimated p1 of about 6.11. Analysis of the full-length PRO323 sequence shown in Figure 38 (SEQ ID NO:98) evidences the presence of a variety of important polypeptide domains, wherein the locations given forthose important polypeptide domains are approximate as described above. Analysis of the full-length PRO323 polypeptide shown in Figure 38 evidences the presence of the following: N-glycosylation sites from about amino acid 58 to about amino acid 62, from about amino acid 123 to about amino acid 127, from about amino acid 182 to about amino acid 186, and from about amino acid 273 to about amino acid 277; N-myristoylation sites from about amino acid 72 to about amino acid 78, from about amino acid 133 to about amino acid 139, from about amino acid 234 to about amino acid 240, from about amino acid 264 to about amino acid 270, from about amino acid 334 to about amino acid 340, and from about amino acid 389 to about amino acid 395; and a renal dipeptidase active site from about amino acid 134 to about amino acid 157. Clone DNA35595-1228 has been deposited with the ATCC on December 10, 1997 and is assigned ATCC deposit no. 209528.

Analysis of the amino acid sequence of the full-length PRO323 sequence shown in Figure 38 (SEQ ID NO: 98), suggests that portions of it possess significant homology to various dipeptidase proteins, thereby indicating that PRO323 may be a novel dipeptidase protein.

EXAMPLE 23

Isolation of cDNA Clones Encoding Human PRO331

A consensus DNA sequence was assembled relative to other EST sequences using phrap as described in Example 1 above. Based on the assembled DNA consensus sequence, oligonucleotides were synthesized: 1) to identify by PCR a cDNA library that contained the sequence of interest, and 2) for use as probes to isolate a clone of the full-length coding sequence for PRO331.

A pair of PCR primers (forward and reverse) were synthesized:

forward PCR primer:

5'-GCCTTTGACAACCTTCAGTCACTAGTGG-3' (SEQ ID NO:108)

reverse PCR primer:

5'-CCCCATGTGTCCATGACTGTTCCC-3' (SEQ ID NO:109)

Additionally, a synthetic oligonucleotide hybridization probe was constructed from the DNA consensus sequence which had the following nucleotide sequence:

hybridization probe:

5'-TACTGCCTCATGACCTCTTCACTCCCTTGCATCATCTTAGAGCGG-3' (SEQ ID NO:110)

In order to screen several libraries for a source of a full-length clone, DNA from the libraries was screened by PCR amplification with the PCR primer pair identified above. A positive library was then used to isolate clones encoding the PRO331 gene using the probe oligonucleotide and one of the PCR primers. RNA for construction of the cDNA libraries was isolated from human fetal brain tissue.

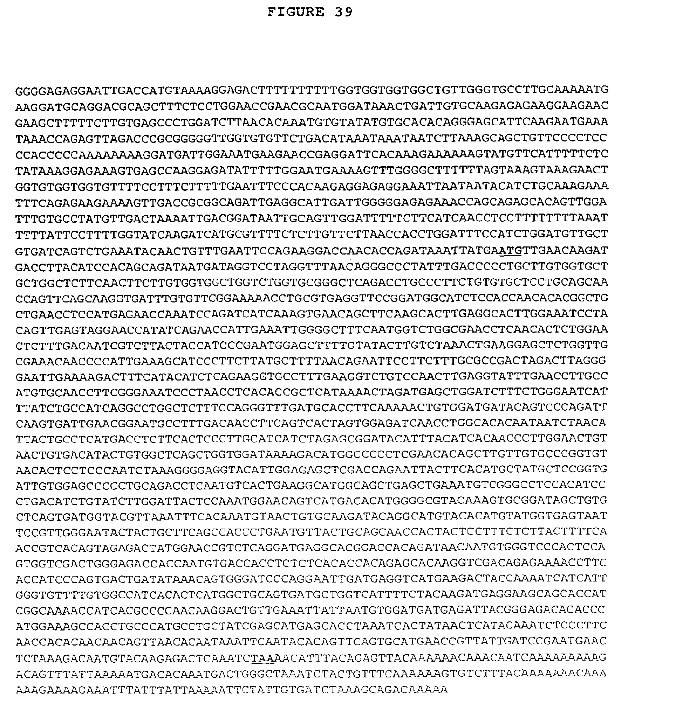

DNA sequencing of the isolated clones isolated as described above gave the full-length DNA sequence for DNA40981-1234 [Figure 39, SEQ ID NO:106]; and the derived protein sequence for PRO331.

The entire coding sequence of DNA40981-1234 is included in Figure 39 (SEQ ID NO:106). Clone DNA40981-1234 contains a single open reading frame with an apparent translational initiation site at nucleotide positions 812-814. and an apparent stop codon at nucleotide positions 2732-2734. The predicted polypeptide precursor is 640 amino acids long with a molecular weight of approximately 71,950 daltons and an estimated p1 of about 7.12. Analysis of the full-length PRO331 sequence shown in Figure 40 (SEQ ID NO:107) evidences the presence of a variety of important polypeptide domains, wherein the locations given for those important polypeptide domains are approximate as described above. Analysis of the full-length PRO331 polypeptide shown in Figure 40 evidences the presence of the following: a signal peptide from about amino acid I to about amino acid 44; a transmembrane domain from about amino acid 528 to about amino acid 543: N-glycosylation sites from about amino acid 278 to about amino acid 282, from about amino acid 364 to about amino acid 368, from about amino acid 390 to about amino acid 394, from about amino acid 412 to about amino acid 416, from about amino acid 415 to about amino acid 419, from about amino acid 434 to about amino acid 438, from about amino acid 442 to about amino acid 446, from about amino acid 488 to about amino acid 492, and from about amino acid 606 to about amino acid 610; a cAMP- and cGMP-dependent protein kinase phosphorylation site from about amino acid 183 to about amino acid 187; N-myristoy lation sites from about amino acid 40 to about amino acid 46. from about amino acid 73 to about amino acid 79. from about amino acid 118 to about amino acid 124, from about amino acid 191 to about amino acid 197. from about amino acid 228 to about amino acid 234, from about amino acid 237 to about amino acid 243, from about amino acid 391 to about amino acid 397, from about amino acid 422 to about amino acid 428, from about amino acid 433 to about amino acid 439, and from about amino acid 531 to about amino acid 537. Clone DNA40981-1234 has been deposited with the ATCC on November 7, 1997 and is assigned ATCC deposit no. 209439.

Analysis of the amino acid sequence of the full-length PR0331 sequence shown in Figure 40 (SEQ ID NO: 107), suggests that portions of it possess significant homology to the LIG-1 protein, thereby indicating that PRO331 may be a novel LIG-1-related protein.

EXAMPLE 24

Isolation of cDNA Clones Encoding Human PRO356

An expressed sequence tag (EST) DNA database (LIFESEQ®, Incyte Pharmaceuticals, Palo Alto, CA) was searched and an EST (#2939340) was identified that had homology to the TIE ligand family. To clone PRO356, a human fetal lung library prepared from mRNA purchased from Clontech, Inc., (Palo Alto, CA), catalog # 6528-1 was used, following the manufacturer's instructions.

The cDNA libraries used to isolate the cDNA clones encoding human PRO356 were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI.

Oligonucleotide probes based upon the above described EST sequence were then synthesized: 1 ) to identify by PCR a cDNA library that contained the sequence of interest, and 2) for use as probes to isolate a clone of the full-length coding sequence for PRO356. Forward and reverse PCR primers generally range from 20-30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubcl *et al.,* Current Protocols in Molecular Biology, *supra,* with the PCR primer pair. A positive library was then used to isolate clones encoding the gene of interest using the probe oligonucleotide and one of the primer pairs.

The oligonucleotide sequences used were as follows;

5'-TTCAGCACCAAGGACAAGGACAATGACAACT-3' (SEQ ID NO:113)

5'-TGTGCACACTTGTCCAAGCAGTTGTCATTGTC-3' (SEQ ID NO:114)

5'-GTAGTACACTCCATTGAGGTTGG-3' (SEQ ID NO:115)

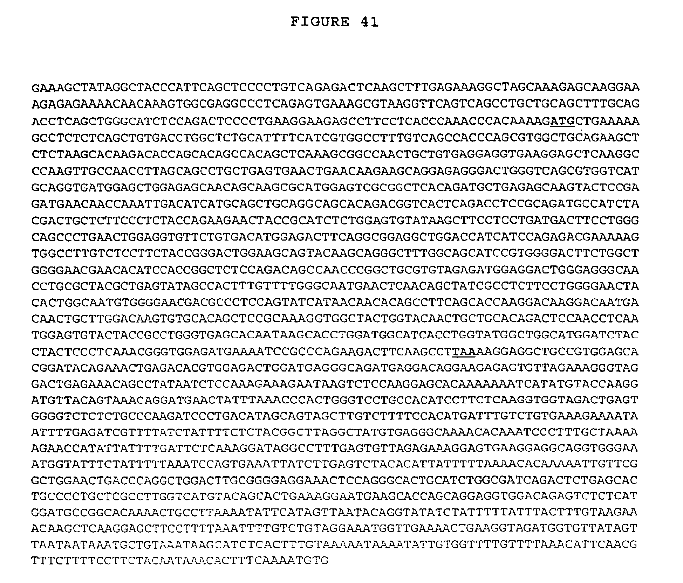

A cDNA clone was identified and sequenced in entirety. The entire nucleotide sequence of DNA47470-1130-P1 is shown in Figure 41 (SEQ ID NO:111). Clone DNA47470-1130-P1 contains a single open reading frame with an apparent translational initiation site at nucleotide positions 215-217, and a stop codon at nucleotide positions 1253-1255 (Figure 41; SEQ ID NO:111). The predicted polypeptide precursor is 346 amino acids long. The full-length PRO356 protein is shown in Figure 42 (SEQ ID NO:112).

Analysis of the full-length PRO356 sequence shown in Figure 42 (SEQ ID NO:112) evidences the presence of important polypeptide domains as shown in Figure 42, wherein the locations given for those important polypeptide domains are approximate as described above. Analysis of the full-length PRO356 sequence (Figure 42; SEQ ID NO: 112) evidences the presence of the following: a signal peptide from about amino acid I to about amino acid 26; N-glycosylation sites from about amino acid 58 to about amino acid 62, from about amino acid 253 to about amino acid 257, and from about amino acid 267 to about amino acid 271; a glycosaminoglycan attachment site from about amino acid 167 to about amino acid 171; a cAMP- and cGMP-dependent protein kinase phosphorylation site from about amino acid 176 to about amino acid 180; N-myristoylation sites from about amino acid 168 to about amino acid 174, from about amino acid 196 to about amno acid 202, from about amino acid 241 to about amino acid 247, from about amino acid 252 to about amino acid 258, from about amino acid 256 to about amino acid 262, and from about amino acid 327 to about amino acid 333; and a cell attachment sequence from about amino acid 199 to about amino acid 202.

Clone DNA47470-1130-PI has been deposited with ATCC on October 28, 1997 and is assigned ATCC deposit no. 209422. It is understood that the deposited clone has the actual correct sequence rather than the representations provided herein.

An analysis of the Dayhoff database (version 35.45 SwissProt 35), using the ALIGN-2 sequence alignment analysis of the full-length sequence shown in Figure 42 (SEQ ID NO:112), shows amino acid sequence identity between the PRO356 amino acid sequence and both TIE-2L1 (32%) and TIE-2L2 (34%). The abbreviation "TIE" is an acronym which stands for "tyrosine kinase containing 1g and EGF homology domains" and was coined to designate a new family of receptor tyrosine kinases.

EXAMPLE 25

Isolation of cDNA Clones Encoding Human PRO364

An expressed sequence tag (EST) DNA database (LIFESEQ®. Incyte Pharmaceuticals, Palo Alto, CA) was searched and an EST (Incyte EST no. 3003460) was identified that encoded a polypeptide which showed homology to members of the tumor necrosis factor receptor (TNFR) family of polypeptides.

A consensus DNA sequence was then assembled relative to Incyte EST no. 3003460 and other EST sequences using repeated cycles of BLAST (Altshul et al., Methods in Enzymology, 266:460-480 (1996)) and "phrap" (Phil Green, University of Washington. Seattle, Washington). This consensus sequence is herein designated "<consen01>", also designated herein as DNA44825. Based upon the DNA44825 and "<consen01>"consensus sequences, oligonucleotide probes were then synthesized: I to identify by PCR a cDNA library that contained the sequence of interest, and 2) for use is probes to isolate a clone of the full-length coding sequence for PRO364. Forward and reverse PCR primers generally range from 20-30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubel *et al.,* Current Protocols in Molecular Biology, *supra,* with the PCR primer pair. A positive library was then used to isolate clones encoding the gene of interest using the probe oligonucleotide and one of the primer pairs.
The oligonucleotide sequences used were as follows:

forward PCR primer (44825.f1):

5'-CACAGCACGGGGCGATGGG-3' (SEQ ID NO:118)

forward PCR primer (44825.f2):

5'-GCTCTGCGTTCTGCTCTG-3' (SEQ ID NO:119)

for-ward PCR primer (44825.GITR.f):

5'-GGCACAGCACGGGGCGATGGGCGCGTTT-3' (SEQ ID NO:120)

reverse PCR primer (44825.r1):

5'-CTGGTCACTGCCACCTTCCTGCAC-3' (SEQ ID NO:121)

reverse PCR primer (44825.r2):

5'-CGCTGACCCAGGCTGAG-3' (SEQ ID NO:122)

reverse PCR primer (44825.GITR.r):

5'-GAAGGTCCCCGAGGCACAGTCGATACA-3' (SEQ ID NO:123)

Additionally, synthetic oligonucleotide hybridization probes were constructed from the consensus DNA44825 sequence which had the following nucleotide sequences:

hybridization probe (44825.p1):

5'-GAGGAGTGCTGTTCCGAGTGGGACTGCATGTGTGTCCAGC-3' (SEQ ID NO:124)

hybridization probe (44825.GITR.p):

5'-AGCCTGGGTCAGCGCCCCACCGGGGGTCCCGGGTGCGGCC-3' (SEQ ID NO:125)

In order to screen several libraries for a source of a full-length clone, DNA from the libraries was screened by PCR amplification with the PCR primer pairs identified above. A positive library was then used to isolate clones encoding the PRO364 gene using the probe oligonucleotides and one of the PCR primers.

RNA for construction of the cDNA libraries was isolated from human small intestine tissue (LIB231). The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253: 1278-1280 (1991)) in the unique XhoI and NotI sites.

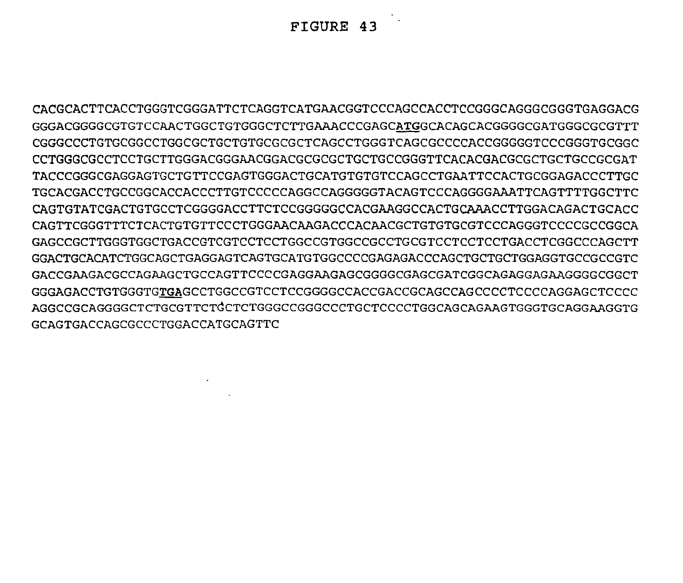

DNA sequencing of the clones isolated as described above gave the full-length DNA sequence for PRO364 [herein designated as DNA47365-1206]. The entire nucleotide sequence of DNA47365-1206 is shown in Figure 43 (SEQ ID NO: 116). Clone DNA47365-1206 contains a single open reading frame with an apparent translational initiation site at nucleotide positions 121-123, and a stop codon at nucleotide positions 844-846 (Figure 43; SEQ ID NO: 116). The predicted polypeptide precursor is 24 amino acids long, and has an estimated molecular weight of about 26,000 daltons, and a pI of about 6.34. The full-length PRO364 protein is shown in Figure 44 (SEQ ID NO:117).

Analysis of the full-length PRO364 sequence shown in Figure 44 (SEQ ID NO:117) evidences the presence of important polypeptide domains as shown in Figure 44, wherein the locations given for those important polypeptide domains are approximate as described above. Analysis of the full-length PRO364 sequence (Figure 44; SEQ ID NO: 117) evidences the presence of the following: a signal peptide from about amino acid 1 to about amino acid 25; a transmembrane domain from about amino acid 163 to about amino acid 183; an N-glycosylation site from about amino acid 146 to about amino acid 150; N-myristoylation sites from about amino acid 5 to about amino acid 11, from about amino acid 8 to about amno acid 14, from about amino acid 25 to about amino acid 31, from about amino acid 30 to about amino acid 36, from about amino acid 33 to about amino acid 39, from about amino acid 118 to about amino acid 124, from about amino acid 122 to about amino acid 128, and from about amino acid 156 to about amino acid 163; a prokaryotic membrane lipoprotein lipid attachment site from about amino acid 166 to about amino acid 177; and a leucine zipper pattern from about amino acid 171 to about amino acid 193. Clone DNA47365-1206 has been deposited with ATCC on November 7, 1997 and is assigned ATCC deposit no. 209436.

An analysis of the full-length PRO364 sequence shown in Figure 44 (SEQ ID NO: 117), suggests that portions of it possess significant homology to members of the tumor necrosis factor receptor family, thereby indicating that PRO364 may be a novel member of the tumor necrosis factor receptor family.

A detailed review of the amino acid sequence of the full-length PRO364 polypeptide and the nucleotide sequence that encodes that amino acid sequence evidences sequence homology with the mouse GITR protein reported by Nocenti et al., Proc. Natl. Acad. Sci. USA, 94:6216-6221 (1997). It is possible, therefore, that PRO364 represents the human counterpart to the mouse GITR protein reported by Nocentini *et al.*

EXAMPLE 26

Isolation of cDNA Clones Encoding Human PRO526

A consensus DNA sequence was assembled relative to other EST sequences using phrap as described in Example I above. An initial consensus DNA sequence was identified and herein is designated DNA39626.init. The initial consensus DNA sequence was extended using repeated cycles of BLAST and phrap to extend the initial consensus sequence as far as possible using the sources of EST sequences discussed above. The extended assembly sequence is herein designated <consen01>. Based on the assembled <consen01> DNA consensus sequence, oligonucleotides were synthesized: 1) to identify by PCR a cDNA library that contained the sequence-of interest, and 2) for use as probes to isolate a clone of the full-length coding sequence for PRO526.

A pair of PCR primers (forward and reverse) were synthesized:

forward PCR primer:

5'-TGGCTGCCCTGCAGTACCTCTACC-3' (SEQ ID NO: 128)

reverse PCR primer:

5'-CCCTGCAGGTCATTGGCAGCTAGG-3' (SEQ ID NO:129)

Additionally, a synthetic oligonucleotide hybridization probe was constructed from the <consen01>DNAconsensus sequence which had the following nucleotide sequence:

hybridization probe:

5'-AGGCACTGCCTGATGACACCTTCCGCGACCTGGGCAACCTCACAC-3' (SEQ 1D NO:130)

In order to screen several libraries for a source of a full-length clone, DNA from the libraries was screened by PCR amplification with the PCR primer pair identified above. A positive library was then used to isolate clones encoding the PRO526 gene using the probe oligonucleotide and one of the PCR primers. RNA for construction of the cDNA libraries was isolated from human fetal liver tissue (LIB228).

DNA sequencing of the isolated clones isolated as described above gave the full-length DNA sequence for DNA44184-1319 [Figure 45, SEQ ID NO:126]; and the derived protein sequence for PRO526.

The entire coding sequence of DNA44184-1319 is included in Figure 45 (SEQ ID NO:126). Clone DNA44184-1319 contains a single open reading frame with an apparent translational initiation site at nucleotide positions 514-516, and an apparent stop codon at nucleotide positions 1933-1935. The predicted polypeptide precursor is 473 amino acids long with a molecular weight of approximately 50,708 daltons and an estimated p1 of about 9.28. Analysis of the full-length PRO526 sequence shown in Figure 46 (SEQ ID NO:127) evidences the presence of a variety of important polypeptide domains, wherein the locations given for those important polypeptide domains are approximate as described above. Analysis of the full-length PRO526 polypeptide shown in Figure 46 evidences the presence of the following: a signal peptide from about amino acid 1 to about amino acid 26; a leucine zipper pattern from about amino acid 135 to about amino acid 157; a glycosaminoglycan attachment site from about amino acid 436 to about amino acid 440; N-glycosylation sites from about amino acid 82 to about amino acid 86, from about amino acid 179 to about amino acid 183, from about amino acid 237 to about amino acid 241, from about amino acid 372 to about amino acid 376, and from about amino acid 423 to about amino acid 427; and a von Willebrand Factor type C domain from about amino acid 411 to about amino acid 427. Clone DNA44184-1319 has been deposited with the ATCC on March 26, 1998 and is assigned ATCC deposit no. 209704.

Analysis of the amino acid sequence of the full-length PRO526 sequence shown in Figure 46 (SEQ ID NO: 127), suggests that portions of it possess significant homology to the leucine repeat rich proteins including ALS, SLIT, carboxypeptidase and platelet glycoprotein V, thereby indicating that PRO526 is a novel protein which is involved in protein-protein interactions.

EXAMPLE 27

Isolation of cDNA Clones Encoding Human PRO538

An expressed sequence tag (EST) DNA database (LIFESEQ®, Incyte Pharmaceuticals, Palo Alto. CA) was searched and an Incyte EST (INC3574209) was identified that had 61% identity to murine GFRα3 ("glial-cell-line-derived neurotrophic factor family receptor alpha"). To clone the corresponding full-length cDNA for PRO538. a panel of cDNA libraries were screened with primers:

newa3.F:

5'-GCCTCTCGCAGCCGGAGACC-3' (SEQ ID NO:133)

newa3.R:

5'-CAGGTGGGATCAGCCTGGCAC-3' (SEQ ID N0:134)

DNA from the libraries was screened by PCR amplification, as per Ausubel et al., Current Protocols in Molecular Biology (1995), with the PCR primer pair. A strong PCR product was identified in all libraries analyzed (fetal lung, fetal kidney, and placenta).

To isolate a cDNA clone encoding this protein, a human fetal lung-pRK5 vector library was selected and enriched for positive cDNA clones by extension of single stranded DNA from plasmid libraries grown in dug-/bung- host using the newa3.R primer. RNA for construction of the cDNA libraries was isolated from human fetal lung tissue.

The cDNA libraries used to isolate the cDNA clones encoding human PRO538 were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI. To enrich for positive cDNA clones the primer extension reaction contained 10 μl of 10x PCR Buffer (Perkin Elmer, USA), 1 μl dNTP (20 mM), 1 μl library DNA (200 ng), 1 μl primer, 86.5 μl $H_2O$ and 1 μl of Amplitaq (Perkin Elmer, USA) added after a hot start. The reaction was denatured for I minute at 95°C. annealed for 1 minute at 60°C, and then extended for 15 minutes at 72°C. The DNA was extracted with phenol/chloroform, precipitated with ethanol, and then transformed by electroporation into a DH10B host bacteria. The entire transformation mixture was plated onto 10 plates and colonies allowed to form. Colonies were lifted onto nylon membranes and screened with an oligonucleotide probe derived from the Incyte EST:

newa3.probe:

5'-TCTCGCAGCCGGAGACCCCCTTCCCACAGAAAGCCGACTCA-3' (SEQ ID NO:135)

Five positive clones were identified. Pure positive clones were obtained after colony purification and secondary screening. Two of the isolated clones were sequenced, designated herein as DNA48613 and DNA48614 (an alternatively spliced form of DNA48613).

The entire nucleotide sequence of DNA48613-1268 is shown in Figure 47 (SEQ ID NO:131). Clone DNA48613-1268 contains a single open reading frame with an apparent translational initiation site at nucleotide positions 38-40, and a stop codon at nucleotide positions 1238-1240 (Figure 47; SEQ ID NO: 131). The predicted polypeptide precursor is 400 amino acids long, with an estimated molecular weight of about 44.511 daltons and a pI of about 8.15.

The full-length PRO538 protein is shown in Figure 48 (SEQ ID NO:132).

Analysis of the full-length PRO538 sequence shown in Figure 48 (SEQ ID NO: 132) evidences the presence of important polypeptide domains as shown in Figure 48, wherein the locations given for those important polypeptide domains are approximate as described above. Analysis of the full-length PRO538 sequence (Figure 48; SEQ ID NO: 132) evidences the presence of the following: a signal peptide from about amino acid I to about amino acid 26; N-glycosylation sites from about amino acid 95 to about amino acid 99, from about amino acid 148 to about amino acid 152, and from about amino acid 309 to about amino acid 313; a CAMP- and cGMP-dependent protein kinase phosphorylation site from about amino acid 231 to about amino acid 235; N-myristoylation sites from about amino acid 279 to about amino acid 285, and from about amino acid 294 to about amino acid 300; and prokaryotic membrane lipoprotein lipid attachment sites from about amino acid 306 to about amino acid 317, and from about amino acid 379 to about amino acid 390. Clone DNA48613-1268 has been deposited with ATCC on April 7, 1998 and is assigned ATCC deposit no. 209752.

As discussed below, a sequence comparison of the protein encoded by DNA48613 to GFRα1 and GFRα2 indicated that the human protein PRO538 is a new member of the GFRα receptor family, and is a human homolog of murine GFRα3. Accordingly, DNA48613-1268encodes a protein designated as human GFRα3, and DNA48614 encodes its splice variant.

Amino acid sequence comparisons between GFRα family members are provided below, based on a BLAST-2 and FastA sequence alignment analysis of the full-length PRO538 polypeptide (shown in Figure 48; SEQ ID NO:132):

Sequence Identity Between Members of the GFRα Family

| Proteins Compared | Percent Identity |
|---|---|
| rGFRα1 versus hGFRα1 | 92% |
| rGFRα2 versus hGFRα2 | 94% |
| mGFRα3 versus hGFRα3 | 77% |
| | |
| hGFRα3 versus hGFRα1 | 34% |
| hGFRα3 versus hGFRα2 | 34% |
| hGFRα1 versus hGFRα2 | 48% |

From the sequence comparisons it can be seen that human GFRα3 is less related to its rodent homolog than is either GFRα1 or GFRα2. In addition. GFRα3 appears to be more distantly related to GFRα I and GFRα2 than GFRα1 and GFRα2 are to each other.

EXAMPLE 28

Isolation of cDNA Clones Encoding Human PRO713

An expressed sequence tag (EST) DNA database (LIFESEQ®, Incyte Pharmaceuticals. Palo Alto, CA) was searched and an EST (Incyte EST no. 1302516) was identified that encoded a polypeptide which showed homology to VEGF. Probes based on the the Incvte EST no. 1302516 were used to screen a cDNA library derived from the human glioma cell line G61. In particular, Incyte clone INC1302516 was used to generate the following four probes:

5'-ACTTCTCAGTGTCCATAAGGG-3' (SEQ ID NO:138)
5'-GAACTAAAGAGAACCGATACCATTTTCTGGCCAGGTTGTC-3' (SEQ ID NO:139)
5'-CACCACAGCGTTTAACCAGG (SEQ ID NO:140)
5'-ACAACAGGCACAGTTCCCAC-3' (SEQ ID NO:141)

Nine positives were identified and characterized. Three clones contained the full coding region and were identical in sequence. Partial clones were also identified from a fetal lung library and were identical with the glioma-derived sequence with the exception of one nucleotide change which did not alter the encoded amino acid.

DNA sequencing ofthe clones isolated as described above gave the full-length DNA sequence for PRO713 [herein designated as DNA29101-1122]. The entire nucleotide sequence of DNA29101-1122 is shown in Figure 49 (SEQ ID NO: 136). Clone DNA29101-1122 contains a single open reading frame with an apparent translational initiation site at nucleotide positions 285-287, and a stop codon at nucleotide positions 1320-1322 (Figure 49; SEQ ID NO:136). The predicted polypeptide precursor is 345 amino acids long, and has an estimated molecular weight of about 39,029 daltons, and a pl of about 6.06. The full-length PRO713 protein is shown in Figure 50 (SEQ ID NO:137).

Analysis of the full-length PRO713 sequence shown in Figure 50 (SEQ ID NO:137) evidences the presence of important polypeptide domains as shown in Figure 50, wherein the locations given for those important polypeptide domains are approximate as described above. Analysis of the full-length PRO713 sequence (Figure 50; SEQ ID NO: 137) evidences the presence of the following: a signal peptide from about amino acid 1 to about amino acid 14; N-glycosylation sites from about amino acid 25 to about amino acid 29, from about amino acid 55 to about amino acid 59, and from about amino acid 254 to about amino acid 258: N-myristoylation sites from about amino acid 15 to about amino acid 21. from about amino acid 117 to about amno acid 123, from about amino acid 127 to about amino acid 133. from about amino acid 281 to about amino acid 287, from about amino acid 282 to about amino acid 288, and from about amino acid 319 to about amino acid 325: and an amidation site from about amino acid 229 to about amino acid 233. Clone DNA29101-1122 has been deposited with ATCC on March 5, 1998 and is assigned ATCC deposit no. 209653.
An analysis of the full-length PRO713 sequence shown in Figure 50 (SEQ ID NO:137), suggests that it is a novel VEGF-related protein (VEGF-E).

EXAMPLE 29

Isolation of cDNA Clones Encoding Human PRO719

A consensus DNA sequence was assembled relative to other EST sequences using phrap as described in Example I above. This consensus sequence is designated herein as DNA44851. which also corresponds exactly to Incyte EST clone no. 179903. Based on the DNA44851 consensus sequence, oligonucleotides were synthesized: 1) to identify by PCR a cDNA library that contained the sequence of interest, and 2) for use as probes to isolate a clone of the full-length coding sequence for PRO719
A pair of PCR primers (forward and reverse) were synthesized:

forward PCR primer (44851.f1):

5'-GTGAGCATGAGCGAGCCGTCCAC-3' (SEQ ID NO:144)

reverse PCR primer (44851.r1):

5'-GCTATTACAACGGTTCTTGCGGCAGC-3' (SEQ ID NO:145)

Additionally, a synthetic oligonucleotide hybridization probe was constructed from the consensus DNA44851 sequence which had the following nucleotide sequence:

hybridization probe (44851.p1):

5'-TTGACTCTCTGGTGAATCAGGACAAGCCGAGTTTTGCCTTCCAG-3' (SEQ ID NO:146)

In order to screen several libraries for a source of a full-length clone, DNA from the libraries was screened by PCR amplification with the PCR primer pair identified above. A positive library was then used to isolate clones encoding the PRO719 gene using the probe oligonucleotide and one of the PCR primers. RNA for construction of the cDNA libraries was isolated from human placenta tissue (LIB90).
DNA sequencing of the isolated clones isolated as described above gave the full-length DNA sequence for DNA49646-1327 [Figure 51. SEQ ID NO:142]; and the derived protein sequence for PRO719.
The entire coding sequence of DNA49646-1327 is included in Figure 51 (SEQ ID NO:142). Clone DNA49646-1327 contains a single open reading frame with an apparent translational initiation site at nucleotide positions 223-225. and an apparent stop codon at nucleotide positions 1285-1287. The predicted polypeptide precursor is 354 amino acids long, and has an estimated molecular weight of about 39,362 daltons and a p1 of about 8.35. Analysis of the full-length PRO719 sequence shown in Figure 52 (SEQ ID NO:143) evidences the presence of a variety of important polypeptide domains, wherein the locations given for those important polypeptide domains are approximate as described above. Analysis of the full-length PRO719 polypeptide shown in Figure 52 evidences the presence of the following: a signal peptide from about amino acid I to about amino acid 16; N-glycosylation sites from about amino acid 80 to about amino acid 84. and from about amino acid 136 to about amino acid 140: cAMP-and cGMP-dependent protein kinase phosphorylation sites from about amino acid 206 to about amino acid 210, and from about amino acid 329 to about amino acid 333: N-myristoylation sites from about amino acid 63 to about amino acid 69, from about amino acid 96 to about amino acid 102, from about amino acid 171 to about amino acid 177, from about amino acid 191 to about amino acid 197, from about amino acid 227 to about amino acid 233. from about amino acid 251 to about amino acid 257, from about amino acid 306 to about amino acid 312, and from about amino acid 346 to about amino acid 352: and a lipases, serine active site from about amino acid 163 to about amino acid 173. Clone DNA49646-1327 has been deposited with the ATCC on March 26, 1998 and is assigned ATCC deposit no. 209705

Analysis of the amino acid sequence of the full-length PRO719 polypeptide suggests that it possesses significant sequence similarity to the lipoprotein lipase H protein, thereby indicating that PRO719 may be a novel, lipoprotein lipase homolog. More specifically, an analysis of the Dayhoff database (version 35.45 SwissProt 35) evidenced significant homology between the PRO719 amino acid sequence and the following Dayhoff sequences: LIPL_HUMAN, LIPH_HUMAN, D83548_1, A24059_1, P_R30740. D88666_1, A43357, A46696, B43357 and A49488.

EXAMPLE 30

Isolation of cDNA Clones Encoding Human PRO771

A consensus DNA sequence was assembled relative to other EST sequences using phrap as described in Example I above. This consensus sequence is designated herein as <consen01>. Based on the <consen01> DNA consensus sequence, oligonucleotides were synthesized: l) to identify by PCR a cDNA library that contained the sequence of interest, and 2) for use as probes to isolate a clone of the full-length coding sequence for PR0771.

A pair of PCR primers (forward and reverse) were synthesized:

forward PCR primer:

5'-CAGCAATATTCAGAAGCGGCAAGGG-3' (SEQ ID NO:149)

reverse PCR primer:

5'-CATCATGGTCATCACCACCATCATCATC-3' (SEQ ID NO:150)

Additionally, a synthetic oligonucleotide hybridization probe was constructed from the <consen01> DNA consensus sequence which had the following nucleotide sequence:

hybridization probe:

5'-GGTTACTACAAGCCAACACAATGTCATGGCAGTGTTGGACAGTGCTGG-3' (SEQ ID NO:151)

In order to screen several libraries for a source of a full-length clone, DNA from the libraries was screened by PCR amplification with the PCR primer pair identified above. A positive library was then used to isolate clones encoding the PRO771 gene using the probe oligonucleotide and one of the PCR primers. RNA for construction of the cDNA libraries was isolated from human fetal kidney tissue (LIB28).

DNA sequencing of the isolated clones isolated as described above gave the full-length DNA sequence for DNA49829-1346 [Figure 53, SEQ ID NO:147]; and the derived protein sequence for PRO771.

The entire coding sequence of DNA49829-1346 is included in Figure 53 (SEQ ID NO:147). Clone DNA49829-1346 contains a single open reading frame with an apparent translational initiation site at nucleotide positions 134-136, and an apparent stop codon at nucleotide positions 1442-1444. The predicted polypeptide precursor is 436 amino acids long, and has an estimated molecular weight of about 49,429 daltons and a pl of about 4.80. Analysis of the full-length PRO771 sequence shown in Figure 54 (SEQ ID NO:148) evidences the presence of a variety of important polypeptide domains, wherein the locations given for those important polypeptide domains are approximate as described above. Analysis of the full-length PRO771 polypeptide shown in Figure 54 evidences the presence of the following: a signal peptide from about amino acid 1 to about amino acid 16; a cAMP- and cGMP-dependent protein kinase phosphorylation site from about amino acid 115 to about amino acid 119; a tyrosine kinase phosphorylation site from about amino acid 62 to about amino acid 70; N-myristoylation sites from about amino acid 357 to about amino acid 363, from about amino acid 371 to about amino acid 377, and from about amino acid 376 to about amino acid 382; and a leucine zipper pattern from about amino acid 246 to about amino acid 268. Clone DNA49829-1346 has been deposited with the ATCC on April 7, 1998 and is assigned ATCC deposit no. 209749.

Analysis ofthe amino acid sequence ofthe full-length PRO771 polypeptide suggests that portions of it possess significant sequence homology to the testican protein, thereby indicating that PRO771 may be a novel testican homologue.

EXAMPLE 31

Isolation of cDNA Clones Encoding Human PRO788

A consensus DNA sequence was assembled relative to other EST sequences using phrap as described in Example I above. This consensus sequence is designated herein as "<consen01>". Based on the data provided herein, Incyte EST 2777282 was identified which showed homology to ARS and E48 anitigen. Based on the assembled "<consen01>'' sequence and other data provided herein, Incyte EST 2777282 was obtained and sequenced in full which gave the full-length DNA sequence for PRO788 herein designated as DNA56405-1357 (Figure 55; SEQ ID NO:152), and the derived protein sequence for PRO788.

The entire coding sequence of DNA56405-1357 is included in Figure 55 (SEQ ID NO:152). Clone DNA56405-1357 contains a single open reading frame with an apparent translational initiation site at nucleotide positions 84-86, and an apparent stop codon at nucleotide positions 459-461. The predicted polypeptide precursor is 125 amino acids long, and has an estimated molecular weight of about 13,115 daltons and a pl of about 5.90. Analysis of the full-length PRO788 sequence shown in Figure 56 (SEQ ID NO:153) evidences the presence of a variety of important polypeptide domains, wherein the locations given for those important polypeptide domains are approximate as described above. Analysis of the full-length PRO788 polypeptide shown in Figure 56 evidences the presence of the following: a signal peptide from about amino acid 1 to about amino acid 17; an N-glycosylation site from about amino acid 46 to about amino acid 50; N-myristoylation sites from about amino acid 3 to about amino acid 9, from about amino acid 33 to about amino acid 39, and from about amino acid 84 to about amino acid 90: and a prokaryotic membrane lipoprotein lipid attachment site from about amino acid 6 to about amino acid 17. Clone DNA56405-1357 has been deposited with the ATCC on May 6, 1998 and is assigned ATCC deposit no. 209849.

EXAMPLE 32

Isolation of cDNA Clones Encoding Human PRO792

A consensus DNA sequence was assembled relative to other EST sequences using phrap as described in Example 1 above. This consensus sequence is designated herein as DNA38106, which also corresponds exactly to Incyte EST clone no. 1988930. Based on the DNA38106 consensus sequence, oligonucleotides were synthesized: 1) to identify by PCR a cDNA library that contained the sequence of interest, and 2) for use as probes to isolate a clone of the full-length coding sequence for PR0792.

A pair of PCR primers (forward and reverse) were synthesized:

fonvard PCR primer (38106.fl):

5'-GCGAGAACTGTGTCATGATGCTGC-3' (SEQ ID NO:156)

reverse PCR primer (38106.rl):

5'-GTTTCTGAGACTCAGCAGCGGTGG-3' (SEQ ID NO:157)

Additionally, a synthetic oligonucleotide hybridization probe was constructed from the consensus DNA38106 sequence which had the following nucleotide sequence:

hybridization probe (38106.pl):

5'-CACCGTGTGACAGCGAGAAGGACGGCTGGATCTGTGAGAAAAGGCACAAC-3' (SEQ ID NO:158)

In order to screen several libraries for a source of a full-length clone, DNA from the libraries was screened by PCR amplification with the PCR primer pair identified above. A positive library was then used to isolate clones encoding the PRO792 gene using the probe oligonucleotide and one of the PCR primers. RNA for construction of the cDNA libraries was isolated from human bone marrow tissue (LIB255).

DNA sequencing of the isolated clones isolated as described above gave the full-length DNA sequence for DNA56352-1358 [Figure 57, SEQ ID NO:154]; and the derived protein sequence for PRO792.

The entire coding sequence of DNA56352-1358 is included in Figure 57 (SEQ ID NO:154). Clone DNA56352-1358 contains a single open reading frame with an apparent translational initiation site at nucleotide positions 67-69, and an apparent stop codon at nucleotide positions 946-948. The predicted polypeptide precursor is 293 amino acids long, and has an estimated molecular weight of about 32,562 daltons and a pI of about 6.53. Analysis of the full-length PRO792 sequence shown in Figure 58 (SEQ ID NO:155) evidences the presence of a variety of important polypeptide domains, wherein the locations given for those important polypeptide domains are approximate as described above. Analysis of the full-length PRO792 polypeptide shown in Figure 58 evidences the presence of the following: a signal peptide from about amino acid 1 to about amino acid 46; a possible type II transmembrane domain from about amino acid 31 to about amino acid 54; N-glycosylation sites from about amino acid 73 to about amino acid 77, and from about amino acid 159 to about amino acid 163; N-myristoylation sites from about amino acid 18 to about amino acid 24, from about amino acid 133 to about amino acid 139, and from about amino acid 242 to about amino acid 248, a C-type lectin domain signature from about amino acid 264 to about amino acid 288; and a leucine zipper pattern from about amino acid 102 to about amino acid 124. Clone DNA56352-1358 has been deposited with the ATCC on May 6, 1998 and is assigned ATCC deposit no. 209846.

Analysis of the amino acid sequence of the full-length PRO792 polypeptide suggests that it possesses significant sequence similarity to the CD23 protein, thereby indicating that PRO792 may be a novel CD23 homolog. More specifically, an analysis of the Dayhoff database (version 35.45 SwissProt 35) evidenced significant homology between the PRO792 amino acid sequence and the following Dayhoff sequences: S34198, A07100-1, A05303_1, P_R41689. P_P82839. A10871_1, P_R12796, P_R47199, A46274 and P_R32188.

EXAMPLE 33

Isolation of cDNA Clones Encoding Human PRO812

DNA59205-1421 was identified by applying the proprietary signal sequence finding algorithm described in Example 3 above. Use of the above described signal sequence algorithm allowed identification of an EST cluster sequence from the LIFESEQ® database, designated Incyte EST cluster no. 170079. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology. 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA55721.

In light of the sequence homology between the DNA55721 sequence and Incyte EST no. 388964, Incyte EST no. 388964 was purchased and the cDNA insert was obtained and sequenced. The sequence of this cDNA insert is shown in Figure 59 (SEQ ID NO:159) and is herein designated as DNA59205-1421.

The entire coding sequence of DNA59205-1421 is included in Figure 59 (SEQ ID NO:159). Clone DNA59205-1421 contains a single open reading frame with an apparent translational initiation site at nucleotide positions 55-57 and ending at the stop codon at nucleotide positions 304-306 (Figure 59). The predicted polypeptide precursor is 83 amino acids long (Figure 60; SEQ ID NO:160). The full-length PRO812 protein shown in Figure 60 has an estimated molecular weight of about 9,201 daltons and a p1 of about 9.30. Analysis of the full-length PRO812 sequence shown in Figure 60 (SEQ ID NO:160) evidences the presence of a variety of important polypeptide domains, wherein the locations given for those important polypeptide domains are approximate as described above. Analysis of the full-length PRO812 sequence shown in Figure 60 evidences the presence of the following: a signal peptide from about amino acid I to about amino acid 15; and a cAMP- and cGMP-dependent protein kinase phosphorylation site from about amino acid 73 to about amino acid 77. Clone DNA59205-1421 has been deposited with ATCC on June 23, 1998 and is assigned ATCC deposit no. 203009.

An analysis of the Dayhoffdatabase (version 35.45 SwissProt 35), using a WU-BLAST2 sequence alignment analysis of the full-length sequence shown in Figure 60 (SEQ ID NO:160), evidenced significant homology between the PRO812 amino acid sequence and the following Dayhoff sequences: P_W35802, P_W35803, PSC1_RAT, S68231, GEN13917, PSC2_RAT, CC10_HUMAN, UTER_RABIT, AF008595_1, and A56413.

EXAMPLE 34

Isolation of cDNA Clones Encoding Human PRO865

A cDNA sequence isolated in the amylase screen described in Example 2 above is herein designated DNA37642 or DNA37642.init. The DNA37642 sequence was then compared to a variety of expressed sequence tag (EST) databases which included public EST databases (*e.g.,* GenBank) and a proprietary EST DNA database (LIFESEQ®, Incyte Pharmaceuticals. Palo Alto, CA) to identify homologies there between. The homology search was performed using the computer program BLAST or BLAST2 (Altshul et al., Methods in Enzymology, 266:46-480 (1996)). Those comparisons resulting in a BLAST score of 70 (or in some cases 90) or greater that did not encode known proteins were clustered and assembled into consensus DNA sequences with the program "phrap" (Phil Green, University of Washington, Seattle, Washington). The assembly and the consensus sequence obtained is herein designated DNA48615.

Based on the DNA48615 consensus sequence, probes were generated from the sequence of the DNA48615 molecule and used to screen a human fetal kidney (LIB227) library prepared as described in paragraph 1 of Example 2 above. The cloning vector was pRK5B (pRK5B is a precursor of pRK5D that does not contain the SfiI *site; see,* Holmes et al., Science, 253:1278-1280 (1991)), and the cDNA size cut was less than 2800 bp.

PCR primers (forward and reverse) were synthesized:

forward PCR primer I (48615.f1):

5'-AAGCTGCCGGAGCTGCAATG-3' (SEQ ID NO:163)

forward PCR primer 2 (48615.f2):

5'TTGCTTCTTAATCCTGAGCGC-3' (SEQ ID NO:164)

forward PCR primer 3 (48615.f3):

5'-AAAGGAGGACTTTCGACTGC-3' (SEQ ID NO:165)

reverse PCR primer 1 (48615.r1):

5'-AGAGATTCATCCACTGCTCCAAGTCG-3' (SEQ ID NO:166)

reverse PCR primer 2 (48615.r2):

5'-TGTCCAGAAACAGGCACATATCAGC-3' (SEQ ID NO:167)

Additionally, a synthetic oligonucleotide hybridization probe was constructed from the consensus DNA48615 sequence which had the following nucleotide sequence:

hybridization probe (48615.p1):

5'-AGACAGCGGCACAGAGGTGCTTCTGCCAGGTTAGTGGTTACTTGGATGAT-3' (SEQ ID NO:168)

In order to screen several libraries for a source of full-length clone, DNA from the libraries was screened by PCR amplification with the PCR primer pairs identified above. A positive library was then used to isolate clones encoding the PRO865 gene using the probe oligonucleotide and one of the PCR primers.

A full length clone [DNA53974-1401] was identified that contained a single open reading frame with an apparent translational initiation site at nucleotide positions 173-175, and it stop signal at nucleotide positions 1577-1579 (Figure 61: SEQ ID NO:161). The predicted pulypeptide precursor is 468 amino acids long, and has a calculated molecular weight of approximately 54.393 daltons and an estimated pI of approximately 5.63. Analysis of the full-length PRO865 sequence shown in Figure 62 (SEQ ID NO:162) evidences the presence of a variety of important polypeptide domains as shown in Figure 62, wherein the locations given for those important polypeptide domains are approximate as described above. Analysis of the full-length PRO865 polypeptide shown in Figure 62 evidences the presence of the following: a signal peptide from about aminn acid 1 to about amino acid 23; N-glycosylation sites from about amino acid 280 to about amino acid 284. and from about amino acid 384 to about amino acid 388; an amidation site from about amino acid 94 to about amino acid 98; glycosaminoglycan attachment sites from about amino acid 20 to about amino acid 24, and from about amino acid 223 to about amino acid 227; an aminotransferases class-V pyridoxal-phosphate site from about amino acid 216 to about amino acid 223; and an interleukin-7 protein site from about amino acid 338 to about amino acid 344. Clone DNA53974-1401 was deposited with the ATCC on April 14, 1998 , and is assigned ATCC deposit no. 209774.

Analysis of the amino acid sequence of the full-length PRO865 (Figure 62; SEQ ID NO:162) polypeptide suggests that it possesses no significant similarity to any known protein. However, an analysis of the Dayhoff database (version 35.45 SwissProt 35) evidenced some degree of homology between the PRO865 amino acid sequence and the following Dayhoff sequences: YMN0_YEAST, ATFCA4_43, S44168, P_W14549 and RABTCRG4_1.

EXAMPLE 35

Isolation of cDNA Clones Encoding Human PRO1075

A consensus DNA sequence was assembled relative to other EST sequences using phrap as described in Example 1 above. This consensus sequence is designated herein as DNA34363. ESTs proprietary to Genentech were employed in the consensus assembly. The Genentech ESTs are herein designated DNA13059 and DNA19463. Based on the DNA34363 consensus sequence, oligonucleotides were synthesized: 1) to identify by PCR a cDNA library that contained the sequence of interest, and 2) for use as probes to isolate a clone of the full-length coding sequence for PRO 1075.

PCR primers (forward and reverse) were synthesized:

forward PCR primer (34363.f1):

5'-TGAGAGGCCTCTCTGGAAGTTG-3' (SEQ ID NO:171)

forward PCR primer (34363.f2):

5'-GTCAGCGATCAGTGAAAGC-3' (SEQ ID NO:172)

forward PCR primer (34363.f3):

5'-CCAGAATGAAGTAGCTCGGC-3' (SEQ ID NO:173)

forward PCR primer (34363.f4):

5'-CCGACTCAAAATGCATTGTC-3' (SFQ ID NO:174)

forward PCR primer (34363.f5):

5'-CATTTGGCAGGAATTGTCC-3' (SEQ ID NO:175)

forward PCR primer (34363.f6):

5'-GGTGCTATAGGCCAAGGG-3' (SEQ ID NO:176)

reverse PCR primer (34363.r1):

5'-CTGTATCTCTGGGCTATGTCAGAG-3' (SEQ ID NO:177)

reverse PCR primer (34363.r2):

5'-CTACATATAATGGCACATGTCAGCC-3' (SEQ ID NO:178)

Additionally, a synthetic oligonucleotide hybridization probe was constructed from the consensus DNA34363 sequence which had the following nucleotide sequence:

hybridization probe (34363.p1):

5'-CGTCTTCCTATCCTTACCCGACCTCAGATGCTCCCTTCTGCTCCTG-3' (SEQ ID NO:179)

In order to screen several libraries for a source of a full-length clone, DNA from the libraries was screened by PCR amplification with the PCR primer pairs identified above. A positive library was then used to isolate clones encoding the PRO1075 gene using the probe oligonucleotide and one of the PCR primers. RNA for construction of the cDNA libraries was isolated from human skin tumor tissue (LIB324).

DNA sequencing of the isolated clones isolated as described above gave the full-length DNA sequence for DNA57689-1385 [Figure 63, SEQ ID NO:169]; and the derived protein sequence for PRO1075.

The entire coding sequence of DNA57689-1385 is included in Figure 63 (SEQ ID NO:169). Clone DNA57689-1385 contains a single open reading frame with an apparent translational initiation site at nucleotide positions 137-139, and an apparent stop codon at nucleotide positions 1355-1357. The predicted polypeptide precursor is 406 amino acids long, and has an estimated molecular weight of about 46,927 daltons and a pl of about 5.21. Analysis of the full-length PRO1075 sequence shown in Figure 64 (SEQ ID NO:170) evidences the presence of a variety of important polypeptide domains, wherein the locations given for those important polypeptidedomains are approximate as described above. Analysis of the full-length PRO1075 polypeptide shown in Figure 64 evidences the presence of the following: a signal peptide from about amino acid I to about amino acid 29: a tyrosine kinase phosphorylation site from about amino acid 203 to about amino acid 212: a N-myristoylation site from about amino acid 225 to about amino acid 231; and an endoplasmic reticulum targeting sequence from about amino acid 403 to about amino acid 408. Clone DNA57689-1385 has been deposited with the ATCC on May 14, 1998 and is assigned ATCC deposit no. 209869.

Analysis of the amino acid sequence of the full-length PRO1075 polypeptide suggests that it possesses significant sequence similarity to protein disulfide isomerase, thereby indicating that PRO1075 may he a novel protein disulfide isomerase. More specifically, an analysis of the Dayhoff database (version 35.45 SwissProt 35) evidenced significant homology between the PRO1075 amino acid sequence and the following Dayhoff sequences: CELC30H7_2, CELC06A6_3, CELF42G8_3, S57942, ER72 CAEEL, CELC07A12_3, CEH06O01_4 and P_R51696.

EXAMPLE 36

Isolation of cDNA Clones Encoding Human PRO1126

DNA606 15-1483 was identified by applying the proprietary signal sequence finding algorithm described in Example 3 above. Use of the above described signal sequence algorithm allowed identification of an EST cluster sequence from the LIFESEQ® database, designated Incyte EST cluster no. 121249. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA56250.

In light of the sequence homology between the DNA56250 sequence and Incyte EST clone no. 1437250, Incyte EST no. 1437250 was purchased and the cDNA insert was obtained and sequenced. The sequence of this cDNA insert is shown in Figure 65 (SEQ ID NO:180) and is herein designated as DNA60615-1483.

The entire coding sequence of DNA60615-1483 is included in Figure 65 (SEQ ID NO:180). Clone DNA60615-1483 contains a single open reading frame with an apparent translational initiation site at nucleotide positions 110-112 and ending at the stop codon at nucleotide positions 1316-1318 (Figure 65). The predicted polypeptide precursor is 402 amino acids long (Figure 66; SEQ ID NO:181). The full-length PRO1126 protein shown in Figure 66 has an estimated molecular weight of about 45,921 daltons and a pl of about 8.60. Analysis of the full-length PRO 1126 sequence shown in Figure 66 (SEQ ID NO:181) evidences the presence of a variety of important polypeptide domains, wherein the locations given for those important polypeptide domains are approximate as described above. Analysis of the full-length PRO1126 sequence shown in Figure 66 evidences the presence of the following: a signal peptide from about amino acid 1 to about amino acid 25; and N-glycosylation sites from about amino acid 66 to about amino acid 70, from about amino acid 138 to about amino acid 142, and from about amino acid 183 to about amino acid 187. Clone DNA60615-1483 has been deposited with ATCC on June 16, 1998 and is assigned ATCC deposit no. 209980.

An analysis of the Dayhoff database (version 35.45 SwissProt 35), using a WU-BLAST2 sequence alignment analysis of the full-length sequence shown in Figure 66 (SEQ ID NO:181), evidenced significant homology between the PRO1126 amino acid sequence and the following Dayhoff sequences: 173636, NOMR_HUMAN. MMUSMYOC3_1, HS454G6_1, P_R98225, RNU78105_1, RNU72487_1, AF035301_1, CEELC48E7_4, and CEF11C3_3.

EXAMPLE 37

Isolation of cDNA Clones Encoding Human PRO1130

A consensus DNA sequence was assembled relative to other EST sequences using phrap as described in Example 1 above. This consensus sequence is designated herein as DNA34360. Based on the DNA34360 consensus sequence, oligonucleotides were synthesized: 1) to identify by PCR a cDNA library that contained the sequence of interest, and 2) for use as probes to isolate a clone of the full-length coding sequence fur PRO1130.

PCR primers (forward and reverse) were synthesized:

forward PCR primer (34360.f1):

5'-GCCATAGTCACGACATGGATG-3' (SEQ ID NO:184)

forward PCR primer (34360.f2):

5'-GGATGGCCAGAGCTGCTG-3' (SEQ ID NO:185)

forward PCR primer (34360.f3):

5'-AAAGTACAAGTGTGGCCTCATCAAGC-3' (SEQ ID NO:186)

reverse PCR primer (34360.r1):

5'-TCTGACTCCTAAGTCAGGCAGGAG-3' (SEQ ID NO:187)

reverse PCR primer (34363.r2):

5'-ATTCTCTCCACAGACAGCTGGTTC-3' (SEQ ID NO:188)

Additionally, a synthetic oligonucleotide hybridization probe was constructed from the consensus DNA34360 sequence which had the following nucleotide sequence:

hybridization probe (34360.pl):

5'-GTACAAGTGTGGCCTCATCAAGCCCTGCCCAGCCAACTACTTTGCG-3' (SEQ ID NO:189)

In order to screen several libraries for a source of a full-length clone, DNA from the libraries was screened by PCR amplification with the PCR primer pairs identified above. A positive library was then used to isolate clones encoding the PRO1130 gene using the probe oligonucleotide and one of the PCR primers. RNA for construction of the cDNA libraries was isolated from human aortic endothelial cell tissue.

DNA sequencing of the isolated clones isolated as described above gave the full-length DNA sequence for DNA59814-1486 [Figure 67, SEQ ID NO:182]; and the derived protein sequence for PRO1130.

The entire coding sequence of DNA59814-1486 is included in Figure 67 (SEQ ID NO:182). Clone DNA59814-1486 contains a single open reading frame with an apparent translational initiation site at nucleotide positions 312-314, and an apparent stop codon at nucleotide positions 984-986. The predicted polypeptide precursor is 224 amino acids long, and has an estimated molecular weight of about 24,963 daltons and a pl of about 9.64. Analysis of the full-length PRO 1130 sequence shown in Figure 68 (SEQ ID NO:183) evidences the presence ofa variety of important polypeptide domains, wherein the locations given for those important polypeptide domains are approximate as described above. Analysis of the full-length PRO1130 polypeptide shown in Figure 68 evidences the presence of the following: a signal peptide from about amino acid 1 to about amino acid 15; an ATP/GTP-binding site motif A (P-loop) from about amino acid 184 to about amino acid 192; and an N-glycosylation site from about amino acid 107 to about amino acid 111. Clone DNA59814-1486 has been deposited with the ATCC on October 28. 1998 and is assigned ATCC deposit no. 203359.

An analysis of the Dayhoff database (version 35.45 SwissProt 35), using a WU-BLAST2 sequence alignment analysis of the full-length sequence shown in Figure 68 (SCQ ID NO:183), evidenced significant homology between the PRO1130 amino acid sequence and the following Dayhoff sequences: P_W06347, 216_ HUMAN, D87120_1, MMU72677_1, LAU04889 1, and D69319.

EXAMPLE 38

Isolation of cDNA Clones Encoding Human PRO 1154

DNA59846-1503 was identified by applying the proprietary signal sequence finding algorithm described in Example 3 above. Use of the above described signal sequence algorithm allowed identification of an EST cluster sequence from the LIFESEQ® database. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA56250.

In light of the discoveries provided herein, the Incyte clone which included EST 2169375 (library 309-ENDCNOT03-from a microvascular endothelial cell library) was purchased and further examined and sequenced. The sequence of this cDNA insert is shown in Figure 69 (SEQ ID NO:190) and is herein designated as DNA59846-1503.

The entire coding sequence of DNA59846-1503 is included in Figure 69 (SEQ ID NO:190). Clone DNA59846-1503 contains a single open reading frame with an apparent translational initiation site at nucleotide positions 86-88 and ending at the stop codon at nucleotide positions 2909-291 (Figure 69). The predicted polypeptide precursor is 941 amino acids long (Figure 70; SEQ ID NO:191). The full-length PRO1154 protein shown in Figure 70 has an estimated molecular weight of about 107,144 daltons and a pl of about 6.26. Analysis of the full-length PRO1154 sequence shown in Figure 70 (SEQ ID NO:191) evidences the presence of a variety of important polypeptide domains, wherein the locations given for those important polypeptide domains are approximate as described above. Analysis of the full-length PRO1154 sequence shown in Figure 70 evidences the presence of the following: a signal peptide from about amino acid I to about amino acid 34; N-glycosylation sites from about amino acid 70 to about amino acid 74, from about amino acid 154 to about amino acid 158, from about amino acid 414 to about amino acid 418, from about amino acid 760 to about amino acid 764, and from about amino acid 901 to about amino acid 905; and a neutral zinc metallopeptidases, zinc-binding region signature from about amino acid 350 to about amino acid 360. Clone DNA59846-1503 has been deposited with ATCC on June 16, 1998 and is assigned ATCC deposit no. 209978.

An analysis of the Dayhoff database (version 35.45 SwissProt 35), using a WU-BLAST2 sequence alignment analysis of the full-length sequence shown in Figure 70 (SEQ ID NO:191), evidenced sequence identity between the PRO1154 amino acid sequence and the following Dayhoff sequences: AB011097_1, AMPN_HUMAN, RNU76997_1, 159331, GEN14047, HSU62768_1, P_R51281, CET07F10_1, SSU66371_1, and AMPRE_HUMAN.

EXAMPLE 39

Isolation of cDNA Clones Encoding Human PRO1244

DNA64883-1526 was identified by applying the proprietary signal sequence finding algorithm described in Example 3 above. Use of the above described signal sequence algorithm allowed identification of an EST cluster sequence from the LIFESEQ® database, designated Incyte EST cluster no. 7874. This EST cluster sequence was then compared to a variety of expressed sequence tag (EST)'databases which included public EST databases (*e.g.*, GenBank) and a proprietary EST DNA database (LIFESEQ®. Incyte Pharmaceuticals, Palo Alto, CA) to identify existing homologies. One or more of the ESTs was derived from a library constructed from tissue of the corpus cavernosum. The homology search was performed using the computer program BLAST or BLAST2 (Altshul et al., Methods in Enzymology, 266: 460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The assembly included EST sequences designated "DNA 18313", "DNA22812", and Incyte EST no. 3202349. The consensus sequence obtained therefrom is herein designated as DNA56011.

In light of the sequence homology between the DNA56011 sequence and Incyte EST clone no. 3202349, Incyte EST no. 3202349 was purchased and the cDNA insert was obtained and sequenced. The sequence of this cDNA insert is shown in Figure 71 (SEQ ID NO:192) and is herein designated as DNA64883-1526.

The entire coding sequence of DNA64883-1526 is included in Figure 71 (SEQ ID NO:192). Clone DNA64883-1526 contains a single open reading frame with an apparent translational initiation site at nucleotide positions 9-11 and ending at the stop codon at nucleotide positions 1014-1016 (Figure 71). The predicted polypeptide precursor is 335 amino acids long (Figure 72; SEQ ID NO:193). The full-length PRO1244 protein shown in Figure 72 has an estimated molecular weight of about 38,037 daltons and a pl of about 9.87. Analysis ofthe full-length PRO1244 sequence shown in Figure 72 (SEQ ID NO:193) evidences the presence of a variety of important polypeptide domains, wherein the locations given for those important polypeptide domains are approximate as described above. Analysis of the full-length PRO1244 sequence shown in Figure 72 evidences the presence of the following: a signal peptide from about amino acid 1 to about amino acid 29: transmembrane domains from about amino acid 183 to about amino acid 205, from about amino acid 217 to about amino acid 237. from about amino acid 217 to about amino acid 287, and from about amino acid 301 to about amino acid 321; N-glycosylation sites from about amino acid 71 to about amino acid 75. and from about amino acid 215 to about amino acid 219: and a cell attachment sequence from about amino acid 150 to about amino acid 153. Clone DNA64883-1526 has been deposited-with ATCC on September 9, 1998 and is assigned ATCC deposit no. 203253

An analysis of the Dayhoff database (version 35.45 SwissProt 35), using a WU-BLAST2 sequence alignment analysis of the full-length sequence shown in Figure 72 (SEQ ID NO:193), revealed homology between the PRO1244 amino acid sequence and the following Dayhoff sequence: AF008554_1, P_485334, G02297. HUMN33S11_1, HUMN33S10_1, YO13_CAEEL, GEN13255, S49758. E70107, and ERP5_MEDSA.

EXAMPLE 40

Isolation of cDNA Clones Encoding Human PRO1246

DNA64885-1529 was identified by applying the proprietary signal sequence finding algorithm described in Example 3 above. Use of the above described signal sequence algorithm allowed identification of an EST cluster sequence from the LIFESEQ® database, designated Incyte EST cluster no. 56853. This EST cluster sequence was then compared to a variety of expressed sequence tag (EST) databases which included public EST databases ( *e.g.,* GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA56021.

In light of the sequence homology between the DNA56021 sequence and Incyte EST clone no. 2481345, Incyte EST no. 2481345 was purchased and the cDNA insert was obtained and sequenced. The sequence of this cDNA insert is shown in Figure 73 (SEQ ID NO:194) and is herein designated as DNA64885-1529.

The entire coding sequence of DNA64885-1529 is included in Figure 73 (SEQ ID NO:194). Clone DNA64885-1529 contains a single open reading frame with an apparent translational initiation site at nucleotide positions 119-121 and ending at the stop codon at nucleotide positions 1727-1729 (Figure 73). The predicted polypeptide precursor is 536 amino acids long (Figure 74; SEQ ID NO:195). The full-length PRO1246 protein shown in Figure 74 has an estimated molecular weight of about 61,450 daltons and a pl of about 9.17. Analysis of the full-length PRO1246 sequence shown in Figure 74 (SEQ ID NO:195) evidences the presence of a variety of important polypeptide domains, wherein the locations given for those important polypeptide domains are approximate as described above. Analysis of the full-length PRO 1246 sequence shown in Figure 74 evidences the presence of the following: a signal peptide from about amino acid 1 to about amino acid 15; N-glycosylation sites from about amino acid 108 to ahout amino acid 112, from about amino acid 166 to about amino acid 170. from about amino acid 193 to about amino acid 197, from about amino acid 262 to about amino acid 266, from about amino acid 375 to about amino acid 379, from about amino acid 413 to about amino acid 417, and from about amino acid 498 to about amino acid 502; and sulfatase proteins homology blocks from about amino acid 286 to about amino acid 3 17. from about amino acid 359 to about amino acid 370, and from about amino acid 78 to about amino acid 98. Clone DNA64885-1529 has been deposited with ATCC on November 3, 1998 and is assigned ATCC deposit no. 203457.

An analysis of the Dayhoff database (version 35.45 SwissProt 35), using a WU-BLAST2 sequence alignment analysis of the full-length sequence shown in Figure 74 (SEQ ID NO:195), evidenced significant homology between the PRO1246 amino acid sequence and the following Dayhoff sequences: P_R51355. CELK09C4_1, BCU44852 1.1DS_HUMAN,G65169,E64903,ARSA_HUMAN,GL6S_HUMAN,HSARSF_I, and GEN12648.

EXAMPLE 41

Isolation of cDNA Clones Encoding Human PRO1274

A novel secreted molecule, DNA57700, was used to BLAST against Incyte's (LIFESEQ®, Incyte Pharmaceuticals, Palo Alto, CA) proprietary database and GenBank's public database. Positive clones were identified and used to generate assembly files by seqext program. The homology search was performed using the computer program BLAST or BLAST2 (Altshul et al., Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA59573.

Based on the DNA59573 consensus sequence and its relation to sequences identified in the assembly, one of the clones (Incyte EST clone no. 2623992), including one of the sequences in the assembly was purchased and the cDNA insert was obtained and sequenced. Incyte clone 2623992 came from a library constructed of RNA from epidermal breast keratinocytes. The sequence of this cDNA insert is shown in Figure 75 (SEQ ID NO:196) and is herein designated as DNA64889-1541.

The entire coding sequence of DNA64889-1541 is included in Figure 75 (SEQ ID NO:196). Clone DNA64889-1541 contains a single open reading frame with an apparent translational initiation site at nucleotide positions 24-26 and ending at the stop codon at nucleotide positions 354-356 (Figure 75). The predicted polypeptide precursor is 110 amino acids long (Figure 76; SEQ ID NO:197). The full-length PRO1274 protein shown in Figure 76 has an estimated molecular weight of about 12,363 daltons and a pl of about 8.31. Analysis of the full-length PRO1274 sequence shown in Figure 76 (SEQ ID NO: 197) evidences the presence of a variety of important polypeptide domains, wherein the locations given for those important polypeptide domains are approximate as described above. Analysis ofthe full-length PRO1274 sequence shown in Figure 76 evidences the presence of the following: a signal peptide from about amino acid 1 to about amino acid 24; an N-glycosylation site from about amino acid 71 to about amino acid 75: and insulin family protein homology blocks from about amino acid 76 to about amino acid 96, and from about amino acid 42 to about amino acid 61. Clone DNA64889-1541 has been deposited with ATCC on September 9, 1998 and is assigned ATCC deposit no. 203250.

An analysis of the Dayhoff database (version 35.45 SwissProt 35), using a WU-BLAST2 sequence alignment analysis of the full-length sequence shown in Figure 76 (SEQ ID NO:197), revealed sequence identity between the PRO1274 amino acid sequence and the following Dayhoff sequences: CEW05B2_9. AF016922_1 (insulin-like growth Factor 1). B48151, A53640, BTIGF2REC 1 (insulin-like growth factor 2), HSNEIGEN12 1, TXA3_RADMA (neurotoxin 3), CXMI_CONGE, P_P61301, TXA4_RADMA (neurotoxin 4).

EXAMPLE 42

Isolation of cDNA Clones Encoding Human PRO1286

DNA64903-1553 was identified by applying the proprietary signal sequence finding algorithm described in Example 3 above. Use of the above described signal sequence algorithm allowed identification of an EST cluster sequence from the LIFESEQ® database. designated Incyte EST cluster no. 86809. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). ESTs in the assembly included those identified from tumors, cell lines, or diseased tissue. One or more ofthe ESTs was obtained from a cDNA library constructed from RNA isolated from diseased colon tissue. The consensus sequence obtained therefrom is herein designated as DNA58822.

In light of the sequence homology between the DNA58822 sequence and Incyte EST clone no. 1695434, Incyte EST no. 1695434 was purchased and the cDNA insert was obtained and sequenced. The sequence of this cDNA insert is shown in Figure 77 (SEQ ID NO:198) and is herein designated as DNA64903-1553.

The entire coding sequence of DNA64903-1553 is included in Figure 77 (SEQ ID NO:198). Clone DNA64903-1553 contains a single open reading frame with an apparent translational initiation site at nucleotide positions 93-95 and ending at the stop codon at nucleotide positions 372-374 (Figure 77). The predicted polypeptide precursor is 93 amino acids long (Figure 78; SEQ ID NO:199). The full-length PRO1286 protein shown in Figure 78 has an estimated molecular weight of about 10, 111 daltons and a pl of about 9.70. Analysis of the full-length PRO1286 sequence shown in Figure 78 (SEQ ID NO:199) evidences the presence of a variety of important polypeptide domains, wherein the locations given for those important polypeptide domains are approximate as described above. Analysis of the full-length PRO 1286 sequence shown in Figure 78 evidences the presence of the following: a signal peptide from about amino acid I to about amino acid 18; and N-myristoylation sites from about amino acid 15 to about amino acid 21, from about amino acid 17 to about amino acid 23, from about amino acid 19 to about amino acid 25, from about amino acid 83 to about amino acid 89, and from about amino acid 86 to about amino acid 92. Clone DNA64903-1553 has been deposited with ATCC on September 15, 1998 and is assigned ATCC deposit no. 203223.

An analysis of the Dayhoff database (version 35.45 SwissProt 35), using a WU-BLAST2 sequence alignment analysis of the full-length sequence shown in Figure 78 (SEQ ID NO: 199), revealed some homology between the PRO1286 amino acid sequence and the following Dayhoff sequences: SR5C_ARATH, CELC17H12_11, MCPD_ENTAE, JQ2283, INVO_LEMCA, P_R07309, ADEVBCAGN_4. AF020947_1, CELT23H2_1, and MDH STRAP.

EXAMPLE 43

Isolation of cDNA Clones Encoding Human PRO1294

DNA64905-1558 was identified by applying the proprietary signal sequence finding algorithm described in Example 3 above. Use of the above described signal sequence algorithm allowed identification of an EST cluster sequence from the LIFESEQ$^k$ database, designated Incyte EST cluster no. 10559. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA57203.

In light of the sequence homology between the DNA57203 sequence and Incyte EST clone no. 3037763, Incyte EST no. 3037763 was purchased and the cDNA insert was obtained and sequenced. The sequence of this cDNA insert is shown in Figure 79 (SEQ ID NO:200) and is herein designated as DNA64905-1558.

The entire coding sequence of DNA64905-1558 is included in Figure 79 (SEQ ID NO:200). Clone DNA64905-1558 contains a single open reading frame with an apparent translational initiation site at nucleotide positions 110-112 and ending at the stop codon at nucleotide positions 1328-1330 (Figure 79). The predicted polypeptide precursor is 406 amino acids long (Figure 80; SEQ ID NO:201). The full-length PRO1294 protein shown in Figure 80 has an estimated molecular weight of about 46,038 daltons and a pl of about 6.50. Analysis of the full-length PRO1294 sequence shown in Figure 80 (SEQ ID NO:201) evidences the presence of a variety of important polypeptide domains, wherein the locations given for those important polypeptide domains are approximate as described above. Analysis of the full-length PRO 1294 sequence shown in Figure 80 evidences the presence of the following: a signal peptide from about amino acid 1 to about amino acid 21; N-glycosylation sites from about amino acid 177 to about amino acid 181, and from about amino acid 248 to about amino acid 252; a cAMP- and cGMP-dependent protein kinase phosphorylation site from about am ino acid 196 to about amino acid 200; a tyrosine kinase phosphorylation site from about amino acid 89 to about amino acid 97; N-myristoylation sites from about amino acid 115 to about amino acid 121, from about amino acid 152 to about amino acid 158, and from about amino acid 370 to about amino acid 376; and an amidation site from about amino acid 122 to about amino acid 126. Clone DNA64905-1558 has been deposited with ATCC on September 15, 1998 and is assigned ATCC deposit no. 203233.

An analysis of the Dayhoff database (version 35.45 SwissProt 35), using a WU-BLAST2 sequence alignment analysis of the full-length sequence shown in Figure 80 (SEQ ID NO:201), evidenced significant homology between the PRO1294 amino acid sequence and the following Dayhoff sequence: 173636. AF028740 I, AB006686S3_1, P_R98225. RNU78105_1, CELC48E7_4, CEF 11C3_3, SCP1_MESAU, TPM3_HUMAN, and CELK05B2_3.

EXAMPLE 44

Isolation of cDNA Clones Encoding Human PRO1303

A consensus DNA sequence was assembled relative to other EST sequences using phrap as described in Example 1 above. This consensus sequence is designated herein as DNA47347. Based on the DNA47347 consensus sequence, and its homology to an Incyte EST within the assembly from which DNA47347 was derived, Incyte clone 1430305 was purchased and sequenced in full. The sequence encoding PRO1303 was thereby identified.

The entire coding sequence of DNA65409-1566 is included in Figure 81 (SEQ ID NO:202). Clone DNA65409-1566 contains a single open reading frame with an apparent translational initiation site at nucleotide positions 121-123, and an apparent stop codon at nucleotide positions 865-867. The predicted polypeptide precursor is 248 amino acids long, and has an estimated molecular weight of about 26,734 daltons and a p1 of about 7.90. Analysis of the full-length PRO 1303 sequence shown in Figure 82 (SEQ ID NO:203) evidences the presence of a variety of important polypeptide domains, wherein the locations given forthose important polypeptide domains are approximate as described above. Analysis of the full-length PRO1303 polypeptide shown in Figure 82 evidences the presence of the following: a signal peptide from about amino acid 1 to about amino acid 17; N-glycosylation sites from about amino acid 24 to about amino acid 28, and from about amino acid 163 to about amino acid 167; a serine proteases, trypsin family, histidine active site from about amino acid 58 to about amino acid 64; serine proteases, trypsin family, histidine protein domains from about amino acid 47 to about amino acid 64, from about amino acid 196 to about amino acid 207, and from about amino acid 218 8 to about amino acid 242: kringle domain proteins homology blocks from about amino acid 194 to about amino acid 207, and from about amino acid 47 to about amino acid 65; and an apple domain from about amino acid 220 to about amino acid 248. Clone DNA65409-1566 has been deposited with the ATCC on September 15, 1998 and is assigned ATCC deposit no. 203232.

An analysis of the Dayhoffdatabase (version 35.45 SwissProt 35), using a WU-BLAST2 sequence alignment analysis of the full-length sequence shown in Figure 82 (SEQ ID NO:203), revealed sequence identity between the PRO1303 amino acid sequence and the following Dayhoff sequences: AB009849_1, P_W08475, AF024605_1, A42048_1, TRY3_RAT, MMAE00066414, TRY1_RAT, MMAE000663_4, MMAE000665_2. and MMAE00066412.

EXAMPLE 45

Isolation of cDNA Clones Encoding Human PRO 1304

A consensus DNA sequence was assembled relative to other EST sequences using phrap as described in Example 1 above. This consensus sequence is designated herein as DNA35745. Based on the DNA35745 consensus sequence, oligonucleotides were synthesized: 1) to identify by PCR a cDNA library that contained the sequence of interest, and 2) for use as probes to isolate a clone of the full-length coding sequence for PRO 1304.

PCR primers (forward and reverse) were synthesized:

forward PCR primer (35745.f1):

5'-GTGTTCTGCTGGAGCCGATGCC-3' (SEQ ID NO:206)

forward PCR primer (35745.f2):

5'-GACATGGACAATGACAGG-3' (SEQ ID NO:207)

forward PCR primer (35745.f3):

5'-CCTTTCAGGATGTAGGAG-3' (SEQ ID NO:208)

forward PCR primer (35745.f4):

5'-GATGTCTGCCACCCCAAG-3' (SEQ ID NO:209)

reverse PCR primer (35745.r1):

5'-GCATCCTGATATGACTTGTCACGTGGC-3' (SEQ ID NO:210)

reverse PCR primer (35745.r2):

5'-TACAAGAGGGAAGAGGAGTTGCAC-3' (SEQ ID NO:211)

Additionally, a synthetic oligonucleotide hybridization probe was constructed from the consensus DNA35745 sequence which had the following nucleotide sequence:

hybridization probe (35745.pl):

5'-GCCCATTATGACGGCTACCTGGCTAAAGACGGCTCGAAATTCTACTGCAGCC-3'(SEQ IDNO:212)

In order to screen several libraries for a source of a full-length clone, DNA from the libraries was screened by PCR amplification with the PCR primer pairs identified above. A positive library was then used to isolate clones encoding the PRO1304 gene using the probe oligonucleotide and one of the PCR primers. RNA for construction of the cDNA libraries was isolated from human ovary tissue.

DNA sequencing of the isolated clones isolated as described above gave the full-length DNA sequence for DNA65406-1567 [Figure 83, SEQ ID NO:204]; and the derived protein sequence for PRO1304.

The entire coding sequence of DNA65406-1567 is included in Figure 83 (SEQ ID NO:204). Clone DNA65406-1567 contains a single open reading frame with an apparent translational initiation site at nucleotide positions 23-25, and an apparent stop codon at nucleotide positions 689-691. The predicted polypeptide precursor is 222 amino acids long, and has an estimated molecular weight of about 25,794 daltons and a pl of about 6.24. Analysis of the full-length PRO1304 sequence shown in Figure 84 (SEQ ID NO:205) evidences the presence of a variety of important polypeptide domains, wherein the locations given for those important polypeptide domains are approximate as described above. Analysis of the full-length PRO1304 polypeptide shown in Figure 84 evidences the presence of the following: an endoplasmic reticulum targeting sequence from about amino acid 219 to about amino acid 224; an N-glycosylation site from about amino acid 45 to about amino acid 49; FKBP-type peptidyl-prolyl cis-trans isomerase homology blocks from about amino acid 87 to about amino acid 124, and from about amino acid 129 to about amino acid 143; and an EF-hand calcium-binding domain protein homology block from about amino acid 202 to about amino acid 215. Clone DNA65406-1567 has been deposited with the ATCC on September 15, 1998 and is assigned ATCC deposit no. 203219.

An analysis of the Dayhoff database (version 35.45 SwissProt 35), using a WU-BLAST2 sequence alignment analysis of the full-length sequence shown in Figure 84 (SEQ ID NO:205), evidenced significant homology between the PRO1304 amino acid sequence and the following Dayhoff sequences: AF040252_1, P_R28980, S71238, CELC05C8_1, VFU52045_1, S75144, FKB3 BOVIN, CELC50F2_6, CELB0511_12, and P_R41781.

The UNA65406-1567 sequence was also obtained by isolating and sequencing the insert of Incyte EST clone no. 2813577.

EXAMPLE 46

Isolation of cDNA Clones Encoding Human PRO1312

DNA55773 was identified in a human fetal kidney cDNA library using a yeast screen, that preferentially represents the 5' ends of the primary cDNA clones. Based on the DNA55773 sequence, oligonucleotides were synthesized for use as probes to isolate a clone of the full-length coding sequence for PRO1312.

A full length clone [DNA61873-1574] was identified that contained a single open reading frame with an apparent translational initiation site at nucleotide positions 7-9, and a stop signal at nucleotide positions 643-645 (Figure 85; SEQ ID NO:213). The predicted polypeptide precursor is 212 amino acids long, and has a calculated molecular weight of approximately 24,024 daltons and an estimated pl of approximately 6.26. Analysis of the full-length PRO1312 sequence shown in Figure 86 (SEQ ID NO:214) evidences the presence of a variety of important polypeptide domains as shown in Figure 86, wherein the locations given for those important polypeptide domains are approximate as described above. Analysis of the full-length PRO1312 polypeptide shown in Figure 86 evidences the presence of the following: a signal peptide from about amino acid 1 to about amino acid 14; a transmembrane domain from about amino acid 141 to about amino acid 160; and N-glycosylation sites from about amino acid 76 to about amino acid 80. and from about amino acid 93 to about amino acid 97. Clone DNA61873-1574 was deposited with the ATCC on August 18, 1998 , and is assigned ATCC deposit no. 203132.

An analysis of the Dayhoffdatabase (version 35.45 SwissProt 35), using a WU-BLAST2 sequence alignment analysis of the full-length sequence shown in Figure 86 (SEQ ID NO:214) revealed some homology between the PRO1312 amino acid sequence and the following Dayhoff sequences: GCINTALPH_1, GIBMUCIA_1, P_R96298, AF001406_1, PVU88874_1, P_R85151, AF041409_1, CELC50F2_7, C45875, and AB009510_21.

EXAMPLE. 47

Isolation of cDNA Clones Encoding Human PRO 1313

A consensus DNA sequence was assembled relative to other EST sequences using phrap as described in Example 1 above. This consensus sequence is designated herein as DNA64876. Based on the DNA64876 consensus sequence and upon a search for sequence homology with a proprietary Genentech EST sequence designated as DNA57711, a Merck Washington University EST sequence (designated R806 13) was found to have significant homology with DNA64876 and DNA57711. Therefore, the Merck/Washington University EST clone no. R80613 was purchased and the insert thereof obtained and sequenced, thereby giving rise to the DNA64966-1575 sequence shown in Figure 87 (SEQ ID NO:215), and the derived protein sequence for PRO1313.

The entire ending sequence of DNA64966-1575 is included in Figure 87 (SEQ ID NO:215). Clone DNA64966-1575 contains a single open reading frame with an apparent translational initiation site at nucleotide positions 115-117. and an apparent stop codon at nucleotide positions 1036-1038. The predicted polypeptide precursor is 307 amino acids long, and has an estimated molecular weight of about 35,098 daltons and a pl of about of about 8.11. Analysis of the full-length PRO 1313 sequence shown in Figure 88 (SEQ ID NO:2 16) evidences the presence of a variety of important polypeptide domains, wherein the locations given for those important polypeptide domains are approximate as described above. Analysis of the full-length PRO1313 polypeptide shown in Figure 88 evidences the presence of the following: transmembrane domains from about amino acid 106 to about amino acid 121, from about amino acid 136 to about amino acid 152, from about amino acid 172 to about amino acid 188, from about amino acid 230 to about amino acid 245, and from about amino acid 272 to about amino acid 285; N-glycosylation sites from about amino acid 34 to about amino acid 38, from about amino acid 135 to about amino acid 139, and from about amino acid 203 to about amino acid 207; a tyrosine kinase phosphorylation site from about amino acid 59 to about amino acid 67; N-myristoylation sites from about amino acid 165 to about amino acid 171, from about amino acid 196 to about amino acid 202, from about amino acid 240 to about amino acid 246, and from about amino acid 247 to about amino acid 253: and an ATP/GTP-binding site motif A (P-loop) from about amino acid 53 to about amino acid 61. Clone DNA64966-1575 has been deposited with the ATCC on January 12, 1999 and is assigned ATCC deposit no. 203575.

An analysis of the Dayhoff database (version 35.45 SwissProt 35), using a WU-BLAST2 sequence alignment analysis of the full-length sequence shown in Figure 88 (SEQ ID NO:216), evidenced significant homology between the PRO1313 amino acid sequence and the following Dayhoff sequences: CELT27A1_3, CEF09C6_7, U93688_9, 1164896, YDCX_ECOLL and RNU06101_1.

EXAMPLE 48

Isolation of cDNA Clones Encoding Human PRO1376

A Merck/Washington University database was searched and a sequence was identified as Accession No. W39620 (identified as a Soares parathyroid tumor protein). This sequence was put into a database wherein it could be compared to other sequences and aligned with other sequences.

A consensus DNA sequence was assembled relative to other EST sequences using phrap as described in Example 1 above. This consensus sequence is designated herein as DNA67221.

The clone encoding Merck W39620 was purchased and fully sequenced. DNA sequencing of the isolated clones isolated as described above gave the full-length DNA sequence for DNA67300-1605 [Figure 89, SEQ ID NO: 217]; and the derived protein sequence for PRO1376.

The entire coding sequence of DNA67300-1605 is included in Figure 89 (SEQ ID NO:217). Clone DNA67300-1605 contains a single open reading frame with an apparent translational initiation site at nucleotide positions 107-109, and an apparent stop codon at nucleotide positions 623-625. The predicted polypeptide precursor is 172 amino acids long, and has an estimated molecular weight of about 19,206 daltons and a pl of about 5.36. Analysis of the full-length PRO11376 sequence shown in Figure 90(SEQ ID NO:218) evidences the presence of a variety of important polypeptide domains, wherein the locations given for those important polypeptide domains are approximate as described above. Analysis of the full-length PRO1376 polypeptide shown in Figure 90 evidences the presence of the following: a signal peptide from about amino acid 1 to about amino acid 23; and a thioredoxin family proteins homology block from about amino acid 58 to about amino acid 75. Clone DNA67300-<HHY>DNA67300-1605 has been deposited with the ATCC on August 25, 1998 and is assigned ATCC deposit no. 203163.

An analysis of the Dayhoff database (version 35.45 SwissProt 35), using a WU-BLAST2 sequence alignment analysis of the full-length sequence shown in Figure 90 (SEQ I D NO:218), revealed sequence identity between the PRO1376 amino acid sequence and the following Dayhoff sequences: XAG_XENLA, AF025474_1, NP77_XENLA, D69100, S75124, ER60_SCHMA, H69466, A57254, AB002234_1, and TATHIORDH_1.

EXAMPLE 49

Isolation of cDNA Clones Encoding Human PRO1387

DNA68872-1620 was identified by applying the proprietary signal sequence finding algorithm described in Example 3 above. Use of the above described signal sequence algorithm allowed identification of an EST cluster sequence from the LIFESEQ® database, designated Incyte EST cluster no. 10298. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul et at., Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington. Seattle. Washington). The consensus sequence obtained therefrom is herein designated as DNA56259. A Genentech proprietary EST sequence was used in the assembly and is herein designated DNA39516.

In light of the sequence homology between the DNA56259 sequence and Incyte EST clone no. 3507924, Incyte EST no. 3507924 was purchased and the cDNA insert was obtained and sequenced. The sequence of this cDNA insert is shown in Figure 91 (SEQ ID NO:219) and is herein designated as DNA68872-1620.

The entire coding sequence of DNA68872-1620 is included in Figure 91 (SEQ ID NO:219). Clone DNA68872-1620 contains a single open reading frame with an apparent translational initiation site at nucleotide positions 85-87 and ending at the stop codon at nucleotide positions 1267-1269 (Figure 91). The predicted polypeptide precursor is 394 amino acids long (Figure 92: SEQ ID NO:220). The full-length PRO1387 protein shown in Figure 92 has an estimated molecular weight of about 44.339 daltons and a p1 of about 7.10. Analysis of the full-length PRO1387 sequence shown in Figure 92 (SEQ ID NO:220) evidences the presence of a variety of important polypeptide domains, wherein the locations given for those important polypeptide domains are approximate as described above. Analysis of the full-length PRO1387 sequence shown in Figure 92 evidences the presence of the following: a signal peptide from about amino acid 1 to about amino acid 19; a transmembrane domain from about amino acid 275 to about amino acid 296; N-glycosylation sites from about amino acid 76 to ahout amino acid 80, from about amino acid 231 to about amino acid 235, from about amino acid 302 to about amino acid 306. from about amino acid 307 to about amino acid 31 and from about amino acid 376 to about amino acid 380: and myelin P0 protein homology blocks from about amino acid 210 to about amino acid 240, and from about amino acid 92 to about amino acid 122. Clone DNA68872-1620 has been deposited with ATCC on August 25. 1998 and is assigned ATCC deposit no. 203160.

An analysis of the Dayhoff database (version 35.45 SwissProt 35), using a WU-BLAST2 sequence alignment analysis of the full-length sequence shown in Figure 92 (SGQ ID NO:220), evidenced significant homology between the PRO1387 amino acid sequence and the following Dayhoff sequences: P_W36955, MYP0_HETFR, HS46KDA_ 1,AF049498_1,MYO0_HUMAN,AF030454_1.A53268,SHPTCRA_1.P_W14146,and GEN 12838.

EXAMPLE 50

Isolation of cDNA Clones Encoding Human PRO1561

A consensus DNA sequence was assembled relative to other EST sequences using phrap as described in Example 1 above. This consensus sequence is designated herein as DNA40630. A proprietary Genentech EST sequence was employed in the consensus assembly and is herein designated as DNA40753. Based on the DNA40630 consensus sequence, oligonucleotides were synthesized: 1) to identify by PCR a cDNA library that contained the sequence of interest, and 2) for use as probes to isolate a clone of the full-length coding sequence for PRO1561.

PCR primers (forward and reverse) were synthesized:

forward PCR primer (40630.f1):

5'-CTGCCTCCACTGCTCTGTGCTGGG-3' (SEQ ID NO:223)

reverse PCR primer (40630.r1):

5'-CAGAGCAGTGGATGTTCCCCTGGG-3' (SEQ ID NO:224)

Additionally, a synthetic oligonucleotide hybridization probe was constructed from the consensus DNA40630 sequence which had the following nucleotide sequence:

hybridization probe (40630.pl):

5'-CTGAACAAGATGGTCAAGCAAGTGACTGGGAAAATGCCCATCCTC-3' (SEQ ID NO:225)

In order to screen several libraries for a source of a full-length clone. DNA from the libraries was screened by PCR amplification with the PCR primer pairs identified above. A positive library was then used to isolate clones encoding the PRO1561 gene using the probe oligonucleotide and one of the PCR primers. RNA for construction of the cDNA libraries was isolated from human breast tumor tissue.

DNA sequencing of the isolated clones isolated as described above gave the full-length DNA sequence for DNA76538-1670 [Figure 93, SEQ ID NO:221]; and the derived protein sequence for PRO1561.

The entire coding sequence of DNA76538-1670 is included in Figure 93 (SEQ ID NO:221). Clone DNA76538-1670 contains a single open reading frame with an apparent translational initiation site at nucleotide positions 29-31, and an apparent stop codon at nucleotide positions 377-379. The predicted polypeptide precursor is 116 amino acids long, and has an estimated molecular weight of about 12,910 daltons and a pl of about 6.41. Analysis of the full-length PRO1561 sequence shown in Figure 94 (SEQ ID NO:222) evidences the presence of a variety of important polypeptide domains, wherein the locations given for those important polypeptide domains are approximate as described above. Analysis of the full-length PRO1561 polypeptide shown in Figure 94 evidences the presence of the follow ing: a signal peptide from about amino acid 1 to about amino acid 17; an N-glycosylation site from about amino acid 86 to about amino acid 90; N-myristoytation sites from about amino acid 20 to about amino acid 26. and from about amino acid 45 to about amino acid 51; and a phospholipase A2 histidine active site from about amino acid 63 to about amino acid 71. Clone DNA76538-1670 has been deposited with the ATCC on October 6, 1998 and is assigned ATCC deposit no. 203313.

An analysis of the Dayhoff database (version 35.45 SwissProt 35), using a WU-BLAST2 sequence alignment analysis of the full-length sequence shown in Figure 94 (SEQ ID NO:222), evidenced significant homology between the PRO1561 amino acid sequence and the following Dayhoff sequences: P_R63053, P_R25416, P_R63055,P_P93363,P_R63046.PA2A_VIPAA,P_W58476,GEN13747,PA2X_HUMAN, and PA2A_CRODU.

In addition to the above, a sequence homology search evidenced significant homology between the DNA40630 consensus sequence and Incyte EST clone no. 1921092. As such, Incyte EST clone no. 1921092 was purchased and the insert obtained and sequenced, thereby giving rise to the DNA76538-1670 sequence shown in Figure 93 (SEQ ID NO:221).

EXAMPLE 51

Isolation of cDNA Clones Encoding Human PRO216

The extracellular domain (ECD) sequences (including the secretion signal sequence, if any) from about 950 known secreted proteins from the Swiss-Prot public database were used to search EST databases. The EST databases included public EST databases (*e.g.*, GenBank) and a proprietary EST database (LIFESEQ®, Incyte Phannaceuticals, Palo Alto, CA). The search was performed using the computer program BLAST or BLAST2 [Altschul et al., Methods in Enzymology, 266:460-480 (1996)] as a comparison of the ECD protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green. University of Washington. Seattle, Washington).

The complete cDNA sequence of DNA33087 is disclosed in Gen Bank under accession numbers AB0001 114_1 and AB009589_1 (human osteornodulin). A related, but probably different protein, corneal keratin sulfate, is disclosed in Funderburgh er al., J. Biol. Chem., 271:31431-31436 (1996).

A consensus DNA sequence was assembled relative to other EST sequences using phrap as described above. This consensus sequence is herein designated DNA28754. In some cases, the consensus sequence derives from an intermediate consensus DNA sequence which was extended using repeated cycles of BLAST and phrap to extend that intermediate consensus sequence as far as possible using the sources of EST sequences discussed above.

Based on the DNA28754 consensus sequence, oligonucleotides were synthesized: 1) to identify by PCR a cDNA library that contained the sequence of interest, and 2) for use as probes to isolate a clone of the full-length coding sequence for PRO216. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5 kbp. In order to screen several libraries for a full-length clone. DNA from the libraries was screened by PCR amplification, as per Ausubel *et al.,* Current Protocols in Molecular Biology, *supra,* with the PCR primer pair. A positive library was then used to isolate clones encoding the gene of interest using the probe oligonucleotide and one of the primer pairs.

PCR primers (forward and reverse) were synthesized:

forward PCR primer:

5'-TCACGATGATCCTGACAATGC-3' (SEQ ID NO:228)

reverse PCR primer:

5'-AATAATGAAGGTCAAAGTGCCCTT-3' (SEQ ID NO:229)

Additionally, a synthetic oligonucleotide hybridization probe was constructed from the consensus DNA28754 sequence which had the following nucleotide sequence:

hybridization probe:

5'-TGCTCCTTCTTGTTCTGGGCTCTCATG-3' (SEQ ID NO:230)

RNA for construction of the cDNA libraries was isolated from human fetal kidney tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI sites.

DNA sequencing of the clones isolated as described above gave the full-length DNA sequence for a full-length PRO216 polypeptide (designated herein as DNA33087 [Figure 95, SEQ ID NO:226]) and the derived protein sequence for that PRO216 polypeptide.

The full length clone identified above contained a single open reading frame with an apparent translational initiation site at nucleotide positions 268-270 and a stop signal at nucleotide positions 1531-1533 (Figure 95, SEQ ID NO:226). The predicted polypeptide precursor is 421 amino acids long [Figure 96; (SEQ ID NO:227)] and has a calulated molecular weight of approximately 49,492 daltons and an estimated pI of about 5.5 1. Analysis of the full-length PRO216 sequence shown in Figure 96 (SEQ ID NO:227) evidences the presence of important polypeptide domains as shown in Figure 96, wherein the locations given for those important polypeptide domains are approximate as described above. Analysis of the full-length PRO216 sequence (Figure 96, SEQ ID NO:227) evidences the following: N-linked glycosylation sites from about amino acid 113 to about amino acid 117, from about amino acid 121 to about amino acid 125, from about amino acid 187 to about amino acid 191, from about amino acid 242 to about amino acid 246 and from about amino acid 316 to about amino acid 320; tyrosine kinase phosphorylation sites from about amino acid 268 to about amino acid 275 and from about amino acid 300 to about amino acid 307; an N-myristoylation site from about amino acid 230 to about amino acid 236; and leucine zipper patterns from about amino acid 146 to about amino acid 168 and from about amino acid 217 to about amino acid 239. Clone DNA33087 has been deposited with ATCC on October 16, 1997 and is assigned ATCC deposit no. 209381.

This clone is the same as human osteomodulin submitted by I. Ohno to GenBank on December 26, 1996 (AB000114_1) and submitted by I. Ohno *et al.,* to GenBank on December 5, 1997 (AB009589-1).

EXAMPLE 52

Stimulation of Heart Neonatal Hypertrophy

This assay is designed to measure the ability of PRO polypeptides to stimulate hypertrophy of neonatal heart. Cardiac myocytes from 1-day old Harlan Sprague Dawley rats were obtained. Cells (180 μl at 7.5 x $10^4$/ml, serum <0.1%, freshly isolated) are added on day 1 to 96-well plates previously coated with DMEM/F12 + 4% FCS. Test samples containing the test PRO polypeptide are added directly to wells on day 2 in 20 μL volumes. Cells are stained with crystal violet after an additional two days and scored visually by the next day. Incubator conditions are critical and require 5% $CO_2$.

Activity reference: phenylephrine at 1-100 μM, PGF-2 alpha at 0.1-1,0 μM, endothelin-1 at 1-10 nM, CT1 (LIF) at 1-10 nM. No PBS is included, since Ca concentration is critical for assay response. Assay media included: DMEM/F12 (with 2.44 gm bicarbonate), 10 μg/ml transferrin, 1 μg/ml insulin, 1 μg/ml aprotinin, 2 mmol/L glutamine, 100 U/ml penicillin G, 100 μg/ml streptomycin. Protein buffer containing mannitol (4%) gave a positive signal (score 3.5) at 1/10 (0.4%) and 1/100 (0.04%), but not at 1/1000 (0.004%). Therefore, the test sample buffer containing mannitol is not run. A secondary assay consists of measuring the ANP levels (ng/ml) by ELISA in conditioned media from the cells. An increase in the ANP message can be measured by PCR from cells after a few hours.

Results are assessed by visually observing cell size: a score = 3.5 or greater is considered positive for conditioned media; a score of 3.0 or greater is considered positive for purified protein.

PRO231, PRO526, PRO713, PRO792, PRO1246, and PRO1312 purified polypeptides were observed to stimulate neonatal heart hypertrophy and exhibited positive scores in this assay compared with positive controls as shown in TABLE 4 below:

TABLE 4

Stimulation of Heart Neonatal Hypertrophy

| PRO SAMPLE | Concentration (or dilution) | Score |
|---|---|---|
| PR0231 | 12.0 μM | 3.375 |
| PRO526 | 1% | 3.50 |
| PRO713 | 10.0 nM | 3.25 |
| PRO713 | 10.0 nM | 3.25 |
| PRO792 | 1% | 4.50 |
| PRO1246 | 1% | 3.25 |

(continued)

| Stimulation of Heart Neonatal Hypertrophy | | |
|---|---|---|
| PRO SAMPLE | Concentration (or dilution) | Score |
| PRO1312 | 85.0 nM | 3.375 |

EXAMPLE 53

Enhancement of Heart Neonatal Hypertrophy Induced by F2a

This assay is designed to measure the ability of PRO polypeptides to stimulate hypertrophy of neonatal heart. Cardiac myocytes from I-day old Harlan Sprague Dawley rats were obtained. Cells (180 µl at 7.5 x $10^4$/ml, serum <0.1%, freshly isolated) are added on day 1 to 96-well plates previously coated with DMEM/F12 + 4% FCS. Test samples containing the test PRO polypeptide (20 µl/well) are added directly to the wells on day 1. PGF (20 µl/well) is then added on day 2 at a final concentration of $10^{-6}$ M. The cells are then stained on day 4 and visually scored on day 5. Visual scores are based on cell size, wherein cells showing no increase in size as compared to negative controls are scored 0.0, cells showing a small to moderate increase in size as compared to negative controls are scored 1.0 and cells showing a large increase in size as compared to negative controls are scored 2.0. A score of 1.0 or greater is considered positive.

No PBS is included, since Ca concentration is critical for assay response. Plates are coated with DMEM/F12 plus 4% FCS (200 µl/well). Assay media included: DMEM/F12 (with 2.44 gm bicarbonate), 10 µg/ml transferrin, I µg/ml insulin, 1 µg/ml aprotinin. 2 mmol/L glutamine, 100 U/ml penicillin G, 100 µg/ml streptomycin. Protein buffer containing mannitol (4%) gave a positive signal (score 3.5) at 1/10 (0.4%) and 1/100 (0.04%), but not at 1/1000 (0.004%). Therefore the test sample buffer containing mannitol is not run.

PRO179, PRO182, PRO195, and PRO224 purified polypeptides showed positive results when assayed by the above method as shown in TABLE 5 below:

TABLE 5

Enhancement of Heart Neonatal Hypertrophy Induced by F2a

| PRO | Concentration | Score |
|---|---|---|
| PRO179 | 0.01% | 0.0 |
| PRO179 | 0.10% | 0.0 |
| PRO179 | 1% | 1.0 |
| PRO182 | 0.01% | 0.0 |
| PRO182 | 0.10% | 0.0 |
| PRO182 | 1% | 1.0 |
| PRO195 | 0.01% | 0.0 |
| PRO195 | 0.1% | 1.0 |
| PRO195 | 1% | 1.0 |
| PRO224 | 0.01% | 0.0 |
| PRO224 | 0.10% | 0.0 |
| PRO224 | 1% | 1.0 |

EXAMPLE 54

Inhibition of Heart Adult Hypertrophy

This assay is designed to measure the inhibition of heart adult hypertrophy. Ventricular myocytes arc freshly isolated from adult (250g) Harlan Sprague Dawley rats and the cells are plated at 2000/well in 180 µl volume. On day two, test samples (20 µl) containing the test PRO polypeptide are added. On day five, the cells are fixed and then stained. An increase in ANP message can also be measured by PCR from cells after a few hours. Results are based on a visual score of cell size: 0 = no inhibition, -1 =small inhibition, -2 = large inhibition. A score of less than 0 is considered positive. Activity reference corresponds to phenylephrin (PE) at 0.1 mM, as a positive control. A score of 2 is considered very responsive. Assay media included: M 199 (modified)-glutamine free, $NaHCO_3$, phenol red, supplemented with 100 nM insulin, 0.2% BSA, 5 mM cretine, 2 mM L-camitine, 5 mM taurine, 100 U/ml penicillin G, 100 µg/ml streptomycin (CCT medium). Only inner 60 wells are used in 96 well plates. Of these, 6 wells are reserved for negative and positive (PE) controls. Initially, quantitative PCR will be run in parallel to determine relative level of sensitivity to the visual scoring system.

PRO269 and PRO356 showed positive results in inhibition of heart adult hypertrophy in the above described assay.

EXAMPLE 55

Stimulation of Endothelial Cell Proliferation

This assay is designed to determine whether PRO polypeptides show the ability to stimulate adrenal cortical capillary endothelial cell (ACE) growth.

Bovine adrenal cortical capillary endothelial (ACE)cells (from primary culture, maximum of 12-14 passages) were plated in 96-well plates at 500 cells/well per 100 microliter. Assay media included low glucose DMEM, 10% calf serum, 2 mM glutamine, and IX penicillin/streptomycin/fungizone. Control wells included the following: (1) no ACE cells added; (2) ACE cells alone; (3) ACE cells plus VEGF (5 ng/ml); and (4) ACE cells plus FGF (5ng/ml). The control or test sample. (in 100 microliter volumes), was then added to the wells (at dilutions of 1%, 0.1% and 0.01%, respectively). The cell cultures were incubated for 6-7 days at 37°C/5% $CO_2$. After the incubation, the media in the wells was aspirated, and the cells were washed I X with PBS. An acid phosphatase reaction mixture (100 microliter: 0.1M sodium acetate, pH 5.5, 0.1% Triton X-100, 10 mM p-nitrophenyl phosphate) was then added to each well. After a 2 hour incubation at 37°C, the reaction was stopped by addition of 10 microliters 1N NaOH. Optical density (OD) was measured on a microplate reader at 405 nm.

The activity of a PRO polypeptide was calculated as the fold increase in proliferation (as determined by the acid phosphatase activity, OD 405 nm) relative to (1) cell only background, and (2) relative to maximum stimulation by VEGF VEGF (at 3-10 ng/ml) and FGF (at 1-5 ng/ml) were employed as an activity reference for maximum stimulation. Results of the assay were considered "positive" if the observed stimulation was ≥ 50% increase over background. VEGF (5 ng/ml) control at 1% dilution gave 1.24 fold stimulation; FGB (5 ng/ml) control at 1% dilution gave 1.46 fold stimulation.

PRO179, PRO212, PRO1075, PRO1154, PRO1244, PRO1286, and PRO1303 assayed "positive" as shown in TABLE 6 below:

TABLE 6

Stimulation of Endothelial Cell Proliferation

| PRO | Concentration | Fold Stimulation |
|---|---|---|
| PRO179 | 0.01% | 1.16 |
| PRO179 | 0.10% | 1.57 |
| PRO179 | 1.0% | 1.38 |
| PRO212 | 0.01% | 4.08 |
| PRO212 | 0.10% | 4.73 |
| PRO212 | 1.0% | 4.75 |
| PRO1075 | 0.01% | 4.21 |
| PRO1075 | 0.10% | 4.52 |
| PRO1075 | 1.0% | 3.39 |
| PRO1154 | 0.0017 nM | 4.63 |
| PRO1154 | 0.017 nM | 5.15 |
| PRO1154 | 0.17 nM | 5.53 |
| PRO1244 | 0.67 nM | 4.60 |
| PRO1244 | 6.7 nM | 4.78 |
| PRO1244 | 67.0 nM | 5.24 |
| PRO1286 | 0.5 nM | 4.61 |
| PRO1286 | 5.0 nM | 4.55 |
| PRO1303 | 0.38 nM | 4.29 |
| PRO1303 | 3.80 nM | 4.28 |
| PRO1303 | 38.0 nM | 3.74 |

EXAMPLE 56

Inhibition of Vascular Endothelial Growth Factor (VEGF) Stimulated Proliferation of Endothelial Cell Growth

The ability of various PRO polypeptides to inhibit VEGF stimulated proliferation of endothelial cells was tested. Specifically, bovine adrenal cortical capillary endothelial (ACE) cells (from primary culture, maximum of 12-14 passages) were plated in 96-well plates at 500 cells/well per 100 microliter. Assay media included low glucose DMEM, 10% calf serum (not fetal calf), 2 mM glutamine, and 1X penicillin/streptomycin/fungizone. Control wells included the following: ( I ) no ACE cells added; (2) ACE cells alone; (3) ACE cells plus 5 ng/ml FGF; (4) ACE cells plus 3 ng/ml VEGF; (5) ACE cells plus 3 ng/ml VEGF plus 1 ng/ml TGF-beta; and (6) ACE cells plus 3 ng/ml VEGF plus 5 ng/ml LIF. The test samples, poly-His tagged PRO polypeptides (in 100 microliter volumes), were then added to the wells (at dilutions of 1%, 0.1% and 0.01%, respectively). The cell cultures were incubated for 6-7 days at 37°C/5% $CO_2$. After the incubation, the media in the wells was aspirated, and the cells were washed IX with PBS. An acid phosphatase reaction mixture (100 microliter; 0.1M sodium acetate, pH 5.5, 0.1% Triton X-100, 10 mM p-nitrophenyl phosphate) was then added to each well. After a 2 hour incubation at 37°C, the reaction was stopped by addition of 10 microliters IN NaOH. Optical density (OD) was measured on a microplate reader at 405 nm.

The activity of the test PRO polypeptide was calculated as the percent inhibition of VEGF (3 ng/ml) stimulated proliferation (as determined by measuring acid phosphatase activity at OD 405 nm) relative to the cells without stimulation. TGF-beta was employed as an activity reference at 1 ng/ml, since TGF-beta blocks 70-90% of VEGF-stimulated ACE cell proliferation. The results, as shown in TABLE 7 below, are indicative of the utility of the PRO polypeptides in cancer therapy and specifically in inhibiting tumor angiogenesis. The numerical values (relative inhibition) shown in TABLE 7 are determined by calculating the percent inhibition of VEGF stimulated proliferation by the PRO polypeptides relative to cells without stimulation and then dividing that percentage into the percent inhibition obtained by TGF-β at 1 ng/ml which is known to block 70-90% of VEGF stimulated cell proliferation. The results are considered positive if the PRO ploypeptide exhibits 30% or greater inhibition of VEGF stimulation of endothelial cell growth (relative inhibition 30% or greater).

TABLE 7

Inhibition of VEGF Stimilated Endothelial Cell Growth

| PRO Name | PRO Concentration | Relative % Inhibition |
|---|---|---|
| PRO187 | 0.01% | 91 |
| PRO187 | 0.10% | 82 |
| PRO187 | 1.0% | 44 |
| PRO214 | 0.01% | 68 |
| PRO214 | 0.10% | 91 |
| PRO214 | 1.0% | 94 |
| PRO216 | 0.01% | 96 |
| PRO216 | 0.10% | 81 |
| PRO216 | 1.0% | 31 |
| PRO216 | 0.01% | 104 |
| PRO216 | 0.10% | 93 |
| PRO216 | 1.0% | 50 |
| PRO323 | 0.01% | 59 |
| PRO323 | 0.10% | 93 |
| PRO323 | 1.0% | 91 |
| PRO812 | 0.025 nM | 101 |
| PRO812 | 0.25 nM | 101 |
| PRO812 | 2.5 nM | 96 |
| PRO1246 | 0.007nM | 90 |
| PRO1246 | 0.07 nM | 87 |
| PRO1246 | 0.70 nM | 60 |
| PRO1246 | 0.007 nM | 99 |
| PRO1246 | 0.07 nM | 94 |
| PRO1246 | 0.70 nM | 73 |

EXAMPLE 57

induction of c-fos in Endothelial Cells

This assay is designed to determine whether PRO polypeptides show the ability to induce c-fos in endothelial cells.

Human venous umbilical vein endothelial cells (HUVEC, Cell Systems) in growth media (50% Ham's F12 w/o GHT: low glucose, and 50% DMEM without glycine: with NaHCO3, 1% glutamine, 10 mM HEPES, 10% FBS, 10 ng/ml bFGF) were plated on 96-well microtiter plates at a cell density of $1x10^4$ cells/well. The day after plating, the cells were starved by removing the growth media and treating the cells with 100 μl/well test samples and controls (positive control = growth media; negative control = Protein 32 buffer = 10 mM HEPES, 140 mM NaCl, 4% (w/v) mannitol, pH 6.8). The cells were incubated for 30 minutes at 37°C, in 5% $CO_2$. The samples were removed, and the first part of the bDNA kit protocol (Chiron Diagnostics, cat. #6005-037) was followed, where each capitalized reagent/buffer listed below was available from the kit.

Briefly, the amounts of the TM Lysis Buffer and Probes needed for the tests were calculated based on information provided by the manufacturer. The appropriate amounts of thawed Probes were added to the TM Lysis Buffer. The Capture Hybridization Buffer was warmed to room temperature. The bDNA strips were set up in the metal strip holders, and 100 μl of Capture Hybridization Buffer was added to each b-DNA well needed, followed by incubation for at least 30 minutes. The test plates with the cells were removed from the incubator, and the media was gently removed using the vacuum manifold. 100 μl of Lysis Hybridization Buffer with Probes were quickly pipetted into each well of the microtiter plates. The plates were then incubated at 55°C for 15 minutes. Upon removal from the incubator, the plates were placed on the vortex mixer with the microtiter adapter head and vortexed on the #2 setting for one minute. 80 μl of the lysate was removed and added to the bDNA wells containing the Capture Hybridization Buffer, and pipetted up and down to mix. The plates were incubated at 53°C for at least 16 hours.

On the next day, the second part of the bDNA kit protocol was followed. Specifically, the plates were removed from the incubator and placed on the bench to cool for 10 minutes. The volumes of additions needed were calculated based upon information provided by the manufacturer. An Amplifier Working Solution was prepared by making a 1:100 dilution of the Amplifier Concentrate (20 fm/μl) in AL Hybridization Buffer. The hybridization mixture was removed from the plates and washed twice with Wash A. 50 μl of Amplifier Working Solution was added to each well and the wells were incubated at 53°C for 30 minutes. The plates were then removed from the incubator and allowed to cool for 10 minutes. The Label Probe Working Solution was prepared by making a 1 : 100 dilution of Label Concentrate (40 pmoles/μl) in AL Hybridization Buffer. After the 10-minute cool-down period, the amplifier hybridization mixture was removed and the plates were washed twice with Wash A. 50 μl of Label Probe Working Solution was added to each well and the wells were incubated at 53°C for 15 minutes. After cooling for 10 minutes, the Substrate was warmed to room temperature. Upon addition of 3 μl of Substrate Enhancer to each ml of Substrate needed for the assay, the plates were allowed to cool for 10 minutes, the label hybridization mixture was removed, and the plates were washed twice with Wash A and three times with Wash D. 50 μl of the Substrate Solution with Enhancer was added to each well. The plates were incubated for 30 minutes at 37°C and RLU was read in an appropriate luminometer.

The replicates were averaged and the coefficient of variation was determined. The measure of activity of the fold increase over the negative control (Protein 32/HEPES buffer described above) value was indicated by chemiluminescence units (RLU). The results are shown in TA BLE 8 below, and are considered positive if the PRO polypeptide exhibits at least a two-fold value over the negative buffer control. Negative control = 1.00 RLU at 1.00% dilution. Positive control = 8.39 RLU at 1.00% dilution.

TABLE 8

Induction of c-fos in Endothelial Cells

| PRO Name | PRO Concentration | RLU values |
|---|---|---|
| PRO287 | 0.018 nM | 2.06 |
| PRO287 | 0.18 nM | 1.89 |
| PRO287 | 1.8 nM | 1.73 |
| PRO287 | 0.18 nM | 1.64 |
| PRO287 | 1.8 nM | 2.14 |
| PRO538 | 0.2149 nM | 1.95 |
| PRO538 | 2.149 nM | 2.15 |
| PRO538 | 21.49 nM | 1.46 |
| PRO538 | 0.2149 nM | 2.38 |

(continued)

Induction of c-fos in Endothelial Cells

| PRO Name | PRO Concentration | RLU values |
|---|---|---|
| PRO538 | 2.149 nM | 2.42 |
| PRO538 | 21.49 nM | 2.84 |
| PRO713 | 0.022 nM | 1.95 |
| PRO713 | 0.22 nM | 2.17 |
| PRO713 | 2.2 nM | 2.13 |
| PRO713 | 0.022 nM | 2.66 |
| PRO713 | 0.22 nM | 2.52 |
| PRO713 | 2.2 nM | 2.69 |
| PRO788 | 0.29 nM | 1.23 |
| PRO788 | 2.9 nM | 2.65 |
| PRO788 | 29.0 nM | 0.93 |
| PRO865 | 0.027 nM | 1.98 |
| PRO865 | 0.27 nM | 3.09 |
| PRO865 | 2.7 nM | 2.90 |
| PRO865 | 0.027 nM | 2.88 |
| PRO865 | 0.27 nM | 2.19 |
| PRO865 | 2.7 nM | 2.64 |
| PRO1126 | 0.029 nM | 1.94 |
| PRO1126 | 0.29 nM | 2.33 |
| PRO1126 | 2.9 nM | 1.81 |
| PRO1130 | 0.6 nM | 1.89 |
| PRO1130 | 6.0 nM | 2.04 |
| PRO1130 | 60.0 nM | 2.06 |
| PRO1274 | 0.365 nM | 0.86 |
| PRO1274 | 3.65 nM | 1.14 |
| PRO1274 | 36.5 nM | 2.11 |
| PRO1274 | 0.365 nM | 2.04 |
| PRO1274 | 3.65 nM | 1.86 |
| PRO1274 | 36.5 nM | 2.41 |
| PRO1294 | 0.13 nM | 2.38 |
| PRO1294 | 1.3 nM | 2.23 |
| PRO1294 | 13.0 nM | 1.39 |
| PRO1294 | 0.13 nM | 2.78 |
| PRO1294 | 1.3 nM | 2.53 |
| PRO1294 | 13.0 nM | 1.39 |
| PRO1304 | 0.074 nM | 1.72 |
| PRO1304 | 0.74 nM | 2.08 |
| PRO1304 | 7.4 nM | 1.41 |
| PRO1304 | 0.074 nM | 2.66 |
| PRO1304 | 0.74 nM | 1.75 |
| PRO1304 | 7.4 nM | 1.45 |
| PRO1376 | 0.86 nM | 1.41 |
| PRO1376 | 8.6 nM | 2.20 |
| PRO1376 | 86.0 nM | 2.59 |
| PRO1376 | 0.86 nM | 1.43 |
| PRO1376 | 8.6 nM | 2.28 |
| PRO1376 | 86.0 nM | 1.49 |
| PRO1387 | 0.1 nM | 2.19 |
| PRO1387 | 1.0 nM | 1.81 |

(continued)

Induction of c-fos in Endothelial Cells

| PRO Name | PRO Concentration | RLU values |
|---|---|---|
| PRO1387 | 10.0 nM | 2.45 |

EXAMPLE 58

Human Venous Endothelial Cell Ca Flux Assay

This assay is designed to determine whether PRO polypeptides show the ability to stimulate calcium flux in human umbilical vein endothelial cells (HUVEC, Cell Systems). Ca influx is a well documented response upon binding of certain ligands to their receptors. A test compound that results in a positive response in the present Ca influx assay can be said to bind to a specific receptor and activate a biological signaling pathway in human endothelial cells. This could ultimately lead, for example to cell division, inhibition of cell proliferation, endothelial tube formation, cell migration, apoptosis, etc.

Human venous umbilical vein endothelial cells (HUVEC, Cell Systems) in growth media (50:50 without glycine, 1% glutamine, 10mM Hepes, 10% FBS, 10ng/mlbFGF), were plated on 96-well microtiter ViewPlates-96 (Packard Instrument Company Part #6005182) microtiter plates at a cell density of 2 x $10^4$ cells/well. The day after plating, the cells were washed three times with buffer (HBSS plus 10 mM Hepes), leaving 100 $\mu$l/well. Then 100 $\mu$l/well of 8 $\mu$M Fluo-3 (2x) was added. The cells were incubated for 1.5 hours at 37°C/5% $CO_2$. After incubation, the cells were then washed 3x with buffer (described above) leaving 100 $\mu$l/well. Test samples of the PRO polypeptides were prepared on different 96-well plates at 5x concentration in buffer. The positive control corresponded to 50 $\mu$M ionomycin (5x); the negative control corresponded to Protein 32. Cell plate and sample plates were run on a FLIPR (Molecular Devices) machine. The FLIPR machine added 25 $\mu$l of test sample to the cells, and readings were taken every second for one minute, then every 3 seconds for the next three minutes.

The fluorescence change from baseline to the maximum rise of the curve ($\Delta$ change) was calculated, and replicates averaged. The rate of fluorescence increase was monitored, and only those samples which had a $\Delta$ change greater than 1000 and a rise within 60 seconds, were considered positive. In the following TABLE 9, the results are expressed relative to the positive control.

TABLE 9

Human Venous Endothelial Cell Ca Flux Assay

| PRO Name | PRO Concentration | Relative $\Delta$ in Fluorescence |
|---|---|---|
| PRO179 | 0.01% | 1.0 |
| PRO179 | 0.10% | 1.0 |
| PRO179 | 1.0% | 3.0 |
| PRO179 | 0.01% | 1.0 |
| PRO179 | 0.10% | 1.0 |
| PRO179 | 1.0% | 3.0 |
| PRO245 | 0.093 nM | 1.0 |
| PRO245 | 0.93 nM | 1.0 |
| PRO245 | 9.3 nM | 2.0 |
| PR0771 | 0.075 nM | 1.0 |
| PRO771 | 0.75 nM | 1.0 |
| PR0771 | 7.5 nM | 2.0 |
| PRO1313 | 0.611 nM | 1.0 |
| PRO1313 | 6.11 nM | 1.0 |
| PRO1313 | 61.1 nM | 4.0 |
| PRO1313 | 0.611 nM | 1.0 |
| PRO1313 | 6.11 nM | 1.0 |
| PRO1313 | 61.1 nM | 3.0 |
| PRO1376 | 0.86 nM | 1.0 |
| PRO1376 | 8.6 nM | 1.0 |

(continued)

Human Venous Endothelial Cell Ca Flux Assay

| PRO Name | PRO Concentration | Relative Δ in Fluorescence |
|---|---|---|
| PRO1376 | 86.0 nM | 2.0 |
| PRO1376 | 0.86 nM | 1.0 |
| PRO1376 | 8.6 nM | 1.0 |
| PRO1376 | 86.0 nM | 2.0 |
| PRO1561 | 0.023 nM | 1.0 |
| PRO1561 | 0.23 nM | 1.0 |
| PRO1561 | 23.0 nM | 2.0 |

EXAMPLE 59

Induction of Endothelial Cell Apoptosis

The ability of PRO polypeptides to induce apoptosis in endothelial cells was tested in human venous umbilical vein endothelial cells (HUVEC, Cell Systems).

The cells were plated on 96-well microtiterplates (Amersham Life Science, cytostar-Tscintillating microplate, RPNQ160, sterile, tissue-culture treated, individually wrapped), in 10% serum (CSG-medium, Cell Systems), at a density of 2 x $10^4$ cells per well in a total volume of 100 $\mu$l. On day 2, test samples containing the PRO polypeptide were added in triplicate at dilutions of 1%, 0.33% and 0.11%. Wells without cells were used as a blank and wells with cells only were used as a negative control. As a positive control 1:3 serial dilutions of 50 $\mu$l of a 3x stock of staurosporine were used. The ability of the PRO polypeptide to induce apoptosis was determined by processing of the 96 well plates for detection of Annexin V, a member of the calcium and phospholipid binding proteins, to detect apoptosis.

0.2 ml Annexin V - Biotin stock solution (100 $\mu$g/ml) was diluted in 4.6 ml 2 x $Ca^{2+}$ binding buffer and 2.5% BSA (1:25 dilution). 50 $\mu$l of the diluted Annexin V - Biotin solution was added to each well (except controls) to a final concentration of 1.0 $\mu$g/ml. The samples were incubated for 10-15 minutes with Annexin-Biotin prior to direct addition of $^{35}$S-Streptavidin. $^{35}$S-Streptavidin was diluted in 2x $Ca^{2+}$ Binding buffer, 2.5% BSA and was added to all wells at a final concentration of 3 x $10^4$ cpm/well. The plates were then sealed, centrifuged at 1000 rpm for 15 minutes and placed on orbital shaker for 2 hours. The analysis was performed on a 1450 Microbeta Trilux (Wallac). Percent above background represents the percentage amount of counts per minute above the negative controls. Percents greater than or equal to 30% above background are considered positive. PRO178, PRO179, PRO188, PRO217, PRO261, PRO301, PRO538 and PRO719 gave positive results (induced endothelial cell apoptosis) in the above described assay.

EXAMPLE 60

Induction of Endothelial Cell Apoptosis (ELISA)

The ability of PRO polypeptides to induce apoptosis in endothelial cells was tested in human venous umbilical vein endothelial cells (HUVEC. Cell Systems) using a 96-well fomiat, in 0% serum media supplemented with 100 ng/ml VEGF, 0.1% BSA, IX penn/strep. The 96-well plates used were manufactured by Falcon (No. 3072). Coating of 96 well plates were prepared by allowing gelatinization to occur for>30 minutes with 100 $\mu$l of 0.2% gelatin in PBS solution. The gelatin mix was aspirated thoroughly before plating HUVE cells at a final concentration of 2 x $10^4$ cells/ml in 10% scrum containing medium - 100 $\mu$l volume per well. The cells were grown for 24 hours before adding test samples containing the PRO polypeptide of interest.

To all wells, 100 $\mu$l of 0% serum media (Cell Systems) complemented with 100 ng/ml VEGF. 0.1% BSA, 1 X penn/strep. was added. Test samples containing PRO polypeptides were added in triplicate at dilutions of 1%. 0.33% and 0.11%. Wells without cells were used as a blank and wells with cells only were used as a negative control. As a positive control 1:3 serial dilutions of 50 $\mu$l of a 3x stock of staurosporine were used. The cells were incubated for 24 to 35 hours prior to ELISA.

ELISA was used to determine levels of apoptosis preparing solutions according to the Boehringer Manual [Boehringer, Cell Death Detection ELISA plus, Cat No. 1 920 685]. Sample preparations: 96 well plates were spun down at 1 krpm for 10 minutes (200g): the supernatant was removed by fast inversion, placing the plate upside down on a paper towel to remove residual liquid. To each well, 200 $\mu$l of IX Lysis buffer was added and incubation allowed at room temperature for 30 minutes without shaking. The plates were spun down for 10 minutes at 1 krpm, and 20 $\mu$l of the lysate (cytoplasmic fraction) was transferred into streptavidin coated MTP. 80 $\mu$l of immunoreagent mix was added to the 20

μl lysate in each well. The MTP was covered with adhesive foil and incubated at room tempearature for 2 hours by placing it on an orbital shaker (200 rpm). After two hours, the supernatant was removed by suction and the wells rinsed three times with 250 μl of IX incubation buffer per well (removed by suction). Substrate solution was added (100 μl) into each well and incubated on an orbital shaker at room temperature at 250 rpm until color development was sufficient for a photometric analysis (approx. after 10-20 minutes). A 96 well reader was used to read the plates at 405 nm, reference wavelength, 492 nm. The levels obtained for PIN 32 (control buffer) was set to 100%. Samples with levels >130% were considered positive for induction of apoptosis. PRO172 and PRO235 gave positive results as measured by the ELISA assay described above.

EXAMPLE 61

Detection of Endothelial Cell Apoptosis ( FACS)

The ability of PRO polypeptides to induce apoptosis in endothelial cells was tested in human venous umbilical vein endothelial cells (HUVEC, Cell Systems) in gelatinized T175 flasks using HUVE cells below passage 10. On day one, the cells were split [420,000 cells per gelatinized 6 cm dishes - (11 x $10^3$ cells/$cm^2$ Falcon, Primaria)] and grown in media containing serum (CS-C. Cell System) overnight or for 16 hours to 24 hours.

On day 2, the cells were washed 1x with 5 ml PBS : 3 ml of 0% serum medium was added with VEGF (100 ng/ml); and 30 μl of the PRO test compound (final dilution 1%) was added. The mixture containing cells, medium and test PRO compound were incubated for 48 hours before harvesting.

The cells were then harvested for FACS analysis. The medium was aspired and the cells washed once with PBS. 5 ml of I x trypsin was added to the cells in a T-175 flask, and the cells were allowed to stand until they were released from the plate (about 5-10 minutes). Trypsinization was stopped by adding 5 ml of growth media. The cells were spun at 1000 rpm for 5 minutes at 4°C. The media was aspirated and the cells were resuspended in 10 ml of 10% serum complemented medium (Cell Systems), 5 μl of Annexin-FTTC (BioVison) added and chilled tubes were submitted for FACS.

PRO331 and PRO364 gave positive results by the above described assay.

EXAMPLE 62

*In situ* Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis, and aid in chromosome mapping.

*In situ* hybridization was performed following an optimized version of the protocol by Lu and Gillett, Cell Vision, 1: 169-176 (1994), using PCR-generated $^{33}$P-labeled riboprobes. Briefly, formalin-fixed, paraffin-embedded human tissues were sectioned, deparaffinized, deproteinated in proteinase K (20 g/ml) for 15 minutes at 37°C, and further processed for *in situ* hybridization as described by Lu and Gillett, *supra.* A ($^{33}$-P)UTP-labeled antisense riboprobe was generated from a PCR product and hybridized at 55°C overnight. The slides were dipped in Kodak NTB2™ nuclear track emulsion and exposed for 4 weeks.

$^{33}$P-Riboprobe synthesis

6.0 μl (125 mCi) of $^{33}$P-UTP (Amersham BF 1002, SA<2000 Ci/mmol) were speed-vacuum dried. To each tube containing dried $^{33}$P-UTP, the following ingredients were added:

2.0 μl 5x transcription buffer
1.0 μl DTT (100 mM)
2.0 μl NTP mix (2.5 mM: 10 μl each of 10 mM GTP, CTP & ATP + 10 μl $H_2O$)
1.0 μl UTP (50 μM)
1.0 μl RNAsin
1.0 μl DNA template (1 μg)
1.0 μl $H_2O$
1.0 μl RNA polymerase (for PCR products T3 = AS, T7 = S, usually)

The tubes were incubated at 37°C for one hour. A total of 1.0 μl RQI DNase was added, followed by incubation at 37°C for 15 minutes. A total of 90 μl TE (10 mM Tris pH 7.6/1 mM EDTA pH 8.0) was added, and the mixture was pipetted onto DE81 paper. The remaining solution was loaded in a MICROCON-50™ ultrafiltration unit, and spun using program 10 (6 minutes). The filtration unit was inverted over a second tube and spun using program 2 (3 minutes). After the final recovery spin, a total of 100 μl TE was added, then I μl of the final product was pipetted on DE81 paper and counted in 6 ml of BIOFLUOR II™.

The probe was run on a TBE/urea gel. A total of 1-3 μl of the probe or 5 μl of RNA Mrk III was added to 3 μl of loading buffer. After heating on a 95°C heat block for three minutes, the gel was immediately placed on ice. The wells of gel were flushed, and the sample was loaded and run at 180-250 volts for 45 minutes. The gel was wrapped in plastic wrap (SARAN™ brand) and exposed to XAR film with an intensifying screen in a -70°C freezer one hour to overnight.

$^{33}$P-Hybridization

A. *Pretreatment of frozen sections*

The slides were removed from the freezer, placed on aluminum trays, and thawed at room temperature for 5 minutes. The trays were placed in a 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ $H_2O$). After deproteination in 0.5 μg/ml proteinase K for 10 minutes at 37°C (12.5 μl of 10 mg/ml stock in 250 ml prewarmed RNAse-free RNAse buffer), the sections were washed in 0.5 x SSC for 10 minutes at room temperature. The sections were dehydrated in 70%, 95%, and 100% ethanol, 2 minutes each.

B. *Pretreatment of paraffin-embedded sections*

The slides were deparaffinized, placed in SQ $H_2O$, and rinsed twice in 2 x SSC at room temperature, for 5 minutes each time. The sections were deproteinated in 20 μg/ml proteinase K (500 μl of 10 mg/ml in 250 ml RNase-free RNase buffer; 37°C, 15 minutes) for human embryo tissue, or 8 x proteinase K(100 μl in 250 ml Rnase buffer, 37°C, 30 minutes) for formalin tissues. Subsequent rinsing in 0.5 x SSC and dehydration were performed as described above.

C. *Prehybridization*

The slides were laid out in a plastic box lined with Box buffer (4 x SSC, 50% formamide) - saturated filter paper. The tissue was covered with 50 μl of hybridization buffer (3.75 g dextran sulfate + 6 ml SQ $H_2O$), vortexed, and heated in the microwave for 2 minutes with the cap loosened. After cooling on ice, 18.75 ml formamide, 3.75 ml 20 x SSC, and 9 ml SQ $H_2O$ were added, and the tissue was vortexed well and incubated at 42°C for 1-4 hours.

D. *Hybridization*

1.0 x $10^6$ cpm probe and 1.0 μl tRNA (50 mg/ml stock) per slide were heated at 95°C for 3 minutes. The slides were cooled on ice, and 48 μl hybridization buffer was added per slide. After vortexing, 50 μl $^{33}$P mix was added to 50 μl prehybridization on the slide. The slides were incubated overnight at 55°C.

E. *Washes*

Washing was done for 2x 10 minutes with 2xSSC, EDTA at room temperature (400 ml20 x SSC + 16 ml 0.25 M EDTA, $V_f$=4L). followed by RNAseA treatment at 37°C for 30 minutes (500 μl of 10 mg/ml in 250 ml Rnase buffer = 20 μg/ml), The slides were washed 2 x10 minutes with 2 x SSC, EDTA at room temperature. The stringency wash conditions were as follows: 2 hours at 55°C, 0.1 x SSC, EDTA (20 ml 20 x SSC+ 16 ml EDTA, $V_f$=4L).

F. *Oligonucleotides*

*In situ* analysis was performed on twelve of the DNA sequences disclosed herein. The oligonucleotides employed for these analyses are as follows:

(I) DNA23339-1130 (PRO178)

p1:

5'-GGA TTC TAA TAC GAC TCA CTA TAG GGC CAC GGG CGC TGTGTG CTG GAG-3' (SEQ ID NO:231)

p2:

5'-CTA TGAAATTAA CCC TCA CTA AAG GGA TGG TGC GGA CCGCAG GGT GAC-3'(SEQ ID NO:232)

p3:

5'-GGATTCTAA TACGACTCACTATAGGGCCCGCCACGAGGAGCTGTTACG-3' (SEQ ID NO:233)

p4:

5'-CTA TGA AAT TAA CCC TCA CTA AAG GGA TGA CCT GCA GGC ATG GGA GAA-3'(SEQ ID NO:234)

p5:

5'-GGA TTC TAA TAC GAC TCA CTA TAG GGC GGC CGC CAC GAG GAG CTG TTA-3' (SEQ ID NO:235)

p6:

5'-CTA TGA AAT TAA CCC TCA CTA AAG GGA GGG GCT CTG GGG CTG GGT C-3' (SEQ ID NO:236)

(2) DNA28497-1130 (PRO188)

p1:

5'-GGA TTC TAA TAC GAC TCA CTA TAG GGC CAA CAC CAA GGG GCA AGA TG-3' (SEQ ID NO:237)

p2:

5'-CTA TGA AAT TAA CCC TCA CTA AAG GGA GGG CTT TTG GTG GGA GAA GTT-3'(SEQ ID NO:238)

(3) DNA30942-1134 (PRO212)

p1:

5'-GGA TTC TAA TAC GAC TCA CTA TAG GGC TCG CTG CTG TGC CTG GTG TTG-3' (SEQ ID NO:239)

p2:

5'-CTA TGA AATTAA CCC TCA CTA AAG GGA CCG CTG CAG CCT CTT GAT GGA-3' (SEQ ID NO:240)

(4) DNA32286-1191 (PRO214)

p1:

5'-GGA TTC TAA TAC GAC TCA CTA TAG GGC CCC TCC TGC CTT CCC TGT CC-3' (SEQ ID NO:241)

p2:

5'-CTA TGA AAT TAA CCC TCA CTA AAG GGA GTG GTG GCC GCG ATT ATC TGC-3' (SEQ ID NO:242)

(5) DNA33094-1131 (PRO217)

p1:

5'-GGA TTC TAA TAC GAC TCA CTA TAG GGCTCA GAA AAG CGC AAC AGA GAA-3'(SEQ ID NO:243)

p2:

5'-CTA TGA AAT TAA CCC TCA CTA AAG GGA TGT CTT CCA TGC CAA CCT TC-3' (SEQ ID NO:244)

(6) DNA33221-1133 (PRO224)

p1:

5'-GGA TTC TAA TAC GAC TCA CTA TAG GGC GCA GCG ATG GCA GCG ATG AGG-3'(SEQ ID NO:245)

p2:

5'-CTA TGA AATTAA CCCTCACTA AAG GGA CAG ACG GGG CAG CAG GGAGTG-3'(SEQ ID NO:246)

(7) DNA35638-1141 (PRO245)

p1:

5'-GGA TTC TAA TAG GAC TCA CTA TAG GGC GGG AAG ATG GCC AGG AGG AG-3' (SEQ ID NO:247)

p2:

5'-CTA TGA AAT TAA CCCTCA CTA AAG GGA CCA AGG CCA CAA ACG GAA ATC-3'(SEQ ID NO:248)

(8) DNA33473-1176(PRO261)

p1:

5'-GGA TTC TAA TAC GAC TCA CTA TAG GGC GCG AGG ACG GCG GCT TCA-3' (SEQ ID NO:249)

p2:

5'-CTA TGA AAT TAA CCC TCA CTA AAG GGA AGA GTC GCG GCC GCC CTT TTT-3' (SEQ ID NO:250)

(9) DNA40628-1216 (PRO301)

p1:

5'-GGA TTC TAA TAC GAC TCA CTA TAG GGC GAG TCC TTC GGC GGC TGT T-3' (SEQ ID NO:251)

p2:

5'-CTA TGA AAT TAA CCC TCA CTA AAG GGA CGG GTG CTT TTG GGA TTC GTA-3' (SEQ ID NO:252)

(10) DNA47365-1206 (PRO364)

p1:

5'-GGA TTC TAA TAC GAC TCA CTA TAG GGC AAC CCG AGC ATG GCA CAG CAC-3' (SEQ ID NO:253)

p2:

5'-CTA TGA AAT TAA CCC TCA CTA AAG GGA TCT CCC AGC CGC CCC TTC TC-3'(SEQ ID NO:254)

(11) DNA29101-1122(PRO713)

p1:

5'-GGA TTC TAA TAC GAC TCA CTA TAG GGC GGC GGA ATC CAA CCT GAG TAG-3'(SEQ ID NO:255)

p2:

5'-CTA TGA AATTAA CCC TCA CTA AAG GGA GCG GCT ATC CTC CTG TGC TC-3' (SEQ ID NO:256)

(12) DNA33087 (PRO216)

p1:

5'-GGA TTC TAA TAC GAC TCA CTA TAG GGC CCC GAG TGT TTT CCA AGA-3' (SEQ ID NO:257)

p2:

5'-CTA TGA AATTAA CCC TCA CTA AAG GGA CAA GTT TAC TAG CCC ATC CAT-3' (SEQ ID NO:258)

p3:

5'-GGA TTC TAA TAC GAC TCA CTA TAG GGC TGG ATG GGC TAG TAA ACTTGA-3' (SEQ ID NO:259)

p4:

5'-CTA TGA AAT TAA CCC TCA CTA AAG GGA CCC TTC TGC TCC TTC TTG TT -3' (SEQ ID NO:260)

*G. Results*

*In situ* analysis was performed on the above twelve DNA sequences disclosed herein. The results from these analyses are as follows:

(1) DNA2339-1130 (PRO178)

In fetal lower limb, a distinctive expression pattern was observed at the connective tissue interface between skeletal muscle and bone (primitive periosteum). Expression was also seen adjacent to vascular tissue, thereby indicating a possible link with angiogenesis. In the body wall, a similar expression pattern to lower limb expression was observed. Expression was seen in the smooth muscle of the trachea. Also, expression was seen in the small intestine and stomach in smooth muscle/connective tissue of lamina propria. Cord vascular smooth muscle was also positive. The highly organized expression pattern in the developing limb, intestine and body wall suggests a distinctive role at these sites. Possibilities would include angiogenesis and patterning.

Possible increased expression appeared in the medulla of the fetal thymus as well as in fetal brain cerebral cortex (cortical neurones). The following fetal tissues did not show positive results: spinal cord, thyroid, adrenals, liver and placenta.

All adult tissues examined were negative.

(2) DNA28497-1130 (PRO188)

In fetal lower limb, high expression was observed at sites of enchondral bone formation, in osteocytes and in periosteum/perichondrium of developing bones. This distribution suggests a role in bone formation/differentiation. A faint increase in expression was seen over thyroid epithelial cells. In the body wall, high expression was observed in osteocytes as well as in the periosteum/perichondrium of developing bones. This distribution suggests a role in bone formation and differentiation. No expression was seen in the following fetal tissues: thymus, trachea, brain (cerebral cortex), spinal cord, small intestine, adrenals, liver, stomach, placenta and cord.

In adult tissues, expression was seen over benign breast epithelium, areas of apocrine metaplasia and sclerosing adenosis. In addition, expression was seen over infiltrating breast ductal carcinoma cells No expression was observed in adult liver, heart or hepatocellular carcinoma.

Possible expression appeared in adult squamous epithelium of skin and in adult adrenal cortex. All other tissues were negative. Fetal multiblock tissues included: liver, kidney, adrenals, thyroid, lungs, heart, great vessels, small intestine, spleen, thymus, pancreas, brain, spinal cord, body wall, pelvis and lower limb. Adult multiblock tissues included: liver, kidney, adrenal, myocardium, aorta, spleen, lymph node, pancreas, lung and skin.

(3)DNA30942-1134 (PRO212)

*Expression in Lung Carcinomas, Tumor Block, and Breast Corcinomas*

Expression was observed in mononuclear phagocytes in the normal chimp thymus, as well as in one out of one gastric carcinomas, one out of one colorecial cancers, two out of five breast cancers and one out of four lung cancers examined. Expression was observed by malignant cells in an ostcosarcoma and a poorfy differentiated liposarcoma. A possible signal was seen in the malignant cells of a testicular teratoma and one out of five breast cancers examined. In one of the lung cancers, a scattered signal was seen over a high endothelial venule within pulmonary lymphoid tissue.

Fetal tissues examined (E12-E 16 weeks) included: placenta, umbilical cord, liver, kidney, adrenals, thyroid, lung, heart, great vessels, oesophagus, stomach, small intestine, spleen, thymus, pancreas, brain, eye, spinal cord, body wall, pelvis and lower limb. Adult human tissues examined included: liver, kidney, adrenals, myocardium, aorta, spleen, lung, skin, chondrosarcoma, eye, stomach, gastric carcinoma, colon, colonic carcinoma, renal cell carcinoma, prostate, bladder mucosa and gallbladder, and acetominophen induced liver injury and hepatic cirrhosis. Rhesus tissues examined included cerebral cortex (rm) and hippocampus (rm). Chimp tissues examined included: thyroid, parathyroid, ovary, nerve, tongue, thymus, adrenal gastric mucosa and salivary gland. *Expression in Lung Adenocarcinoma and Squamous Carcinomas*

Eight adenocarcinomas and seven squamous lung carcinomas were examined. Actins were strongly positive in all tumors, indicating that all were suitable for *in situ* hybridization analysis. Expression was observed in six of the tumors as follows:

| | |
|---|---|
| 6727-95 squamous carcinoma | - strongly expressed over neoplastic epithelium |
| 9558-95 squamous carcinoma | - expression over neoplastic epithelium |
| 12235-95 adenocarcinoma | - expression over *in situ* and infiltrating tumor cells |
| 6545-95 & 4187-97 squamous carcinomas | - expression over cells in tumor stroma, no expression seen over tumor cells |
| 12954-94 squamous carcinoma | - possible weak expression over stromal cells |

(4) DNA32286-1191 (PRO214)

In fetal tissue, low level expression was seen throughout the mesenchyme. Moderate expression was observed in placental stromal cells in membraneous tissues and in the thyroid. Low level expression was also seen in cortical neurones. Adult tissues were all negative.

Fetal tissues examined (E12-E16 weeks) included: placenta, umbilical cord, liver, kidney, adrenals, thyroid, lungs, heart, great vessels, oesophagus, stomach, small intestine, spleen, thymus, pancreas, brain, eye, spinal cord, body wall, pelvis and lower limb. Adult tissues examined included: liver, kidney, adrenal, myocardium, aorta, spleen, lymph node, pancreas, lung and skin.

(5) DNA33094-1131 (PRO217)

A highly distinctive expression pattern was observed as follows: in the human embryo, expression was observed in the outer smooth muscle layer of the GI tract, respiratory cartilage, branching respiratory epithelium, osteoblasts, tendons, gonad, in the optic nerve head and developing dermis. In the adult, expression was observed in the epidermal pegs of the chimp tongue, the basal epithelial/myoepithelial cells of the prostate and urinary bladder. Also, expression was seen in the alveolar lining cells of the adult lung, mesenchymal cells juxtaposed to erectile tissue in the penis and the cerebral cortex (probably glial cells). In the kidney, expression was only seen in disease, in cells surrounding thyroidized renal tubules.

Human fetal tissues examined (E12-E16 weeks) included: placenta, umbilical cord, liver, kidney, adrenals, thyroid, lung, heart, great vessels, oesophagus, stomach, small intestine, spleen, thymus, pancreas, brain, eye, spinal cord, body wall, pelvis and lower limb. Adult human tissues examined included: kidney (normal and end stage), adrenals, myocardium, aorta, spleen, lymph node, gall bladder, pancreas, lung, skin, eye (including retina), prostate, bladder, and liver (normal, cirrhotic, acute failure).

Non-human primate tissues examined included: Chimp tissues (salivary gland, stomach, thyroid, parathyroid, skin, thymus, ovary, lymph node): Rhesus Monkey tissues (cerebral cortex, hippocampus, cerebellum, penis).

(6) DNA33221-1133 (PRO224)

Expression was limited to vascular endothelium in fetal spleen, adult spleen, fetal liver, adult thyroid and adult lymph node (chimp). Additional site of expression was seen in the developing spinal ganglia. All other tissues were negative.

Human fetal tissues examined (E12-E16 weeks) included: placenta, umbilical cord, liver, kidney, adrenals, thyroid, lung, heart, great vessels, oesophagus, stomach, small intestine, spleen, thymus, pancreas, brain, eye, spinal cord, body wall, pelvis and lower limb. Adult human tissues examined included: kidney (normal and end-stage), adrenals, myocardium, aorta, spleen, lymph node, pancreas, lung, skin, eye (including retina), bladder, liver, (normal, cirrhotic, acute failure). Non-human primate tissues examined included: Chimp tissues (salivary gland, stomach, thyroid, parathyroid, skin, thymus, ovary, lymph node); Rhesus Monkey tissues (cerebral cortex, hippocampus, cerebellum, penis).

(7) DNA35638-1141 (PRO245)

*Expression Pattern in Human Adult and Fetal Tissues*

Expression was observed in the endothelium lining a subset of fetal and placental vessels. Endothelial expression was confined to these tissue blocks. Expression was also observed over intermediate trophoblast cells of the placenta. All other tissues were negative.

Fetal tissues examined (E 12-E 16 weeks) included: placenta, umbilical cord, liver, kidney, adrenals, thyroid, lung, heart, great vessels, oesophagus, stomach, small intestine, spleen, thymus, pancreas, brain, cye, spinal cord, body wall, pelvis and lower limb. Adult tissues examined included: liver, kidney, adrenals, myocardium, aorta, spleen, lymph node, pancreas, lung, skin, cerebral cortex (rm), hippocampus (rm), cerebellum (rm), penis, eye, bladder, stomach, gastriccarcinoma.colon, colonic carcinorna, thyroid (chimp), parathyroid (chimp), ovary (chimp) and chondrosarcoma, acetominophen induced liver injury and hepatic cirrhosis.

*Expression in Inflamed Tissues (Psoriasis, IBD, Inflamed Kidney, Inflamed Lung, Hepatitis (Liver Block), Normal Tonsil, Adult and Chimp Multiblockst*

This molecule has been shown to be immunostimulatory (enhances T lymphocyte proliferation in the MLR and costimulation) and has proinflammatory properties (induces a neutrophil infiltrate *in vivo*). As indicated above, this molecule has been shown to be expressed on a subset of fetal vessels in the endothelium/intima and in the placenta but was not found to be expressed in a variety of normal adult human tissues or vessels in those tissues. An evaluation was performed for the expression of this molecule in vessels of inflamed human tissues as compared to non-inflammed tissues. In summary, expression was seen in the endothelium/intima of large vessels in the lung afflicted with chronic inflammation, in the superficial dermal vessels of psoriatic skin, in arterioles in a specimen of chronic sclerosing nephritis and in capillaries including the perifollicular sinuses of tonsil.

Expression was not observed in normal skin (human foreskin specimens), normal lung, inflamed (eight IBD specimens) or normal large bowel, chronically inflamed or cirrhotic liver, normal adult cardiac tissue, or adrenal gland.

(8) DNA33473-1176 (PRO261)

*Expression Pattern in Human Adult and Fetal Tissues*

Strong expression was observed in dermal fibroblasts in normal adult skin. Strong expression was also seen in two cirrhotic livers, at sites of active hepatic fibrosis. In addition, moderate expression was seen over fasiculata cells of the adrenal cortex. Localization of expression supports a role for this molecule in extracellular matrix formation/turnover.

*Expression in Human Breast Carcinoma and Normal Breast Tissue, and in Lung Carcinoma*

A weak diffuse signal was seen in two breast tumors examined. No expression was seen on benign and malignant epithelial cells, but specific hybridization was observed in mesenchymal cells, particularly in areas of tissue repair including dystrophic ossification. The signal appears to be localized to the same cell population, however in some areas (breast tumor 02), the signal was significantly strong. Most positive cells have the morphology of fibroblasts, but smooth muscle cells appear to be negative. The signal was less intense in lung tumor tissue, however, this section showed less tissue repair compared with the breast tumor slides. Normal lung and kidney tissue were essentially negative.

In summary, this study showed expression in mesenchymal cells involved in tissue repair and/or collagen deposition. The signal was particularly strong in benign fibroblast-like cells adjacent to either infiltrating breast carcinoma cells or tissue destruction due to benign, inflammatory conditions (duct rupture). Of note. is the fact that deposition of benign osteoid seems to correlate with strong expression of RNA.

*Expression in Normal Human Colon and Colon Carcinoma*

None of the tissue sections showed a positive hybridization signal in tumor cells. Positive signals of variable intensity were observed in mesenchy mal cells of either fibroblast or smooth muscle differentiation. Fibroblasts with a positive signal was observed adjacent to invasive tumor, if this tumor elicits a so called desmoplastic response (fibroblast proliferation with deposition of collagenous fibrosis). Positive fibroblasts were also seen in areas of tissue repair (granulation tissue or granulomatous response). Positive smooth muscle cells were seen in mostly arterial vessels of medium size

(9) DNA40628-1216 (PRO301)

*Expression in Inflamed Human Tissues (Psoriasis, IBD, Inflamed Kidney, InflamedLung, Hepatitis, Normal Tonsil, Adult and Chimp Multiblocks)*

Expression was evaluated in predominantly inflamed human tissue with few normal human and non-human primate tissues. Expression was seen in every epithelial structure evaluated including the mucosal epithelium of the colon, bronchial large airway epithelium, oral mucosa (tongue), tonsillar crypt mucosa, placental mucosa, prostatic mucosa, glandular stomach mucosa, epithelial cells of thymic Hassall's corpuscles, hepatocytes, biliary epithelium, and placental epithelium. The only evidence for expression outside of an epithelial structure was weak low, inconsistent expression in the germinal centers of follicles in a tonsil with reactive hyperplasia.

In non-human primate tissues (chimp): weak diffuse expression was seen in the epidermis of tongue epithelium; weak expression was seen in thymic epithelium of Hassall's corpuscles; and in the stomach, mild diffuse expression in the epithelium of the glandular mucosa was observed.

In human tissues:

(1) in liver (multiblock including: chronic cholangitis, lobular hyperplasia, acetominophen toxicity), there was a diffuse, low to moderate, expression in hepatocytes and biliary epithelium. Expression was most prominent in perilobular/periportal hepatocytes. It was most prominent in biliary epithelium in sections of liver with chronic sclerosing cholangitis.
(2) Weak expression was observed in psoriasis samples in the epidermis.
(3) In lung with chronic interstitial pneumonia or chronic bronchitis, low diffuse expression was seen in the mucosal epithelium of large airways: weak diffuse expression was also seen in alveolar epithelium. There was no expression in the epithelium of the submucosal glands of bronchi/bronchioles.
(4) Moderate diffuse expression was observed in both placental epithelium and in the mucosal epithelium of the gall bladder.
(5) In prostate epithelium, low diffuse expression was seen.
(6) High diffuse expression was observed in the epithelium of the tonsillar mucosa and crypts; the signal was highest in the mucosal cells which line the tonsillar crypts. There was weak inconsistent diffuse expression in the germinal centers of cortical folicles (B lymphocyte areas); however, in no other tissue evaluated with lymphoid structures or lymphocytic inflammation was there any expression in B lymphocytes.
(7) In colon with inflammatory bowel disease and polyp/adenomatous change: low expression in the mucosal epithelium was seen with expression greatest in the villi tips. In one specimen with a polyp, there was no evidence of increased expression in the dysplastic epithelium of the polyp as compared to the adjacent mucosa. There was no apparent expression in reactive mucosal lympohid tissue that was present in many of the sections.

Tissues with no expression included: heart, peripheral nerve, and muscle (cardiac, smooth).

(10) DNA47365-1206 (PRO364)

Expression was observed in the fetus, in the fascia lining the anterior surface of the vertebral body. Expression was also seen over the fetal retina. Low level expression occurred over fetal neurones. All other tissues were negative.

(11) DNA29101-1122 (PRO713)

In fetal tissues: Expression was observed in developing lower limb bones at the edge of the cartilagenous anlage (i.e. around the outer edge): in developing tendons, in vascular smooth muscle and in cells embracing developing skeletal muscle myocytes and myotubes. Expression was also observed at the epiphyseal growth plate. In fetal lymph node, expression was seen in the marginal sinus of developing lymph nodes. Expression was observed in the subcapsular region of the thymic cortex, possibly representing either the subcapsular epithelial cells or the proliferating thymocytes that are found in this region. Expression was seen additionally in the following tissues: expression in the smooth muscle of the trachea; focal expression in cortical neurones in the brain cerebral cortex; expression in the smooth muscle of the small intestine; generalized expression over the thyroid epithelium; expression in ductal plate cells of liver; expression in mural smooth muscle of the stomach; expression in the basal layer of squamous epithelium in fetal skin; expression in interstitial cells in trophoblastic villi in the placenta; and expression in wall of arteries and veins in the umbilical cord. Expression patterns suggest that this molecule may be involved in cell differentiation/proliferation.

No expression was observed in fetal spleen, spinal cord, or adrenals.

High expression was observed in the following sites:

(1) chimp ovary - granulosa cells of maturing follicles, lower intensity signal was observed over thecal cells;
(2) Chimp parathyroid - high expression was seen over chief cells;
(3) Human fetal testis - moderate expression was seen over stromal cells surrounding developing tubules;
(4) Human fetal lung - high expression was seen over chondrocytes in developing bronchial tree, and low level expression was seen over branching bronchial epithelium.

Specific expression was not observed over the renal cell, gastric and colonic carcinomas.

Fetal tissues examined (E12-E16 weeks) included: placenta, umbilical cord, liver, kidney, adrenals, thyroid, lungs, heart, great vessels, oesophagus, stomach, small intestine, spleen, thymus, pancreas, brain, eye, spinal cord, body wall, pelvis and lower limb. Adult tissues examined included: liver, kidney, adrenals, myocardium, aorta, spleen, lymph node, pancreas, lung, skin, cerebral cortex (rm), hippocampus (rm), cerebellum (rm), penis, eye, bladder, stomach, gastric carcinoma, colon, colonic carcinoma and chondrosarcoma, acetominophen induced liver injury and hepatic cirrhosis.

(12) <u>DNA33087 (PRO216)</u>

Strong specific expression was seen in osteoblasts at all sites of enchondral and periosteal new bone formation. Additional sites of expression included the developing pulmonary arterial and aortic trunks. Otherwise, all fetal tissues were negative. Tissues examined included: placenta, umbilical cord, brain, spinal cord, eye, optic nerve, trachea, lung, heart, thymus, liver, spleen, esophanus, small intestine, pancreas, adrenals, thyroid, body wall and lower limb.

Adult tissues examined were all negative and included: liver, kidney, adrenals, myocardium, aorta, spleen, lymph node, pancreas, lung and skin. All adult tissues in the multiblock were positive for beta-actin.

This molecule's probable role is in control of bone matrix deposition and/or osteoblast growth.

EXAMPLE 63

<u>Use of PRO Polypeptides as a Hybridization Probe</u>

The following method describes use of a nucleotide sequence encoding PRO polypeptides as a hybridization probe.

DNA comprising the coding sequence of full-length or mature PRO polypeptides (as shown in Figures 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, and 95, respectively, SEQ ID NOS: 3, 8, 13, 15, 20, 25, 30, 35, 40, 45, 50, 55, 61, 66, 71, 76, 84, 89, 97, 106, 111, 116, 126, 131, 136, 142, 147, 152, 154, 159, 161, 169, 180, 182, 190, 192, 194, 196, 198, 200, 202, 204, 213, 215, 217, 219, 221 and 226, respectively) or a fragment thereof is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high-stringency conditions. Hybridization of radiolabeled probe derived from the gene encoding a PRO polypeptide to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1 x SSC and 0.1% SDS at 42°C.

DNAs having a desired sequence identity with the DNA encoding full-length native sequence can then be identified using standard techniques known in the an.

EXAMPLE 64

Expression of Nucleic Acid Encoding PRO Polypeptides in *E. coli*

This Example illustrates preparation of an unglycosylated form of PRO polypeptides by recombinant expression in *E. coli.*

The DNA sequence encoding PRO187,PRO195,PRO224,PRO301,PRO364,PRO713.PRO788,PRO1274, PRO 1286, PRO1303, PRO 1312, PRO1313, and PRO 1376 (SEQ ID NOS: 20, 30, 50, 89, 116, 136, 152, 196, 198, 202, 213,215, and 217, respectively) was initially amplified using selected PCR primers. The primers should contain restriction enzyme sites that correspond to the restriction enzyme sites on the selected expression vector. A variety of expression vectors may be employed. An example of a suitable vector is pBR322 (derived from *E. coli; see* Bolivar et a/., Gene, 2: 95 (1977)), which contains genes for ampicillin and tetracycline resistance. The vector was digested with restriction enzyme and dephosphorylated. The PCR-amplified sequences were then ligated into the vector. The vector will preferably include sequences that encode an antibiotic-resistance gene, a trp promoter, a poly-His leader (including the first six STH codons, poly-His sequence, and enterokinase cleavage site), the region encoding PRO187. PRO195. PRO224, PRO301, PRO364. PRO713, PRO788, PRO1274, PRO1286, PRO1303, PRO1312. PRO1313, and PRO1376. lambda transcriptional terminator, and an argU gene.

The ligation mixture was then used to transform a selected *E. coli* strain using the methods described in Sambrook *et al., supra.* Transformants were identified by their ability to grow on LB plates and antibiotic-resistant colonies were then selected. Plasmid DNA was isolated and confirmed by restriction analysis and DNA sequencing.

Selected clones were grown overnight in liquid culture medium such as LB broth supplemented with antibiotics. The overnight culture may subsequently be used to inoculate a larger-scale culture. The cells were then grown to a desired optical density, during which the expression promoter was turned on.

After culturing the cells for several more hours, the cells were harvested by centrifugation. The cell pellet obtained by the centrifugation was solubilized using various agents known in the art, and the solubilized PRO187, PRO195, PRO224, PRO301, PRO364, PRO713, PR0788. PRO1274, PRO1286, PRO1303, PRO1312, PRO1313, and PRO1376 polypeptides were then purified using a metal-chelating column under conditions that allow tight binding of the polypeptide.

EXAMPLE 65

Expression of Nucleic Acid Encoding PRO Polypeptides in Mammalian Cells

This Example illustrates preparation of a potentially glycosylated form of PRO polypeptides by recombinant expression in mammalian cells.

The vector, pRK5 (*see,* EP 307,247, published March 15, 1989), is employed as the expression vector. Optionally, the PRO DNA is ligated into pRK5 with selected restriction enzymes to allow insertion of the DNA encoding PRO polypeptides using ligation methods such as described in Sambrook *et al., supra.* The resulting vector is called pRK5-(DNA encoding PRO).

In one embodiment, the selected host cells are 293 cells. Human 293 cells (ATCC CCL 1573) are grown to confluence in tissue culture plates in medium such as DMEM supplemented with fetal calf serum and optionally, nutrient components and or antibiotics. About 10 $\mu$g DNA of pRK5-(DNA encoding PRO) is mixed with about I $\mu$g DNA encoding the VA RNA gene (Thimmappaya et al., Cell, 31: 543 (1982)) and dissolved in 500 $\mu$l of I mM Tris-HCl, 0.1 mM EDTA. 0.227 M $CaCl_2$. To this mixture is added, dropwise, 500 $\mu$l of 50 mM HEPES (pH 7.35), 280 mM NaCl. 1.5 mM $NaPO_4$, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum-free medium, fresh medium is added, and the cells are incubated for about 5 days.

Approximately 24 hours after the transfections, the culture medium is removed and replaced with culture medium (alone) or culture medium containing 200 $\mu$Ci/ml $^{35}$S-cysteine and 200 $\mu$Ci/ml $^{35}$S-methionine. After a 12-hour incubation, the conditioned medium is collected, concentrated un a spin filter, and loaded onto a 15% SDS gel. The processed gel may be dried and exposed to film for a selected period of time to reveal the presence of the PRO polypeptide. The cultures containing transfected cells may undergo further incubation (in serum-freemedium) and the medium is tested in selected bioassays.

In an alternative technique, the gene encoding the PRO polypeptide may be introduced into 293 cells transiently using the dextran sulfate method described by Somparyrac et al., Proc. Natl. Acad. Sci., 12:7575(1981). 293 cells are grown to maximal density in a spinner flask and 700 $\mu$g pRK5-(DNA encoding PRO) is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 μg/ml bovine insulin, and 0.1 μg/ml bovine transferrin. After about four days, the conditioned media is centrifuged and filtered to remove cells and debris. The sample containing the expressed gene encoding the PRO polypeptide can then be concentrated and purified by any selected method, such as dialysis and/or column chromatography.

In another embodiment, the gene encoding the PRO polypeptide can be expressed in CHO cells. The pRK5-(DNA encoding PRO) nucleic acid can be transfected into CHO cells using known reagents such as $CaPO_4$ or DEAE-dextran. As described above, the cell cultures can be incubated, and the medium replaced with culture medium (alone) or medium containing a radiolabel such as $^{35}S$-methionine. After determining the presence of the PRO polypeptide, the culture medium may be replaced with serum-free medium. Preferably, the cultures are incubated for about 6 days, and then the conditioned medium is harvested. The medium containing the expressed PRO polypeptide can then be concentrated and purified by any selected method.

Epitope-tagged gene encoding the PRO polypeptide may also be expressed in host CHO cells. The gene encoding the PRO polypeptide may be subcloned out of the pRK5 vector. The subclone insert can undergo PCR amplification to fuse in frame with a selected epitope tag such as a poly-His tag into a baculovirus expression vector. The gene insert encoding the poly-His-tagged-PRO polypeptide can then be subcloned into a SV40- driven vector containing a selection marker such as DHFR for selection of stable clones. Finally, the CHO cells can be transfected (as described above) with the SV40-driven vector. Labeling may be performed, as described above, to verify expression. The culture medium containing the expressed gene encoding the poly-His-tagged-PRO polypeptide can then be concentrated and purified by any selected method, such as by $Ni^{2-}$-chelate affinity chromatography.

PRO172, PRO 178, PRO179, PRO182, PRO188, PRO212, PRO214, PRO216, PRO217, PRO224, PRO245, PRO261, PRO301, PRO356, PRO364, PRO713, PRO788, PRO792, PRO865, PRO1126, PRO1130, PRO1244, PRO1246, PRO 1274, PRO 1286, PRO 1294, PRO1303, PRO 1304, PRO1376, and PRO 1387 were stably expressed in CHO cells by the above described method. In addition, PRO172, PRO178, PRO182, PRO231, PRO245, PRO301, PRO356 and PRO364 were expressed in CHO cells by a transient procedure.

EXAMPLE 66

Expression of Nucleic Acid Encoding PRO Polypeptides in Yeast

The following method describes recombinant expression of the gene encoding PRO polypeptides in yeast.

First, yeast expression vectors arc constructed for intracellular production or secretion of the PRO polypeptide from the ADH2/GAPDH promoter. UNA encoding the PRO polypeptide and the promoter is inserted into suitable restriction enzyme sites in the selected plasmid to direct intracellular expression of the gene encoding the PRO polypeptide For secretion, DNA encoding the PRO polypeptide can be cloned into the selected plasmid, together with DNA encoding the ADH2/GAPDH promoter, a native PRO polypeptide signal peptide or other mammalian signal peptide, or, for example, a yeast alpha-factor or invertase secretory signal/leader sequence, and linker sequences (if needed) for expression of the gene encoding the PRO polypeptide.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO polypeptides can subsequently be isolated and purified by removing the yeast cells from the fermentation medium by centrifugation and then concentrating the medium using selected cartridge filters. The concentrate containing the PRO polypeptide may further be purified using selected column-chromatography resins.

EXAMPLE 67

Expression of Nucleic Acid Encoding PRO Polypeptides in Baculovirus-Infected Insect Cells

The following method describes recombinant expression in Baculovirus-infected insect cells.

The sequence coding for the PRO polypeptide is fused upstream of an epitope tag contained within a baculovirus expression vector. Such epitope tags include poly-His tags and immunoglobulin tags (like Fc regions of IgG). A variety of plasmids may be employed, including plasmids derived from commercially available plasmids such as pVL1393 (Novagen). Briefly, the sequence encoding the PRO polypeptide or the desired portion of the coding sequence of the PRO polypeptide [such as the sequence encoding the extracellular domain of a transmembrane protein or the sequence encoding the mature protein if the protein is extracellular] is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGoldTM virus DNA (Pharmingen) into *Spodoptera frugiperda* ("Sf9") cells (ATCCCRL 1711) using lipofectin (commercially available from GIBCO-BRL). After 4 - 5 days of incubation at 28°C, the released viruses are harvested and used for further amplifications. Viral infection and protein expression are performed as described by O'Reillcy et al., Baculovirus Expression Vectors: A Laboratory Manual (Oxford: Oxford University Press (1994)).

Expressed poly-His tagged-PRO polypeptides can then be purified, for example, by Ni $^{2+}$-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 ml Hepes, pH 7.9; 12.5 mM $MgCl_2$; 0.1 mM EDTA; 10% glycerol; 0.1% NP-40; 0.4 M KCl), and sonicated twice for 20 seconds on ice. The sonicates are cleared by centrifugation and the supernatant is diluted 50-fold in loading buffer (50 mM phosphate, 300 mM NaCl. 10% glycerol, pH 7.8) and filtered through a 0.45 $\mu$m filter. A $Ni^{2+}$-NTA agarose column (commercially available from Qiagen) is prepared with a bed volume of 5 ml, washed with 25 ml of water and equilibrated with 25 ml of loading buffer. The filtered cell extract is loaded onto the column at 0.5 ml per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes non-specifically-bound protein. After reaching an $A_{280}$ baseline again, the column is developed with a 0 to 500 mM imidazole gradient in the secondary wash buffer. One ml fractions arc collected and analyzed by SDS-PAGE and silver staining or Western blot with Ni $^{2+}$NTA-conjugated to alkaline phosphatase (Qiagen). Fractions containing the eluted $His_{10}$-tagged-PRO polypeptide are pooled and dialyzed against loading buffer.

Alternatively, purification of the IgG-tagged (or Fc tagged)-PRO polypeptide can be performed using known chromatography techniques, including for instance, Protein A or protein G column chromatography.

PRO172, PRO178, PRO214, PRO216, PRO231, PRO235, PRO269, PRO301, PRO356, PRO538, PRO719, and PRO1376 were expressed in baculovirus infected Sf9 insect cells. While expression was actually performed in a 0.5-2 L scale, it can be readily scaled up for larger (*e.g.,* 8 L) preparations. The proteins are expressed as an IgG construct (immunoadhesin), in which the protein extracellular region is fused to an IgG1 constant region sequence containing the hinge, CH2 and CH3 domains and/or in poly-His tagged forms.

Following PCR amplification. the respective coding sequences are subcloned into a baculovirus expression vector (pb.PH.IgG for IgG fusions and pb.PH.His.c for poly-His tagged proteins), and the vector and Baculogold® baculovirus DNA (Pharmingen) are co-transfected into 105 *Spodoptera frugiperda* ("Sf9") cells (ATCC CRL 1711), using Lipofectin (Gibco BRL). pb.PH.IgG and pb.PH.His are modifications of the commercially available baculovirus expression vector pVL1393 (Pharmingen), with modified polylinker regions to include the His or Fc tag sequences. The cells are grown in Hink's TNM-FH medium supplemented with 10% FBS (Hyclone). Cells are incubated for 5 days at 28°C. The supernatant is harvested and subsequently used for the first viral amplification by infecting Sf9 cells in Hink's TNM-FH medium supplemented with 10% FBS at an approximate multiplicity of infection (MOI) of 10. Cells are incubated for 3 days at 28°C. The supernatant is harvested and the expression of the constructs in the baculovirus expression vector is determined by batch binding of 1 ml of supernatant to 25 ml of Ni $^{2+}$-NTA beads (QIAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The first viral amplification supernatant is used to infect a spinner culture (500 ml) of Sf9 cells grown in ESF-921 medium (Expression Systems LLC) at an approximate MOI of 0.1. Cells arc incubated for 3 days at 28 °C. The supernatant is harvested and Filtered. Batch binding and SDS-PAGE analysis is repeated, as necessary, until expression of the spinner culture is confirmed.

The conditioned medium from the transfected cells (0.5 to 3 L) is harvested by centrifugation to remove the cells and filtered through 0.22 micron filters. Fur the poly-His tagged constructs, the protein construct is purified using a Ni $^{2+}$-NTA column (Qiagen) Before purification, imidazole is added to the conditioned media to a concentration of 5 mM. The conditioned media is pumped onto a 6 ml Ni $^{2+}$-NTA column equilibrated in 20 mM Hepes, pH 7.4. buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at-80°C.

Immunoadhesin (Fc containing) constructs of proteins are purified from the conditioned media as follows. The conditioned media is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 ml of 1 M Tris buffer, pH 9. The highly purified protein is subsequently desalted into storage buffer as described above for the poly-His tagged proteins. The homogeneity of the proteins is verified by SDS polyacrylamide gel (PEG) electrophoresis and N-terminal amino acid sequencing by Edman degradation.

Alternatively, a modified baculovirus procedure may be used incorporating high-5 cells. In this procedure, the DNA encoding the desired sequence is amplified with suitable systems, such as Pfu (Stratagene), or fused upstream (5'-of) of an epitope tag contained with a baculovirus expression vector. Such epitope tags include poly-His tags and immunoglobulin tags (like Feregions of 1gG). A variety of plasmids may be employed, including plasmids derived from commercially available plasmids such as pIE1-1 (Novagen). The pIE1-1 and pIE1-2 vectors are designed for constitutive expression of recombinant proteins from the baculovirus iel promoter in stably-transformed insect cells. The plasmids differ only in the orientation ofthe multiple cloning sites and contain all promoter sequences known to be important for ie I-mediated gene expression in uninfected insect cells as well as the hr5 enhancer element. pIE1-1 and pIE1-2 include the translation initiation site and can be used to produce fusion proteins. Briefly, the desired sequence or the desired portion of the sequence (such as the sequence encoding the extracellular domain of a transmembrane protein) is amplified by PCR with primers complementary to the 5' and 3' regions. The 5' primer may incorporate flanking (selected) restriction enzyme sites. The product is then digested with those selected restriction enzymes and subcloned into the expression vector. For example, derivatives of pIE1-1 can include the Fc region of human IgG (pb. PH.IgG) or an 8 histidine (pb.PH.His) tag downstream (3'-of the desired sequence). Preferably. the vector construct is sequenced for confirmation.

High-5 cells are grown to a confluency of 50% under the conditions of. 27°C, no $CO_2$, NO pen/strep. For each 150 mm plate, 30 μg of pIE based vector containing the sequence is mixed with I ml Ex-Cell medium [Media: Ex-Cell 401 + 1/100 L-Glu JRH Biosciences #14401-78P (note: this media is light sensitive)], and in a separate tube, 100 μl of CellFectin [CellFECTIN (GibcoBRL #10362-010) (vortexed to mix)] is mixed with I ml of Ex-Cell medium. The two solutions are combined and allowed to incubate at room temperature for 15 minutes. 8 ml of Ex-Cell media is added to the 2 ml of DNA/CellFECTIN mix and this is layered on high-5 cells that have been washed once with Ex-Cell media. The plate is then incubated in darkness for 1 hour at room temperature. The DNA/CellFECTIN mix is then aspirated, and the cells are washed once with Ex-Cell to remove excess CellFECTIN, 30 ml of fresh Ex-Cell media is added and the cells are incubated for 3 days at 28°C. The supernatant is harvested and the expression of the sequence in the baculovirus expression vector is determined by batch binding of I ml of supernatant to 25 ml of Ni $^{2+}$-NTA beads (QIAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The conditioned media from the transfected cells (0.5 to 3 L) is harvested by centrifugation to remove the cells and filtered through 0.22 micron filters. For the poly-His tagged constructs, the protein comprising the sequence is purified using a Ni $^{2+}$-NTA column (Qiagen). Before purification, imidazole is added to the conditioned media to a concentration of 5 mM. The conditioned media is pumped onto a 6 ml Ni $^{2+}$-NTA column equilibrated in 20 mM Hepes, pH 7.4, buffer containing 0.3 M NaCl and 5 mM imidazole at a flow rate of 4-5 ml/min. at 48°C. After loading, the column is washed with additional equilibration buffer and the protein eluted with equilibration buffer containing 0.25 M imidazole. The highly purified protein is then subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc containing) constructs of proteins are purified from the conditioned media as follows. The conditioned media is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting I ml fractions into tubes containing 275 ml of 1 M Tris buffer, pH 9. The highly purified protein is subsequently desalted into storage buffer as described above for the poly-His tagged proteins. The homogeneity of the sequence is assessed by SDS polyacrylamide gels and by N-terminal amino acid sequencing by Edman degradation and other analytical procedures as desired or necessary.

PRO172, PRO178, PRO179, PRO182, PRO187, PRO188, PRO212, PRO216, PRO217, PRO224, PRO231, PRO235, PRO269, PRO287, PRO301, PRO323, PRO331, PRO356, PRO364, PRO526, PRO538, PRO713, PRO771, PRO788, PRO792, PRO812, PRO865, PRO1075, PRO1154, PRO1244, PRO1286, PRO1304, PRO1312, PRO1376, PRO1387, and PRO1561 were successfully expressed by the above modified baculovirus procedure incorporating high-5 cells.

EXAMPLE 68

Preparation of Antibodies that Bind PRO Polypeptides

This Example illustrates preparation of monoclonal antibodies that can specifically bind PRO polypeptides.

Techniques for producing the monoclonal antibodies are known in the art and are described, for instance, in Coding, *supra.* Immunogens that may be employed include purified PRO fusion proteins containing PRO polypeptides. and cells expressing the gene encoding the PRO polypeptide on the cell surface. Selection of the immunogen can be made by the skilled artisan without undue experimentation.

Mice, such as Balb/c, are immunized with the PRO polypeptide immunogen emulsified in complete Freund's adjuvant and injected subcutaneously or intraperitoneally in an amount from I to 100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, fur several weeks, the mice may also be boosted with additional immunization injections. Serum samples may be periodically obtained from the mice by retro-orbital bleecling for testing in ELISA assays to detect anti-PRO antibodies.

After a suitable antibody titer has been detected, the animals "positive" for antibodies can be injected with a final intravenous injection of the PRO polypeptide. Three to four days later, the mice are sacrificed and the spleen cells are harvested. The spleen cells are then fused (using 35% polyethylene glycol) to a selected murine myeloma cell line such as P3X63AgU,I, available from ATCC, No. CRL 1597. The fusions generate hybridoma cells that can then be plated in 96-well tissue culture plates containing HAT medium to inhibit proliferation of non-fused cells, myeloma hybrids, and spleen cell hybrids.

The hybridoma cells will be screened in an ELISA for reactivity against the PRO polypeptide. Determination of "positive" hybridoma cells secreting the desired monoclonal antibodies against the PRO polypeptide is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue-culture flasks or roller bottles. Purification of the monoclonal antibodies produced in the ascites can be accomplished using ammonium-sulfate precipitation, followed by gel-exclusion chromatography. Alternatively, affinity chromatography based upon binding of antibody to protein A or protein G can be employed.

Deposit of Material

The following material(s) has have been deposited with the American Type Culture Collection, 10801 University Blvd., Manassas, VA 20110-2209, USA (ATCC):

| Material | ATCC Dep. No. | Deposit Date |
|---|---|---|
| DNA35916-1161 | 209419 | October 28, 1997 |
| DNA23339-1130 | 209282 | September 18, 1997 |
| DNA16451-1388 | 209776 | April 14, 1998 |
| DNA27865-1091 | 209296 | September 23, 1997 |
| DNA27864-1155 | 209375 | October 16, 1997 |
| DNA28497-1130 | 209279 | September 18, 1997 |
| DNA26847-1395 | 209772 | April 14, 1998 |
| DNA30942-1134 | 209254 | September 16, 1997 |
| DNA32286-1191 | 209385 | October 16, 1997 |
| DNA33094-1131 | 209256 | September 16, 1997 |
| DNA33221-1133 | 209263 | September 16, 1997 |
| DNA34434-1139 | 209252 | September 16, 1998 |
| DNA35558-1167 | 209374 | October 16, 1997 |
| DNA35638-1141 | 209265 | September 16, 1997 |
| DNA33473-1176 | 209391 | October 17. 1997 |
| DNA38260-1180 | 209397 | October 17, 1997 |
| DNA39969-1185 | 209400 | October 17, 1997 |
| DNA40628-1216 | 209432 | November 7, 1997 |
| DNA35595-1228 | 209528 | December 10, 1997 |
| DNA40981-1234 | 209439 | November 7, 1997 |
| DNA47470-1130-P1 | 209422 | October 28, 1997 |
| DNA47365-1206 | 209436 | November 7, 1997 |
| DNA44184-1319 | 209704 | March 26, 1998 |
| DNA48613-1268 | 209752 | April 7, 1998 |
| DNA29101-1122 | 209653 | March 5, 1998 |
| DNA49646-1327 | 209705 | March 26, 1998 |
| DNA49829-1346 | 209749 | April 7, 1998 |
| DNA56405-1357 | 209849 | May 6, 1998 |

(continued)

| Material | ATCC Dep. No. | Deposit Date |
|---|---|---|
| DNA56352-1358 | 209846 | May 6, 1998 |
| DNA59205-1421 | 203009 | June 23, 1998 |
| DNA53974-1401 | 209774 | April 14, 1998 |
| DNA57689-1385 | 209869 | May 14, 1998 |
| DNA60615-1483 | 209980 | June 16, 1998 |
| DNA59814-1486 | 203359 | October 28, 1998 |
| DNA59846-1503 | 209978 | June 16, 1998 |
| DNA64883-1526 | 203253 | September 9, 1998 |
| DNA64885-1529 | 203457 | November 3, 1998 |
| DNA64889-1541 | 203250 | September9, 1998 |
| DNA64903-1553 | 203223 | September 15, 1998 |
| DNA64905-1558 | 203233 | September 15, 1998 |
| DNA65409-1566 | 203232 | September 15, 1998 |
| DNA65406-1567 | 203219 | September 15, 1998 |
| DNA61873-1574 | 203132 | August 18, 1998 |
| DNA64966-1575 | 203575 | January 12, 1999 |
| DNA67300-1605 | 203163 | August 25, 1998 |
| DNA68872-1620 | 203160 | August 25, 1998 |
| DNA76538-1670 | 203313 | October 6, 1998 |
| DNA33087 | 203381 | October 16, 1997 |

This deposit was made under the provisions of the Budapest Treaty on the International Recognition of the Deposit of Microorganisms for the Purpose of Patent Procedure and the Regulations thereunder (Budapest Treaty) This assures maintenance of a viable culture of the deposit fur 30 years from the date of deposit. The deposit will be made available by ATCC under die terms of the Budapest Treaty, and subject to an agreement between Genentech, Inc., and ATCC. which assures permanent and unrestricted availability of the progeny of the culture of the deposit to the public upon issuance of the pertinent U.S. patent or upon laying open to the public of any U.S or foreign patent application, whichever comes first, and assures availability of the progeny to one determined by the U.S. Commissioner of Patents and Trademarks to be entitled thereto according to 35 USC §122 and the Commissioner's rules pursuant thereto (including 37 CFR §1.14 with particular reference to 886 OG 638).

The assignee of the present application has agreed that if a culture of the material(s) on deposit should die or be lost or destroyed when cultivated under suitable conditions, the material(s) will be promptly replaced on notification with another of the same. Availability of the deposited material(s) is not to be construed as a license to practice the invention in contravention of the rights granted under the authority of any govemment in accordance with its patent laws.

The foregoing written specification is considered to be sufficient to enable one skilled in the art to practice the invention. The present invention is not to be limited in scope by the construct(s) deposited, since the deposited embodiment(s) is/are intended as single illustration(s) of certain aspects of the invention and any constructs that are functionally equivalent are within the scope of this invention. The deposit of material(s) herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

```
Fri Dec  7 11:17:45 2001 [BLASTN 2.2.1 [Jul-12-2001], NCBI]
Repeats masked (summary below)
/home/ruby/va/Molbio/carpenda/tempids/ss.DNA65409 (1091 bp)

Sequences producing High-scoring Segment Pairs:          Frame Score Match Pct E-val
  1 P_AAC58114 Human PRO1303 nucleotide sequence SEQ ID   +   1091  1091 100   0.0
  2 P_AAA77671 Human PRO1303 cDNA sequence SEQ ID NO:20   +   1091  1091 100   0.0
  3 P_AAA37075 Human PRO1303 (UNQ669) cDNA sequence SEQ   +   1091  1091 100   0.0

>1 P_AAC58114 Human PRO1303 nucleotide sequence SEQ ID NO:32. (1091 bp) [2 segs]
 Score = 1091 (2163 bits), Expect = 0.0 [P_AAC58114, seg 1/2]
 Identities = 1091/1091 (100%), at 1,1-1091,1091, Strand +/+

ss.DNA65409     1 CAAGCAGGTCATCCCCTTGGTGACCTTCAAAGAGAAGCAGAGAGGGCAGAGGTGGGGGC
                  ************************************************************
P_AAC58114      1 CAAGCAGGTCATCCCCTTGGTGACCTTCAAAGAGAAGCAGAGAGGGCAGAGGTGGGGGC

ss.DNA65409    61 ACAGGGAAAGGGTGACCTCTGAGATTCCCCTTTTCCCCCAGACTTTGGAAGTGACCCACC
                  ************************************************************
P_AAC58114     61 ACAGGGAAAGGGTGACCTCTGAGATTCCCCTTTTCCCCCAGACTTTGGAAGTGACCCACC

ss.DNA65409   121 ATGGGGCTCAGCATCTTTTTGCTCCTGTGTGTTCTTGGGCTCAGCCAGGCAGCCACACCG
                  ************************************************************
P_AAC58114    121 ATGGGGCTCAGCATCTTTTTGCTCCTGTGTGTTCTTGGGCTCAGCCAGGCAGCCACACCG

ss.DNA65409   181 AAGATTTTCAATGGCACTGAGTGTGGGCGTAACTCACAGCCGTGGCAGGTGGGGCTGTTT
                  ************************************************************
P_AAC58114    181 AAGATTTTCAATGGCACTGAGTGTGGGCGTAACTCACAGCCGTGGCAGGTGGGGCTGTTT

ss.DNA65409   241 GAGGGCACCAGCCTGCGCTGCGGGGGTGTCCTTATTGACCACAGGTGGGTCCTCACAGCG
                  ************************************************************
P_AAC58114    241 GAGGGCACCAGCCTGCGCTGCGGGGGTGTCCTTATTGACCACAGGTGGGTCCTCACAGCG

ss.DNA65409   301 GCTCACTGCAGCGGCAGCAGGTACTGGGTGCGCCTGGGGGAACACAGCCTCAGCCAGCTC
                  ************************************************************
P_AAC58114    301 GCTCACTGCAGCGGCAGCAGGTACTGGGTGCGCCTGGGGGAACACAGCCTCAGCCAGCTC

ss.DNA65409   361 GACTGGACCGAGCAGATCCGGCACAGCGGCTTCTCTGTGACCCATCCCGGCTACCTGGGA
                  ************************************************************
P_AAC58114    361 GACTGGACCGAGCAGATCCGGCACAGCGGCTTCTCTGTGACCCATCCCGGCTACCTGGGA

ss.DNA65409   421 GCCTCGACGAGCCACGAGCACGACCTCCGGCTGCTGCGGCTGCGCCTGCCCGTCCGCGTA
                  ************************************************************
P_AAC58114    421 GCCTCGACGAGCCACGAGCACGACCTCCGGCTGCTGCGGCTGCGCCTGCCCGTCCGCGTA

ss.DNA65409   481 ACCAGCAGCGTTCAACCCCTGCCCCTGCCCAATGACTGTGCAACCGCTGGCACCGAGTGC
                  ************************************************************
P_AAC58114    481 ACCAGCAGCGTTCAACCCCTGCCCCTGCCCAATGACTGTGCAACCGCTGGCACCGAGTGC

ss.DNA65409   541 CACGTCTCAGGCTGGGGCATCACCAACCACCCACGGAACCCATTCCCGGATCTGCTCCAG
                  ************************************************************
P_AAC58114    541 CACGTCTCAGGCTGGGGCATCACCAACCACCCACGGAACCCATTCCCGGATCTGCTCCAG

ss.DNA65409   601 TGCCTCAACCTCTCCATCGTCTCCCATGCCACCTGCCATGGTGTGTATCCCGGGAGAATC
                  ************************************************************
P_AAC58114    601 TGCCTCAACCTCTCCATCGTCTCCCATGCCACCTGCCATGGTGTGTATCCCGGGAGAATC

ss.DNA65409   661 ACGAGCAACATGGTGTGTGCAGGCGGCGTCCCGGGGCAGGATGCCTGCCAGGGTGATTCT
                  ************************************************************
P_AAC58114    661 ACGAGCAACATGGTGTGTGCAGGCGGCGTCCCGGGGCAGGATGCCTGCCAGGGTGATTCT

ss.DNA65409   721 GGGGGCCCCCTGGTGTGTGGGGGAGTCCTTCAAGGTCTGGTGTCCTGGGGGTCTGTGGGG
                  ************************************************************
```

```
P_AAC58114     721 GGGGGCCCCCTGGTGTGTGGGGGAGTCCTTCAAGGTCTGGTGTCCTGGGGGTCTGTGGGG

ss.DNA65409    781 CCCTGTGGACAAGATGGCATCCCTGGAGTCTACACCTATATTTGCAAGTATGTGGACTGG
                   ************************************************************
P_AAC58114     781 CCCTGTGGACAAGATGGCATCCCTGGAGTCTACACCTATATTTGCAAGTATGTGGACTGG

ss.DNA65409    841 ATCCGGATGATCATGAGGAACAACTGACCTGTTTCCTCCACCTCCACCCCCACCCCTTAA
                   ************************************************************
P_AAC58114     841 ATCCGGATGATCATGAGGAACAACTGACCTGTTTCCTCCACCTCCACCCCCACCCCTTAA

ss.DNA65409    901 CTTGGGTACCCCTCTGGCCCTCAGAGCACCAATATCTCCTCCATCACTTCCCCTAGCTCC
                   ************************************************************
P_AAC58114     901 CTTGGGTACCCCTCTGGCCCTCAGAGCACCAATATCTCCTCCATCACTTCCCCTAGCTCC

ss.DNA65409    961 ACTCTTGTTGGCCTGGGAACTTCTTGGAACTTTAACTCCTGCCAGCCCTTCTAAGACCCA
                   ************************************************************
P_AAC58114     961 ACTCTTGTTGGCCTGGGAACTTCTTGGAACTTTAACTCCTGCCAGCCCTTCTAAGACCCA

ss.DNA65409   1021 CGAGCGGGGTGAGAGAAGTGTGCAATAGTCTGGAATAAATATAAATGAAGGAGGGGCAAA
                   ************************************************************
P_AAC58114    1021 CGAGCGGGGTGAGAGAAGTGTGCAATAGTCTGGAATAAATATAAATGAAGGAGGGGCAAA

ss.DNA65409   1081 AAAAAAAAAAA
                   ***********
P_AAC58114    1081 AAAAAAAAAAA


>2 P_AAA77671 Human PRO1303 cDNA sequence SEQ ID NO:202.  cDNA, PAT 07-NOV-2000
(1091 bp) [1 seg]
 Score = 1091 (2163 bits), Expect = 0.0
 Identities = 1091/1091 (100%), at 1,1-1091,1091, Strand +/+

ss.DNA65409      1 CAAGCAGGTCATCCCCTTGGTGACCTTCAAAGAGAAGCAGAGAGGGCAGAGGTGGGGGGC
                   ************************************************************
P_AAA77671       1 CAAGCAGGTCATCCCCTTGGTGACCTTCAAAGAGAAGCAGAGAGGGCAGAGGTGGGGGGC

ss.DNA65409     61 ACAGGGAAAGGGTGACCTCTGAGATTCCCCTTTTCCCCCAGACTTTGGAAGTGACCCACC
                   ************************************************************
P_AAA77671      61 ACAGGGAAAGGGTGACCTCTGAGATTCCCCTTTTCCCCCAGACTTTGGAAGTGACCCACC

ss.DNA65409    121 ATGGGGCTCAGCATCTTTTTGCTCCTGTGTGTTCTTGGGCTCAGCCAGGCAGCCACACCG
                   ************************************************************
P_AAA77671     121 ATGGGGCTCAGCATCTTTTTGCTCCTGTGTGTTCTTGGGCTCAGCCAGGCAGCCACACCG

ss.DNA65409    181 AAGATTTTCAATGGCACTGAGTGTGGGCGTAACTCACAGCCGTGGCAGGTGGGGCTGTTT
                   ************************************************************
P_AAA77671     181 AAGATTTTCAATGGCACTGAGTGTGGGCGTAACTCACAGCCGTGGCAGGTGGGGCTGTTT

ss.DNA65409    241 GAGGGCACCAGCCTGCGCTGCGGGGGTGTCCTTATTGACCACAGGTGGGTCCTCACAGCG
                   ************************************************************
P_AAA77671     241 GAGGGCACCAGCCTGCGCTGCGGGGGTGTCCTTATTGACCACAGGTGGGTCCTCACAGCG

ss.DNA65409    301 GCTCACTGCAGCGGCAGCAGGTACTGGGTGCGCCTGGGGGAACACAGCCTCAGCCAGCTC
                   ************************************************************
P_AAA77671     301 GCTCACTGCAGCGGCAGCAGGTACTGGGTGCGCCTGGGGGAACACAGCCTCAGCCAGCTC

ss.DNA65409    361 GACTGGACCGAGCAGATCCGGCACAGCGGCTTCTCTGTGACCCATCCCGGCTACCTGGGA
                   ************************************************************
P_AAA77671     361 GACTGGACCGAGCAGATCCGGCACAGCGGCTTCTCTGTGACCCATCCCGGCTACCTGGGA

ss.DNA65409    421 GCCTCGACGAGCCACGAGCACGACCTCCGGCTGCTGCGGCTGCGCCTGCCCGTCCGCGTA
                   ************************************************************
P_AAA77671     421 GCCTCGACGAGCCACGAGCACGACCTCCGGCTGCTGCGGCTGCGCCTGCCCGTCCGCGTA
```

```
ss.DNA65409   481 ACCAGCAGCGTTCAACCCCTGCCCCTGCCCAATGACTGTGCAACCGCTGGCACCGAGTGC
                  ************************************************************
P_AAA77671    481 ACCAGCAGCGTTCAACCCCTGCCCCTGCCCAATGACTGTGCAACCGCTGGCACCGAGTGC

ss.DNA65409   541 CACGTCTCAGGCTGGGGCATCACCAACCACCCACGGAACCCATTCCCGGATCTGCTCCAG
                  ************************************************************
P_AAA77671    541 CACGTCTCAGGCTGGGGCATCACCAACCACCCACGGAACCCATTCCCGGATCTGCTCCAG

ss.DNA65409   601 TGCCTCAACCTCTCCATCGTCTCCCATGCCACCTGCCATGGTGTGTATCCCGGGAGAATC
                  ************************************************************
P_AAA77671    601 TGCCTCAACCTCTCCATCGTCTCCCATGCCACCTGCCATGGTGTGTATCCCGGGAGAATC

ss.DNA65409   661 ACGAGCAACATGGTGTGTGCAGGCGGCGTCCCGGGGCAGGATGCCTGCCAGGGTGATTCT
                  ************************************************************
P_AAA77671    661 ACGAGCAACATGGTGTGTGCAGGCGGCGTCCCGGGGCAGGATGCCTGCCAGGGTGATTCT

ss.DNA65409   721 GGGGGCCCCCTGGTGTGTGGGGGAGTCCTTCAAGGTCTGGTGTCCTGGGGGTCTGTGGGG
                  ************************************************************
P_AAA77671    721 GGGGGCCCCCTGGTGTGTGGGGGAGTCCTTCAAGGTCTGGTGTCCTGGGGGTCTGTGGGG

ss.DNA65409   781 CCCTGTGGACAAGATGGCATCCCTGGAGTCTACACCTATATTTGCAAGTATGTGGACTGG
                  ************************************************************
P_AAA77671    781 CCCTGTGGACAAGATGGCATCCCTGGAGTCTACACCTATATTTGCAAGTATGTGGACTGG

ss.DNA65409   841 ATCCGGATGATCATGAGGAACAACTGACCTGTTTCCTCCACCTCCACCCCCACCCCTTAA
                  ************************************************************
P_AAA77671    841 ATCCGGATGATCATGAGGAACAACTGACCTGTTTCCTCCACCTCCACCCCCACCCCTTAA

ss.DNA65409   901 CTTGGGTACCCCTCTGGCCCTCAGAGCACCAATATCTCCTCCATCACTTCCCCTAGCTCC
                  ************************************************************
P_AAA77671    901 CTTGGGTACCCCTCTGGCCCTCAGAGCACCAATATCTCCTCCATCACTTCCCCTAGCTCC

ss.DNA65409   961 ACTCTTGTTGGCCTGGGAACTTCTTGGAACTTTAACTCCTGCCAGCCCTTCTAAGACCCA
                  ************************************************************
P_AAA77671    961 ACTCTTGTTGGCCTGGGAACTTCTTGGAACTTTAACTCCTGCCAGCCCTTCTAAGACCCA

ss.DNA65409  1021 CGAGCGGGGTGAGAGAAGTGTGCAATAGTCTGGAATAAATATAAATGAAGGAGGGGCAAA
                  ************************************************************
P_AAA77671   1021 CGAGCGGGGTGAGAGAAGTGTGCAATAGTCTGGAATAAATATAAATGAAGGAGGGGCAAA

ss.DNA65409  1081 AAAAAAAAAAA
                  ***********
P_AAA77671   1081 AAAAAAAAAAA

>3 P_AAA37075 Human PRO1303 (UNQ669) cDNA sequence SEQ ID NO:193. (1091 bp) [1
seg]
 Score = 1091 (2163 bits), Expect = 0.0
 Identities = 1091/1091 (100%), at 1,1-1091,1091, Strand +/+

ss.DNA65409     1 CAAGCAGGTCATCCCCTTGGTGACCTTCAAAGAGAAGCAGAGAGGGCAGAGGTGGGGGGC
                  ************************************************************
P_AAA37075      1 CAAGCAGGTCATCCCCTTGGTGACCTTCAAAGAGAAGCAGAGAGGGCAGAGGTGGGGGGC

ss.DNA65409    61 ACAGGGAAAGGGTGACCTCTGAGATTCCCCTTTTCCCCCAGACTTTGGAAGTGACCCACC
                  ************************************************************
P_AAA37075     61 ACAGGGAAAGGGTGACCTCTGAGATTCCCCTTTTCCCCCAGACTTTGGAAGTGACCCACC

ss.DNA65409   121 ATGGGGCTCAGCATCTTTTTGCTCCTGTGTGTTCTTGGGCTCAGCCAGGCAGCCACACCG
                  ************************************************************
P_AAA37075    121 ATGGGGCTCAGCATCTTTTTGCTCCTGTGTGTTCTTGGGCTCAGCCAGGCAGCCACACCG

ss.DNA65409   181 AAGATTTTCAATGGCACTGAGTGTGGGCGTAACTCACAGCCGTGGCAGGTGGGGCTGTTT
                  ************************************************************
P_AAA37075    181 AAGATTTTCAATGGCACTGAGTGTGGGCGTAACTCACAGCCGTGGCAGGTGGGGCTGTTT
```

```
ss.DNA65409  241 GAGGGCACCAGCCTGCGCTGCGGGGGTGTCCTTATTGACCACAGGTGGGTCCTCACAGCG
                 ************************************************************
P_AAA37075   241 GAGGGCACCAGCCTGCGCTGCGGGGGTGTCCTTATTGACCACAGGTGGGTCCTCACAGCG

ss.DNA65409  301 GCTCACTGCAGCGGCAGCAGGTACTGGGTGCGCCTGGGGGAACACAGCCTCAGCCAGCTC
                 ************************************************************
P_AAA37075   301 GCTCACTGCAGCGGCAGCAGGTACTGGGTGCGCCTGGGGGAACACAGCCTCAGCCAGCTC

ss.DNA65409  361 GACTGGACCGAGCAGATCCGGCACAGCGGCTTCTCTGTGACCCATCCCGGCTACCTGGGA
                 ************************************************************
P_AAA37075   361 GACTGGACCGAGCAGATCCGGCACAGCGGCTTCTCTGTGACCCATCCCGGCTACCTGGGA

ss.DNA65409  421 GCCTCGACGAGCCACGAGCACGACCTCCGGCTGCTGCGGCTGCGCCTGCCCGTCCGCGTA
                 ************************************************************
P_AAA37075   421 GCCTCGACGAGCCACGAGCACGACCTCCGGCTGCTGCGGCTGCGCCTGCCCGTCCGCGTA

ss.DNA65409  481 ACCAGCAGCGTTCAACCCCTGCCCCTGCCCAATGACTGTGCAACCGCTGGCACCGAGTGC
                 ************************************************************
P_AAA37075   481 ACCAGCAGCGTTCAACCCCTGCCCCTGCCCAATGACTGTGCAACCGCTGGCACCGAGTGC

ss.DNA65409  541 CACGTCTCAGGCTGGGGCATCACCAACCACCCACGGAACCCATTCCCGGATCTGCTCCAG
                 ************************************************************
P_AAA37075   541 CACGTCTCAGGCTGGGGCATCACCAACCACCCACGGAACCCATTCCCGGATCTGCTCCAG

ss.DNA65409  601 TGCCTCAACCTCTCCATCGTCTCCCATGCCACCTGCCATGGTGTGTATCCCGGGAGAATC
                 ************************************************************
P_AAA37075   601 TGCCTCAACCTCTCCATCGTCTCCCATGCCACCTGCCATGGTGTGTATCCCGGGAGAATC

ss.DNA65409  661 ACGAGCAACATGGTGTGTGCAGGCGGCGTCCCGGGGCAGGATGCCTGCCAGGGTGATTCT
                 ************************************************************
P_AAA37075   661 ACGAGCAACATGGTGTGTGCAGGCGGCGTCCCGGGGCAGGATGCCTGCCAGGGTGATTCT

ss.DNA65409  721 GGGGGCCCCCTGGTGTGTGGGGGAGTCCTTCAAGGTCTGGTGTCCTGGGGGTCTGTGGGG
                 ************************************************************
P_AAA37075   721 GGGGGCCCCCTGGTGTGTGGGGGAGTCCTTCAAGGTCTGGTGTCCTGGGGGTCTGTGGGG

ss.DNA65409  781 CCCTGTGGACAAGATGGCATCCCTGGAGTCTACACCTATATTTGCAAGTATGTGGACTGG
                 ************************************************************
P_AAA37075   781 CCCTGTGGACAAGATGGCATCCCTGGAGTCTACACCTATATTTGCAAGTATGTGGACTGG

ss.DNA65409  841 ATCCGGATGATCATGAGGAACAACTGACCTGTTTCCTCCACCTCCACCCCCACCCCTTAA
                 ************************************************************
P_AAA37075   841 ATCCGGATGATCATGAGGAACAACTGACCTGTTTCCTCCACCTCCACCCCCACCCCTTAA

ss.DNA65409  901 CTTGGGTACCCCTCTGGCCCTCAGAGCACCAATATCTCCTCCATCACTTCCCCTAGCTCC
                 ************************************************************
P_AAA37075   901 CTTGGGTACCCCTCTGGCCCTCAGAGCACCAATATCTCCTCCATCACTTCCCCTAGCTCC

ss.DNA65409  961 ACTCTTGTTGGCCTGGGAACTTCTTGGAACTTTAACTCCTGCCAGCCCTTCTAAGACCCA
                 ************************************************************
P_AAA37075   961 ACTCTTGTTGGCCTGGGAACTTCTTGGAACTTTAACTCCTGCCAGCCCTTCTAAGACCCA

ss.DNA65409 1021 CGAGCGGGGTGAGAGAAGTGTGCAATAGTCTGGAATAAATATAAATGAAGGAGGGGCAAA
                 ************************************************************
P_AAA37075  1021 CGAGCGGGGTGAGAGAAGTGTGCAATAGTCTGGAATAAATATAAATGAAGGAGGGGCAAA

ss.DNA65409 1081 AAAAAAAAAAA
                 ***********
P_AAA37075  1081 AAAAAAAAAAA
```

###########################################

```
Fri Dec  7 10:35:14 2001 [BLASTP 2.2.1 [Jul-12-2001], NCBI]
/home/ruby/va/Molbio/carpenda/tempids/p1.DNA65409 (248 aa)

Sequences producing High-scoring Segment Pairs:                 Score Match Pct E-val
  1 P_AAM23994  Human EST encoded protein SEQ ID NO: 1519        1374   248 100 e-151
  2 P_AAY99393  Human PRO1303 (UNQ669) amino acid sequence       1374   248 100 e-151
  3 P_AAB24428  Human PRO1303 protein sequence SEQ ID NO:2       1374   248 100 e-151
  4 P_AAB24032  Human PRO1303 protein sequence SEQ ID NO:3       1374   248 100 e-151
  5 P_AAB21304  Human KLK-L5 protein #4 - Homo sapiens.          1374   248 100 e-151
  6 KLKC_HUMAN  Kallikrein 12 precursor /pid=AAD26426.2 -        1374   248 100 e-151
  7 P_AAB21303  Human KLK-L5 protein #3 - Homo sapiens.          1301   235 100 e-142
  8 NP_062544.1 kallikrein 12 - Homo sapiens                     1301   235 100 e-142

>1 P_AAM23994 Human EST encoded protein SEQ ID NO: 1519 - Homo sapiens. (248 aa)
[1 seg]
 Score =  1374 (533 bits), Expect = e-151
 Identities = 248/248 (100%), Positives = 248/248 (100%), at 1,1-248,248

  DNA65409     1 MGLSIFLLLCVLGLSQAATPKIFNGTECGRNSQPWQVGLFEGTSLRCGGVLIDHRWVLTA
                 ************************************************************
P_AAM23994     1 MGLSIFLLLCVLGLSQAATPKIFNGTECGRNSQPWQVGLFEGTSLRCGGVLIDHRWVLTA

  DNA65409    61 AHCSGSRYWVRLGEHSLSQLDWTEQIRHSGFSVTHPGYLGASTSHEHDLRLLRLRLPVRV
                 ************************************************************
P_AAM23994    61 AHCSGSRYWVRLGEHSLSQLDWTEQIRHSGFSVTHPGYLGASTSHEHDLRLLRLRLPVRV

  DNA65409   121 TSSVQPLPLPNDCATAGTECHVSGWGITNHPRNPFPDLLQCLNLSIVSHATCHGVYPGRI
                 ************************************************************
P_AAM23994   121 TSSVQPLPLPNDCATAGTECHVSGWGITNHPRNPFPDLLQCLNLSIVSHATCHGVYPGRI

  DNA65409   181 TSNMVCAGGVPGQDACQGDSGGPLVCGGVLQGLVSWGSVGPCGQDGIPGVYTYICKYVDW
                 ************************************************************
P_AAM23994   181 TSNMVCAGGVPGQDACQGDSGGPLVCGGVLQGLVSWGSVGPCGQDGIPGVYTYICKYVDW

  DNA65409   241 IRMIMRNN
                 ********
P_AAM23994   241 IRMIMRNN

>2 P_AAY99393 Human PRO1303 (UNQ669) amino acid sequence SEQ ID NO:194 - Homo
(248 aa) [1 seg]
 Score =  1374 (533 bits), Expect = e-151
 Identities = 248/248 (100%), Positives = 248/248 (100%), at 1,1-248,248

  DNA65409     1 MGLSIFLLLCVLGLSQAATPKIFNGTECGRNSQPWQVGLFEGTSLRCGGVLIDHRWVLTA
                 ************************************************************
P_AAY99393     1 MGLSIFLLLCVLGLSQAATPKIFNGTECGRNSQPWQVGLFEGTSLRCGGVLIDHRWVLTA

  DNA65409    61 AHCSGSRYWVRLGEHSLSQLDWTEQIRHSGFSVTHPGYLGASTSHEHDLRLLRLRLPVRV
                 ************************************************************
P_AAY99393    61 AHCSGSRYWVRLGEHSLSQLDWTEQIRHSGFSVTHPGYLGASTSHEHDLRLLRLRLPVRV

  DNA65409   121 TSSVQPLPLPNDCATAGTECHVSGWGITNHPRNPFPDLLQCLNLSIVSHATCHGVYPGRI
                 ************************************************************
P_AAY99393   121 TSSVQPLPLPNDCATAGTECHVSGWGITNHPRNPFPDLLQCLNLSIVSHATCHGVYPGRI
```

```
  DNA65409   181 TSNMVCAGGVPGQDACQGDSGGPLVCGGVLQGLVSWGSVGPCGQDGIPGVYTYICKYVDW
                 ************************************************************
P_AAY99393   181 TSNMVCAGGVPGQDACQGDSGGPLVCGGVLQGLVSWGSVGPCGQDGIPGVYTYICKYVDW

  DNA65409   241 IRMIMRNN
                 ********
P_AAY99393   241 IRMIMRNN
```

```
>3 P_AAB24428 Human PRO1303 protein sequence SEQ ID NO:203 - Homo sapiens. (248
aa) [1 seg]
 Score =  1374 (533 bits), Expect = e-151
 Identities = 248/248 (100%), Positives = 248/248 (100%), at 1,1-248,248

  DNA65409     1 MGLSIFLLLCVLGLSQAATPKIFNGTECGRNSQPWQVGLFEGTSLRCGGVLIDHRWVLTA
                 ************************************************************
P_AAB24428     1 MGLSIFLLLCVLGLSQAATPKIFNGTECGRNSQPWQVGLFEGTSLRCGGVLIDHRWVLTA

  DNA65409    61 AHCSGSRYWVRLGEHSLSQLDWTEQIRHSGFSVTHPGYLGASTSHEHDLRLLRLRLPVRV
                 ************************************************************
P_AAB24428    61 AHCSGSRYWVRLGEHSLSQLDWTEQIRHSGFSVTHPGYLGASTSHEHDLRLLRLRLPVRV

  DNA65409   121 TSSVQPLPLPNDCATAGTECHVSGWGITNHPRNPFPDLLQCLNLSIVSHATCHGVYPGRI
                 ************************************************************
P_AAB24428   121 TSSVQPLPLPNDCATAGTECHVSGWGITNHPRNPFPDLLQCLNLSIVSHATCHGVYPGRI

  DNA65409   181 TSNMVCAGGVPGQDACQGDSGGPLVCGGVLQGLVSWGSVGPCGQDGIPGVYTYICKYVDW
                 ************************************************************
P_AAB24428   181 TSNMVCAGGVPGQDACQGDSGGPLVCGGVLQGLVSWGSVGPCGQDGIPGVYTYICKYVDW

  DNA65409   241 IRMIMRNN
                 ********
P_AAB24428   241 IRMIMRNN
```

```
>4 P_AAB24032 Human PRO1303 protein sequence SEQ ID NO:33 - Homo sapiens. (248
aa) [1 seg]
 Score =  1374 (533 bits), Expect = e-151
 Identities = 248/248 (100%), Positives = 248/248 (100%), at 1,1-248,248

  DNA65409     1 MGLSIFLLLCVLGLSQAATPKIFNGTECGRNSQPWQVGLFEGTSLRCGGVLIDHRWVLTA
                 ************************************************************
P_AAB24032     1 MGLSIFLLLCVLGLSQAATPKIFNGTECGRNSQPWQVGLFEGTSLRCGGVLIDHRWVLTA

  DNA65409    61 AHCSGSRYWVRLGEHSLSQLDWTEQIRHSGFSVTHPGYLGASTSHEHDLRLLRLRLPVRV
                 ************************************************************
P_AAB24032    61 AHCSGSRYWVRLGEHSLSQLDWTEQIRHSGFSVTHPGYLGASTSHEHDLRLLRLRLPVRV

  DNA65409   121 TSSVQPLPLPNDCATAGTECHVSGWGITNHPRNPFPDLLQCLNLSIVSHATCHGVYPGRI
                 ************************************************************
P_AAB24032   121 TSSVQPLPLPNDCATAGTECHVSGWGITNHPRNPFPDLLQCLNLSIVSHATCHGVYPGRI

  DNA65409   181 TSNMVCAGGVPGQDACQGDSGGPLVCGGVLQGLVSWGSVGPCGQDGIPGVYTYICKYVDW
                 ************************************************************
P_AAB24032   181 TSNMVCAGGVPGQDACQGDSGGPLVCGGVLQGLVSWGSVGPCGQDGIPGVYTYICKYVDW

  DNA65409   241 IRMIMRNN
                 ********
```

```
P_AAB24032   241 IRMIMRNN

>5 P_AAB21304 Human KLK-L5 protein #4 - Homo sapiens. (248 aa) [1 seg]
 Score =  1374 (533 bits), Expect = e-151
 Identities = 248/248 (100%), Positives = 248/248 (100%), at 1,1-248,248

   DNA65409    1 MGLSIFLLLCVLGLSQAATPKIFNGTECGRNSQPWQVGLFEGTSLRCGGVLIDHRWVLTA
                 ************************************************************
 P_AAB21304    1 MGLSIFLLLCVLGLSQAATPKIFNGTECGRNSQPWQVGLFEGTSLRCGGVLIDHRWVLTA

   DNA65409   61 AHCSGSRYWVRLGEHSLSQLDWTEQIRHSGFSVTHPGYLGASTSHEHDLRLLRLRLPVRV
                 ************************************************************
 P_AAB21304   61 AHCSGSRYWVRLGEHSLSQLDWTEQIRHSGFSVTHPGYLGASTSHEHDLRLLRLRLPVRV

   DNA65409  121 TSSVQPLPLPNDCATAGTECHVSGWGITNHPRNPFPDLLQCLNLSIVSHATCHGVYPGRI
                 ************************************************************
 P_AAB21304  121 TSSVQPLPLPNDCATAGTECHVSGWGITNHPRNPFPDLLQCLNLSIVSHATCHGVYPGRI

   DNA65409  181 TSNMVCAGGVPGQDACQGDSGGPLVCGGVLQGLVSWGSVGPCGQDGIPGVYTYICKYVDW
                 ************************************************************
 P_AAB21304  181 TSNMVCAGGVPGQDACQGDSGGPLVCGGVLQGLVSWGSVGPCGQDGIPGVYTYICKYVDW

   DNA65409  241 IRMIMRNN
                 ********
 P_AAB21304  241 IRMIMRNN

>6 KLKC_HUMAN Kallikrein 12 precursor /pid=AAD26426.2 - homo sapiens (248 aa) [1
seg]
 Score =  1374 (533 bits), Expect = e-151
 Identities = 248/248 (100%), Positives = 248/248 (100%), at 1,1-248,248

   DNA65409    1 MGLSIFLLLCVLGLSQAATPKIFNGTECGRNSQPWQVGLFEGTSLRCGGVLIDHRWVLTA
                 ************************************************************
 KLKC_HUMAN    1 MGLSIFLLLCVLGLSQAATPKIFNGTECGRNSQPWQVGLFEGTSLRCGGVLIDHRWVLTA

   DNA65409   61 AHCSGSRYWVRLGEHSLSQLDWTEQIRHSGFSVTHPGYLGASTSHEHDLRLLRLRLPVRV
                 ************************************************************
 KLKC_HUMAN   61 AHCSGSRYWVRLGEHSLSQLDWTEQIRHSGFSVTHPGYLGASTSHEHDLRLLRLRLPVRV

   DNA65409  121 TSSVQPLPLPNDCATAGTECHVSGWGITNHPRNPFPDLLQCLNLSIVSHATCHGVYPGRI
                 ************************************************************
 KLKC_HUMAN  121 TSSVQPLPLPNDCATAGTECHVSGWGITNHPRNPFPDLLQCLNLSIVSHATCHGVYPGRI

   DNA65409  181 TSNMVCAGGVPGQDACQGDSGGPLVCGGVLQGLVSWGSVGPCGQDGIPGVYTYICKYVDW
                 ************************************************************
 KLKC_HUMAN  181 TSNMVCAGGVPGQDACQGDSGGPLVCGGVLQGLVSWGSVGPCGQDGIPGVYTYICKYVDW

   DNA65409  241 IRMIMRNN
                 ********
 KLKC_HUMAN  241 IRMIMRNN

>7 P_AAB21303 Human KLK-L5 protein #3 - Homo sapiens. (254 aa) [1 seg]
 Score =  1301 (505 bits), Expect = e-142
 Identities = 235/235 (100%), Positives = 235/235 (100%), at 1,1-235,235

   DNA65409    1 MGLSIFLLLCVLGLSQAATPKIFNGTECGRNSQPWQVGLFEGTSLRCGGVLIDHRWVLTA
                 ************************************************************
```

```
P_AAB21303     1 MGLSIFLLLCVLGLSQAATPKIFNGTECGRNSQPWQVGLFEGTSLRCGGVLIDHRWVLTA

  DNA65409    61 AHCSGSRYWVRLGEHSLSQLDWTEQIRHSGFSVTHPGYLGASTSHEHDLRLLRLRLPVRV
                 ************************************************************
P_AAB21303    61 AHCSGSRYWVRLGEHSLSQLDWTEQIRHSGFSVTHPGYLGASTSHEHDLRLLRLRLPVRV

  DNA65409   121 TSSVQPLPLPNDCATAGTECHVSGWGITNHPRNPFPDLLQCLNLSIVSHATCHGVYPGRI
                 ************************************************************
P_AAB21303   121 TSSVQPLPLPNDCATAGTECHVSGWGITNHPRNPFPDLLQCLNLSIVSHATCHGVYPGRI

  DNA65409   181 TSNMVCAGGVPGQDACQGDSGGPLVCGGVLQGLVSWGSVGPCGQDGIPGVYTYIC
                 *******************************************************
P_AAB21303   181 TSNMVCAGGVPGQDACQGDSGGPLVCGGVLQGLVSWGSVGPCGQDGIPGVYTYIC

>8 NP_062544.1 kallikrein 12 - Homo sapiens (254 aa) [1 seg]
 Score =  1301 (505 bits), Expect = e-142
 Identities = 235/235 (100%), Positives = 235/235 (100%), at 1,1-235,235

  DNA65409     1 MGLSIFLLLCVLGLSQAATPKIFNGTECGRNSQPWQVGLFEGTSLRCGGVLIDHRWVLTA
                 ************************************************************
NP_062544.1    1 MGLSIFLLLCVLGLSQAATPKIFNGTECGRNSQPWQVGLFEGTSLRCGGVLIDHRWVLTA

  DNA65409    61 AHCSGSRYWVRLGEHSLSQLDWTEQIRHSGFSVTHPGYLGASTSHEHDLRLLRLRLPVRV
                 ************************************************************
NP_062544.1   61 AHCSGSRYWVRLGEHSLSQLDWTEQIRHSGFSVTHPGYLGASTSHEHDLRLLRLRLPVRV

  DNA65409   121 TSSVQPLPLPNDCATAGTECHVSGWGITNHPRNPFPDLLQCLNLSIVSHATCHGVYPGRI
                 ************************************************************
NP_062544.1  121 TSSVQPLPLPNDCATAGTECHVSGWGITNHPRNPFPDLLQCLNLSIVSHATCHGVYPGRI

  DNA65409   181 TSNMVCAGGVPGQDACQGDSGGPLVCGGVLQGLVSWGSVGPCGQDGIPGVYTYIC
                 *******************************************************
NP_062544.1  181 TSNMVCAGGVPGQDACQGDSGGPLVCGGVLQGLVSWGSVGPCGQDGIPGVYTYIC
```

```
Wed Oct  9 09:24:37 2002 [BLASTN 2.2.2 [Jan-08-2002], NCBI]
Repeats masked (summary below)
/home/ruby/va/Molbio/carpenda/tempids/ss.DNA64966 (1181 bp)

Sequences producing High-scoring Segment Pairs:      Frame Score Match Pct E-val
  1 P_ABK69975 cDNA encoding human Pro peptide #15.      +   1181  1181 100   0.0
  2 P_AAC91566 Human PRO1313 cDNA.                       +   1181  1181 100   0.0
  3 P_AAA77681 Human PRO1313 cDNA sequence SEQ ID NO:21  +   1181  1181 100   0.0
  4 P_AAC85948 Native sequence of PRO1313 cDNA, clone D  +   1181  1181 100   0.0
  5 AX055712   Sequence 27 from Patent WO0073348.  DNA,  +   1181  1181 100   0.0
  6 AX150811   Sequence 7 from Patent WO0140464.  DNA,   +   1181  1181 100   0.0
  7 MGC33214   Homo sapiens hypothetical protein MGC332  +   1018  1018 100   0.0
  8 BC037287   Homo sapiens, Similar to hypothetical pr  +   1018  1018 100   0.0
  9 P_AAS63166 Human purified secretory polynucleotide   +   1014  1014 100   0.0
 10 P_ABN99385 Human secreted protein (SCEP) coding seq  +   1010  1013 100   0.0
 11 XM_087662  Homo sapiens similar to hypothetical pro  +   1006  1009 100   0.0


>1 P_ABK69975 cDNA encoding human Pro peptide #15. (1181 bp) [1 seg]
 Score = 1181 (2341 bits), Expect = 0.0
 Identities = 1181/1181 (100%), at 1,1-1181,1181, Strand +/+

ss.DNA64966      1 AAGACCCTCTCTTTCGCTGTTTGAGAGTCTCTCGGCTCAAGGACCGGGAGGTAAGAGGTT
                   ************************************************************
P_ABK69975       1 AAGACCCTCTCTTTCGCTGTTTGAGAGTCTCTCGGCTCAAGGACCGGGAGGTAAGAGGTT

ss.DNA64966     61 TGGGACTGCCCCGGCAACTCCAGGGTGTCTGGTCCACGACCTATCCTAGGCGCCATGGGT
                   ************************************************************
P_ABK69975      61 TGGGACTGCCCCGGCAACTCCAGGGTGTCTGGTCCACGACCTATCCTAGGCGCCATGGGT

ss.DNA64966    121 GTGATAGGTATACAGCTGGTTGTTACCATGGTGATGGCCAGTGTCATGCAGAAGATTATA
                   ************************************************************
P_ABK69975     121 GTGATAGGTATACAGCTGGTTGTTACCATGGTGATGGCCAGTGTCATGCAGAAGATTATA

ss.DNA64966    181 CCTCACTATTCTCTTGCTCGATGGCTACTCTGTAATGGCAGTTTGAGGTGGTATCAACAT
                   ************************************************************
P_ABK69975     181 CCTCACTATTCTCTTGCTCGATGGCTACTCTGTAATGGCAGTTTGAGGTGGTATCAACAT

ss.DNA64966    241 CCTACAGAAGAAGAATTAAGAATTCTTGCAGGGAAACAACAAAAAGGGAAAACCAAAAAA
                   ************************************************************
P_ABK69975     241 CCTACAGAAGAAGAATTAAGAATTCTTGCAGGGAAACAACAAAAAGGGAAAACCAAAAAA

ss.DNA64966    301 GATAGGAAATATAATGGTCACATTGAAAGTAAGCCATTAACCATTCCAAAGGATATTGAC
                   ************************************************************
P_ABK69975     301 GATAGGAAATATAATGGTCACATTGAAAGTAAGCCATTAACCATTCCAAAGGATATTGAC

ss.DNA64966    361 CTTCATCTAGAAACAAAGTCAGTTACAGAAGTGGATACTTTAGCATTGCATTACTTTCCA
                   ************************************************************
P_ABK69975     361 CTTCATCTAGAAACAAAGTCAGTTACAGAAGTGGATACTTTAGCATTGCATTACTTTCCA

ss.DNA64966    421 GAATACCAGTGGCTGGTGGATTTCACAGTGGCTGCTACAGTTGTGTATCTAGTAACTGAA
                   ************************************************************
P_ABK69975     421 GAATACCAGTGGCTGGTGGATTTCACAGTGGCTGCTACAGTTGTGTATCTAGTAACTGAA

ss.DNA64966    481 GTCTACTACAATTTTATGAAGCCTACACAGGAAATGAATATCAGCTTAGTCTGGTGCCTA
                   ************************************************************
```

```
P_ABK69975      481 GTCTACTACAATTTTATGAAGCCTACACAGGAAATGAATATCAGCTTAGTCTGGTGCCTA

ss.DNA64966     541 CTTGTTTTGTCTTTTGCAATCAAAGTTCTATTTTCATTAACTACACACTATTTTAAAGTA
                    ************************************************************
P_ABK69975      541 CTTGTTTTGTCTTTTGCAATCAAAGTTCTATTTTCATTAACTACACACTATTTTAAAGTA

ss.DNA64966     601 GAAGATGGTGGTGAAAGATCTGTTTGTGTCACCTTTGGATTTTTTTTCTTTGTCAAAGCA
                    ************************************************************
P_ABK69975      601 GAAGATGGTGGTGAAAGATCTGTTTGTGTCACCTTTGGATTTTTTTTCTTTGTCAAAGCA

ss.DNA64966     661 ATGGCAGTGTTGATTGTAACAGAAAATTATCTGGAATTTGGACTTGAAACAGGGTTTACA
                    ************************************************************
P_ABK69975      661 ATGGCAGTGTTGATTGTAACAGAAAATTATCTGGAATTTGGACTTGAAACAGGGTTTACA

ss.DNA64966     721 AATTTTTCAGACAGTGCGATGCAGTTTCTTGAAAAGCAAGGTTTAGAATCTCAGAGTCCT
                    ************************************************************
P_ABK69975      721 AATTTTTCAGACAGTGCGATGCAGTTTCTTGAAAAGCAAGGTTTAGAATCTCAGAGTCCT

ss.DNA64966     781 GTTTCAAAACTTACTTTCAAATTTTTCCTGGCTATTTTCTGTTCATTCATTGGGGCTTTT
                    ************************************************************
P_ABK69975      781 GTTTCAAAACTTACTTTCAAATTTTTCCTGGCTATTTTCTGTTCATTCATTGGGGCTTTT

ss.DNA64966     841 TTGACATTTCCTGGATTACGACTGGCTCAAATGCATCTGGATGCCCTGAATTTGGCAACA
                    ************************************************************
P_ABK69975      841 TTGACATTTCCTGGATTACGACTGGCTCAAATGCATCTGGATGCCCTGAATTTGGCAACA

ss.DNA64966     901 GAAAAAATTACACAAACTTTACTTCATATCAACTTCTTGGCACCTTTATTTATGGTTTTG
                    ************************************************************
P_ABK69975      901 GAAAAAATTACACAAACTTTACTTCATATCAACTTCTTGGCACCTTTATTTATGGTTTTG

ss.DNA64966     961 CTCTGGGTAAAACCAATCACCAAAGACTACATTATGAACCCACCACTGGGCAAAGAAATT
                    ************************************************************
P_ABK69975      961 CTCTGGGTAAAACCAATCACCAAAGACTACATTATGAACCCACCACTGGGCAAAGAAATT

ss.DNA64966    1021 TCCCCATCTGGAAGATGAAGATAATAGTATCTAACTCACAAGGTTATCATTGGAATAAAT
                    ************************************************************
P_ABK69975     1021 TCCCCATCTGGAAGATGAAGATAATAGTATCTAACTCACAAGGTTATCATTGGAATAAAT

ss.DNA64966    1081 GAAAGAACACATGTAATGCAACCAGCTGGAATTAAGTGCTTAATAAATGTTCTTTTCACT
                    ************************************************************
P_ABK69975     1081 GAAAGAACACATGTAATGCAACCAGCTGGAATTAAGTGCTTAATAAATGTTCTTTTCACT

ss.DNA64966    1141 GCTTTGCCTCATCAGAATTAAAATAGAAATACTTGACTAGT
                    *****************************************
P_ABK69975     1141 GCTTTGCCTCATCAGAATTAAAATAGAAATACTTGACTAGT

>2 P_AAC91566 Human PRO1313 cDNA. (1181 bp) [1 seg]
 Score = 1181 (2341 bits), Expect = 0.0
 Identities = 1181/1181 (100%), at 1,1-1181,1181, Strand +/+

ss.DNA64966       1 AAGACCCTCTCTTTCGCTGTTTGAGAGTCTCTCGGCTCAAGGACCGGGAGGTAAGAGGTT
                    ************************************************************
P_AAC91566        1 AAGACCCTCTCTTTCGCTGTTTGAGAGTCTCTCGGCTCAAGGACCGGGAGGTAAGAGGTT

ss.DNA64966      61 TGGGACTGCCCCGGCAACTCCAGGGTGTCTGGTCCACGACCTATCCTAGGCGCCATGGGT
                    ************************************************************
P_AAC91566       61 TGGGACTGCCCCGGCAACTCCAGGGTGTCTGGTCCACGACCTATCCTAGGCGCCATGGGT
```

```
ss.DNA64966   121 GTGATAGGTATACAGCTGGTTGTTACCATGGTGATGGCCAGTGTCATGCAGAAGATTATA
                  ************************************************************
P_AAC91566    121 GTGATAGGTATACAGCTGGTTGTTACCATGGTGATGGCCAGTGTCATGCAGAAGATTATA

ss.DNA64966   181 CCTCACTATTCTCTTGCTCGATGGCTACTCTGTAATGGCAGTTTGAGGTGGTATCAACAT
                  ************************************************************
P_AAC91566    181 CCTCACTATTCTCTTGCTCGATGGCTACTCTGTAATGGCAGTTTGAGGTGGTATCAACAT

ss.DNA64966   241 CCTACAGAAGAAGAATTAAGAATTCTTGCAGGGAAACAACAAAAAGGGAAAACCAAAAAA
                  ************************************************************
P_AAC91566    241 CCTACAGAAGAAGAATTAAGAATTCTTGCAGGGAAACAACAAAAAGGGAAAACCAAAAAA

ss.DNA64966   301 GATAGGAAATATAATGGTCACATTGAAAGTAAGCCATTAACCATTCCAAAGGATATTGAC
                  ************************************************************
P_AAC91566    301 GATAGGAAATATAATGGTCACATTGAAAGTAAGCCATTAACCATTCCAAAGGATATTGAC

ss.DNA64966   361 CTTCATCTAGAAACAAAGTCAGTTACAGAAGTGGATACTTTAGCATTGCATTACTTTCCA
                  ************************************************************
P_AAC91566    361 CTTCATCTAGAAACAAAGTCAGTTACAGAAGTGGATACTTTAGCATTGCATTACTTTCCA

ss.DNA64966   421 GAATACCAGTGGCTGGTGGATTTCACAGTGGCTGCTACAGTTGTGTATCTAGTAACTGAA
                  ************************************************************
P_AAC91566    421 GAATACCAGTGGCTGGTGGATTTCACAGTGGCTGCTACAGTTGTGTATCTAGTAACTGAA

ss.DNA64966   481 GTCTACTACAATTTTATGAAGCCTACACAGGAAATGAATATCAGCTTAGTCTGGTGCCTA
                  ************************************************************
P_AAC91566    481 GTCTACTACAATTTTATGAAGCCTACACAGGAAATGAATATCAGCTTAGTCTGGTGCCTA

ss.DNA64966   541 CTTGTTTTGTCTTTTGCAATCAAAGTTCTATTTTCATTAACTACACACTATTTTAAAGTA
                  ************************************************************
P_AAC91566    541 CTTGTTTTGTCTTTTGCAATCAAAGTTCTATTTTCATTAACTACACACTATTTTAAAGTA

ss.DNA64966   601 GAAGATGGTGGTGAAAGATCTGTTTGTGTCACCTTTGGATTTTTTTTCTTTGTCAAAGCA
                  ************************************************************
P_AAC91566    601 GAAGATGGTGGTGAAAGATCTGTTTGTGTCACCTTTGGATTTTTTTTCTTTGTCAAAGCA

ss.DNA64966   661 ATGGCAGTGTTGATTGTAACAGAAAATTATCTGGAATTTGGACTTGAAACAGGGTTTACA
                  ************************************************************
P_AAC91566    661 ATGGCAGTGTTGATTGTAACAGAAAATTATCTGGAATTTGGACTTGAAACAGGGTTTACA

ss.DNA64966   721 AATTTTTCAGACAGTGCGATGCAGTTTCTTGAAAAGCAAGGTTTAGAATCTCAGAGTCCT
                  ************************************************************
P_AAC91566    721 AATTTTTCAGACAGTGCGATGCAGTTTCTTGAAAAGCAAGGTTTAGAATCTCAGAGTCCT

ss.DNA64966   781 GTTTCAAAACTTACTTTCAAATTTTTCCTGGCTATTTTCTGTTCATTCATTGGGGCTTTT
                  ************************************************************
P_AAC91566    781 GTTTCAAAACTTACTTTCAAATTTTTCCTGGCTATTTTCTGTTCATTCATTGGGGCTTTT

ss.DNA64966   841 TTGACATTTCCTGGATTACGACTGGCTCAAATGCATCTGGATGCCCTGAATTTGGCAACA
                  ************************************************************
P_AAC91566    841 TTGACATTTCCTGGATTACGACTGGCTCAAATGCATCTGGATGCCCTGAATTTGGCAACA

ss.DNA64966   901 GAAAAAATTACACAAACTTTACTTCATATCAACTTCTTGGCACCTTTATTTATGGTTTTG
                  ************************************************************
P_AAC91566    901 GAAAAAATTACACAAACTTTACTTCATATCAACTTCTTGGCACCTTTATTTATGGTTTTG
```

```
ss.DNA64966      961 CTCTGGGTAAAACCAATCACCAAAGACTACATTATGAACCCACCACTGGCAAAGAAATT
                     ************************************************************
P_AAC91566       961 CTCTGGGTAAAACCAATCACCAAAGACTACATTATGAACCCACCACTGGCAAAGAAATT

ss.DNA64966     1021 TCCCCATCTGGAAGATGAAGATAATAGTATCTAACTCACAAGGTTATCATTGGAATAAAT
                     ************************************************************
P_AAC91566      1021 TCCCCATCTGGAAGATGAAGATAATAGTATCTAACTCACAAGGTTATCATTGGAATAAAT

ss.DNA64966     1081 GAAAGAACACATGTAATGCAACCAGCTGGAATTAAGTGCTTAATAAATGTTCTTTTCACT
                     ************************************************************
P_AAC91566      1081 GAAAGAACACATGTAATGCAACCAGCTGGAATTAAGTGCTTAATAAATGTTCTTTTCACT

ss.DNA64966     1141 GCTTTGCCTCATCAGAATTAAAATAGAAATACTTGACTAGT
                     *****************************************
P_AAC91566      1141 GCTTTGCCTCATCAGAATTAAAATAGAAATACTTGACTAGT

>3 P_AAA77681 Human PRO1313 cDNA sequence SEQ ID NO:215.  cDNA, PAT 07-NOV-2000
(1181 bp) [1 seg]
 Score = 1181 (2341 bits), Expect = 0.0
 Identities = 1181/1181 (100%), at 1,1-1181,1181, Strand +/+

ss.DNA64966        1 AAGACCCTCTCTTTCGCTGTTTGAGAGTCTCTCGGCTCAAGGACCGGGAGGTAAGAGGTT
                     ************************************************************
P_AAA77681         1 AAGACCCTCTCTTTCGCTGTTTGAGAGTCTCTCGGCTCAAGGACCGGGAGGTAAGAGGTT

ss.DNA64966       61 TGGGACTGCCCCGGCAACTCCAGGGTGTCTGGTCCACGACCTATCCTAGGCGCCATGGGT
                     ************************************************************
P_AAA77681        61 TGGGACTGCCCCGGCAACTCCAGGGTGTCTGGTCCACGACCTATCCTAGGCGCCATGGGT

ss.DNA64966      121 GTGATAGGTATACAGCTGGTTGTTACCATGGTGATGGCCAGTGTCATGCAGAAGATTATA
                     ************************************************************
P_AAA77681       121 GTGATAGGTATACAGCTGGTTGTTACCATGGTGATGGCCAGTGTCATGCAGAAGATTATA

ss.DNA64966      181 CCTCACTATTCTCTTGCTCGATGGCTACTCTGTAATGGCAGTTTGAGGTGGTATCAACAT
                     ************************************************************
P_AAA77681       181 CCTCACTATTCTCTTGCTCGATGGCTACTCTGTAATGGCAGTTTGAGGTGGTATCAACAT

ss.DNA64966      241 CCTACAGAAGAAGAATTAAGAATTCTTGCAGGGAAACAACAAAAAGGGAAAACCAAAAAA
                     ************************************************************
P_AAA77681       241 CCTACAGAAGAAGAATTAAGAATTCTTGCAGGGAAACAACAAAAAGGGAAAACCAAAAAA

ss.DNA64966      301 GATAGGAAATATAATGGTCACATTGAAAGTAAGCCATTAACCATTCCAAAGGATATTGAC
                     ************************************************************
P_AAA77681       301 GATAGGAAATATAATGGTCACATTGAAAGTAAGCCATTAACCATTCCAAAGGATATTGAC

ss.DNA64966      361 CTTCATCTAGAAACAAAGTCAGTTACAGAAGTGGATACTTTAGCATTGCATTACTTTCCA
                     ************************************************************
P_AAA77681       361 CTTCATCTAGAAACAAAGTCAGTTACAGAAGTGGATACTTTAGCATTGCATTACTTTCCA

ss.DNA64966      421 GAATACCAGTGGCTGGTGGATTTCACAGTGGCTGCTACAGTTGTGTATCTAGTAACTGAA
                     ************************************************************
P_AAA77681       421 GAATACCAGTGGCTGGTGGATTTCACAGTGGCTGCTACAGTTGTGTATCTAGTAACTGAA

ss.DNA64966      481 GTCTACTACAATTTTATGAAGCCTACACAGGAAATGAATATCAGCTTAGTCTGGTGCCTA
                     ************************************************************
P_AAA77681       481 GTCTACTACAATTTTATGAAGCCTACACAGGAAATGAATATCAGCTTAGTCTGGTGCCTA
```

```
ss.DNA64966     541 CTTGTTTTGTCTTTTGCAATCAAAGTTCTATTTTCATTAACTACACACTATTTTAAAGTA
                    ************************************************************
P_AAA77681      541 CTTGTTTTGTCTTTTGCAATCAAAGTTCTATTTTCATTAACTACACACTATTTTAAAGTA

ss.DNA64966     601 GAAGATGGTGGTGAAAGATCTGTTTGTGTCACCTTTGGATTTTTTTTCTTTGTCAAAGCA
                    ************************************************************
P_AAA77681      601 GAAGATGGTGGTGAAAGATCTGTTTGTGTCACCTTTGGATTTTTTTTCTTTGTCAAAGCA

ss.DNA64966     661 ATGGCAGTGTTGATTGTAACAGAAAATTATCTGGAATTTGGACTTGAAACAGGGTTTACA
                    ************************************************************
P_AAA77681      661 ATGGCAGTGTTGATTGTAACAGAAAATTATCTGGAATTTGGACTTGAAACAGGGTTTACA

ss.DNA64966     721 AATTTTTCAGACAGTGCGATGCAGTTTCTTGAAAAGCAAGGTTTAGAATCTCAGAGTCCT
                    ************************************************************
P_AAA77681      721 AATTTTTCAGACAGTGCGATGCAGTTTCTTGAAAAGCAAGGTTTAGAATCTCAGAGTCCT

ss.DNA64966     781 GTTTCAAAACTTACTTTCAAATTTTTCCTGGCTATTTTCTGTTCATTCATTGGGGCTTTT
                    ************************************************************
P_AAA77681      781 GTTTCAAAACTTACTTTCAAATTTTTCCTGGCTATTTTCTGTTCATTCATTGGGGCTTTT

ss.DNA64966     841 TTGACATTTCCTGGATTACGACTGGCTCAAATGCATCTGGATGCCCTGAATTTGGCAACA
                    ************************************************************
P_AAA77681      841 TTGACATTTCCTGGATTACGACTGGCTCAAATGCATCTGGATGCCCTGAATTTGGCAACA

ss.DNA64966     901 GAAAAAATTACACAAACTTTACTTCATATCAACTTCTTGGCACCTTTATTTATGGTTTTG
                    ************************************************************
P_AAA77681      901 GAAAAAATTACACAAACTTTACTTCATATCAACTTCTTGGCACCTTTATTTATGGTTTTG

ss.DNA64966     961 CTCTGGGTAAAACCAATCACCAAAGACTACATTATGAACCCACCACTGGGCAAAGAAATT
                    ************************************************************
P_AAA77681      961 CTCTGGGTAAAACCAATCACCAAAGACTACATTATGAACCCACCACTGGGCAAAGAAATT

ss.DNA64966    1021 TCCCCATCTGGAAGATGAAGATAATAGTATCTAACTCACAAGGTTATCATTGGAATAAAT
                    ************************************************************
P_AAA77681     1021 TCCCCATCTGGAAGATGAAGATAATAGTATCTAACTCACAAGGTTATCATTGGAATAAAT

ss.DNA64966    1081 GAAAGAACACATGTAATGCAACCAGCTGGAATTAAGTGCTTAATAAATGTTCTTTTCACT
                    ************************************************************
P_AAA77681     1081 GAAAGAACACATGTAATGCAACCAGCTGGAATTAAGTGCTTAATAAATGTTCTTTTCACT

ss.DNA64966    1141 GCTTTGCCTCATCAGAATTAAAATAGAAATACTTGACTAGT
                    *****************************************
P_AAA77681     1141 GCTTTGCCTCATCAGAATTAAAATAGAAATACTTGACTAGT

>4 P_AAC85948 Native sequence of PRO1313 cDNA, clone DNA64966-1575. (1181 bp) [1
seg]
 Score = 1181 (2341 bits), Expect = 0.0
 Identities = 1181/1181 (100%), at 1,1-1181,1181, Strand +/+

ss.DNA64966       1 AAGACCCTCTCTTTCGCTGTTTGAGAGTCTCTCGGCTCAAGGACCGGGAGGTAAGAGGTT
                    ************************************************************
P_AAC85948        1 AAGACCCTCTCTTTCGCTGTTTGAGAGTCTCTCGGCTCAAGGACCGGGAGGTAAGAGGTT

ss.DNA64966      61 TGGGACTGCCCCGGCAACTCCAGGGTGTCTGGTCCACGACCTATCCTAGGCGCCATGGGT
                    ************************************************************
P_AAC85948       61 TGGGACTGCCCCGGCAACTCCAGGGTGTCTGGTCCACGACCTATCCTAGGCGCCATGGGT
```

```
ss.DNA64966    121 GTGATAGGTATACAGCTGGTTGTTACCATGGTGATGGCCAGTGTCATGCAGAAGATTATA
                   ************************************************************
P_AAC85948     121 GTGATAGGTATACAGCTGGTTGTTACCATGGTGATGGCCAGTGTCATGCAGAAGATTATA

ss.DNA64966    181 CCTCACTATTCTCTTGCTCGATGGCTACTCTGTAATGGCAGTTTGAGGTGGTATCAACAT
                   ************************************************************
P_AAC85948     181 CCTCACTATTCTCTTGCTCGATGGCTACTCTGTAATGGCAGTTTGAGGTGGTATCAACAT

ss.DNA64966    241 CCTACAGAAGAAGAATTAAGAATTCTTGCAGGGAAACAACAAAAAGGGAAAACCAAAAAA
                   ************************************************************
P_AAC85948     241 CCTACAGAAGAAGAATTAAGAATTCTTGCAGGGAAACAACAAAAAGGGAAAACCAAAAAA

ss.DNA64966    301 GATAGGAAATATAATGGTCACATTGAAAGTAAGCCATTAACCATTCCAAAGGATATTGAC
                   ************************************************************
P_AAC85948     301 GATAGGAAATATAATGGTCACATTGAAAGTAAGCCATTAACCATTCCAAAGGATATTGAC

ss.DNA64966    361 CTTCATCTAGAAACAAAGTCAGTTACAGAAGTGGATACTTTAGCATTGCATTACTTTCCA
                   ************************************************************
P_AAC85948     361 CTTCATCTAGAAACAAAGTCAGTTACAGAAGTGGATACTTTAGCATTGCATTACTTTCCA

ss.DNA64966    421 GAATACCAGTGGCTGGTGGATTTCACAGTGGCTGCTACAGTTGTGTATCTAGTAACTGAA
                   ************************************************************
P_AAC85948     421 GAATACCAGTGGCTGGTGGATTTCACAGTGGCTGCTACAGTTGTGTATCTAGTAACTGAA

ss.DNA64966    481 GTCTACTACAATTTTATGAAGCCTACACAGGAAATGAATATCAGCTTAGTCTGGTGCCTA
                   ************************************************************
P_AAC85948     481 GTCTACTACAATTTTATGAAGCCTACACAGGAAATGAATATCAGCTTAGTCTGGTGCCTA

ss.DNA64966    541 CTTGTTTTGTCTTTTGCAATCAAAGTTCTATTTTCATTAACTACACACTATTTTAAAGTA
                   ************************************************************
P_AAC85948     541 CTTGTTTTGTCTTTTGCAATCAAAGTTCTATTTTCATTAACTACACACTATTTTAAAGTA

ss.DNA64966    601 GAAGATGGTGGTGAAAGATCTGTTTGTGTCACCTTTGGATTTTTTTTCTTTGTCAAAGCA
                   ************************************************************
P_AAC85948     601 GAAGATGGTGGTGAAAGATCTGTTTGTGTCACCTTTGGATTTTTTTTCTTTGTCAAAGCA

ss.DNA64966    661 ATGGCAGTGTTGATTGTAACAGAAAATTATCTGGAATTTGGACTTGAAACAGGGTTTACA
                   ************************************************************
P_AAC85948     661 ATGGCAGTGTTGATTGTAACAGAAAATTATCTGGAATTTGGACTTGAAACAGGGTTTACA

ss.DNA64966    721 AATTTTTCAGACAGTGCGATGCAGTTTCTTGAAAAGCAAGGTTTAGAATCTCAGAGTCCT
                   ************************************************************
P_AAC85948     721 AATTTTTCAGACAGTGCGATGCAGTTTCTTGAAAAGCAAGGTTTAGAATCTCAGAGTCCT

ss.DNA64966    781 GTTTCAAAACTTACTTTCAAATTTTTCCTGGCTATTTTCTGTTCATTCATTGGGGCTTTT
                   ************************************************************
P_AAC85948     781 GTTTCAAAACTTACTTTCAAATTTTTCCTGGCTATTTTCTGTTCATTCATTGGGGCTTTT

ss.DNA64966    841 TTGACATTTCCTGGATTACGACTGGCTCAAATGCATCTGGATGCCCTGAATTTGGCAACA
                   ************************************************************
P_AAC85948     841 TTGACATTTCCTGGATTACGACTGGCTCAAATGCATCTGGATGCCCTGAATTTGGCAACA

ss.DNA64966    901 GAAAAAATTACACAAACTTTACTTCATATCAACTTCTTGGCACCTTTATTTATGGTTTTG
                   ************************************************************
P_AAC85948     901 GAAAAAATTACACAAACTTTACTTCATATCAACTTCTTGGCACCTTTATTTATGGTTTTG

ss.DNA64966    961 CTCTGGGTAAAACCAATCACCAAAGACTACATTATGAACCCACCACTGGGCAAAGAAATT
```

```
                        ************************************************************
P_AAC85948         961 CTCTGGGTAAAACCAATCACCAAAGACTACATTATGAACCCACCACTGGCAAAGAAATT

ss.DNA64966       1021 TCCCCATCTGGAAGATGAAGATAATAGTATCTAACTCACAAGGTTATCATTGGAATAAAT
                       ************************************************************
P_AAC85948        1021 TCCCCATCTGGAAGATGAAGATAATAGTATCTAACTCACAAGGTTATCATTGGAATAAAT

ss.DNA64966       1081 GAAAGAACACATGTAATGCAACCAGCTGGAATTAAGTGCTTAATAAATGTTCTTTTCACT
                       ************************************************************
P_AAC85948        1081 GAAAGAACACATGTAATGCAACCAGCTGGAATTAAGTGCTTAATAAATGTTCTTTTCACT

ss.DNA64966       1141 GCTTTGCCTCATCAGAATTAAAATAGAAATACTTGACTAGT
                       *****************************************
P_AAC85948        1141 GCTTTGCCTCATCAGAATTAAAATAGAAATACTTGACTAGT

>5 AX055712 Sequence 27 from Patent WO0073348.  DNA, linear, PAT 13-JAN-2001
(1181 bp) [1 seg]
 Score = 1181 (2341 bits), Expect = 0.0
 Identities = 1181/1181 (100%), at 1,1-1181,1181, Strand +/+

ss.DNA64966          1 AAGACCCTCTCTTTCGCTGTTTGAGAGTCTCTCGGCTCAAGGACCGGGAGGTAAGAGGTT
                       ************************************************************
  AX055712           1 AAGACCCTCTCTTTCGCTGTTTGAGAGTCTCTCGGCTCAAGGACCGGGAGGTAAGAGGTT

ss.DNA64966         61 TGGGACTGCCCCGGCAACTCCAGGGTGTCTGGTCCACGACCTATCCTAGGCGCCATGGGT
                       ************************************************************
  AX055712          61 TGGGACTGCCCCGGCAACTCCAGGGTGTCTGGTCCACGACCTATCCTAGGCGCCATGGGT

ss.DNA64966        121 GTGATAGGTATACAGCTGGTTGTTACCATGGTGATGGCCAGTGTCATGCAGAAGATTATA
                       ************************************************************
  AX055712         121 GTGATAGGTATACAGCTGGTTGTTACCATGGTGATGGCCAGTGTCATGCAGAAGATTATA

ss.DNA64966        181 CCTCACTATTCTCTTGCTCGATGGCTACTCTGTAATGGCAGTTTGAGGTGGTATCAACAT
                       ************************************************************
  AX055712         181 CCTCACTATTCTCTTGCTCGATGGCTACTCTGTAATGGCAGTTTGAGGTGGTATCAACAT

ss.DNA64966        241 CCTACAGAAGAAGAATTAAGAATTCTTGCAGGGAAACAACAAAAAGGGAAAACCAAAAAA
                       ************************************************************
  AX055712         241 CCTACAGAAGAAGAATTAAGAATTCTTGCAGGGAAACAACAAAAAGGGAAAACCAAAAAA

ss.DNA64966        301 GATAGGAAATATAATGGTCACATTGAAAGTAAGCCATTAACCATTCCAAAGGATATTGAC
                       ************************************************************
  AX055712         301 GATAGGAAATATAATGGTCACATTGAAAGTAAGCCATTAACCATTCCAAAGGATATTGAC

ss.DNA64966        361 CTTCATCTAGAAACAAAGTCAGTTACAGAAGTGGATACTTTAGCATTGCATTACTTTCCA
                       ************************************************************
  AX055712         361 CTTCATCTAGAAACAAAGTCAGTTACAGAAGTGGATACTTTAGCATTGCATTACTTTCCA

ss.DNA64966        421 GAATACCAGTGGCTGGTGGATTTCACAGTGGCTGCTACAGTTGTGTATCTAGTAACTGAA
                       ************************************************************
  AX055712         421 GAATACCAGTGGCTGGTGGATTTCACAGTGGCTGCTACAGTTGTGTATCTAGTAACTGAA

ss.DNA64966        481 GTCTACTACAATTTTATGAAGCCTACACAGGAAATGAATATCAGCTTAGTCTGGTGCCTA
                       ************************************************************
  AX055712         481 GTCTACTACAATTTTATGAAGCCTACACAGGAAATGAATATCAGCTTAGTCTGGTGCCTA

ss.DNA64966        541 CTTGTTTTGTCTTTTGCAATCAAAGTTCTATTTTCATTAACTACACACTATTTTAAAGTA
```

```
                    ************************************************************
AX055712       541  CTTGTTTTGTCTTTTGCAATCAAAGTTCTATTTCATTAACTACACACTATTTAAAGTA

ss.DNA64966    601  GAAGATGGTGGTGAAAGATCTGTTTGTGTCACCTTTGGATTTTTTTTCTTTGTCAAAGCA
                    ************************************************************
AX055712       601  GAAGATGGTGGTGAAAGATCTGTTTGTGTCACCTTTGGATTTTTTTTCTTTGTCAAAGCA

ss.DNA64966    661  ATGGCAGTGTTGATTGTAACAGAAAATTATCTGGAATTTGGACTTGAAACAGGGTTTACA
                    ************************************************************
AX055712       661  ATGGCAGTGTTGATTGTAACAGAAAATTATCTGGAATTTGGACTTGAAACAGGGTTTACA

ss.DNA64966    721  AATTTTTCAGACAGTGCGATGCAGTTTCTTGAAAAGCAAGGTTTAGAATCTCAGAGTCCT
                    ************************************************************
AX055712       721  AATTTTTCAGACAGTGCGATGCAGTTTCTTGAAAAGCAAGGTTTAGAATCTCAGAGTCCT

ss.DNA64966    781  GTTTCAAAACTTACTTTCAAATTTTTCCTGGCTATTTTCTGTTCATTCATTGGGGCTTTT
                    ************************************************************
AX055712       781  GTTTCAAAACTTACTTTCAAATTTTTCCTGGCTATTTTCTGTTCATTCATTGGGGCTTTT

ss.DNA64966    841  TTGACATTTCCTGGATTACGACTGGCTCAAATGCATCTGGATGCCCTGAATTTGGCAACA
                    ************************************************************
AX055712       841  TTGACATTTCCTGGATTACGACTGGCTCAAATGCATCTGGATGCCCTGAATTTGGCAACA

ss.DNA64966    901  GAAAAAATTACACAAACTTTACTTCATATCAACTTCTTGGCACCTTTATTTATGGTTTTG
                    ************************************************************
AX055712       901  GAAAAAATTACACAAACTTTACTTCATATCAACTTCTTGGCACCTTTATTTATGGTTTTG

ss.DNA64966    961  CTCTGGGTAAAACCAATCACCAAAGACTACATTATGAACCCACCACTGGGCAAAGAAATT
                    ************************************************************
AX055712       961  CTCTGGGTAAAACCAATCACCAAAGACTACATTATGAACCCACCACTGGGCAAAGAAATT

ss.DNA64966   1021  TCCCCATCTGGAAGATGAAGATAATAGTATCTAACTCACAAGGTTATCATTGGAATAAAT
                    ************************************************************
AX055712      1021  TCCCCATCTGGAAGATGAAGATAATAGTATCTAACTCACAAGGTTATCATTGGAATAAAT

ss.DNA64966   1081  GAAAGAACACATGTAATGCAACCAGCTGGAATTAAGTGCTTAATAAATGTTCTTTTCACT
                    ************************************************************
AX055712      1081  GAAAGAACACATGTAATGCAACCAGCTGGAATTAAGTGCTTAATAAATGTTCTTTTCACT

ss.DNA64966   1141  GCTTTGCCTCATCAGAATTAAAATAGAAATACTTGACTAGT
                    *****************************************
AX055712      1141  GCTTTGCCTCATCAGAATTAAAATAGAAATACTTGACTAGT
```

```
>6 AX150811 Sequence 7 from Patent WO0140464.  DNA, linear, PAT 22-JUN-2001
(1181 bp) [1 seg]
 Score = 1181 (2341 bits), Expect = 0.0
 Identities = 1181/1181 (100%), at 1,1-1181,1181, Strand +/+
```

```
ss.DNA64966      1  AAGACCCTCTCTTTCGCTGTTTGAGAGTCTCTCGGCTCAAGGACCGGGAGGTAAGAGGTT
                    ************************************************************
AX150811         1  AAGACCCTCTCTTTCGCTGTTTGAGAGTCTCTCGGCTCAAGGACCGGGAGGTAAGAGGTT

ss.DNA64966     61  TGGGACTGCCCCGGCAACTCCAGGGTGTCTGGTCCACGACCTATCCTAGGCGCCATGGGT
                    ************************************************************
AX150811        61  TGGGACTGCCCCGGCAACTCCAGGGTGTCTGGTCCACGACCTATCCTAGGCGCCATGGGT

ss.DNA64966    121  GTGATAGGTATACAGCTGGTTGTTACCATGGTGATGGCCAGTGTCATGCAGAAGATTATA
```

```
                     ************************************************************
AX150811        121  GTGATAGGTATACAGCTGGTTGTTACCATGGTGATGGCCAGTGTCATGCAGAAGATTATA

ss.DNA64966     181  CCTCACTATTCTCTTGCTCGATGGCTACTCTGTAATGGCAGTTTGAGGTGGTATCAACAT
                     ************************************************************
AX150811        181  CCTCACTATTCTCTTGCTCGATGGCTACTCTGTAATGGCAGTTTGAGGTGGTATCAACAT

ss.DNA64966     241  CCTACAGAAGAAGAATTAAGAATTCTTGCAGGGAAACAACAAAAAGGGAAAACCAAAAAA
                     ************************************************************
AX150811        241  CCTACAGAAGAAGAATTAAGAATTCTTGCAGGGAAACAACAAAAAGGGAAAACCAAAAAA

ss.DNA64966     301  GATAGGAAATATAATGGTCACATTGAAAGTAAGCCATTAACCATTCCAAAGGATATTGAC
                     ************************************************************
AX150811        301  GATAGGAAATATAATGGTCACATTGAAAGTAAGCCATTAACCATTCCAAAGGATATTGAC

ss.DNA64966     361  CTTCATCTAGAAACAAAGTCAGTTACAGAAGTGGATACTTTAGCATTGCATTACTTTCCA
                     ************************************************************
AX150811        361  CTTCATCTAGAAACAAAGTCAGTTACAGAAGTGGATACTTTAGCATTGCATTACTTTCCA

ss.DNA64966     421  GAATACCAGTGGCTGGTGGATTTCACAGTGGCTGCTACAGTTGTGTATCTAGTAACTGAA
                     ************************************************************
AX150811        421  GAATACCAGTGGCTGGTGGATTTCACAGTGGCTGCTACAGTTGTGTATCTAGTAACTGAA

ss.DNA64966     481  GTCTACTACAATTTTATGAAGCCTACACAGGAAATGAATATCAGCTTAGTCTGGTGCCTA
                     ************************************************************
AX150811        481  GTCTACTACAATTTTATGAAGCCTACACAGGAAATGAATATCAGCTTAGTCTGGTGCCTA

ss.DNA64966     541  CTTGTTTTGTCTTTTGCAATCAAAGTTCTATTTTCATTAACTACACACTATTTTAAAGTA
                     ************************************************************
AX150811        541  CTTGTTTTGTCTTTTGCAATCAAAGTTCTATTTTCATTAACTACACACTATTTTAAAGTA

ss.DNA64966     601  GAAGATGGTGGTGAAAGATCTGTTTGTGTCACCTTTGGATTTTTTTTCTTTGTCAAAGCA
                     ************************************************************
AX150811        601  GAAGATGGTGGTGAAAGATCTGTTTGTGTCACCTTTGGATTTTTTTTCTTTGTCAAAGCA

ss.DNA64966     661  ATGGCAGTGTTGATTGTAACAGAAAATTATCTGGAATTTGGACTTGAAACAGGGTTTACA
                     ************************************************************
AX150811        661  ATGGCAGTGTTGATTGTAACAGAAAATTATCTGGAATTTGGACTTGAAACAGGGTTTACA

ss.DNA64966     721  AATTTTTCAGACAGTGCGATGCAGTTTCTTGAAAAGCAAGGTTTAGAATCTCAGAGTCCT
                     ************************************************************
AX150811        721  AATTTTTCAGACAGTGCGATGCAGTTTCTTGAAAAGCAAGGTTTAGAATCTCAGAGTCCT

ss.DNA64966     781  GTTTCAAAACTTACTTTCAAATTTTTCCTGGCTATTTTCTGTTCATTCATTGGGGCTTTT
                     ************************************************************
AX150811        781  GTTTCAAAACTTACTTTCAAATTTTTCCTGGCTATTTTCTGTTCATTCATTGGGGCTTTT

ss.DNA64966     841  TTGACATTTCCTGGATTACGACTGGCTCAAATGCATCTGGATGCCCTGAATTTGGCAACA
                     ************************************************************
AX150811        841  TTGACATTTCCTGGATTACGACTGGCTCAAATGCATCTGGATGCCCTGAATTTGGCAACA

ss.DNA64966     901  GAAAAAATTACACAAACTTTACTTCATATCAACTTCTTGGCACCTTTATTTATGGTTTTG
                     ************************************************************
AX150811        901  GAAAAAATTACACAAACTTTACTTCATATCAACTTCTTGGCACCTTTATTTATGGTTTTG

ss.DNA64966     961  CTCTGGGTAAAACCAATCACCAAAGACTACATTATGAACCCACCACTGGGCAAAGAAATT
                     ************************************************************
```

```
AX150811       961 CTCTGGGTAAAACCAATCACCAAAGACTACATTATGAACCCACCACTGGGCAAAGAAATT

ss.DNA64966   1021 TCCCCATCTGGAAGATGAAGATAATAGTATCTAACTCACAAGGTTATCATTGGAATAAAT
                   ************************************************************
AX150811      1021 TCCCCATCTGGAAGATGAAGATAATAGTATCTAACTCACAAGGTTATCATTGGAATAAAT

ss.DNA64966   1081 GAAAGAACACATGTAATGCAACCAGCTGGAATTAAGTGCTTAATAAATGTTCTTTTCACT
                   ************************************************************
AX150811      1081 GAAAGAACACATGTAATGCAACCAGCTGGAATTAAGTGCTTAATAAATGTTCTTTTCACT

ss.DNA64966   1141 GCTTTGCCTCATCAGAATTAAAATAGAAATACTTGACTAGT
                   *****************************************
AX150811      1141 GCTTTGCCTCATCAGAATTAAAATAGAAATACTTGACTAGT

>7 MGC33214 Homo sapiens hypothetical protein MGC33214 (MGC33214), mRNA. (2675
bp) [1 seg]
 Score = 1018 (2018 bits), Expect = 0.0
 Identities = 1018/1018 (100%), at 1,17-1018,1034, Strand +/+

ss.DNA64966      1 AAGACCCTCTCTTTCGCTGTTTGAGAGTCTCTCGGCTCAAGGACCGGGAGGTAAGAGGTT
                   ************************************************************
MGC33214        17 AAGACCCTCTCTTTCGCTGTTTGAGAGTCTCTCGGCTCAAGGACCGGGAGGTAAGAGGTT

ss.DNA64966     61 TGGGACTGCCCCGGCAACTCCAGGGTGTCTGGTCCACGACCTATCCTAGGCGCCATGGGT
                   ************************************************************
MGC33214        77 TGGGACTGCCCCGGCAACTCCAGGGTGTCTGGTCCACGACCTATCCTAGGCGCCATGGGT

ss.DNA64966    121 GTGATAGGTATACAGCTGGTTGTTACCATGGTGATGGCCAGTGTCATGCAGAAGATTATA
                   ************************************************************
MGC33214       137 GTGATAGGTATACAGCTGGTTGTTACCATGGTGATGGCCAGTGTCATGCAGAAGATTATA

ss.DNA64966    181 CCTCACTATTCTCTTGCTCGATGGCTACTCTGTAATGGCAGTTTGAGGTGGTATCAACAT
                   ************************************************************
MGC33214       197 CCTCACTATTCTCTTGCTCGATGGCTACTCTGTAATGGCAGTTTGAGGTGGTATCAACAT

ss.DNA64966    241 CCTACAGAAGAAGAATTAAGAATTCTTGCAGGGAAACAACAAAAAGGGAAAACCAAAAAA
                   ************************************************************
MGC33214       257 CCTACAGAAGAAGAATTAAGAATTCTTGCAGGGAAACAACAAAAAGGGAAAACCAAAAAA

ss.DNA64966    301 GATAGGAAATATAATGGTCACATTGAAAGTAAGCCATTAACCATTCCAAAGGATATTGAC
                   ************************************************************
MGC33214       317 GATAGGAAATATAATGGTCACATTGAAAGTAAGCCATTAACCATTCCAAAGGATATTGAC

ss.DNA64966    361 CTTCATCTAGAAACAAAGTCAGTTACAGAAGTGGATACTTTAGCATTGCATTACTTTCCA
                   ************************************************************
MGC33214       377 CTTCATCTAGAAACAAAGTCAGTTACAGAAGTGGATACTTTAGCATTGCATTACTTTCCA

ss.DNA64966    421 GAATACCAGTGGCTGGTGGATTTCACAGTGGCTGCTACAGTTGTGTATCTAGTAACTGAA
                   ************************************************************
MGC33214       437 GAATACCAGTGGCTGGTGGATTTCACAGTGGCTGCTACAGTTGTGTATCTAGTAACTGAA

ss.DNA64966    481 GTCTACTACAATTTTATGAAGCCTACACAGGAAATGAATATCAGCTTAGTCTGGTGCCTA
                   ************************************************************
MGC33214       497 GTCTACTACAATTTTATGAAGCCTACACAGGAAATGAATATCAGCTTAGTCTGGTGCCTA

ss.DNA64966    541 CTTGTTTTGTCTTTTGCAATCAAAGTTCTATTTTCATTAACTACACACTATTTTAAAGTA
                   ************************************************************
```

```
MGC33214       557 CTTGTTTTGTCTTTTGCAATCAAAGTTCTATTTTCATTAACTACACACTATTTTAAAGTA

ss.DNA64966    601 GAAGATGGTGGTGAAAGATCTGTTTGTGTCACCTTTGGATTTTTTTCTTTGTCAAAGCA
                   ************************************************************
MGC33214       617 GAAGATGGTGGTGAAAGATCTGTTTGTGTCACCTTTGGATTTTTTTCTTTGTCAAAGCA

ss.DNA64966    661 ATGGCAGTGTTGATTGTAACAGAAAATTATCTGGAATTTGGACTTGAAACAGGGTTTACA
                   ************************************************************
MGC33214       677 ATGGCAGTGTTGATTGTAACAGAAAATTATCTGGAATTTGGACTTGAAACAGGGTTTACA

ss.DNA64966    721 AATTTTTCAGACAGTGCGATGCAGTTTCTTGAAAAGCAAGGTTTAGAATCTCAGAGTCCT
                   ************************************************************
MGC33214       737 AATTTTTCAGACAGTGCGATGCAGTTTCTTGAAAAGCAAGGTTTAGAATCTCAGAGTCCT

ss.DNA64966    781 GTTTCAAAACTTACTTTCAAATTTTTCCTGGCTATTTTCTGTTCATTCATTGGGGCTTTT
                   ************************************************************
MGC33214       797 GTTTCAAAACTTACTTTCAAATTTTTCCTGGCTATTTTCTGTTCATTCATTGGGGCTTTT

ss.DNA64966    841 TTGACATTTCCTGGATTACGACTGGCTCAAATGCATCTGGATGCCCTGAATTTGGCAACA
                   ************************************************************
MGC33214       857 TTGACATTTCCTGGATTACGACTGGCTCAAATGCATCTGGATGCCCTGAATTTGGCAACA

ss.DNA64966    901 GAAAAAATTACACAAACTTTACTTCATATCAACTTCTTGGCACCTTTATTTATGGTTTTG
                   ************************************************************
MGC33214       917 GAAAAAATTACACAAACTTTACTTCATATCAACTTCTTGGCACCTTTATTTATGGTTTTG

ss.DNA64966    961 CTCTGGGTAAAACCAATCACCAAAGACTACATTATGAACCCACCACTGGCAAAGAAA
                   *********************************************************
MGC33214       977 CTCTGGGTAAAACCAATCACCAAAGACTACATTATGAACCCACCACTGGCAAAGAAA

>8 BC037287 Homo sapiens, Similar to hypothetical protein FLJ20422, clone (2675
bp) [1 seg]
 Score = 1018 (2018 bits), Expect = 0.0
 Identities = 1018/1018 (100%), at 1,17-1018,1034, Strand +/+

ss.DNA64966      1 AAGACCCTCTCTTTCGCTGTTTGAGAGTCTCTCGGCTCAAGGACCGGGAGGTAAGAGGTT
                   ************************************************************
BC037287        17 AAGACCCTCTCTTTCGCTGTTTGAGAGTCTCTCGGCTCAAGGACCGGGAGGTAAGAGGTT

ss.DNA64966     61 TGGGACTGCCCCGGCAACTCCAGGGTGTCTGGTCCACGACCTATCCTAGGCGCCATGGGT
                   ************************************************************
BC037287        77 TGGGACTGCCCCGGCAACTCCAGGGTGTCTGGTCCACGACCTATCCTAGGCGCCATGGGT

ss.DNA64966    121 GTGATAGGTATACAGCTGGTTGTTACCATGGTGATGGCCAGTGTCATGCAGAAGATTATA
                   ************************************************************
BC037287       137 GTGATAGGTATACAGCTGGTTGTTACCATGGTGATGGCCAGTGTCATGCAGAAGATTATA

ss.DNA64966    181 CCTCACTATTCTCTTGCTCGATGGCTACTCTGTAATGGCAGTTTGAGGTGGTATCAACAT
                   ************************************************************
BC037287       197 CCTCACTATTCTCTTGCTCGATGGCTACTCTGTAATGGCAGTTTGAGGTGGTATCAACAT

ss.DNA64966    241 CCTACAGAAGAAGAATTAAGAATTCTTGCAGGGAAACAACAAAAAGGGAAAACCAAAAAA
                   ************************************************************
BC037287       257 CCTACAGAAGAAGAATTAAGAATTCTTGCAGGGAAACAACAAAAAGGGAAAACCAAAAAA

ss.DNA64966    301 GATAGGAAATATAATGGTCACATTGAAAGTAAGCCATTAACCATTCCAAAGGATATTGAC
                   ************************************************************
```

```
BC037287      317 GATAGGAAATATAATGGTCACATTGAAAGTAAGCCATTAACCATTCCAAAGGATATTGAC

ss.DNA64966   361 CTTCATCTAGAAACAAAGTCAGTTACAGAAGTGGATACTTTAGCATTGCATTACTTTCCA
                  ************************************************************
BC037287      377 CTTCATCTAGAAACAAAGTCAGTTACAGAAGTGGATACTTTAGCATTGCATTACTTTCCA

ss.DNA64966   421 GAATACCAGTGGCTGGTGGATTTCACAGTGGCTGCTACAGTTGTGTATCTAGTAACTGAA
                  ************************************************************
BC037287      437 GAATACCAGTGGCTGGTGGATTTCACAGTGGCTGCTACAGTTGTGTATCTAGTAACTGAA

ss.DNA64966   481 GTCTACTACAATTTTATGAAGCCTACACAGGAAATGAATATCAGCTTAGTCTGGTGCCTA
                  ************************************************************
BC037287      497 GTCTACTACAATTTTATGAAGCCTACACAGGAAATGAATATCAGCTTAGTCTGGTGCCTA

ss.DNA64966   541 CTTGTTTTGTCTTTTGCAATCAAAGTTCTATTTTCATTAACTACACACTATTTTAAAGTA
                  ************************************************************
BC037287      557 CTTGTTTTGTCTTTTGCAATCAAAGTTCTATTTTCATTAACTACACACTATTTTAAAGTA

ss.DNA64966   601 GAAGATGGTGGTGAAAGATCTGTTTGTGTCACCTTTGGATTTTTTTTCTTTGTCAAAGCA
                  ************************************************************
BC037287      617 GAAGATGGTGGTGAAAGATCTGTTTGTGTCACCTTTGGATTTTTTTTCTTTGTCAAAGCA

ss.DNA64966   661 ATGGCAGTGTTGATTGTAACAGAAAATTATCTGGAATTTGGACTTGAAACAGGGTTTACA
                  ************************************************************
BC037287      677 ATGGCAGTGTTGATTGTAACAGAAAATTATCTGGAATTTGGACTTGAAACAGGGTTTACA

ss.DNA64966   721 AATTTTTCAGACAGTGCGATGCAGTTTCTTGAAAAGCAAGGTTTAGAATCTCAGAGTCCT
                  ************************************************************
BC037287      737 AATTTTTCAGACAGTGCGATGCAGTTTCTTGAAAAGCAAGGTTTAGAATCTCAGAGTCCT

ss.DNA64966   781 GTTTCAAAACTTACTTTCAAATTTTTCCTGGCTATTTTCTGTTCATTCATTGGGGCTTTT
                  ************************************************************
BC037287      797 GTTTCAAAACTTACTTTCAAATTTTTCCTGGCTATTTTCTGTTCATTCATTGGGGCTTTT

ss.DNA64966   841 TTGACATTTCCTGGATTACGACTGGCTCAAATGCATCTGGATGCCCTGAATTTGGCAACA
                  ************************************************************
BC037287      857 TTGACATTTCCTGGATTACGACTGGCTCAAATGCATCTGGATGCCCTGAATTTGGCAACA

ss.DNA64966   901 GAAAAAATTACACAAACTTTACTTCATATCAACTTCTTGGCACCTTTATTTATGGTTTTG
                  ************************************************************
BC037287      917 GAAAAAATTACACAAACTTTACTTCATATCAACTTCTTGGCACCTTTATTTATGGTTTTG

ss.DNA64966   961 CTCTGGGTAAAACCAATCACCAAAGACTACATTATGAACCCACCACTGGGCAAAGAAA
                  **********************************************************
BC037287      977 CTCTGGGTAAAACCAATCACCAAAGACTACATTATGAACCCACCACTGGGCAAAGAAA

>9 P_AAS63166 Human purified secretory polynucleotide #22. (2482 bp) [1 seg]
 Score = 1014 (2010 bits), Expect = 0.0
 Identities = 1014/1014 (100%), at 5,1-1018,1014, Strand +/+

ss.DNA64966     5 CCCTCTCTTTCGCTGTTTGAGAGTCTCTCGGCTCAAGGACCGGGAGGTAAGAGGTTTGGG
                  ************************************************************
P_AAS63166      1 CCCTCTCTTTCGCTGTTTGAGAGTCTCTCGGCTCAAGGACCGGGAGGTAAGAGGTTTGGG

ss.DNA64966    65 ACTGCCCCGGCAACTCCAGGGTGTCTGGTCCACGACCTATCCTAGGCGCCATGGGTGTGA
                  ************************************************************
P_AAS63166     61 ACTGCCCCGGCAACTCCAGGGTGTCTGGTCCACGACCTATCCTAGGCGCCATGGGTGTGA
```

```
ss.DNA64966   125 TAGGTATACAGCTGGTTGTTACCATGGTGATGGCCAGTGTCATGCAGAAGATTATACCTC
                  ************************************************************
P_AAS63166    121 TAGGTATACAGCTGGTTGTTACCATGGTGATGGCCAGTGTCATGCAGAAGATTATACCTC

ss.DNA64966   185 ACTATTCTCTTGCTCGATGGCTACTCTGTAATGGCAGTTTGAGGTGGTATCAACATCCTA
                  ************************************************************
P_AAS63166    181 ACTATTCTCTTGCTCGATGGCTACTCTGTAATGGCAGTTTGAGGTGGTATCAACATCCTA

ss.DNA64966   245 CAGAAGAAGAATTAAGAATTCTTGCAGGGAAACAACAAAAAGGGAAAACCAAAAAAGATA
                  ************************************************************
P_AAS63166    241 CAGAAGAAGAATTAAGAATTCTTGCAGGGAAACAACAAAAAGGGAAAACCAAAAAAGATA

ss.DNA64966   305 GGAAATATAATGGTCACATTGAAAGTAAGCCATTAACCATTCCAAAGGATATTGACCTTC
                  ************************************************************
P_AAS63166    301 GGAAATATAATGGTCACATTGAAAGTAAGCCATTAACCATTCCAAAGGATATTGACCTTC

ss.DNA64966   365 ATCTAGAAACAAAGTCAGTTACAGAAGTGGATACTTTAGCATTGCATTACTTTCCAGAAT
                  ************************************************************
P_AAS63166    361 ATCTAGAAACAAAGTCAGTTACAGAAGTGGATACTTTAGCATTGCATTACTTTCCAGAAT

ss.DNA64966   425 ACCAGTGGCTGGTGGATTTCACAGTGGCTGCTACAGTTGTGTATCTAGTAACTGAAGTCT
                  ************************************************************
P_AAS63166    421 ACCAGTGGCTGGTGGATTTCACAGTGGCTGCTACAGTTGTGTATCTAGTAACTGAAGTCT

ss.DNA64966   485 ACTACAATTTTATGAAGCCTACACAGGAAATGAATATCAGCTTAGTCTGGTGCCTACTTG
                  ************************************************************
P_AAS63166    481 ACTACAATTTTATGAAGCCTACACAGGAAATGAATATCAGCTTAGTCTGGTGCCTACTTG

ss.DNA64966   545 TTTTGTCTTTTGCAATCAAAGTTCTATTTTCATTAACTACACACTATTTTAAAGTAGAAG
                  ************************************************************
P_AAS63166    541 TTTTGTCTTTTGCAATCAAAGTTCTATTTTCATTAACTACACACTATTTTAAAGTAGAAG

ss.DNA64966   605 ATGGTGGTGAAAGATCTGTTTGTGTCACCTTTGGATTTTTTTTCTTTGTCAAAGCAATGG
                  ************************************************************
P_AAS63166    601 ATGGTGGTGAAAGATCTGTTTGTGTCACCTTTGGATTTTTTTTCTTTGTCAAAGCAATGG

ss.DNA64966   665 CAGTGTTGATTGTAACAGAAAATTATCTGGAATTTGGACTTGAAACAGGGTTTACAAATT
                  ************************************************************
P_AAS63166    661 CAGTGTTGATTGTAACAGAAAATTATCTGGAATTTGGACTTGAAACAGGGTTTACAAATT

ss.DNA64966   725 TTTCAGACAGTGCGATGCAGTTTCTTGAAAAGCAAGGTTTAGAATCTCAGAGTCCTGTTT
                  ************************************************************
P_AAS63166    721 TTTCAGACAGTGCGATGCAGTTTCTTGAAAAGCAAGGTTTAGAATCTCAGAGTCCTGTTT

ss.DNA64966   785 CAAAACTTACTTTCAAATTTTTCCTGGCTATTTTCTGTTCATTCATTGGGGCTTTTTTGA
                  ************************************************************
P_AAS63166    781 CAAAACTTACTTTCAAATTTTTCCTGGCTATTTTCTGTTCATTCATTGGGGCTTTTTTGA

ss.DNA64966   845 CATTTCCTGGATTACGACTGGCTCAAATGCATCTGGATGCCCTGAATTTGGCAACAGAAA
                  ************************************************************
P_AAS63166    841 CATTTCCTGGATTACGACTGGCTCAAATGCATCTGGATGCCCTGAATTTGGCAACAGAAA

ss.DNA64966   905 AAATTACACAAACTTTACTTCATATCAACTTCTTGGCACCTTTATTTATGGTTTTGCTCT
                  ************************************************************
P_AAS63166    901 AAATTACACAAACTTTACTTCATATCAACTTCTTGGCACCTTTATTTATGGTTTTGCTCT
```

```
ss.DNA64966    965 GGGTAAAACCAATCACCAAAGACTACATTATGAACCCACCACTGGGCAAAGAAA
                   ******************************************************
P_AAS63166     961 GGGTAAAACCAATCACCAAAGACTACATTATGAACCCACCACTGGGCAAAGAAA
```

```
>10 P_ABN99385 Human secreted protein (SCEP) coding sequence 26. (2644 bp) [1
seg]
 Score = 1010 (2002 bits), Expect = 0.0
 Identities = 1013/1014 (99%), at 5,1-1018,1014, Strand +/+
```

```
ss.DNA64966      5 CCCTCTCTTTCGCTGTTTGAGAGTCTCTCGGCTCAAGGACCGGGAGGTAAGAGGTTTGGG
                   ************************************************************
P_ABN99385       1 CCCTCTCTTTCGCTGTTTGAGAGTCTCTCGGCTCAAGGACCGGGAGGTAAGAGGTTTGGG

ss.DNA64966     65 ACTGCCCCGGCAACTCCAGGGTGTCTGGTCCACGACCTATCCTAGGCGCCATGGGTGTGA
                   ************************************************************
P_ABN99385      61 ACTGCCCCGGCAACTCCAGGGTGTCTGGTCCACGACCTATCCTAGGCGCCATGGGTGTGA

ss.DNA64966    125 TAGGTATACAGCTGGTTGTTACCATGGTGATGGCCAGTGTCATGCAGAAGATTATACCTC
                   ************************************************************
P_ABN99385     121 TAGGTATACAGCTGGTTGTTACCATGGTGATGGCCAGTGTCATGCAGAAGATTATACCTC

ss.DNA64966    185 ACTATTCTCTTGCTCGATGGCTACTCTGTAATGGCAGTTTGAGGTGGTATCAACATCCTA
                   **************************************************** *******
P_ABN99385     181 ACTATTCTCTTGCTCGATGGCTACTCTGTAATGGCAGTTTGAGGTGGTATCAGCATCCTA

ss.DNA64966    245 CAGAAGAAGAATTAAGAATTCTTGCAGGGAAACAACAAAAAGGGAAAACCAAAAAAGATA
                   ************************************************************
P_ABN99385     241 CAGAAGAAGAATTAAGAATTCTTGCAGGGAAACAACAAAAAGGGAAAACCAAAAAAGATA

ss.DNA64966    305 GGAAATATAATGGTCACATTGAAAGTAAGCCATTAACCATTCCAAAGGATATTGACCTTC
                   ************************************************************
P_ABN99385     301 GGAAATATAATGGTCACATTGAAAGTAAGCCATTAACCATTCCAAAGGATATTGACCTTC

ss.DNA64966    365 ATCTAGAAACAAAGTCAGTTACAGAAGTGGATACTTTAGCATTGCATTACTTTCCAGAAT
                   ************************************************************
P_ABN99385     361 ATCTAGAAACAAAGTCAGTTACAGAAGTGGATACTTTAGCATTGCATTACTTTCCAGAAT

ss.DNA64966    425 ACCAGTGGCTGGTGGATTTCACAGTGGCTGCTACAGTTGTGTATCTAGTAACTGAAGTCT
                   ************************************************************
P_ABN99385     421 ACCAGTGGCTGGTGGATTTCACAGTGGCTGCTACAGTTGTGTATCTAGTAACTGAAGTCT

ss.DNA64966    485 ACTACAATTTTATGAAGCCTACACAGGAAATGAATATCAGCTTAGTCTGGTGCCTACTTG
                   ************************************************************
P_ABN99385     481 ACTACAATTTTATGAAGCCTACACAGGAAATGAATATCAGCTTAGTCTGGTGCCTACTTG

ss.DNA64966    545 TTTTGTCTTTTGCAATCAAAGTTCTATTTTCATTAACTACACACTATTTTAAAGTAGAAG
                   ************************************************************
P_ABN99385     541 TTTTGTCTTTTGCAATCAAAGTTCTATTTTCATTAACTACACACTATTTTAAAGTAGAAG

ss.DNA64966    605 ATGGTGGTGAAAGATCTGTTTGTGTCACCTTTGGATTTTTTTCTTTGTCAAAGCAATGG
                   ************************************************************
P_ABN99385     601 ATGGTGGTGAAAGATCTGTTTGTGTCACCTTTGGATTTTTTTCTTTGTCAAAGCAATGG

ss.DNA64966    665 CAGTGTTGATTGTAACAGAAAATTATCTGGAATTTGGACTTGAAACAGGGTTTACAAATT
                   ************************************************************
P_ABN99385     661 CAGTGTTGATTGTAACAGAAAATTATCTGGAATTTGGACTTGAAACAGGGTTTACAAATT
```

```
ss.DNA64966     725 TTTCAGACAGTGCGATGCAGTTTCTTGAAAAGCAAGGTTTAGAATCTCAGAGTCCTGTTT
                    ************************************************************
P_ABN99385      721 TTTCAGACAGTGCGATGCAGTTTCTTGAAAAGCAAGGTTTAGAATCTCAGAGTCCTGTTT

ss.DNA64966     785 CAAAACTTACTTTCAAATTTTTCCTGGCTATTTTCTGTTCATTCATTGGGGCTTTTTTGA
                    ************************************************************
P_ABN99385      781 CAAAACTTACTTTCAAATTTTTCCTGGCTATTTTCTGTTCATTCATTGGGGCTTTTTTGA

ss.DNA64966     845 CATTTCCTGGATTACGACTGGCTCAAATGCATCTGGATGCCCTGAATTTGGCAACAGAAA
                    ************************************************************
P_ABN99385      841 CATTTCCTGGATTACGACTGGCTCAAATGCATCTGGATGCCCTGAATTTGGCAACAGAAA

ss.DNA64966     905 AAATTACACAAACTTTACTTCATATCAACTTCTTGGCACCTTTATTTATGGTTTTGCTCT
                    ************************************************************
P_ABN99385      901 AAATTACACAAACTTTACTTCATATCAACTTCTTGGCACCTTTATTTATGGTTTTGCTCT

ss.DNA64966     965 GGGTAAAACCAATCACCAAAGACTACATTATGAACCCACCACTGGGCAAAGAAA
                    ******************************************************
P_ABN99385      961 GGGTAAAACCAATCACCAAAGACTACATTATGAACCCACCACTGGGCAAAGAAA

>11 XM_087662 Homo sapiens (2477 bp) [1 seg]
 Score = 1006 (1994 bits), Expect = 0.0
 Identities = 1009/1010 (99%), at 9,1-1018,1010, Strand +/+

ss.DNA64966       9 CTCTTTCGCTGTTTGAGAGTCTCTCGGCTCAAGGACCGGGAGGTAAGAGGTTTGGGACTG
                    ************************************************************
 XM_087662        1 CTCTTTCGCTGTTTGAGAGTCTCTCGGCTCAAGGACCGGGAGGTAAGAGGTTTGGGACTG

ss.DNA64966      69 CCCCGGCAACTCCAGGGTGTCTGGTCCACGACCTATCCTAGGCGCCATGGGTGTGATAGG
                    ************************************************************
 XM_087662       61 CCCCGGCAACTCCAGGGTGTCTGGTCCACGACCTATCCTAGGCGCCATGGGTGTGATAGG

ss.DNA64966     129 TATACAGCTGGTTGTTACCATGGTGATGGCCAGTGTCATGCAGAAGATTATACCTCACTA
                    ************************************************************
 XM_087662      121 TATACAGCTGGTTGTTACCATGGTGATGGCCAGTGTCATGCAGAAGATTATACCTCACTA

ss.DNA64966     189 TTCTCTTGCTCGATGGCTACTCTGTAATGGCAGTTTGAGGTGGTATCAACATCCTACAGA
                    ************************************************************
 XM_087662      181 TTCTCTTGCTCGATGGCTACTCTGTAATGGCAGTTTGAGGTGGTATCAACATCCTACAGA

ss.DNA64966     249 AGAAGAATTAAGAATTCTTGCAGGGAAACAACAAAAAGGGAAAACCAAAAAAGATAGGAA
                    ************************************************************
 XM_087662      241 AGAAGAATTAAGAATTCTTGCAGGGAAACAACAAAAAGGGAAAACCAAAAAAGATAGGAA

ss.DNA64966     309 ATATAATGGTCACATTGAAAGTAAGCCATTAACCATTCCAAAGGATATTGACCTTCATCT
                    ************************************************************
 XM_087662      301 ATATAATGGTCACATTGAAAGTAAGCCATTAACCATTCCAAAGGATATTGACCTTCATCT

ss.DNA64966     369 AGAAACAAAGTCAGTTACAGAAGTGGATACTTTAGCATTGCATTACTTTCCAGAATACCA
                    ************************************************************
 XM_087662      361 AGAAACAAAGTCAGTTACAGAAGTGGATACTTTAGCATTGCATTACTTTCCAGAATACCA

ss.DNA64966     429 GTGGCTGGTGGATTTCACAGTGGCTGCTACAGTTGTGTATCTAGTAACTGAAGTCTACTA
                    ************************************************************
 XM_087662      421 GTGGCTGGTGGATTTCACAGTGGCTGCTACAGTTGTGTATCTAGTAACTGAAGTCTACTA

ss.DNA64966     489 CAATTTTATGAAGCCTACACAGGAAATGAATATCAGCTTAGTCTGGTGCCTACTTGTTTT
```

```
                                       ************************************************************
XM_087662             481  CAATTTTATGAAGCCTACACAGGAAATGAATATCAGCTTAGTCTGGTGCCTACTTGTTTT

ss.DNA64966           549  GTCTTTTGCAATCAAAGTTCTATTTTCATTAACTACACACTATTTTAAAGTAGAAGATGG
                           ************************************************************
XM_087662             541  GTCTTTTGCAATCAAAGTTCTATTTTCATTAACTACACACTATTTTAAAGTAGAAGATGG

ss.DNA64966           609  TGGTGAAAGATCTGTTTGTGTCACCTTTGGATTTTTTTTCTTTGTCAAAGCAATGGCAGT
                           ************************************************************
XM_087662             601  TGGTGAAAGATCTGTTTGTGTCACCTTTGGATTTTTTTTCTTTGTCAAAGCAATGGCAGT

ss.DNA64966           669  GTTGATTGTAACAGAAAATTATCTGGAATTTGGACTTGAAACAGGGTTTACAAATTTTTC
                           ************************************************************
XM_087662             661  GTTGATTGTAACAGAAAATTATCTGGAATTTGGACTTGAAACAGGGTTTACAAATTTTTC

ss.DNA64966           729  AGACAGTGCGATGCAGTTTCTTGAAAAGCAAGGTTTAGAATCTCAGAGTCCTGTTTCAAA
                           ************************************************************
XM_087662             721  AGACAGTGCGATGCAGTTTCTTGAAAAGCAAGGTTTAGAATCTCAGAGTCCTGTTTCAAA

ss.DNA64966           789  ACTTACTTTCAAATTTTTCCTGGCTATTTTCTGTTCATTCATTGGGGCTTTTTTGACATT
                           ************************************************************
XM_087662             781  ACTTACTTTCAAATTTTTCCTGGCTATTTTCTGTTCATTCATTGGGGCTTTTTTGACATT

ss.DNA64966           849  TCCTGGATTACGACTGGCTCAAATGCATCTGGATGCCCTGAATTTGGCAACAGAAAAAAT
                           ************************************************************
XM_087662             841  TCCTGGATTACGACTGGCTCAAATGCATCTGGATGCCCTGAATTTGGCAACAGAAAAAAT

ss.DNA64966           909  TACACAAACTTTACTTCATATCAACTTCTTGGCACCTTTATTTATGGTTTTGCTCTGGGT
                           ************************************************* **********
XM_087662             901  TACACAAACTTTACTTCATATCAACTTCTTGGCACCTTTATTTATGGTTCTGCTCTGGGT

ss.DNA64966           969  AAAACCAATCACCAAAGACTACATTATGAACCCACCACTGGGCAAAGAAA
                           **************************************************
XM_087662             961  AAAACCAATCACCAAAGACTACATTATGAACCCACCACTGGGCAAAGAAA
```

```
Wed Oct  9 14:17:24 2002 [BLASTP 2.2.2 [Jan-08-2002], NCBI]
/home/ruby/va/Molbio/carpenda/tempids/p1.DNA64966 (307 aa)

Sequences producing High-scoring Segment Pairs:               Score Match Pct E-val
   1 P_ABG34044  Human Pro peptide #15 - Homo sapiens.         1595    307 100 e-176
   2 P_AAB50964  Human PRO1313 protein - Homo sapiens.         1595    307 100 e-176
   3 P_AAB47290  PRO1313 polypeptide - Homo sapiens.           1595    307 100 e-176
   4 P_AAB24431  Human PRO1313 protein sequence SEQ ID NO:2    1595    307 100 e-176
   5 P_ABP43502  Human secreted protein (SCEP) 26 - Homo sa    1568    302  99 e-173
   6 P_AAU69453  Human purified secretory polypeptide #22 -    1568    302  99 e-173
   7 AAH37287.1  Similar to hypothetical protein FLJ20422 -    1568    302  99 e-173
   8 NP_699185.1 hypothetical protein MGC33214 - Homo sapie    1568    302  99 e-173

>1 P_ABG34044 Human Pro peptide #15 - Homo sapiens. (307 aa) [1 seg]
 Score =  1595 (619 bits), Expect = e-176
 Identities = 307/307 (100%), Positives = 307/307 (100%), at 1,1-307,307

   DNA64966     1 MGVIGIQLVVTMVMASVMQKIIPHYSLARWLLCNGSLRWYQHPTEEELRILAGKQQKGKT
                  ************************************************************
 P_ABG34044     1 MGVIGIQLVVTMVMASVMQKIIPHYSLARWLLCNGSLRWYQHPTEEELRILAGKQQKGKT

   DNA64966    61 KKDRKYNGHIESKPLTIPKDIDLHLETKSVTEVDTLALHYFPEYQWLVDFTVAATVVYLV
                  ************************************************************
 P_ABG34044    61 KKDRKYNGHIESKPLTIPKDIDLHLETKSVTEVDTLALHYFPEYQWLVDFTVAATVVYLV

   DNA64966   121 TEVYYNFMKPTQEMNISLVWCLLVLSFAIKVLFSLTTHYFKVEDGGERSVCVTFGFFFFV
                  ************************************************************
 P_ABG34044   121 TEVYYNFMKPTQEMNISLVWCLLVLSFAIKVLFSLTTHYFKVEDGGERSVCVTFGFFFFV

   DNA64966   181 KAMAVLIVTENYLEFGLETGFTNFSDSAMQFLEKQGLESQSPVSKLTFKFFLAIFCSFIG
                  ************************************************************
 P_ABG34044   181 KAMAVLIVTENYLEFGLETGFTNFSDSAMQFLEKQGLESQSPVSKLTFKFFLAIFCSFIG

   DNA64966   241 AFLTFPGLRLAQMHLDALNLATEKITQTLLHINFLAPLFMVLLWVKPITKDYIMNPPLGK
                  ************************************************************
 P_ABG34044   241 AFLTFPGLRLAQMHLDALNLATEKITQTLLHINFLAPLFMVLLWVKPITKDYIMNPPLGK

   DNA64966   301 EISPSGR
                  *******
 P_ABG34044   301 EISPSGR

>2 P_AAB50964 Human PRO1313 protein - Homo sapiens. (307 aa) [1 seg]
 Score =  1595 (619 bits), Expect = e-176
 Identities = 307/307 (100%), Positives = 307/307 (100%), at 1,1-307,307

   DNA64966     1 MGVIGIQLVVTMVMASVMQKIIPHYSLARWLLCNGSLRWYQHPTEEELRILAGKQQKGKT
                  ************************************************************
 P_AAB50964     1 MGVIGIQLVVTMVMASVMQKIIPHYSLARWLLCNGSLRWYQHPTEEELRILAGKQQKGKT

   DNA64966    61 KKDRKYNGHIESKPLTIPKDIDLHLETKSVTEVDTLALHYFPEYQWLVDFTVAATVVYLV
                  ************************************************************
 P_AAB50964    61 KKDRKYNGHIESKPLTIPKDIDLHLETKSVTEVDTLALHYFPEYQWLVDFTVAATVVYLV

   DNA64966   121 TEVYYNFMKPTQEMNISLVWCLLVLSFAIKVLFSLTTHYFKVEDGGERSVCVTFGFFFFV
                  ************************************************************
```

```
P_AAB50964  121 TEVYYNFMKPTQEMNISLVWCLLVLSFAIKVLFSLTTHYFKVEDGGERSVCVTFGFFFFV

  DNA64966  181 KAMAVLIVTENYLEFGLETGFTNFSDSAMQFLEKQGLESQSPVSKLTFKFFLAIFCSFIG
                ************************************************************
P_AAB50964  181 KAMAVLIVTENYLEFGLETGFTNFSDSAMQFLEKQGLESQSPVSKLTFKFFLAIFCSFIG

  DNA64966  241 AFLTFPGLRLAQMHLDALNLATEKITQTLLHINFLAPLFMVLLWVKPITKDYIMNPPLGK
                ************************************************************
P_AAB50964  241 AFLTFPGLRLAQMHLDALNLATEKITQTLLHINFLAPLFMVLLWVKPITKDYIMNPPLGK

  DNA64966  301 EISPSGR
                *******
P_AAB50964  301 EISPSGR

>3 P_AAB47290 PRO1313 polypeptide - Homo sapiens. (307 aa) [1 seg]
 Score =  1595 (619 bits), Expect = e-176
 Identities = 307/307 (100%), Positives = 307/307 (100%), at 1,1-307,307

  DNA64966    1 MGVIGIQLVVTMVMASVMQKIIPHYSLARWLLCNGSLRWYQHPTEEELRILAGKQQKGKT
                ************************************************************
P_AAB47290    1 MGVIGIQLVVTMVMASVMQKIIPHYSLARWLLCNGSLRWYQHPTEEELRILAGKQQKGKT

  DNA64966   61 KKDRKYNGHIESKPLTIPKDIDLHLETKSVTEVDTLALHYFPEYQWLVDFTVAATVVYLV
                ************************************************************
P_AAB47290   61 KKDRKYNGHIESKPLTIPKDIDLHLETKSVTEVDTLALHYFPEYQWLVDFTVAATVVYLV

  DNA64966  121 TEVYYNFMKPTQEMNISLVWCLLVLSFAIKVLFSLTTHYFKVEDGGERSVCVTFGFFFFV
                ************************************************************
P_AAB47290  121 TEVYYNFMKPTQEMNISLVWCLLVLSFAIKVLFSLTTHYFKVEDGGERSVCVTFGFFFFV

  DNA64966  181 KAMAVLIVTENYLEFGLETGFTNFSDSAMQFLEKQGLESQSPVSKLTFKFFLAIFCSFIG
                ************************************************************
P_AAB47290  181 KAMAVLIVTENYLEFGLETGFTNFSDSAMQFLEKQGLESQSPVSKLTFKFFLAIFCSFIG

  DNA64966  241 AFLTFPGLRLAQMHLDALNLATEKITQTLLHINFLAPLFMVLLWVKPITKDYIMNPPLGK
                ************************************************************
P_AAB47290  241 AFLTFPGLRLAQMHLDALNLATEKITQTLLHINFLAPLFMVLLWVKPITKDYIMNPPLGK

  DNA64966  301 EISPSGR
                *******
P_AAB47290  301 EISPSGR

>4 P_AAB24431 Human PRO1313 protein sequence SEQ ID NO:216 - Homo sapiens. (307
aa) [1 seg]
 Score =  1595 (619 bits), Expect = e-176
 Identities = 307/307 (100%), Positives = 307/307 (100%), at 1,1-307,307

  DNA64966    1 MGVIGIQLVVTMVMASVMQKIIPHYSLARWLLCNGSLRWYQHPTEEELRILAGKQQKGKT
                ************************************************************
P_AAB24431    1 MGVIGIQLVVTMVMASVMQKIIPHYSLARWLLCNGSLRWYQHPTEEELRILAGKQQKGKT

  DNA64966   61 KKDRKYNGHIESKPLTIPKDIDLHLETKSVTEVDTLALHYFPEYQWLVDFTVAATVVYLV
                ************************************************************
P_AAB24431   61 KKDRKYNGHIESKPLTIPKDIDLHLETKSVTEVDTLALHYFPEYQWLVDFTVAATVVYLV

  DNA64966  121 TEVYYNFMKPTQEMNISLVWCLLVLSFAIKVLFSLTTHYFKVEDGGERSVCVTFGFFFFV
                ************************************************************
```

```
P_AAB24431   121 TEVYYNFMKPTQEMNISLVWCLLVLSFAIKVLFSLTTHYFKVEDGGERSVCVTFGFFFFV

  DNA64966   181 KAMAVLIVTENYLEFGLETGFTNFSDSAMQFLEKQGLESQSPVSKLTFKFFLAIFCSFIG
                 ************************************************************
P_AAB24431   181 KAMAVLIVTENYLEFGLETGFTNFSDSAMQFLEKQGLESQSPVSKLTFKFFLAIFCSFIG

  DNA64966   241 AFLTFPGLRLAQMHLDALNLATEKITQTLLHINFLAPLFMVLLWVKPITKDYIMNPPLGK
                 ************************************************************
P_AAB24431   241 AFLTFPGLRLAQMHLDALNLATEKITQTLLHINFLAPLFMVLLWVKPITKDYIMNPPLGK

  DNA64966   301 EISPSGR
                 *******
P_AAB24431   301 EISPSGR
```

```
>5 P_ABP43502 Human secreted protein (SCEP) 26 - Homo sapiens. (487 aa) [1 seg]
 Score =  1568 (608 bits), Expect = e-173
 Identities = 302/304 (99%), Positives = 302/304 (99%), at 1,1-304,304

  DNA64966     1 MGVIGIQLVVTMVMASVMQKIIPHYSLARWLLCNGSLRWYQHPTEEELRILAGKQQKGKT
                 ************************************************************
P_ABP43502     1 MGVIGIQLVVTMVMASVMQKIIPHYSLARWLLCNGSLRWYQHPTEEELRILAGKQQKGKT

  DNA64966    61 KKDRKYNGHIESKPLTIPKDIDLHLETKSVTEVDTLALHYFPEYQWLVDFTVAATVVYLV
                 ************************************************************
P_ABP43502    61 KKDRKYNGHIESKPLTIPKDIDLHLETKSVTEVDTLALHYFPEYQWLVDFTVAATVVYLV

  DNA64966   121 TEVYYNFMKPTQEMNISLVWCLLVLSFAIKVLFSLTTHYFKVEDGGERSVCVTFGFFFFV
                 ************************************************************
P_ABP43502   121 TEVYYNFMKPTQEMNISLVWCLLVLSFAIKVLFSLTTHYFKVEDGGERSVCVTFGFFFFV

  DNA64966   181 KAMAVLIVTENYLEFGLETGFTNFSDSAMQFLEKQGLESQSPVSKLTFKFFLAIFCSFIG
                 ************************************************************
P_ABP43502   181 KAMAVLIVTENYLEFGLETGFTNFSDSAMQFLEKQGLESQSPVSKLTFKFFLAIFCSFIG

  DNA64966   241 AFLTFPGLRLAQMHLDALNLATEKITQTLLHINFLAPLFMVLLWVKPITKDYIMNPPLGK
                 ************************************************************
P_ABP43502   241 AFLTFPGLRLAQMHLDALNLATEKITQTLLHINFLAPLFMVLLWVKPITKDYIMNPPLGK

  DNA64966   301 EISP
                 *  *
P_ABP43502   301 ESIP
```

```
>6 P_AAU69453 Human purified secretory polypeptide #22 - Homo sapiens. (419 aa)
[1 seg]
 Score =  1568 (608 bits), Expect = e-173
 Identities = 302/304 (99%), Positives = 302/304 (99%), at 1,21-304,324

  DNA64966     1 MGVIGIQLVVTMVMASVMQKIIPHYSLARWLLCNGSLRWYQHPTEEELRILAGKQQKGKT
                 ************************************************************
P_AAU69453    21 MGVIGIQLVVTMVMASVMQKIIPHYSLARWLLCNGSLRWYQHPTEEELRILAGKQQKGKT

  DNA64966    61 KKDRKYNGHIESKPLTIPKDIDLHLETKSVTEVDTLALHYFPEYQWLVDFTVAATVVYLV
                 ************************************************************
P_AAU69453    81 KKDRKYNGHIESKPLTIPKDIDLHLETKSVTEVDTLALHYFPEYQWLVDFTVAATVVYLV

  DNA64966   121 TEVYYNFMKPTQEMNISLVWCLLVLSFAIKVLFSLTTHYFKVEDGGERSVCVTFGFFFFV
                 ************************************************************
```

```
P_AAU69453  141 TEVYYNFMKPTQEMNISLVWCLLVLSFAIKVLFSLTTHYFKVEDGGERSVCVTFGFFFFV

  DNA64966  181 KAMAVLIVTENYLEFGLETGFTNFSDSAMQFLEKQGLESQSPVSKLTFKFFLAIFCSFIG
                ************************************************************
P_AAU69453  201 KAMAVLIVTENYLEFGLETGFTNFSDSAMQFLEKQGLESQSPVSKLTFKFFLAIFCSFIG

  DNA64966  241 AFLTFPGLRLAQMHLDALNLATEKITQTLLHINFLAPLFMVLLWVKPITKDYIMNPPLGK
                ************************************************************
P_AAU69453  261 AFLTFPGLRLAQMHLDALNLATEKITQTLLHINFLAPLFMVLLWVKPITKDYIMNPPLGK

  DNA64966  301 EISP
                *  *
P_AAU69453  321 ESIP
```

```
>7 AAH37287.1 Similar to hypothetical protein FLJ20422 - Homo sapiens (487 aa)
[1 seg]
 Score =  1568 (608 bits), Expect = e-173
 Identities = 302/304 (99%), Positives = 302/304 (99%), at 1,1-304,304

  DNA64966    1 MGVIGIQLVVTMVMASVMQKIIPHYSLARWLLCNGSLRWYQHPTEEELRILAGKQQKGKT
                ************************************************************
AAH37287.1    1 MGVIGIQLVVTMVMASVMQKIIPHYSLARWLLCNGSLRWYQHPTEEELRILAGKQQKGKT

  DNA64966   61 KKDRKYNGHIESKPLTIPKDIDLHLETKSVTEVDTLALHYFPEYQWLVDFTVAATVVYLV
                ************************************************************
AAH37287.1   61 KKDRKYNGHIESKPLTIPKDIDLHLETKSVTEVDTLALHYFPEYQWLVDFTVAATVVYLV

  DNA64966  121 TEVYYNFMKPTQEMNISLVWCLLVLSFAIKVLFSLTTHYFKVEDGGERSVCVTFGFFFFV
                ************************************************************
AAH37287.1  121 TEVYYNFMKPTQEMNISLVWCLLVLSFAIKVLFSLTTHYFKVEDGGERSVCVTFGFFFFV

  DNA64966  181 KAMAVLIVTENYLEFGLETGFTNFSDSAMQFLEKQGLESQSPVSKLTFKFFLAIFCSFIG
                ************************************************************
AAH37287.1  181 KAMAVLIVTENYLEFGLETGFTNFSDSAMQFLEKQGLESQSPVSKLTFKFFLAIFCSFIG

  DNA64966  241 AFLTFPGLRLAQMHLDALNLATEKITQTLLHINFLAPLFMVLLWVKPITKDYIMNPPLGK
                ************************************************************
AAH37287.1  241 AFLTFPGLRLAQMHLDALNLATEKITQTLLHINFLAPLFMVLLWVKPITKDYIMNPPLGK

  DNA64966  301 EISP
                *  *
AAH37287.1  301 ESIP
```

```
>8 NP_699185.1 hypothetical protein MGC33214 - Homo sapiens (487 aa) [1 seg]
 Score =  1568 (608 bits), Expect = e-173
 Identities = 302/304 (99%), Positives = 302/304 (99%), at 1,1-304,304

  DNA64966    1 MGVIGIQLVVTMVMASVMQKIIPHYSLARWLLCNGSLRWYQHPTEEELRILAGKQQKGKT
                ************************************************************
NP_699185.1   1 MGVIGIQLVVTMVMASVMQKIIPHYSLARWLLCNGSLRWYQHPTEEELRILAGKQQKGKT

  DNA64966   61 KKDRKYNGHIESKPLTIPKDIDLHLETKSVTEVDTLALHYFPEYQWLVDFTVAATVVYLV
                ************************************************************
NP_699185.1  61 KKDRKYNGHIESKPLTIPKDIDLHLETKSVTEVDTLALHYFPEYQWLVDFTVAATVVYLV

  DNA64966  121 TEVYYNFMKPTQEMNISLVWCLLVLSFAIKVLFSLTTHYFKVEDGGERSVCVTFGFFFFV
                ************************************************************
```

```
NP_699185.1   121 TEVYYNFMKPTQEMNISLVWCLLVLSFAIKVLFSLTTHYFKVEDGGERSVCVTFGFFFFV

   DNA64966   181 KAMAVLIVTENYLEFGLETGFTNFSDSAMQFLEKQGLESQSPVSKLTFKFFLAIFCSFIG
                  ************************************************************
NP_699185.1   181 KAMAVLIVTENYLEFGLETGFTNFSDSAMQFLEKQGLESQSPVSKLTFKFFLAIFCSFIG

   DNA64966   241 AFLTFPGLRLAQMHLDALNLATEKITQTLLHINFLAPLFMVLLWVKPITKDYIMNPPLGK
                  ************************************************************
NP_699185.1   241 AFLTFPGLRLAQMHLDALNLATEKITQTLLHINFLAPLFMVLLWVKPITKDYIMNPPLGK

   DNA64966   301 EISP
                  *  *
NP_699185.1   301 ESIP
```

## Claims

1. A composition comprising a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide or agonist or antagonist thereof, in admixture with a pharmaceutically acceptable carrier.

2. The composition of Claim I comprising a therapeutically effective amount of said polypeptide or said agonist or antagonist thereof.

3. The composition of Claim 1, wherein

(i) the agonist is an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, anti-PRO812, anti-PRO865, anti-PRO1075, anti-PRO1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PRO216 antibody; or
(ii) the antagonist is an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, anti-PRO812, anti-PRO865, anti-PRO1075, anti-PRO1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PRO216 antibody.

4. The composition of Claim 1 further comprising a cardiovascular, endothelial, angiogenic or angiostatic agent.

5. A method of preparing the composition of Claim I comprising admixing a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide or agonist or antagonist thereof, with a pharmaceutically acceptable carrier.

6. An article of manufacture comprising:

(1) composition comprising (a) a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide, (b) an agonist of a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide, or (c) an antagonist of a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526 PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide, in admixture with a pharmaceutically acceptable carrier;
(2) a container containing said composition; and
(3) a label affixed to said container, or a package insert included in said container referring to the use of said composition in the treatment of a cardiovascular, endothelial, and angiogenic disorder.

**7.** The article of manufacture of Claim 6, wherein:

(i) said agonist is an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, anti-PRO812, anti-PRO865, anti-PRO1075, anti-PRO 1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PRO216 antibody; or
- (ii) said antagonist is an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, anti-PRO812, anti-PRO865, anti-PRO1075, anti-PRO1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PRO216 antibody.

**8.** The article of manufacture of Claim 6, wherein said composition comprises a therapeutically effective amount of said polypeptide or agonist or antagonist thereof, in admixture with said pharmaceutically acceptable carrier.

**9.** A method for identifying an agonist of a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide comprising:

(a) contacting cells and a test compound to be screened under conditions suitable for the induction of a cellular response normally induced by a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide; and
(b) determining the induction of said cellular response to determine if the test compound is an effective agonist, wherein the induction of said cellular response is indicative of said test compound being an effective agonist.

**10.** The method of Claim 9, wherein the cellular response normally induced by said PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PR0331, PRO356, PRO364, PRO526, PRO538 PRO713, PRO719, PR0771, PRO788, PRO792, PR0812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide is stimulation of cell proliferation.

**11.** A method for identifying a compound that inhibits an activity of a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538 PRO713, PRO719, PRO771, PRO788, PRO792, PRO812, PRO865, PRO1075, PRO1126, PRO1130, PRO1154, PRO1244, PRO1246, PRO1274, PRO1286 PRO1294, PRO1304, PRO1312, PRO1313, PRO1376, PRO1387, PRO1561 or PRO216 polypeptide comprising:

(i) contacting a test compound with said polypeptide under conditions and for a time sufficient to allow the test compound and polypeptide to interact and determining whether the activity of said polypeptide is inhibited; or
(ii) the steps of:

(a) contacting cells and a test compound to be screened in the presence of said polypeptide under conditions suitable for the induction of a cellular response normally induced by said polypeptide; and
(b) determining the induction of said cellular response to determine if the test compound is an effective antagonist.

**12.** The method of Claim 11, wherein the cellular response normally induced by said polypeptide is stimulation of cell proliferation.

**13.** A method for identifying a compound that inhibits the expression of a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide in cells that normally expresses the polypeptide, wherein the method comprises contacting the cells with a test compound under conditions suitable for allowing expression of said polypeptide and determining whether the expression of said polypeptide is inhibited.

**14.** An agonist of a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PR0216 polypeptide; or
an antagonist of a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide.

**15.** A compound that inhibits the expression of a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide in a mammalian cell which expresses said polypeptide.

**16.** The compound of Claim 15, wherein said compound is an antisense oligonucleotide.

**17.** An isolated antibody that binds to a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526 PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide.

**18.** The antibody of Claim 17 which is a monoclonal antibody, an antibody fragment, or a single-chain antibody.

**19.** A method for diagnosing:

(i) a disease or susceptibility to a disease which is related to a mutation in a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide-encoding nucleic acid sequence comprising determining the presence or absence of said mutation in said polypeptide-encoding nucleic acid sequence, wherein the presence or absence of said mutation is indicative of the presence of said disease or susceptibility to said disease; or

(ii) a cardiovascular, endothelial or angiogenic disorder in a mammal which comprises analyzing the level of expression of a gene encoding a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide (a) in a test sample of tissue cells obtained from said mammal, and (b) in a control sample of known normal tissue cells of the same cell type, wherein a higher or lower expression level in the test sample as compared to the control sample is indicative of the presence of a cardiovascular, endothelial or angiogenic disorder in said mammal; or

(iii) a cardiovascular, endothelial or angiogenic disorder in a mammal which comprises detecting the presence or absence of a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide in a test sample of tissue cells obtained from said mammal, wherein the presence or absence of said polypeptide in said test sample is indicative of the presence of a cardiovascular, endothelial or angiogenic disorder in said mammal; or

(iv) a cardiovascular, endothelial or angiogenic disorder in a mammal comprising (a) contacting an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, anti-PRO812, anti-PRO865, anti-PRO1075, anti-PRO1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PRO216 antibody with a test sample of tissue cells obtained from the mammal, and (b) detecting the formation of a complex between said antibody and a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PR0356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide in the test sample, wherein the formation of said complex is indicative of the presence of a cardiovascular, endothelial or angiogenic disorder in the mammal.

**20.** A method for determining the presence of a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide in a sample comprising contacting a sample suspected of containing said polypeptide with an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, anti-PRO812, anti-PRO865, anti-PRO1075, anti-PRO1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PRO216 antibody and determining binding of said antibody to a component of said sample.

**21.** A cardiovascular, endothelial or angiogenic disorder diagnostic kit comprising an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, anti-PRO812, anti-PRO865, anti-PRO1075, anti-PRO1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PRO216 antibody and a carrier in suitable packaging.

**22.** A method for treating a cardiovascular, endothelial or angiogenic disorder in a mammal comprising administering to the mammal a therapeutically effective amount of a PRO1303, PRO172, PRO178, PRO179, PRO182, PRO187, PRO188, PRO195, PR0212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide or an agonist or antagonist thereof.

**23.** The method according to Claim 22, wherein the mammal is human.

**24.** The method of Claim 23, wherein:

(i) the human has suffered myocardial infarction; or
(ii) the human has cardiac hypertrophy, trauma, a cancer, or age-related macular degeneration.

**25.** The method of Claim 24, wherein the cardiac hypertrophy is **characterized by** the presence of an elevated level of $PGF_{2\alpha}$.

**26.** The method of Claim 22, wherein the PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide is administered together with a cardiovascular, endothelial or angiogenic agent.

**27.** The method of Claim 24, part (ii), wherein the PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538 PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide is administered following primary angioplasty.

**28.** The method of Claim 22, wherein the cardiovascular, endothelial or angiogenic disorder is cancer.

**29.** The method of Claim 28, wherein the PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide is administered in combination with a chemotherapeutic agent, a growth inhibitory agent or a cytotoxic agent.

**30.** The method of Claim 22 wherein:

(i) said agonist is an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, anti-PRO812, anti-PRO865, anti-PRO1075, anti-PRO1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PRO216 antibody; or
(ii) said antagonist is an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, anti-PRO812, anti-PRO865, anti-PRO1075, anti-PRO1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PRO216 antibody.

**31.** A method for treating a cardiovascular, endothelial or angiogenic disorder in a mammal comprising administering to the mammal a nucleic acid molecule that encodes a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide or agonist or antagonist thereof

**32.** The method of Claim 31 wherein:

(i) said agonist is an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, anti-PRO812, anti-PRO865, anti-PRO1075, anti-PRO1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PR0216 antibody; or
(ii) said antagonist is an anti-PRO1303, anti-PRO172, anti-PRO178, anti-PRO179, anti-PRO182, anti-PRO187, anti-PRO188, anti-PRO195, anti-PRO212, anti-PRO214, anti-PRO217, anti-PRO224, anti-PRO231, anti-PRO235, anti-PRO245, anti-PRO261, anti-PRO269, anti-PRO287, anti-PRO301, anti-PRO323, anti-PRO331, anti-PRO356, anti-PRO364, anti-PRO526, anti-PRO538, anti-PRO713, anti-PRO719, anti-PRO771, anti-PRO788, anti-PRO792, atiti-PRO812, anti-PRO865, anti-PRO1075, anti-PRO1126, anti-PRO1130, anti-PRO1154, anti-PRO1244, anti-PRO1246, anti-PRO1274, anti-PRO1286, anti-PRO1294, anti-PRO1304, anti-PRO1312, anti-PRO1313, anti-PRO1376, anti-PRO1387, anti-PRO1561 or anti-PRO216 antibody.

**33.** The method of Claim 31, wherein the mammal is human.

**34.** The method of Claim 31, wherein the nucleic acid molecule is administered via *ex vivo* gene therapy.

**35.** A recombinant retroviral particle comprising a retroviral vector consisting essentially of (1) a promoter, (2) nucleic acid encoding a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide or agonist or antagonist thereof, and (3) a signal sequence for cellular secretion of the polypeptide, wherein the retroviral vector is in association with retroviral structural proteins.

**36.** An *ex vivo* producer cell comprising a nucleic acid construct that expresses retroviral structural proteins and also comprises a retroviral vector consisting essentially of (1) a promoter, (2) nucleic acid encoding a PRO1303, PRO172, PRO178, PRO179, PRO182, PRO187, PRO188, PRO195, PRO212, PRO214, PRO217, PRO224, PRO231, PRO235, PRO245, PRO261, PRO269, PRO287, PRO301, PRO323, PRO331, PRO356, PRO364, PRO526, PRO538, PRO713, PRO719, PRO771, PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561 or PRO216 polypeptide or agonist or antagonist thereof, and (3) a signal sequence for cellular secretion of the polypeptide, wherein said producer cell packages the retroviral vector in association with the structural proteins to produce recombinant retroviral particles.

**37.** A method for:

(i) inhibiting endothelial cell growth in a mammal comprising administering to the mammal (a) a PRO172, PRO178, PRO179, PRO187, PRO188, PRO214, PRO216, PRO217, PRO235, PRO261, PRO287, PRO301, PRO323, PRO331, PRO364, PRO538, PRO713, PRO719, PRO788, PRO812, PRO865, PRO1126, PRO1130, PRO1246, PRO1274, PRO1294, PRO1304, PRO1376 or PRO1387 polypeptide or agonist thereof, wherein endothelial cell growth in said mammal is inhibited; or
(ii) stimulating endothelial cell growth in a mammal comprising administering to the mammal a PRO1303, PRO179, PRO212, PRO245, PRO771, PRO1075, PRO1154, PRO1244, PRO1286, PRO1313, PRO1376 or PRO1561 polypeptide or agonist thereof, wherein endothelial cell growth in said mammal is stimulated; or
(iii) inhibiting endothelial cell growth in a mammal comprising administering to the mammal an antagonist of a PRO1303, PRO179, PRO212, PRO245, PRO771, PRO1075, PRO1154, PRO1244, PRO1286, PRO1313, PRO 1376 or PRO 1561 polypeptide, wherein endothelial cell growth in said mammal is inhibited; or
(iv) stimulating endothelial cell growth in a mammal comprising administering to the mammal an antagonist of a PRO172, PRO178, PRO179, PRO187, PRO188, PRO214, PRO216, PRO217, PRO235, PRO261, PRO287, PRO301, PRO323, PRO331, PRO364, PRO538, PRO713, PRO719, PRO788, PRO812, PRO865, PRO1126, PRO1130, PRO1246, PRO1274, PRO1294, PRO1304, PRO1376 or PRO1387 polypeptide,

wherein endothelial cell growth in said mammal is stimulated.

**38.** A method for:

(i) reducing cardiac hypertrophy in a mammal comprising administering to the mammal a PRO269 or PRO356 polypeptide or agonist thereof, wherein cardiac hypertrophy in said mammal is reduced; or
(ii) inducing cardiac hypertrophy in a mammal comprising administering to the mammal a PRO179, PRO182, PRO195, PRO224, PRO231, PRO526, PRO713, PRO792, PRO1246 or PRO1312 polypeptide or agonist thereof, wherein said cardiac hypertrophy in said mammal is induced; or
(iii) reducing cardiac hypertrophy in a mammal comprising administering to the mammal an antagonist of a PRO179, PRO182, PRO195, PRO224, PRO231, PRO526, PRO713, PRO792, PRO1246 or PRO1312 polypeptide, wherein cardiac hypertrophy in said mammal is reduced; or
(iv) inducing cardiac hypertrophy in a mammal comprising administering to the mammal an antagonist of a PRO269 or PRO356 polypeptide, wherein cardiac hypertrophy in said mammal is induced.

**39.** The method of Claim 38, part (i), wherein the cardiac hypertrophy has been induced by myocardial infarction.

**40.** A method for:

(i) inhibiting angiogenesis induced by a PRO1303, PRO179, PRO212, PRO245, PRO771, PRO1075, PRO1154, PRO1244, PRO1286, PRO1313, PRO1376 or PRO1561 polypeptide in a mammal comprising administering a therapeutically effective amount of an anti-PRO1303, anti-PRO179, anti-PRO212, anti-PRO245, anti-PRO771, anti-PRO1075, anti-PRO1154, anti-PRO1244, anti-PRO1286, anti-PRO1313, anti-PRO1376 or anti-PRO1561 antibody to the mammal, wherein said angiogenesis is inhibited; or
(ii)stimulating angiogenesis induced by a PRO1303, PRO179, PRO212, PRO245, PRO771, PRO1075, PRO1154, PRO1244, PRO1286, PRO1313, PRO1376 or PRO1561polypeptide in a mammal comprising administering a therapeutically effective amount of said polypeptide to the mammal, whereby said angiogenesis is stimulated.

**41.** Isolated nucleic acid having at least 80% nucleic acid sequence identity to:

(i) a nucleotide sequence that encodes an amino acid sequence selected from the group consisting of the amino acid sequence shown in Figure 82 (SEQ IDNO:203), Figure 2 (SEQ ID NO:4), Figure 4 (SEQ ID NO:9), Figure 6 (SEQ ID NO:14), Figure 8 (SEQ ID NO:16), Figure 10 (SEQ ID NO:21), Figure 12 (SEQ ID NO:26), Figure 14 (SEQ ID N0:31), Figure 16 (SEQ ID NO:36), Figure 18 (SEQ ID NO:41), Figure 20 (SEQ ID NO:46), Figure 22 (SEQ ID NO:51), Figure 24 (SEQ ID NO:56), Figure 26 (SEQ ID NO:62), Figure 28 (SEQ ID NO:67), Figure 30 (SEQ ID NO:72), Figure 32 (SEQ ID NO:77), Figure 34 (SEQ ID NO:85), Figure 36 (SEQ ID NO:90), Figure 38 (SEQ ID NO:98), Figure 40 (SEQ ID NO:107), Figure 42 (SEQ ID NO:112), Figure 44 (SEQ ID NO:117), Figure 46 (SEQ ID NO:127), Figure 48 (SEQ ID NO:132), Figure 50 (SEQ ID NO:137), Figure 52 (SEQ ID NO: 143), Figure 54 (SEQ ID NO:148), Figure 56 (SEQ ID NO:153), Figure 58 (SEQ ID NO:155), Figure 60 (SEQ ID NO:160), Figure 62 (SEQ ID NO:162), Figure 64 (SEQ ID NO:170), Figure 66 (SEQ ID NO:181), Figure 68 (SEQ ID NO:183), Figure 70 (SEQ ID NO:191), Figure 72 (SEQ ID NO:193), Figure 74 (SEQ ID NO:195), Figure 76 (SEQ ID NO:197), Figure 78 (SEQ ID NO:199), Figure 80 (SEQ IDNO:201), Figure 84 (SEQ ID NO:205), Figure 86 (SEQ ID NO:214), Figure 88 (SEQ ID NO:216), Figure 90 (SEQ ID NO:218), Figure 92 (SEQ ID NO:

220), Figure 94 (SEQ ID NO:222), and Figure 96 (SEQ ID NO:227); or

(ii) a nucleotide sequence selected from the group consisting of the nucleotide sequence shown in Figure 81 (SEQ ID NO:202), Figure 1 (SEQ ID NO:3), Figure 3 (SEQ ID NO:8), Figure 5 (SEQ ID NO:13), Figure 7 (SEQ ID NO:15), Figure 9 (SEQ ID NO:20), Figure 11 (SEQ ID NO:25), Figure 13 (SEQ ID NO:30), Figure 15 (SEQ ID NO:35), Figure 17 (SEQ ID NO:40), Figure 19 (SEQ ID NO:45), Figure 21 (SEQ ID NO:50), Figure 23 (SEQ ID NO:55), Figure 25 (SEQ ID NO:61), Figure 27 (SEQ ID NO:66), Figure 29 (SEQ ID NO:71), Figure 31 (SEQ ID NO:76), Figure 33 (SEQ ID NO:84), Figure 35 (SEQ ID NO:89), Figure 37 (SEQ ID NO:97), Figure 39 (SEQ ID NO:106), Figure 41 (SEQ ID NO:111), Figure 43 (SEQ ID NO:116), Figure 45 (SEQ ID NO:126), Figure 47 (SEQ ID NO:131), Figure 49 (SEQ ID NO:136), Figure 51 (SEQ ID NO:142), Figure 53 (SEQ ID NO:147), Figure 55 (SEQ ID NO:152), Figure 57 (SEQ ID NO:154), Figure 59 (SEQ ID NO:159), Figure 61 (SEQ ID NO:161), Figure 63 (SEQ ID NO:169), Figure 65 (SEQ ID NO:180), Figure 67 (SEQ ID NO:182), Figure 69 (SEQ ID NO: 190), Figure 71 (SEQ ID NO:192), Figure 73 (SEQ ID NO:194), Figure 75 (SEQ ID NO:196), Figure 77 (SEQ ID NO:198), Figure 79 (SEQ ID NO:200), Figure 83 (SEQ ID NO:204), Figure 85 (SEQ ID NO:213), Figure 87 (SEQ ID NO:215), Figure 89 (SEQ ID NO:217), Figure 91 (SEQ ID NO:219), Figure 93 (SEQ ID NO:221), and Figure 95 (SEQ ID NO:226); or

(iii) a nucleotide sequence selected from the group consisting of the full-length coding sequence of the nucleotide sequence shown in Figure 81 (SEQ ID NO:202), Figure 1 (SEQ ID NO:3), Figure 3 (SEQ ID NO:8), Figure 5 (SEQ ID NO:13), Figure 7 (SEQ ID NO:15), Figure 9 (SEQ ID NO:20), Figure 11 (SEQ ID NO:25), Figure 13 (SEQ ID NO:30), Figure 15 (SEQ ID NO:35), Figure 17 (SEQ ID NO:40), Figure 19 (SEQ ID NO:45), Figure 21 (SEQ ID NO:50), Figure 23 (SEQ ID NO:55), Figure 25 (SEQ ID NO:61), Figure 27 (SEQ ID NO:66), Figure 29 (SEQ ID NO:71), Figure 31 (SEQ ID NO:76), Figure 33 (SEQ ID NO:84), Figure 35 (SEQ ID NO:89), Figure 37 (SEQ ID NO:97), Figure 39 (SEQ ID NO:106), Figure 41 (SEQ ID NO:111), Figure 43 (SEQ ID NO:116), Figure 45 (SEQ ID NO:126), Figure 47 (SEQ ID NO:131), Figure 49 (SEQ ID NO:136), Figure 51 (SEQ ID NO:142), Figure 53 (SEQ ID NO:147), Figure 55 (SEQ ID NO:152), Figure 57 (SEQ ID NO:154), Figure 59 (SEQ ID NO: 159), Figure 61 (SEQ ID NO:161), Figure 63 (SEQ ID NO:169), Figure 65 (SEQ ID NO:180), Figure 67 (SEQ ID NO:182), Figure 69 (SEQ ID NO:190), Figure 71 (SEQ ID NO:192), Figure 73 (SEQ ID NO:194), Figure 75 (SEQ ID NO:196), Figure 77 (SEQ ID NO:198), Figure 79 (SEQ ID NO:200), Figure 83 (SEQ ID NO:204), Figure 85 (SEQ ID NO:213), Figure 87 (SEQ ID NO:215), Figure 89 (SEQ ID NO:217), Figure 91 (SEQ ID NO:219), Figure 93 (SEQ ID NO:221), AND Figure 95 (SEQ ID NO:126); or

(iv) the full-length coding sequence of the DNA deposited under ATCC accession number 203232, 209419, 209282, 209776, 209296, 209375, 209279, 209772, 209254, 209385, 209256, 209263, 209252, 209374, 209265, 209391, 209397, 209400, 209432, 209528, 209439, 209422, 209436, 209704, 209752, 209653, 209705, 209749, 209849, 209846, 203009, 209774, 209869, 209980, 203359, 209978, 203253, 203457, 203250, 203223, 203233, 203219, 203132, 203575, 203163, 203160, 203313, or 209381.

**42.** A vector comprising the nucleic acid of Claim 41.

**43.** The vector of Claim 42 operably linked to control sequences recognized by a host cell transformed with the vector.

**44.** A host cell comprising the vector of Claim 43.

**45.** The host cell of Claim 66, wherein said cell is a CHO cell, an *E. coli,* a yeast cell, or a Baculovirus-infected insect cell.

**46.** A process for producing a PRO1303, PRO172, PRO178, PRO179, PRO182, PRO187, PRO188, PRO195, PRO212, PRO214, PRO217, PRO224, PRO231 PRO235, PRO245, PRO261 PRO269, PRO287, PRO301, PRO323, PRO331, PRO356, PRO364, PRO526, PRO538, PRO713, PRO719, PRO771 PRO788, PRO792, PRO812, PRO865, PRO1075, PRO1126, PRO1130, PRO1154, PRO1244, PRO1246, PRO1274, PRO1286, PRO1294, PRO1304, PRO1312, PRO1313, PRO1376, PRO1387, PRO1561, or PRO216 polypeptide comprising culturing the host cell of Claim 66 under conditions suitable for expression of said polypeptide and recovering said polypeptide from the cell culture.

**47.** An isolated polypeptide:

(i) having at least 80% amino acid sequence identity to an amino acid sequence selected from the group consisting of the amino acid sequence shown in Figure 82 (SEQ IDNO:203), Figure 2 (SEQ ID NO:4), Figure 4 (SEQ ID NO:9), Figure 6 (SEQ ID NO:14), Figure 8 (SEQ ID NO:16), Figure 10 (SEQ ID NO:21), Figure 12 (SEQ ID NO:26), Figure 14 (SEQ ID NO:31), Figure 16 (SEQ ID NO:36), Figure 18 (SEQ ID NO:41), Figure 20 (SEQ ID NO:46), Figure 22 (SEQ ID NO:51), Figure 24 (SEQ ID NO:56), Figure 26 (SEQ ID NO:62), Figure 28 (SEQ ID NO:67), Figure 30 (SEQ ID NO:72), Figure 32 (SEQ ID NO:77), Figure 34 (SEQ ID NO:85), Figure 36 (SEQ ID NO:90), Figure 38 (SEQ ID NO:98), Figure 40 (SEQ ID NO:107), Figure 42 (SEQ ID NO:112), Figure 44 (SEQ ID NO:117), Figure 46 (SEQ ID NO:127), Figure 48 (SEQ ID NO:132), Figure 50 (SEQ ID NO:137), Figure 52 (SEQ ID NO:143), Figure 54 (SEQ ID NO:148), Figure 56 (SEQ ID NO:153), Figure 58 (SEQ ID NO: 155), Figure 60 (SEQ ID NO:160), Figure 62 (SEQ ID NO:162), Figure 64 (SEQ ID NO:170), Figure 66 (SEQ ID NO:181), Figure 68 (SEQ ID NO:183), Figure 70 (SEQ ID NO:191), Figure 72 (SEQ ID NO:193), Figure 74 (SEQ ID NO:195), Figure 76 (SEQ ID NO:197), Figure 78 (SEQ ID NO:199), Figure 80 (SEQ IDNO:201), Figure 84 (SEQ ID NO:205), Figure 86 (SEQ ID NO:214), Figure 88 (SEQ ID NO:216), Figure 90 (SEQ ID NO:218), Figure 92 (SEQ ID NO:220), Figure 94 (SEQ ID NO:222) and Figure 96 (SEQ ID NO:227); or

(ii) scoring at least 80% positives when compared to an amino acid sequence selected from the group consisting of the amino acid sequence shown in Figure 82 (SEQ IDNO:203), Figure 2 (SEQ ID NO:4), Figure 4 (SEQ ID NO:9), Figure 6 (SEQ ID NO:14), Figure 8 (SEQ ID NO:16), Figure 10 (SEQ ID NO:21), Figure 12 (SEQ ID NO: 26), Figure 14 (SEQ ID NO:31), Figure 16 (SEQ ID NO:36), Figure 18 (SEQ ID NO:41), Figure 20 (SEQ ID NO: 46), Figure 22 (SEQ ID NO:51), Figure 24 (SEQ ID NO:56), Figure 26 (SEQ ID NO:62), Figure 28 (SEQ ID NO: 67), Figure 30 (SEQ ID NO:72), Figure 32 (SEQ ID NO:77), Figure 34 (SEQ ID NO:85), Figure 36 (SEQ ID NO: 90), Figure 38 (SEQ ID NO:98), Figure 40 (SEQ ID NO:107), Figure 42 (SEQ ID NO:112), Figure 44 (SEQ ID NO:117), Figure 46 (SEQ ID NO:127), Figure 48 (SEQ ID NO:132), Figure 50 (SEQ ID NO:137), Figure 52 (SEQ ID NO:143), Figure 54 (SEQ ID NO:148), Figure 56 (SEQ ID NO:153), Figure 58 (SEQ ID NO:155), Figure 60 (SEQ ID NO:160), Figure 62 (SEQ ID NO:162), Figure 64 (SEQ ID NO:170), Figure 66 (SEQ ID NO:181), Figure 68 (SEQ ID NO:183), Figure 70 (SEQ ID NO:191), Figure 72 (SEQ ID NO:193), Figure 74 (SEQ ID NO: 195), Figure 76 (SEQ ID NO:197), Figure 78 (SEQ ID NO:199), Figure 80 (SEQ IDNO:201), Figure 84 (SEQ ID NO:205), Figure 86 (SEQ ID NO:214), Figure 88 (SEQ ID NO:216), Figure 90 (SEQ ID NO:218), Figure 92 (SEQ ID NO:220), Figure 94 (SEQ ID NO:222), and Figure 96 (SEQ ID NO:227); or

(iii) having at least 80% amino acid sequence identity to an amino acid sequence encoded by the full-length coding sequence of the DNA deposited under ATCC accession number 203232, 209419, 209282, 209776, 209296, 209375, 209279, 209772, 209254, 209385, 209256, 209263, 209252, 209374, 209265, 209391, 209397, 209400, 209432, 209528, 209439, 209422, 209436, 209704, 209752, 209653, 209705, 209749, 209849, 209846, 203009, 209774, 209869, 209980, 203359, 209978, 203253, 203457, 203250, 203223, 203233, 203219, 203132, 203575, 203163, 203160, 203313 or 209381.

**48.** A chimeric molecule comprising a polypeptide according to Claim 47 fused to a heterologous amino acid sequence.

**49.** The chimeric molecule of Claim 75, wherein said heterologous amino acid sequence is an epitope tag sequence, or a Fc region of an immunoglobulin.

**50.** An antibody which specifically binds to a polypeptide according to Claim 47.

**51.** The antibody of Claim 50, wherein said antibody is a monoclonal antibody, a humanized antibody or a single-chain antibody.

**52.** Isolated nucleic acid having at least 80% nucleic acid sequence identity to:

(a) a nucleotide sequence encoding the polypeptide shown in Figure 82 (SEQ IDNO:203), Figure 2 (SEQ ID NO:4), Figure 4 (SEQ ID NO:9), Figure 6 (SEQ ID NO:14), Figure 8 (SEQ ID NO:16), Figure 10 (SEQ ID NO: 21), Figure 12 (SEQ ID NO:26), Figure 14 (SEQ ID NO:31), Figure 16 (SEQ ID NO:36), Figure 18 (SEQ ID NO: 41), Figure 20 (SEQ ID NO:46), Figure 22 (SEQ ID NO:51), Figure 24 (SEQ ID NO:56), Figure 26 (SEQ ID NO: 62), Figure 28 (SEQ ID NO:67), Figure 30 (SEQ ID NO:72), Figure 32 (SEQ ID NO:77), Figure 34 (SEQ ID NO: 85), Figure 36 (SEQ ID NO:90), Figure 38 (SEQ ID NO:98), Figure 40 (SEQ ID NO:107), Figure 42 (SEQ ID NO:112), Figure 44 (SEQ ID NO:117), Figure 46 (SEQ ID NO:127), Figure 48 (SEQ ID NO:132), Figure 50 (SEQ ID NO:137), Figure 52 (SEQ ID NO:143), Figure 54 (SEQ ID NO:148), Figure 56 (SEQ ID NO:153), Figure 58 (SEQ ID NO:155), Figure 60 (SEQ ID NO:160), Figure 62 (SEQ ID NO:162), Figure 64 (SEQ ID NO:170), Figure 66 (SEQ ID N0:181), Figure 68 (SEQ ID NO:183), Figure 70 (SEQ ID N0:191), Figure 72 (SEQ ID NO: 193), Figure 74 (SEQ ID NO:195), Figure 76 (SEQ ID NO:197), Figure 78 (SEQ ID NO:199), Figure 80 (SEQ IDNO:201), Figure 84 (SEQ ID NO:205), Figure 86 (SEQ ID NO:214); Figure 88 (SEQ ID NO:216), Figure 90 (SEQ ID NO:218), Figure 92 (SEQ ID NO:220), Figure 94 (SEQ ID NO:222), or Figure 96 (SEQ ID NO:227), lacking its associated signal peptide;

(b) a nucleotide sequence encoding an extracellular domain of the polypeptide shown in Figure 82 (SEQ IDNO: 203), Figure 2 (SEQ ID NO:4), Figure 4 (SEQ ID NO:9), Figure 6 (SEQ ID NO:14), Figure 8 (SEQ ID NO:16), Figure 10 (SEQ ID NO:21), Figure 12 (SEQ ID NO:26), Figure 14 (SEQ ID NO:31), Figure 16 (SEQ ID NO:36), Figure 18 (SEQ ID NO:41), Figure 20 (SEQ ID NO:46), Figure 22 (SEQ ID NO:51), Figure 24 (SEQ ID NO:56), Figure 26 (SEQ ID NO:62), Figure 28 (SEQ ID NO:67), Figure 30 (SEQ ID NO:72), Figure 32 (SEQ ID NO:77), Figure 34 (SEQ ID NO:85), Figure 36 (SEQ ID NO:90), Figure 38 (SEQ ID NO:98), Figure 40 (SEQ ID NO: 107), Figure 42 (SEQ ID NO:112), Figure 44 (SEQ ID NO:117), Figure 46 (SEQ ID NO:127), Figure 48 (SEQ ID NO:132), Figure 50 (SEQ ID NO:137), Figure 52 (SEQ ID NO:143), Figure 54 (SEQ ID NO:148), Figure 56 (SEQ ID NO:153), Figure 58 (SEQ ID NO:155), Figure 60 (SEQ ID NO:160), Figure 62 (SEQ ID NO:162), Figure 64 (SEQ ID NO:170), Figure 66 (SEQ ID NO:181), Figure 68 (SEQ ID NO:183), Figure 70 (SEQ ID NO:191), Figure 72 (SEQ ID NO:193), Figure 74 (SEQ ID NO:195), Figure 76 (SEQ ID NO:197), Figure 78 (SEQ ID NO: 199), Figure 80 (SEQ IDNO:201), Figure 84 (SEQ ID NO:205), Figure 86 (SEQ ID NO:214), Figure 88 (SEQ ID NO:216), Figure 90 (SEQ ID NO:218), Figure 92 (SEQ ID NO:220), Figure 94 (SEQ ID NO:222), or Figure 96 (SEQ ID NO:227), with its associated signal peptide; or

(c) a nucleotide sequence encoding an extracellular domain of the polypeptide shown in Figure 82 (SEQ IDNO: 203), Figure 2 (SEQ ID NO:4), Figure 4 (SEQ ID NO:9), Figure 6 (SEQ ID NO:14), Figure 8 (SEQ ID NO:16), Figure 10 (SEQ ID NO:21), Figure 12 (SEQ ID NO:26), Figure 14 (SEQ ID NO:31), Figure 16 (SEQ ID NO:36), Figure 18 (SEQ ID NO:41), Figure 20 (SEQ ID NO:46), Figure 22 (SEQ ID NO:51), Figure 24 (SEQ ID NO:56), Figure 26 (SEQ ID NO:62), Figure 28 (SEQ ID NO:67), Figure 30 (SEQ ID NO:72), Figure 32 (SEQ ID NO:77), Figure 34 (SEQ ID NO:85), Figure 36 (SEQ ID NO:90), Figure 38 (SEQ ID NO:98), Figure 40 (SEQ ID NO: 107), Figure 42 (SEQ ID NO:112), Figure 44 (SEQ ID NO:117), Figure 46 (SEQ ID NO:127), Figure 48 (SEQ ID NO:132), Figure 50 (SEQ ID NO:137), Figure 52 (SEQ ID NO:143), Figure 54 (SEQ ID NO:148), Figure 56 (SEQ ID NO:153), Figure 58 (SEQ ID NO:155), Figure 60 (SEQ ID NO:160), Figure 62 (SEQ ID NO:162), Figure 64 (SEQ ID NO:170), Figure 66 (SEQ ID NO:181), Figure 68 (SEQ ID NO:183), Figure 70 (SEQ ID NO:191), Figure 72 (SEQ ID NO:193), Figure 74 (SEQ ID NO:195), Figure 76 (SEQ ID NO:197), Figure 78 (SEQ ID NO: 199), Figure 80 (SEQ IDNO:201), Figure 84 (SEQ ID NO:205), Figure 86 (SEQ ID NO:214), Figure 88 (SEQ ID NO:216), Figure 90 (SEQ ID NO:218), Figure 92 (SEQ ID NO:220), Figure 94 (SEQ ID NO:222), or Figure 96 (SEQ ID NO:227), lacking its associated signal peptide.

**53.** An isolated polypeptide having at least 80% amino acid sequence identity to:

(a) the polypeptide shown in Figure 82 (SEQ IDNO:203), Figure 2 (SEQ ID NO:4), Figure 4 (SEQ ID NO:9), Figure 6 (SEQ ID NO:14), Figure 8 (SEQ ID NO:16), Figure 10 (SEQ ID NO:21), Figure 12 (SEQ ID NO:26), Figure 14 (SEQ ID NO:31), Figure 16 (SEQ ID NO:36), Figure 18 (SEQ ID NO:41), Figure 20 (SEQ ID NO:46), Figure 22 (SEQ ID NO:51), Figure 24 (SEQ ID NO:56), Figure 26 (SEQ ID NO:62), Figure 28 (SEQ ID NO:67), Figure 30 (SEQ ID NO:72), Figure 32 (SEQ ID NO:77), Figure 34 (SEQ ID NO:85), Figure 36 (SEQ ID NO:90), Figure 38 (SEQ ID NO:98), Figure 40 (SEQ ID NO:107), Figure 42 (SEQ ID NO:112), Figure 44 (SEQ ID NO: 117), Figure 46 (SEQ ID NO:127), Figure 48 (SEQ ID NO:132), Figure 50 (SEQ ID NO:137), Figure 52 (SEQ ID NO:143), Figure 54 (SEQ ID NO:148), Figure 56 (SEQ ID NO:153), Figure 58 (SEQ ID NO:155), Figure 60 (SEQ ID NO:160), Figure 62 (SEQ ID NO:162), Figure 64 (SEQ ID NO:170), Figure 66 (SEQ ID NO:181), Figure 68 (SEQ ID NO:183), Figure 70 (SEQ ID NO:191), Figure 72 (SEQ ID NO:193), Figure 74 (SEQ ID NO:195), Figure 76 (SEQ ID NO:197), Figure 78 (SEQ ID NO:199), Figure 80 (SEQ IDNO:201), Figure 84 (SEQ ID NO: 205), Figure 86 (SEQ ID NO:214), Figure 88 (SEQ ID NO:216), Figure 90 (SEQ ID NO:218), Figure 92 (SEQ ID NO:220), Figure 94 (SEQ ID NO:222), or Figure 96 (SEQ ID NO:227), lacking its associated signal peptide;

(b) an extracellular domain of the polypeptide shown in Figure 82 (SEQ IDNO:203), Figure 2 (SEQ ID NO:4), Figure 4 (SEQ ID NO:9), Figure 6 (SEQ ID NO:14), Figure 8 (SEQ ID NO:16), Figure 10 (SEQ ID NO:21), Figure 12 (SEQ ID NO:26), Figure 14 (SEQ ID NO:31), Figure 16 (SEQ ID NO:36), Figure 18 (SEQ ID NO:41), Figure 20 (SEQ ID NO:46), Figure 22 (SEQ ID NO:51), Figure 24 (SEQ ID NO:56), Figure 26 (SEQ ID NO:62), Figure 28 (SEQ ID NO:67), Figure 30 (SEQ ID NO:72), Figure 32 (SEQ ID NO:77), Figure 34 (SEQ ID NO:85), Figure 36 (SEQ ID NO:90), Figure 38 (SEQ ID NO:98), Figure 40 (SEQ ID NO:107), Figure 42 (SEQ ID NO:112), Figure 44 (SEQ ID NO:117), Figure 46 (SEQ ID NO:127), Figure 48 (SEQ ID NO:132), Figure 50 (SEQ ID NO: 137), Figure 52 (SEQ ID NO:143), Figure 54 (SEQ ID NO:148), Figure 56 (SEQ ID NO:153), Figure 58 (SEQ ID NO:155), Figure 60 (SEQ ID NO:160), Figure 62 (SEQ ID NO:162), Figure 64 (SEQ ID NO:170), Figure 66 (SEQ ID NO:181), Figure 68 (SEQ ID NO:183), Figure 70 (SEQ ID NO:191), Figure 72 (SEQ ID NO:193), Figure 74 (SEQ ID NO:195), Figure 76 (SEQ ID NO:197), Figure 78 (SEQ ID NO:199), Figure 80 (SEQ IDNO:201), Figure 84 (SEQ ID NO:205), Figure 86 (SEQ ID NO:214), Figure 88 (SEQ ID NO:216), Figure 90 (SEQ ID NO: 218), Figure 92 (SEQ ID NO:220), Figure 94 (SEQ ID NO:222), or Figure 96 (SEQ ID NO:227), with its associated signal peptide; or

(c) an extracellular domain of the polypeptide shown in Figure 82 (SEQ IDNO:203), Figure 2 (SEQ ID NO:4), Figure 4 (SEQ ID NO:9), Figure 6 (SEQ ID NO:14), Figure 8 (SEQ ID NO:16), Figure 10 (SEQ ID NO:21), Figure 12 (SEQ ID NO:26), Figure 14 (SEQ ID NO:31), Figure 16 (SEQ ID NO:36), Figure 18 (SEQ ID NO:41), Figure 20 (SEQ ID NO:46), Figure 22 (SEQ ID NO:51), Figure 24 (SEQ ID NO:56), Figure 26 (SEQ ID NO:62), Figure 28 (SEQ ID NO:67), Figure 30 (SEQ ID NO:72), Figure 32 (SEQ ID NO:77), Figure 34 (SEQ ID NO:85), Figure 36 (SEQ ID NO:90), Figure 38 (SEQ ID NO:98), Figure 40 (SEQ ID NO:107), Figure 42 (SEQ ID NO:112), Figure 44 (SEQ ID NO:117), Figure 46 (SEQ ID NO:127), Figure 48 (SEQ ID NO:132), Figure 50 (SEQ ID NO: 137), Figure 52 (SEQ ID NO:143), Figure 54 (SEQ ID NO:148), Figure 56 (SEQ ID NO:153), Figure 58 (SEQ ID NO:155), Figure 60 (SEQ ID NO:160), Figure 62 (SEQ ID NO:162), Figure 64 (SEQ ID NO:170), Figure 66 (SEQ ID NO:181), Figure 68 (SEQ ID NO:183), Figure 70 (SEQ ID NO:191), Figure 72 (SEQ ID NO:193), Figure 74 (SEQ ID NO:195), Figure 76 (SEQ ID NO:197), Figure 78 (SEQ ID NO:199), Figure 80 (SEQ IDNO:201), Figure 84 (SEQ ID NO:205), Figure 86 (SEQ ID NO:214), Figure 88 (SEQ ID NO:216), Figure 90 (SEQ ID NO: 218), Figure 92 (SEQ ID NO:220), Figure 94 (SEQ ID NO:222), or Figure 96 (SEQ ID NO:227), lacking its associated signal peptide.

# FIGURE 1

TGGGGGCCCCCCAGGCTCGCGCGTGGAGCGAAGCAGC**ATG**GGCAGTCGGTGCGCGCTGGCCCTGGCGGTGCTCTC
GGCCTTGCTGTGTCAGGTCTGGAGCTCTGGGGTGTTCGAACTGAAGCTGCAGGAGTTCGTCAACAAGAAGGGGCT
GCTGGGGAACCGCAATTGCTGCCGCGGGGGCGCGGGGCCACCGCCGTGCGCCTGCCGGACCTTCTTCCGCGTGTG
CCTCAAGCACTACCAGGCCAGCGTGTCCCCCGAGCCGCCCTGCACCTACGGCAGCGCCGTCACCCCCGTGCTGGG
CGTCGACTCCTTCAGTCTGCCCGACGGCGGGGGCGCCGACTCCGCGTTCAGCAACCCCATCCGCTTCCCCTTCGG
CTTCACCTGGCCGGGCACCTTCTCTCTGATTATTGAAGCTCTCCACACAGATTCTCCTGATGACCTCGCAACAGA
AAACCCAGAAAGACTCATCAGCCGCCTGGCCACCCAGAGGCACCTGACGGTGGGCGAGGAGTGGTCCCAGGACCT
GCACAGCAGCGGCCGCACGGACCTCAAGTACTCCTACCGCTTCGTGTGTGACGAACACTACTACGGAGAGGGCTG
CTCCGTTTTCTGCCGTCCCCGGGACGATGCCTTCGGCCACTTCACCTGTGGGGAGCGTGGGGAGAAAGTGTGCAA
CCCTGGCTGGAAAGGGCCCTACTGCACAGAGCCGATCTGCCTGCCTGGATGTGATGAGCAGCATGGATTTTGTGA
CAAACCAGGGGAATGCAAGTGCAGAGTGGGCTGGCAGGGCCGGTACTGTGACGAGTGTATCCGCTATCCAGGCTG
TCTCCATGGCACCTGCCAGCAGCCCTGGCAGTGCAACTGCCAGGAAGGCTGGGGGGGCCTTTTCTGCAACCAGGA
CCTGAACTACTGCACACACCATAAGCCCTGCAAGAATGGAGCCACCTGCACCAACACGGGCCAGGGGAGCTACAC
TTGCTCTTGCCGGCCTGGGTACACAGGTGCCACCTGCGAGCTGGGGATTGACGAGTGTGACCCCAGCCCTTGTAA
GAACGGAGGGAGCTGCACGGATCTCGAGAACAGCTACTCCTGTACCTGCCCACCCGGCTTCTACGGCAAAATCTG
TGAATTGAGTGCCATGACCTGTGCGGACGGCCCTTGCTTTAACGGGGGTCGGTGCTCAGACAGCCCCGATGGAGG
GTACAGCTGCCGCTGCCCCGTGGGCTACTCCGGCTTCAACTGTGAGAAGAAAATTGACTACTGCAGCTCTTCACC
CTGTTCTAATGGTGCCAAGTGTGTGGACCTCGGTGATGCCTACCTGTGCCGCTGCCAGGCCGGCTTCTCGGGGAG
GCACTGTGACGACAACGTGGACGACTGCGCCTCCTCCCCGTGCGCCAACGGGGGCACCTGCCGGGATGGCGTGAA
CGACTTCTCCTGCACCTGCCCGCCTGGCTACACGGGCAGGAACTGCAGTGCCCCCGTCAGCAGGTGCGAGCACGC
ACCCTGCCACAATGGGGCCACCTGCCACGAGAGGGGCCACCGCTATGTGTGCGAGTGTGCCCGAGGCTACGGGGG
TCCCAACTGCCAGTTCCTGCTCCCCGAGCTGCCCCCGGGCCCAGCGGTGGTGGACCTCACTGAGAAGCTAGAGGG
CCAGGGCGGGCCATTCCCCTGGGTGGCCGTGTGCGCCGGGGTCATCCTTGTCCTCATGCTGCTGCTGGGCTGTGC
CGCTGTGGTGGTCTGCGTCCGGCTGAGGCTGCAGAAGCACCGGCCCCCAGCCGACCCCTGCCGGGGGGAGACGGA
GACCATGAACAACCTGGCCAACTGCCAGCGTGAGAAGGACATCTCAGTCAGCATCATCGGGGCCACGCAGATCAA
GAACACCAACAAGAAGGCGGACTTCCACGGGGACCACAGCGCCGACAAGAATGGCTTCAAGGCCCGCTACCCAGC
GGTGGACTATAACCTCGTGCAGGACCTCAAGGGTGACGACACCGCCGTCAGGGACGCGCACAGCAAGCGTGACAC
CAAGTGCCAGCCCCAGGGCTCCTCAGGGGAGGAGAAGGGGACCCCGACCACACTCAGGGGTGGAGAAGCATCTGA
AAGAAAAAGGCCGGACTCGGGCTGTTCAACTTCAAAAGACACCAAGTACCAGTCGGTGTACGTCATATCCGAGGA
GAAGGATGAGTGCGTCATAGCAACTGAGGTG**TAA**AATGGAAGTGAGATGGCAAGACTCCCGTTTCTCTTAAAATA
AGTAAAATTCCAAGGATATATGCCCCAACGAATGCTGCTGAAGAGGAGGGAGGCCTCGTGGACTGCTGCTGAGAA
ACCGAGTTCAGACCGAGCAGGTTCTCCTCCTGAGGTCCTCGACGCCTGCCGACAGCCTGTCGCGGCCCGGCCGCC
TGCGGCACTGCCTTCCGTGACGTCGCCGTTGCACTATGGACAGTTGCTCTTAAGAGAATATATATTTAAATGGGT
GAACTGAATTACGCATAAGAAGCATGCACTGCCTGAGTGTATATTTTGGATTCTTATGAGCCAGTCTTTTCTTGA
ATTAGAAACACAAACACTGCCTTTATTGTCCTTTTTGATACGAAGATGTGCTTTTTCTAGATGGAAAAGATGTGT
GTTATTTTTTGGATTTGTAAAAATATTTTTCATGATATCTGTAAAGCTTGAGTATTTTGTGATGTTCGTTTTTTA
TAATTTAAATTTTGGTAAATATGTACAAAGGCACTTCGGGTCTATGTGACTATATTTTTTTGTATATAAATGTAT
TTATGGAATATTGTGCAAATGTTATTTGAGTTTTTTACTGTTTTGTTAATGAAGAAATTCCTTTTTAAAATATTT
TTCCAAAATAAATTTATGAATGACAAAAAAAAAAAAAAAAAAAAAAAAAAAAAAAAAAAAAAAAAAAAAAAAAAA
AAAAAAAA

# FIGURE 2

Signal sequence: Amino acids 1-21

Transmembrane domain: Amino acids 546-566

N-glycosylation site: Amino acids 477-481

cAMP- and cGMP-dependent protein kinase phosphorylation site:
Amino acids 660-664

Tyrosine kinase phosphorylation sites: Amino acids 176-185;252-261

N-myristoylation sites: Amino acids 2-8;37-43;40-46;
98-104;99-105;262-268;281-287;
282-288;301-307;310-316;328-334;
340-344;378-384;387-393;512-518;
676-682;683-689;695-701

Aspartic acid and asparagine hydroxylation sites:
Amino acids 343-355;420-432;
458-470

Prokaryotic membrane lipoprotein lipid attachment site:
Amino acids 552-563

EGF-like domain cysteine pattern signature:
Amino acids 243-255;274-286;
314-326;352-364;391-403;429-441;
467-479;505-517

MGSRCALALAVLSALLCQVWSSGVFELKLQEFVNKKGLLGNRNCCRGGAGPPPCACRTFFRVCLKHYQASVSPEP
PCTYGSAVTPVLGVDSFSLPDGGGADSAFSNPIRFPFGFTWPGTFSLIIEALHTDSPDDLATENPERLISRLATQ
RHLTVGEEWSQDLHSSGRTDLKYSYRFVCDEHYYGEGCSVFCRPRDDAFGHFTCGERGEKVCNPGWKGPYCTEPI
CLPGCDEQHGFCDKPGECKCRVGWQGRYCDECIRYPGCLHGTCQQPWQCNCQEGWGGLFCNQDLNYCTHHKPCKN
GATCTNTGQGSYTCSCRPGYTGATCELGIDECDPSPCKNGGSCTDLENSYSCTCPPGFYGKICELSAMTCADGPC
FNGGRCSDSPDGGYSCRCPVGYSGFNCEKKIDYCSSSPCSNGAKCVDLGDAYLCRCQAGFSGRHCDDNVDDCASS
PCANGGTCRDGVNDFSCTCPPGYTGRNCSAPVSRCEHAPCHNGATCHERGHRYVCECARGYGGPNCQFLLPELPP
GPAVVDLTEKLEGQGGPFPWVAVCAGVILVLMLLLGCAAVVVCVRLRLQKHRPPADPCRGETETMNNLANCQREK
DISVSIIGATQIKNTNKKADFHGDHSADKNGFKARYPAVDYNLVQDLKGDDTAVRDAHSKRDTKCQPQGSSGEEK
GTPTTLRGGEASERKRPDSGCSTSKDTKYQSVYVISEEKDECVIATEV

# FIGURE 3

GGCTCAGAGGCCCCACTGGACCCTCGGCTCTTCCTTGGACTTCTTGTGTGTTCTGTGAGCTTCGCTGGATTCAGG
GTCTTGGGCATCAGAGGTGAGAGGGTGGAAGGTCCGCCGCG**ATG**GGGAAGCCCTGGCTGCGTGCGCTACAGCTG
CTGCTCCTGCTGGGCGCGTCGTGGGCGCGGGCGGGCGCCCCGCGCTGCACCTACACCTTCGTGCTGCCCCCGCAG
AAGTTCACGGGCGCTGTGTGCTGGAGCGGCCCCGCATCCACGCGGGCGACGCCCGAGGCCGCCAACGCCAGCGAG
CTGGCGGCGCTGCGCATGCGCGTCGGCCGCCACGAGGAGCTGTTACGCGAGCTGCAGAGGCTGGCGGCGGCCGAC
GGCGCCGTGGCCGGCGAGGTGCGCGCGCTGCGCAAGGAGAGCCGCGGCCTGAGCGCGCGCCTGGGCCAGTTGCGC
GCGCAGCTGCAGCACGAGGCGGGGCCCGGGGCGGGCCCGGGGGCGGATCTGGGGGCGGAGCCTGCCGCGGCGCTG
GCGCTGCTCGGGGAGCGCGTGCTCAACGCGTCCGCCGAGGCTCAGCGCGCAGCCGCCCGGTTCCACCAGCTGGAC
GTCAAGTTCCGCGAGCTGGCGCAGCTCGTCACCCAGCAGAGCAGTCTCATCGCCCGCCTGGAGCGCCTGTGCCCG
GGAGGCGCGGGCGGGCAGCAGCAGGTCCTGCCGCCACCCCCACTGGTGCCTGTGGTTCCGGTCCGTCTTGTGGGT
AGCACCAGTGACACCAGTAGGATGCTGGACCCAGCCCCAGAGCCCCAGAGAGACCAGACCCAGAGACAGCAGGAG
CCCATGGCTTCTCCCATGCCTGCAGGTCACCCTGCGGTCCCCACCAAGCCTGTGGGCCCGTGGCAGGATTGTGCA
GAGGCCCGCCAGGCAGGCCATGAACAGAGTGGAGTGTATGAACTGCGAGTGGGCCGTCACGTAGTGTCAGTATGG
TGTGAGCAGCAACTGGAGGGTGGAGGCTGGACTGTGATCCAGCGGAGGCAAGATGGTTCAGTCAACTTCTTCACT
ACCTGGCAGCACTATAAGGCGGGCTTTGGGCGGCCAGACGGAGAATACTGGCTGGGCCTTGAACCCGTGTATCAG
CTGACCAGCCGTGGGGACCATGAGCTGCTGGTTCTCCTGGAGGACTGGGGGGCCGTGGAGCACGTGCCCACTAT
GATGGCTTCTCCCTGGAACCCGAGAGCGACCACTACCGCCTGCGGCTTGGCCAGTACCATGGTGATGCTGGAGAC
TCTCTTTCCTGGCACAATGACAAGCCCTTCAGCACCGTGGATAGGGACCGAGACTCCTATTCTGGTAACTGTGCC
CTGTACCAGCGGGGAGGCTGGTGGTACCATGCCTGTGCCCACTCCAACCTCAACGGTGTGTGGCACCACGGCGGC
CACTACCGAAGCCGCTACCAGGATGGTGTCTACTGGGCTGAGTTTCGTGGTGGGGCATATTCTCTCAGGAAGGCC
GCCATGCTCATTCGGCCCCTGAAGCTG**TGA**CTCTGTGTTCCTCTGTCCCCTAGGCCCTAGAGGACATTGGTCAGC
AGGAGCCCAAGTTGTTCTGGCCACACCTTCTTTGTGGCTCAGTGCCAATGTGTCCCACAGAACTTCCCACTGTGG
ATCTGTGACCCTGGGCGCTGAAAATGGACCCAGGAATCCCCCCCGTCAATATCTTGGCCTCAGATGGCTCCCCA
AGGTCATTCATATCTCGGTTTGAGCTCATATCTTATAATAACACAAAGTAGCCAC

# FIGURE 4

Signal sequence: Amino acids 1-20

N-glycosylation sites: Amino acids 58-62;145-149

cAMP- and cGMP-dependent protein kinase phosphorylation site:
Amino acids 97-101

Tyrosine kinase phosphorylation site: Amino acids 441-448

N-myristoylation sites: Amino acids 16-22;23-29;87-93;
108-114;121-127;125-131;129-135;
187-193;293-299;353-359;378-384;
445-451;453-459

Cell attachment sequence: Amino acids 340-343

Fibrinogen beta and gamma chains C-terminal domain signature:
Amino acids 418-431

MGKPWLRALQLLLLLGASWARAGAPRCTYTFVLPPQKFTGAVCWSGPASTRATPEAANASELAALRMRVGRHEEL
LRELQRLAAADGAVAGEVRALRKESRGLSARLGQLRAQLQHEAGPGAGPGADLGAEPAAALALLGERVLNASAEA
QRAAARFHQLDVKFRELAQLVTQQSSLIARLERLCPGGAGGQQQVLPPPPLVPVVPVRLVGSTSDTSRMLDPAPE
PQRDQTQRQQEPMASPMPAGHPAVPTKPVGPWQDCAEARQAGHEQSGVYELRVGRHVVSVWCEQQLEGGGWTVIQ
RRQDGSVNFFTTWQHYKAGFGRPDGEYWLGLEPVYQLTSRGDHELLVLLEDWGGRGARAHYDGFSLEPESDHYRL
RLGQYHGDAGDSLSWHNDKPFSTVDRDRDSYSGNCALYQRGGWWYHACAHSNLNGVWHHGGHYRSRYQDGVYWAE
FRGGAYSLRKAAMLIRPLKL

FIGURE 5

GCGGACGCGTGGGTGAAATTGAAAATCAAGATAAAAATGTTCACAATTAAGCTCCTTCTTTTTATTGTTCCTCTA
GTTATTTCCTCCAGAATTGATCAAGACAATTCATCATTTGATTCTCTATCTCCAGAGCCAAAATCAAGATTTGCT
ATGTTAGACGATGTAAAAATTTTAGCCAATGGCCTCCTTCAGTTGGGACATGGTCTTAAAGACTTTGTCCATAAG
ACGAAGGGCCAAATTAATGACATATTTCAAAAACTCAACATATTTGATCAGTCTTTTTATGATCTATCGCTGCAA
ACCAGTGAAATCAAAGAAGAAGAAAAGGAACTGAGAAGAACTACATATAAACTACAAGTCAAAAATGAAGAGGTA
AAGAATATGTCACTTGAACTCAACTCAAAACTTGAAAGCCTCCTAGAAGAAAAAATTCTACTTCAACAAAAAGTG
AAATATTTAGAAGAGCAACTAACTAACTTAATTCAAAATCAACCTGAAACTCCAGAACACCCAGAAGTAACTTCA
CTTAAAACTTTTGTAGAAAAACAAGATAATAGCATCAAAGACCTTCTCCAGACCGTGGAAGACCAATATAAACAA
TTAAACCAACAGCATAGTCAAATAAAAGAAATAGAAAATCAGCTCAGAAGGACTAGTATTCAAGAACCCACAGAA
ATTTCTCTATCTTCCAAGCCAAGAGCACCAAGAACTACTCCCTTTCTTCAGTTGAATGAAATAAGAAATGTAAAA
CATGATGGCATTCCTGCTGAATGTACCACCATTTATAACAGAGGTGAACATACAAGTGGCATGTATGCCATCAGA
CCCAGCAACTCTCAAGTTTTTCATGTCTACTGTGATGTTATATCAGGTAGTCCATGGACATTAATTCAACATCGA
ATAGATGGATCACAAAACTTCAATGAAACGTGGGAGAACTACAAATATGGTTTTGGGAGGCTTGATGGAGAATTT
TGGTTGGGCCTAGAGAAGATATACTCCATAGTGAAGCAATCTAATTATGTTTTACGAATTGAGTTGGAAGACTGG
AAAGACAACAAACATTATATTGAATATTCTTTTTACTTGGGAAATCACGAAACCAACTATACGCTACATCTAGTT
GCGATTACTGGCAATGTCCCCAATGCAATCCCGGAAAACAAAGATTTGGTGTTTTCTACTTGGGATCACAAAGCA
AAAGGACACTTCAACTGTCCAGAGGGTTATTCAGGAGGCTGGTGGTGGCATGATGAGTGTGGAGAAAACAACCTA
AATGGTAAATATAACAAACCAAGAGCAAAATCTAAGCCAGAGAGGAGAAGAGGATTATCTTGGAAGTCTCAAAAT
GGAAGGTTATACTCTATAAAATCAACCAAAATGTTGATCCATCCAACAGATTCAGAAAGCTTTGAATGAACTGAG
GCAATTTAAAGGCATATTTAACCATTAACTCATTCCAAGTTAATGTGGTCTAATAATCTGGTATAAATCCTTAAG
AGAAAGCTTGAGAAATAGATTTTTTTATCTTAAAGTCACTGTCTATTTAAGATTAAACATACAATCACATAACC
TTAAAGAATACCGTTTACATTTCTCAATCAAAATTCTTATAATACTATTTGTTTTAAATTTTGTGATGTGGGAATC
AATTTTAGATGGTCACAATCTAGATTATAATCAATAGGTGAACTTATTAAATAACTTTTCTAAATAAAAAATTTA
GAGACTTTTATTTTAAAAGGCATCATATGAGCTAATATCACAACTTTCCCAGTTTAAAAAACTAGTACTCTTGTT
AAAACTCTAAACTTGACTAAATACAGAGGACTGGTAATTGTACAGTTCTTAAATGTTGTAGTATTAATTTCAAAA
CTAAAAATCGTCAGCACAGAGTATGTGTAAAAATCTGTAATACAAATTTTTAAACTGATGCTTCATTTTGCTACA
AAATAATTTGGAGTAAATGTTTGATATGATTTATTTATGAAACCTAATGAAGCAGAATTAAATACTGTATTAAAA
TAAGTTCGCTGTCTTT

## FIGURE 6

| | |
|---|---|
| Signal sequence: | Amino acids 1-16 |
| N-glycosylation sites: | Amino acids 23-27;115-119; 296-300;357-361 |
| cAMP- and cGMP-dependent protein kinase phosphorylation sites: | Amino acids 100-104;204-208 |
| Tyrosine kinase phosphorylation site: | Amino acids 342-351 |
| N-myristoylation sites: | Amino acids 279-285;352-358; 367-373 |
| Leucine zipper patterns: | Amino acids 120-142;127-149 |

MFTIKLLLFIVPLVISSRIDQDNSSFDSLSPEPKSRFAMLDDVKILANGLLQLGHGLKDFVHKTKGQINDIFQKL
NIFDQSFYDLSLQTSEIKEEEKELRRTTYKLQVKNEEVKNMSLELNSKLESLLEEKILLQQKVKYLEEQLTNLIQ
NQPETPEHPEVTSLKTFVEKQDNSIKDLLQTVEDQYKQLNQQHSQIKEIENQLRRTSIQEPTEISLSSKPRAPRT
TPFLQLNEIRNVKHDGIPAECTTIYNRGEHTSGMYAIRPSNSQVFHVYCDVISGSPWTLIQHRIDGSQNFNETWE
NYKYGFGRLDGEFWLGLEKIYSIVKQSNYVLRIELEDWKDNKHYIEYSFYLGNHETNYTLHLVAITGNVPNAIPE
NKDLVFSTWDHKAKGHFNCPEGYSGGWWWHDECGENNLNGKYNKPRAKSKPERRRGLSWKSQNGRLYSIKSTKML
IHPTDSESFE

FIGURE 7

TATTTACCATATCAGATTCACATTCAGTCCTCAGCAAAATGAAGGGCTCCATTTTCACTCTGTTTTTATTCTCTG
TCCTATTTGCCATCTCAGAAGTGCGGAGCAAGGAGTCTGTGAGACTCTGTGGGCTAGAATACATACGGACAGTCA
TCTATATCTGTGCTAGCTCCAGGTGGAGAAGGCATCTGGAGGGGATCCCTCAAGCTCAGCAAGCTGAGACAGGAA
ACTCCTTCCAGCTCCCACATAAACGTGAGTTTTCTGAGGAAAATCCAGCGCAAAACCTTCCGAAGGTGGATGCCT
CAGGGGAAGACCGTCTTTGGGGTGGACAGATGCCCACTGAAGAGCTTTGGAAGTCAAAGAAGCATTCAGTGATGT
CAAGACAAGATTTACAAACTTTGTGTTGCACTGATGGCTGTTCCATGACTGATTTGAGTGCTCTTTGCTAAGACA
AGAGCAAATACCCAATGGGTGGCAGAGCTTTATCACATGTTTAATTACAGTGTTTTACTGCCTGGTAGAACACTA
ATATTGTGTTATTAAAATGATGGCTTTTGGGTAGGCAAAACTTCTTTTCTAAAAGGTATAGCTGAGCGGTTGAAA
CCACAGTGATCTCTATTTTCTCCCTTTGCCAAGGTTAATGAACTGTTCTTTTCAAATTCTACTAATGCTTTGAAA
TTTCAAATGCTGCGCAAAATTGCAATAAAAATGCTATAAA

## FIGURE 8

| | |
|---|---|
| Signal sequence: | Amino acids 1-18 |
| cAMP- and cGMP-dependent protein kinase phosphorylation site: | Amino acids 107-111 |
| N-myristoylation sites: | Amino acids 3-9;52-58;96-102;<br>125-131 |
| Insulin family signature: | Amino acids 121-136 |

MKGSIFTLFLFSVLFAISEVRSKESVRLCGLEYIRTVIYICASSRWRRHLEGIPQAQQAETGNSFQLPHKREFSE
ENPAQNLPKVDASGEDRLWGGQMPTEELWKSKKHSVMSRQDLQTLCCTDGCSMTDLSALC

FIGURE 9

CCCACGCGTCCGAACCTCTCCAGCG**ATG**GGAGCCGCCCGCCTGCTGCCCAACCTCACTCTGTGCTTACAGCTGCT
GATTCTCTGCTGTCAAACTCAGTACGTGAGGGACCAGGGCGCCATGACCGACCAGCTGAGCAGGCGGCAGATCCG
CGAGTACCAACTCTACAGCAGGACCAGTGGCAAGCACGTGCAGGTCACCGGGCGTCGCATCTCCGCCACCGCCGA
GGACGGCAACAAGTTTGCCAAGCTCATAGTGGAGACGGACACGTTTGGCAGCCGGGTTCGCATCAAAGGGGCTGA
GAGTGAGAAGTACATCTGTATGAACAAGAGGGGCAAGCTCATCGGGAAGCCCAGCGGGAAGAGCAAAGACTGCGT
GTTCACGGAGATCGTGCTGGAGAACAACTATACGGCCTTCCAGAACGCCCGGCACGAGGGCTGGTTCATGGCCTT
CACGCGGCAGGGGCGGCCCCGCCAGGCTTCCCGCAGCCGCCAGAACCAGCGCGAGGCCCACTTCATCAAGCGCCT
CTACCAAGGCCAGCTGCCCTTCCCCAACCACGCCGAGAAGCAGAAGCAGTTCGAGTTTGTGGGCTCCGCCCCCAC
CCGCCGGACCAAGCGCACACGGCGGCCCCAGCCCCTCACG**TAG**TCTGGGAGGCAGGGGGCAGCAGCCCCTGGGCC
GCCTCCCCACCCCTTTCCCTTCTTAATCCAAGGACTGGGCTGGGGTGGCGGGAGGGGAGCCAGATCCCCGAGGGA
GGACCCTGAGGGCCGCGAAGCATCCGAGCCCCCAGCTGGGAAGGGGCAGGCCGGTGCCCCAGGGGCGGCTGGCAC
AGTGCCCCCTTCCCGGACGGGTGGCAGGCCCTGGAGAGGAACTGAGTGTCACCCTGATCTCAGGCCACCAGCCTC
TGCCGGCCTCCCAGCCGGGCTCCTGAAGCCCGCTGAAAGGTCAGCGACTGAAGGCCTTGCAGACAACCGTCTGGA
GGTGGCTGTCCTCAAAATCTGCTTCTCGGATCTCCCTCAGTCTGCCCCCAGCCCCCAAACTCCTCCTGGCTAGAC
TGTAGGAAGGGACTTTTGTTTGTTTGTTTGTTTCAGGAAAAAAGAAAGGGAGAGAGAGGAAAATAGAGGGTTGTC
CACTCCTCACATTCCACGACCCAGGCCTGCACCCCACCCCCAACTCCCAGCCCCGGAATAAAACCATTTTCCTGC

## FIGURE 10

| | |
|---|---|
| Signal sequence: | Amino acids 1-22 |
| N-glycosylation sites: | Amino acids 9-13;126-130 |
| cAMP- and cGMP-dependent protein kinase phosphorylation site: | Amino acids 60-64 |
| Tyrosine kinase phosphorylation sites: | Amino acids 39-48;89-97 |
| N-myristoylation sites: | Amino acids 69-75;188-194 |
| Amidation site: | Amino acids 58-62 |
| HBGF/FGF family signature: | Amino acids 103-128 |

MGAARLLPNLTLCLQLLILCCQTQYVRDQGAMTDQLSRRQIREYQLYSRTSGKHVQVTGRRISATAEDGNKFAKL
IVETDTFGSRVRIKGAESEKYICMNKRGKLIGKPSGKSKDCVFTEIVLENNYTAFQNARHEGWFMAFTRQGRPRQ
ASRSRQNQREAHFIKRLYQGQLPFPNHAEKQKQFEFVGSAPTRRTKRTRRPQPLT

# FIGURE 11

GCAGCTGGTTACTGCATTTCTCCATGTGGCAGACAGAGCAAAGCCACAACGCTTTCTCTGCTGGATTAAAGACGG
CCCACAGACCAGAACTTCCACTATACTACTTAAAATTACATAGGTGGCTTGTCAAATTCAATTGATTAGTATTGT
AAAAGGAAAAAGAAGTTCCTTCTTACAGCTTGGATTCAACGGTCCAAAACAAAAATGCAGCTGCCATTAAAGTCT
CAGATGAACAAACTTCTACACTGATTTTTAAAATCAAGAATAAGGGCAGCAAGTTTCTGGATTCACTGAATCAAC
AGACACAAAAAGCTGGCAATATAGCAACTATGAAGAGAAAAGCTACTAATAAAATTAACCCAACGCATAGAAGAC
TTTTTTTTCTCTTCTAAAAACAACTAAGTAAAGACTTAAATTTAAACACATCATTTTACAACCTCATTTCAAAAT
GAAGACTTTTACCTGGACCCTAGGTGTGCTATTCTTCCTACTAGTGGACACTGGACATTGCAGAGGTGGACAATT
CAAAATTAAAAAAATAAACCAGAGAAGATACCCTCGTGCCACAGATGGTAAAGAGGAAGCAAAGAAATGTGCATA
CACATTCCTGGTACCTGAACAAAGAATAACAGGGCCAATCTGTGTCAACACCAAGGGGCAAGATGCAAGTACCAT
TAAAGACATGATCACCAGGATGGACCTTGAAAACCTGAAGGATGTGCTCTCCAGGCAGAAGCGGGAGATAGATGT
TCTGCAACTGGTGGTGGATGTAGATGGAAACATTGTGAATGAGGTAAAGCTGCTGAGAAAGGAAAGCCGTAACAT
GAACTCTCGTGTTACTCAACTCTATATGCAATTATTACATGAGATTATCCGTAAGAGGGATAATTCACTTGAACT
TTCCCAACTGGAAAACAAAATCCTCAATGTCACCACAGAAATGTTGAAGATGGCAACAAGATACAGGGAACTAGA
GGTGAAATACGCTTCCTTGACTGATCTTGTCAATAACCAATCTGTGATGATCACTTTGTTGGAAGAACAGTGCTT
GAGGATATTTTCCCGACAAGACACCCATGTGTCTCCCCCACTTGTCCAGGTGGTGCCACAACATATTCCTAACAG
CCAACAGTATACTCCTGGTCTGCTGGGAGGTAACGAGATTCAGAGGGATCCAGGTTATCCCAGAGATTTAATGCC
ACCACCTGATCTGGCAACTTCTCCCACCAAAAGCCCTTTCAAGATACCACCGGTAACTTTCATCAATGAAGGACC
ATTCAAAGACTGTCAGCAAGCAAAAGAAGCTGGGCATTCGGTCAGTGGGATTTATATGATTAAACCTGAAAACAG
CAATGGACCAATGCAGTTATGGTGTGAAAACAGTTTGGACCCTGGGGGTTGGACTGTTATTCAGAAAAGAACAGA
CGGCTCTGTCAACTTCTTCAGAAATTGGGAAAATTATAAGAAAGGGTTTGGAAACATTGACGGAGAATACTGGCT
TGGACTGGAAAATATCTATATGCTTAGCAATCAAGATAATTACAAGTTATTGATTGAATTAGAAGACTGGAGTGA
TAAAAAAGTCTATGCAGAATACAGCAGCTTTCGTCTGGAACCTGAAAGTGAATTCTATAGACTGCGCCTGGGAAC
TTACCAGGGAAATGCAGGGGATTCTATGATGTGGCATAATGGTAAACAATTCACCACACTGGACAGAGATAAAGA
TATGTATGCAGGAAACTGCGCCCACTTTCATAAAGGAGGCTGGTGGTACAATGCCTGTGCACATTCTAACCTAAA
TGGAGTATGGTACAGAGGAGGCCATTACAGAAGCAAGCACCAAGATGGAATTTTCTGGCCGAATACAGAGGCGG
GTCATACTCCTTAAGAGCAGTTCAGATGATGATCAAGCCTATTGACTGAAGAGAGACACTCGCCAATTTAAATGA
CACAGAACTTTGTACTTTTCAGCTCTTAAAAATGTAAATGTTACATGTATATTACTTGGCACAATTTATTTCTAC
ACAGAAAGTTTTTAAAATGAATTTTACCGTAACTATAAAAGGGAACCTATAAATGTAGTTTCATCTGTCGTCAAT
TACTGCAGAAAATTATGTGTATCCACAACCTAGTTATTTTAAAAATTATGTTGACTAAATACAAAGTTTGTTTTC
TAAAATGTAAATATTTGCCACAATGTAAAGCAAATCTTAGCTATATTTTAAATCATAAATAACATGTTCAAGATA
CTTAACAATTTATTTAAAATCTAAGATTGCTCTAACGTCTAGTGAAAAAAATATTTTTTAAATTTCAGCCAAATA
ATGCATTTTATTTTATAAAAATACAGACAGAAAATTAGGGAGAAACTTCTAGTTTTGCCAATAGAAAATGTTCTT
CCATTGAATAAAAGTTATTTCAAATTGAATTTGTGCCTTTCACACGTAATGATTAAATCTGAATTCTTAATAATA
TATCCTATGCTGATTTTCCCAAAACATGACCCATAGTATTAAATACATATCATTTTTAAAAATAAAAAAAAACCC
AAAAATAATGCATGCATAATTTAAATGGTCAATTTATAAAGACAAATCTATGAATGAATTTTTCAGTGTTATCTT
CATATGATATGCTGAACACCAAAATCTCCAGAAATGCATTTTATGTAGTTCTAAAATCAGCAAAATATTGGTATT
ACAAAAATGCAGAATATTTAGTGTGCTACAGATCTGAATTATAGTTCTAATTTATTATTACTTTTTTTCTAATTT
ACTGATCTTACTACTACAAAGAAAAAAAAACCCAACCCATCTGCAATTCAAATCAGAAAGTTTGGACAGCTTTAC
AAGTATTAGTGCATGCTCAGAACAGGTGGGACTAAAACAAACTCAAGGAACTGTTGGCTGTTTTCCCGATACTGA
GAATTCAACAGCTCCAGAGCAGAAGCCACAGGGGCATAGCTTAGTCCAAACTGCTAATTTCATTTTACAGTGTAT
GTAACGCTTAGTCTCACAGTGTCTTTAACTCATCTTTGCAATCAACAACTTTACTAGTGACTTTCTGGAACAATT
TCCTTTCAGGAATACATATTCACTGCTTAGAGGTGACCTTGCCTTAATATATTTGTGAAGTTAAAATTTTAAAGA
TAGCTCATGAAACTTTTGCTTAAGCAAAAAGAAAACCTCGAATTGAAATGTGTGAGGCAAACTATGCATGGGAAT
AGCTTAATGTGAAGATAATCATTTGGACAACTCAAATCCATCAACATGACCAATGTTTTTCATCTGCCACATCTC
AAAATAAAACTTCTGGTGAAACAAATTAAACAAAATATCCAAACCTCAAAAAAAA

## FIGURE 12

Signal sequence: Amino acids 1-23

N-glycosylation sites: Amino acids 160-164;188-192

cAMP- and cGMP-dependent protein kinase phosphorylation site:
Amino acids 120-124

Tyrosine kinase phosphorylation sites: Amino acids 173-180;387-396

N-myristoylation sites: Amino acids 70-76;110-116;232-238
343-349;400-406;467-473;475-487

Fibrinogen beta and gamma chains C-terminal domain signature:
Amino acids 440-453

MKTFTWTLGVLFFLLVDTGHCRGGQFKIKKINQRRYPRATDGKEEAKKCAYTFLVPEQRITGPICVNTKGQDAST
IKDMITRMDLENLKDVLSRQKREIDVLQLVVDVDGNIVNEVKLLRKESRNMNSRVTQLYMQLLHEIIRKRDNSLE
LSQLENKILNVTTEMLKMATRYRELEVKYASLTDLVNNQSVMITLLEEQCLRIFSRQDTHVSPPLVQVVPQHIPN
SQQYTPGLLGGNEIQRDPGYPRDLMPPPDLATSPTKSPFKIPPVTFINEGPFKDCQQAKEAGHSVSGIYMIKPEN
SNGPMQLWCENSLDPGGWTVIQKRTDGSVNFFRNWENYKKGFGNIDGEYWLGLENIYMLSNQDNYKLLIELEDWS
DKKVYAEYSSFRLEPESEFYRLRLGTYQGNAGDSMMWHNGKQFTTLDRDKDMYAGNCAHFHKGGWWYNACAHSNL
NGVWYRGGHYRSKHQDGIFWAEYRGGSYSLRAVQMMIKPID

FIGURE 13

CGGACGCGTGGGGGAAACCCTTCCGAGAAAACAGCAACAAGCTGAGCTGCTGTGACAGAGGGGAACAAGATGGCG
GCGCCGAAGGGGAGCCTCTGGGTGAGGACCCAACTGGGGCTCCCGCCGCTGCTGCTGCTGACCATGGCCTTGGCC
GGAGGTTCGGGGACCGCTTCGGCTGAAGCATTTGACTCGGTCTTGGGTGATACGGCGTCTTGCCACCGGGCCTGT
CAGTTGACCTACCCCTTGCACACCTACCCTAAGGAAGAGGAGTTGTACGCATGTCAGAGAGGTTGCAGGCTGTTT
TCAATTTGTCAGTTTGTGGATGATGGAATTGACTTAAATCGAACTAAATTGGAATGTGAATCTGCATGTACAGAA
GCATATTCCCAATCTGATGAGCAATATGCTTGCCATCTTGGTTGCCAGAATCAGCTGCCATTCGCTGAACTGAGA
CAAGAACAACTTATGTCCCTGATGCCAAAAATGCACCTACTCTTTCCTCTAACTCTGGTGAGGTCATTCTGGAGT
GACATGATGGACTCCGCACAGAGCTTCATAACCTCTTCATGGACTTTTATCTTCAAGCCGATGACGGAAAAATA
GTTATATTCCAGTCTAAGCCAGAAATCCAGTACGCACCACATTTGGAGCAGGAGCCTACAAATTTGAGAGAATCA
TCTCTAAGCAAAATGTCCTATCTGCAAATGAGAAATTCACAAGCGCACAGGAATTTTCTTGAAGATGGAGAAAGT
GATGGCTTTTTAAGATGCCTCTCTCTTAACTCTGGGTGGATTTTAACTACAACTCTTGTCCTCTCGGTGATGGTA
TTGCTTTGGATTTGTTGTGCAACTGTTGCTACAGCTGTGGAGCAGTATGTTCCCTCTGAGAAGCTGAGTATCTAT
GGTGACTTGGAGTTTATGAATGAACAAAAGCTAAACAGATATCCAGCTTCTTCTCTTGTGGTTGTTAGATCTAAA
ACTGAAGATCATGAAGAAGCAGGGCCTCTACCTACAAAAGTGAATCTTGCTCATTCTGAAATTTAAGCATTTTTC
TTTTAAAAGACAAGTGTAATAGACATCTAAAATTCCACTCCTCATAGAGCTTTTAAAATGGTTTCATTGGATATA
GGCCTTAAGAAATCACTATAAAATGCAAATAAAGTTACTCAAATCTGTG

## FIGURE 14

| | |
|---|---|
| Signal sequence: | Amino acids 1-31 |
| Transmembrane domain: | Amino acids 242-262 |
| N-glycosylation site: | Amino acids 90-94 |
| N-myristoylation sites: | Amino acids 28-34;29-35;31-37;86-92 |

MAAPKGSLWVRTQLGLPPLLLLTMALAGGSGTASAEAFDSVLGDTASCHRACQLTYPLHTYPKEEELYACQRGCR
LFSICQFVDDGIDLNRTKLECESACTEAYSQSDEQYACHLGCQNQLPFAELRQEQLMSLMPKMHLLFPLTLVRSF
WSDMMDSAQSFITSSWTFYLQADDGKIVIFQSKPEIQYAPHLEQEPTNLRESSLSKMSYLQMRNSQAHRNFLEDG
ESDGFLRCLSLNSGWILTTTLVLSVMVLLWICCATVATAVEQYVPSEKLSIYGDLEFMNEQKLNRYPASSLVVVR
SKTEDHEEAGPLPTKVNLAHSEI

FIGURE 15

TCCGCAGGCGGACCGGGGGCAAAGGAGGTGGCATGTCGGTCAGGCACAGCAGGGTCCTGTGTCCGCGCTGAGCCG
CGCTCTCCCTGCTCCAGCAAGGACC**<u>ATG</u>**AGGGCGCTGGAGGGGCCAGGCCTGTCGCTGCTGTGCCTGGTGTTGGC
GCTGCCTGCCCTGCTGCCGGTGCCGGCTGTACGCGGAGTGGCAGAAACACCCACCTACCCCTGGCGGGACGCAGA
GACAGGGGAGCGGCTGGTGTGCGCCCAGTGCCCCCCAGGCACCTTTGTGCAGCGGCCGTGCCGCCGAGACAGCCC
CACGACGTGTGGCCCGTGTCCACCGCGCCACTACACGCAGTTCTGGAACTACCTGGAGCGCTGCCGCTACTGCAA
CGTCCTCTGCGGGGAGCGTGAGGAGGAGGCACGGGCTTGCCACGCCACCCACAACCGTGCCTGCCGCTGCCGCAC
CGGCTTCTTCGCGCACGCTGGTTTCTGCTTGGAGCACGCATCGTGTCCACCTGGTGCCGGCGTGATTGCCCCGGG
CACCCCCAGCCAGAACACGCAGTGCCAGCCGTGCCCCCCAGGCACCTTCTCAGCCAGCAGCTCCAGCTCAGAGCA
GTGCCAGCCCCACCGCAACTGCACGGCCCTGGGCCTGGCCCTCAATGTGCCAGGCTCTTCCTCCCATGACACCCT
GTGCACCAGCTGCACTGGCTTCCCCCTCAGCACCAGGGTACCAGGAGCTGAGGAGTGTGAGCGTGCCGTCATCGA
CTTTGTGGCTTTCCAGGACATCTCCATCAAGAGGCTGCAGCGGCTGCTGCAGGCCCTCGAGGCCCCGGAGGGCTG
GGGTCCGACACCAAGGGCGGGCCGCGCGGCCTTGCAGCTGAAGCTGCGTCGGCGGCTCACGGAGCTCCTGGGGGC
GCAGGACGGGGCGCTGCTGGTGCGGCTGCTGCAGGCGCTGCGCGTGGCCAGGATGCCCGGGCTGGAGCGGAGCGT
CCGTGAGCGCTTCCTCCCTGTGCAC**<u>TGA</u>**TCCTGGCCCCCTCTTATTTATTCTACATCCTTGGCACCCCACTTGCA
CTGAAAGAGGCTTTTTTTAAATAGAAGAAATGAGGTTTCTTAAAAAAAAAAAAAAAAAAAAA

## FIGURE 16

Signal sequence: Amino acids 1-23

N-glycosylation site: Amino acids 173-177

cAMP- and cGMP-dependent protein kinase phosphorylation sites:
Amino acids 63-67;259-263

Tyrosine kinase phosphorylation site: Amino acids 28-37

N-myristoylation sites: Amino acids 156-162;178-184;
207-213;266-272;287-293

MRALEGPGLSLLCLVLALPALLPVPAVRGVAETPTYPWRDAETGERLVCAQCPPGTFVQRPCRRDSPTTCGPCPP
RHYTQFWNYLERCRYCNVLCGEREEEARACHATHNRACRCRTGFFAHAGFCLEHASCPPGAGVIAPGTPSQNTQC
QPCPPGTFSASSSSSEQCQPHRNCTALGLALNVPGSSSHDTLCTSCTGFPLSTRVPGAEECERAVIDFVAFQDIS
IKRLQRLLQALEAPEGWGPTPRAGRAALQLKLRRRLTELLGAQDGALLVRLLQALRVARMPGLERSVRERFLPVH

FIGURE 17

CGGACGCGTGGGCGGACGCGTGGGCGGCCCACGGCGCCCGCGGGCTGGGGCGGTCGCTTCTTCCTTCTCCGTGGC
CTACGAGGGTCCCCAGCCTGGGTAAAG**ATG**GCCCCATGGCCCCCGAAGGGCCTAGTCCCAGCTGTGCTCTGGGGC
CTCAGCCTCTTCCTCAACCTCCCAGGACCTATCTGGCTCCAGCCCTCTCCACCTCCCCAGTCTTCTCCCCCGCCT
CAGCCCCATCCGTGTCATACCTGCCGGGGACTGGTTGACAGCTTTAACAAGGGCCTGGAGAGAACCATCCGGGAC
AACTTTGGAGGTGGAAACACTGCCTGGGAGGAAGAGAATTTGTCCAAATACAAAGACAGTGAGACCCGCCTGGTA
GAGGTGCTGGAGGGTGTGTGCAGCAAGTCAGACTTCGAGTGCCACCGCCTGCTGGAGCTGAGTGAGGAGCTGGTG
GAGAGCTGGTGGTTTCACAAGCAGCAGGAGGCCCCGGACCTCTTCCAGTGGCTGTGCTCAGATTCCCTGAAGCTC
TGCTGCCCCGCAGGCACCTTCGGGCCCTCCTGCCTTCCCTGTCCTGGGGGAACAGAGAGGCCCTGCGGTGGCTAC
GGGCAGTGTGAAGGAGAAGGGACACGAGGGGGCAGCGGGCACTGTGACTGCCAAGCCGGCTACGGGGGTGAGGCC
TGTGGCCAGTGTGGCCTTGGCTACTTTGAGGCAGAACGCAACGCCAGCCATCTGGTATGTTCGGCTTGTTTTGGC
CCCTGTGCCCGATGCTCAGGACCTGAGGAATCAAACTGTTTGCAATGCAAGAAGGGCTGGGCCCTGCATCACCTC
AAGTGTGTAGACATTGATGAGTGTGGCACAGAGGGAGCCAACTGTGGAGCTGACCAATTCTGCGTGAACACTGAG
GGCTCCTATGAGTGCCGAGACTGTGCCAAGGCCTGCCTAGGCTGCATGGGGGCAGGGCCAGGTCGCTGTAAGAAG
TGTAGCCCTGGCTATCAGCAGGTGGGCTCCAAGTGTCTCGATGTGGATGAGTGTGAGACAGAGGTGTGTCCGGGA
GAGAACAAGCAGTGTGAAAACACCGAGGGCGGTTATCGCTGCATCTGTGCCGAGGGCTACAAGCAGATGGAAGGC
ATCTGTGTGAAGGAGCAGATCCCAGAGTCAGCAGGCTTCTTCTCAGAGATGACAGAAGACGAGTTGGTGGTGCTG
CAGCAGATGTTCTTTGGCATCATCATCTGTGCACTGGCCACGCTGGCTGCTAAGGGCGACTTGGTGTTCACCGCC
ATCTTCATTGGGGCTGTGGCGGCCATGACTGGCTACTGGTTGTCAGAGCGCAGTGACCGTGTGCTGGAGGGCTTC
ATCAAGGGCAGA**TAA**TCGCGGCCACCACCTGTAGGACCTCCTCCCACCCACGCTGCCCCCAGAGCTTGGGCTGCC
CTCCTGCTGGACACTCAGGACAGCTTGGTTTATTTTTGAGAGTGGGGTAAGCACCCCTACCTGCCTTACAGAGCA
GCCCAGGTACCCAGGCCCGGGCAGACAAGGCCCCTGGGGTAAAAAGTAGCCCTGAAGGTGGATACCATGAGCTCT
TCACCTGGCGGGGACTGGCAGGCTTCACAATGTGTGAATTTCAAAAGTTTTTCCTTAATGGTGGCTGCTAGAGCT
TTGGCCCCTGCTTAGGATTAGGTGGTCCTCACAGGGGTGGGGCCATCACAGCTCCCTCCTGCCAGCTGCATGCTG
CCAGTTCCTGTTCTGTGTTCACCACATCCCCACACCCCATTGCCACTTATTTATTCATCTCAGGAAATAAAGAAA
GGTCTTGGAAAGTTAAAAAAAAAAAAAAAAAAAAAAA

## FIGURE 18

Signal sequence: Amino acids 1-29

Transmembrane domain: Amino acids 342-392

N-glycosylation sites: Amino acids 79-83;205-209

cAMP- and cGMP-dependent protein kinase phosphorylation site:
Amino acids 290-294

Aspartic acid and asparagine hydroxylation site:
Amino acids 321-333

EGF-like domain cysteine pattern signature:
Amino acids 181-193

MAPWPPKGLVPAVLWGLSLFLNLPGPIWLQPSPPPQSSPPPQPHPCHTCRGLVDSFNKGLERTIRDNFGGGNTAW
EEENLSKYKDSETRLVEVLEGVCSKSDFECHRLLELSEELVESWWFHKQQEAPDLFQWLCSDSLKLCCPAGTFGP
SCLPCPGGTERPCGGYGQCEGEGTRGGSGHCDCQAGYGGEACGQCGLGYFEAERNASHLVCSACFGPCARCSGPE
ESNCLQCKKGWALHHLKCVDIDECGTEGANCGADQFCVNTEGSYECRDCAKACLGCMGAGPGRCKKCSPGYQQVG
SKCLDVDECETEVCPGENKQCENTEGGYRCICAEGYKQMEGICVKEQIPESAGFFSEMTEDELVVLQQMFFGIII
CALATLAAKGDLVFTAIFIGAVAAMTGYWLSERSDRVLEGFIKGR

FIGURE 19

CCAGGCCGGGAGGCGACGCGCCCAGCCGTCTAAACGGGAACAGCCCTGGCTGAGGGAGCTGCAGCGCAGCAGAGT
ATCTGACGGCGCCAGGTTGCGTAGGTGCGGCACGAGGAGTTTTCCCGGCAGCGAGGAGGTCCTGAGCAGCATGGC
CCGGAGGAGCGCCTTCCCTGCCGCCGCGCTCTGGCTCTGGAGCATCCTCCTGTGCCTGCTGGCACTGCGGGCGGA
GGCCGGGCCGCCGCAGGAGGAGAGCCTGTACCTATGGATCGATGCTCACCAGGCAAGAGTACTCATAGGATTTGA
AGAAGATATCCTGATTGTTTCAGAGGGGAAAATGGCACCTTTTACACATGATTTCAGAAAAGCGCAACAGAGAAT
GCCAGCTATTCCTGTCAATATCCATTCCATGAATTTTACCTGGCAAGCTGCAGGGCAGGCAGAATACTTCTATGA
ATTCCTGTCCTTGCGCTCCCTGGATAAAGGCATCATGGCAGATCCAACCGTCAATGTCCCTCTGCTGGGAACAGT
GCCTCACAAGGCATCAGTTGTTCAAGTTGGTTTCCCATGTCTTGGAAAACAGGATGGGGTGGCAGCATTTGAAGT
GGATGTGATTGTTATGAATTCTGAAGGCAACACCATTCTCCAAACACCTCAAAATGCTATCTTCTTTAAAACATG
TCAACAAGCTGAGTGCCCAGGCGGGTGCCGAAATGGAGGCTTTTGTAATGAAAGACGCATCTGCGAGTGTCCTGA
TGGGTTCCACGGACCTCACTGTGAGAAAGCCCTTTGTACCCCACGATGTATGAATGGTGGACTTTGTGTGACTCC
TGGTTTCTGCATCTGCCCACCTGGATTCTATGGAGTGAACTGTGACAAAGCAAACTGCTCAACCACCTGCTTTAA
TGGAGGGACCTGTTTCTACCCTGGAAAATGTATTTGCCCTCCAGGACTAGAGGGAGAGCAGTGTGAAATCAGCAA
ATGCCCACAACCCTGTCGAAATGGAGGTAAATGCATTGGTAAAAGCAAATGTAAGTGTTCCAAAGGTTACCAGGG
AGACCTCTGTTCAAAGCCTGTCTGCGAGCCTGGCTGTGGTGCACATGGAACCTGCCATGAACCCAACAAATGCCA
ATGTCAAGAAGGTTGGCATGGAAGACACTGCAATAAAAGGTACGAAGCCAGCCTCATACATGCCCTGAGGCCAGC
AGGCGCCCAGCTCAGGCAGCACACGCCTTCACTTAAAAAGGCCGAGGAGCGGCGGGATCCACCTGAATCCAATTA
CATCTGGTGAACTCCGACATCTGAAACGTTTTAAGTTACACCAAGTTCATAGCCTTTGTTAACCTTTCATGTGTT
GAATGTTCAAATAATGTTCATTACACTTAAGAATACTGGCCTGAATTTTATTAGCTTCATTATAAATCACTGAGC
TGATATTTACTCTTCCTTTTAAGTTTTCTAAGTACGTCTGTAGCATGATGGTATAGATTTTCTTGTTTCAGTGCT
TTGGGACAGATTTTATATTATGTCAATTGATCAGGTTAAAATTTTCAGTGTGTAGTTGGCAGATATTTTCAAAAT
TACAATGCATTTATGGTGTCTGGGGGCAGGGGAACATCAGAAAGGTTAAATTGGGCAAAAATGCGTAAGTCACAA
GAATTTGGATGGTGCAGTTAATGTTGAAGTTACAGCATTTCAGATTTTATTGTCAGATATTTAGATGTTTGTTAC
ATTTTTAAAAATTGCTCTTAATTTTTAAACTCTCAATACAATATATTTTGACCTTACCATTATTCCAGAGATTCA
GTATTAAAAAAAAAAAAAATTACACTGTGGTAGTGGCATTTAAACAATATAATATATTCTAAACACAATGAAATAG
GGAATATAATGTATGAACTTTTTGCATTGGCTTGAAGCAATATAATATATTGTAAACAAAACACAGCTCTTACCT
AATAAACATTTTATACTGTTTGTATGTATAAAATAAAGGTGCTGCTTTAGTTTTTTGGAAAAAAAAAAAAAAAAAA
AAAAAAAA

## FIGURE 20

| | |
|---|---|
| Signal sequence: | Amino acids 1-28 |
| N-glycosylation sites: | Amino acids 88-92;245-249 |
| Tyrosine kinase phosphorylation site: | Amino acids 370-378 |
| N-myristoylation sites: | Amino acids 184-190;185-191; 189-195;315-321 |
| ATP/GTP-binding site motif A (P-loop): | Amino acids 285-293 |
| EGF-like domain cysteine pattern signatures: | Amino acids 198-210;230-242; 262-274;294-306;326-338 |

MARRSAFPAAALWLWSILLCLLALRAEAGPPQEESLYLWIDAHQARVLIGFEEDILIVSEGKMAPFTHDFRKAQQ
RMPAIPVNIHSMNFTWQAAGQAEYFYEFLSLRSLDKGIMADPTVNVPLLGTVPHKASVVQVGFPCLGKQDGVAAF
EVDVIVMNSEGNTILQTPQNAIFFKTCQQAECPGGCRNGGFCNERRICECPDGFHGPHCEKALCTPRCMNGGLCV
TPGFCICPPGFYGVNCDKANCSTTCFNGGTCFYPGKCICPPGLEGEQCEISKCPQPCRNGGKCIGKSKCKCSKGY
QGDLCSKPVCEPGCGAHGTCHEPNKCQCQEGWHGRHCNKRYEASLIHALRPAGAQLRQHTPSLKKAEERRDPPES
NYIW

FIGURE 21

CAGCGCGTGGCCGGCGCCGCTGTGGGGACAGC**<u>ATG</u>**AGCGGCGGTTGGATGGCGCAGGTTGGAGCGTGGCGAACAG
GGGCTCTGGGCCTGGCGCTGCTGCTGCTGCTCGGCCTCGGACTAGGCCTGGAGGCCGCCGCGAGCCCGCTTTCCACC
CCGACCTCTGCCCAGGCCGCAGGCCCCAGCTCAGGCTCGTGCCCACCCACCAAGTTCCAGTGCCGCACCAGTGGC
TTATGCGTGCCCCTCACCTGGCGCTGCGACAGGGACTTGGACTGCAGCGATGGCAGCGATGAGGAGGAGTGCAGG
ATTGAGCCATGTACCCAGAAAGGGCAATGCCCACCGCCCCCTGGCCTCCCCTGCCCCTGCACCGGCGTCAGTGAC
TGCTCTGGGGGAACTGACAAGAAACTGCGCAACTGCAGCCGCCTGGCCTGCCTAGCAGGCGAGCTCCGTTGCACG
CTGAGCGATGACTGCATTCCACTCACGTGGCGCTGCGACGGCCACCCAGACTGTCCCGACTCCAGCGACGAGCTC
GGCTGTGGAACCAATGAGATCCTCCCGGAAGGGGATGCCACAACCATGGGGCCCCCTGTGACCCTGGAGAGTGTC
ACCTCTCTCAGGAATGCCACAACCATGGGGCCCCCTGTGACCCTGGAGAGTGTCCCCTCTGTCGGGAATGCCACA
TCCTCCTCTGCCGGAGACCAGTCTGGAAGCCCAACTGCCTATGGGGTTATTGCAGCTGCTGCGGTGCTCAGTGCA
AGCCTGGTCACCGCCACCCTCCTCCTTTTGTCCTGGCTCCGAGCCCAGGAGCGCCTCCGCCCACTGGGGTTACTG
GTGGCCATGAAGGAGTCCCTGCTGCTGTCAGAACAGAAGACCTCGCTGCCC**<u>TGA</u>**GGACAAGCACTTGCCACCACC
GTCACTCAGCCCTGGGCGTAGCCGGACAGGAGGAGAGCAGTGATGCGGATGGGTACCCGGGCACACCAGCCCTCA
GAGACCTGAGTTCTTCTGGCCACGTGGAACCTCGAACCCGAGCTCCTGCAGAAGTGGCCCTGGAGATTGAGGGTCCC
TGGACACTCCCTATGGAGATCCGGGGAGCTAGGATGGGGAACCTGCCACAGCCAGAACTGAGGGGCTGGCCCCAG
GCAGCTCCCAGGGGGTAGAACGGCCCTGTGCTTAAGACACTCCCTGCTGCCCCGTCTGAGGGTGGCGATTAAAGT
TGCTTC

# FIGURE 22

Signal sequence: Amino acids 1-30

Transmembrane domain: Amino acids 231-248

N-glycosylation sites: Amino acids 126-130;195-199; 213-217

N-myristoylation sites: Amino acids 3-9;10-16;26-32; 30-36;112-118;166-172;212-218; 224-230;230-236;263-269

Prokaryotic membrane lipoprotein lipid attachment site: Amino acids 44-55

Leucine zipper pattern: Amino acids 17-39

MSGGWMAQVGAWRTGALGLALLLLLGLGLGLEAAASPLSTPTSAQAAGPSSGSCPPTKFQCRTSGLCVPLTWRCD
RDLDCSDGSDEEECRIEPCTQKGQCPPPPGLPCPCTGVSDCSGGTDKKLRNCSRLACLAGELRCTLSDDCIPLTW
RCDGHPDCPDSSDELGCGTNEILPEGDATTMGPPVTLESVTSLRNATTMGPPVTLESVPSVGNATSSSAGDQSGS
PTAYGVIAAAAVLSASLVTATLLLLSWLRAQERLRPLGLLVAMKESLLLSEQKTSLP

FIGURE 23

GGGGTCTCCCTCAGGGCCGGGAGGCACAGCGGTCCCTGCTTGCTGAAGGGCTGGATGTACGCATCCGCAGGTTCC
CGCGGACTTGGGGGCGCCCGCTGAGCCCCGGCGCCCGCAGAAGACTTGTGTTTGCCTCCTGCAGCCTCAACCCGG
AGGGCAGCGAGGGCCTACCACC<u>ATG</u>ATCACTGGTGTGTTCAGCATGCGCTTGTGGACCCCAGTGGGCGTCCTGAC
CTCGCTGGCGTACTGCCTGCACCAGCGGCGGGTGGCCCTGGCCGAGCTGCAGGAGGCCGATGGCCAGTGTCCGGT
CGACCGCAGCCTGCTGAAGTTGAAAATGGTGCAGGTCGTGTTTCGACACGGGGCTCGGAGTCCTCTCAAGCCGCT
CCCGCTGGAGGAGCAGGTAGAGTGGAACCCCCAGCTATTAGAGGTCCCACCCCAAACTCAGTTTGATTACACAGT
CACCAATCTAGCTGGTGGTCCGAAACCATATTCTCCTTACGACTCTCAATACCATGAGACCACCCTGAAGGGGGG
CATGTTTGCTGGGCAGCTGACCAAGGTGGGCATGCAGCAAATGTTTGCCTTGGGAGAGAGACTGAGGAAGAACTA
TGTGGAAGACATTCCCTTTCTTTCACCAACCTTCAACCCACAGGAGGTCTTTATTCGTTCCACTAACATTTTTCG
GAATCTGGAGTCCACCCGTTGTTTGCTGGCTGGGCTTTTCCAGTGTCAGAAAGAAGGACCCATCATCATCCACAC
TGATGAAGCAGATTCAGAAGTCTTGTATCCCAACTACCAAAGCTGCTGGAGCCTGAGGCAGAGAACCAGAGGCCG
GAGGCAGACTGCCTCTTTACAGCCAGGAATCTCAGAGGATTTGAAAAAGGTGAAGGACAGGATGGGCATTGACAG
TAGTGATAAAGTGGACTTCTTCATCCTCCTGGACAACGTGGCTGCCGAGCAGGCACACAACCTCCCAAGCTGCCC
CATGCTGAAGAGATTTGCACGGATGATCGAACAGAGAGCTGTGGACACATCCTTGTACATACTGCCCAAGGAAGA
CAGGGAAAGTCTTCAGATGGCAGTAGGCCCATTCCTCCACATCCTAGAGAGCAACCTGCTGAAAGCCATGGACTC
TGCCACTGCCCCCGACAAGATCAGAAAGCTGTATCTCTATGCGGCTCATGATGTGACCTTCATACCGCTCTTAAT
GACCCTGGGGATTTTTGACCACAAATGGCCACCGTTTGCTGTTGACCTGACCATGGAACTTTACCAGCACCTGGA
ATCTAAGGAGTGGTTTGTGCAGCTCTATTACCACGGGAAGGAGCAGGTGCCGAGAGGTTGCCCTGATGGGCTCTG
CCCGCTGGACATGTTCTTGAATGCCATGTCAGTTTATACCTTAAGCCCAGAAAAATACCATGCACTCTGCTCTCA
AACTCAGGTGATGGAAGTTGGAAATGAAGAG<u>TAA</u>CTGATTTATAAAAGCAGGATGTGTTGATTTTAAAATAAAGT
GCCTTTATACAATG

# FIGURE 24

| | |
|---|---|
| Signal sequence: | Amino acids 1-23 |
| cAMP- and cGMP-dependent protein kinase phosphorylation site: | Amino acids 218-222 |
| Tyrosine kinase phosphorylation site: | Amino acids 280-288 |
| N-myristoylation sites: | Amino acids 15-21;117-123; 118-124;179-185;240-246;387-393 |
| Amidation site: | Amino acids 216-220 |
| Leucine zipper pattern: | Amino acids 10-32 |
| Histidine acid phosphatases phosphohistidine signature: | Amino acids 50-65 |

MITGVFSMRLWTPVGVLTSLAYCLHQRRVALAELQEADGQCPVDRSLLKLKMVQVVFRHGARSPLKPLPLEEQVE
WNPQLLEVPPQTQFDYTVTNLAGGPKPYSPYDSQYHETTLKGGMFAGQLTKVGMQQMFALGERLRKNYVEDIPFL
SPTFNPQEVFIRSTNIFRNLESTRCLLAGLFQCQKEGPIIIHTDEADSEVLYPNYQSCWSLRQRTRGRRQTASLQ
PGISEDLKKVKDRMGIDSSDKVDFFILLDNVAAEQAHNLPSCPMLKRFARMIEQRAVDTSLYILPKEDRESLQMA
VGPFLHILESNLLKAMDSATAPDKIRKLYLYAAHDVTFIPLLMTLGIFDHKWPPFAVDLTMELYQHLESKEWFVQ
LYYHGKEQVPRGCPDGLCPLDMFLNAMSVYTLSPEKYHALCSQTQVMEVGNEE

FIGURE 25

CGAGGGCTTTTCCGGCTCCGGAATGGCACATGTGGGAATCCCAGTCTTGTTGGCTACAACATTTTTCCCTTTCCT
AACAAGTTCTAACAGCTGTTCTAACAGCTAGTGATCAGGGGTTCTTCTTGCTGGAGAAGAAAGGGCTGAGGGCAG
AGCAGGGCACTCTCACTCAGGGTGACCAGCTCCTTGCCTCTCTGTGGATAACAGAGCATGAGAAAGTGAAGAGAT
GCAGCGGAGTGAGGTGATGGAAGTCTAAAATAGGAAGGAATTTTGTGTGCAATATCAGACTCTGGGAGCAGTTGA
CCTGGAGAGCCTGGGGGAGGGCCTGCCTAACAAGCTTTCAAAAAACAGGAGCGACTTCCACTGGGCTGGGATAAG
ACGTGCCGGTAGGATAGGGAAGACTGGGTTTAGTCCTAATATCAAATTGACTGGCTGGGTGAACTTCAACAGCCT
TTTAACCTCTCTGGGAGATGAAAACGATGGCTTAAGGGGCCAGAAATAGAGATGCTTTGTAAAATAAAATTTTAA
AAAAAGCAAGTATTTTATAGCATAAAGGCTAGAGACCAAAATAGATAACAGGATTCCCTGAACATTCCTAAGAGG
GAGAAAGTATGTTAAAAATAGAAAAACCAAAATGCAGAAGGAGGAGACTCACAGAGCTAAACCAGG**ATG**GGGACC
CTGGGTCAGGCCAGCCTCTTTGCTCCTCCCGGAAATTATTTTTGGTCTGACCACTCTGCCTTGTGTTTTGCAGAA
TCATGTGAGGGCCAACCGGGGAAGGTGGAGCAGATGAGCACACACAGGAGCCGTCTCCTCACCGCCGCCCCTCTC
AGCATGGAACAGAGGCAGCCCTGGCCCCGGGCCCTGGAGGTGGACAGCCGCTCTGTGGTCCTGCTCTCAGTGGTC
TGGGTGCTGCTGGCCCCCCCAGCAGCCGGCATGCCTCAGTTCAGCACCTTCCACTCTGAGAATCGTGACTGGACC
TTCAACCACTTGACCGTCCACCAAGGGACGGGGGCCGTCTATGTGGGGGCCATCAACCGGGTCTATAAGCTGACA
GGCAACCTGACCATCCAGGTGGCTCATAAGACAGGGCCAGAAGAGGACAACAAGTCTCGTTACCCGCCCCTCATC
GTGCAGCCCTGCAGCGAAGTGCTCACCCTCACCAACAATGTCAACAAGCTGCTCATCATTGACTACTCTGAGAAC
CGCCTGCTGGCCTGTGGGAGCCTCTACCAGGGGGTCTGCAAGCTGCTGCGGCTGGATGACCTCTTCATCCTGGTG
GAGCCATCCCACAAGAAGGAGCACTACCTGTCCAGTGTCAACAAGACGGGCACCATGTACGGGGTGATTGTGCGC
TCTGAGGGTGAGGATGGCAAGCTCTTCATCGGCACGGCTGTGGATGGGAAGCAGGATTACTTCCCGACCCTGTCC
AGCCGGAAGCTGCCCCGAGACCCTGAGTCCTCAGCCATGCTCGACTATGAGCTACACAGCGATTTTGTCTCCTCT
CTCATCAAGATCCCTTCAGACACCCTGGCCCTGGTCTCCCACTTTGACATCTTCTACATCTACGGCTTTGCTAGT
GGGGGCTTTGTCTACTTTCTCACTGTCCAGCCCGAGACCCCTGAGGGTGTGGCCATCAACTCCGCTGGAGACCTC
TTCTACACCTCACGCATCGTGCGGCTCTGCAAGGATGACCCCAAGTTCCACTCATACGTGTCCCTGCCCTTCGGC
TGCACCCGGGCCGGGGTGGAATACCGCCTCCTGCAGGCTGCTTACCTGGCCAAGCCTGGGGACTCACTGGCCCAG
GCCTTCAATATCACCAGCCAGGACGATGTACTCTTTGCCATCTTCTCCAAAGGGCAGAAGCAGTATCACCACCCG
CCCGATGACTCTGCCCTGTGTGCCTTCCCTATCCGGGCCATCAACTTGCAGATCAAGGAGCGCCTGCAGTCCTGC
TACCAGGGCGAGGGCAACCTGGAGCTCAACTGGCTGCTGGGGAAGGACGTCCAGTGCACGAAGGCGCCTGTCCCC
ATCGATGATAACTTCTGTGGACTGGACATCAACCAGCCCCTGGGAGGCTCAACTCCAGTGGAGGGCCTGACCCTG
TACACCACCAGCAGGGACCGCATGACCTCTGTGGCCTCCTACGTTTACAACGGCTACAGCGTGGTTTTTGTGGGG
ACTAAGAGTGGCAAGCTGAAAAAGGTAAGAGTCTATGAGTTCAGATGCTCCAATGCCATTCACCTCCTCAGCAAA
GAGTCCCTCTTGGAAGGTAGCTATTGGTGGAGATTTAACTATAGGCAACTTTATTTTCTTGGGAACAAAGG**TGA**
AATGGGGAGGTAAGAAGGGGTTAATTTTGTGACTTAGCTTCTAGCTACTTCCTCCAGCCATCAGTCATTGGGTATG
TAAGGAATGCAAGCGTATTTCAATATTTCCCAAACTTTAAGAAAAAACTTTAAGAAGGTACATCTGCAAAAGCAAA

## FIGURE 26

| | |
|---|---|
| Signal sequence: | Amino acids 1-32 |
| Transmembrane domain: | Amino acids 71-86 |
| N-glycosylation sites: | Amino acids 130-134;145-149;<br>217-221;380-385 |
| N-myristoylation sites: | Amino acids 220-226;319-325;<br>353-359;460-466;503-509 |

MGTLGQASLFAPPGNYFWSDHSALCFAESCEGQPGKVEQMSTHRSRLLTAAPLSMEQRQPWPRALEVDSRSVVLL
SVVWVLLAPPAAGMPQFSTFHSENRDWTFNHLTVHQGTGAVYVGAINRVYKLTGNLTIQVAHKTGPEEDNKSRYP
PLIVQPCSEVLTLTNNVNKLLIIDYSENRLLACGSLYQGVCKLLRLDDLFILVEPSHKKEHYLSSVNKTGTMYGV
IVRSEGEDGKLFIGTAVDGKQDYFPTLSSRKLPRDPESSAMLDYELHSDFVSSLIKIPSDTLALVSHFDIFYIYG
FASGGFVYFLTVQPETPEGVAINSAGDLFYTSRIVRLCKDDPKFHSYVSLPFGCTRAGVEYRLLQAAYLAKPGDS
LAQAFNITSQDDVLFAIFSKGQKQYHHPPDDSALCAFPIRAINLQIKERLQSCYQGEGNLELNWLLGKDVQCTKA
PVPIDDNFCGLDINQPLGGSTPVEGLTLYTTSRDRMTSVASYVYNGYSVVFVGTKSGKLKKVRVYEFRCSNAIHL
LSKESLLEGSYWWRFNYRQLYFLGEQR

FIGURE 27

CCCAGAAGTTCAAGGGCCCCCGGCCTCCTGCGCTCCTGCCGCCGGGACCCTCGACCTCCTCAGAGCAGCCGGCTG
CCGCCCCGGGAAG**ATG**GCGAGGAGGAGCCGCCACCGCCTCCTCCTGCTGCTGCTGCGCTACCTGGTGGTCGCCCT
GGGCTATCATAAGGCCTATGGGTTTTCTGCCCCAAAAGACCAACAAGTAGTCACAGCAGTAGAGTACCAAGAGGC
TATTTTAGCCTGCAAAACCCCAAAGAAGACTGTTTCCTCCAGATTAGAGTGGAAGAAACTGGGTCGGAGTGTCTC
CTTTGTCTACTATCAACAGACTCTTCAAGGTGATTTTAAAAATCGAGCTGAGATGATAGATTTCAATATCCGGAT
CAAAAATGTGACAAGAAGTGATGCGGGGAAATATCGTTGTGAAGTTAGTGCCCCATCTGAGCAAGGCCAAAACCT
GGAAGAGGATACAGTCACTCTGGAAGTATTAGTGGCTCCAGCAGTTCCATCATGTGAAGTACCCTCTTCTGCTCT
GAGTGGAACTGTGGTAGAGCTACGATGTCAAGACAAAGAAGGGAATCCAGCTCCTGAATACACATGGTTTAAGGA
TGGCATCCGTTTGCTAGAAAATCCCAGACTTGGCTCCCAAAGCACCAACAGCTCATACACAATGAATACAAAAAC
TGGAACTCTGCAATTTAATACTGTTTCCAAACTGGACACTGGAGAATATTCCTGTGAAGCCCGCAATTCTGTTGG
ATATCGCAGGTGTCCTGGGAAACGAATGCAAGTAGATGATCTCAACATAAGTGGCATCATAGCAGCCGTAGTAGTTGT
GGCCTTAGTGATTTCCGTTTGTGGCCTTGGTGTATGCTATGCTCAGAGGAAAGGCTACTTTTCAAAAGAAACCTC
CTTCCAGAAGAGTAATTCTTCATCTAAAGCCACGACAATGAGTGAAAATGTGCAGTGGCTCACGCCTGTAATCCC
AGCACTTTGGAAGGCCGCGGCGGGCGGATCACGAGGTCAGGAGTTC**TAG**ACCAGTCTGGCCAATATGGTGAAACC
CCATCTCTACTAAAATACAAAAATTAGCTGGGCATGGTGGCATGTGCCTGCAGTTCCAGCTGCTTGGGAGACAGG
AGAATCACTTGAACCCGGGAGGCGGAGGTTGCAGTGAGCTGAGATCACGCCACTGCAGTCCAGCCTGGGTAACAG
AGCAAGATTCCATCTCAAAAAATAAAATAAATAAATAAATAAATACTGGTTTTTACCTGTAGAATTCTTACAATA
AATATAGCTTGATATTC

## FIGURE 28

| | |
|---|---|
| signal sequence: | Amino acids 1-20 |
| Transmembrane domain: | Amino acids 237-258 |
| N-glycosylation sites: | Amino acids 98-102;187-191; 236-240;277-281 |
| N-myristoylation sites: | Amino acids 182-188;239-245; 255-261;257-263;305-311 |
| Amidation site: | Amino acids 226-230 |

MARRSRHRLLLLLLRYLVVALGYHKAYGFSAPKDQQVVTAVEYQEAILACKTPKKTVSSRLEWKKLGRSVSFVYY
QQTLQGDFKNRAEMIDFNIRIKNVTRSDAGKYRCEVSAPSEQGQNLEEDTVTLEVLVAPAVPSCEVPSSALSGTV
VELRCQDKEGNPAPEYTWFKDGIRLLENPRLGSQSTNSSYTMNTKTGTLQFNTVSKLDTGEYSCEARNSVGYRRC
PGKRMQVDDLNISGIIAAVVVVALVISVCGLGVCYAQRKGYFSKETSFQKSNSSSKATTMSENVQWLTPVIPALW
KAAAGGSRGQEF

FIGURE 29

GCTGGGGAC<u>ATG</u>AGAGGCACACCGAAGACCCACCTCCTGGCCTTCTCCCTCCTCTGCCTCCTCTCAAAGGTGCGT
ACCCAGCTGTGCCCGACACCATGTACCTGCCCCTGGCCACCTCCCCGATGCCCGCTGGGAGTACCCCTGGTGCTG
GATGGCTGTGGCTGCTGCCGGGTATGTGCACGGCGGCTGGGGGAGCCCTGCGACCAACTCCACGTCTGCGACGCC
AGCCAGGGCCTGGTCTGCCAGCCCGGGGCAGGACCCGGTGGCCGGGGGGCCCTGTGCCTCTTGGCAGAGGACGAC
AGCAGCTGTGAGGTGAACGGCCGCCTGTATCGGGAAGGGGAGACCTTCCAGCCCCACTGCAGCATCCGCTGCCGC
TGCGAGGACGGCGGCTTCACCTGCGTGCCGCTGTGCAGCGAGGATGTGCGGCTGCCCAGCTGGGACTGCCCCCAC
CCCAGGAGGGTCGAGGTCCTGGGCAAGTGCTGCCCTGAGTGGGTGTGCGGCCAAGGAGGGGGACTGGGGACCCAG
CCCCTTCCAGCCCAAGGACCCCAGTTTTCTGGCCTTGTCTCTTCCCTGCCCCCTGGTGTCCCCTGCCCAGAATGG
AGCACGGCCTGGGGACCCTGCTCGACCACCTGTGGGCTGGGCATGGCCACCCGGGTGTCCAACCAGAACCGCTTC
TGCCGACTGGAGACCCAGCGCCGCCTGTGCCTGTCCAGGCCCTGCCCACCCTCCAGGGGTCGCAGTCCACAAAAC
AGTGCCTTC<u>TAG</u>AGCCGGGCTGGGAATGGGGACACGGTGTCCACCATCCCCAGCTGGTGGCCCTGTGCCTGGGCC
CTGGGCTGATGGAAGATGGTCCGTGCCCAGGCCCTTGGCTGCAGGCAACACTTTAGCTTGGGTCCACCATGCAGA
ACACCAATATTAACACGCTGCCTGGTCTGTCTGGATCCCGAGGTATGGCAGAGGTGCAAGACCTAGTCCCCTTTC
CTCTAACTCACTGCCTAGGAGGCTGGCCAAGGTGTCCAGGGTCCTCTAGCCCACTCCCTGCCTACACACACAGCC
TATATCAAACATGCACACGGGCGAGCTTTCTCTCCGACTTCCCCTGGGCAAGAGATGGGACAAGCAGTCCCTTAA
TATTGAGGCTGCAGCAGGTGCTGGGCTGGACTGGCCATTTTTCTGGGGGTAGGATGAAGAGAAGGCACACAGAGA
TTCTGGATCTCCTGCTGCCTTTTCTGGAGTTTGTAAAATTGTTCCTGAATACAAGCCTATGCGTGA

## FIGURE 30

| | |
|---|---|
| Signal sequence: | Amino acids 1-23 |
| N-myristoylation sites: | Amino acids 3-9;49-55;81-87; 85-91;126-132;164-170;166-172; 167-173;183-189;209-215 |
| Insulin-like growth factor binding proteins signature: | Amino acids 49-65 |
| von Willebrand C1 domain: | Amino acids 107-124 |
| Thrombospondin 1 Homology Block: | Amino acids 201-216 |
| IGF binding protein site: | Amino acids 49-58 |

MRGTPKTHLLAFSLLCLLSKVRTQLCPTPCTCPWPPPRCPLGVPLVLDGCGCCRVCARRLGEPCDQLHVCDASQG
LVCQPGAGPGGRGALCLLAEDDSSCEVNGRLYREGETFQPHCSIRCRCEDGGFTCVPLCSEDVRLPSWDCPHPRR
VEVLGKCCPEWVCGQGGGLGTQPLPAQGPQFSGLVSSLPPGVPCPEWSTAWGPCSTTCGLGMATRVSNQNRFCRL
ETQRRLCLSRPCPPSRGRSPQNSAF

FIGURE 31

AGTCGACTGCGTCCCCTGTACCCGGCGCCAGCTGTGTTCCTGACCCCAGAATAACTCAGGGCTGCACCGGGCCTG
GCAGCGCTCCGCACACATTTCCTGTCGCGGCCTAAGGGAAACTGTTGGCCGCTGGGCCCGCGGGGGATTCTTGG
CAGTTGGGGGGTCCGTCGGGAGCGAGGGCGGAGGGGAAGGGAGGGGGAACCGGGTTGGGGAAGCCAGCTGTAGAG
GGCGGTGACCGCGCTCCAGACACAGCTCTGCGTCCTCGAGCGGGACAGATCCAAGTTGGGAGCAGCTCTGCGTGC
GGGGCCTCAGAGA**ATG**AGGCCGGCGTTCGCCCTGTGCCTCCTCTGGCAGGCGCTCTGGCCCGGGCCGGGCGGCGG
CGAACACCCCACTGCCGACCGTGCTGGCTGCTCGGCCTCGGGGGCCTGCTACAGCCTGCACCACGCTACCATGAA
GCGGCAGGCGGCCGAGGAGGCCTGCATCCTGCGAGGTGGGGCGCTCAGCACCGTGCGTGCGGGCGCCGAGCTGCG
CGCTGTGCTCGCGCTCCTGCGGGCAGGCCCAGGGCCCGGAGGGGGCTCCAAAGACCTGCTGTTCTGGGTCGCACT
GGAGCGCAGGCGTTCCCACTGCACCCTGGAGAACGAGCCTTTGCGGGGTTTCTCCTGGCTGTCCTCCGACCCCGG
CGGTCTCGAAAGCGACACGCTGCAGTGGGTGGAGGAGCCCCAACGCTCCTGCACCGCGCGGAGATGCGCGGTACT.
CCAGGCCACCGGTGGGGTCGAGCCCGCAGGCTGGAAGGAGATGCGATGCCACCTGCGCGCCAACGGCTACCTGTG
CAAGTACCAGTTTGAGGTCTTGTGTCCTGCGCCGCGCCCCGGGGCCGCCTCTAACTTGAGCTATCGCGCGCCCTT
CCAGCTGCACAGCGCCGCTCTGGACTTCAGTCCACCTGGGACCGAGGTGAGTGCGCTCTGCCGGGGACAGCTCCC
GATCTCAGTTACTTGCATCGCGGACGAAATCGGCGCTCGCTGGGACAAACTCTCGGGCGATGTGTTGTGTCCCTG
CCCCGGGAGGTACCTCCGTGCTGGCAAATGCGCAGAGCTCCCTAACTGCCTAGACGACTTGGGAGGCTTTGCCTG
CGAATGTGCTACGGGCTTCGAGCTGGGGAAGGACGGCCGCTCTTGTGTGACCAGTGGGGAAGGACAGCCGACCCT
TGGGGGGACCGGGGTGCCCACCAGGCGCCCGCCGGCCACTGCAACCAGCCCCGTGCCGCAGAGAACATGGCCAAT
CAGGGTCGACGAGAAGCTGGGAGAGACACCACTTGTCCCTGAACAAGACAATTCAGTAACATCTATTCCTGAGAT
TCCTCGATGGGGATCACAGAGCACGATGTCTACCCTTCAAATGTCCCTTCAAGCCGAGTCAAAGGCCACTATCAC
CCCATCAGGGAGCGTGATTTCCAAGTTTAATTCTACGACTTCCTCTGCCACTCCTCAGGCTTTCGACTCCTCCTC
TGCCGTGGTCTTCATATTTGTGAGCACAGCAGTAGTAGTGTTGGTGATCTTGACCATGACAGTACTGGGGCTTGT
CAAGCTCTGCTTTCACGAAAGCCCCTCTTCCCAGCCAAGGAAGGAGTCTATGGGCCCGCCGGGCCTGGAGAGTGA
TCCTGAGCCCGCTGCTTTGGGCTCCAGTTCTGCACATTGCACAAACAATGGGGTGAAAGTCGGGGACTGTGATCT
GCGGGACAGAGCAGAGGGTGCCTTGCTGGCGGAGTCCCCTCTTGGCTCTAGTGATGCA**TAG**GGAAACAGGGGACA
TGGGCACTCCTGTGAACAGTTTTTCACTTTTGATGAAACGGGGAACCAAGAGGAACTTACTTGTGTAACTGACAA
TTTCTGCAGAAATCCCCCTTCCTCTAAATTCCCTTTACTCCACTGAGGAGCTAAATCAGAACTGCACACTCCTTC
CCTGATGATAGAGGAAGTGGAAGTGCCTTTAGGATGGTGATACTGGGGACCGGGTAGTGCTGGGGAGAGATATT
TTCTTATGTTTATTCGGAGAATTTGGAGAAGTGATTGAACTTTTCAAGACATTGGAAACAAATAGAACACAATAT
AATTTACATTAAAAAATAATTTCTACCAAAATGGAAAGGAAATGTTCTATGTTGTTCAGGCTAGGAGTATATTGG
TTCGAAATCCCAGGGAAAAAAATAAAAATAAAAAATTAAAGGATTGTTGAT

# FIGURE 32

| | |
|---|---|
| Signal sequence: | Amino acids 1-16 |
| Transmembrane domain: | Amino acids 397-418 |
| N-glycosylation sites: | Amino acids 189-193;381-385 |
| Glycosaminoglycan attachment site: | Amino acids 289-293 |
| cAMP- and cGMP-dependent protein kinase phosphorylation sites: | Amino acids 98-102;434-438 |
| N-myristoylation sites: | Amino acids 30-36;35-41;58-64; 59-65;121-127;151-157;185-191; 209-215;267-273;350-356;374-380; 453-459;463-469;477-483 |
| Aspartic acid and asparagine hydroxylation site: | Amino acids 262-274 |

MRPAFALCLLWQALWPGPGGGEHPTADRAGCSASGACYSLHHATMKRQAAEEACILRGGALSTVRAGAELRAVLA
LLRAGPGPGGGSKDLLFWVALERRRSHCTLENEPLRGFSWLSSDPGGLESDTLQWVEEPQRSCTARRCAVLQATG
GVEPAGWKEMRCHLRANGYLCKYQFEVLCPAPRPGAASNLSYRAPFQLHSAALDFSPPGTEVSALCRGQLPISVT
CIADEIGARWDKLSGDVLCPCPGRYLRAGKCAELPNCLDDLGGFACECATGFELGKDGRSCVTSGEGQPTLGGTG
VPTRRPPATATSPVPQRTWPIRVDEKLGETPLVPEQDNSVTSIPEIPRWGSQSTMSTLQMSLQAESKATITPSGS
VISKFNSTTSSATPQAFDSSSAVVFIFVSTAVVVLVILTMTVLGLVKLCFHESPSSQPRKESMGPPGLESDPEPA
ALGSSSAHCTNNGVKVGDCDLRDRAEGALLAESPLGSSDA

FIGURE 33

CGGACGCGTGGGATTCAGCAGTGGCCTGTGGCTGCCAGAGCAGCTCCTCAGGGGAAACTAAGCGTCGAGTCAGAC
GGCACCATAATCGCCTTTAAAAGTGCCTCCGCCCTGCCGGCCGCGTATCCCCCGGCTACCTGGGCCGCCCCGCGG
CGGTGCGCGCGTGAGAGGGAGCGCGCGGGCAGCCGAGCGCCGGTGTGAGCCAGCGCTGCTGCCAGTGTGAGCGGC
GGTGTGAGCGCGGTGGGTGCGGAGGGGCGTGTGTGCCGGCGCGCGCGCCGTGGGGTGCAAACCCCGAGCGTCTAC
GCTGCC**ATG**AGGGGCGCGAACGCCTGGGCGCCACTCTGCCTGCTGCTGGCTGCCGCCACCCAGCTCTCGCGGCAG
CAGTCCCCAGAGAGACCTGTTTTCACATGTGGTGGCATTCTTACTGGAGAGTCTGGATTTATTGGCAGTGAAGGT
TTTCCTGGAGTGTACCCTCCAAATAGCAAATGTACTTGGAAAATCACAGTTCCCGAAGGAAAAGTAGTCGTTCTC
AATTTCCGATTCATAGACCTCGAGAGTGACAACCTGTGCCGCTATGACTTTGTGGATGTGTACAATGGCCATGCC
AATGGCCAGCGCATTGGCCGCTTCTGTGGCACTTTCCGGCCTGGAGCCCTTGTGTCCAGTGGCAACAAGATGATG
GTGCAGATGATTTCTGATGCCAACACAGCTGGCAATGGCTTCATGGCCATGTTCTCCGCTGCTGAACCAAACGAA
AGAGGGGATCAGTATTGTGGAGGACTCCTTGACAGACCTTCCGGCTCTTTTAAAACCCCCAACTGGCCAGACCGG
GATTACCCTGCAGGAGTCACTTGTGTGTGGCACATTGTAGCCCCAAAGAATCAGCTTATAGAATTAAAGTTTGAG
AAGTTTGATGTGGAGCGAGATAACTACTGCCGATATGATTATGTGGCTGTGTTTAATGGCGGGGAAGTCAACGAT
GCTAGAAGAATTGGAAAGTATTGTGGTGATAGTCCACCTGCGCCAATTGTGTCTGAGAGAAATGAACTTCTTATT
CAGTTTTTATCAGACTTAAGTTTAACTGCAGATGGGTTTATTGGTCACTACATATTCAGGCCAAAAAAACTGCCT
ACAACTACAGAACAGCCTGTCACCACCACATTCCCTGTAACCACGGGTTTAAAACCCACCGTGGCCTTGTGTCAA
CAAAAGTGTAGACGGACGGGGACTCTGGAGGGCAATTATTGTTCAAGTGACTTTGTATTAGCCGGCACTGTTATC
ACAACCATCACTCGCGATGGGAGTTTGCACGCCACAGTCTCGATCATCAACATCTACAAAGAGGGAAATTTGGCG
ATTCAGCAGGCGGGCAAGAACATGAGTGCCAGGCTGACTGTCGTCTGCAAGCAGTGCCCTCTCCTCAGAAGAGGT
CTAAATTACATTATTATGGGCCAAGTAGGTGAAGATGGGCGAGGCAAAATCATGCCAAACAGCTTTATCATGATG
TTCAAGACCAAGAATCAGAAGCTCCTGGATGCCTTAAAAAATAAGCAATGT**TAA**CAGTGAACTGTGTCCATTTAA
GCTGTATTCTGCCATTGCCTTTGAAAGATCTATGTTCTCTCAGTAGAAAAAAAAATACTTATAAAATTACATATT
CTGAAAGAGGATTCCGAAAGATGGACTGGTTGACTCTTCACATGATGGAGGTATGAGGCCTCCGAGATAGCTGA
GGGAAGTTCTTTGCCTGCTGTCAGAGGAGCAGCTATCTGATTGGAAACCTGCCGACTTAGTGCGGTGATAGGAAGC
TAAAAGTGTCAAGCGTTGACAGCTTGGAAGCGTTTATTTATACATCTCTGTAAAAGGATATTTTAGAATTGAGTT
GTGTGAAGATGTCAAAAAAAGATTTTAGAAGTGCAATATTTATAGTGTTATTTGTTTCACCTTCAAGCCTTTGCC
CTGAGGTGTTACAATCTTGTCTTGCGTTTTCTAAATCAATGCTTAATAAAATATTTTTAAAGGAAAAAAAAAAAA

# FIGURE 34

| | |
|---|---|
| Signal sequence: | Amino acids 1-23 |
| N-glycosylation site: | Amino acids 355-359 |
| Tyrosine kinase phosphorylation site: | Amino acids 199-208 |
| N-myristoylation sites: | Amino acids 34-40;35-41;100-106;<br>113-119;218-224;289-295;305-311;<br>309-315;320-326;330-336 |
| Cell attachment sequence: | Amino acids 149-152 |

MRGANAWAPLCLLLAAATQLSRQQSPERPVFTCGGILTGESGFIGSEGFPGVYPPNSKCTWKITVPEGKVVVLNF
RFIDLESDNLCRYDFVDVYNGHANGQRIGRFCGTFRPGALVSSGNKMMVQMISDANTAGNGFMAMFSAAEPNERG
DQYCGGLLDRPSGSFKTPNWPDRDYPAGVTCVWHIVAPKNQLIELKFEKFDVERDNYCRYDYVAVFNGGEVNDAR
RIGKYCGDSPPAPIVSERNELLIQFLSDLSLTADGFIGHYIFRPKKLPTTTEQPVTTTFPVTTGLKPTVALCQQK
CRRTGTLEGNYCSSDFVLAGTVITTITRDGSLHATVSIINIYKEGNLAIQQAGKNMSARLTVVCKQCPLLRRGLN
YIIMGQVGEDGRGKIMPNSFIMMFKTKNQKLLDALKNKQC

FIGURE 35

GTCTGTTCCCAGGAGTCCTTCGGCGGCTGTTGTGTCAGTGGCCTGATCGCGATGGGGACAAAGGCGCAAGTCGAG
AGGAAACTGTTGTGCCTCTTCATATTGGCGATCCTGTTGTGCTCCCTGGCATTGGGCAGTGTTACAGTGCACTCT
TCTGAACCTGAAGTCAGAATTCCTGAGAATAATCCTGTGAAGTTGTCCTGTGCCTACTCGGGCTTTTCTTCTCCC
CGTGTGGAGTGGAAGTTTGACCAAGGAGACACCACCAGACTCGTTTGCTATAATAACAAGATCACAGCTTCCTAT
GAGGACCGGGTGACCTTCTTGCCAACTGGTATCACCTTCAAGTCCGTGACACGGGAAGACACTGGGACATACACT
TGTATGGTCTCTGAGGAAGGCGGCAACAGCTATGGGGAGGTCAAGGTCAAGCTCATCGTGCTTGTGCCTCCATCC
AAGCCTACAGTTAACATCCCCTCCTCTGCCACCATTGGGAACCGGGCAGTGCTGACATGCTCAGAACAAGATGGT
TCCCCACCTTCTGAATACACCTGGTTCAAAGATGGGATAGTGATGCCTACGAATCCCAAAAGCACCCGTGCCTTC
AGCAACTCTTCCTATGTCCTGAATCCCACAACAGGAGAGCTGGTCTTTGATCCCCTGTCAGCCTCTGATACTGGA
GAATACAGCTGTGAGGCACGGAATGGGTATGGGACACCCATGACTTCAAATGCTGTGCGCATGGAAGCTGTGGAG
CGGAATGTGGGGGTCATCGTGGCAGCCGTCCTTGTAACCCTGATTCTCCTGGGAATCTTGGTTTTTGGCATCTGG
TTTGCCTATAGCCGAGGCCACTTTGACAGAACAAAGAAAGGGACTTCGAGTAAGAAGGTGATTTACAGCCAGCCT
AGTGCCCGAAGTGAAGGAGAATTCAAACAGACCTCGTCATTCCTGGTGTGAGCCTGGTCGGCTCACCGCCTATCA
TCTGCATTTGCCTTACTCAGGTGCTACCGGACTCTGGCCCCTGATGTCTGTAGTTTCACAGGATGCCTTATTTGT
CTTCTACACCCCACAGGGCCCCCTACTTCTTCGGATGTGTTTTTAATAATGTCAGCTATGTGCCCCATCCTCCTT
CATGCCCTCCCTCCCTTTCCTACCACTGCTGAGTGGCCTGGAACTTGTTTAAAGTGTTTATTCCCCATTTCTTTG
AGGGATCAGGAAGGAATCCTGGGTATGCCATTGACTTCCCTTCTAAGTAGACAGCAAAAATGGCGGGGGTCGCAG
GAATCTGCACTCAACTGCCCACCTGGCTGGCAGGGATCTTTGAATAGGTATCTTGAGCTTGGTTCTGGGCTCTTT
CCTTGTGTACTGACGACCAGGGCCAGCTGTTCTAGAGCGGGAATTAGAGGCTAGAGCGGCTGAAATGGTTGTTTG
GTGATGACACTGGGGTCCTTCCATCTCTGGGGCCCACTCTCTTCTGTCTTCCCATGGGAAGTGCCACTGGGATCC
CTCTGCCCTGTCCTCCTGAATACAAGCTGACTGACATTGACTGTGTCTGTGGAAAATGGGAGCTCTTGTTGTGGA
GAGCATAGTAAATTTTCAGAGAACTTGAAGCCAAAAGGATTTAAAACCGCTGCTCTAAAGAAAAGAAAACTGGAG
GCTGGGCGCAGTGGCTCACGCCTGTAATCCCAGAGGCTGAGGCAGGCGGATCACCTGAGGTCGGGAGTTCGGGAT
CAGCCTGACCAACATGGAGAAACCCTACTGGAAATACAAAGTTAGCCAGGCATGGTGGTGCATGCCTGTAGTCCC
AGCTGCTCAGGAGCCTGGCAACAAGAGCAAAACTCCAGCTCAAAAAAAAAAAAAAAA

FIGURE 36

Signal sequence: Amino acids 1-27

Transmembrane domain: Amino acids 235-256

N-glycosylation site: Amino acids 185-189

cAMP- and cGMP-dependent protein kinase phosphorylation site: Amino acids 270-274

N-myristoylation sites: Amino acids 105-111;116-122; 158-164;219-225;237-243;256-262

MGTKAQVERKLLCLFILAILLCSLALGSVTVHSSEPEVRIPENNPVKLSCAYSGFSSPRVEWKFDQGDTTRLVCY
NNKITASYEDRVTFLPTGITFKSVTREDTGTYTCMVSEEGGNSYGEVKVKLIVLVPPSKPTVNIPSSATIGNRAV
LTCSEQDGSPPSEYTWFKDGIVMPTNPKSTRAFSNSSYVLNPTTGELVFDPLSASDTGEYSCEARNGYGTPMTSN
AVRMEAVERNVGVIVAAVLVTLILLGILVFGIWFAYSRGHFDRTKKGTSSKKVIYSQPSARSEGEFKQTSSFLV

FIGURE 37

GCTGAGTCTGCTGCTCCTGCTGCTGCTGCTCCAGCCTGTAACCTGTGCCTACACCACGCCAGGCCCCCCAGAGC
CCTCACCACGCTGGGCGCCCCCAGAGCCCACACC<u>ATG</u>CCGGGCACCTACGCTCCCTCGACCACACTCAGTAGTCC
CAGCACCCAGGGCCTGCAAGAGCAGGCACGGGCCCTGATGCGGGACTTCCCGCTCGTGGACGGCCACAACGACCT
GCCCCTGGTCCTAAGGCAGGTTTACCAGAAAGGGCTACAGGATGTTAACCTGCGCAATTTCAGCTACGGCCAGAC
CAGCCTGGACAGGCTTAGAGATGGCCTCGTGGGCGCCCAGTTCTGGTCAGCCTATGTGCCATGCCAGACCCAGGA
CCGGGATGCCCTGCGCCTCACCCTGGAGCAGATTGACCTCATACGCCGCATGTGTGCCTCCTATTCTGAGCTGGA
GCTTGTGACCTCGGCTAAAGCTCTGAACGACACTCAGAAATTGGCCTGCCTCATCGGTGTAGAGGGTGGCCACTC
GCTGGACAATAGCCTCTCCATCTTACGTACCTTCTACATGCTGGGAGTGCGCTACCTGACGCTCACCCACACCTG
CAACACACCCTGGGCAGAGAGCTCCGCTAAGGGCGTCCACTCCTTCTACAACAACATCAGCGGGCTGACTGACTT
TGGTGAGAAGGTGGTGGCAGAAATGAACCGCCTGGGCATGATGGTAGACTTATCCCATGTCTCAGATGCTGTGGC
ACGGCGGGCCCTGGAAGTGTCACAGGCACCTGTGATCTTCTCCCACTCGGCTGCCCGGGGTGTGTGCAACAGTGC
TCGGAATGTTCCTGATGACATCCTGCAGCTTCTGAAGAAGAACGGTGGCGTCGTGATGGTGTCTTTGTCCATGGG
AGTAATACAGTGCAACCCATCAGCCAATGTGTCCACTGTGGCAGATCACTTCGACCACATCAAGGCTGTCATTGG
ATCCAAGTTCATCGGGATTGGTGGAGATTATGATGGGGCCGGCAAATTCCCTCAGGGGCTGGAAGACGTGTCCAC
ATACCCGGTCCTGATAGAGGAGTTGCTGAGTCGTGGCTGGAGTGAGGAAGAGCTTCAGGGTGTCCTTCGTGGAAA
CCTGCTGCGGGTCTTCAGACAAGTGGAAAAGGTACAGGAAGAAAACAAATGGCAAAGCCCCTTGGAGGACAAGTT
CCCGGATGAGCAGCTGAGCAGTTCCTGCCACTCCGACCTCTCACGTCTGCGTCAGAGACAGAGTCTGACTTCAGG
CCAGGAACTCACTGAGATTCCCATACACTGGACAGCCAAGTTACCAGCCAAGTGGTCAGTCTCAGAGTCCTCCCC
CCACATGGCCCCAGTCCTTGCAGTTGTGGCCACCTTCCCAGTCCTTATTCTGTGGCTC<u>TGA</u>TGACCCAGTTAGTC
CTGCCAGATGTCACTGTAGCAAGCCACAGACACCCCACAAAGTTCCCCTGTTGTGCAGGCACAAATATTTCCTGA
AATAAATGTTTTGGACATAG

## FIGURE 38

| | |
|---|---|
| N-glycosylation sites: | Amino acids 58-62;123-127; 182-186;273-277 |
| N-myristoylation sites: | Amino acids 72-78;133-139; 234-240;264-270;334-340; 389-395 |
| Renal dipeptidase active site: | Amino acids 134-157 |

MPGTYAPSTTLSSPSTQGLQEQARALMRDFPLVDGHNDLPLVLRQVYQKGLQDVNLRNFSYGQTSLDRLRDGLVG
AQFWSAYVPCQTQDRDALRLTLEQIDLIRRMCASYSELELVTSAKALNDTQKLACLIGVEGGHSLDNSLSILRTF
YMLGVRYLTLTHTCNTPWAESSAKGVHSFYNNISGLTDFGEKVVAEMNRLGMMVDLSHVSDAVARRALEVSQAPV
IFSHSAARGVCNSARNVPDDILQLLKKNGGVVMVSLSMGVIQCNPSANVSTVADHFDHIKAVIGSKFIGIGGDYD
GAGKFPQGLEDVSTYPVLIEELLSRGWSEEELQGVLRGNLLRVFRQVEKVQEENKWQSPLEDKFPDEQLSSSCHS
DLSRLRQRQSLTSGQELTEIPIHWTAKLPAKWSVSESSPHMAPVLAVVATFPVLILWL

FIGURE 39

GGGGAGAGGAATTGACCATGTAAAAGGAGACTTTTTTTTTGGTGGTGGTGGCTGTTGGGTGCCTTGCAAAAATG
AAGGATGCAGGACGCAGCTTTCTCCTGGAACCGAACGCAATGGATAAACTGATTGTGCAAGAGAGAAGGAAGAAC
GAAGCTTTTTCTTGTGAGCCCTGGATCTTAACACAAATGTGTATATGTGCACACAGGGAGCATTCAAGAATGAAA
TAAACCAGAGTTAGACCCGCGGGGGTTGGTGTGTTCTGACATAAATAAATAATCTTAAAGCAGCTGTTCCCCTCC
CCACCCCCAAAAAAAAAGGATGATTGGAAATGAAGAACCGAGGATTCACAAAGAAAAAAGTATGTTCATTTTTCTC
TATAAAGGAGAAAGTGAGCCAAGGAGATATTTTTGGAATGAAAAGTTTGGGGCTTTTTTAGTAAAGTAAAGAACT
GGTGTGGTGGTGTTTTCCTTTCTTTTTGAATTTCCCACAAGAGGAGAGGAAATTAATAATACATCTGCAAAGAAA
TTTCAGAGAAGAAAAGTTGACCGCGGCAGATTGAGGCATTGATTGGGGGAGAGAAACCAGCAGAGCACAGTTGGA
TTTGTGCCTATGTTGACTAAAATTGACGGATAATTGCAGTTGGATTTTTCTTCATCAACCTCCTTTTTTTTAAAT
TTTTATTCCTTTTGGTATCAAGATCATGCGTTTTCTCTTGTTCTTAACCACCTGGATTTCCATCTGGATGTTGCT
GTGATCAGTCTGAAATACAACTGTTTGAATTCCAGAAGGACCAACACCAGATAAATTATGAATGTTGAACAAGAT
GACCTTACATCCACAGCAGATAATGATAGGTCCTAGGTTTAACAGGGCCCTATTTGACCCCCTGCTTGTGGTGCT
GCTGGCTCTTCAACTTCTTGTGGTGGCTGGTCTGGTGCGGGCTCAGACCTGCCCTTCTGTGTGCTCCTGCAGCAA
CCAGTTCAGCAAGGTGATTTGTGTTCGGAAAAACCTGCGTGAGGTTCCGGATGGCATCTCCACCAACACACGGCTG
CTGAACCTCCATGAGAACCAAATCCAGATCATCAAAGTGAACAGCTTCAAGCACTTGAGGCACTTGGAAATCCTA
CAGTTGAGTAGGAACCATATCAGAACCATTGAAATTGGGGCTTTCAATGGTCTGGCGAACCTCAACACTCTGGAA
CTCTTTGACAATCGTCTTACTACCATCCCGAATGGAGCTTTTGTATACTTGTCTAAACTGAAGGAGCTCTGGTTG
CGAAACAACCCCATTGAAAGCATCCCTTCTTATGCTTTTAACAGAATTCCTTCTTTGCGCCGACTAGACTTAGGG
GAATTGAAAAGACTTTCATACATCTCAGAAGGTGCCTTTGAAGGTCTGTCCAACTTGAGGTATTTGAACCTTGCC
ATGTGCAACCTTCGGGAAATCCCTAACCTCACACCGCTCATAAAACTAGATGAGCTGGATCTTTCTGGGAATCAT
TTATCTGCCATCAGGCCTGGCTCTTTCCAGGGTTTGATGCACCTTCAAAAACTGTGGATGATACAGTCCCAGATT
CAAGTGATTGAACGGAATGCCTTTGACAACCTTCAGTCACTAGTGGAGATCAACCTGGCACACAATAATCTAACA
TTACTGCCTCATGACCTCTTCACTCCCTTGCATCATCTAGAGCGGATACATTTACATCACAACCCTTGGAACTGT
AACTGTGACATACTGTGGCTCAGCTGGTGGATAAAAGACATGGCCCCCTCGAACACAGCTTGTTGTGCCCGGTGT
AACACTCCTCCCAATCTAAAGGGGAGGTACATTGGAGAGCTCGACCAGAATTACTTCACATGCTATGCTCCGGTG
ATTGTGGAGCCCCCTGCAGACCTCAATGTCACTGAAGGCATGGCAGCTGAGCTGAAATGTCGGGCCTCCACATCC
CTGACATCTGTATCTTGGATTACTCCAAATGGAACAGTCATGACACATGGGGCGTACAAAGTGCGGATAGCTGTG
CTCAGTGATGGTACGTTAAATTTCACAAATGTAACTGTGCAAGATACAGGCATGTACACATGTATGGTGAGTAAT
TCCGTTGGGAATACTACTGCTTCAGCCACCCTGAATGTTACTGCAGCAACCACTACTCCTTTCTCTTACTTTTCA
ACCGTCACAGTAGAGACTATGGAACCGTCTCAGGATGAGGCACGGACCACAGATAACAATGTGGGTCCCACTCCA
GTGGTCGACTGGGAGACCACCAATGTGACCACCTCTCTCACACCACAGAGCACAAGGTCGACAGAGAAAACCTTC
ACCATCCCAGTGACTGATATAAACAGTGGGATCCCAGGAATTGATGAGGTCATGAAGACTACCAAAATCATCATT
GGGTGTTTTGTGGCCATCACACTCATGGCTGCAGTGATGCTGGTCATTTTCTACAAGATGAGGAAGCAGCACCAT
CGGCAAAACCATCACGCCCCAACAAGGACTGTTGAAATTATTAATGTGGATGATGAGATTACGGGAGACACACCC
ATGGAAAGCCACCTGCCCATGCCTGCTATCGAGCATGAGCACCTAAATCACTATAACTCATACAAATCTCCCTTC
AACCACACAACAACAGTTAACACAATAAATTCAATACACAGTTCAGTGCATGAACCGTTATTGATCCGAATGAAC
TCTAAAGACAATGTACAAGAGACTCAAATCTAAAACATTTACAGAGTTACAAAAAACAAACAATCAAAAAAAAAG
ACAGTTTATTAAAAATGACACAAATGACTGGGCTAAATCTACTGTTTCAAAAAAGTGTCTTTACAAAAAAACAAA
AAAGAAAAGAAATTTATTTATTAAAAATTCTATTGTGATCTAAAGCAGACAAAAA

## FIGURE 40

| | |
|---|---|
| Signal sequence: | Amino acids 1-44 |
| Transmembrane domain: | Amino acids 528-543 |
| N-glycosylation sites: | Amino acids 278-282;364-368; 390-394;412-416;415-419;434-438; 442-446;488-492;606-610 |
| cAMP- and cGMP-dependent protein kinase phosphorylation site: | Amino acids 183-187 |
| N-myristoylation sites: | Amino acids 40-46;73-79;118-124; 191-197;228-234;237-243;391-397; 422-428;433-439;531-537 |

MLNKMTLHPQQIMIGPRFNRALFDPLLVVLLALQLLVVAGLVRAQTCPSVCSCSNQFSKVICVRKNLREVPDGIS
TNTRLLNLHENQIQIIKVNSFKHLRHLEILQLSRNHIRTIEIGAFNGLANLNTLELFDNRLTTIPNGAFVYLSKL
KELWLRNNPIESIPSYAFNRIPSLRRLDLGELKRLSYISEGAFEGLSNLRYLNLAMCNLREIPNLTPLIKLDELD
LSGNHLSAIRPGSFQGLMHLQKLWMIQSQIQVIERNAFDNLQSLVEINLAHNNLTLLPHDLFTPLHHLERIHLHH
NPWNCNCDILWLSWWIKDMAPSNTACCARCNTPPNLKGRYIGELDQNYFTCYAPVIVEPPADLNVTEGMAAELKC
RASTSLTSVSWITPNGTVMTHGAYKVRIAVLSDGTLNFTNVTVQDTGMYTCMVSNSVGNTTASATLNVTAATTTP
FSYFSTVTVETMEPSQDEARTTDNNVGPTPVVDWETTNVTTSLTPQSTRSTEKTFTIPVTDINSGIPGIDEVMKT
TKIIIGCFVAITLMAAVMLVIFYKMRKQHHRQNHHAPTRTVEIINVDDEITGDTPMESHLPMPAIEHEHLNHYNS
YKSPFNHTTTVNTINSIHSSVHEPLLIRMNSKDNVQETQI

FIGURE 41

GAAAGCTATAGGCTACCCATTCAGCTCCCCTGTCAGAGACTCAAGCTTTGAGAAAGGCTAGCAAAGAGCAAGGAA
AGAGAGAAAACAACAAAGTGGCGAGGCCCTCAGAGTGAAAGCGTAAGGTTCAGTCAGCCTGCTGCAGCTTTGCAG
ACCTCAGCTGGGCATCTCCAGACTCCCCTGAAGGAAGAGCCTTCCTCACCCAAACCCACAAAAGATGCTGAAAAA
GCCTCTCTCAGCTGTGACCTGGCTCTGCATTTTCATCGTGGCCTTTGTCAGCCACCCAGCGTGGCTGCAGAAGCT
CTCTAAGCACAAGACACCAGCACAGCCACAGCTCAAAGCGGCCAACTGCTGTGAGGAGGTGAAGGAGCTCAAGGC
CCAAGTTGCCAACCTTAGCAGCCTGCTGAGTGAACTGAACAAGAAGCAGGAGAGGGACTGGGTCAGCGTGGTCAT
GCAGGTGATGGAGCTGGAGAGCAACAGCAAGCGCATGGAGTCGCGGCTCACAGATGCTGAGAGCAAGTACTCCGA
GATGAACAACCAAATTGACATCATGCAGCTGCAGGCAGCACAGACGGTCACTCAGACCTCCGCAGATGCCATCTA
CGACTGCTCTTCCCTCTACCAGAAGAACTACCGCATCTCTGGAGTGTATAAGCTTCCTCCTGATGACTTCCTGGG
CAGCCCTGAACTGGAGGTGTTCTGTGACATGGAGACTTCAGGCGGAGGCTGGACCATCATCCAGAGACGAAAAAG
TGGCCTTGTCTCCTTCTACCGGGACTGGAAGCAGTACAAGCAGGGCTTTGGCAGCATCCGTGGGGACTTCTGGCT
GGGGAACGAACACATCCACCGGCTCTCCAGACAGCCAACCCGGCTGCGTGTAGAGATGGAGGACTGGGAGGGCAA
CCTGCGCTACGCTGAGTATAGCCACTTTGTTTTGGGCAATGAACTCAACAGCTATCGCCTCTTCCTGGGGAACTA
CACTGGCAATGTGGGGAACGACGCCCTCCAGTATCATAACAACACAGCCTTCAGCACCAAGGACAAGGACAATGA
CAACTGCTTGGACAAGTGTGCACAGCTCCGCAAAGGTGGCTACTGGTACAACTGCTGCACAGACTCCAACCTCAA
TGGAGTGTACTACCGCCTGGGTGAGCACAATAAGCACCTGGATGGCATCACCTGGTATGGCTGGCATGGATCTAC
CTACTCCCTCAAACGGGTGGAGATGAAAATCCGCCCAGAAGACTTCAAGCCTTAAAAGGAGGCTGCCGTGGAGCA
CGGATACAGAAACTGAGACACGTGGAGACTGGATGAGGGCAGATGAGGACAGGAAGAGAGTGTTAGAAAGGGTAG
GACTGAGAAACAGCCTATAATCTCCAAAGAAAGAATAAGTCTCCAAGGAGCACAAAAAAATCATATGTACCAAGG
ATGTTACAGTAAACAGGATGAACTATTTAAACCCACTGGGTCCTGCCACATCCTTCTCAAGGTGGTAGACTGAGT
GGGGTCTCTCTGCCCAAGATCCCTGACATAGCAGTAGCTTGTCTTTTCCACATGATTTGTCTGTGAAAGAAAATA
ATTTTGAGATCGTTTTATCTATTTTCTCTACGGCTTAGGCTATGTGAGGGCAAAACACAAATCCCTTTGCTAAAA
AGAACCATATTATTTTGATTCTCAAAGGATAGGCCTTTGAGTGTTAGAGAAAGGAGTGAAGGAGGCAGGTGGGAA
ATGGTATTTCTATTTTTAAATCCAGTGAAATTATCTTGAGTCTACACATTATTTTTAAAACACAAAAATTGTTCG
GCTGGAACTGACCCAGGCTGGACTTGCGGGGAGGAAACTCCAGGGCACTGCATCTGGCGATCAGACTCTGAGCAC
TGCCCCTGCTCGCCTTGGTCATGTACAGCACTGAAAGGAATGAAGCACCAGCAGGAGGTGGACAGAGTCTCTCAT
GGATGCCGGCACAAAACTGCCTTAAAATATTCATAGTTAATACAGGTATATCTATTTTTATTTACTTTGTAAGAA
ACAAGCTCAAGGAGCTTCCTTTTAAATTTTGTCTGTAGGAAATGGTTGAAAACTGAAGGTAGATGGTGTTATAGT
TAATAATAAATGCTGTAAATAAGCATCTCACTTTGTAAAAATAAAATATTGTGGTTTTGTTTTAAACATTCAACG
TTTCTTTTCCTTCTACAATAAACACTTTCAAAATGTG

## FIGURE 42

| | |
|---|---|
| Signal sequence: | Amino acids 1-26 |
| N-glycosylation sites: | Amino acids 58-62;253-257;267-271 |
| Glycosaminoglycan attachment site: | Amino acids 167-171 |
| cAMP- and cGMP-dependent protein kinase phosphorylation site: | Amino acids 176-180 |
| N-myristoylation sites: | Amino acids 168-174;196-202; 241-247;252-258;256-262;327-333 |
| Cell attachment sequence: | Amino acids 199-202 |

MLKKPLSAVTWLCIFIVAFVSHPAWLQKLSKHKTPAQPQLKAANCCEEVKELKAQVANLSSLLSELNKKQERDWV
SVVMQVMELESNSKRMESRLTDAESKYSEMNNQIDIMQLQAAQTVTQTSADAIYDCSSLYQKNYRISGVYKLPPD
DFLGSPELEVFCDMETSGGGWTIIQRRKSGLVSFYRDWKQYKQGFGSIRGDFWLGNEHIHRLSRQPTRLRVEMED
WEGNLRYAEYSHFVLGNELNSYRLFLGNYTGNVGNDALQYHNNTAFSTKDKDNDNCLDKCAQLRKGGYWYNCCTD
SNLNGVYYRLGEHNKHLDGITWYGWHGSTYSLKRVEMKIRPEDFKP

FIGURE 43

CACGCACTTCACCTGGGTCGGGATTCTCAGGTCATGAACGGTCCCAGCCACCTCCGGGCAGGGCGGGTGAGGACG
GGGACGGGGCGTGTCCAACTGGCTGTGGGCTCTTGAAACCCGAGC**ATG**GCACAGCACGGGGCGATGGGCGCGTTT
CGGGCCCTGTGCGGCCTGGCGCTGCTGTGCGCGCTCAGCCTGGGTCAGCGCCCCACCGGGGGTCCCGGGTGCGGC
CCTGGGCGCCTCCTGCTTGGGACGGGAACGGACGCGCGCTGCTGCCGGGTTCACACGACGCGCTGCTGCCGCGAT
TACCCGGGCGAGGAGTGCTGTTCCGAGTGGGACTGCATGTGTGTCCAGCCTGAATTCCACTGCGGAGACCCTTGC
TGCACGACCTGCCGGCACCACCCTTGTCCCCCAGGCCAGGGGGTACAGTCCCAGGGGAAATTCAGTTTTGGCTTC
CAGTGTATCGACTGTGCCTCGGGGACCTTCTCCGGGGGCCACGAAGGCCACTGCAAACCTTGGACAGACTGCACC
CAGTTCGGGTTTCTCACTGTGTTCCCTGGGAACAAGACCCACAACGCTGTGTGCGTCCCAGGGTCCCCGCCGGCA
GAGCCGCTTGGGTGGCTGACCGTCGTCCTCCTGGCCGTGGCCGCCTGCGTCCTCCTCCTGACCTCGGCCCAGCTT
GGACTGCACATCTGGCAGCTGAGGAGTCAGTGCATGTGGCCCCGAGAGACCCAGCTGCTGCTGGAGGTGCCGCCGTC
GACCGAAGACGCCAGAAGCTGCCAGTTCCCCGAGGAAGAGCGGGGCGAGCGATCGGCAGAGGAGAAGGGGCGGCT
GGGAGACCTGTGGGTG**TGA**GCCTGGCCGTCCTCCGGGGCCACCGACCGCAGCCAGCCCCTCCCCAGGAGCTCCCC
AGGCCGCAGGGGCTCTGCGTTCTGCTCTGGGCCGGGCCCTGCTCCCCTGGCAGCAGAAGTGGGTGCAGGAAGGTG
GCAGTGACCAGCGCCCTGGACCATGCAGTTC

# FIGURE 44

| | |
|---|---|
| Signal sequence: | Amino acids 1-25 |
| Transmembrane domain: | Amino acids 163-183 |
| N-glycosylation site: | Amino acids 146-150 |
| N-myristoylation sites: | Amino acids 5-11;8-14;25-31;<br>30-36;33-39;118-124;122-128;<br>156-162 |
| Prokaryotic membrane lipoprotein lipid attachment site: | Amino acids 166-177 |
| Leucine zipper pattern: | Amino acids 171-193 |

MAQHGAMGAFRALCGLALLCALSLGQRPTGGPGCGPGRLLLGTGTDARCCRVHTTRCCRDYPGEECCSEWDCMCV
QPEFHCGDPCCTTCRHHPCPPGQGVQSQGKFSFGFQCIDCASGTFSGGHEGHCKPWTDCTQFGFLTVFPGNKTHN
AVCVPGSPPAEPLGWLTVVLLAVAACVLLLTSAQLGLHIWQLRSQCMWPRETQLLLEVPPSTEDARSCQFPEEER
GERSAEEKGRLGDLWV

FIGURE 45

GGCGCCGGTGCACCGGGCGGGCTGAGCGCCTCCTGCGGCCCGGCCTGCGCGCCCCGGCCCGCCGCGCCGCCCACG
CCCCAACCCCGGCCCGCGCCCCCTAGCCCCCGCCCGGGCCCGCGCCCGCGCCCGCGCCCAGGTGAGCGCTCCGCC
CGCCGCGAGGCCCCGCCCCGGCCCGCCCCCGCCCCGCCCCGGCCGGCGGGGAACCGGGCGGATTCCTCGCGCGT
CAAACCACCTGATCCCATAAAACATTCATCCTCCCGGCGGCCCGCGCTGCGAGCGCCCCGCCAGTCCGCGCCGCC
GCCGCCCTCGCCCTGTGCGCCCTGCGCGCCCTGCGCACCCGCGGCCCGAGCCCAGCCAGAGCCGGGCGGAGCGGA
GCGCGCCGAGCCTCGTCCCGCGGCCGGGCCGGGGCCGGGCCGTAGCGGCGGCGCCTGGATGCGGACCCGGCCGCG
GGGAGACGGGCGCCCGCCCCGAAACGACTTTCAGTCCCCGACGCGCCCCGCCCAACCCCTACG<u>ATG</u>AAGAGGGCG
TCCGCTGGAGGGAGCCGGCTGCTGGCATGGGTGCTGTGGCTGCAGGCCTGGCAGGTGGCAGCCCCATGCCCAGGT
GCCTGCGTATGCTACAATGAGCCCAAGGTGACGACAAGCTGCCCCCAGCAGGGCCTGCAGGCTGTGCCCGTGGGC
ATCCCTGCTGCCAGCCAGCGCATCTTCCTGCACGGCAACCGCATCTCGCATGTGCCAGCTGCCAGCTTCCGTGCCTGC
CGCAACCTCACCATCCTGTGGCTGCACTCGAATGTGCTGGCCCGAATTGATGCGGCTGCCTTCACTGGCCTGGCC
CTCCTGGAGCAGCTGGACCTCAGCGATAATGCACAGCTCCGGTCTGTGGACCCTGCCACATTCCACGGCCTGGGC
CGCCTACACACGCTGCACCTGGACCGCTGCGGCCTGCAGGAGCTGGGCCCGGGGCTGTTCCGCGGCCTGGCTGCC
CTGCAGTACCTCTACCTGCAGGACAACGCGCTGCAGGCACTGCCTGATGACACCTTCCGCGACCTGGGCAACCTC
ACACACCTCTTCCTGCACGGCAACCGCATCTCCAGCGTGCCCGAGCGCGCCTTCCGTGGGCTGCACAGCCTCGAC
CGTCTCCTACTGCACCAGAACCGCGTGGCCCATGTGCACCCGCATGCCTTCCGTGACCTTGGCCGCCTCATGACA
CTCTATCTGTTTGCCAACAATCTATCAGCGCTGCCCACTGAGGCCCTGGCCCCCCTGCGTGCCCTGCAGTACCTG
AGGCTCAACGACAACCCCTGGGTGTGTGACTGCCGGGCACGCCCACTCTGGGCCTGGCTGCAGAAGTTCCGCGGC
TCCTCCTCCGAGGTGCCCTGCAGCCTCCCGCAACGCCTGGCTGGCCGTGACCTCAAACGCCTAGCTGCCAATGAC
CTGCAGGGCTGCGCTGTGGCCACCGGCCCTTACCATCCCATCTGGACCGGCAGGGCCACCGATGAGGAGCCGCTG
GGGCTTCCCAAGTGCTGCCAGCCAGATGCCGCTGACAAGGCCTCAGTACTGGAGCCTGGAAGACCAGCTTCGGCA
GGCAATGCGCTGAAGGGACGCGTGCCGCCCGGTGACAGCCCGCCGGGCAACGGCTCTGGCCCACGGCACATCAAT
GACTCACCCTTTGGGACTCTGCCTGGCTCTGCTGAGCCCCCGCTCACTGCAGTGCGGCCCGAGGGCTCCGAGCCA
CCAGGGTTCCCCACCTCGGGCCCTCGCCGGAGGCCAGGCTGTTCACGCAAGAACCGCACCCGCAGCCACTGCCGT
CTGGGCCAGGCAGGCAGCGGGGGTGGCGGGACTGGTGACTCAGAAGGCTCAGGTGCCCTACCCAGCCTCACCTGC
AGCCTCACCCCCCTGGGCCTGGCGCTGGTGCTGTGGACAGTGCTTGGGCCCTGC<u>TGA</u>CCCCCAGCGGACACAAGA
GCGTGCTCAGCAGCCAGGTGTGTGTACATACGGGGTCTCTCTCCACGCCGCCAAGCCAGCCGGGCGGCCGACCCG
TGGGGCAGGCCAGGCCAGGTCCTCCCTGATGGACGCCTGCCGCCCGCCACCCCCATCTCCACCCCATCATGTTTA
CAGGGTTCGGCGGCAGCGTTTGTTCCAGAACGCCGCCTCCCACCCAGATCGCGGTATATAGAGATATGCATTTTA
TTTTACTTGTGTAAAAATATCGGACGACGTGGAATAAAGAGCTCTTTTCTTAAAAAAA

FIGURE 46

| | |
|---|---|
| Signal peptide: | Amino acids 1-26 |
| Leucine zipper pattern: | Amino acids 135-157 |
| Glycosaminoglycan attachment site: | Amino acids 436-440 |
| N-glycosylation sites: | Amino acids 82-86;179-183;237-241;<br>372-376;423-427 |
| Von Willebrand type C domain: | Amino acids 411-427 |

MKRASAGGSRLLAWVLWLQAWQVAAPCPGACVCYNEPKVTTSCPQQGLQAVPVGIPAASQRIFLHGNRISHVPAA
SFRACRNLTILWLHSNVLARIDAAAFTGLALLEQLDLSDNAQLRSVDPATFHGLGRLHTLHLDRCGLQELGPGLF
RGLAALQYLYLQDNALQALPDDTFRDLGNLTHLFLHGNRISSVPERAFRGLHSLDRLLLHQNRVAHVHPHAFRDL
GRLMTLYLFANNLSALPTEALAPLRALQYLRLNDNPWVCDCRARPLWAWLQKFRGSSSEVPCSLPQRLAGRDLKR
LAANDLQGCAVATGPYHPIWTGRATDEEPLGLPKCCQPDAADKASVLEPGRPASAGNALKGRVPPGDSPPGNGSG
PRHINDSPFGTLPGSAEPPLTAVRPEGSEPPGFPTSGPRRRPGCSRKNRTRSHCRLGQAGSGGGTGDSEGSGAL
PSLTCSLTPLGLALVLWTVLGPC

FIGURE 47

GCGAGGGGAGCGCGGAGCCCGGCGCCTACAGCTCGCC**ATG**GTGCGCCCCCTGAACCCGCGACCGCTGCCGCCCGT
AGTCCTGATGTTGCTGCTGCTGCTGCCGCCGTCGCCGCTGCCTCTCGCAGCCGGAGACCCCCTTCCCACAGAAAG
CCGACTCATGAACAGCTGTCTCCAGGCCAGGAGGAAGTGCCAGGCTGATCCCACCTGCAGTGCTGCCTACCACCA
CCTGGATTCCTGCACCTCTAGCATAAGCACCCCACTGCCCTCAGAGGAGCCTTCGGTCCCTGCTGACTGCCTGGA
GGCAGCACAGCAACTCAGGAACAGCTCTCTGATAGGCTGCATGTGCCACCGGCGCATGAAGAACCAGGTTGCCTG
CTTGGACATCTATTGGACCGTTCACCGTGCCCGCAGCCTTGGTAACTATGAGCTGGATGTCTCCCCCTATGAAGA
CACAGTGACCAGCAAACCCTGGAAAATGAATCTCAGCAAACTGAACATGCTCAAACCAGACTCAGACCTCTGCCT
CAAGTTTGCCATGCTGTGTACTCTCAATGACAAGTGTGACCGGCTGCGCAAGGCCTACGGGGAGGCGTGCTCCGG
GCCCCACTGCCAGCGCCACGTCTGCCTCAGGCAGCTGCTCACTTTCTTCGAGAAGGCCGCCGAGCCCCACGCGCA
GGGCCTGCTACTGTGCCCATGTGCCCCCAACGACCGGGGCTGCGGGGAGCGCCGGCGCAACACCATCGCCCCCAA
CTGCGCGCTGCCGCCTGTGGCCCCCAACTGCCTGGAGCTGCGGCGCCTCTGCTTCTCCGACCCGCTTTGCAGATC
ACGCCTGGTGGATTTCCAGACCCACTGCCATCCCATGGACATCCTAGGAACTTGTGCAACAGAGCAGTCCAGATG
TCTACGAGCATACCTGGGGCTGATTGGGACTGCCATGACCCCCAACTTTGTCAGCAATGTCAACACCAGTGTTGC
CTTAAGCTGCACCTGCCGAGGCAGTGGCAACCTGCAGGAGGAGTGTGAAATGCTGGAAGGGTTCTTCTCCCACAA
CCCCTGCCTCACGGAGGCCATTGCAGCTAAGATGCGTTTTCACAGCCAACTCTTCTCCCAGGACTGGCCACACCC
TACCTTTGCTGTGATGGCACACCAGAATGAAAACCCTGCTGTGAGGCCACAGCCCTGGGTGCCCTCTCTTTTCTC
CTGCACGCTTCCCTTGATTCTGCTCCTGAGCCTATGG**TAG**CTGGACTTCCCCAGGGCCCTCTTCCCCTCCACCAC
ACCCAGGTGGACTTGCAGCCCACAAGGGGTGAGGAAAGGACAGCAGCAGGAAGGAGGTGCAGTGCGCAGATGAGG
GCACAGGAGAAGCTAAGGGTTATGACCTCCAGATCCTTACTGGTCCAGTCCTCATTCCCTCCACCCCATCTCCAC
TTCTGATTCATGCTGCCCCTCCTTGGTGGCCACAATTTAGCCATGTCATCTGGTGGTGACCAGCTCCACCAAGCC
CCTTTCTGAGCCCTTCCTCTTGACTACCAGGATCACCAGAATCTAATAAGTTAGCCTTTCTCTATTGCATTCCAG
ATTAGGGTTAGGGTAGGGAGGACTGGGTGTTCTGAGGCAGCCTAGAAAGTCATTCTCCTTTGTGAAGAAGGCTCC
TGCCCCCTCGTCTCCTCCTCTGAGTGGAGGATGGAAAACTACTGCCTGCACTGCCCTGTCCCCGGATCCTGCCGA
ACATCTGGGCATCAGGAGCTGGAGCCTGTGGGCCTTGCTTTATTCCTATTATTGTCCTAAAGTCTCTCTGGGCTC
TTGGATCATGATTAAACCTTTGACTTAAG

# FIGURE 48

Signal sequence: Amino acids 1-26

N-glycosylation sites: Amino acids 95-99;148-152;309-313

cAMP- and cGMP-dependent protein kinase phosphorylation site:
Amino acids 231-235

N-myristoylation sites: Amino acids 279-285;294-300

Prokaryotic membrane lipoprotein lipid attachment sites:
Amino acids 306-317;379-390

MVRPLNPRPLPPVVLMLLLLLPPSPLPLAAGDPLPTESRLMNSCLQARRKCQADPTCSAAYHHLDSCTSSISTPL
PSEEPSVPADCLEAAQQLRNSSLIGCMCHRRMKNQVACLDIYWTVHRARSLGNYELDVSPYEDTVTSKPWKMNLS
KLNMLKPDSDLCLKFAMLCTLNDKCDRLRKAYGEACSGPHCQRHVCLRQLLTFFEKAAEPHAQGLLLCPCAPNDR
GCGERRRNTIAPNCALPPVAPNCLELRRLCFSDPLCRSRLVDFQTHCHPMDILGTCATEQSRCLRAYLGLIGTAM
TPNFVSNVNTSVALSCTCRGSGNLQEECEMLEGFFSHNPCLTEAIAAKMRFHSQLFSQDWPHPTFAVMAHQNENP
AVRPQPWVPSLFSCTLPLILLLSLW

FIGURE 49

CGGACGCGTGGGCGGACGCGTGGGCGGACGCGTGGGCGGACGCGTGGGCTGGTTCAGGTCCAGGTTTTGCTTTGA
TCCTTTTCAAAAACTGGAGACACAGAAGAGGGCTCTAGGAAAAAGTTTTGGATGGGATTATGTGGAAACTACCCT
GCGATTCTCTGCTGCCAGAGCAGGCTCGGCGCTTCCACCCCAGTGCAGCCTTCCCCTGGCGGTGGTGAAAGAGAC
TCGGGAGTCGCTGCTTCCAAAGTGCCCGCCGTGAGTGAGCTCTCACCCCAGTCAGCCAA**ATG**AGCCTCTTCGGGC
TTCTCCTGCTGACATCTGCCCTGGCCGGCCAGAGACAGGGGACTCAGGCGGAATCCAACCTGAGTAGTAAATTCC
AGTTTTCCAGCAACAAGGAACAGAACGGAGTACAAGATCCTCAGCATGAGAGAATTATTACTGTGTCTACTAATG
GAAGTATTCACAGCCCAAGGTTTCCTCATACTTATCCAAGAAATACGGTCTTGGTATGGAGATTAGTAGCAGTAG
AGGAAAATGTATGGATACAACTTACGTTTGATGAAAGATTTGGGCTTGAAGACCCAGAAGATGACATATGCAAGT
ATGATTTTGTAGAAGTTGAGGAACCCAGTGATGGAACTATATTAGGGCGCTGGTGTGGTTCTGGTACTGTACCAG
GAAAACAGATTTCTAAAGGAAATCAAATTAGGATAAGATTTGTATCTGATGAATATTTTCCTTCTGAACCAGGGT
TCTGCATCCACTACAACATTGTCATGCCACAATTCACAGAAGCTGTGAGTCCTTCAGTGCTACCCCCTTCAGCTT
TGCCACTGGACCTGCTTAATAATGCTATAACTGCCTTTAGTACCTTGGAAGACCTTATTCGATATCTTGAACCAG
AGAGATGGCAGTTGGACTTAGAAGATCTATATAGGCCAACTTGGCAACTTCTTGGCAAGGCTTTTGTTTTTGGAA
GAAAATCCAGAGTGGTGGATCTGAACCTTCTAACAGAGGAGGTAAGATTATACAGCTGCACACCTCGTAACTTCT
CAGTGTCCATAAGGGAAGAACTAAAGAGAACCGATACCATTTTCTGGCCAGGTTGTCTCCTGGTTAAACGCTGTG
GTGGGAACTGTGCCTGTTGTCTCCACAATTGCAATGAATGTCAATGTGTCCCAAGCAAAGTTACTAAAAAATACC
ACGAGGTCCTTCAGTTGAGACCAAAGACCGGTGTCAGGGGATTGCACAAATCACTCACCGACGTGGCCCTGGAGC
ACCATGAGGAGTGTGACTGTGTGTGCAGAGGGAGCACAGGAGGA**TAG**CCGCATCACCACCAGCAGCTCTTGCCCA
GAGCTGTGCAGTGCAGTGGCTGATTCTATTAGAGAACGTATGCGTTATCTCCATCCTTAATCTCAGTTGTTTGCT
TCAAGGACCTTTCATCTTCAGGATTTACAGTGCATTCTGAAAGAGGAGACATCAAACAGAATTAGGAGTTGTGCA
ACAGCTCTTTTGAGAGGAGGCCTAAAGGACAGGAGAAAAGGTCTTCAATCGTGGAAAGAAAATTAAATGTTGTAT
TAAATAGATCACCAGCTAGTTTCAGAGTTACCATGTACGTATTCCACTAGCTGGGTTCTGTATTTCAGTTCTTTC
GATACGGCTTAGGGTAATGTCAGTACAGGAAAAAAACTGTGCAAGTGAGCACCTGATTCCGTTGCCTTGCTTAAC
TCTAAAGCTCCATGTCCTGGGCCTAAAATCGTATAAAATCTGGATTTTTTTTTTTTTTTTTGCTCATATTCACAT
ATGTAAACCAGAACATTCTATGTACTACAAACCTGGTTTTTAAAAAGGAACTATGTTGCTATGAATTAAACTTGT
GTCATGCTGATAGGACAGACTGGATTTTTCATATTTCTTATTAAAATTTCTGCCATTTAGAAGAAGAGAACTACA
TTCATGGTTTGGAAGAGATAAACCTGAAAAGAAGAGTGGCCTTATCTTCACTTTATCGATAAGTCAGTTTATTTG
TTTCATTGTGTACATTTTTATATTCTCCTTTTGACATTATAACTGTTGGCTTTTCTAATCTTGTTAAATATATCT
ATTTTTACCAAAGGTATTTAATATTCTTTTTTATGACAACTTAGATCAACTATTTTTAGCTTGGTAAATTTTTCT
AAACACAATTGTTATAGCCAGAGGAACAAAGATGATATAAAATATTGTTGCTCTGACAAAAATACATGTATTTCA
TTCTCGTATGGTGCTAGAGTTAGATTAATCTGCATTTTAAAAAACTGAATTGGAATAGAATTGGTAAGTTGCAAA
GACTTTTTGAAAATAATTAAATTATCATATCTTCCATTCCTGTTATTGGAGATGAAAATAAAAAGCAACTTATGA
AAGTAGACATTCAGATCCAGCCATTACTAACCTATTCCTTTTTTGGGGAAATCTGAGCCTAGCTCAGAAAAACAT
AAAGCACCTTGAAAAAGACTTGGCAGCTTCCTGATAAAGCGTGCTGTGCTGTGCAGTAGGAACACATCCTATTTA
TTGTGATGTTGTGGTTTTATTATCTTAAACTCTGTTCCATACACTTGTATAAATACATGGATATTTTTATGTACA
GAAGTATGTCTCTTAACCAGTTCACTTATTGTACTCTGGCAATTTAAAAGAAAATCAGTAAAATATTTTGCTTGT
AAAATGCTTAATATNGTGCCTAGGTTATGTGGTGACTATTTGAATCAAAAATGTATTGAATCATCAAATAAAAGA
ATGTGGCTATTTTGGGGAGAAAATTAAAAAAAAAAAAAAAAAAAAAAGGTTTAGGATAACAGGGTAATGCGGCC

# FIGURE 50

| | |
|---|---|
| signal sequence: | Amino acids 1-14 |
| N-glycosylation sites: | Amino acids 25-29;55-59;254-258 |
| N-myristoylation sites: | Amino acids 15-21;117-123;127-133;<br>281-287;282-288;319-325 |
| Amidation site: | Amino acids 229-233 |

MSLFGLLLLTSALAGQRQGTQAESNLSSKFQFSSNKEQNGVQDPQHERIITVSTNGSIHSPRFPHTYPRNTVLVW
RLVAVEENVWIQLTFDERFGLEDPEDDICKYDFVEVEEPSDGTILGRWCGSGTVPGKQISKGNQIRIRFVSDEYF
PSEPGFCIHYNIVMPQFTEAVSPSVLPPSALPLDLLNNAITAFSTLEDLIRYLEPERWQLDLEDLYRPTWQLLGK
AFVFGRKSRVVDLNLLTEEVRLYSCTPRNFSVSIREELKRTDTIFWPGCLLVKRCGGNCACCLHNCNECQCVPSK
VTKKYHEVLQLRPKTGVRGLHKSLTDVALEHHEECDCVCRGSTGG

FIGURE 51

GGACGAGGGCAGATCTCGTTCTGGGGCAAGCCGTTGACACTCGCTCCCTGCCACCGCCCGGGCTCCGTGCCGCCA
AGTTTTCATTTTCCACCTTCTCTGCCTCCAGTCCCCCAGCCCCTGGCCGAGAGAAGGGTCTTACCGGCCGGGATT
GCTGGAAACACCAAGAGGTGGTTTTTGTTTTTTAAAACTTCTGTTTCTTGGGAGGGGGTGTGGCGGGGCAGG**ATG**
AGCAACTCCGTTCCTCTGCTCTGTTTCTGGAGCCTCTGCTATTGCTTTGCTGCGGGGAGCCCCGTACCTTTTGGT
CCAGAGGGACGGCTGGAAGATAAGCTCCACAAACCCAAAGCTACACAGACTGAGGTCAAACCATCTGTGAGGTTT
AACCTCCGCACCTCCAAGGACCCAGAGCATGAAGGATGCTACCTCTCCGTCGGCCACAGCCAGCCCTTAGAAGAC
TGCAGTTTCAACATGACAGCTAAAACCTTTTTCATCATTCACGGATGGACGATGAGCGGTATCTTTGAAAACTGG
CTGCACAAACTCGTGTCAGCCCTGCACACAAGAGAGAAAGACGCCAATGTAGTTGTGGTTGACTGGCTCCCCCTG
GCCCACCAGCTTTACACGGATGCGGTCAATAATACCAGGGTGGTGGGACACAGCATTGCCAGGATGCTCGACTGG
CTGCAGGAGAAGGACGATTTTTCTCTCGGGAATGTCCACTTGATCGGCTACAGCCTCGGAGCGCACGTGGCCGGG
TATGCAGGCAACTTCGTGAAAGGAACGGTGGGCCGAATCACAGGTTTGGATCCTGCCGGGCCCATGTTTGAAGGG
GCCGACATCCACAAGAGGCTCTCTCCGGACGATGCAGATTTTGTGGATGTCCTCCACACCTACACGCGTTCCTTC
GGCTTGAGCATTGGTATTCAGATGCCTGTGGGCCACATTGACATCTACCCCAATGGGGGTGACTTCCAGCCAGGC
TGTGGACTCAACGATGTCTTGGGATCAATTGCATATGGAACAATCACAGAGGTGGTAAAATGTGAGCATGAGCGA
GCCGTCCACCTCTTTGTTGACTCTCTGGTGAATCAGGACAAGCCGAGTTTTGCCTTCCAGTGCACTGACTCCAAT
CGCTTCAAAAAGGGGATCTGTCTGAGCTGCCGCAAGAACCGTTGTAATAGCATTGGCTACAATGCCAAGAAAATG
AGGAACAAGAGGAACAGCAAAATGTACCTAAAAACCCGGGCAGGCATGCCTTTCAGAGGTAACCTTCAGTCCCTG
GAGTGTCCC**TGA**GGAAGGCCCTTAATACCTCCTTCTTAATACCATGCTGCAGAGCAGGGCACATCCTAGCCCAGG
AGAAGTGGCCAGCACAATCCAATCAAATCGTTGCAAATCAGATTACACTGTGCATGTCCTAGGAAAGGGAATCTT
TACAAAATAAACAGTGTGGACCCCTAATAAAAAAAAAAAAAAAAAAAAAAAAAAAAAAAAAAAAAAAAAAAAAAA
AAAAAAAAAA

## FIGURE 52

| | |
|---|---|
| Signal sequence: | Amino acids 1-16 |
| N-glycosylation sites: | Amino acids 80-84;136-140 |
| cAMP- and cGMP-dependent protein kinase phosphorylation sites: | Amino acids 206-210;329-333 |
| N-myristoylation sites: | Amino acids 63-69;96-102;171-177;191-197;227-233;251-257;306-312;346-352 |
| Lipases, serine active site: | Amino acids 163-173 |

MSNSVPLLCFWSLCYCFAAGSPVPFGPEGRLEDKLHKPKATQTEVKPSVRFNLRTSKDPEHEGCYLSVGHSQPLE
DCSFNMTAKTFFIIHGWTMSGIFENWLHKLVSALHTREKDANVVVVDWLPLAHQLYTDAVNNTRVVGHSIARMLD
WLQEKDDFSLGNVHLIGYSLGAHVAGYAGNFVKGTVGRITGLDPAGPMFEGADIHKRLSPDDADFVDVLHTYTRS
FGLSIGIQMPVGHIDIYPNGGDFQPGCGLNDVLGSIAYGTITEVVKCEHERAVHLFVDSLVNQDKPSFAFQCTDS
NRFKKGICLSCRKNRCNSIGYNAKKMRNKRNSKMYLKTRAGMPFRGNLQSLECP

# FIGURE 53

CGCGCCGGGCGCAGGGAGCTGAGTGGACGGCTCGAGACGGCGGCGTGCAGCAGCTCCAGAAAGCAGCGAGTTG
GCAGAGCAGGGCTGCATTTCCAGCAGGAGCTGCGAGCACAGTGCTGGCTCACAACAAG**ATG**CTCAAGGTGTCAGC
CGTACTGTGTGTGTGTGCAGCCGCTTGGTGCAGTCAGTCTCTCGCAGCTGCCGCGGCGGTGGCTGCAGCCGGGGG
GCGGTCGGACGGCGGTAATTTTCTGGATGATAAACAATGGCTCACCACAATCTCTCAGTATGACAAGGAAGTCGG
ACAGTGGAACAAATTCCGAGACGAAGTAGAGGATGATTATTTCCGCACTTGGAGTCCAGGAAAACCCTTCGATCA
GGCTTTAGATCCAGCTAAGGATCCATGCTTAAAGATGAAATGTAGTCGCCATAAAGTATGCATTGCTCAAGATTC
TCAGACTGCAGTCTGCATTAGTCACCGGAGGCTTACACACAGGATGAAAGAAGCAGGAGTAGACCATAGGCAGTG
GAGGGGTCCCATATTATCCACCTGCAAGCAGTGCCCAGTGGTCTATCCCAGCCCTGTTTGTGGTTCAGATGGTCA
TACCTACTCTTTTCAGTGCAAACTAGAATATCAGGCATGTGTCTTAGGAAAACAGATCTCAGTCAAATGTGAAGG
ACATTGCCCATGTCCTTCAGATAAGCCCACCAGTACAAGCAGAAATGTTAAGAGAGCATGCAGTGACCTGGAGTT
CAGGGAAGTGGCAAACAGATTGCGGACTGGTTCAAGGCCCTTCATGAAAGTGGAAGTCAAAACAAGAAGACAAA
AACATTGCTGAGGCCTGAGAGAAGCAGATTCGATACCAGCATCTTGCCAATTTGCAAGGACTCACTTGGCTGGAT
GTTTAACAGACTTGATACAAACTATGACCTGCTATTGGACCAGTCAGAGCTCAGAAGCATTTACCTTGATAAGAA
TGAACAGTGTACCAAGGCATTCTTCAATTCTTGTGACACATACAAGGACAGTTTAATATCTAATAATGAGTGGTG
CTACTGCTTCCAGAGACAGCAAGACCCACCTTGCCAGACTGAGCTCAGCAATATTCAGAAGCGGCAAGGGGTAAA
GAAGCTCCTAGGACAGTATATCCCCCTGTGTGATGAAGATGGTTACTACAAGCCAACACAATGTCATGGCAGTGT
TGGACAGTGCTGGTGTGTTGACAGATATGGAAATGAAGTCATGGGATCCAGAATAAATGGTGTTGCAGATTGTGC
TATAGATTTTGAGATCTCCGGAGATTTTGCTAGTGGCGATTTTCATGAATGGACTGATGATGAGGATGATGAAGA
CGATATTATGAATGATGAAGATGAAATTGAAGATGATGATGAAGATGAAGGGGATGATGATGATGGTGGTGATGA
CCATGATGTATACATT**TGA**TTGATGACAGTTGAAATCAATAAATTCTACATTTCTAATATTTACAAAAATGATAG
CCTATTTAAAATTATCTTCTTCCCCAATAACAAAATGATTCTAAACCTCACATATATTTTGTATAATTATTTGAA
AAATTGCAGCTAAAGTTATAGAACTTTATGTTTAAATAAGAATCATTTGCTTTGAGTTTTTATATTCCTTACACA
AAAAGAAAATACATATGCAGTCTAGTCAGACAAAATAAAGTTTTGAAGTGCTACTATAATAAATTTTTCACGAGA
ACAAACTTTGTAAATCTTCCATAAGCAAAATGACAGCTAGTGCTTGGGATCGTACATGTTAATTTTTTGAAAGAT
AATTCTAAGTGAAATTTAAAATAAATAAATTTTTAATGACCTGGGTCTTAAGGATTTAGGAAAAATATGCATGCT
TTAATTGCATTTCCAAAGTAGCATCTTGCTAGACCTAGATGAGTCAGGATAACAGAGAGATACCACATGACTCCA
AAAAAAAAAAAAAA

## FIGURE 54

Signal sequence: Amino acids 1-16

cAMP- and cGMP-dependent protein kinase phosphorylation site: Amino acids 115-119

Tyrosine kinase phosphorylation site: Amino acids 62-70

N-myristoylation sites: Amino acids 357-363;371-377; 376-382

Leucine zipper pattern: Amino acids 246-268

MLKVSAVLCVCAAAWCSQSLAAAAAVAAAGGRSDGGNFLDDKQWLTTISQYDKEVGQWNKFRDEVEDDYFRTWSP
GKPFDQALDPAKDPCLKMKCSRHKVCIAQDSQTAVCISHRRLTHRMKEAGVDHRQWRGPILSTCKQCPVVYPSPV
CGSDGHTYSFQCKLEYQACVLGKQISVKCEGHCPCPSDKPTSTSRNVKRACSDLEFREVANRLRDWFKALHESGS
QNKKTKTLLRPERSRFDTSILPICKDSLGWMFNRLDTNYDLLLDQSELRSIYLDKNEQCTKAFFNSCDTYKDSLI
SNNEWCYCFQRQQDPPCQTELSNIQKRQGVKKLLGQYIPLCDEDGYYKPTQCHGSVGQCWCVDRYGNEVMGSRIN
GVADCAIDFEISGDFASGDFHEWTDDEDDEDDIMNDEDEIEDDDEDEGDDDDGGDDHDVYI

FIGURE 55

CCAGTCTGTCGCCACCTCACTTGGTGTCTGCTGTCCCCGCCAGGCAAGCCTGGGGTGAGAGCACAGAGGAGTGGG
CCGGGACC**ATG**CGGGGGACGCGGCTGGCGCTCCTGGCGCTGGTGCTGGCTGCCTGCGGAGAGCTGGCGCCGGCCC
TGCGCTGCTACGTCTGTCCGGAGCCCACAGGAGTGTCGGACTGTGTCACCATCGCCACCTGCACCACCAACGAAA
CCATGTGCAAGACCACACTCTACTCCCGGGAGATAGTGTACCCCTTCCAGGGGGACTCCACGGTGACCAAGTCCT
GTGCCAGCAAGTGTAAGCCCTCGGATGTGGATGGCATCGGCCAGACCCTGCCCGTGTCCTGCTGCAATACTGAGC
TGTGCAATGTAGACGGGGCGCCCGCTCTGAACAGCCTCCACTGCGGGGCCCTCACGCTCCTCCCACTCTTGAGCC
TCCGACTG**TAG**AGTCCCCGCCCACCCCCATGGCCCTATGCGGCCCAGCCCCGAATGCCTTGAAGAAGTGCCCCCT
GCACCAGGAAAAAAAAAAAAAAAAA

FIGURE 56

| | |
|---|---|
| Signal sequence: | Amino acids 1-17 |
| N-glycosylation site: | Amino acids 46-50 |
| N-myristoylation sites: | Amino acids 3-9;33-39;84-90 |

Prokaryotic membrane lipoprotein lipid attachment site:
Amino acids 6-17

MRGTRLALLALVLAACGELAPALRCYVCPEPTGVSDCVTIATCTTNETMCKTTLYSREIVYPFQGDSTVTKSCAS
KCKPSDVDGIGQTLPVSCCNTELCNVDGAPALNSLHCGALTLLPLLSLRL

FIGURE 57

CGGACGCGTGGGCGGACGCGTGGGCGGACGCGTGGGCGGACGCGTGGGCTGGGTGCCTGCATCGCC<u>ATG</u>GACACC
ACCAGGTACAGCAAGTGGGGCGGCAGCTCCGAGGAGGTCCCCGGAGGGCCCTGGGGACGCTGGGTGCACTGGAGC
AGGAGACCCCTCTTCTTGGCCCTGGCTGTCCTGGTCACCACAGTCCTTTGGGCTGTGATTCTGAGTATCCTATTG
TCCAAGGCCTCCACGGAGCGCGCGGCGCTGCTTGACGGCCACGACCTGCTGAGGACAAACGCCTCGAAGCAGACG
GCGGCGCTGGGTGCCCTGAAGGAGGAGGTCGGAGACTGCCACAGCTGCTGCTCGGGGACGCAGGCGCAGCTGCAG
ACCACGCGCGCGGAGCTTGGGGAGGCGCAGGCGAAGCTGATGGAGCAGGAGAGCGCCCTGCGGGAACTGCGTGAG
CGCGTGACCCAGGGCTTGGCTGAAGCCGGCAGGGGCCGTGAGGACGTCCGCACTGAGCTGTTCCGGGCGCTGGAG
GCCGTGAGGCTCCAGAACAACTCCTGCGAGCCGTGCCCCACGTCGTGGCTGTCCTTCGAGGGCTCCTGCTACTTT
TTCTCTGTGCCAAAGACGACGTGGGCGGCGGCGCAGGATCACTGCGCAGATGCCAGCGCGCACCTGGTGATCGTT
GGGGGCCTGGATGAGCAGGGCTTCCTCACTCGGAACACGCGTGGCCGTGGTTACTGGCTGGGCCTGAGGGCTGTG
CGCCATCTGGGCAAGGTTCAGGGCTACCAGTGGGTGGACGGAGTCTCTCTCAGCTTCAGCCACTGGAACCAGGGA
GAGCCCAATGACGCTTGGGGGCGCGAGAACTGTGTCATGATGCTGCACACGGGGCTGTGGAACGACGCACCGTGT
GACAGCGAGAAGGACGGCTGGATCTGTGAGAAAAGGCACAACTGC<u>TGA</u>CCCCGCCCAGTGCCCTGGAGCCGCGCC
CATTGCAGCATGTCGTATCCTGGGGGCTGCTCACCTCCCTGGCTCCTGGAGCTGATTGCCAAAGAGTTTTTTTCT
TCCTCATCCACCGCTGCTGAGTCTCAGAAACACTTGGCCCAACATAGCCCTGTCCAGCCCAGTGCCTGGGCTCTG
GGACCTCCATGCCGACCTCATCCTAACTCCACTCACGCAGACCCAACCTAACCTCCACTAGCTCCAAAATCCCTG
CTCCTGCGTCCCCGTGATATGCCTCCACTTCTCTCCCTAACCAAGGTTAGGTGACTGAGGACTGGAGCTGTTTGG
TTTTCTCGCATTTTCCACCAAACTGGAAGCTGTTTTTGCAGCCTGAGGAAGCATCAATAAATATTTGAGAAATGA
AAAAA

## FIGURE 58

| | |
|---|---|
| Signal sequence: | Amino acids 1-46 |
| Transmembrane domain: | Amino acids 31-54 |
| N-glycosylation sites: | Amino acids 73-77;159-163 |
| N-myristoylation sites: | Amino acids 18-24;133-139;242-248 |
| C-type lectin domain signature: | Amino acids 264-288 |
| Leucine zipper pattern: | Amino acids 102-124 |

MDTTRYSKWGGSSEEVPGGPWGRWVHWSRRPLFLALAVLVTTVLWAVILSILLSKASTERAALLDGHDLLRTNAS
KQTAALGALKEEVGDCHSCCSGTQAQLQTTRAELGEAQAKLMEQESALRELRERVTQGLAEAGRGREDVRTELFR
ALEAVRLQNNSCEPCPTSWLSFEGSCYFFSVPKTTWAAAQDHCADASAHLVIVGGLDEQGFLTRNTRGRGYWLGL
RAVRHLGKVQGYQWVDGVSLSFSHWNQGEPNDAWGRENCVMMLHTGLWNDAPCDSEKDGWICEKRHNC

FIGURE 59

GTCGAATCCAAATCACTCATTGTGAAAGCTGAGCTCACAGCCGAATAAGCCACC**ATG**AGGCTGTCAGTGTGTCTC
CTGATGGTCTCGCTGGCCCTTTGCTGCTACCAGGCCCATGCTCTTGTCTGCCCAGCTGTTGCTTCTGAGATCACA
GTCTTCTTATTCTTAAGTGACGCTGCGGTAAACCTCCAAGTTGCCAAACTTAATCCACCTCCAGAAGCTCTTGCA
GCCAAGTTGGAAGTGAAGCACTGCACCGATCAGATATCTTTTAAGAAACGACTCTCATTGAAAAAGTCCTGGTGGAA
A**TAG**TGAAAAAATGTGGTGTGTGACATGTAAAAATGCTCAACCTGGTTTCCAAAGTCTTTCAACGACACCCTGAT
CTTCACTAAAAATTGTAAAGGTTTCAACACGTTGCTTTAATAAATCACTTGCCCTGC

FIGURE 60

Signal peptide: Amino acids 1-15

cAMP- and cGMP-dependent protein kinase phosphorylation site: Amino acids 73-77

MRLSVCLLMVSLALCCYQAHALVCPAVASEITVFLFLSDAAVNLQVAKLNPPPEALAAKLEVKHCTDQISFKKRL
SLKKSWWK

FIGURE 61

GCGAGGTGGCGATCGCTGAGAGGCAGGAGGGCCGAGGCGGGCCTGGGAGGCGGCCCGGAGGTGGGGCGCCGCTGG
GGCCGGCCCGCACGGGCTTCATCTGAGGGCGCACGGCCCGCGACCGAGCGTGCGGACTGGCCTCCCAAGCGTGGG
GCGACAAGCTGCCGGAGCTGCAATGGGCCGCGGCTGGGGATTCTTGTTTGGCCTCCTGGGCGCCGTGTGGCTGCT
CAGCTCGGGCCACGGAGAGGAGCAGCCCCCGGAGACAGCGGCACAGAGGTGCTTCTGCCAGGTTAGTGGTTACTT
GGATGATTGTACCTGTGATGTTGAAACCATTGATAGATTTAATAACTACAGGCTTTTCCCAAGACTACAAAAACT
TCTTGAAAGTGACTACTTTAGGTATTACAAGGTAAACCTGAAGAGGCCGTGTCCTTTCTGGAATGACATCAGCCA
GTGTGGAAGAAGGGACTGTGCTGTCAAACCATGTCAATCTGATGAAGTTCCTGATGGAATTAAATCTGCGAGCTA
CAAGTATTCTGAAGAAGCCAATAATCTCATTGAAGAATGTGAACAAGCTGAACGACTTGGAGCAGTGGATGAATC
TCTGAGTGAGGAAACACAGAAGGCTGTTCTTCAGTGGACCAAGCATGATGATTCTTCAGATAACTTCTGTGAAGC
TGATGACATTCAGTCCCCTGAAGCTGAATATGTAGATTTGCTTCTTAATCCTGAGCGCTACACTGGTTACAAGGG
ACCAGATGCTTGGAAAATATGGAATGTCATCTACGAAGAAAACTGTTTTAAGCCACAGACAATTAAAAGACCTTT
AAATCCTTTGGCTTCTGGTCAAGGGACAAGTGAAGAGAACACTTTTTACAGTTGGCTAGAAGGTCTCTGTGTAGA
AAAAAGAGCATTCTACAGACTTATATCTGGCCTACATGCAAGCATTAATGTGCATTTGAGTGCAAGATATCTTTT
ACAAGAGACCTGGTTAGAAAAGAAATGGGGACACAACATTACAGAATTTCAACAGCGATTTGATGGAATTTTGAC
TGAAGGAGAAGGTCCAAGAAGGCTTAAGAACTTGTATTTTCTCTACTTAATAGAACTAAGGGCTTTATCCAAAGT
GTTACCATTCTTCGAGCGCCCAGATTTTCAACTCTTTACTGGAAATAAAATTCAGGATGAGGAAAACAAAATGTT
ACTTCTGGAAATACTTCATGAAATCAAGTCATTTCCTTTGCATTTTGATGAGAATTCATTTTTTGCTGGGGATAA
AAAAGAAGCACACAAACTAAAGGAGGACTTTCGACTGCATTTTAGAAATATTTCAAGAATTATGGATTGTGTTGG
TTGTTTTAAATGTCGTCTGTGGGGAAAGCTTCAGACTCAGGGTTTGGGCACTGCTCTGAAGATCTTATTTTCTGA
GAAATTGATAGCAAATATGCCAGAAAGTGGACCTAGTTATGAATTCCATCTAACCAGACAAGAAATAGTATCATT
ATTCAACGCATTTGGAAGAATTTCTACAAGTGTGAAAGAATTAGAAAACTTCAGGAACTTGTTACAGAATATTCA
TTAAAGAAAACAAGCTGATATGTGCCTGTTTCTGGACAATGGAGGCGAAAGAGTGGAATTTCATTCAAAGGCATA
ATAGCAATGACAGTCTTAAGCCAAACATTTTATATAAAGTTGCTTTTGTAAAGGAGAATTATATTGTTTTAAGTA
AACACATTTTTAAAAATTGTGTTAAGTCTATGTATAATACTACTGTGAGTAAAAGTAATACTTTAATAATGTGGT
ACAAATTTTAAAGTTTAATATTGAATAAAAGGAGGATTATCAAATTAAAAAAAAAAAAAAAAAAAAAAAAAAAAA
AAAAAAAAAA

**FIGURE 62**

| | |
|---|---|
| Signal peptide: | Amino acids 1-23 |
| N-glycosylation sites: | Amino acids 280-284;384-388 |
| Amidation site: | Amino acids 94-98 |
| Glycosaminoglycan attachment sites: | Amino acids 20-24;223-227 |
| Aminotransferases class-V pyridoxal-phosphate site: | Amino acids 216-223 |
| Interleukin-7 proteins site: | Amino acids 338-344 |

MGRGWGFLFGLLGAVWLLSSGHGEEQPPETAAQRCFCQVSGYLDDCTCDVETIDRFNNYRLFPRLQKLLESDYFR
YYKVNLKRPCPFWNDISQCGRPDCAVKPCQSDEVPDGIKSASYKYSEEANNLIEECEQAERLGAVDESLSEETQK
AVLQWTKHDDSSDNFCEADDIQSPEAEYVDLLLNPERYTGYKGPDAWKIWNVIYEENCFKPQTIKRPLNPLASGQ
GTSEENTFYSWLEGLCVEKRAFYRLISGLHASINVHLSARYLLQETWLEKKWGHNITEFQQRFDGILTEGEGPRR
LKNLYFLYLIELRALSKVLPFFERPDFQLFTGNKIQDEENKMLLLEILHEIKSFPLHFDENSFFAGDKKEAHKLK
EDFRLHFRNISRIMDCVGCFKCRLWGKLQTQGLGTALKILFSEKLIANMPESGPSYEFHLTRQEIVSLFNAFGRI
STSVKELENFRNLLQNIH

FIGURE 63

GAGAGGACGAGGTGCCGCTGCCTGGAGAATCCTCCGCTGCCGTCGGCTCCCGGAGCCCAGCCCTTTCCTAACCCA
ACCCAACCTAGCCCAGTCCCAGCCGCCAGCGCCTGTCCCTGTGACGGACCCCAGCGTTACCATGCATCCTGCCGT
CTTCCTATCCTTACCCGACCTCAGATGCTCCCTTCTGCTCCTGGTAACTTGGGTTTTTACTCCTGTAACAACTGA
AATAACAAGTCTTGCTACAGAGAATATAGATGAAATTTTAAACAATGCTGATGTTGCTTTAGTAAATTTTTATGC
TGACTGGTGTCGTTTCAGTCAGATGTTGCATCCAATTTTTGAGGAAGCTTCCGATGTCATTAAGGAAGAATTTCC
AAATGAAAATCAAGTAGTGTTTGCCAGAGTTGATTGTGATCAGCACTCTGACATAGCCCAGAGATACAGGATAAG
CAAATACCCAACCCTCAAATTGTTTCGTAATGGGATGATGATGAAGAGAGAATACAGGGGTCAGCGATCAGTGAA
AGCATTGGCAGATTACATCAGGCAACAAAAAAGTGACCCCATTCAAGAAATTCGGGACTTAGCAGAAATCACCAC
TCTTGATCGCAGCAAAAGAAATATCATTGGATATTTTGAGCAAAAGGACTCGGACAACTATAGAGTTTTTGAACG
AGTAGCGAATATTTTGCATGATGACTGTGCCTTTCTTTCTGCATTTGGGGATGTTTCAAAACCGGAAAGATATAG
TGGCGACAACATAATCTACAAACCACCAGGGCATTCTGCTCCGGATATGGTGTACTTGGGAGCTATGACAAATTT
TGATGTGACTTACAATTGGATTCAAGATAAATGTGTTCCTCTTGTCCGAGAAATAACATTTGAAAATGGAGAGGA
ATTGACAGAAGAAGGACTGCCTTTTCTCATACTCTTTCACATGAAAGAAGATACAGAAAGTTTAGAAATATTCCA
GAATGAAGTAGCTCGGCAATTAATAAGTGAAAAAGGTACAATAAACTTTTTACATGCCGATTGTGACAAATTTAG
ACATCCTCTTCTGCACATACAGAAAACTCCAGCAGATTGTCCTGTAATCGCTATTGACAGCTTTAGGCATATGTA
TGTGTTTGGAGACTTCAAAGATGTATTAATTCCTGGAAAACTCAAGCAATTCGTATTTGACTTACATTCTGGAAA
ACTGCACAGAGAATTCCATCATGGACCTGACCCAACTGATACAGCCCCAGGAGAGCAAGCCCAAGATGTAGCAAG
CAGTCCACCTGAGAGCTCCTTCCAGAAACTAGCACCCAGTGAATATAGGTATACTCTATTGAGGGATCGAGATGAGCT
TTAAAAACTTGAAAAACAGTTTGTAAGCCTTTCAACAGCAGCATCAACCTACGTGGTGGAAATAGTAAACCTATA
TTTTCATAATTCTATGTGTATTTTTATTTTGAATAAACAGAAAGAAATTTAAAAAAAAAAAAAAAAAAAAAAAAA
AAAAAAAAAAAAAAAAAAAA

FIGURE 64

Signal sequence: Amino acids 1-29

Tyrosine kinase phosphorylation site: Amino acids 203-212

N-myristoylation site: Amino acids 225-231

Endoplasmic reticulum targeting sequence: Amino acids 403-408

MHPAVFLSLPDLRCSLLLLVTWVFTPVTTEITSLATENIDEILNNADVALVNFYADWCRFSQMLHPIFEEASDVI
KEEFPNENQVVFARVDCDQHSDIAQRYRISKYPTLKLFRNGMMMKREYRGQRSVKALADYIRQQKSDPIQEIRDL
AEITTLDRSKRNIIGYFEQKDSDNYRVFERVANILHDDCAFLSAFGDVSKPERYSGDNIIYKPPGHSAPDMVYLG
AMTNFDVTYNWIQDKCVPLVREITFENGEELTEEGLPFLILFHMKEDTESLEIFQNEVARQLISEKGTINFLHAD
CDKFRHPLLHIQKTPADCPVIAIDSFRHMYVFGDFKDVLIPGKLKQFVFDLHSGKLHREFHHGPDPTDTAPGEQA
QDVASSPPESSFQKLAPSEYRYTLLRDRDEL

FIGURE 65

GAGGATTTGCCACAGCAGCGGATAGAGCAGGAGAGCACCACCGGAGCCCTTGAGACATCCTTGAGAAGAGCCACA
GCATAAGAGACTGCCCTGCTTGGTGTTTTGCAGG**ATG**ATGGTGGCCCTTCGAGGAGCTTCTGCATTGCTGGTTCT
GTTCCTTGCAGCTTTTCTGCCCCCGCCGCAGTGTACCCAGGACCCAGCCATGGTGCATTACATCTACCAGCGCTT
TCGAGTCTTGGAGCAAGGGCTGGAAAAATGTACCCAAGCAACGAGGGCATACATTCAAGAATTCCAAGAGTTCTC
AAAAAATATATCTGTCATGCTGGGAAGATGTCAGACCTACACAAGTGAGTACAAGAGTGCAGTGGGTAACTTGGC
ACTGAGAGTTGAACGTGCCCAACGGGAGATTGACTACATACAATACCTTCGAGAGGCTGACGAGTGCATCGTATC
AGAGGACAAGACACTGGCAGAAATGTTGCTCCAAGAAGCTGAAGAAGAGAAAAAGATCCGGACTCTGCTGAATGC
AAGCTGTGACAACATGCTGATGGGCATAAAGTCTTTGAAAATAGTGAAGAAGATGATGGACACACATGGCTCTTG
GATGAAAGATGCTGTCTATAACTCTCCAAAGGTGTACTTATTAATTGGATCCAGAAACAACACTGTTTGGGAATT
TGCAAACATACGGGCATTCATGGAGGATAACACCAAGCCAGCTCCCCGGAAGCAAATCCTAACACTTTCCTGGCA
GGGAACAGGCCAAGTGATCTACAAAGGTTTTCTATTTTTTCATAACCAAGCAACTTCTAATGAGATAATCAAATA
TAACCTGCAGAAGAGGACTGTGGAAGATCGAATGCTGCTCCCAGGAGGGGTAGGCCGAGCATTGGTTTACCAGCA
CTCCCCCTCAACTTACATTGACCTGGCTGTGGATGAGCATGGGCTCTGGGCCATCCACTCTGGGCCAGGCACCCA
TAGCCATTTGGTTCTCACAAAGATTGAGCCGGGCACACTGGGAGTGGAGCATTCATGGGATACCCCATGCAGAAG
CCAGGATGCTGAAGCCTCATTCCTCTTGTGTGGGGTTCTCTATGTGGTCTACAGTACTGGGGGCCAGGGCCCTCA
TCGCATCACCTGCATCTATGATCCACTGGGCACTATCAGTGAGGAGGACTTGCCCAACTTGTTCTTCCCCAAGAG
ACCAAGAAGTCACTCCATGATCCATTACAACCCCAGAGATAAGCAGCTCTATGCCTGGAATGAAGGAAACCAGAT
CATTTACAAACTCCAGACAAAGAGAAAGCTGCCTCTGAAG**TAA**TGCATTACAGCTGTGAGAAAGAGCACTGTGGC
TTTGGCAGCTGTTCTACAGGACAGTGAGGCTATAGCCCCTTCACAATATAGTATCCCTCTAATCACACACAGGAA
GAGTGTGTAGAAGTGGAAATACGTATGCCTCCTTTCCCAAATGTCACTGCCTTAGGTATCTTCCAAGAGCTTAGA
TGAGAGCATATCATCAGGAAAGTTTCAACAATGTCCATTACTCCCCCAAACCTCCTGGCTCTCAAGGATGACCAC
ATTCTGATACAGCCTACTTCAAGCCTTTTGTTTTACTGCTCCCCAGCATTTACTGTAACTCTGCCATCTTCCCTC
CCACAATTAGAGTTGTATGCCAGCCCCTAATATTCACCACTGGCTTTTCTCTCCCCTGGCCTTTGCTGAAGCTCT
TCCCTCTTTTTCAAATGTCTATTGATATTCTCCCATTTTCACTGCCCAACTAAAATACTATTAATATTTCTTTCT
TTTCTTTTCTTTTTTTTGAGACAAGGTCTCACTATGTTGCCCAGGCTGGTCTCAAACTCCAGAGCTCAAGAGATC
CTCCTGCCTCAGCCTCCTAAGTACCTGGGATTACAGGCATGTGCCACCACACCTGGCTTAAAATACTATTTCTTA
TTGAGGTTTAACCTCTATTTCCCCTAGCCCTGTCCTTCCACTAAGCTTGGTAGATGTAATAATAAAGTGAAAATA
TTAACATTTGAATATCGCTTTCCAGGTGTGGAGTGTTTGCACATCATTGAATTCTCGTTTCACCTTTGTGAAACA
TGCACAAGTCTTTACAGCTGTCATTCTAGAGTTTAGGTGAGTAACACAATTACAAAGTGAAAGATACAGCTAGAA
AATACTACAAATCCCATAGTTTTTCCATTGCCCAAGGAAGCATCAAATACGTATGTTTGTTCACCTACTCTTATA
GTCAATGCGTTCATCGTTTCAGCCTAAAAATAATAGTCTGTCCCTTTAGCCAGTTTTCATGTCTGCACAAGACCT
TTCAATAGGCCTTTCAAATGATAATTCCTCCAGAAAACCAGTCTAAGGGTGAGGACCCCAACTCTAGCCTCCTCT
TGTCTTGCTGTCCTCTGTTTCTCTCTTTCTGCTTTAAATTCAATAAAAGTGACACTGAGCAAAAAAAAAAAAAA

FIGURE 66

Signal peptide: Amino acids 1-25

N-glycosylation sites: Amino acids 66-70;138-142;183-187

MMVALRGASALLVLFLAAFLPPPQCTQDPAMVHYIYQRFRVLEQGLEKCTQATRAYIQEFQEFSKNISVMLGRCQ
TYTSEYKSAVGNLALRVERAQREIDYIQYLREADECIVSEDKTLAEMLLQEAEEEKKIRTLLNASCDNMLMGIKS
LKIVKKMMDTHGSWMKDAVYNSPKVYLLIGSRNNTVWEFANIRAFMEDNTKPAPRKQILTLSWQGTGQVIYKGFL
FFHNQATSNEIIKYNLQKRTVEDRMLLPGGVGRALVYQHSPSTYIDLAVDEHGLWAIHSGPGTHSHLVLTKIEPG
TLGVEHSWDTPCRSQDAEASFLLCGVLYVVYSTGGQGPHRITCIYDPLGTISEEDLPNLFFPKRPRSHSMIHYNP
RDKQLYAWNEGNQIIYKLQTKRKLPLK

FIGURE 67

GTTGATGGCAAACTTCCTCAAAGGAGGGGCAGAGCCTGCGCAGGGCAGGAGCAGCTGGCCCACTGGCGGCCCGCA
ACACTCCGTCTCACCCTCTGGGCCCACTGCATCTAGAGGAGGGCCGTCTGTGAGGCCACTACCCCTCCAGCAACT
GGGAGGTGGGACTGTCAGAAGCTGGCCCAGGGTGGTGGTCAGCTGGGTCAGGGACCTACGGCACCTGCTGGACCA
CCTCGCCTTCTCCATCGAAGCAGGGAAGTGGGAGCCTCGAGCCCTCGGGTGGAAGCTGACCCCAAGCCACCCTTC
ACCTGGACAGGATGAGAGTGTCAGGTGTGCTTCGCCTCCTGGCCCTCATCTTTGCCATAGTCACGACATGGATGT
TTATTCGAAGCTACATGAGCTTCAGCATGAAAACCATCCGTCTGCCACGCTGGCTGGCAGCCTCGCCCACCAAGG
AGATCCAGGTTAAAAAGTACAAGTGTGGCCTCATCAAGCCCTGCCCAGCCAACTACTTTGCGTTTAAAATCTGCA
GTGGGGCCGCCAACGTCGTGGGCCCTACTATGTGCTTTGAAGACCGCATGATCATGAGTCCTGTGAAAAACAATG
TGGGCAGAGGCCTAAACATCGCCCTGGTGAATGGAACCACGGGAGCTGTGCTGGGACAGAAGGCATTTGACATGT
ACTCTGGAGATGTTATGCACCTAGTGAAATTCCTTAAAGAAATTCCGGGGGGTGCACTGGTGCTGGTGGCCTCCT
ACGACGATCCAGGGACCAAAATGAACGATGAAAGCAGGAAACTCTTCTCTGACTTGGGGAGTTCCTACGCAAAAC
AACTGGGCTTCCGGGACAGCTGGGTCTTCATAGGAGCCAAAGACCTCAGGGGTAAAAGCCCCTTTGAGCAGTTCT
TAAAGAACAGCCCAGACACAAACAAATACGAGGGATGGCCAGAGCTGCTGGAGATGGAGGGCTGCATGCCCCCGA
AGCCATTT**TAG**GGTGGCTGTGGCTCTTCCTCAGCCAGGGGCCTGAAGAAGCTCCTGCCTGACTTAGGAGTCAGAG
CCCGGCAGGGGCTGAGGAGGAGGAGCAGGGGGTGCTGCGTGGAAGGTGCTGCAGGTCCTTGCACGCTGTGTCGCG
CCTCTCCTCCTCGGAAACAGAACCCTCCCACAGCACATCCTACCCGGAAGACCAGCCTCAGAGGGTCCTTCTGGA
ACCAGCTGTCTGTGGAGAGAATGGGGTGCTTTCGTCAGGGACTGCTGACGGCTGGTCCTGAGGAAGGACAAACTG
CCCAGACTTGAGCCCAATTAAATTTTATTTTTGCTGGTTTTGAAAAAAAAAAAAAAAAAAAA

FIGURE 68

| | |
|---|---|
| Signal peptide: | Amino acids 1-15 |
| ATP/GTP-binding site motif A (P-loop): | Amino acids 184-192 |
| N-glycosylation site: | Amino acids 107-111 |

MRVSGVLRLLALIFAIVTTWMFIRSYMSFSMKTIRLPRWLAASPTKEIQVKKYKCGLIKPCPANYFAFKICSGAA
NVVGPTMCFEDRMIMSPVKNNVGRGLNIALVNGTTGAVLGQKAFDMYSGDVMHLVKFLKEIPGGALVLVASYDDP
GTKMNDESRKLFSDLGSSYAKQLGFRDSWVFIGAKDLRGKSPFEQFLKNSPDTNKYEGWPELLEMEGCMPPKPF

FIGURE 69

GGGGGAGCTAGGCCGGCGGCAGTGGTGGTGGCGGCGGCGCAAGGGTGAGGGCGGCCCCAGAACCCCAGGTAGGTA
GAGCAAGAAGATGGTGTTTCTGCCCCTCAAATGGTCCCTTGCAACCATGTCATTTCTACTTTCCTCACTGTTGGC
TCTCTTAACTGTGTCCACTCCTTCATGGTGTCAGAGCACTGAAGCATCTCCAAAACGTAGTGATGGACACCATT
TCCTTGGAATAAAATACGACTTCCTGAGTACGTCATCCCAGTTCATTATGATCTCTTGATCCATGCAAACCTTAC
CACGCTGACCTTCTGGGGAACCACGAAAGTAGAAATCACAGCCAGTCAGCCCACCAGCACCATCATCCTGCATAG
TCACCACCTGCAGATATCTAGGGCCACCCTCAGGAAGGGAGCTGGAGAGAGGCTATCGGAAGAACCCCTGCAGGT
CCTGGAACACCCCCCTCAGGAGCAAATTGCACTGCTGGCTCCCGAGCCCCTCCTTGTCGGGCTCCCGTACACAGT
TGTCATTCACTATGCTGGCAATCTTTCGGAGACTTTCCACGGATTTTACAAAAGCACCTACAGAACCAAGGAAGG
GGAACTGAGGATACTAGCATCAACACAATTTGAACCCACTGCAGCTAGAATGGCCTTTCCCTGCTTTGATGAACC
TGCCTTCAAAGCAAGTTTCTCAATCAAAATTAGAAGAGAGCCAAGGCACCTAGCCATCTCCAATATGCCATTGGT
GAAATCTGTGACTGTTGCTGAAGGACTCATAGAAGACCATTTTGATGTCACTGTGAAGATGAGCACCTATCTGGT
GGCCTTCATCATTTCAGATTTTGAGTCTGTCAGCAAGATAACCAAGAGTGGAGTCAAGGTTTCTGTTTATGCTGT
GCCAGACAAGATAAATCAAGCAGATTATGCACTGGATGCTGCGGTGACTCTTCTAGAATTTTATGAGGATTATTT
CAGCATACCGTATCCCCTACCCAAACAAGATCTTGCTGCTATTCCCGACTTTCAGTCTGGTGCTATGGAAAACTG
GGGACTGACAACATATAGAGAATCTGCTCTGTTGTTTGATGCAGAAAAGTCTTCTGCATCAAGTAAGCTTGGCAT
CACAGTGACTGTGGCCCATGAACTGGCCCACCAGTGGTTTGGGAACCTGGTCACTATGGAATGGTGGAATGATCT
TTGGCTAAATGAAGGATTTGCCAAATTTATGGAGTTTGTGTCTGTCAGTGTGACCCATCCTGAACTGAAAGTTGG
AGATTATTTCTTTGGCAAATGTTTTGACGCAATGGAGGTAGATGCTTTAAATTCCTCACACCCTGTGTCTACACC
TGTGGAAAATCCTGCTCAGATCCGGGAGATGTTTGATGATGTTTCTTATGATAAGGGAGCTTGTATTCTGAATAT
GCTAAGGGAGTATCTTAGCGCTGACGCATTTAAAAGTGGTATTGTACAGTATCTCCAGAAGCATAGCTATAAAAA
TACAAAAAACGAGGACCTGTGGGATAGTATGGCAAGTATTTGCCCTACAGATGGTGTAAAAGGGATGGATGGCTT
TTGCTCTAGAAGTCAACATTCATCTTCATCCTCACATTGGCATCAGGAAGGGGTGGATGTGAAAACCATGATGAA
CACTTGGACACTGCAGAGGGGTTTTCCCCTAATAACCATCACAGTGAGGGGGAGGAATGTACACATGAAGCAAGA
GCACTACATGAAGGGCTCTGACGGCGCCCCGGACACTGGGTACCTGTGGCATGTTCCATTGACATTCATCACCAG
CAAATCCAACATGGTCCATCGATTTTTGCTAAAAACAAAAACAGATGTGCTCATCCTCCCAGAAGAGGTGGAATG
GATCAAATTTAATGTGGGCATGAATGGCTATTACATTGTGCATTACGAGGATGATGGATGGGACTCTTTGACTGG
CCTTTTAAAAGGAACACACACAGCAGTCAGCAGTAATGATCGGGCAAGTCTCATTAACAATGCATTTCAGCTCGT
CAGCATTGGGAAGCTGTCCATTGAAAAGGCCTTGGATTTATCCCTGTACTTGAAACATGAAACTGAAATTATGCC
CGTGTTTCAAGGTTTGAATGAGCTGATTCCTATGTATAAGTTAATGGAGAAAAGAGATATGAATGAAGTGGAAAC
TCAATTCAAGGCCTTCCTCATCAGGCTGCTAAGGGACCTCATTGATAAGCAGACATGGACAGACGAGGGCTCAGT
CTCAGAGCAAATGCTGCGGAGTGAACTACTACTCCTCGCCTGTGTGCACAACTATCAGCCGTGCGTACAGAGGGC
AGAAGGCTATTTCAGAAAGTGGAAGGAATCCAATGGAAACTTGAGCCTGCCTGTCGACGTGACCTTGGCAGTGTT
TGCTGTGGGGGCCCAGAGCACAGAAGGCTGGGATTTTCTTTATAGTAAATATCAGTTTTCTTTGTCCAGTACTGA
GAAAAGCCAAATTGAATTTGCCCTCTGCAGAACCCAAAATAAGGAAAAGCTTCAATGGCTACTAGATGAAAGCTT
TAAGGGAGATAAAATAAAAACTCAGGAGTTTCCACAAATTCTTACACTCATTGGCAGGAACCCAGTAGGATACCC
ACTGGCCTGGCAATTTCTGAGGAAAAACTGGAACAAACTTGTACAAAAGTTTGAACTTGGCTCATCTTCCATAGC
CCACATGGTAATGGGTACAACAAATCAATTCTCCACAAGAACACGGCTTGAAGAGGTAAAAGGATTCTTCAGCTC
TTTGAAAGAAAATGGTTCTCAGCTCCGTTGTGTCCAACAGACAATTGAAACCATTGAAGAAAACATCGGTTGGAT
GGATAAGAATTTTGATAAAATCAGAGTGTGGCTGCAAAGTGAAAAGCTTGAACGTATGTAAAAATTCCTCCCTTG
CCCGGTTCCTGTTATCTCTAATCACCAACATTTTGTTGAGTGTATTTTCAAACTAGAGATGGCTGTTTTGGCTCC
AACTGGAGATACTTTTTTCCCTTCAACTCATTTTTTGACTATCCCTGTGAAAAGAATAGCTGTTAGTTTTTCATG
AATGGGCTTTTTCATGAATGGGCTATCGCTACCATGTGTTTTGTTCATCACAGGTGTTGCCCTGCAACGTAAACC
CAAGTGTTGGGTTCCCTGCCACAGAAGAATAAAGTACCTTATTCTTCTCAAAAAAAAAAAAAAAAAAAAAAAAAA
A

FIGURE 70

Signal peptide: Amino acids 1-34

N-glycosylation sites: Amino acids 70-74;154-158;414-418;760-764;901-905

Neutral zinc metallopeptidases, zinc-binding region signature: Amino acids 350-360

MVFLPLKWSLATMSFLLSSLLALLTVSTPSWCQSTEASPKRSDGTPFPWNKIRLPEYVIPVHYDLLIHANLTTLT
FWGTTKVEITASQPTSTIILHSHHLQISRATLRKGAGERLSEEPLQVLEHPPQEQIALLAPEPLLVGLPYTVVIH
YAGNLSETFHGFYKSTYRTKEGELRILASTQFEPTAARMAFPCFDEPAFKASFSIKIRREPRHLAISNMPLVKSV
TVAEGLIEDHFDVTVKMSTYLVAFIISDFESVSKITKSGVKVSVYAVPDKINQADYALDAAVTLLEFYEDYFSIP
YPLPKQDLAAIPDFQSGAMENWGLTTYRESALLFDAEKSSASSKLGITVTVAHELAHQWFGNLVTMEWWNDLWLN
EGFAKFMEFVSVSVTHPELKVGDYFFGKCFDAMEVDALNSSHPVSTPVENPAQIREMFDDVSYDKGACILNMLRE
YLSADAFKSGIVQYLQKHSYKNTKNEDLWDSMASICPTDGVKGMDGFCSRSQHSSSSSHWHQEGVDVKTMMNTWT
LQRGFPLITITVRGRNVHMKQEHYMKGSDGAPDTGYLWHVPLTFITSKSNMVHRFLLKTKTDVLILPEEVEWIKF
NVGMNGYYIVHYEDDGWDSLTGLLKGTHTAVSSNDRASLINNAFQLVSIGKLSIEKALDLSLYLKHETEIMPVFQ
GLNELIPMYKLMEKRDMNEVETQFKAFLIRLLRDLIDKQTWTDEGSVSEQMLRSELLLLACVHNYQPCVQRAEGY
FRKWKESNGNLSLPVDVTLAVFAVGAQSTEGWDFLYSKYQFSLSSTEKSQIEFALCRTQNKEKLQWLLDESFKGD
KIKTQEFPQILTLIGRNPVGYPLAWQFLRKNWNKLVQKFELGSSSIAHMVMGTTNQFSTRTRLEEVKGFFSSLKE
NGSQLRCVQQTIETIEENIGWMDKNFDKIRVWLQSEKLERM

FIGURE 71

GAGCGAAC<u>ATG</u>GCAGCGCGTTGGCGGTTTTGGTGTGTCTCTGTGACCATGGTGGTGGCGCTGCTCATCGTTTGCG
ACGTTCCCTCAGCCTCTGCCCAAAGAAAGAAGGAGATGGTGTTATCTGAAAAGGTTAGTCAGCTGATGGAATGGA
CTAACAAAAGACCTGTAATAAGAATGAATGGAGACAAGTTCCGTCGCCTTGTGAAAGCCCCACCGAGAAATTACT
CCGTTATCGTCATGTTCACTGCTCTCCAACTGCATAGACAGTGTGTCGTTTGCAAGCAAGCTGATGAAGAATTCC
AGATCCTGGCAAACTCCTGGCGATACTCCAGTGCATTCACCAACAGGATATTTTTTGCCATGGTGGATTTTGATG
AAGGCTCTGATGTATTTCAGATGCTAAACATGAATTCAGCTCCAACTTTCATCAACTTTCCTGCAAAAGGGAAAC
CCAAACGGGGTGATACATATGAGTTACAGGTGCGGGGTTTTTCAGCTGAGCAGATTGCCCGGTGGATCGCCGACA
GAACTGATGTCAATATTAGAGTGATTAGACCCCCAAATTATGCTGGTCCCCTTATGTTGGGATTGCTTTTGGCTG
TTATTGGTGGACTTGTGTATCTTCGAAGAAGTAATATGGAATTTCTCTTTAATAAAACTGGATGGGCTTTTGCAG
CTTTGTGTTTTGTGCTTGCTATGACATCTGGTCAAATGTGGAACCATATAAGAGGACCACCATATGCCCATAAGA
ATCCCCACACGGGACATGTGAATTATATCCATGGAAGCAGTCAAGCCCAGTTTGTAGCTGAAACACACATTGTTC
TTCTGTTTAATGGTGGAGTTACCTTAGGAATGGTGCTTTTATGTGAAGCTGCTACCTCTGACATGGATATTGGAA
AGCGAAAGATAATGTGTGTGGCTGGTATTGGACTTGTTGTATTATTCTTCAGTTGGATGCTCTCTATTTTTAGAT
CTAAATATCATGGCTACCCATACAGCTTTCTGATGAGT<u>TAA</u>AAAGGTCCCAGAGATATATAGACACTGGAGTACT
GGAAATTGAAAAACGAAAATCGTGTGTGTTTGAAAAGAAGAATGCAACTTGTATATTTTGTATTACCTCTTTTTT
TCAAGTGATTTAAATAGTTAATCATTTAACCAAAGAAGATGTGTAGTGCCTTAACAAGCAATCCTCTGTCAAAAT
CTGAGGTATTTGAAAATAATTATCCTCTTAACCTTCTCTTCCCAGTGAACTTTATGGAACATTTAATTTAGTACA
ATTAAGTATATTATAAAAATTGTAAAACTACTACTTTGTTTTAGTTAGAACAAAGCTCAAAACTACTTTAGTTAA
CTTGGTCATCTGATTTTATATTGCCTTATCCAAAGATGGGGAAAGTAAGTCCTGACCAGGTGTTCCCACATATGC
CTGTTACAGATAACTACATTAGGAATTCATTCTTAGCTTCTTCATCTTTGTGTGGATGTGTATACTTTACGCATC
TTTCCTTTTGAGTAGAGAAATTATGTGTGTCATGTGGTCTTCTGAAAATGGAACACCATTCTTCAGAGCACACGT
CTAGCCCTCAGCAAGACAGTTGTTTCTCCTCCTCCTTGCATATTTCCTACTGCGCTCCAGCCTGAGTGATAGAGT
GAGACTCTGTCTCAAAAAAAAGTATCTCTAAATACAGGATTATAATTTCTGCTTGAGTATGGTGTTAACTACCTT
GTATTTAGAAAGATTTCAGATTCATTCCATCTCCTTAGTTTTCTTTTAAGGTGACCCATCTGTGATAAAAATATA
GCTTAGTGCTAAAATCAGTGTAACTTATACATGGCCTAAAATGTTTCTACAAATTAGAGTTTGTCACTTATTCCA
TTTGTACCTAAGAGAAAAATAGGCTCAGTTAGAAAAGGACTCCCTGGCCAGGCGCAGTGACTTACGCCTGTAATC
TCAGCACTTTGGGAGGCCAAGGCAGGCAGATCACGAGGTCAGGAGTTCGAGACCATCCTGGCCAACATGGTGAAA
CCCCGTCTCTACTAAAAATATAAAAATTAGCTGGGTGTGGTGGCAGGAGCCTGTAATCCCAGCTACACAGGAGGC
TGAGGCACGAGAATCACTTGAACTCAGGAGATGGAGGTTTCAGTGAGCCGAGATCACGCCACTGCACTCCAGCCT
GGCAACAGAGCGAGACTCCATCTCAAAAAAAAAAAAAA

## FIGURE 72

| | |
|---|---|
| Signal peptide: | Amino acids 1-29 |
| Transmembrane domains: | Amino acids 183-205;217-237;<br>217-287;301-321 |
| N-glycosylation sites: | Amino acids 71-75;215-219 |
| Cell attachment sequence: | Amino acids 150-153 |

MAARWRFWCVSVTMVVALLIVCDVPSASAQRKKEMVLSEKVSQLMEWTNKRPVIRMNGDKFRRLVKAPPRNYSVI
VMFTALQLHRQCVVCKQADEEFQILANSWRYSSAFTNRIFFAMVDFDEGSDVFQMLNMNSAPTFINFPAKGKPKR
GDTYELQVRGFSAEQIARWIADRTDVNIRVIRPPNYAGPLMLGLLLAVIGGLVYLRRSNMEFLFNKTGWAFAALC
FVLAMTSGQMWNHIRGPPYAHKNPHTGHVNYIHGSSQAQFVAETHIVLLFNGGVTLGMVLLCEAATSDMDIGKRK
IMCVAGIGLVVLFFSWMLSIFRSKYHGYPYSFLMS

FIGURE 73

AAGCAACCAAACTGCAAGCTTTGGGAGTTGTTCGCTGTCCCTGCCCTGCTCTGCTAGGGAGAGAACGCCAGAGGG
AGGCGGCTGGCCCGGCGGCAGGCTCTCAGAACCGCTACCGGCGATGCTACTGCTGTGGGTGTCGGTGGTCGCAGC
CTTGGCGCTGGCGGTACTGGCCCCCGGAGCAGGGGAGCAGAGGCGGAGAGCAGCCAAAGCGCCCAATGTGGTGCT
GGTCGTGAGCGACTCCTTCGATGGAAGGTTAACATTTCATCCAGGAAGTCAGGTAGTGAAACTTCCTTTTATCAA
CTTTATGAAGACACGTGGGACTTCCTTTCTGAATGCCTACACAAACTCTCCAATTTGTTGCCCATCACGCGCAGC
AATGTGGAGTGGCCTCTTCACTCACTTAACAGAATCTTGGAATAATTTTAAGGGTCTAGATCCAAATTATACAAC
ATGGATGGATGTCATGGAGAGGCATGGCTACCGAACACAGAAATTTGGGAAACTGGACTATACTTCAGGACATCA
CTCCATTAGTAATCGTGTGGAAGCGTGGACAAGAGATGTTGCTTTCTTACTCAGACAAGAAGGCAGGCCCATGGT
TAATCTTATCCGTAACAGGACTAAAGTCAGAGTGATGGAAAGGGATTGGCAGAATACAGACAAAGCAGTAAACTG
GTTAAGAAAGGAAGCAATTAATTACACTGAACCATTTGTTATTTACTTGGGATTAAATTTACCACACCCTTACCC
TTCACCATCTTCTGGAGAAAATTTTGGATCTTCAACATTTCACACATCTCTTTATTGGCTTGAAAAAGTGTCTCA
TGATGCCATCAAAATCCCAAAGTGGTCACCTTTGTCAGAAATGCACCCTGTAGATTATTACTCTTCTTATACAAA
AAACTGCACTGGAAGATTTACAAAAAAAGAAATTAAGAATATTAGAGCATTTTATTATGCTATGTGTGCTGAGAC
AGATGCCATGCTTGGTGAAATTATTTTGGCCCTTCATCAATTAGATCTTCTTCAGAAAACTATTGTCATATACTC
CTCAGACCATGGAGAGCTGGCCATGGAACATCGACAGTTTTATAAAATGAGCATGTACGAGGCTAGTGCACATGT
TCCGCTTTTGATGATGGGACCAGGAATTAAAGCCGGCCTACAAGTATCAAATGTGGTTTCTCTTGTGGATATTTA
CCCTACCATGCTTGATATTGCTGGAATTCCTCTGCCTCAGAACCTGAGTGGATACTCTTTGTTGCCGTTATCATC
AGAAACATTTAAGAATGAACATAAAGTCAAAAACCTGCATCCACCCTGGATTCTGAGTGAATTCCATGGATGTAA
TGTGAATGCCTCCACCTACATGCTTCGAACTAACCACTGGAAATATATAGCCTATTCGGATGGTGCATCAATATT
GCCTCAACTCTTTGATCTTTCCTCGGATCCAGATGAATTAACAAATGTTGCTGTAAAATTTCCAGAAATTACTTA
TTCTTTGGATCAGAAGCTTCATTCCATTATAAACTACCCTAAAGTTTCTGCTTCTGTCCACCAGTATAATAAAGA
GCAGTTTATCAAGTGGAAACAAAGTATAGGACAGAATTATTCAAACGTTATAGCAAATCTTAGGTGGCACCAAGA
CTGGCAGAAGGAACCAAGGAAGTATGAAAATGCAATTGATCAGTGGCTTAAAACCCATATGAATCCAAGAGCAGT
TTGAACAAAAAGTTTAAAAATAGTGTTCTAGAGATACATATAAATATATTACAAGATCATAATTATGTATTTTAA
ATGAAACAGTTTTAATAATTACCAAGTTTTGGCCGGGCACAGTGGCTCACACCTGTAATCCCAGGACTTTGGGAG
GCTGAGGAAAGCAGATCACAAGGTCAAGAGATTGAGACCATCCTGGCCAACATGGTGAAACCCTGTCTCTACTAA
AAATACAAAAATTAGCTGGGCGCGGTGGTGCACACCTATAGTCTCAGCTACTCAGAGGCTGAGGCAGGAGGATCG
CTTGAACCCGGGAGGCAGCAGTTGCAGTGAGCTGAGATTGCGCCACTGTACTCCAGCCTGGCAACAGAGTGAGAC
TGTGTCGCAAAAAAATAAAAATAAAATAATAATAATTACCAATTTTTCATTATTTTGTAAGAATGTAGTGTATTT
TAAGATAAAATGCCAATGATTATAAAATCACATATTTTCAAAAATGGTTATTATTTAGGCCTTTGTACAATTTCT
AACAATTTAGTGGAAGTATCAAAAGGATTGAAGCAAATACTGTAACAGTTATGTTCCTTTAAATAATAGAGAATA
TAAAATATTGTAATAATATGTATCATAAAATAGTTGTATGTGAGCATTTGATGGTGAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAA

# FIGURE 74

| | |
|---|---|
| Signal peptide: | Amino acids 1-15 |
| N-glycosylation sites: | Amino acids 108-112;166-170; 193-197;262-266;375-379;413-417; 498-502 |
| Sulfatases proteins Homology Blocks: | Amino acids 286-316;359-370; 78-98 |

MLLLWVSVVAALALAVLAPGAGEQRRRAAKAPNVVLVVSDSFDGRLTFHPGSQVVKLPFINFMKTRGTSFLNAYT
NSPICCPSRAAMWSGLFTHLTESWNNFKGLDPNYTTWMDVMERHGYRTQKFGKLDYTSGHHSISNRVEAWTRDVA
FLLRQEGRPMVNLIRNRTKVRVMERDWQNTDKAVNWLRKEAINYTEPFVIYLGLNLPHPYPSPSSGENFGSSTFH
TSLYWLEKVSHDAIKIPKWSPLSEMHPVDYYSSYTKNCTGRFTKKEIKNIRAFYYAMCAETDAMLGEIILALHQL
DLLQKTIVIYSSDHGELAMEHRQFYKMSMYEASAHVPLLMMGPGIKAGLQVSNVVSLVDIYPTMLDIAGIPLPQN
LSGYSLLPLSSETFKNEHKVKNLHPPWILSEFHGCNVNASTYMLRTNHWKYIAYSDGASILPQLFDLSSDPDELT
NVAVKFPEITYSLDQKLHSIINYPKVSASVHQYNKEQFIKWKQSIGQNYSNVIANLRWHQDWQKEPRKYENAIDQ
WLKTHMNPRAV

FIGURE 75

CTCCACTGCAACCACCCAGAGCCATGGCTCCCCGAGGCTGCATCGTAGCTGTCTTTGCCATTTTCTGCATCTCCA
GGCTCCTCTGCTCACACGGAGCCCCAGTGGCCCCCATGACTCGTTACCTGATGCTGTGCCAGCCACACAAGAGAT
GTGGGGACAAGTTCTACGACCCCCTGCAGCACTGTTGCTATGATGATGCCGTCGTGCCCTTGGCCAGGACCCAGA
CGTGTGGAAACTGCACCTTCAGAGTCTGCTTTGAGCAGTGCTGCCCCTGGACCTTCATGGTGAAGCTGATAAACC
AGAACTGCGACTCAGCCCGGACCTCGGATGACAGGCTTTGTCGCAGTGTCAGCTAATGGAACATCAGGGGAACGA
TGACTCCTGGATTCTCCTTCCTGGGTGGGCCTGGAGAAAGAGGCTGGTGTTACCTGAGATCTGGGATGCTGAGTG
GCTGTTTGGGGGCCAGAGAAACACACACTCAACTGCCCACTTCATTCTGTGACCTGTCTGAGGCCCACCCTGCAG
CTGCCCTGAGGAGGCCCACAGGTCCCCTTCTAGAATTCTGGACAGCATGAGATGCGTGTGCTGATGGGGGCCCAG
GGACTCTGAACCCTCCTGATGACCCCTATGGCCAACATCAACCCGGCACCACCCCAAGGCTGGCTGGGGAACCCT
TCACCCTTCTGTGAGATTTTCCATCATCTCAAGTTCTCTTCTATCCAGGAGCAAAGCACAGGATCATAATAAATT
TATGTACTTTATAAATGAAAA

## FIGURE 76

| | |
|---|---|
| Signal peptide: | Amino acids 1-24 |
| N-glycosylation site: | Amino acids 71-75 |
| Insulin family proteins Homology Blocks: | Amino acids 76-96;42-61 |

MAPRGCIVAVFAIFCISRLLCSHGAPVAPMTPYLMLCQPHKRCGDKFYDPLQHCCYDDAVVPLARTQTCGNCTFR
VCFEQCCPWTFMVKLINQNCDSARTSDDRLCRSVS

FIGURE 77

CTAGCCTGCGCCAAGGGGTAGTGAGACCGCGCGGCAACAGCTTGCGGCTGCGGGGAGCTCCCGTGGGCGCTCCGC
TGGCTGTGCAGGCGGCC**ATG**GATTCCTTGCGGAAAATGCTGATCTCAGTCGCAATGCTGGGCGCAGGGGCTGGCG
TGGGCTACGCGCTCCTCGTTATCGTGACCCCGGGAGAGCGGCGGAAGCAGGAAATGCTAAAGGAGATGCCACTGC
AGGACCCAAGGAGCAGGGAGGAGGCGGCCAGGACCCAGCAGCTATTGCTGGCCACTCTGCAGGAGGCAGCGACCA
CGCAGGAGAACGTGGCCTGGAGGAAGAACTGGATGGTTGGCGGCGAAGGCGGCGCCAGCGGGAGGTCACCGTGAG
ACCGGACTTGCCTCCGTGGGCGCCGGACCTTGGCTTGGGCGCAGGAATCCGAGGCAGCCTTTCTCCTTCGTGGGC
CCAGCGGAGAGTCCGGACCGAGATACCATGCCAGGACTCTCCGGGGTCCTGTGAGCTGCCGTCGGGTGAGCACGT
TTCCCCCAAACCCTGGACTGACTGCTTTAAGGTCCGCAAGGCGGGCCAGGGCCGAGACGCGAGTCGGATGTGGTG
AACTGAAAGAACCAATAAAATCATGTTCCTCCAAAAAAAAAAAAAAAAAAAAAAAAAAAAAAAAAAAAAAAAAAA
AAAAAAAAAAAAAAAAAA

FIGURE 78

| | |
|---|---|
| Signal peptide: | Amino acids 1-18 |
| N-myristoylation sites: | Amino acids 15-21;17-23;19-25;<br>83-89;86-92 |

MDSLRKMLISVAMLGAGAGVGYALLVIVTPGERRKQEMLKEMPLQDPRSREEAARTQQLLLATLQEAATTQENVA
WRKNWMVGGEGGASGRSP

FIGURE 79

CAGGAGAGAAGGCACCGCCCCCACCCCGCCTCCAAAGCTAACCCTCGGGCTTGAGGGGAAGAGGCTGACTGTACG
TTCCTTCTACTCTGGCACCACTCTCCAGGCTGCC**ATG**GGGCCCAGCACCCCTCTCCTCATCTTGTTCCTTTTGTC
ATGGTCGGGACCCCTCCAAGGACAGCAGCACCACCTTGTGGAGTACATGGAACGCCGACTAGCTGCTTTAGAGGA
ACGGCTGGCCCAGTGCCAGGACCAGAGTAGTCGGCATGCTGCTGAGCTGCGGGACTTCAAGAACAAGATGCTGCC
ACTGCTGGAGGTGGCAGAGAAGGAGCGGGAGGCACTCAGAACTGAGGCCGACACCATCTCCGGGAGAGTGGATCG
TCTGGAGCGGGAGGTAGACTATCTGGAGACCCAGAACCCAGCTCTGCCCTGTGTAGAGTTTGATGAGAAGGTGAC
TGGAGGCCCTGGGACCAAAGGCAAGGGAAGAAGGAATGAGAAGTACGATATGGTGACAGACTGTGGCTACACAAT
CTCTCAAGTGAGATCAATGAAGATTCTGAAGCGATTTGGTGGCCCAGCTGGTCTATGGACCAAGGATCCACTGGG
GCAAACAGAGAAGATCTACGTGTTAGATGGGACACAGAATGACACAGCCTTTGTCTTCCCAAGGCTGCGTGACTT
CACCCTTGCCATGGCTGCCCGGAAAGCTTCCCGAGTCCGGGTGCCCTTCCCCTGGGTAGGCACAGGGCAGCTGGT
ATATGGTGGCTTTCTTTATTTTGCTCGGAGGCCTCCTGGAAGACCTGGTGGAGGTGGTGAGATGGAGAACACTTT
GCAGCTAATCAAATTCCACCTGGCAAACCGAACAGTGGTGGACAGCTCAGTATTCCCAGCAGAGGGGCTGATCCC
CCCCTACGGCTTGACAGCAGACACCTACATCGACCTGGTAGCTGATGAGGAAGGTCTTTGGGCTGTCTATGCCAC
CCGGGAGGATGACAGGCACTTGTGTCTGGCCAAGTTAGATCCACAGACACTGGACACAGAGCAGCAGTGGGACAC
ACCATGTCCCAGAGAGAATGCTGAGGCTGCCTTTGTCATCTGTGGGACCCTCTATGTCGTCTATAACACCCGTCC
TGCCAGTCGGGCCCGCATCCAGTGCTCCTTTGATGCCAGCGGCACCCTGACCCCTGAACGGGCAGCACTCCCTTA
TTTTCCCCGCAGATATGGTGCCCATGCCAGCCTCCGCTATAACCCCCGAGAACGCCAGCTCTATGCCTGGGATGA
TGGCTACCAGATTGTCTATAAGCTGGAGATGAGGAAGAAAGAGGAGGAGGTT**TGA**GGAGCTAGCCTTGTTTTTG
CATCTTTCTCACTCCCATACATTTATATTATATCCCCACTAAATTTCTTGTTCCTCATTCTTCAAATGTGGGCCA
GTTGTGGCTCAAATCCTCTATATTTTTAGCCAATGGCAATCAAATTCTTTCAGCTCCTTTGTTTCATACGGAACT
CCAGATCCTGAGTAATCCTTTTAGAGCCCGAAGAGTCAAAACCCTCAATGTTCCCTCCTGCTCTCCTGCCCCATG
TCAACAAATTTCAGGCTAAGGATGCCCCAGACCCAGGGCTCTAACCTTGTATGCGGGCAGGCCCAGGGAGCAGGC
AGCAGTGTTCTTCCCCTCAGAGTGACTTGGGGAGGGAGAAATAGGAGGAGACGTCCAGCTCTGTCCTCTCTTCCT
CACTCCTCCCTTCAGTGTCCTGAGGAACAGGACTTTCTCCACATTGTTTTGTATTGCAACATTTTGCATTAAAAG
GAAAATCCACAAAAAAAAAAAAAAAAAAAAAAAAAAAAAAAAAAAAAAAAAAAAAAAAAAAAAAAAAAAAAAAA
AAAAAAAA

## FIGURE 80

Signal peptide: Amino acids 1-21

N-glycosylation sites: Amino acids 177-181;248-252

cAMP- and cGMP-dependent protein kinase phosphorylation site:
Amino acids 196-200

Tyrosine kinase phosphorylation site: Amino acids 89-97

N-myristoylation sites: Amino acids 115-121;152-158;
370-376

Amidation site: Amino acids 122-126

MGPSTPLLILFLLSWSGPLQGQQHHLVEYMERRLAALEERLAQCQDQSSRHAAELRDFKNKMLPLLEVAEKEREA
LRTEADTISGRVDRLEREVDYLETQNPALPCVEFDEKVTGGPGTKGKGRRNEKYDMVTDCGYTISQVRSMKILKR
FGGPAGLWTKDPLGQTEKIYVLDGTQNDTAFVFPRLRDFTLAMAARKASRVRVPFPWVGTGQLVYGGFLYFARRP
PGRPGGGGEMENTLQLIKFHLANRTVVDSSVFPAEGLIPPYGLTADTYIDLVADEEGLWAVYATREDDRHLCLAK
LDPQTLDTEQQWDTPCPRENAEAAFVICGTLYVVYNTRPASRARIQCSFDASGTLTPERAALPYFPRRYGAHASL
RYNPRERQLYAWDDGYQIVYKLEMRKKEEEV

FIGURE 81

CAAGCAGGTCATCCCCTTGGTGACCTTCAAAGAGAAGCAGAGAGGGCAGAGGTGGGGGCACAGGGAAAGGGTGA
CCTCTGAGATTCCCCTTTTCCCCCAGACTTTGGAAGTGACCCACC<u>ATG</u>GGGCTCAGCATCTTTTTGCTCCTGTGT
GTTCTTGGGCTCAGCCAGGCAGCCACACCGAAGATTTTCAATGGCACTGAGTGTGGGCGTAACTCACAGCCGTGG
CAGGTGGGGCTGTTTGAGGGCACCAGCCTGCGCTGCGGGGGTGTCCTTATTGACCACAGGTGGGTCCTCACAGCG
GCTCACTGCAGCGGCAGCAGGTACTGGGTGCGCCTGGGGGAACACAGCCTCAGCCAGCTCGACTGGACCGAGCAG
ATCCGGCACAGCGGCTTCTCTGTGACCCATCCCGGCTACCTGGGAGCCTCGACGAGCCACGAGCACGACCTCCGG
CTGCTGCGGCTGCGCCTGCCCGTCCGCGTAACCAGCAGCGTTCAACCCCTGCCCCTGCCCAATGACTGTGCAACC
GCTGGCACCGAGTGCCACGTCTCAGGCTGGGGCATCACCAACCACCCACGGAACCCATTCCCGGATCTGCTCCAG
TGCCTCAACCTCTCCATCGTCTCCCATGCCACCTGCCATGGTGTGTATCCCGGGAGAATCACGAGCAACATGGTG
TGTGCAGGCGGCGTCCCGGGGCAGGATGCCTGCCAGGGTGATTCTGGGGGCCCCCTGGTGTGTGGGGGAGTCCTT
CAAGGTCTGGTGTCCTGGGGGTCTGTGGGGCCCTGTGGACAAGATGGCATCCCTGGAGTCTACACCTATATTTGC
AAGTATGTGGACTGGATCCGGATGATCATGAGGAACAAC<u>TGA</u>CCTGTTTCCTCCACCTCCACCCCCACCCCTTAA
CTTGGGTACCCCTCTGGCCCTCAGAGCACCAATATCTCCTCCATCACTTCCCCTAGCTCCACTCTTGTTGGCCTG
GGAACTTCTTGGAACTTTAACTCCTGCCAGCCCTTCTAAGACCCACGAGCGGGGTGAGAGAAGTGTGCAATAGTC
TGGAATAAATATAAATGAAGGAGGGGCAAAAAAAAAAAAAA

# FIGURE 82

| | |
|---|---|
| Signal peptide: | Amino acids 1-17 |
| N-glycosylation sites: | Amino acids 24-28;163-167 |
| Serine proteases, trypsin family, histidine active site: | Amino acids 58-64 |
| Serine proteases, trypsin family, histidine protein Homology Blocks: | Amino acids 47-64;196-207;218-242 |
| Kringle domain proteins Homology Blocks: | Amino acids 194-207;47-65 |
| Apple domain proteins Homology Block: | Amino acids 220-248 |

MGLSIFLLLCVLGLSQAATPKIFNGTECGRNSQPWQVGLFEGTSLRCGGVLIDHRWVLTAAHCSGSRYWVRLGEH
SLSQLDWTEQIRHSGFSVTHPGYLGASTSHEHDLRLLRLRLPVRVTSSVQPLPLPNDCATAGTECHVSGWGITNH
PRNPFPDLLQCLNLSIVSHATCHGVYPGRITSNMVCAGGVPGQDACQGDSGGPLVCGGVLQGLVSWGSVGPCGQD
GIPGVYTYICKYVDWIRMIMRNN

FIGURE 83

GAGCAGTGTTCTGCTGGAGCCG**ATG**CCAAAAACCATGCATTTCTTATTCAGATTCATTGTTTTCTTTTATCTGTG
GGGCCTTTTTACTGCTCAGAGACAAAAGAAAGAGGAGAGCACCGAAGAAGTGAAAATAGAAGTTTTGCATCGTCC
AGAAAACTGCTCTAAGACAAGCAAGAAGGGAGACCTACTAAATGCCCATTATGACGGCTACCTGGCTAAAGACGG
CTCGAAATTCTACTGCAGCCGGACACAAAATGAAGGCCACCCCAAATGGTTTGTTCTTGGTGTTGGGCAAGTCAT
AAAAGGCCTAGACATTGCTATGACAGATATGTGCCCTGGAGAAAAGCGAAAAGTAGTTATACCCCCTTCATTTGC
ATACGGAAAGGAAGGCTATGCAGAAGGCAAGATTCCACCGGATGCTACATTGATTTTTGAGATTGAACTTTATGC
TGTGACCAAAGGACCACGGAGCATTGAGACATTTAAACAAATAGACATGGACAATGACAGGCAGCTCTCTAAAGC
CGAGATAAACCTCTACTTGCAAAGGGAATTTGAAAAAGATGAGAAGCCACGTGACAAGTCATATCAGGATGCAGT
TTTAGAAGATATTTTTAAGAAGAATGACCATGATGGTGATGGCTTCATTTCTCCCAAGGAATACAATGTATACCA
ACACGATGAACTA**TAG**CATATTTGTATTTCTACTTTTTTTTTTTAGCTATTTACTGTACTTTATGTATAAAACAA
AGTCACTTTTCTCCAAGTTGTATTTGCTATTTTTCCCCTATGAGAAGATATTTTGATCTCCCCAATACATTGATT
TTGGTATAATAAATGTGAGGCTGTTTTGCAAACTTAAAAAAAAAAAAAAAAAAAAAAAAAAAAAAAAAAAAAAAA
AAAAAAA

## FIGURE 84

Endoplasmic reticulum targeting sequence: Amino acids 219-224

N-glycosylation site: Amino acids 45-49

FKBP-type peptidyl-prolyl cis-trans isomerase Homology Blocks:
Amino acids 87-124;129-143

EF-hand calcium-binding domain proteins Homology Block:
Amino acids 202-215

MPKTMHFLFRFIVFFYLWGLFTAQRQKKEESTEEVKIEVLHRPENCSKTSKKGDLLNAHYDGYLAKDGSKFYCSR
TQNEGHPKWFVLGVGQVIKGLDIAMTDMCPGEKRKVVIPPSFAYGKEGYAEGKIPPDATLIFEIELYAVTKGPRS
IETFKQIDMDNDRQLSKAEINLYLQREFEKDEKPRDKSYQDAVLEDIFKKNDHDGDGFISPKEYNVYQHDEL

FIGURE 85

GAAAGA<u>**ATG**</u>TTGTGGCTGCTCTTTTTCTGGTGACTGCCATTCATGCTGAACTCTGTCAACCAGGTGCAGAAAAT
GCTTTTAAAGTGAGACTTAGTATCAGAACAGCTCTGGGAGATAAAGCATATGCCTGGGATACCAATGAAGAATAC
CTCTTCAAAGCGATGGTAGCTTTCTCCATGAGAAAAGTTCCCAACAGAGAAGCAACAGAAATTTCCCATGTCCTA
CTTTGCAATGTAACCCAGAGGGTATCATTCTGGTTTGTGGTTACAGACCCTTCAAAAAATCACACCCTTCCTGCT
GTTGAGGTGCAATCAGCCATAAGAATGAACAAGAACCGGATCAACAATGCCTTCTTTCTAAATGACCAAACTCTG
GAATTTTTAAAAATCCCTTCCACACTTGCACCACCCATGGACCCATCTGTGCCCATCTGGATTATTATATTTGGT
GTGATATTTTGCATCATCATAGTTGCAATTGCACTACTGATTTTATCAGGGATCTGGCAACGTAGAAGAAAGAAC
AAAGAACCATCTGAAGTGGATGACGCTGAAGATAAGTGTGAAAACATGATCACAATTGAAAATGGCATCCCCTCT
GATCCCCTGGACATGAAGGGGGGCATATTAATGATGCCTTCA<u>**TGA**</u>CAGAGGATGAGAGGCTCACCCCTCTCTGAA
GGGCTGTTGTTCTGCTTCCTCAAGAAATTAAACATTTGTTTCTGTGTGACTGCTGAGCATCCTGAAATACCAAGA
GCAGATCATATATTTGTTTCACCATTCTTCTTTTGTAATAAATTTTGAATGTGCTTGAAAGTGAAAAGCAATCA
ATTATACCCACCAACACCACTGAAATCATAAGCTATTCACGACTCAAAATATTCTAAAATATTTTTCTGACAGTA
TAGTGTATAAATGTGGTCATGTGGTATTTGTAGTTATTGATTTAAGCATTTTTAGAAATAAGATCAGGCATATGT
ATATATTTTCACACTTCAAAGACCTAAGGAAAAATAAATTTTCCAGTGGAGAATACATATAATATGGTGTAGAAA
TCATTGAAAATGGATCCTTTTTGACGATCACTTATATCACTCTGTATATGACTAAGTAAACAAAAGTGAGAAGTA
ATTATTGTAAATGGATGGATAAAAATGGAATTACTCATATACAGGGTGGAATTTTATCCTGTTATCACACCAACA
GTTGATTATATATTTTCTGAATATCAGCCCCTAATAGGACAATTCTATTTGTTGACCATTTCTACAATTTGTAAA
AGTCCAATCTGTGCTAACTTAATAAAGTAATAATCATCTCTTTTAAAAAAAAAAAAAAAAAAAAAAAAA

# FIGURE 86

| | |
|---|---|
| Signal peptide: | Amino acids 1-14 |
| Transmembrane domain: | Amino acids 141-160 |
| N-glycosylation sites: | Amino acids 76-80;93-97 |

MLWLLFFLVTAIHAELCQPGAENAFKVRLSIRTALGDKAYAWDTNEEYLFKAMVAFSMRKVPNREATEISHVLLC
NVTQRVSFWFVVTDPSKNHTLPAVEVQSAIRMNKNRINNAFFLNDQTLEFLKIPSTLAPPMDPSVPIWIIIFGVI
FCIIIVAIALLILSGIWQRRRKNKEPSEVDDAEDKCENMITIENGIPSDPLDMKGGILMMPS

FIGURE 87

AAGACCCTCTCTTTCGCTGTTTGAGAGTCTCTCGGCTCAAGGACCGGGAGGTAAGAGGTTTGGGACTGCCCCGGC
AACTCCAGGGTGTCTGGTCCACGACCTATCCTAGGCGCC**ATG**GGTGTGATAGGTATACAGCTGGTTGTTACCATG
GTGATGGCCAGTGTCATGCAGAAGATTATACCTCACTATTCTCTTGCTCGATGGCTACTCTGTAATGGCAGTTTG
AGGTGGTATCAACATCCTACAGAAGAAGAATTAAGAATTCTTGCAGGGAAACAACAAAAAGGGAAAACCAAAAAA
GATAGGAAATATAATGGTCACATTGAAAGTAAGCCATTAACCATTCCAAAGGATATTGACCTTCATCTAGAAACA
AAGTCAGTTACAGAAGTGGATACTTTAGCATTGCATTACTTTCCAGAATACCAGTGGCTGGTGGATTTCACAGTG
GCTGCTACAGTTGTGTATCTAGTAACTGAAGTCTACTACAATTTTATGAAGCCTACACAGGAAATGAATATCAGC
TTAGTCTGGTGCCTACTTGTTTTGTCTTTTGCAATCAAAGTTCTATTTTCATTAACTACACACTATTTTAAAGTA
GAAGATGGTGGTGAAAGATCTGTTTGTGTCACCTTTGGATTTTTTTTCTTTGTCAAAGCAATGGCAGTGTTGATT
GTAACAGAAAATTATCTGGAATTTGGACTTGAAACAGGGTTTACAAATTTTTCAGACAGTGCGATGCAGTTTCTT
GAAAAGCAAGGTTTAGAATCTCAGAGTCCTGTTTCAAAACTTACTTTCAAATTTTTCCTGGCTATTTTCTGTTCA
TTCATTGGGGCTTTTTTGACATTTCCTGGATTACGACTGGCTCAAATGCATCTGGATGCCCTGAATTTGGCAACA
GAAAAAATTACACAAACTTTACTTCATATCAACTTCTTGGCACCTTTATTTATGGTTTTGCTCTGGGTAAAACCA
ATCACCAAAGACTACATTATGAACCCACCACTGGGCAAAGAAATTTCCCCATCTGGAAGA**TGA**AGATAATAGTAT
CTAACTCACAAGGTTATCATTGGAATAAATGAAAGAACACATGTAATGCAACCAGCTGGAATTAAGTGCTTAATA
AATGTTCTTTTCACTGCTTTGCCTCATCAGAATTAAAATAGAAATACTTGACTAGT

## FIGURE 88

| | |
|---|---|
| Transmembrane domains: | Amino acids 106-121;136-152; 172-188; 230-245;272-285 |
| N-glycosylation sites: | Amino acids 34-38;135-139;203-207 |
| Tyrosine kinase phosphorylation site: | Amino acids 59-67 |
| N-myristoylation sites: | Amino acids 165-171;196-202; 240-246;247-253 |
| ATP/GTP-binding site motif A (P-loop): | Amino acids 53-61 |

MGVIGIQLVVTMVMASVMQKIIPHYSLARWLLCNGSLRWYQHPTEEELRILAGKQQKGKTKKDRKYNGHIESKPL
TIPKDIDLHLETKSVTEVDTLALHYFPEYQWLVDFTVAATVVYLVTEVYYNFMKPTQEMNISLVWCLLVLSFAIK
VLFSLTTHYFKVEDGGERSVCVTFGFFFFVKAMAVLIVTENYLEFGLETGFTNFSDSAMQFLEKQGLESQSPVSK
LTFKFFLAIFCSFIGAFLTFPGLRLAQMHLDALNLATEKITQTLLHINFLAPLFMVLLWVKPITKDYIMNPPLGK
EISPSGR

FIGURE 89

GAAGTAGAGGTGTTGTGCTGAGCGGCGCTCGGCGAACTGTGTGGACCGTCTGCTGGGACTCCGGCCCTGCGTCCG
CTCAGCCCCGTGGCCCCGCGCACCTACTGCC<u>ATG</u>GAGACGCGGCCTCGTCTCGGGGCCACCTGTTTGCTGGGCTT
CAGTTTCCTGCTCCTCGTCATCTCTTCTGATGGACATAATGGGCTTGGAAAGGGTTTTGGAGATCATATTCATTGG
AGGACACTGGAAGATGGGAAGAAAGAAGCAGCTGCCAGTGGACTGCCCCTGATGGTGATTATTCATAAATCCTGG
TGTGGAGCTTGCAAAGCTCTAAAGCCCAAATTTGCAGAATCTACGGAAATTTCAGAACTCTCCCATAATTTTGTT
ATGGTAAATCTTGAGGATGAAGAGGAACCCAAAGATGAAGATTTCAGCCCTGACGGGGGTTATATTCCACGAATC
CTTTTTCTGGATCCCAGTGGCAAGGTGCATCCTGAAATCATCAATGAGAATGGAAACCCCAGCTACAAGTATTTT
TATGTCAGTGCCGAGCAAGTTGTTCAGGGGATGAAGGAAGCTCAGGAAAGGCTGACGGGTGATGCCTTCAGAAAG
AAACATCTTGAAGATGAATTG<u>TAA</u>CATGAATGTGCCCCTTCTTTCATCAGAGTTAGTGTTCTGGAAGGAAAGCAG
CAGGGAAGGGAATATTGAGGAATCATCTAGAACAATTAAGCCGACCAGGAAACCTCATTCCTACCTACACTGGAA
GGAGCGCTCTCACTGTGGAAGAGTTCTGCTAACAGAAGCTGGTCTGCATGTTTGTGGATCCAGCGGAGAGTGGCA
GACTTTCTTCTCCTTTTCCCTCTCACCTAAATGTCAACTTGTCATTGAATGTAAAGAATGAAACCTTCTGACACAAAA

FIGURE 90

Signal peptide: Amino acids 1-23

Thioredoxin family proteins Homology Block: Amino acids 58-75

METRPRLGATCLLGFSFLLLVISSDGHNGLGKGFGDHIHWRTLEDGKKEAAASGLPLMVIIHKSWCGACKALKPK
FAESTEISELSHNFVMVNLEDEEEPKDEDFSPDGGYIPRILFLDPSGKVHPEIINENGNPSYKYFYVSAEQVVQG
MKEAQERLTGDAFRKKHLEDEL

FIGURE 91

CGGCTCGAGTGCAGCTGTGGGGAGATTTCAGTGCATTGCCTCCCCTGGGTGCTCTTCATCTTGGATTTGAAAGTT
GAGAGCAGC<u>ATG</u>TTTTGCCCACTGAAACTCATCCTGCTGCCAGTGTTACTGGATTATTCCTTGGGCCTGAATGAC
TTGAATGTTTCCCCGCCTGAGCTAACAGTCCATGTGGGTGATTCAGCTCTGATGGGATGTGTTTTCCAGAGCACA
GAAGACAAATGTATATTCAAGATAGACTGGACTCTGTCACCAGGAGAGCACGCCAAGGACGAATATGTGCTATAC
TATTACTCCAATCTCAGTGTGCCTATTGGGCGCTTCCAGAACCGCGTACACTTGATGGGGGACATCTTATGCAAT
GATGGCTCTCTCCTGCTCCAAGATGTGCAAGAGGCTGACCAGGGAACCTATATCTGTGAAATCCGCCTCAAAGGG
GAGAGCCAGGTGTTCAAGAAGGCGGTGGTACTGCATGTGCTTCCAGAGGAGCCCAAAGAGCTCATGGTCCATGTG
GGTGGATTGATTCAGATGGGATGTGTTTTCCAGAGCACAGAAGTGAAACACGTGACCAAGGTAGAATGGATATTT
TCAGGACGGCGCGCAAAGGAGGAGATTGTATTTCGTTACTACCACAAACTCAGGATGTCTGTGGAGTACTCCCAG
AGCTGGGGCCACTTCCAGAATCGTGTGAACCTGGTGGGGGACATTTTCCGCAATGACGGTTCCATCATGCTTCAA
GGAGTGAGGGAGTCAGATGGAGGAAACTACACCTGCAGTATCCACCTAGGGAACCTGGTGTTCAAGAAAACCATT
GTGCTGCATGTCAGCCCGGAAGAGCCTCGAACACTGGTGACCCCGGCAGCCCTGAGGCCTCTGGTCTTGGGTGGTAA
TCAGTTGGTGATCATTGTGGGAATTGTCTGTGCCACAATCCTGCTGCTCCCTGTTCTGATATTGATCGTGAAGAA
GACCTGTGGAAATAAGAGTTCAGTGAATTCTACAGTCTTGGTGAAGAACACGAAGAAGACTAATCCAGAGATAAA
AGAAAAACCCTGCCATTTTGAAAGATGTGAAGGGGAGAAACACATTTACTCCCCAATAATTGTACGGGAGGTGAT
CGAGGAAGAAGAACCAAGTGAAAAATCAGAGGCCACCTACATGACCATGCACCCAGTTTGGCCTTCTCTGAGGTC
AGATCGGAACAACTCACTTGAAAAAAAGTCAGGTGGGGGAATGCCAAAAACACAGCAAGCCTTT<u>TGA</u>GAAGAATG
GAGAGTCCCTTCATCTCAGCAGCGGTGGAGACTCTCTCCTGTGTGTGTCCTGGGCCACTCTACCAGTGATTTCAG
ACTCCCGCTCTCCCAGCTGTCCTCCTGTCTCATTGTTTGGTCAATACACTGAAGATGGAGAATTTGGAGCCTGGC
AGAGAGACTGGACAGCTCTGGAGGAACAGGCCTGCTGAGGGGAGGGGAGCATGGACTTGGCCTCTGGAGTGGGAC
ACTGGCCCTGGGAACCAGGCTGAGCTGAGTGGCCTCAAACCCCCCGTTGGATCAGACCCTCCTGTGGCAGGGTT
CTTAGTGGATGAGTTACTGGGAAGAATCAGAGATAAAAACCAACCCAAATCAA

## FIGURE 92

| | |
|---|---|
| Signal peptide: | Amino acids 1-19 |
| Transmembrane domain: | Amino acids 275-296 |
| N-glycosylation sites: | Amino acids 76-80;231-235;302-306;<br>307-311;376-380 |
| Myelin P0 protein Homology Blocks: | Amino acids 210-240;92-122 |

MFCPLKLILLPVLLDYSLGLNDLNVSPPELTVHVGDSALMGCVFQSTEDKCIFKIDWTLSPGEHAKDEYVLYYYS
NLSVPIGRFQNRVHLMGDILCNDGSLLLQDVQEADQGTYICEIRLKGESQVFKKAVVLHVLPEEPKELMVHVGGL
IQMGCVFQSTEVKHVTKVEWIFSGRRAKEEIVFRYYHKLRMSVEYSQSWGHFQNRVNLVGDIFRNDGSIMLQGVR
ESDGGNYTCSIHLGNLVFKKTIVLHVSPEEPRTLVTPAALRPLVLGGNQLVIIVGIVCATILLLPVLILIVKKTC
GNKSSVNSTVLVKNTKKTNPEIKEKPCHFERCEGEKHIYSPIIVREVIEEEEPSEKSEATYMTMHPVWPSLRSDR
NNSLEKKSGGGMPKTQQAF

FIGURE 93

TCTGCCTCCACTGCTCTGTGCTGGGATCATGGAACTTGCACTGCTGTGTGGCTGGTGGTGATGGCTGGTGTGAT
TCCAATCCAGGGCGGGATCCTGAACCTGAACAAGATGGTCAAGCAAGTGACTGGGAAAATGCCCATCCTCTCCTA
CTGGCCCTACGGCTGTCACTGCGGACTAGGTGGCAGAGGCCAACCCAAAGATGCCACGGACTGGTGCTGCCAGAC
CCATGACTGCTGCTATGACCACCTGAAGACCCAGGGGTGCGGCATCTACAAGGACAACAACAAAAGCAGCATACA
TTGTATGGATTTATCTCAACGCTATTGTTTAATGGCTGTGTTTAATGTGATCTATCTGGAAAATGAGGACTCCGA
ATAAAAAGCTATTACTAWTTNAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAA

## FIGURE 94

| | |
|---|---|
| Signal sequence: | Amino acids 1-17 |
| N-glycosylation site: | Amino acids 86-90 |
| N-myristoylation sites: | Amino acids 20-26;45-51 |
| Phospholipase A2 histidine active site: | Amino acids 63-71 |

MELALLCGLVVMAGVIPIQGGILNLNKMVKQVTGKMPILSYWPYGCHCGLGGRGQPKDATDWCCQTHDCCYDHLK
TQGCGIYKDNNKSSIHCMDLSQRYCLMAVFNVIYLENEDSE

FIGURE 95

CACAAGCATCTTAATTTGAATCCACAAAGTTTCATGTAATGAAAAGAAATACATAATTTTAATTCAACCCGAGTG
TTTTCCAAGAAGATTGTATTTGCTTAAATTGCTACAGTAATTCAAGAGACAGCCCTGTCTGGACACAGAGTTACT
GTGGATTTTTAAGAGACTCAGTTAAAGAATTTAGGAATTTCTGATTCATTTAAAGGATTTACAAATTCATCAACC
CCTGAAAACTAAAGCAAATTGAACAGGAAAAAAAAAAAAGAAG<u>ATG</u>GGTTTTTTAAGTCCAATATATGTTATTTTC
TTCTTTTTTGGAGTCAAAGTACATTGCCAATATGAAACTTATCAGTGGGATGAAGACTATGACCAAGAGCCAGAT
GATGATTACCAAACAGGATTCCCATTTCGTCAAAATGTAGACTACGGAGTTCCTTTTCATCAGTATACTTTAGGC
TGTGTCAGTGAATGCTTCTGTCCAACTAACTTTCCATCATCAATGTACTGTGATAATCGCAAACTCAAGACTATC
CCAAATATTCCGATGCACATTCAGCAACTCTACCTTCAGTTCAATGAAATTGAGGCTGTGACTGCAAATTCATTC
ATCAATGCAACTCATCTTAAAGAAATTAACCTCAGCCACAACAAAATTAAATCTCAAAAGATTGATTATGGTGTG
TTTGCTAAGCTTCCAAATCTACTACAACTTCATCTAGAGCATAATAATTTAGAAGAATTTCCATTTCCTCTTCCT
AAATCTCTGGAAAGACTCCTTCTTGGTTACAATGAAATCTCCAAACTGCAGACAAATGCTATGGATGGGCTAGTA
AACTTGACCATGCTTGATCTCTGTTATAATTATCTTCATGATTCTCTGCTAAAAGACAAAATCTTTGCCAAAATG
GAAAAACTAATGCAGCTCAACCTCTGCAGTAACAGATTAGAATCAATGCCTCCTGGTTTGCCTTCTTCACTTATG
TATCTGTCTTTAGAAAATAATTCAATTTCTTCTATACCCGAAAAATACTTCGACAAACTTCCAAAACTTCATACT
CTAAGAATGTCACACAACAAACTACAAGACATCCCATATAATATTTTTAATCTTCCCAACATTGTAGAACTCAGT
GTTGGACACAACAAATTGAAGCAAGCATTCTATATTCCAAGAAATTTGGAACACCTATACCTACAAAATAATGAA
ATAGAAAAGATGAATCTTACAGTGATGTGTCCTTCTATTGACCCACTACATTACCACCATTTAACATACATTCGT
GTGGACCAAAATAAACTAAAAGAACCAATAAGCTCATACATCTTCTTCTGCTTCCCTCATATACACACTATTTAT
TATGGTGAACAACGAAGCACTAATGGTCAAACAATACAACTAAAGACACAAGTTTTCAGGAGATTTCCAGATGAT
GATGATGAAAGTGAAGATCACGATGATCCTGACAATGCTCATGAGAGCCCAGAACAAGAAGGAGCAGAAGGGCAC
TTTGACCTTCATTATTATGAAAATCAAGAA<u>TAG</u>CAAGAAACTATATAGGTATACACTTACGACTTCACAAAACCTA
TACTTAATATAGTAAATCTAAGTAAACATGTATTACTCAAAGTAATATATTTAGAATTATGTATTAGTATAAGAT
CAGAATTGAATTTAAGTTGTTGGTGACATCTGCATCATTTCATAGGATTAGAACTTACTCAAAATAATGTAAATC
TTTAAAAATATAAATTAGAATGACAAGTGGGAATCATAAATTAAACGTTAATGGTTTCTTATGCTCTTTTTAAAT
ATAGAAATATCATGTTAAAGAAAAAAAAAAAAAA

## FIGURE 96

| | |
|---|---|
| N-glycosylation sites: | Amino acids 113-117;121-125; 187-191;242-246;316-320 |
| Tyrosine kinase phosphorylation sites: | Amino acids 268-275;300-307 |
| N-myristoylation site: | Amino acids 230-236 |
| Leucine zipper patterns: | Amino acids 146-168;217-239 |

MGFLSPIYVIFFFFGVKVHCQYETYQWDEDYDQEPDDDYQTGFPFRQNVDYGVPFHQYTLGCVSECFCPTNFPSS
MYCDNRKLKTIPNIPMHIQQLYLQFNEIEAVTANSFINATHLKEINLSHNKIKSQKIDYGVFAKLPNLLQLHLEH
NNLEEFPFPLPKSLERLLLGYNEISKLQTNAMDGLVNLTMLDLCYNYLHDSLLKDKIFAKMEKLMQLNLCSNRLE
SMPPGLPSSLMYLSLENNSISSIPEKYFDKLPKLHTLRMSHNKLQDIPYNIFNLPNIVELSVGHNKLKQAFYIPR
NLEHLYLQNNEIEKMNLTVMCPSIDPLHYHHLTYIRVDQNKLKEPISSYIFFCFPHIHTIYYGEQRSTNGQTIQL
KTQVFRRFPDDDDESEDHDDPDNAHESPEQEGAEGHFDLHYYENQE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5573762 A
- US 5935924 A
- US 5624806 A
- US 5661122 A
- US 5610134 A
- WO 9528173 A
- US 5773414 A
- JP 31302991991 B
- EP 457195 A
- EP 460679 A
- EP 552489 A
- US 5773223 A
- EP 471754 B
- EP 370989 A
- EP 817648 A
- US 9806724 W
- WO 9617931 A
- US 5650282 A
- US 4675187 A
- WO 9101753 A
- WO 8906692 A
- WO 9222653 A
- US 4816567 A
- EP 404097 A
- WO 9311161 A
- US 4275149 A
- US 5364934 A
- WO 8705330 A
- US 4640835 A
- US 4496689 A
- US 4301144 A
- US 4670417 A
- US 4791192 A
- US 4179337 A
- US 5428130 A
- WO 8905859 A
- US 4399216 A
- DD 266710
- US 4946783 A
- EP 139383 A
- US 4943529 A
- EP 402226 A
- EP 183070 A
- EP 244234 A
- EP 394538 A
- WO 9100357 A
- US 5010182 A
- EP 362179 A
- WO 9013646 A
- EP 36776 A
- EP 73657 A
- GB 2211504 A
- EP 117060 A
- EP 117058 A
- WO 9516035 A
- WO 9505846 A
- WO 9107491 A
- US 5773415 A
- WO 9733551 A
- US 4873191 A
- US 4736866 A
- US 4376110 A
- US 4892538 A
- US 5283187 A
- WO 9325673 A
- US 5681746 A
- EP 257956 A
- US 3773919 A
- EP 58481 A
- EP 133988 A
- DE 3218121
- EP 52322 A
- EP 36676 A
- EP 88046 A
- EP 143949 A
- EP 142641 A
- JP 58118008 A
- US 4485045 A
- US 4544545 A
- EP 102324 A
- US 5679545 A
- US 5545807 A
- US 5545806 A
- US 5569825 A
- US 5625126 A
- US 5633425 A
- US 5661016 A
- WO 9308829 A
- US 4676980 A
- WO 9100360 A
- WO 92200373 A
- EP 03089 A
- WO 9411026 A
- US 5013556 A
- EP 616812 A
- EP 307247 A

**Non-patent literature cited in the description**


- **EICHHORN.** *American Journal of Medicine,* 1998, vol. 104, 163-169
- **BROWN ; VAUGHAN.** *Circulation,* 1998, vol. 97, 1411-1420
- **WEBER.** *Circulation,* 1998, vol. 96, 4065-4082
- **MORGAN ; BAKER.** *Circulation,* 1991, vol. 83, 13-25
- Heart Failure. **KATZ.** Physiology of the Heart. Raven Press, 1992, 638-668
- **KATZ.** *Trends Cardiovasc. Med.,* 1995, vol. 5, 37-44
- Pathophysiology of Heart Failure. Heart Disease. A Textbook of Cardiovascular Medicine. W.B. Saunders Co, 1988
- **CHIEN.** *FASEB J.,* 1991, vol. 5, 3037-3046
- **CHIEN et al.** *Annu. Rev. Physiol.,* 1993, vol. 55, 77-95
- **CASPARI et al.** *Cardiovase. Res.,* 1977, vol. II, 554-558
- **SCHWARZ et al.** *Am. J. Cardiol.,* 1978, vol. 42, 895-903
- **HESS et al.** *Circulation,* 1981, vol. 63, 360-371
- **PEARLMAN et al.** *Lab. Invest.,* 1982, vol. 46, 158-164
- **METCALF.** *Growth Factors,* 1992, vol. 7, 169-173
- **KURZROCK et al.** *Endocrine Reviews,* 1991, vol. 12, 208-217
- **INOUE et al.** *Proc. Natl. Acad. Sci. USA,* 1989, vol. 86, 2863-2867
- **YANAGISAWA ; MASAKI.** *Trends Pharm. Sci.,* 1989, vol. 10, 374-378
- **PENNICA et al.** *Proc. Nat. Acad. Sci. USA,* 1995, vol. 92, 1142-1146
- **THOMPSON et al.** *Br. Heart J.,* 1980, vol. 44, 488-98
- **HARRISON et al.** *Circulation,* 1964, vol. 29, 84-98
- **BONOW et al.** *Circulation,* 1985, vol. 72, 853-64
- **LORELL et al.** *Circulation,* 1982, vol. 65, 499-507
- **BETOCCHI et al.** *Am. J. Cardiol.,* 1996, vol. 78, 451-457
- **POLLICK.** *N. Engl. J. Med.,* 1982, vol. 307, 997-999
- **WIGLE et al.** *Circulation,* 1995, vol. 92, 1680-1692
- **SZLACHCIC et al.** *Am. J. Cardiol.,* 1989, vol. 63, 198-201
- **SHAHI et al.** *Lancet,* 1990, vol. 336, 458-461
- **ROSSI et al.** *Am. Heart J.,* 1992, vol. 124, 700-709
- **MASSIE.** *Curr. Op. in Cardiology,* 1997, vol. 12, 209-217
- **REDDY et al.** *Curr. Opin. Cardiol.,* 1997, vol. 12, 233-241
- **YANAGISAWA et al.** *Nature,* 1988, vol. 332, 411-415
- **KRAMER et al.** *Circulation,* 1983, vol. 67 (4), 807-816
- *J.A.C.C.,* 1983, vol. 2 (4), 755-763
- *N. Engl. J. Med.,* 1987, vol. 316 (23), 1429-1435
- *N. Engl. J. Med.,* 1991, vol. 325 (5), 293-302
- *N. Engl. J. Med.,* 1987, vol. 316 (23), 1429-1453
- **COHN et al.** *N. Engl. J. Med.,* 1991, vol. 325 (5), 303-310
- *JAMA,* 1988, vol. 259 (4), 539-544
- **HARADA et al.** *Circulation,* 1998, vol. 97, 1952-1959
- **HOMCY.** *Circulation,* 1998, vol. 97, 1890-1892
- **TOPOL.** *Am. J. Cardiol.,* 1988, vol. 61, 723-728
- **NEUHAUS et al.** *J. Am. Coll. Cardiol.,* 1988, vol. 12, 581-587
- **NEUHAUS et al.** *J. Am. Coll. Cardiol.,* 1989, vol. 14, 1566-1569
- **TOPOL.** *J. Am. Coll. Cardiol.,* 1990, vol. 15, 922-924
- **TEBBE et al.** *Am. J. Cardiol.,* 1989, vol. 64, 448-453
- **KEYT et al.** *Proc. Natl. Acad. Sci. USA,* 1994, vol. 91, 3670-3674
- **BURGESS ; MACIAG.** *Annual Rev. Biochem.,* 1989, vol. 58, 575
- **ISHIKAWA et al.** *Nature,* 1989, vol. 338, 557
- **LEUNG et al.** *Science,* 1989, vol. 246, 1306
- **FERRARA ; HENZEL.** *Biochem. Biophys. Res. Commun.,* 1989, vol. 161, 851
- **TISCHER et al.** *Biochem. Biophys. Res. Commun.,* 1989, vol. 165, 1198
- **HOUCK et al.** *Mol. Endocrin.,* 1991, vol. 5, 1806
- **FERRARA et al.** *Cell. Biochem.,* 1991, vol. 47, 211
- **FERRARA et al.** *Endocrine Reviews,* 1992, vol. 13, 18
- **KECK et al.** *Science,* 1989, vol. 246, 1309
- **CONNOLLY et al.** *J. Biol. Chem.,* 1989, vol. 264, 20017
- **FOLKMAN et al.** *J. Biol. Chem.,* 1992, vol. 267, 10931-10934
- **KLAGSBRUN et al.** *Annu. Rev. Physiol.,* 1991, vol. 53, 217-239

- Vascular diseases. **GARNER A.** Pathobiology of Ocular Disease. A Dynamic Approach. Marcel Dekker, 1994, 1625-1710
- **FOLKMAN.** *Nature,* 1989, vol. 339, 58
- **WEIDNER et al.** *N. Engl. J. Med,* 1991, vol. 324, 1-6
- **HORAK.** *Lancet,* 1992, vol. 340, 1120-1124
- **MACCHIARINI et al.** *Lancet,* 1992, vol. 340, 145-146
- **GOOD et al.** *Proc. Natl. Acad. Sci. USA.,* 1990, vol. 87, 6624-6628
- **CLAPP et al.** *Endocrinology,* 1993, vol. 133, 1292-1299
- **O'REILLY et al.** *Cell,* 1994, vol. 79, 315-328
- **O'REILLY et al.** *Cell,* 1996, vol. 88, 277-285
- **FERRARA et al.** *Endocr. Rev.,* 1997, vol. 18, 4-25
- **BERKMAN et al.** *J. Clin. Invest.,* 1993, vol. 91, 153-159
- **BROWN et al.** *Human Pathol.,* 1995, vol. 26, 86-91
- **BROWN et al.** *Cancer Res.,* 1993, vol. 53, 4727-4735
- **MATTERN et al.** *Brit. J. Cancer.,* 1996, vol. 73, 931-934
- **DVORAK et al.** *Am. J. Pathol.,* 1995, vol. 146, 1029-1039
- **AIELLO et al.** *N. Engl. J. Med.,* 1994, vol. 331, 1480-1487
- **LOPEZ et al.** *Invest. Ophthalmol. Vis. Sci.,* 1996, vol. 37, 855-868
- **KIM et al.** *Nature,* 1993, vol. 362, 841-844
- **WARREN et al.** *J. Clin. Invest.,* 1995, vol. 95, 1789-1797
- **BORGSTRÖM et al.** *Cancer Res.,* 1996, vol. 56, 4032-4039
- **MELNYK et al.** *Cancer Res.,* 1996, vol. 56, 921-924
- **ADAMIS et al.** *Arch. Ophthalmol.,* 1996, vol. 114, 66-71
- **LAU ; NATHANS.** *Molecular Aspects of Cellular Regulation,* 1991, vol. 6, 165-202
- **RYSECK et al.** *Cell Growth Differ.,* 1991, vol. 2, 235-233
- **SIMMONS et al.** *Proc. Natl. Acad. Sci. USA,* 1989, vol. 86, 1178-1182
- **O'BRIEN et al.** *Mol. Cell Biol.,* 1990, vol. 10, 3569-3577
- **BRADHAM et al.** *J. Cell. Biol.,* 1991, vol. 114, 1285-1294
- **JOLOIT et al.** *Mol. Cell. Biol.,* 1992, vol. 12, 10-21
- **CLEMMONS et al.** *J. Clin. Invest.,* 1986, vol. 77, 1548
- **ZAPF et al.** *J. Clin. Invest.,* 1979, vol. 63, 1077
- **FOUAD et al.** *J. Am. Coll. Cardiol.,* 1984, vol. 4, 1500-1506
- **SMITH et al.** *J. Am. Coll. Cardiol.,* 1985, vol. 5, 869-874
- **HARTFORD et al.** *Hypertension,* 1984, vol. 6, 329-338
- **INOUYE et al.** *Am. J. Cardiol.,* 1984, vol. 53, 1583-7
- **MARON et al.** *N. Engl. J. Med.,* 1987, vol. 316, 780-789
- **SPIRITO et al.** *N. Engl. J. Med.,* 1989, vol. 320, 749-755
- **LOUIE ; EDWARDS.** *Prog. Cardiovasc. Dis.,* 1994, vol. 36, 275-308
- **SPIRITO et al.** *N. Engl. J. Med.,* 1997, vol. 336, 775-785
- **WATKINS et al.** *N. Engl.J. Med.,* 1992, vol. 326, 1108-1114
- **SCHWARTZ et al.** *Circulation,* 1995, vol. 91, 532-540
- **MARIAN ; ROBERTS.** *Circulation,* 1995, vol. 92, 1336-1347
- **THIERFCLDER et al.** *Cell,* 1994, vol. 77, 701-712
- **WATKINS et al.** *Nat. Gen.,* 1995, vol. 11, 434-437
- **MALIK ; WATKINS.** *Curr. Opin. Cardiol.,* 1997, vol. 12, 295-302
- Cell cycle regulation, oncogenes, and antineoplasticdrugs. **MURAKAMI et al.** The Molecular Basis of Cancer. WB Saunders, 1995, 13
- **NIELSEN et al.** *Prot. Eng.,* 1997, vol. 10, 1-6
- **HEINJE et al.** *Nucl. Acids Res.,* 1986, vol. 14, 4683-4690
- **ALTSCHUL et al.** *Nucleic Acids Res.,* 1997, vol. 25, 3389-3402
- **ALTSCHUL et al.** *Methods in Enzymology,* 1996, vol. 266, 460-480
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **KABAT et al.** *NIH Publ. No.91-3242.,* 1991, vol. I, 647-669
- **ZAPATA et al.** *Protein Eng.,* 1995, vol. 8 (10), 1057-1062
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **MORRISON et al.** *Proc. Natl. Acad. Sci. USA,* 1984, vol. 81, 6851-6855
- **JONES.** *Nature,* 1986, vol. 321, 522-525
- **REICHMANN.** *Nature,* 1988, vol. 332, 323-329
- **PRESTA.** *Curr. Op. Struct. Biol.,* 1992, vol. 2, 593-596

- **PLUCKTHUN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **CARTER et al.** *Nucl. Acids Res.,* 1986, vol. 13, 4331
- **ZOLLER et al.** *Nucl. Acids Res.,* 1987, vol. 10, 6487
- **WELLS et al.** *Gene,* 1985, vol. 34, 315
- **WELLS.** *Philos. Trans. R. Soc. London SerA.,* 1986, vol. 317, 415
- **CUNNINGHAM ; WELLS.** *Science,* 1989, vol. 244, 1081-1085
- **CREIGHTON.** The Proteins. W.H. Freeman & Co,
- **CHOTHIA.** *J. Mol. Biol.,* 1976, vol. 150, 1
- **T.E. CREIGHTON.** Proteins: Structure and Molecular Properties. W.H. Freeman & Co, 1983, 79-86
- **APLIN ; WRISTON.** *CRC Crit. Rev. Biochem.,* 1981, 259-306
- **HAKIMUDDIN et al.** *Arch. Biochem. Biophys.,* 1987, vol. 259, 52
- **EDGE et al.** *Anal. Biochem.,* 1981, vol. 118, 131
- **THOTAKURA et al.** *Meth. Enzymol.,* 1987, vol. 138, 350
- **FIELD.** *Mol. Cell. Biol.,* 1988, vol. 8, 2159-2165
- **EVAN.** *Molecular and Cellular Biology,* 1985, vol. 5, 3610-3616
- **PABORSKY et al.** *Protein Engineering,* 1990, vol. 3 (6), 547-553
- **HOPP et al.** *BioTechnology,* 1988, vol. 6, 1204-1210
- **MARTIN et al.** *Science,* 1992, vol. 255, 192-194
- **SKINNER et al.** *J. Biol. Chem.,* 1991, vol. 266, 15163-15166
- **LUTZ-FREYETMUTH et al.** *Proc. Natl. Acad. Sci. USA,* 1990, vol. 87, 6393-6397
- **STEWART et al.** Solid-Phase Peptide Synthesis. W.H. Freeman Co, 1969
- **MERRIFIELD.** *J. Am. Chem. Soc.,* 1963, vol. 85, 2149-2154
- **DIEFFENBACH et al.** PCR Primer: A Laboratory Manual. Cold Spring Harbor Laboratory Press, 1995
- Mammalian Cell Biotechnology: A Practical Approach. IRL Press, 1991
- **SHAW et al.** *Gene,* 1983, vol. 23, 315
- **GRAHAM ; VAN DER EB.** *Virology,* 1978, vol. 52, 456-457
- **VAN SOLINGEN et al.** *J. Bact.,* 1977, vol. 130, 946
- **HSIAO et al.** *Proc. Natl. Acad. Sci.,* 1979, vol. 76, 3829
- **KEOWN et al.** *Methods in Enzymology,* 1990, vol. 185, 527-537
- **MANSOUR et al.** *Nature,* 1988, vol. 336, 348-352
- **BEACH ; NURSE.** *Nature,* 1981, vol. 290, 140
- **FLEER et al.** *Bio/Technology,* 1991, vol. 9, 968-975
- **LOUVENCOURT et al.** *J. Bacteriol.,* 1983, vol. 737
- **VAN DEN BERG et al.** *Bio/Technology,* 1990, vol. 8, 135
- **SREEKRISHNA et al.** *J. Basic Microbiol,* 1988, vol. 28, 265-278
- **CASE et al.** *Proc. Natl. Acad. Sci. USA,* 1979, vol. 76, 5259-5263
- **BALLANCE et al.** *Biochem. Biophys. Res. Commun.,* 1983, vol. 112, 284-289
- **TILBUM et al.** *Gene,* 1983, vol. 26, 205-221
- **YELTON et al.** *Proc. Natl. Acad. Sci. USA,* 1984, vol. 81, 1470-1474
- **KELLY ; HYNES.** *EMBO J.,* 1985, vol. 4, 475-479
- **C. ANTHONY.** *The Biochemistry of Methylotrophs,* 1982, vol. 269
- **GRAHAM et al.** *J. Gen Virol.,* 1977, vol. 36, 59
- **URLAUB ; CHASIN.** *Proc. Natl. Acad. Sci. USA.,* 1980, vol. 77, 4216
- **MATHER.** *Biol, Reprod.,* 1980, vol. 23, 243-251
- **URLAUB et al.** *Proc. Natl. Acad. Sci. USA,* 1980, vol. 77, 4216
- **STINCHCOMB et al.** *Nature,* 1979, vol. 282, 39
- **KINGSMAN et al.** *Gene,* 1979, vol. 7, 141
- **TSCHEMPER et al.** *Gene,* 1980, vol. 10, 157
- **JONES.** *Genetics,* 1977, vol. 85, 12
- **CHANG et al.** *Nature,* 1978, vol. 275, 615
- **GOEDDEL et al.** *Nature,* 1979, vol. 281, 544
- **GOEDDEL.** *Nucleic Acids Res.,* 1980, vol. 8, 4057
- **DEBOER et al.** *Proc. Natl. Acad. Sci. USA,* 1983, vol. 80, 21-25
- **HITZEMAN et al.** *J. Biol. Chem.,* 1980, vol. 255, 2073
- **HESS et al.** *J. Adv. Enzyme Reg.,* 1968, vol. 7, 149
- **HOLLAND.** *Biochemistry,* 1978, vol. 17, 4900
- **GETHING et al.** *Nature,* 1981, vol. 293, 620-625
- **MANTEI et al.** *Nature,* 1979, vol. 281, 40-46
- **THOMAS.** *Proc. Natl. Acad. Sci. USA.,* 1980, vol. 77, 5201-5205
- **DEUTSCHER.** *Methods in Enzymology,* 1990, vol. 182
- **SCOPES.** Protein Purification: Principles and Practice. Springer-Verlag, 1982
- **WINTER.** Epidermal Wound Healing. Year Book Medical Publishers, Inc, 71-112

- **EAGLSTEIN ; MERTZ.** *J. Invest. Dermatol.,* 1978, vol. 71, 382-384
- Adult ventricular rat heart muscle cells. **PIPER et al.** Cell Culture Techniques in Heart and Vessel Research. Springer-Verlag, 1990, 36-60
- **LAI et al.** *Am. J. Physiol. (Heart Circ. Physiol.),* 1996, vol. 271, H2197-H2208
- **BEZNAK M.** *Can. J. Biochem. Physiol.,* 1955, vol. 33, 985-94
- **O'ROURKE ; REIBEL.** *P.S.E.M.B.,* 1992, vol. 200, 95-100
- The Nude Mouse in Oncology Research. CRC Press, Inc, 1991
- **KARMALI et al.** *Br. J. Cancer.,* 1983, vol. 48, 689-696
- **DREBIN et al.** *Proc. Nat. Acad. Sci. USA,* 1986, vol. 83, 9129-9133
- **WANG et al.** *Cancer Research,* 1994, vol. 54, 4726-4728
- **TOO et al.** *Cancer Research,* 1995, vol. 55, 681-684
- **DELEO et al.** *J. Exp. Med.,* 1977, vol. 146, 720
- **PALLADINO et al.** *J. Immunol.,* 1987, vol. 138, 4023-4032
- **ZUPI et al.** *Br. J. Cancer.,* 1980, vol. 41, 30
- **ZACHARSKI.** *Haemostasis,* 1986, vol. 16, 300-320
- **RYGAARD ; SPANG-THOMSEN.** Proc. 6th Int. Workshop on Immune-Deficient Animals. Basel, 1989, 301
- **VAN DER PUTTEN et al.** *Proc. Natl. Acad. Sci. USA,* 1985, vol. 82, 6148-615
- **THOMPSON et al.** *Cell,* 1989, vol. 56, 313-321
- **LO.** *Mol. Cell. Biol.,* 1983, vol. 3, 1803-1814
- **LAVITRANO et al.** *Cell,* 1989, vol. 57, 717-73
- **LASKO et al.** *Proc. Natl. Acad. Sci. USA,* 1992, vol. 89, 6232-636
- **THOMAS ; CAPECCHI.** *Cell,* 1987, vol. 51, 503
- **LI et al.** *Cell,* 1992, vol. 69, 915
- **BRADLEY.** Teratocarcinomas and Embryonic Stem Cells: A Practical Approach. IRL, 1987, 113-152
- **THOMAS.** *Proc. Natl. Acad. Sci. USA,* 1980, vol. 77, 5201-5205
- **ZOLA.** Monoclonal Antibodies: A Manual of Techniques. CRC Press, Inc, 1987, 147-158
- **SMALL et al.** *Mol. Cell. Biol.,* 1985, vol. 5, 642-648
- **TONKINSON et al.** *Cancer Investigation,* 1996, vol. 14 (1), 54-65
- **WU et al.** *J. Biol. Chem.,* 1987, vol. 262, 4429-4432
- **WAGNER et al.** *Proc. Natl. Acad. Sci. USA,* 1990, vol. 87, 3410-3414
- **ANDERSON et al.** *Science,* 1992, vol. 256, 808-813
- **SAIKI et al.** *Nature,* 1986, vol. 324, 163-166
- **MYERS et al.** *Science,* 1985, vol. 230, 1242
- **COTTON et al.** *Proc. Natl. Acad. Sci. USA,* 1985, vol. 85, 4397-4401
- **VERMA et al.** Human Chromosomes: a Manual of Basic Techniques. Pergamon Press, 1988
- **FIELDS ; SONG.** *Nature (London),* 1989, vol. 340, 245-246
- **CHIEN et al.** *Proc. Natl. Acad. Sci. USA,* 1991, vol. 88, 9578-9582
- **CHEVRAY ; NATHANS.** *Proc. Natl. Acad. Sci. USA.,* 1991, vol. 89, 5789-5793
- **COLIGAN et al.** *Current Protocols in Immun.,* 1991, vol. 1 (2
- **LEE et al.** *Nucl. Acids Res.,* 1979, vol. 6, 3073
- **COONEY et al.** *Science,* 1988, vol. 241, 456
- **DERVAN et al.** *Science,* 1991, vol. 251, 1360
- **OKANO.** *Neurochem.,* 1991, vol. 56, 560
- Oligodeoxynucleotides as Antisense Inhibitors of Gene Expression. CRC Press, 1988
- **ROSSI.** *Current Biology,* 1994, vol. 4, 469-471
- **GRIENER et al.** *Lymphology,* 1971, vol. 4, 140-144
- Remington's Pharmaceutical Sciences. 1980
- **LANGER et al.** *J. Biomed. Mater. Res.,* 1981, vol. 15, 167-277
- **LANGER.** *Chem. Tech.,* 1982, vol. 12, 98-105
- **SIDMAN.** *Biopolymers,* 1983, vol. 22, 547-556
- **EPSTEIN et al.** *Proc. Natl. Acad. Sci. USA.,* 1985, vol. 82, 3688-3692
- **HWANG et al.** *Proc. Natl. Acad. Sci. USA,* 1980, vol. 77, 4030-4034
- Physicians' Desk Reference. Medical Economics Data Production Co, 1997
- **KOHLER ; MILSTEIN.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON ; POLLARD.** *Anal. Biochem.,* 1980, vol. 107, 220
- **JONES et al.** *Nature,* 1986, vol. 321, 522-525
- **RIECHMANN et al.** *Nature,* 1988, vol. 332, 323-329
- **PRESTA.** *Curr. Op Struct. Biol.,* 1992, vol. 2, 593-596
- **RIECHMANN et al.** *Nature,* 1988, vol. 332, 323-327
- **VERHOEYEN et al.** *Science,* 1988, vol. 239, 1534-1536
- **WINTER.** *J. Mol. Biol.,* 1991, vol. 227, 381
- **MARKS et al.** *J. Mol. Biol.,* 1991, vol. 222, 581

- **COLE.** Monoclonal Antibodies and Cancer Therapy. Alan R. Liss, 1985, 77
- **BOEMER et al.** *J. Immunol.,* 1991, vol. 147 ((1)), 86-95
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-813
- **FISHWILD et al.** *Nature Biotechnology,* 1996, vol. 14, 845-851
- **NEUBERGER.** *Nature Biotechnology,* 1996, vol. 14, 826
- **LONBERG ; HUSZAR.** *Intern. Rev. Immunol.,* 1995, vol. 13, 65-93
- **MILSTEIN ; CUELLO.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **CARON et al.** *J. Exp. Med,* 1992, vol. 176, 1191-1195
- **SHOPES.** *J. Immunol,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 219-230
- **VITETTA.** *Science,* 1987, vol. 238, 1098
- **EPSTEIN et al.** *Proc. Natl. Acad. Sci. USA,* 1985, vol. 82, 3688
- **HWANG et al.** *Proc. Natl. Acad. Sci. USA,* 1980, vol. 77, 4030
- **MARTIN.** *J. Biol. Chem.,* 1982, vol. 257, 286-288
- **GABIZON et al.** *J. National Cancer Inst.,* 1989, vol. 81 (19), 1484
- **MARASCO et al.** *Proc. Nail. Acad. Sci. USA.,* 1993, vol. 90, 7889-7893
- Chemotherapy Service. Willianns & Wilkins, 1992
- **AUSUBEL et al.** Current Protocols in Molecular Biology. Green Publishing Associates and Wiley Interscience, 1989
- **INNIS et al.** PCR Protocols: A Guide to Methods and Applications. Academic Press, 1990
- **HARLOW et al.** Antibodies: A Laboratory Manual. Cold Spring Harbor Press, 1988
- **GAIT.** Oligonucleotide Synthesis. IRL Press, 1984
- **FRESHNEY.** *Animal Cell Culture,* 1987
- **COLIGAN et al.** *Current Protocols in Immunology,* 1991
- **HOLMES et al.** *Science,* 1991, vol. 253, 1278-1280
- **GIETZ et al.** *Nucl. Acid. Res.,* 1992, vol. 20, 1425
- **KAISER.** Methods in Yeast Genetics. Cold Spring Harbor Press, 1994, 207
- **KAISER et al.** Methods in Yeast Genetics. Cold Spring Harbor Press, 1994, 208-210
- **BIELY et al.** *Anal. Biochem.,* 1988, vol. 172, 176-179
- **ALTSHUL et al.** *Methods in Enzymology,* 1996, vol. 266, 460-480
- **NOCENTI et al.** *Proc. Natl. Acad. Sci. USA,* 1997, vol. 94, 6216-6221
- **AUSUBEL et al.** *Current Protocols in Molecular Biology,* 1995
- **ALTSHUL et al.** *Methods in Enzymology,* 1996, vol. 266, 46-480
- **ALTSHUL.** *Methods in Enzymology,* 1996, vol. 266, 460-480
- **FUNDERBURGH.** *J. Biol. Chem.,* 1996, vol. 271, 31431-31436
- **BOEHRINGER.** Cell Death Detection ELISA plus
- **LU ; GILLETT.** *Cell Vision,* 1994, vol. 1, 169-176
- **BOLIVAR.** *Gene,* 1977, vol. 2, 95
- **THIMMAPPAYA et al.** *Cell,* 1982, vol. 31, 543
- **SOMPARYRAC et al.** *Proc. Natl. Acad. Sci.,* 1981, vol. 12, 7575
- **O'REILLCY et al.** Baculovirus Expression Vectors: A Laboratory Manual. Oxford University Press, 1994
- **RUPERT et al.** *Nature,* 1993, vol. 362, 175-179